# EUROPEAN PATENT SPECIFICATION

(11) **EP 3 870 582 B1**
(45) Date of publication and mention of the grant of the patent: **19.07.2023**
(21) Application number: 19789689.7
(22) Date of filing: 22.10.2019
(51) Int. Cl.: C07D 471/04, C07D 519/00, A61P 31/00, A61P 35/00

(54) **5-AZAINDAZOLE DERIVATIVES AS ADENOSINE RECEPTOR ANTAGONISTS**
5-AZAINDAZOL-DERIVATE ALS ADENOSINREZEPTORANTAGONISTEN
DÉRIVÉS DE 5-AZAINDAZOLE UTILISÉS EN TANT QU'ANTAGONISTES DU RÉCEPTEUR DE L'ADÉNOSINE

(30) Priority: 25.10.2018 EP 18306389
(43) Date of publication of application: 01.09.2021
(73) Proprietor: Merck Patent GmbH, 64293 Darmstadt (DE)
(72) Inventor: BLAYO, Anne-Laure, 56580 CREDIN (FR); MANTEAU, Baptiste, 1474 WAYS (BE); DORANGE, Ismet, 12063 STOCKHOLM (SE); MAYER, Stanislas, 67114 ESCHAU (FR); SCHANN, Stephan, 67400 ILLKIRCH (FR); CATELAIN, Thomas, 67000 STRASBOURG (FR)
(86) International application number: PCT/EP2019/078665
(87) International publication number: WO 2020/083878

(56) References cited:
- WO-A1-2016/008590
- WO-A1-2017/028314

## Description

### Field of the invention

The present invention relates to novel 5-azaindazole derivatives of formula (I), as described and defined herein below, and pharmaceutically acceptable salts, solvates and prodrug thereof, as well as pharmaceutical compositions comprising such compounds. The 5-azaindazole derivatives according to the invention have been found to be highly effective dual A_{2A}/A_{2B} adenosine receptor antagonists, and can thus be used as therapeutic agents, particularly in the treatment or prevention of hyperproliferative or infectious diseases or disorders.

### Background of the invention

Adenosine is an ubiquitous modulator of numerous physiological activities, particularly within the cardiovascular, nervous and immune systems. Adenosine is related both structurally and metabolically to the bioactive nucleotides adenosine triphosphate (ATP), adenosine diphosphate (ADP), adenosine monophosphate (AMP) and cyclic adenosine monophosphate (cAMP), to the biochemical methylating agent S-adenosyl-L-methione (SAM) and structurally to the coenzymes NAD, FAD and coenzyme A and to RNA.

Via cell surface receptors, adenosine modulates diverse physiological functions including induction of sedation, vasodilatation, suppression of cardiac rate and contractility, inhibition of platelet aggregability, stimulation of gluconeogenesis and inhibition of lipolysis. Studies show that adenosine is able to activate adenylate cyclases, open potassium channels, reduce flux through calcium channels, and inhibit or stimulate phosphoinositide turnover through receptor-mediated mechanisms (Muller C. E. and Stein B., Current Pharmaceutical Design, 2: 501, 1996; Muller C. E., Exp. Opin. Ther. Patents, 7(5): 419, 1997).

Adenosine receptors belong to the superfamily of G-protein-coupled receptors (GPCRs). Four major subtypes of adenosine receptors have been pharmacologically, structurally and functionally characterized (Fredholm et al., Pharm. Rev., 46: 143-156, 1994) and referred to as A₁, A_{2A}, A_{2B} and A₃. Though the same adenosine receptor can couple to different G-proteins, adenosine A₁ and A₃ receptors usually couple to inhibitory G-proteins referred to as Gᵢ and G₀ which inhibit adenylate cyclase and down-regulate cellular cAMP levels. In contrast, the adenosine A_{2A} and A_{2B} receptors couple to stimulatory G-proteins referred to as Gₛ that activate adenylate cyclase and increase intracellular levels of cAMP (Linden J., Annu. Rev. Pharmacol. Toxicol., 41: 775-87 2001).

According to the invention, "adenosine-receptor-selective ligands" are substances which bind selectively to one or more subtypes of the adenosine receptors, thus either mimicking the action of adenosine (adenosine agonists) or blocking its action (adenosine antagonists). According to their receptor selectivity, adenosine-receptor-selective ligands can be divided into different categories, for example ligands which bind selectively to the A₁ or A₂ receptors and in the case of the latter also, for example, those which bind selectively to the A_{2A} or the A_{2B} receptors. Also possible are adenosine receptor ligands which bind selectively to a plurality of subtypes of the adenosine receptors, for example ligands which bind selectively to the A₁ and the A₂, but not to the A₃ receptors. The above-mentioned receptor selectivity can be determined by the effect of the substances on cell lines which, after stable transfection with the corresponding cDNA, express the receptor subtypes in question (Olah, M. E. et al., J. Biol. Chem., 267: 10764-10770, 1992). The effect of the substances on such cell lines can be monitored by biochemical measurement of the intracellular messenger cAMP (Klotz, K. N. et al., Naunyn Schmiedebergs Arch. Pharmacol. 357: 1-9, 1998).

It is known that the A₁ receptor system includes the activation of phospholipase C and modulation of both potassium and calcium ion channels. The A₃ subtype, in addition to its association with adenylate cyclase, also stimulates phospholipase C and so activates calcium ion channels.

The A₁ receptor (326-328 amino acids) was cloned from various species (canine, human, rat, dog, chick, bovine, guinea-pig) with 90-95 % sequence identify among the mammalian species. The A_{2A} receptor (409-412 amino acids) was cloned from canine, rat, human, guinea pig and mouse. The A_{2B} receptor (332 amino acids) was cloned from human and mouse with 45 % homology of human A_{2B} with human A₁ and A_{2A} receptors. The A₃ receptor (317-320 amino acids) was cloned from human, rat, dog, rabbit and sheep.

The A₁ and A_{2A} receptor subtypes are proposed to play complementary roles in adenosine's regulation of the energy supply. Adenosine, which is a metabolic product of ATP, diffuses from the cell and acts locally to activate adenosine receptors to decrease the oxygen demand (A₁ and A₃) or increase the oxygen supply (A_{2A}) and so reinstate the balance of energy supply / demand within the tissue. The action of both subtypes is to increase the amount of available oxygen to tissue and to protect cells against damage caused by a short-term imbalance of oxygen. One of the important functions of endogenous adenosine is preventing damage during traumas such as hypoxia, ischaemia, hypotension and seizure activity. Furthermore, it is known that the binding of the adenosine receptor agonist to mast cells expressing the rat A₃ receptor resulted in increased inositol triphosphate and intracellular calcium concentrations, which potentiated antigen induced secretion of inflammatory mediators. Therefore, the A₃ receptor plays a role in mediating asthmatic attacks and other allergic responses.

These adenosine receptors are encoded by distinct genes and are classified according to their affinities for adenosine analogues and methylxanthine antagonists (Klinger et al., Cell Signal., 14 (2): 99-108, 2002).

Concerning the role of adenosine on the nervous system, the first observations were made on the effects of the most widely used of all psychoactive drugs being caffeine. Actually, caffeine is a well-known adenosine receptor antagonist that is able to enhance the awareness and learning abilities of mammals. The adenosine A_{2A} receptor pathway is responsible for these effects (Fredholm et al., Pharmacol. Rev., 51 (1): 83-133, 1999; Huang et al., Nat Neurosci., 8 (7): 858-9, 2005), and the effects of caffeine on the adenosine A_{2A} receptor signaling pathway encouraged the research of highly specific and potent adenosine A_{2A} antagonists.

In mammals, adenosine A_{2A} receptors have a limited distribution in the brain and are found in the striatum, olfactory tubercle and nucleus acumbens (Dixon et al., Br. J. Pharmacol., 118 (6): 1461-8, 1996). High and intermediate levels of expression can be observed in immune cells, heart, lung and blood vessels. In the peripheral system, G₃ seems to be the major G-protein associated with adenosine A_{2A} receptor but in the striatum, it has been shown that striatal adenosine A_{2A} receptors mediate their effects through activation of a G-protein referred to as Gₒ (Kull et al., Mol. Pharmacol., 58 (4): 772-7, 2000), which is similar to G₃ and also couples to adenylate cyclase.

To date, studies on genetically modified mice and pharmacological analysis suggest that A_{2A} receptor is a promising therapeutic target for the treatment of central nervous system (CNS) disorders and diseases such as Parkinson's disease, Huntington's disease, attention deficit hyperactivity disorders (ADHD), stroke (ischemic brain injury), and Alzheimer's disease (Fredholm et al., Annu. Rev. Pharmacol. Toxicol., 45: 385-412, 2005; Higgins et al.; Behav. Brain Res. 185: 32-42, 2007; Dall' Igna et al., Exp. Neurol., 203 (1): 241-5, 2007; Arendash et al., Neuroscience, 142 (4): 941-52, 2006; Trends in Neurosci., 29 (11), 647-654, 2006; Expert Opinion Ther. Patents, 17, 979-991, 2007, Exp. Neurol., 184 (1), 285-284, 2003, Prog. Brain Res, 183, 183-208, 2010, J. Alzheimer Dis., Suppl 1, 1 17-126, 2010, J. Neurosci., 29 (47), 14741-14751, 2009, Neuroscience, 166 (2), 590-603, 2010, J. Pharmacol. Exp. Ther., 330 (1), 294-303, 2009; Frontiers Biosci., 13, 2614-2632, 2008) but also for various psychoses of organic origin (Weiss et al., Neurology, 61 (11 Suppl 6): 88-93, 2003).

The use of adenosine A_{2A} receptor knockout mice has shown that adenosine A_{2A} receptor inactivation protects against neuronal cell death induced by ischemia (Chen et al., J. Neurosci., 19 (21): 9192-200, 1999 and Monopoli et al., Neuroreport, 9 (17): 3955-9, 1998) and the mitochondrial toxin 3-NP (Blum et al., J. Neurosci., 23 (12): 5361-9, 2003). Those results provided a basis for treating ischaemia and Huntington's disease with adenosine A_{2A} antagonists. The blockade of adenosine A_{2A} receptors has also an antidepressant effect (El Yacoubi et al., Neuropharmacology, 40 (3): 424-32, 2001). Finally, this blockade prevents memory dysfunction (Cunha et al., Exp. Neurol., 210 (2): 776-81, 2008; Takahashi et al., Front. Biosci., 13: 2614-32, 2008) and this could be a promising therapeutic route for the treatment and/or prevention of Alzheimer's disease.

For reviews concerning A_{2A} adenosine receptors see e.g. Moreau et al. (Brain Res. Reviews 31: 65-82, 1999) and Svenningsson et al. (Progress in Neurobiology 59: 355-396, 1999).

To date, several adenosine A_{2A} receptor antagonists have shown promising potential for treatment of Parkinson's disease. As an example, KW-6002 (Istradefylline) completed a phase III clinical trial in the USA after studies demonstrated its efficacy in alleviation of symptoms of the disease (Bara-Himenez et al., Neurology, 61 (3): 293-6, 2003 and Hauser et al., Neurology, 61 (3): 297-303, 2003). SCH420814 (Preladenant), which is now in phase II clinical trial in the USA, produces an improvement in motor function in animal models of Parkinson's disease (Neustadt et al., Bioorg. Med. Chem. Lett., 17 (5): 1376-80, 2001) and also in human patients (Hunter J. C, poster Boston 2006 - http://www.a2apd.org/Speaker abstracts/Hunter. pdf).

Besides the welcome utility of A_{2A} receptor antagonists to treat neurodegenerative diseases, those compounds have been considered for complementary symptomatic indications. These are based on the evidence that A_{2A} receptor activation may contribute to the pathophysiology of a range of neuropsychiatric disorders and dysfunctions such as depression, excessive daytime sleepiness, restless legs syndrome, attention deficit hyperactivity disorder, and cognitive fatigue (Neurology, 61 (Suppl 6), 82-87, 2003; Behav. Pharmacol., 20 (2), 134-145, 2009; CNS Drug Discov., 2 (1), 1-21, 2007).

Some authors suggest the application of A_{2A} antagonists for the treatment of diabetes (WO1999035147; WO2001002400). Other studies suggest the involvement of A_{2A} adenosine receptors in wound healing or atrial fibrillation (Am. J. Path., 6, 1774-1778, 2007; Arthritis & Rheumatism, 54 (8), 2632-2642, 2006).

Some of the potent adenosine A_{2A} antagonists discovered in the past by the pharmaceutical companies, have advanced into clinical trials showing positive results and demonstrating the potential of this compound class for the treatment of neurodegenerative disorders like Parkinson's, Huntington's or Alzheimer's disease, but also in other CNS related diseases like depression, restless legs syndrome, sleep and anxiety disorders (Clin. Neuropharmacol., 33, 55-60, 2010; J. Neurosci., 30 (48), 2010), 16284-16292; Parkinson Relat. Disord., 16 (6), 423-426, 2010; Expert Opinion Ther. Patents, 20(8), 987-1005, 2010; Current Opinion in Drug Discovery & Development, 13 (4), 466-480, 2010 and references therein; Mov. Disorders, 25 (2), S305, 2010).

Known A_{2A} inhibitors are Istradefylline (KW-6002), Preladenant (SCH420814), SCH58261, CGS15943, Tozadenant, Vipadenant (V-2006), V-81444 (CPI-444, HTL-1071, PBF-509, Medi-9447, PNQ-370, ZM-241385, ASO-5854, ST-1535, ST-4206, DT1133 and DT0926, which are in most cases developed for Parkinson's disease.

Adenosine A_{2B} receptors were cloned from rat hypothalamus (Rivkees and Reppert, Mol Endocrinol. 1992 Oct;6(10):1598-604), human hippocampus (Pierce et al., Biochem Biophys Res Commun. 1992 Aug 31;187(1):86-93), and mouse mast cells (Marquardt et al., J Immunol. 1994 May 1;152(9):4508-15), employing standard polymerase chain reaction techniques with degenerate oligonucleotide primers designed to recognize conserved regions of most G protein-coupled receptors. The human A_{2B} receptor shares 86 to 87% amino acid sequence homology with the rat and mouse A_{2B} receptors (Rivkees and Reppert, 1992; Pierce et al., 1992; Marquardt et al., 1994) and 45% amino acid sequence homology with human A₁ and A_{2A} receptors. As expected for closely related species, the rat and mouse A_{2B} receptors share 96% amino acid sequence homology. By comparison, the overall amino acid identity between A₁ receptors from various species is 87% (Palmer and Stiles, Neuropharmacology, 1995 Jul;34(7):683-94). A_{2A} receptors share 90% of homology between species (Ongini and Fredholm, Trends Pharmacol Sci. 1996 Oct; 17(10): 364-72), with most differences occurring in the 2^{nd} extracellular loop and the long C-terminal domain (Palmer and Stiles, 1995). The lowest (72%) degree of identity between species is observed for A₃ receptor sequences (Palmer and Stiles, 1995).

The adenosine analog NECA remains the most potent A_{2B} agonist (Bruns, Biochem Pharmacol. 1981 Feb 15; 30(4): 325-33; Feoktistov and Biaggioni, Mol. Pharmacol. 1993 Jun; 43(6):909-14, Pharmacol. Rev. 1997 Dec;49(4):381-402; Brackett and Daly, Biochem Pharmacol, 1994 Mar 2;47(5):801-14), with a concentration producing a half-maximal effect (EC₅₀) for stimulation of adenyl cyclase of approximately 2 µM. It is, however, nonselective and activates other adenosine receptors with even greater affinity, with an EC₅₀ in the low nanomolar (A₁ and A_{2A}) or high nanomolar (A₃) range. The characterization of A_{2B} receptors, therefore, often relies on the lack of effectiveness of compounds that are potent and selective agonists of other receptor types. A_{2B} receptors have been characterized by a method of exclusion, i.e., by the lack of efficacy of agonists that are specific for other receptors. The A_{2A} selective agonist CGS-21680 (Webb et al., J Biol Chem. 1992 Dec 5;267(34):24661-8), for example, has been useful in differentiating between A_{2A} and A_{2B} adenosine receptors (Hide et al., Mol Pharmacol. 1992 Feb;41(2):352-9; Chern et al., Mol Pharmacol. 1993 Nov;44(5):950-8; Feoktistov and Biaggioni, J Clin Invest. 1995 Oct;96(4):1979-86; van der Ploeg et al., Naunyn Schmiedebergs Arch Pharmacol. 1996 Feb; 353(3): 250-60). Both receptors are positively coupled to adenyl cyclase and are activated by the nonselective agonist NECA. CGS-21680 is virtually ineffective on A_{2B} receptors but is as potent as NECA in activating A_{2A} receptors, with an EC₅₀ in the low nanomolar range for both agonists (Jarvis et al., Brain Res. 1989 Apr 10;484(1-2):111-8; Nakane and Chiba, Heart Vessels. 1990; 5(2):71-5; Webb et al., 1992; Hide et al., 1992; Feoktistov and Biaggioni, 1993; Alexander et al., BrJ Pharmacol. 1996 Nov, 119(6):1286-90). A_{2B} receptors have also a very low affinity for the A₁ selective agonist R-PIA (Feoktistov and Biaggioni, 1993; Brackett and Daly, Biochem Pharmacol. 1994 Mar 2;47(5):801-14) as well as for the A₃ selective agonist N⁶-(3-iodobenzyl)-N-methyl-5'-carbamoyladenosine (IB-MECA) (Feoktistov and Biaggioni, 1997). The agonist profile NECA > R-PIA = IB-MECA > CGS-21680 was determined in human erythroleukemia (HEL) cells for A_{2B}-mediated cAMP accumulation. The difference between EC₅₀ for NECA and the rest of the agonists is approximately 2 orders of magnitude. Therefore, responses elicited by NECA at concentrations in the low micromolar range (1-10 µM), but not by R-PIA, IB-MECA or CGS-21680, are characteristic of A_{2B} receptors.

Whereas A_{2B} receptors have, in general, a lower affinity for agonists compared to other receptor subtypes, this is not true for antagonists. The structure activity relationship of adenosine antagonists on A_{2B} receptors has not been fully characterized, but at least some xanthines are as or more potent antagonists of A_{2B} receptor subtypes than of other subtypes. In particular, DPSPX (1,3-dipropyl-8-sulphophenylxanthine), DPCPX (1,3-dipropyl-8-cyclopentylxanthine), DPX (1,3-diethylphenylxanthine), the antiasthmatic drug enprofylline (3-n-propylxanthine) and the non-xanthine compound 2,4-dioxobenzopteridine (alloxazine) have affinities in the mid to high nM range.

Other known A_{2B} inhibitors are ATL801, PSB-605, PSB-1115, ISAM-140, GS6201, MRS1706 and MRS1754.

It is disclosed herein that adenosine receptors play a non-redundant role in down-regulation of inflammation in vivo by acting as a physiological "STOP" (a termination mechanism) that can limit the immune response and thereby protect normal tissues form excessive immune damage during pathogenesis of different diseases.

A_{2A} receptor antagonists provide long term enhancement of immune responses by reducing T-cell mediated tolerance to antigenic stimuli, enhancing the induction of memory T cells and enhancing the efficacy of passive antibody administration for the treatment of cancer and infectious diseases while A_{2A} receptor agonists provide long term reduction of immune responses by enhancing T-cell mediated tolerance to antigenic stimuli, in particular to reduce use of immunosuppressive agents in certain conditions.

Immune modulation is a critical aspect of the treatment of a number of diseases and disorders. T cells in particular play a vital role in fighting infections and have the capability to recognize and destroy cancer cells. Enhancing T cell mediated responses is a key component to enhancing responses to therapeutic agents. However, it is critical in immune modulation that any enhancement of an immune response is balanced against the need to prevent autoimmunity as well as chronic inflammation. Chronic inflammation and self-recognition by T cells is a major cause for the pathogenesis of systemic disorders such as rheumatoid arthritis, multiple sclerosis and systemic lupus erythematosus. Furthermore, long term immune-suppression is required in preventing rejection of transplanted organs or grafts.

Tumor-induced immunosuppression is a major hurdle to the efficacy of current cancer therapies. Because of their remarkable clinical efficacy against a broader range of cancers, recent successes with immune checkpoint blockade inhibitors such as anti-CTLA-4 and anti-PD-1/PDL1 are revolutionizing cancer treatment.

Adenosine is one of the new promising immunosuppressive targets revealed in preclinical studies. This metabolite is produced by the ectoenzyme - CD73 expressed on host suppressor cells and tumor cells. Increased expression of CD73 correlates with poor prognosis in patients with a number of cancers, including colorectal cancer (Liu et al, J. Surgical Oncol, 2012), gastric cancer (Lu et al., World J. Gastroenterol., 2013), gallbladder cancer (Xiong et al., Cell and Tissue Res., 2014). Preclinical studies demonstrated that protumor effects of CD73 can be driven (at least in part) by adenosine-mediated immunosuppression. As disclosed above, adenosine binds to four known receptors A₁, A_{2A}, A_{2B}, and A₃, with the activation of A_{2A} and A_{2B} receptors known to suppress the effector functions of many immune cells, i.e. A_{2A} and A_{2B} receptors induce adenylate-cyclase-dependent accumulation of cAMP leading to immunosuppression. Since antagonizing A₁ and A₃ would counteract the desired effect and A₁ and A₃ agonists serve as potential cardioprotective agents, selectivity towards A₁ and A₃ needs to be achieved (Antonioli et al., Nat. rev. Cancer, 2013, Thiel et al., Microbes and Infection, 2003). In the microenvironment of the tumor, both A_{2A} and A_{2B} receptor activation has been demonstrated to suppress antitumor immunity and increase the spread of CD73 tumors. In addition, either A_{2A} or A_{2B} blockade with small molecule antagonists can reduce tumor metastasis. It has been found that blocking of A_{2A} receptor can overcome tumor escape mechanisms including both anergy and regulatory T cell induction caused by tumor cells and cause long-term tumor susceptibility to treatment. Ohta et al. demonstrated rejection of approximately 60% of established CL8-1 melanoma tumors in A_{2A} receptor-deficient mice compared to no rejection in normal mice (Ohta, et al.; PNAS 103 (35): 13132-7, 2006). In agreement, the investigators also showed improved inhibition of tumor growth, destruction of metastases and prevention of neovascularization by anti-tumor T cells after treatment with an A_{2A} receptor antagonist.

Tumors have been shown to evade immune destruction by impeding T cell activation through inhibition of co-stimulatory factors in the B7-CD28 and TNF families, as well as by attracting regulatory T cells, which inhibit anti-tumor T cell responses (Wang, Cancer. Semin. Cancer. Biol. 16: 73-79, 2006; Greenwald, et al., Ann. Rev. Immunol. 23: 515-48, 2005; Watts, Ann. Rev. Immunol. 23: 23-68, 2005; Sadum et al., Clin. Cane. Res. 13 (13): 4016-4025, 2007). Because A_{2A} receptor expression is increased in lymphocytes following activation, therapies that liberate lymphocyte effector responses, such as anti-CTLA-4 and anti-PD-1, may also increase the effects of A_{2A}-mediated immunosuppression. Immune checkpoint blockade in combination with A_{2A} or dual A_{2A/2B} antagonists increase the magnitude of immune responses to tumors and metastasis. Accordingly, combination of A_{2A} inhibition with anti-PD-1 therapy enhances IFN-γ production by T-cells in a co-culture with MC38 tumor cells, improves mouse survival in 4T1 mammary tumor model and decreases tumor growth in AT-3ova^{dim} CD73⁺ tumors (Beavis et al., Cancer Immunol. Res., 2015; Mittal et al., Cancer Res., 2014).

Furthermore, preclinical studies demonstrated that A_{2B} inhibition leads to decreased tumor growth and extended survival of mice in Lewis lung carcinoma, MB49 bladder carcinoma, ortho 4T1 mammary carcinoma models (Ryzhov et al., Purinergic Signal. 2009 Sep;5(3):289-98, Cekic et al., J Immunol. 2012 Jan 1;188(1):198-205) and the combination of A_{2B} inhibition with anti-PD-1 therapy reduces lung metastases of B16-F10 melanoma tumors and improves mouse survival in the 4T1 mammary tumor model.

WO 03/050241 describes the methods to increase an immune response to an antigen, increasing vaccine efficacy or increasing an immune response to a tumor antigen or immune cell-mediated tumor destruction by administering an agent that inhibits extracellular adenosine or inhibits adenosine receptors.

WO 2004/089942, WO 2005/000842 and WO 2006/008041 disclose benzothiazole derivatives and WO 2010/084425 discloses imidazopyridine-carbonyl-benzamide derivatives as A_{2A} inhibitors for the treatment of Parkinson's disease. WO 2004/092171 and WO 2005/028484 disclose similar thiazolopyridine and pyrazolo-pyrimidine derivatives also as A_{2A} inhibitors for the treatment of Parkinson's disease. However, these compounds do not show significant A_{2B} inhibitory activity and do only show good pharmacokinetic properties in the rat, the Parkinson's disease animal model but not in the mouse, the cancer animal model. Furthermore, the compounds do not show that they are able to prevent immunosuppression and thus are able to support anti-tumor T cell induced inhibition of tumor growth, reduction or destruction of metastases and prevention of neovascularization.

Furthermore, WO 2017/028314, WO 2008/112695 and WO 2014/052563 disclose pyrazolo fused heterocyclic compounds as ERK or protein kinase inhibitors, respectively.

Thus, there remains a need for therapies that provide long term enhancement of immune responses to specific antigens, particularly for the treatment and prevention of hyperproliferative and infectious diseases and disorders. The object of the present invention is thus to provide methods of treatment that allow simplified treatment protocols and enhance immune responses against certain antigens. It is a specific object of the invention to provide improved methods of preventing or treating hyperproliferative and infectious diseases and disorders in a host, especially to provide effective dual A_{2A/2B} antagonists for the treatment and prevention of such diseases.

### Summary of the invention

Surprisingly, it has been found that the 5-azaindazole derivatives according to the present invention are highly effective inhibitors of both the A_{2A} and A_{2B} adenosine receptors and can thus be used as therapeutic agents, particularly for the treatment or prevention of hyperproliferative diseases and disorders (such as, e.g., cancer) as well as infectious diseases and disorders.

Particularly, in contrast to the known monospecific adenosine A_{2A} receptor antagonists, e.g. the compounds disclosed in WO 2010/084425, the compounds of the present invention surprisingly show an A_{2A}/A_{2B} dual activity which is preferred for the treatment and/or prevention of hyperproliferative and infectious diseases and disorders as it is disclosed above.

Furthermore, as discussed above, adenosine in tumor microenvironment can inhibit T cell activity by signaling through A_{2A} receptors and suppress cytokine secretion by T cells. A_{2A} specific agonists like NECA, similarly to adenosine, inhibit T cell cytokine secretion in vitro and in vivo. In contrast, potential A_{2A} antagonists or A_{2A}/A_{2B} dual antagonists can rescue T cells from this inhibition. The compounds of the present invention are able to prevent the suppression of cytokine secretion as induced by adenosine or A_{2A} specific agonists like CGS-2168, which is preferred for the treatment and/or prevention of hyperproliferative and infectious diseases and disorders as it is disclosed above. Therefore, the compounds of the present invention surprisingly are able to prevent immunosuppression and thus are able to support anti-tumor T cell induced inhibition of tumor growth, reduction or destruction of metastases and prevention of neovascularization.

The present invention provides a 5-azaindazole derivative of the following formula (I) or a pharmaceutically acceptable salt, or solvate thereof:

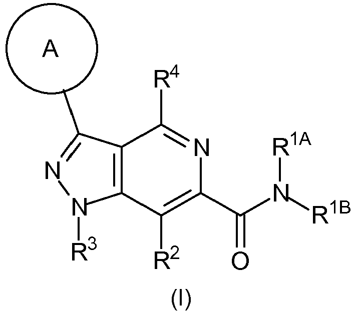

In formula (I), the groups R^{1A} and R^{1B} are mutually linked to form, together with the nitrogen atom that they are attached to, a heterocycloalkyl which is optionally substituted with one or more (e.g., one, two or three) groups R¹¹; or, alternatively, R^{1A} and R^{1B} are each independently selected from hydrogen, C₁₋₅ alkyl, C₂₋₅ alkenyl, C₂₋₅ alkynyl, -CO(C₁₋₅ alkyl), carbocyclyl, and heterocyclyl, wherein said alkyl, said alkenyl, said alkynyl, and the alkyl moiety of said -CO(C₁₋₅ alkyl) are each optionally substituted with one or more groups R¹², and further wherein said carbocvclvl and said heterocyclyl are each optionally substituted with one or more groups R¹³.

Each R¹¹ is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CON(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

Each R¹² is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

Each R¹³ is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CON(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-cycloalkyl, and -(C₀₋₃ alkylene)-heterocycloalkyl.

R² and R⁴ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CON(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂C₁₋₅ alkyl), -SO-(C₁₋₅ alkyl), -SO₂-(C₁₋₅ alkyl), cycloalkyl, and heterocycloalkyl.

R³ is selected from hydrogen, C₁₋₅ alkyl, C₂₋₅ alkenyl, C₂₋₅ alkynyl, -(C₁₋₅ alkylene)-OH, -(C₁₋₅ alkylene)-O(C₁₋₅ alkyl), -(C₁₋₅ alkylene)-O(C₁₋₅ alkylene)-OH, -(C₁₋₅ alkylene)-O(C₁₋₅ alkylene)-O(C₁₋₅ alkyl), -(C₁₋₅ alkylene)-SH, -(C₁₋₅ alkylene)-S(C₁₋₅ alkyl), -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -(C₁₋₅ alkylene)-halogen, C₁₋₅ haloalkyl, -(C₁₋₅ alkylene)-O-(C₁₋₅ haloalkyl), -(C₁₋₅ alkylene)-CF₃, -(C₁₋₅ alkylene)-CN, -(C₁₋₅ alkylene)-NO₂, -(C₁₋₅ alkylene)-Si(C₁₋₅ alkyl)₃, -(C₁₋₅ alkylene)-O(C₁₋₅ alkylene)-Si(C₁₋₅ alkyl)₃, -(C₁₋₅ alkylene)-CHO, -(C₁₋₅ alkylene)-CO-(C₁₋₅ alkyl), -(C₁₋₅ alkylene)-COOH, -(C₁₋₅ alkylene)-CO-O-(C₁₋₅ alkyl), -(C₁₋₅ alkylene)-O-CO-(C₁₋₅ alkyl), -(C₁₋₅ alkylene)-CO-NH₂, -(C₁₋₅ alkylene)-CO-NH(C₁₋₅ alkyl), -(C₁₋₅ alkylene)-CO-N(C₁₋₅ alkyl)(C₁₋₅ alkyl), -(C₁₋₅ alkylene)-NH-CO-(C₁₋₅ alkyl), -(C₁₋₅ alkylene)-N(C₁₋₅ alkyl)-CO-(C₁₋₅ alkyl), -(C₁₋₅ alkylene)-NH-CO-O-(C₁₋₅ alkyl), -(C₁₋₅ alkylene)-N(C₁₋₅ alkyl)-CO-O-(C₁₋₅ alkyl), -(C₁₋₅ alkylene)-O-CO-NH-(C₁₋₅ alkyl), -(C₁₋₅ alkylene)-O-CON(C₁₋₅ alkyl)-(C₁₋₅ alkyl), -(C₁₋₅ alkylene)-SO₂-NH₂, -(C₁₋₅ alkylene)-SO₂-NH(C₁₋₅ alkyl), -(C₁₋₅ alkylene)-SO₂-N(C₁₋₅ alkyl)(C₁₋₅ alkyl), -(C₁₋₅ alkylene)-NH-SO₂-(C₁₋₅ alkyl), -(C₁₋₅ alkylene)-N(C₁₋₅ alkyl)-SO₂C₁₋₅ alkyl), -(C₁₋₅ alkylene)-SO-(C₁₋₅ alkyl), -(C₁₋₅ alkylene)-SO₂-(C₁₋₅ alkyl), -(C₀₋₅ alkylene)-carbocyclyl, and -(C₀₋₅ alkylene)-heterocyclyl, wherein the carbocyclyl moiety of said -(C₀₋₅ alkylene)-carbocyclyl and the heterocyclyl moiety of said -(C₀₋₅ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

The ring group A is a bicyclic heteroaryl, wherein said bicyclic heteroaryl is optionally substituted with one or more groups R^{A}.

Each R^{A} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CON(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

The present invention also relates to a pharmaceutical composition comprising a compound of formula (I) or a pharmaceutically acceptable salt, or solvate thereof, in combination with a pharmaceutically acceptable excipient. Accordingly, the invention relates to a compound of formula (I) or a pharmaceutically acceptable salt, or solvate thereof, or a pharmaceutical composition comprising any of the afore-mentioned entities and a pharmaceutically acceptable excipient, for use as a medicament. Moreover, the pharmaceutical composition may also comprise one or more further therapeutic agents (or active compounds).

The invention likewise relates to a pharmaceutical preparation comprising a compound of formula (I) or a pharmaceutically acceptable salt, or solvate thereof. The pharmaceutical preparation optionally further comprises one or more excipients and/or adjuvants. Moreover, the pharmaceutical preparation may also comprise one or more further therapeutic agents (or active compounds).

The invention further relates to a compound of formula (I) or a pharmaceutically acceptable salt, or solvate thereof, or a pharmaceutical composition comprising any of the afore-mentioned entities and a pharmaceutically acceptable excipient, for use in the treatment or prevention of a hyperproliferative disease or disorder or an infectious disease or disorder.

Moreover, the present invention relates to the use of a compound of formula (I) or a pharmaceutically acceptable salt, or solvate thereof in the preparation of a medicament for the treatment or prevention of a hyperproliferative disease or disorder or an infectious disease or disorder.

### Detailed description of the invention

As explained above, it has surprisingly been found that the 5-azaindazole derivatives of formula (I) according to the present invention are highly effective dual inhibitors of the A_{2A} and A_{2B} adenosine receptors, as also demonstrated in the appended examples, which renders them particularly suitable as therapeutic agents, including for the treatment or prevention of a disease or disorder which is caused, promoted and/or propagated by adenosine or other A_{2A} and/or A_{2B} receptor agonists, or a disease or disorder which is connected to (or associated with) adenosine A_{2A} and/or A_{2B} receptors; in particular, the compounds of formula (I) can advantageously be used for the treatment or prevention of a hyperproliferative disease or disorder (such as, e.g., cancer) or an infectious disease or disorder.

The hyperproliferative disease or disorder to be treated or prevented in accordance with the present invention is preferably cancer. The cancer is preferably selected from the group consisting of acute granulocytic leukemia, acute lymphocytic leukemia, acute myeloid leukemia, adrenal cortex cancer, bladder cancer, brain cancer, breast cancer, cervical cancer, cervical hyperplasia, cervical cancer, chorio cancer, chronic granulocytic leukemia, chronic lymphocytic leukemia, chronic myeloid leukemia, colon cancer, endometrial cancer, esophageal cancer, essential thrombocytosis, genitourinary carcinoma, glioma, glioblastoma, hairy cell leukemia, head and neck carcinoma, Hodgkin's disease, Kaposi's sarcoma, lung carcinoma, lymphoma, malignant carcinoid carcinoma, malignant hypercalcemia, malignant melanoma, malignant pancreatic insulinoma, medullary thyroid carcinoma, melanoma, multiple myeloma, mycosis fungoides, neuroblastoma, non-Hodgkin's lymphoma, non-small cell lung cancer, osteogenic sarcoma, ovarian carcinoma, pancreatic carcinoma, polycythemia vera, primary brain carcinoma, primary macroglobulinemia, prostatic cancer, renal cell cancer, rhabdomyosarcoma, skin cancer, small-cell lung cancer, soft-tissue sarcoma, squamous cell cancer, stomach cancer, testicular cancer, thyroid cancer, and Wilms' tumor.

The hyperproliferative disease or disorder to be treated or prevented in accordance with the invention may also be selected from age-related macular degeneration, Crohn's disease, cirrhosis, a chronic inflammatory-related disorder, proliferative diabetic retinopathy, proliferative vitreoretinopathy, retinopathy of prematurity, granulomatosis, immune hyperproliferation associated with organ or tissue transplantation, and an immunoproliferative disease or disorder. The immunoproliferative disease/disorder is preferably selected from inflammatory bowel disease, psoriasis, rheumatoid arthritis, systemic lupus erythematosus (SLE), vascular hyperproliferation secondary to retinal hypoxia, and vasculitis.

Moreover, the infectious disease or disorder to be treated or prevented in accordance with the present invention is preferably selected from the group consisting of:
(a) virally induced infectious diseases, preferably those which are caused by retroviruses, hepadnaviruses, herpesviruses, flaviviridae and/or adenoviruses, wherein the retroviruses are preferably selected from lentiviruses or oncoretroviruses, wherein the lentivirus is preferably selected from the group consisting of HIV-1, HIV-2, FIV, BIV, SIVs, SHIV, CAEV, VMV and EIAV, wherein the oncoretrovirus is preferably selected from the group consisting of HTLV-I, HTLV-II and BLV, wherein the hepadnavirus is preferably selected from the group consisting of HBV, GSHV and WHV, wherein the herpesvirus is preferably selected from the group consisting of HSV I, HSV II, EBV, VZV, HCMV and HHV 8, and wherein the flaviviridae are preferably selected from the group consisting of HCV, West nile and Yellow Fever;
(b) bacterial infectious diseases which are caused by Gram-positive bacteria, wherein the Gram-positive bacteria are preferably selected from the group consisting of methicillin-susceptible and methicillin-resistant staphylococci (including, e.g., Staphylococcus aureus, Staphylococcus epidermidis, Staphylococcus haemolyticus, Staphylococcus hominis, Staphylococcus saprophyticus, and coagulase-negative staphylococci), glycopeptides-intermediate susceptible Staphylococcus aureus (GISA), penicillin-susceptible and penicillin-resistant streptococci (including, e.g., Streptococcus pneumoniae, Streptococcus pyogenes, Streptococcus agalactiae, Streptococcus avium, Streptococcus bovis, Streptococcus lactis, Streptococcus sanguis and Streptococci Group C (GCS), Streptococci Group G (GGS) and viridans streptococci), enterococci (including, e.g., vancomycin-susceptible and vancomycin-resistant strains such as Enterococcus faecalis and Enterococcus faecium), Clostridium difficile, Listeria monocytogenes, Corynebacterium jeikeium, Chlamydia spp (including, e.g., C. pneumoniae) and Mycobacterium tuberculosis;
(c) bacterial infectious diseases which are caused by Gram-negative bacteria, wherein the Gram-negative bacteria are preferably selected from the group consisting of the genus Enterobacteriaceae, including e.g. Escherichia spp. (including, e.g., Escherichia coli), Klebsiella spp., Enterobacter spp., Citrobacter spp., Serratia spp., Proteus spp., Providencia spp., Salmonella spp., Shigella spp., the genus Pseudomonas (including, e.g., P. aeruginosa), Moraxella spp. (including, e.g., M. catarrhalis), Haemophilus spp. and Neisseria spp.; and
(d) infectious diseases induced by intracellular active parasites which are preferably selected from the group consisting of the phylum Apicomplexa, Sarcomastigophora (including, e.g., Trypanosoma, Plasmodia, Leishmania, Babesia or Theileria), Cryptosporidia, Sacrocystida, Amoebia, Coccidia and Trichomonadia.

Since the compounds of formula (I) according to the present invention are highly efficient A_{2A} receptor antagonists, they can also be used in the treatment or prevention of movement disorders, acute and chronic pain, affective disorders, central and peripheric nervous system degeneration disorders, schizophrenia and related psychosis, cognitive disorders, attention disorders, central nervous system injury, cerebral ischemia, myocardial ischemia, muscle ischemia, sleep disorders, eye disorders, cardiovascular disorders, hepatic fibrosis, cirrhosis, fatty liver, substance abuse, Parkinson's disease, Alzheimer's disease or attention-deficit hyperactivity disorder.

The compound of formula (I) or the pharmaceutically acceptable salt, or solvate thereof, as provided in accordance with the present invention, will be described in more detail in the following:

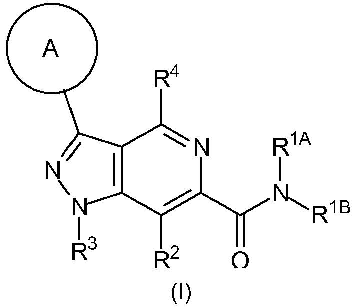

In formula (I), the groups R^{1A} and R^{1B} are mutually linked to form, together with the nitrogen atom that they are attached to, a heterocycloalkyl which is optionally substituted with one or more (e.g., one, two or three) groups R¹¹; or, alternatively, R^{1A} and R^{1B} are each independently selected from hydrogen, C₁₋₅ alkyl, C₂₋₅ alkenyl, C₂₋₅ alkynyl, -CO(C₁₋₅ alkyl), carbocyclyl (e.g., cycloalkyl or aryl), and heterocyclyl (e.g., heterocycloalkyl or heteroaryl), wherein said alkyl, said alkenyl, said alkynyl, and the alkyl moiety of said -CO(C₁₋₅ alkyl) are each optionally substituted with one or more (e.g., one, two or three) groups R¹², and further wherein said carbocyclyl and said heterocyclyl are each optionally substituted with one or more (e.g., one, two or three) groups R¹³.

Preferably, the groups R^{1A} and R^{1B} are mutually linked to form, together with the nitrogen atom that they are attached to, a heterocycloalkyl which is optionally substituted with one or more groups R¹¹.

The heterocycloalkyl which is formed from R^{1A}, R^{1B} and the nitrogen atom linking R^{1A} and R^{1B} (and which is optionally substituted with one or more groups R¹¹) is preferably a monocyclic, bicyclic or tricyclic heterocycloalkyl (e.g., a bicyclic fused, bridged or spirocyclic heterocycloalkyl, or a tricyclic fused, bridged and/or spirocyclic heterocycloalkyl); more preferably, it is a monocyclic heterocycloalkyl having 3, 4, 5, 6, 7 or 8 ring atoms (particularly 5, 6, 7 or 8 ring atoms), a bicyclic heterocycloalkyl (e.g., a bicyclic fused, bridged or spirocyclic heterocycloalkyl; particularly a bicyclic bridged heterocycloalkyl) wherein each ring of said bicyclic heterocycloalkyl independently has 3, 4, 5, 6, 7 or 8 ring atoms (particularly 5, 6, 7 or 8 ring atoms), or a tricyclic heterocycloalkyl (e.g., a tricyclic fused, bridged and/or spirocyclic heterocycloalkyl; particularly a tricyclic bridged heterocycloalkyl) wherein each ring of said tricyclic heterocycloalkyl independently has 3, 4, 5, 6, 7 or 8 ring atoms (particularly 5, 6, 7 or 8 ring atoms). In the heterocycloalkyl formed from R^{1A}, R^{1B} and the nitrogen atom linking R^{1A} and R^{1B}, one or more carbon ring atoms may optionally be oxidized (to form an oxo group -C(=O)-), one or more nitrogen ring atoms (if present) may optionally be oxidized, and/or one or more sulfur ring atoms (if present) may optionally be oxidized (e.g., to form a sulfonyl group -S(=O)₂-). It is furthermore preferred that the heterocycloalkyl (including any one of the aforementioned preferred examples of the heterocycloalkyl) contains one nitrogen ring atom (i.e., the nitrogen atom linking R^{1A} and R^{1B}) and optionally one further ring heteroatom selected from nitrogen, oxygen and sulfur (wherein said optional further ring heteroatom, if present, is preferably an oxygen ring atom), wherein the remaining ring atoms are all carbon atoms.

Examples of the heterocycloalkyl, which is formed from R^{1A}, R^{1B} and the nitrogen atom linking R^{1A} and R^{1B}, include any of the respective heterocycloalkyl groups comprised in any of the specific compounds of the invention disclosed herein below in the examples section. In particular, the heterocycloalkyl formed from R^{1A}, R^{1B} and the nitrogen atom linking R^{1A} and R^{1B} may be selected, for example, from any one of the following groups:

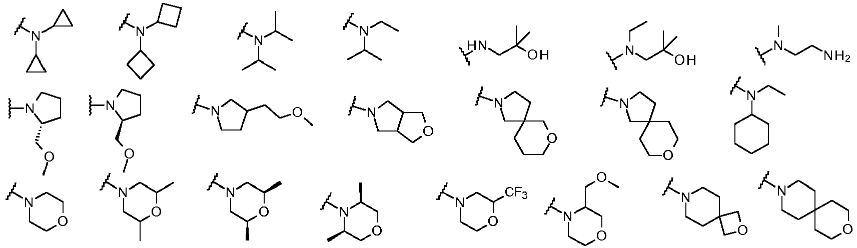

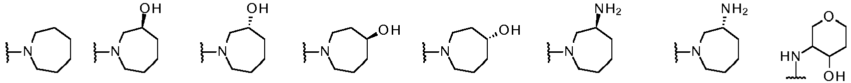

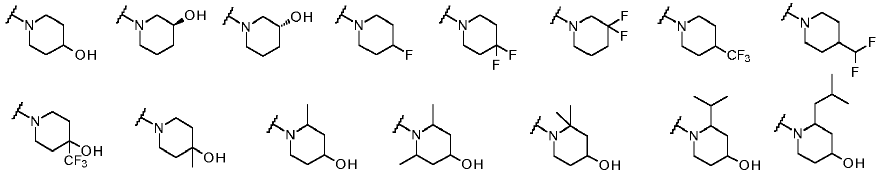

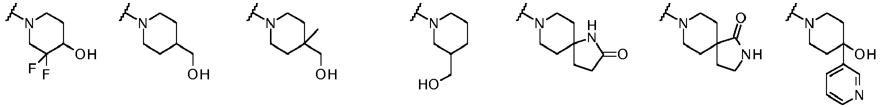

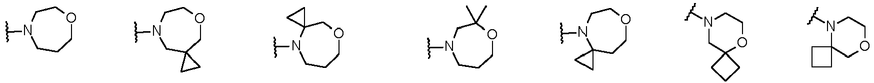

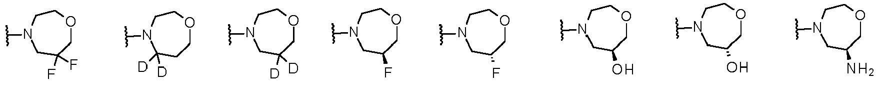

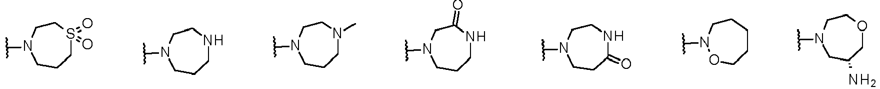

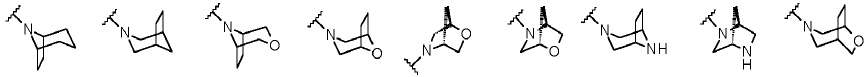

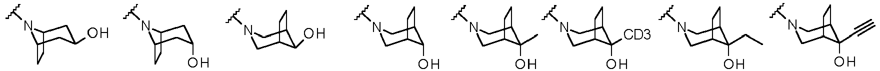

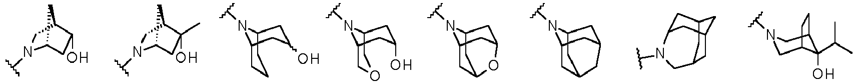

Particularly preferred examples of the heterocycloalkyl, which is formed from R^{1A}, R^{1B} and the nitrogen atom which R^{1A} and R^{1B} are attached to (and which is optionally substituted with one or more groups R¹¹), include 1,4-oxazepan-4-yl, piperidin-1-yl, 8-oxa-3-azabicyclo[3.2.1]octan-3-yl, or 8-azabicyclo[3.2.1]octan-8-yl, wherein said 1,4-oxazepan-4-yl, said piperidin-1-yl, said 8-oxa-3-azabicyclo[3.2.1]octan-3-yl, and said 8-azabicyclo[3.2.1]octan-8-yl are each optionally substituted with one or more groups R¹¹; said piperidin-1-yl is preferably substituted with -OH in position 4 (or with -OH and -CH₃ in position 4), and is optionally further substituted with one or more groups R¹¹; said 8-azabicyclo[3.2.1]octan-8-yl is preferably substituted with -OH in position 3 (particularly in endo-configuration; i.e., 3-endo-hydroxy-8-azabicyclo[3.2.1]octan-8-yl), and is optionally further substituted with one or more groups R¹¹; and said 1,4-oxazepan-4-yl may be substituted with -OH in position 6, and may optionally be further substituted with one or more groups R¹¹.

Accordingly, it is particularly preferred that R^{1A} and R^{1B} are mutually linked to form, together with the nitrogen atom that they are attached to, a group which is selected from 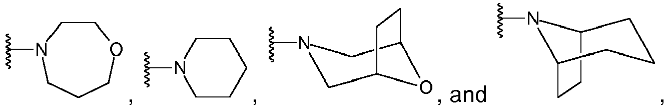 wherein each one of the above-depicted groups is optionally substituted with one or more R¹¹.

Even more preferably, R^{1A} and R^{1B} are mutually linked to form, together with the nitrogen atom that they are attached to, a group which is selected from 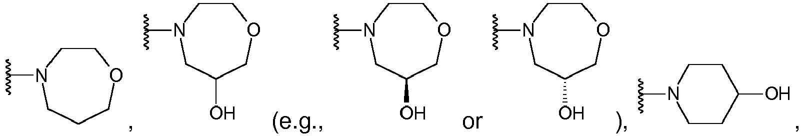 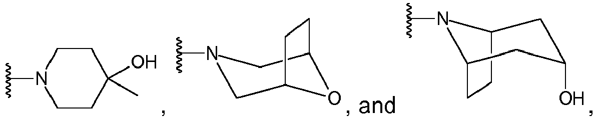 wherein each one of the above-depicted groups is optionally substituted with one or more R¹¹.

Yet even more preferably, R^{1A} and R^{1B} are mutually linked to form, together with the nitrogen atom that they are attached to, a group which is selected from 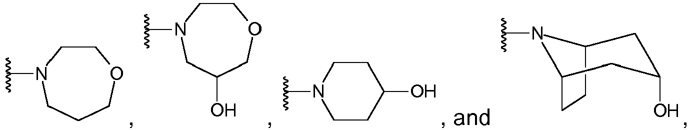 wherein each one of the above-depicted groups is optionally substituted with one or more R¹¹.

Most preferably, R^{1A} and R^{1B} are mutually linked to form, together with the nitrogen atom that they are attached to, a group 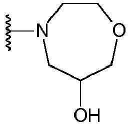 which is optionally substituted with one or more R¹¹.

Each R¹¹ is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CON(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more (e.g., one, two or three) groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CON(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

Preferably, each R¹¹ is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂C₁₋₅ alkyl), -SO-(C₁₋₅ alkyl), -SO₂-(C₁₋₅ alkyl), cycloalkyl, and heterocycloalkyl. More preferably, each R¹¹ is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN. Even more preferably, each R¹¹ is independently selected from C₁₋₄ alkyl (e.g., methyl or ethyl), -OH, -O(C₁₋₄ alkyl) (e.g., -OCH₃ or -OCH₂CH₃), -NH₂, -NH(C₁₋₄ alkyl) (e.g., -NHCH₃), -N(C₁₋₄ alkyl)(C₁₋₄ alkyl) (e.g., -N(CH₃)₂), halogen (e.g., -F, -Cl, -Br, or -I), -CF₃, and -CN.

Each R¹² is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

Preferably, each R¹² is independently selected from -OH, -O(C₁₋₄ alkyl), -NH₂, -NH(C₁₋₄ alkyl), -N(C₁₋₄ alkyl)(C₁₋₄ alkyl), halogen, -CF₃, and -CN.

Each R¹³ is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CON(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-cycloalkyl, and -(C₀₋₃ alkylene)-heterocyclo-alkyl.

Preferably, each R¹³ is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl. More preferably, each R¹³ is independently selected from C₁₋₄ alkyl, -OH, -O(C₁₋₄ alkyl), -NH₂, -NH(C₁₋₄ alkyl), -N(C₁₋₄ alkyl)(C₁₋₄ alkyl), halogen, -CF₃, and -CN.

R² and R⁴ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CON(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more (e.g., one, two or three) groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CON(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

Preferably, R² and R⁴ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-cycloalkyl, and -(C₀₋₃ alkylene)-heterocycloalkyl. More preferably, R² and R⁴ are each independently selected from hydrogen, C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂C₁₋₅ alkyl), -SO-(C₁₋₅ alkyl), -SO₂-(C₁₋₅ alkyl), cycloalkyl, and heterocycloalkyl. Even more preferably, R² and R⁴ are each independently selected from hydrogen, C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN. Yet even more preferably, R² and R⁴ are each independently selected from hydrogen, C₁₋₄ alkyl (e.g., methyl or ethyl), -OH, -O(C₁₋₄ alkyl) (e.g., -OCH₃ or -OCH₂CH₃), -NH₂, -NH(C₁₋₄ alkyl) (e.g., -NHCH₃), -N(C₁₋₄ alkyl)(C₁₋₄ alkyl) (e.g., -N(CH₃)₂), halogen (e.g., -F, -Cl, -Br, or -I), -CF₃, and -CN. Still more preferably, R² and R⁴ are each hydrogen.

R³ is selected from hydrogen, C₁₋₅ alkyl, C₂₋₅ alkenyl, C₂₋₅ alkynyl, -(C₁₋₅ alkylene)-OH, -(C₁₋₅ alkylene)-O(C₁₋₅ alkyl), -(C₁₋₅ alkylene)-O(C₁₋₅ alkylene)-OH, -(C₁₋₅ alkylene)-O(C₁₋₅ alkylene)-O(C₁₋₅ alkyl), -(C₁₋₅ alkylene)-SH, -(C₁₋₅ alkylene)-S(C₁₋₅ alkyl), -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -(C₁₋₅ alkylene)-halogen, C₁₋₅ haloalkyl, -(C₁₋₅ alkylene)-O-(C₁₋₅ haloalkyl), -(C₁₋₅ alkylene)-CF₃, -(C₁₋₅ alkylene)-CN, -(C₁₋₅ alkylene)-NO₂, -(C₁₋₅ alkylene)-Si(C₁₋₅ alkyl)₃, -(C₁₋₅ alkylene)-O(C₁₋₅ alkylene)-Si(C₁₋₅ alkyl)₃, -(C₁₋₅ alkylene)-CHO, -(C₁₋₅ alkylene)-CO-(C₁₋₅ alkyl), -(C₁₋₅ alkylene)-COOH, -(C₁₋₅ alkylene)-CO-O-(C₁₋₅ alkyl), -(C₁₋₅ alkylene)-O-CO-(C₁₋₅ alkyl), -(C₁₋₅ alkylene)-CO-NH₂, -(C₁₋₅ alkylene)-CO-NH(C₁₋₅ alkyl), -(C₁₋₅ alkylene)-CO-N(C₁₋₅ alkyl)(C₁₋₅ alkyl), -(C₁₋₅ alkylene)-NH-CO-(C₁₋₅ alkyl), -(C₁₋₅ alkylene)-N(C₁₋₅ alkyl)-CO-(C₁₋₅ alkyl), -(C₁₋₅ alkylene)-NH-CO-O-(C₁₋₅ alkyl), -(C₁₋₅ alkylene)-N(C₁₋₅ alkyl)-CO-O-(C₁₋₅ alkyl), -(C₁₋₅ alkylene)-O-CO-NH-(C₁₋₅ alkyl), -(C₁₋₅ alkylene)-O-CON(C₁₋₅ alkyl)-(C₁₋₅ alkyl), -(C₁₋₅ alkylene)-SO₂-NH₂, -(C₁₋₅ alkylene)-SO₂-NH(C₁₋₅ alkyl), -(C₁₋₅ alkylene)-SO₂-N(C₁₋₅ alkyl)(C₁₋₅ alkyl), -(C₁₋₅ alkylene)-NH-SO₂-(C₁₋₅ alkyl), -(C₁₋₅ alkylene)-N(C₁₋₅ alkyl)-SO₂C₁₋₅ alkyl), -(C₁₋₅ alkylene)-SO-(C₁₋₅ alkyl), -(C₁₋₅ alkylene)-SO₂-(C₁₋₅ alkyl), -(C₀₋₅ alkylene)-carbocyclyl, and -(C₀₋₅ alkylene)-heterocyclyl, wherein the carbocyclyl moiety of said -(C₀₋₅ alkylene)-carbocyclyl and the heterocyclyl moiety of said -(C₀₋₅ alkylene)-heterocyclyl are each optionally substituted with one or more (e.g., one, two or three) groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CON(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

Preferably, R³ is selected from C₁₋₅ haloalkyl, -(C₁₋₅ alkylene)-CN, -(C₁₋₅ alkylene)-SO-(C₁₋₅ alkyl) and -(C₁₋₅ alkylene)-SO₂-(C₁₋₅ alkyl). More preferably, R³ is selected from C₁₋₃ haloalkyl (e.g., -CH₂-CF₃, -CH₂-CHF₂, or-CH₂-CH₂F), -(C₁₋₃ alkylene)-CN (e.g.,-CH₂-CN), -(C₁₋₃ alkylene)-SO-(C₁₋₃ alkyl) (e.g., -CH₂-SO-CH₃) and -(C₁₋₃ alkylene)-SO₂-(C₁₋₃ alkyl) (e.g., -CH₂-SO₂-CH₃, -CH₂CH₂-SO₂-CH₃, or -CH₂-SO₂-CH₂CH₃). Even more preferably, R³ is selected from C₁₋₃ haloalkyl (particularly -CH₂-CF₃) and -(C₁₋₃ alkylene)-SO₂C₁₋₃ alkyl) (particularly -CH₂-SO₂-CH₃). Yet even more preferably, R³ is -CH₂-CF₃ or -CH₂-SO₂-CH₃.

The ring group A is a bicyclic heteroaryl, wherein said bicyclic heteroaryl is optionally substituted with one or more (e.g., one, two or three) groups R^{A}.

Preferably, ring A is a fused bicyclic heteroaryl (i.e., a heteroaryl composed of two rings which are fused; the two rings thus share two adjacent ring atoms), wherein said bicyclic heteroaryl is optionally substituted with one or more groups R^{A}. More preferably, ring A is a fused bicyclic heteroaryl, wherein each of the two fused rings (of said fused bicyclic heteroaryl) independently has 5 or 6 ring atoms, and wherein said bicyclic heteroaryl is optionally substituted with one or more groups R^{A}. Even more preferably, ring A is a fused bicyclic heteroaryl, wherein one of the two fused rings has 5 ring atoms and the other fused ring has 6 ring atoms, and further wherein said bicyclic heteroaryl is optionally substituted with one or more groups R^{A}. Yet even more preferably, ring A is a fused bicyclic heteroaryl, wherein each of the two fused rings is aromatic, wherein one of the two fused rings has 5 ring atoms and the other fused ring has 6 ring atoms, and further wherein said bicyclic heteroaryl is optionally substituted with one or more groups R^{A}; accordingly, it is particularly preferred that ring A is a fused bicyclic heteroaryl, wherein one of the two fused rings is a 5-membered aromatic ring and the other fused ring is a 6-membered aromatic ring, and further wherein said bicyclic heteroaryl is optionally substituted with one or more groups R^{A}.

If ring A is a fused bicyclic heteroaryl composed of a 5-membered ring and a 6-membered ring (e.g., as described herein above), wherein the bicyclic heteroaryl is optionally substituted with one or more groups R^{A}, it is furthermore preferred that the corresponding bicyclic heteroaryl is attached via its fused 5-membered ring to the 5-azaindazole moiety of the compound of formula (I). Even more preferably, the bicyclic heteroaryl is attached to the 5-azaindazole moiety of the compound of formula (I) via a ring atom of its fused 5-membered ring, which ring atom is adjacent to one of the two ring atoms that are shared by the two fused rings, as illustrated in the following (the heteroatoms comprised in the fused bicyclic heteroaryl are not shown):

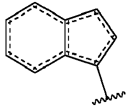

It is particularly preferred that ring A is a bicyclic heteroaryl having the following formula (II): 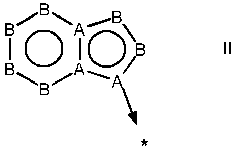 wherein each of the two fused rings (of the bicyclic heteroaryl) is aromatic, wherein each ring atom A (of the bicyclic heteroaryl) is independently a carbon or nitrogen atom, wherein each ring atom B is independently a carbon, nitrogen, oxygen or sulfur atom, wherein at least one of the ring atoms A and/or B is different from carbon, and wherein said bicyclic heteroaryl is optionally substituted with one or more groups R^{A}. It will be understood that the arrow with the asterisk shown in formula (II) indicates the point of attachment of the bicyclic heteroaryl to the remainder of the compound of formula (I), i.e. to the 5-azaindazole moiety of the compound of formula (I).

Moreover, for each of the general or preferred definitions of ring A described herein above, it is further preferred that the corresponding bicyclic heteroaryl comprises 1, 2, 3 or 4 ring heteroatoms (more preferably, 1, 2 or 3 ring heteroatoms; even more preferably, 1 or 2 ring heteroatoms) which are independently selected from nitrogen, oxygen and sulfur, wherein the remaining ring atoms are all carbon atoms; the aforementioned 1, 2, 3 or 4 ring heteroatoms may be present in only one or in both of the two fused rings.

Even more preferably, ring A is selected from any one of the following groups: 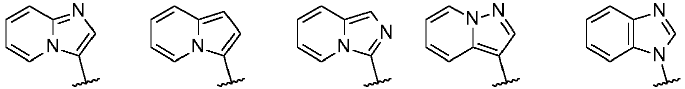 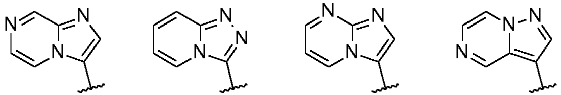 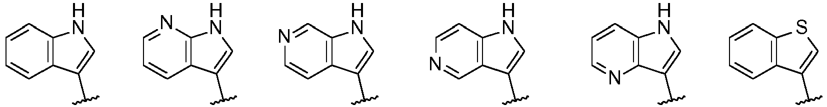 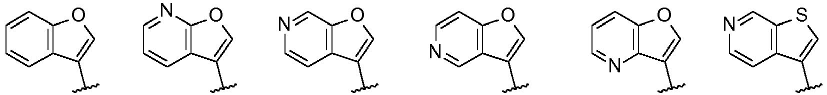 wherein each of the above-depicted groups is optionally substituted with one or more R^{A}.

Yet even more preferably, ring A is selected from any one of the following groups: 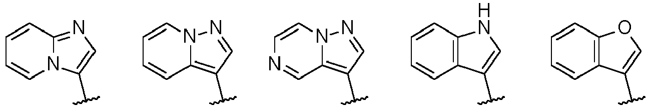 wherein each of the above-depicted groups is optionally substituted with one or more R^{A}.

Still more preferably, ring A is selected from any one of the following groups: 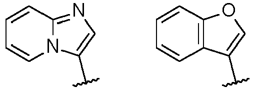 wherein each of the above-depicted groups is optionally substituted with one or more R^{A}.

Each R^{A} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-hetero-cyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more (e.g., one, two or three) groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl.

It will be understood that each group R^{A} (if present) may be attached to a carbon ring atom or a nitrogen ring atom (if present) of either one of the two rings of the bicyclic heteroaryl ring A, and is preferably attached to a carbon ring atom of the bicyclic heteroaryl ring A. If ring A is substituted with two or more groups R^{A}, the corresponding groups R^{A} may be attached to only one ring or to both rings of the bicyclic heteroaryl ring A (i.e., they may be attached to the first ring and/or the second ring of the bicyclic heteroaryl ring A). It will further be understood that only such ring atoms of ring A which carry a hydrogen atom can be substituted with R^{A}.

Preferably, each R^{A} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl. More preferably, each R^{A} is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN. Even more preferably, each R^{A} is independently selected from C₁₋₄ alkyl (e.g., methyl or ethyl), -OH, -O(C₁₋₄ alkyl) (e.g., -OCH₃ or -OCH₂CH₃), halogen (e.g., -F, -Cl, -Br, or -I; particularly -F or -CI), -CF₃, and -CN. It is particularly preferred that each R^{A} is independently halogen, and still more preferably each R^{A} is -F.

In accordance with the above-described meanings of the groups A and R^{A}, it is particularly preferred that ring A is one of the following groups: 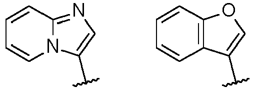 wherein the fused 6-membered ring comprised in each of the above-depicted groups is optionally substituted with one or two groups R^{A}, wherein each R^{A} is independently halogen (particularly -F or -CI), and wherein each R^{A} is preferably -F. Accordingly, ring A may be, for example:

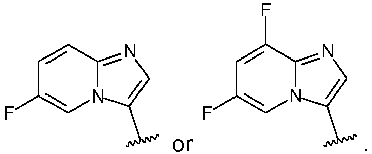

It is particularly preferred that the compound of formula (I) according to the present invention is one of the specific compounds of formula (I) described further below in the examples section of this specification, either in non-salt form (e.g., free base/acid form) or as a pharmaceutically acceptable salt, or solvate of the respective compound.

Accordingly, it is particularly preferred that the compound of formula (I) is selected from:

| **No.** | **IUPAC name** |
|---|---|
| 1 | [3-(1-Methyl-1H-indol-3-yl)-1-(2,2,2-trifluoro-ethyl)-1H-pyrazolo[4,3-c]pyridine-6-yl]-1,4-oxazepan-4-yl-methanone |
| 2 | Azepan-1-yl-[3-(6-fluoro-imidazo[1,2-a]pyridin-3-yl)-1-(2-methanesulfonyl-ethyl)-1H-pyrazolo[4,3-c]pyridin-6-yl]-methanone |
| 3 | Azepan-1-yl-[1-(2,2-difluoro-ethyl)-3-(6-fluoro-imidazo[1,2-a]pyridin-3-yl)-1H-pyrazolo[4,3-c]pyridin-6-yl]-methanone |
| 4 | Azepan-1-yl-[1-(2-fluoro-ethyl)-3-(6-fluoro-imidazo[1,2-a]pyridin-3-yl)-1H-pyrazolo[4,3-c]pyridin-6-yl]-methenone |
| 5 | Azepan-1-yl-[3-(6-fluoro-imidazo[1,2-a]pyridin-3-yl)-1-propyl-1H-pyrazolo[4,3-c]pyridin-6-yl]-methanone |
| 6 | Azepan-1-yl-[3-(6-fluoro-imidazo[1,2-a]pyridin-3-yl)-1-methoxymethyl-1H-pyrazolo[4,3-c]pyridin-6-yl]-methanone |
| 7 | [6-(Azepane-1-carbonyl)-3-(6-fluoro-imidazo[1,2-a]pyridin-3-yl)-pyrazolo[4,3-c]pyridin-1-yl]-acetonitrile |
| 8 | Azepan-1-yl-[3-(6,8-difluoro-imidazo[1,2-a]pyridin-3-yl)-1-(2-methoxy-ethyl)-1H-pyrazolo[4,3-c]pyridin-6-yl]-methanone |
| 9 | [1-Cyclopropylmethyl-3-(6,8-difluoro-imidazo[1,2-a]pyridin-3-yl)-1H-pyrazolo[4,3-c]pyridin-6-yl]-1,4-oxazepan-4-yl-methanone |
| 10 | [3-(6,8-Difluoro-imidazo[1,2-a]pyridin-3-yl)-1-methanesulfonyl-1H-pyrazolo[4,3-c]pyridin-6-yl]-1,4-oxazepan-4-yl-methanone |
| 11 | 3-[3-(6,8-Difluoro-imidazo[1,2-a]pyridin-3-yl)-6-(1,4-oxazepane-4-carbonyl)-pyrazolo[4,3-c]pyridin-1-ylmethyl]-azetidine-1-carboxylicacidtert-butyl ester |
| 12 | [3-(6,8-Difluoro-imidazo[1,2-a]pyridin-3-yl)-1-hydroxymethyl-1H-pyrazolo[4,3-c]pyridin-6-yl]-1,4-oxazepan-4-yl-methanone |
| 13 | [3-Benzofuran-3-yl-6-(1,4-oxazepane-4-carbonyl)-pyrazolo[4,3-c]pyridin-1-yl]-acetonitrile |
| 14 | (3-Aza-bicyclo[3.2.1]oct-3-yl)-[3-(6,8-difluoro-imidazo[1,2-a]pyridin-3-yl)-1-methylsulfanylmethyl-1H-pyrazolo[4,3-c]pyridin-6-yl]-methanone |
| 15 | [3-(6,8-Difluoro-imidazo[1,2-a]pyridin-3-yl)-1-methanesulfonylmethyl-1H-pyrazolo[4,3-c]pyridin-6-yl]-1,4-oxazepan-4-yl-methanone |
| 16 | (3-Benzofuran-3-yl-1-methanesulfonylmethyl-1H-pyrazolo[4,3-c]pyridine-6-yl)-1,4-oxazepan-4-yl-methanone |
| 17 | (1-Methanesulfonylmethyl-3-pyrazolo[1,5-a]pyridin-3-yl-1H-pyrazolo[4,3-c]pyridin-6-yl)-1,4-oxazepan-4-yl-methanone |
| 18 | (3-Aza-bicyclo[3.2.1]oct-3-yl)-(1-methanesulfonylmethyl-3-pyrazolo[1,5-a]pyridin-3-yl-1H-pyrazolo[4,3-c]pyridin-6-yl)-methanone |
| 19 | (3-Aza-bicyclo[3.2.1]oct-3-yl)-(3-benzofuran-3-yl-1-methanesulfonylmethyl-1H-pyrazolo[4,3-c]pyridin-6-yl)-methanone |
| 20 | Azepan-1-yl-[3-(6-fluoro-imidazo[1,2-a]pyridin-3-yl)-1-methanesulfonylmethyl-1H-pyrazolo[4,3-c]pyridin-6-yl]-methanone |
| 21 | Azepan-1-yl-[3-(6-fluoro-imidazo[1,2-a]pyridin-3-yl)-1-methanesulfinylmethyl-1H-pyrazolo[4,3-c]pyridin-6-yl]-methanone |
| 22 | [3-(6,8-Difluoro-imidazo[1,2-a]pyridin-3-yl)-1-ethanesulfonylmethyl-1H-pyrazolo[4,3-c]pyridin-6-yl]-1,4-oxazepan-4-yl-methanone |
| 23 | (3-Aza-bicyclo[3.2.1]oct-3-yl)-[3-(6,8-difluoro-imidazo[1,2-a]pyridin-3-yl)-1-methanesulfonylmethyl-1H-pyrazolo[4,3-c]pyridin-6-yl]-methanone |
| 24 | [3-(6,8-Difluoro-imidazo[1,2-a]pyridin-3-yl)-1-methanesulfonylmethyl-1H-pyrazolo[4,3-c]pyridin-6-yl]-(8-endo-hydroxy-8-exo-methyl-3-aza-bicyclo[3.2.1]oct-3-yl)-methanone |
| 25 | [3-(6,8-Difluoro-imidazo[1,2-a]pyridin-3-yl)-1-methanesulfonylmethyl-1H-pyrazolo[4,3-c]pyridin-6-yl]-(3-endo-hydroxy-8-aza-bicyclo[3.2.1]oct-8-yl)-methanone |
| 26 | [3-(6,8-Difluoro-imidazo[1,2-a]pyridin-3-yl)-1-methanesulfonylmethyl-1H-pyrazolo[4,3-c]pyridin-6-yl]-(2-oxa-5-aza-bicyclo[2.2.1]hept-5-yl)-methanone |
| 27 | [3-(6,8-Difluoro-imidazo[1,2-a]pyridin-3-yl)-1-methanesulfonylmethyl-1H-pyrazolo[4,3c]pyridin-6-yl]-(8-oxa-3-aza-bicyclo[3.2.1]oct-3-yl)-methanone |
| 28 | (3-Benzofuran-3-yl-1-methanesulfonylmethyl-1H-pyrazolo[4,3-c]pyridin-6-yl)-(8-endo-hydroxy-8-exo-methyl-d3-3-aza-bicyclo[3.2.1]oct-3-yl)-methanone |
| 29 | (3-Benzofuran-3-yl-1-methanesulfonylmethyl-1H-pyrazolo[4,3-c]pyridin-6-yl)-(6,6-difluoro-1,4-oxazepan-4-yl)-methanone |
| 30 | (3-Benzofuran-3-yl-1-methanesulfonylmethyl-1H-pyrazolo[4,3-c]pyridin-6-yl)-(8-endo-hydroxy-3-aza-bicyclo[3.2.1]oct-3-yl)-methanone |
| 31 | (3-Benzofuran-3-yl-1-methanesulfonylmethyl-1H-pyrazolo[4,3-c]pyridin-6-yl)-(5-endo-hydroxy-5-exo-methyl-2-aza-bicyclo[2.2.1]hept-2-yl)-methanone |
| 32 | (3-Benzofuran-3-yl-1-methanesulfonylmethyl-1H-pyrazolo[4,3-c]pyridin-6-yl)-(4-hydroxy-4-methyl-piperidin-1-yl)-methanone |
| 33 | (3-Benzofuran-3-yl-1-methanesulfonylmethyl-1H-pyrazolo[4,3-c]pyridin-6-yl)-(4,4-difluoro-piperidin-1-yl)-methanone |
| 34 | (3-Benzofuran-3-yl-1-methanesulfonylmethyl-1H-pyrazolo[4,3-c]pyridin-6-yl)-(2-methoxymethyl-pyrrolidin-1-yl)-methanone |
| 35 | (3-Benzofuran-3-yl-1-methanesulfonylmethyl-1H-pyrazolo[4,3-c]pyridin-6-yl)-(4-hydroxy-2,2dimethyl-piperidin-1-yl)-methanone |
| 36 | (3-Benzofuran-3-yl-1-methanesulfonylmethyl-1H-pyrazolo[4,3-c]pyridin-6-yl)-(4-hydroxy-piperidin-1-yl)-methanone |
| 37 | [3-(benzofuran-3-yl)-1-(methylsulfonylmethyl)pyrazolo[4,3-c]pyridin-6-yl]-(3-endo-hydroxy-8-azabicyclo[3.2.1]octan-8-yl)methanone |
| 38 | (3-Benzofuran-3-yl-1-methanesulfonylmethyl-1H-pyrazolo[4,3-c]pyridin-6-yl)-(3-hydroxy-piperidin-1-yl)-methanone |
| 39 | (3-Benzofuran-3-yl-1-methanesulfonylmethyl-1H-pyrazolo[4,3-c]pyridin-6-yl)-(3,3-difluoro-piperidin-1-yl)-methanone |
| 40 | (3-Benzofuran-3-yl-1-methanesulfonylmethyl-1H-pyrazolo[4,3-c]pyridin-6-yl)-(4-hydroxyazepan-1-yl)-methanone |
| 41 | Azepan-1-yl-[3-(6-fluoro-imidazo[1,2-a]pyridin-3-yl)-1-methyl-1H-pyrazolo[4,3-c]pyridin-6-yl]-methanone |
| 42 | 3-[6-(Azepane-1-carbonyl)-1-methyl-1H-pyrazolo[4,3-c]pyridin-3-yl]-imidazo[1,2-a]pyridine-6-carbonitrile |
| 43 | 3-[6-(Azepane-1-carbonyl)-1-propyl-1H-pyrazolo[4,3-c]pyridin-3-yl]-imidazo[1,2-a]pyridine-6-carbonitrile |
| 44 | Azepan-1-yl-[3-(6-fluoro-imidazo[1,2-a]pyridin-3-yl)-1-(2,2,2-trifluoro-ethyl)-1H-pyrazolo[4,3-c]pyridin-6-yl]-methanone |
| 45 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carbonitrile |
| 46 | 3-[6-(Azepane-1-carbonyl)-1-(2,2,2-trifluoro-ethyl)-1H-pyrazolo[4,3-c]-pyridin-3-yl]-imidazo[1,2-a]pyridine-6-carboxylic acid amide |
| 47 | 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carbonitrile |
| 48 | Azepan-1-yl-[3-(6-chloro-imidazo[1,2-a]pyridin-3-yl)-1-(2,2,2-trifluoro-ethyl)-1H-pyrazolo[4,3-c]pyridin-6-yl]-methanone |
| 49 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carboxylic acid methylamide |
| 50 | Azepan-1-yl-[3-(6-fluoro-imidazo[1,2-a]pyrimidin-3-yl)-1-(2,2,2-trifluoro-ethyl)-1H-pyrazolo[4,3-c]pyridin-6-yl]-methanone |
| 51 | Azepan-1-yl-[3-(6-methoxymethyl-imidazo[1,2-a]pyridin-3-yl)-1-(2,2,2-trifluoro-ethyl)-1H-pyrazolo[4,3-c]pyridin-6-yl]-methanone |
| 52 | Azepan-1-yl-[3-indolizin-3-yl-1-(2,2,2-trifluoro-ethyl)-1H-pyrazolo[4,3-c]pyridin-6-yl]-methanone |
| 53 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carboxylic acid dimethylamide |
| 54 | Azepan-1-yl-[3-imidazo[1,5-a]pyridin-3-yl-1-(2,2,2-trifluoro-ethyl)-1H-pyrazolo[4,3-c]pyridin-6-yl]-methanone |
| 55 | Azepan-1-yl-[3-imidazo[1,2-a]pyridin-3-yl-1-(2,2,2-trifluoro-ethyl)-1H-pyrazolo[4,3-c]pyridin-6-yl]-methanone |
| 56 | Azepan-1-yl-[3-imidazo[1,2-a]pyrazin-3-yl-1-(2,2,2-trifluoro-ethyl)-1H-pyrazolo[4,3-c]pyridin-6-yl]-methanone |
| 57 | 3-[6-(Azepane-1-carbonyl)-1-(2,2,2-trifluoro-ethyl)-1H-pyrazolo[4,3-c]pyridin-3-yl]-7-methyl-imidazo[1,2-a]pyridine-6-carboxylicacidamide |
| 58 | Azepan-1-yl-[1-(2,2,2-trifluoro-ethyl)-3-(6-trifluoromethyl-imidazo[1,2-a]pyridin-3-yl)-1H-pyrazolo[4,3-c]pyridin-6-yl]-methanone |
| 59 | Azepan-1-yl-[3-(6-methoxy-imidazo[1,2-a]pyridin-3-yl)-1-(2,2,2-trifluoro-ethyl)-1H-pyrazolo[4,3-c]pyridin-6-yl]-methanone |
| 60 | Azepan-1-yl-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 61 | Azepan-1-yl-[3-(6-methyl-imidazo[1,2-a]pyridin-3-yl)-1-(2,2,2-trifluoro-ethyl)-1H-pyrazolo[4,3-c]pyridin-6-yl]-methanone |
| 62 | Azepan-1-yl-[3-(8-fluoro-imidazo[1,2-a]pyridin-3-yl)-1-(2,2,2-trifluoro-ethyl)-1H-pyrazolo[4,3-c]pyridin-6-yl]-methanone |
| 63 | 3-[6-(Azepane-1-carbonyl)-1-(2,2,2-trifluoro-ethyl)-1H-pyrazolo[4,3-c]pyridin-3-yl]-7-hydroxy-imidazo[1,2-a]pyridine-6-carbonitrile |
| 64 | Azepan-1-yl-[3-[6-(1-hydroxy-ethyl)-imidazo[1,2-a]pyridin-3-yl]-1-(2,2,2-trifluoro-ethyl)-1H-pyrazolo[4,3-c]pyridin-6-yl]-methanone |
| 65 | Azepan-1-yl-[3-(6-cyclopropyl-imidazo[1,2-a]pyridin-3-yl)-1-(2,2,2-trifluoro-ethyl)-1H-pyrazolo[4,3-c]pyridin-6-yl]-methanone |
| 66 | Azepan-1-yl-[1-(2,2,2-trifluoro-ethyl)-3-(6-trifluoromethoxy-imidazo[1,2-a]pyridin-3-yl)-1H-pyrazolo[4,3-c]pyridin-6-yl]-methanone |
| 67 | Azepan-1-yl-[3-(7-fluoro-imidazo[1,2-a]pyridin-3-yl)-1-(2,2,2-trifluoro-ethyl)-1H-pyrazolo[4,3-c]pyridin-6-yl]-methanone |
| 68 | Azepan-1-yl-[3-(6-hydroxy-imidazo[1,2-a]pyridin-3-yl)-1-(2,2,2-trifluoro-ethyl)-1H-pyrazolo[4,3-c]pyridin-6-yl]-methanone |
| 69 | Azepan-1-yl-[3-(6-difluoromethyl-imidazo[1,2-a]pyridin-3-yl)-1-(2,2,2-trifluoro-ethyl)-1H-pyrazolo[4,3-c]pyridin-6-yl]-methanone |
| 70 | Azepan-1-yl-[3-[6-(2,2,2-trifluoro-ethoxy)-imidazo[1,2-a]pyridin-3-yl]-1-(2,2,2-trifluoro-ethyl)-1H-pyrazolo[4,3-c]pyridin-6-yl]-methanone |
| 71 | Azepan-1-yl-[3-[1,2,4]triazolo[4,3-a]pyridin-3-yl-1-(2,2,2-trifluoro-ethyl)-1H-pyrazolo[4,3-c]pyridin-6-yl]-methanone |
| 72 | Azepan-1-yl-[3-[6-(1-hydroxy-1-methyl-ethyl)-imidazo[1,2-a]pyridin-3-yl]-1-(2,2,2-trifluoro-ethyl)-1H-pyrazolo[4,3-c]pyridin-6-yl]-methanone |
| 73 | Azepan-1-yl-[3-(7-chloro-6-fluoro-imidazo[1,2-a]pyridin-3-yl)-1-(2,2,2-trifluoro-ethyl)-1H-pyrazolo[4,3-c]pyridin-6-yl]-methanone |
| 74 | Azepan-1-yl-[3-(6-chloro-7-fluoro-imidazo[1,2-a]pyridin-3-yl)-1-(2,2,2-trifluoro-ethyl)-1H-pyrazolo[4,3-c]pyridin-6-yl]-methanone |
| 75 | Azepan-1-yl-[3-(6-chloro-7-hydroxy-imidazo[1,2-a]pyridin-3-yl)-1-(2,2,2-trifluoro-ethyl)-1H-pyrazolo[4,3-c]pyridin-6-yl]-methanone |
| 76 | Azepan-1-yl-[3-(6,8-difluoro-imidazo[1,2-a]pyridin-3-yl)-1-(2-trimethylsilanyl-ethoxymethyl)-1H-pyrazolo[4,3-c]pyridin-6-yl]-methanone |
| 77 | Azepan-1-yl-[3-(6-fluoro-imidazo[1,2-a]pyridin-3-yl)-1-(2-trimethylsilanyl-ethoxymethyl)-1H-pyrazolo[4,3-c]pyridin-6-yl]-methanone |
| 78 | Azepan-1-yl-[3-(6,8-difluoro-imidazo[1,2-a]pyridin-3-yl)-1-(4-methoxy-benzyl)-1H-pyrazolo[4,3-c]pyridin-6-yl]-methanone |
| 79 | Azepan-1-yl-[3-(6,8-difluoro-imidazo[1,2-a]pyridin-3-yl)-1-(tetrahydro-pyran-2-yl)-1H-pyrazolo[4,3-c]pyridin-6-yl]-methanone |
| 80 | [3-(6-Fluoro-imidazo[1,2-a]pyridin-3-yl)-1-(2,2,2-trifluoro-ethyl)-1H-pyrazolo[4,3-c]pyridin-6-yl]-1,4-oxazepan-4-yl-methanone |
| 81 | 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carbonitrile |
| 82 | [3-(6-Fluoro-imidazo[1,2-a]pyridin-3-yl)-1-(2,2,2-trifluoro-ethyl)-1H-pyrazolo[4,3-c]pyridin-6-yl]-(8-oxa-3-aza-bicyclo[3.2.1]oct-3-yl)-methanone |
| 83 | [3-(6,8-Difluoro-imidazo[1,2-a]pyridin-3-yl)-1-(2,2,2-trifluoro-ethyl)-1H-pyrazolo[4,3c]pyridin-6-yl]-(8-oxa-3-aza-bicyclo[3.2.1]oct-3-yl)-methanone |
| 84 | 3-[6-(8-Oxa-3-aza-bicyclo[3.2.1]octane-3-carbonyl)-1-(2,2,2-trifluoro-ethyl)-1H-pyrazolo[4,3-c]pyridin-3-yl]-imidazo[1,2-a]pyridine-6-carbonitrile |
| 85 | [3-(8-fluoroimidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone |
| 86 | [3-(6-fluoroimidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone |
| 87 | [3-(imidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone |
| 88 | (3-endo-hydroxy-8-azabicyclo[3.2.1]octan-8-yl)-[3-(6-hydroxyimidazo[1,2-a]pyridin-3-yl)-1-(2,2,2-trifluoroethyl)pyrazolo[4,3-c]pyridin-6-yl]methanone |
| 89 | Azepan-1-yl-[3-pyrazolo[1,5-a]pyridin-3-yl-1-(2,2,2-trifluoro-ethyl)-1H-pyrazolo[4,3-c]pyridin-6-yl]-methanonehydrochloride |
| 90 | Azepan-1-yl-[3-benzofuran-3-yl-1-(2,2,2-trifluoro-ethyl)-1H-pyrazolo[4,3-c]pyridin-6-yl]-methanone |
| 91 | Azepan-1-yl-[3-(1H-indol-3-yl)-1-(2,2,2-trifluoro-ethyl)-1H-pyrazolo[4,3-c]pyridin-6-yl]-methanone |
| 92 | Azepan-1-yl-[3-(1H-pyrrolo[2,3-b]pyridin-3-yl)-1-(2,2,2-trifluoro-ethyl)-1H-pyrazolo[4,3-c]pyridin-6-yl]-methanone |
| 93 | 1,4-Oxazepan-4-yl-[3-thieno[2,3-c]pyridin-3-yl-1-(2,2,2-trifluoro-ethyl)-1H-pyrazolo[4,3-c]pyridin-6-yl]-methanone |
| 94 | [3-Benzofuran-3-yl-1-(2,2,2-trifluoro-ethyl)-1H-pyrazolo[4,3-c]pyridine-6-yl]-1,4-oxazepan-4-yl-methanone |
| 95 | 1,4-Oxazepan-4-yl-[3-pyrazolo[1,5-a]pyridine-3-yl-1-(2,2,2-trifluoro-ethyl)-1H-pyrazolo[4,3-c]pyridine-6-yl]-methanone |
| 96 | [3-(1H-Indol-3-yl)-1-(2,2,2-trifluoro-ethyl)-1H-pyrazolo[4,3-c]pyridine-6-yl]-1,4-oxazepan-4-yl-methanone |
| 97 | 1,4-Oxazepan-4-yl-[3-(1H-pyrrolo[2,3-b]pyridin-3-yl)-1-(2,2,2-trifluoro-ethyl)-1H-pyrazolo[4,3-c]pyridine-6-yl]-methanone |
| 98 | [3-Benzo[b]thiophen-3-yl-1-(2,2,2-trifluoro-ethyl)-1H-pyrazolo[4,3-c]pyridine-6-yl]-1,4-oxazepan-4-yl-methanone |
| 99 | (8-Oxa-3-aza-bicyclo[3.2.1]oct-3-yl)-[3-pyrazolo[1,5-a]pyridin-3-yl-1-(2,2,2-trifluoro-ethyl)-1H-pyrazolo[4,3-c]pyrid in-6-yl]-methanone |
| 100 | [3-Benzofuran-3-yl-1-(2,2,2-trifluoro-ethyl)-1H-pyrazolo[4,3-c]pyridin-6-yl]-(8-oxa-3-aza-bicyclo[3.2.1]oct-3-yl)-methanone |
| 101 | (3-Aza-bicyclo[3.2.1]oct-3-yl)-[3-benzofuran-3-yl-1-(2,2,2-trifluoro-ethyl)-1H-pyrazolo[4,3-c]pyridin-6-yl]-methanone |
| 102 | (3-Aza-bicyclo[3.2.1]oct-3-yl)-[3-pyrazolo[1,5-a]pyridin-3-yl-1-(2,2,2-trifluoroethyl)-1H-pyrazolo[4,3-c]pyridin-6-yl]-methanone |
| 103 | [3-Benzofuran-3-yl-1-(2,2-difluoro-ethyl)-1H-pyrazolo[4,3-c]pyridin-6-yl]-1,4-oxazepan-4-yl-methanone |
| 104 | [3-Benzofuran-3-yl-1-(2-fluoro-ethyl)-1H-pyrazolo[4,3-c]pyridin-6-yl]-1,4-oxazepan-4-yl-methanone |
| 105 | 2-[3-Benzofuran-3-yl-6-(1,4-oxazepane-4-carbonyl)-pyrazolo[4,3-c]pyridin-1-yl]-acetamide |
| 106 | (3-Benzofuran-3-yl-1-propyl-1H-pyrazolo[4,3-c]pyridin-6-yl)-1 ,4-oxazepan-4-yl-methanone |
| 107 | (3-Benzofuran-3-yl-1-methanesulfonylmethyl-1H-pyrazolo[4,3-c]pyridine-6-yl)-(8-oxa-3-aza-bicyclo[3.2.1]oct-3-yl)-methanone |
| 108 | (1-Methanesulfonylmethyl-3-pyrazolo[1,5-a]pyridine-3-yl-1H-pyrazolo[4,3-c]pyridine-6-yl)- (8-oxa-3-aza-bicyclo[3.2.1]oct-3-yl)-methanone |
| 109 | [3-(5-fluorobenzofuran-3-yl)-1-(2,2,2-trifluoroethyl)pyrazolo[4,3-c]pyridin-6-yl]-(1,4-oxazepan-4-yl)methanone |
| 110 | [3-(5-fluorobenzofuran-3-yl)-1-(methylsulfonylmethyl)pyrazolo[4,3-c]pyridin-6-yl]-(1,4-oxazepan-4-yl)methanone |
| 111 | [3-(5-fluorobenzofuran-3-yl)-1-(methylsulfonylmethyl)pyrazolo[4,3-c]pyridin-6-yl]-(8-oxa-3-azabicyclo[3.2.1]octan-3-yl)methanone |
| 112 | (3-endo-hydroxy-8-azabicyclo[3.2.1]octan-8-yl)-[3-(1H-indol-3-yl)-1-(2,2,2-trifluoroethyl)pyrazolo[4,3-c]pyridin-6-yl]methanone |
| 113 | (3-endo-hydroxy-8-azabicyclo[3.2.1]octan-8-yl)-[3-pyrazolo[1,5-a]pyridin-3-yl-1-(2,2,2-trifluoroethyl)pyrazolo[4,3-c]pyridin-6-yl]methanone |
| 114 | (4-hydroxy-1-piperidyl)-[3-pyrazolo[1,5-a]pyridin-3-yl-1-(2,2,2-trifluoroethyl)pyrazolo[4,3-c]pyridin-6-yl]methanone |
| 115 | [3-(5-fluorobenzofuran-3-yl)-1-(2,2,2-trifluoroethyl)pyrazolo[4,3-c]pyridin-6-yl]-(4-hydroxy-1-piperidyl)methanone |
| 116 | [3-(5-fluorobenzofuran-3-yl)-1-(2,2,2-trifluoroethyl)pyrazolo[4,3-c]pyridin-6-yl]-(4-hydroxy-4-methyl-1-piperidyl)methanone |
| 117 | (4-hydroxy-4-methyl-1-piperidyl)-[3-pyrazolo[1,5-a]pyridin-3-yl-1-(2,2,2-trifluoroethyl)pyrazolo[4,3-c]pyridin-6-yl]methanone |
| 118 | [3-(5-fluorobenzofuran-3-yl)-1-(2,2,2-trifluoroethyl)pyrazolo[4,3-c]pyridin-6-yl]-(6-hydroxy-1,4-oxazepan-4-yl)methanone |
| 119 | (6-hydroxy-1,4-oxazepan-4-yl)-[3-pyrazolo[1,5-a]pyridin-3-yl-1-(2,2,2-trifluoroethyl)pyrazolo[4,3-c]pyridin-6-yl]methanone |
| 120 | [3-(5-fluorobenzofuran-3-yl)-1-(2,2,2-trifluoroethyl)pyrazolo[4,3-c]pyridin-6-yl]-(6,6-dideuterio-1,4-oxazepan-4-yl)methanone |
| 121 | (6,6-dideuterio-1,4-oxazepan-4-yl)-[3-pyrazolo[1,5-a]pyridin-3-yl-1-(2,2,2-trifluoroethyl)pyrazolo[4,3-c]pyridin-6-yl]methanone |
| 122 | [3-(5-fluorobenzofuran-3-yl)-1-(2,2,2-trifluoroethyl)pyrazolo[4,3-c]pyridin-6-yl]-(4-hydroxyazepan-1-yl)methanone |
| 123 | [3-(5-fluoro-1H-indol-3-yl)-1-(2,2,2-trifluoroethyl)pyrazolo[4,3-c]pyridin-6-yl]-(1,4-oxazepan-4-yl)methanone |
| 124 | methyl 3-[6-(1,4-oxazepane-4-carbonyl)-1-(2,2,2-trifluoroethyl)pyrazolo[4,3-c]pyridin-3-yl]benzofuran-5-carboxylate |
| 125 | [3-(5-methylbenzofuran-3-yl)-1-(2,2,2-trifluoroethyl)pyrazolo[4,3-c]pyridin-6-yl]-(1,4-oxazepan-4-yl)methanone |
| 126 | [3-(2-methylbenzofuran-3-yl)-1-(2,2,2-trifluoroethyl)pyrazolo[4,3-c]pyridin-6-yl]-(1,4-oxazepan-4-yl)methanone |
| 127 | [3-(5-fluorobenzofuran-3-yl)-1-(2,2,2-trifluoroethyl)pyrazolo[4,3-c]pyridin-6-yl]-(8-oxa-3-azabicyclo[3.2.1]octan-3-yl)methanone |
| 128 | 3-(6,8-Difluoro-imidazo[1,2-a]pyridin-3-yl)-1-(2,2,2-trifluoro-ethyl)-1H-pyrazolo[4,3-c]pyridine-6-carboxylic acid amide |
| 129 | 3-[6-(1,4-oxazepane-4-carbonyl)-1-(2,2,2-trifluoroethyl)pyrazolo[4,3-c]pyridin-3-yl]benzofuran-5-carboxylic acid |
| 130 | 1-[3-(benzofuran-3-yl)-1-(2,2,2-trifluoroethyl)pyrazolo[4,3-c]pyridi ne-6-carbonyl]piperidine-4-carboxylic acid |
| 131 | [3-(6-Fluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone |
| 132 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone |
| 133 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-methyl-piperidin-1-yl)-methanone |
| 134 | [3-(6,8-Difluoro-imidazo[1,2-a]pyridin-3-yl)-1-(2,2,2-trifluoro-ethyl)-1H-pyrazolo[4,3-c]pyrid in-6-yl]-(3-hydroxy-azepan-1-yl)-methanone |
| 135 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[2.2.1]hept-5-yl)-methanone |
| 136 | [3-(6,8-Difluoro-[1,2,4]triazolo[4,3-a]pyridin-3-yl)-1-(2,2,2-trifluoro-ethyl)-1H-pyrazolo[4,3-c]pyridin-6-yl]-(3-hydroxy-8-aza-bicyclo[3.2.1]oct-8-yl)-methanone |
| 137 | [3-(6,8-Difluoro-[1,2,4]triazolo[4,3-a]pyridin-3-yl)-1-(2,2,2-trifluoro-ethyl)-1H-pyrazolo[4,3-c]pyridin-6-yl]-1,4-oxazepan-4-yl-methanone |
| 138 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-methyl-piperidin-1-yl)-methanone |
| 139 | (3-Aza-bicyclo[3.2.1]oct-3-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 140 | [3-(6,8-Difluoro-imidazo[1,2-a]pyridin-3-yl)-1-(2,2,2-trifluoro-ethyl)-1H-pyrazolo[4,3-c]pyridin-6-yl]-(9-hydroxy-3-aza-bicyclo[3.3.1]non-3-yl)-methanone |
| 141 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-isopropyl-piperidin-1-yl)-methanone |
| 142 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2,2-dimethyl-piperidin-1-yl)-methanone |
| 143 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-3-isobutyl-piperidin-1-yl)-methanone |
| 144 | [3-(6,8-Difluoro-imidazo[1,2-a]pyridin-3-yl)-1-(2,2,2-trifluoro-ethyl)-1H-pyrazolo[4,3-c]pyridin-6-yl]-(4-fluoro-piperidin-1-yl)-methanone |
| 145 | [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-2-aza-spiro[4.5]dec-2-yl)-methanone |
| 146 | [3-(6,8-Difluoro-imidazo[1,2-a]pyridin-3-yl)-1-(2,2,2-trifluoro-ethyl)-1H-pyrazolo[4,3-c]pyridin-6-yl]-(4,4-difluoro-piperidin-1-yl)-methanone |
| 147 | [3-(6,8-Difluoro-imidazo[1,2-a]pyridin-3-yl)-1-(2,2,2-trifluoro-ethyl)-1H-pyrazolo[4,3-c]pyridin-6-yl]-(4'-hydroxy-3',4',5',6'-tetrahydro-2'H-[3,4']bipyridinyl-1'-yl)-methanone |
| 148 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-trifluoromethyl-piperidin-1-yl)-methanone |
| 149 | [3-(6,8-Difluoro-imidazo[1,2-a]pyridin-3-yl)-1-(2,2,2-trifluoro-ethyl)-1H-pyrazolo[4,3-c]pyridin-6-yl]-(2-methoxymethyl-pyrrolidin-1-yl)-methanone |
| 150 | [3-(6,8-Difluoro-imidazo[1,2-a]pyridin-3-yl)-1-(2,2,2-trifluoro-ethyl)-1H-pyrazolo[4,3-c]pyridin-6-yl]-(4-methyl-1,4-diazepan-1-yl)-methanone |
| 151 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxy-piperidin-1-yl)-methanone |
| 152 | [3-(6,8-Difluoro-imidazo[1,2-a]pyridin-3-yl)-1-(2,2,2-trifluoro-ethyl)-1H-pyrazolo[4,3-c]pyridin-6-yl]-(7-oxa-2-aza-spiro[4.5]dec-2-yl)-methanone |
| 153 | [3-(6,8-Difluoro-imidazo[1,2-a]pyridin-3-yl)-1-(2,2,2-trifluoro-ethyl)-1H-pyrazolo[4,3-c]pyridin-6-yl]-(3-oxa-9-aza-spiro[5.5]undec-9-yl)-methanone |
| 154 | [3-(6,8-Difluoro-imidazo[1,2-a]pyridin-3-yl)-1-(2,2,2-trifluoro-ethyl)-1H-pyrazolo[4,3-c]pyridin-6-yl]-(tetrahydro-furo[3,4-c]pyrrol-5-yl)-methanone |
| 155 | 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-3-one |
| 156 | 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-1-one |
| 157 | [3-(6,8-Difluoro-imidazo[1,2-a]pyridin-3-yl)-1-(2,2,2-trifluoro-ethyl)-1H-pyrazolo[4,3-c]pyridin-6-yl]-[3-(2-methoxy-ethyl)-pyrrolidin-1-yl]-methanone |
| 158 | [3-(6,8-Difluoro-imidazo[1,2-a]pyridin-3-yl)-1-(2,2,2-trifluoro-ethyl)-1H-pyrazolo[4,3-c]pyridin-6-yl]-morpholin-4-yl-methanone |
| 159 | [3-(6,8-Difluoro-imidazo[1,2-a]pyridin-3-yl)-1-(2,2,2-trifluoro-ethyl)-1H-pyrazolo[4,3-c]pyridin-6-yl]-(3,3-difluoro-piperidin-1-yl)-methanone |
| 160 | [3-(6,8-Difluoro-imidazo[1,2-a]pyridin-3-yl)-1-(2,2,2-trifluoro-ethyl)-1H-pyrazolo[4,3-c]pyridin-6-yl]-(3,3-difluoro-piperidin-1-yl)-methanone |
| 161 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxymethyl-piperidin-1-yl)-methanone |
| 162 | [3-(6,8-Difluoro-imidazo[1,2-a]pyridin-3-yl)-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone |
| 163 | [3-(6,8-Difluoro-imidazo[1,2-a]pyridin-3-yl)-1-(2,2,2-trifluoro-ethyl)-1H-pyrazolo[4,3-c]pyridin-6-yl]-(2-trifluoromethyl-morpholin-4-yl)-methanone |
| 164 | [3-(6,8-Difluoro-imidazo[1,2-a]pyridin-3-yl)-1-(2,2,2-trifluoro-ethyl)-1H-pyrazolo[4,3-c]pyridin-6-yl]-(4-trifluoromethyl-piperidin-1-yl)-methanone |
| 165 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-piperidin-1-yl)-methanone |
| 166 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-7-aza-spiro[3.5]non-7-yl)-methanone |
| 167 | [3-(6,8-Difluoro-imidazo[1,2-a]pyridin-3-yl)-1-(2,2,2-trifluoro-ethyl)-1H-pyrazolo[4,3-c]pyridin-6-yl]-(3-methoxymethyl-morpholin-4-yl)-methanone |
| 168 | [3-(6,8-Difluoro-imidazo[1,2-a]pyridin-3-yl)-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone |
| 169 | (4-Aza-tricyclo[4.3.1.1*3,8*]undec-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 170 | [3-(6,8-Difluoro-imidazo[1,2-a]pyridin-3-yl)-1-(2,2,2-trifluoro-ethyl)-1H-pyrazolo[4,3-c]pyridin-6-yl]-(8-exo-hydroxy-3-aza-bicyclo[3.2.1]oct-3-yl)-methanone |
| 171 | [3-(6,8-Difluoro-imidazo[1,2-a]pyridin-3-yl)-1-(2,2,2-trifluoro-ethyl)-1H-pyrazolo[4,3-c]pyridin-6-yl]-(3-exo-hydroxy-8-aza-bicyclo[3.2.1]oct-8-yl)-methanone |
| 172 | (8-Aza-bicyclo[3.2.1]oct-8-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 173 | [3-(6,8-Difluoro-imidazo[1,2-a]pyridin-3-yl)-1-(2,2,2-trifluoro-ethyl)-1H-pyrazolo[4,3-c]pyridin-6-yl]-(4-difluoromethyl-piperidin-1-yl)-methanone |
| 174 | 3-(6,8-Difluoro-imidazo[1,2-a]pyridin-3-yl)-1-(2,2,2-trifluoro-ethyl)-1H-pyrazolo[4,3-c]pyridine-6-carboxylic acid (2-hydroxy-2-methyl-propyl)-amide |
| 175 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-6-aza-tricyclo[3.3.1.1*3,7*]dec-6-yl)-methanone |
| 176 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-4-methyl-piperidin-1-yl)-methanone |
| 177 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-azepan-1-yl)-methanone |
| 178 | (2-Aza-tricyclo[3.3.1.1*3,7*]dec-2-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 179 | [3-(6,8-Difluoro-imidazo[1,2-a]pyridin-3-yl)-1-(2,2,2-trifluoro-ethyl)-1H-pyrazolo[4,3-c]pyridin-6-yl]-(5-oxa-8-aza-spiro[2.6]non-8-yl)-methanone |
| 180 | [3-(6,8-Difluoro-imidazo[1,2-a]pyridin-3-yl)-1-(2,2,2-trifluoro-ethyl)-1H-pyrazolo[4,3-c]pyridin-6-yl]-1,4-thiazepan-1,1-dioxide-4-yl-methanone |
| 181 | Cis-[3-(6,8-Difluoro-imidazo[1,2-a]pyridin-3-yl)-1-(2,2,2-trifluoro-ethyl)-1H-pyrazolo[4,3-c]pyridin-6-yl]-(2,6-dimethyl-morpholin-4-yl)-methanone |
| 182 | [3-(6,8-Difluoro-imidazo[1,2-a]pyridin-3-yl)-1-(2,2,2-trifluoro-ethyl)-1H-pyrazolo[4,3-c]pyridin-6-yl]-(2 ,2-dimethyl-1,4-oxazepan-4-yl)-methanone |
| 183 | Cis-[3-(6,8-Difluoro-imidazo[1,2-a]pyridin-3-yl)-1-(2,2,2-trifluoro-ethyl)-1H-pyrazolo[4,3-c]pyridin-6-yl]-(3,5-dimethyl-morpholin-4-yl)-methanone |
| 184 | [3-(6,8-Difluoro-imidazo[1,2-a]pyridin-3-yl)-1-(2,2,2-trifluoro-ethyl)-1H-pyrazolo[4,3-c]pyridin-6-yl]-(3-oxa-8-aza-bicyclo[3.2.1]oct-8-yl)-methanone |
| 185 | [3-(6,8-Difluoro-imidazo[1,2-a]pyridin-3-yl)-1-(2,2,2-trifluoro-ethyl)-1H-pyrazolo[4,3-c]pyridin-6-yl]-(6,6-difluoro-1,4-oxazepan-4-yl)-methanone |
| 186 | [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-4-aza-spiro[2.6]non-4-yl)-methanone |
| 187 | [3-(6,8-Difluoro-imidazo[1,2-a]pyridin-3-yl)-1-(2,2,2-trifluoro-ethyl)-1H-pyrazolo[4,3-c]pyridin-6-yl]-(8-endo-hydroxy-3-aza-bicyclo[3.2.1]oct-3-yl)-methanone |
| 188 | [3-(6,8-Difluoro-imidazo[1,2-a]pyridin-3-yl)-1-(2,2,2-trifluoro-ethyl)-1H-pyrazolo[4,3-c]pyridin-6-yl]-(7-endo-hydroxy-3-oxa-9-aza-bicyclo[3.3.1]non-9-yl)-methanone |
| 189 | 1-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-diazepan-5-one |
| 190 | [3-(6,8-Difluoro-imidazo[1,2-a]pyridin-3-yl)-1-(2,2,2-trifluoro-ethyl)-1H-pyrazolo[4,3-c]pyridin-6-yl]-1,4-oxazepan-deuterium-4-yl-methanone |
| 191 | [3-(6,8-Difluoro-imidazo[1,2-a]pyridin-3-yl)-1-(2,2,2-trifluoro-ethyl)-1H-pyrazolo[4,3-c]pyridin-6-yl]-[1,2]oxazepan-2-yl-methanone |
| 192 | [3-(6,8-Difluoro-imidazo[1,2-a]pyridin-3-yl)-1-(2,2,2-trifluoro-ethyl)-1H-pyrazolo[4,3-c]pyridin-6-yl]-(5-oxa-8-aza-spiro[3.5]non-8-yl)-methanone |
| 193 | [3-(6,8-Difluoro-imidazo[1,2-a]pyridin-3-yl)-1-(2,2,2-trifluoro-ethyl)-1H-pyrazolo[4,3-c]pyridin-6-yl]-(6-hydroxy-1,4-oxazepan-4-yl)-methanone |
| 194 | 5-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,5-diaza-bicyclo[2.2.1]heptane-2-carboxylic acid tert-butyl ester |
| 195 | 1,4-Diazepan-1-yl-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 196 | [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-5-aza-spiro[3.5]non-5-yl)-methanone |
| 197 | (2-{[3-(6,8-Difluoro-imidazo[1,2-a]pyrid in-3-yl)-1-(2,2,2-trifluoro-ethyl)-1H-pyrazolo[4,3-c]pyridine-6-carbonyl]-methyl-amino}-ethyl)-carbamic acid tert-butyl ester |
| 198 | 3-(6,8-Difluoro-imidazo[1,2-a]pyridin-3-yl)-1-(2,2,2-trifluoro-ethyl)-1H-pyrazolo[4,3-c]pyridine-6-carboxylic acid (4-hydroxy-tetrahydro-pyran-3-yl)-amide |
| 199 | [3-(6,8-Difluoro-imidazo[1,2-a]pyridin-3-yl)-1-(2,2,2-trifluoro-ethyl)-1H-pyrazolo[4,3-c]pyridin-6-yl]-(6-oxa-3-aza-bicyclo[3.2.1]oct-3-yl)-methanone |
| 200 | 3-[3-(6,8-Difluoro-imidazo[1,2-a]pyridin-3-yl)-1-(2,2,2-trifluoro-ethyl)-1H-pyrazolo[4,3-c]pyridine-6-carbonyl]-3,8-diaza-bicyclo[3.2.1]octane-8-carboxylic acid tert-butyl ester |
| 201 | 4-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-diazepan-2-one |
| 202 | {1-[3-(6,8-Difluoro-imidazo[1,2-a]pyridin-3-yl)-1-(2,2,2-trifluoro-ethyl)-1H-pyrazolo[4,3-c]pyridine-6-carbonyl]-azepan-3-yl}-carbamic acid tert-butyl ester |
| 203 | (3,3-Difluoro-4-hydroxy-piperidin-1-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 204 | {4-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-oxazepan-6-yl}-carbamic acid tert-butyl ester |
| 205 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[4.1.0]hept-5-yl)-methanone |
| 206 | [3-(6,8-Difluoro-imidazo[1,2-a]pyridin-3-yl)-1-(2,2,2-trifluoro-ethyl)-1H-pyrazolo[4,3-c]pyridin-6-yl]-(7-oxa-4-aza-spiro[2.6]non-4-yl)-methanone |
| 207 | [3-Benzofuran-3-yl-1-(2,2,2-trifluoro-ethyl)-1H-pyrazolo[4,3-c]pyridin-6-yl]-(4-hydroxy-piperidin-1-yl)-methanone |
| 208 | [3-Benzofuran-3-yl-1-(2,2,2-trifluoro-ethyl)-1H-pyrazolo[4,3-c]pyridin-6-yl]-(4-hydroxy-4-methyl-piperidin-1-yl)-methanone |
| 209 | [3-Benzofuran-3-yl-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone |
| 210 | [3-Benzofuran-3-yl-1-(2,2,2-trifluoro-ethyl)-1H-pyrazolo[4,3-c]pyridin-6-yl]-(8-exo-ethyl-8-endo-hydroxy-3-aza-bicyclo[3.2.1]oct-3-yl)-methanone |
| 211 | [3-Benzofuran-3-yl-1-(2,2,2-trifluoro-ethyl)-1H-pyrazolo[4,3-c]pyridin-6-yl]-(5-endo-hydroxy-5-exo-methyl-2-aza-bicyclo[2.2.1]hept-2-yl)-methanone |
| 212 | [3-Benzofuran-3-yl-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone |
| 213 | [3-Benzofuran-3-yl-1-(2,2,2-trifluoro-ethyl)-1H-pyrazolo[4,3-c]pyridin-6-yl]-(8-endo-hydroxy-8-exo-methyl-d3-3-aza-bicyclo[3.2.1]oct-3-yl)-methanone |
| 214 | [3-Benzofuran-3-yl-1-(2,2,2-trifluoro-ethyl)-1H-pyrazolo[4,3-c]pyridin-6-yl]-(8-exo-ethynyl-8-endo-hydroxy-3-aza-bicyclo[3.2.1]oct-3-yl)-methanone |
| 215 | [3-Benzofuran-3-yl-1-(2,2,2-trifluoro-ethyl)-1H-pyrazolo[4,3-c]pyridin-6-yl]-(2-methoxymethyl-pyrrolidin-1-yl)-methanone |
| 216 | [3-Benzofuran-3-yl-1-(2,2,2-trifluoro-ethyl)-1H-pyrazolo[4,3-c]pyridin-6-yl]-(4-fluoro-piperidin-1-yl)-methanone |
| 217 | 3-Benzofuran-3-yl-1-(2,2,2-trifluoro-ethyl)-1H-pyrazolo[4,3-c]pyridin-6-yl]-(6,6-difluoro-1,4-oxazepan-4-yl)-methanone |
| 218 | [3-Benzofuran-3-yl-1-(2,2,2-trifluoro-ethyl)-1H-pyrazolo[4,3-c]pyridin-6-yl]-(8-endo-hydroxy-3-aza-bicyclo[3.2.1]oct-3-yl)-methanone |
| 219 | [3-Benzofuran-3-yl-1-(2,2,2-trifluoro-ethyl)-1H-pyrazolo[4,3-c]pyridin-6-yl]-(4-hydroxy-2,2-dimethyl-piperidin-1-yl)-methanone |
| 220 | [3-Benzofuran-3-yl-1-(2,2-difluoro-ethyl)-1H-pyrazolo[4,3-c]pyridin-6-yl]-(3-endo-hydroxy-8-aza-bicyclo[3.2.1]oct-8-yl)-methanone |
| 221 | [3-Benzofuran-3-yl-1-(2-fluoro-ethyl)-1H-pyrazolo[4,3-c]pyridin-6-yl]-(3-endo-hydroxy-8-aza-bicyclo[3.2.1]oct-8-yl)-methanone |
| 222 | (3-Benzofuran-3-yl-1-propyl-1H-pyrazolo[4,3-c]pyridin-6-yl)-(3-endo-hydroxy-8-aza-bicyclo[3.2.1]oct-8-yl)-methanone |
| 223 | tert-butyl N-[4-[3-(6,8-difluoroimidazo[1,2-a]pyridin-3-yl)-1-(2,2,2-trifluoroethyl)pyrazolo[4,3-c]pyridine-6-carbonyl]-1,4-oxazepan-6-yl]carbamate |
| 224 | [3-(benzofuran-3-yl)-1-(2,2,2-trifluoroethyl)pyrazolo[4,3-c]pyridin-6-yl]-(6-hydroxy-1,4-oxazepan-4-yl)methanone |
| 225 | [3-(benzofuran-3-yl)-1-(2,2,2-trifluoroethyl)pyrazolo[4,3-c]pyridin-6-yl]-(4,4-difluoro-1-piperidyl)methanone |
| 226 | [3-(benzofuran-3-yl)-1-(2,2,2-trifluoroethyl)pyrazolo[4,3-c]pyridin-6-yl]-(3,3-difluoro-1-piperidyl)methanone |
| 227 | [3-Benzofuran-3-yl-1-(2,2,2-trifluoro-ethyl)-1H-pyrazolo[4,3-c]pyridin-6-yl]-(3-hydroxy-piperidin-1-yl)-methanone |
| 228 | [3-(benzofuran-3-yl)-1-(2,2,2-trifluoroethyl)pyrazolo[4,3-c]pyridin-6-yl]-(4-hydroxyazepan-1-yl)methanone |
| 229 | [3-(benzofuran-3-yl)-1-(2,2,2-trifluoroethyl)pyrazolo[4,3-c]pyridin-6-yl]-(6,6-dideuterio-1,4-oxazepan-4-yl)methanone |
| 230 | [3-(benzofuran-3-yl)-1-(2,2,2-trifluoroethyl)pyrazolo[4,3-c]pyridin-6-yl]-[6-fluoro-1,4-oxazepan-4-yl]methanone |
| 231 | 3-(benzofuran-3-yl)-N,N-di(cyclobutyl)-1-(2,2,2-trifluoroethyl)pyrazolo[4,3-c]pyridine-6-carboxamide |
| 232 | 3-(benzofuran-3-yl)-N,N-di(cyclopropyl)-1-(2,2,2-trifluoroethyl)pyrazolo[4,3-c]pyridine-6-carboxamide |
| 233 | 3-(benzofuran-3-yl)-N,N-diisopropyl-1-(2,2,2-trifluoroethyl)pyrazolo[4,3-c]pyridine-6-carboxamide |
| 234 | 3-(benzofuran-3-yl)-N-ethyl-N-isopropyl-1-(2,2,2-trifluoroethyl)pyrazolo[4,3-c]pyridine-6-carboxamide |
| 235 | 3-(benzofuran-3-yl)-N-cyclohexyl-N-ethyl-1-(2,2,2-trifluoroethyl)pyrazolo[4,3-c]pyridine-6-carboxamide |
| 236 | [3-(benzofuran-3-yl)-1-(2,2,2-trifluoroethyl)pyrazolo[4,3-c]pyridin-6-yl]-(8-hydroxy-8-isopropyl-3-azabicyclo[3.2.1]octan-3-yl)methanone |
| 237 | 3-[6-(1,4-oxazepane-4-carbonyl)-1-(2,2,2-trifluoroethyl)pyrazolo[4,3-c]pyridin-3-yl]benzofuran-5-carboxamide |
| 238 | (3,8-Diaza-bicyclo[3.2.1]oct-3-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 239 | (2,5-Diaza-bicyclo[2.2.1]hept-2-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 240 | (3-Amino-azepan-1-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 241 | (6-Amino-1,4-oxazepan-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 242 | (6,6-dideuterio-1,4-oxazepan-4-yl)-[3-(1H-indol-3-yl)-1-(2,2,2-trifluoroethyl)pyrazolo[4,3-c]pyridin-6-yl]methanone |
| 243 | [3-(1H-indol-3-yl)-1-(2,2,2-trifluoroethyl)pyrazolo[4,3-c]pyridin-6-yl]-(8-oxa-3-azabicyclo[3.2.1]octan-3-yl)methanone |
| 244 | 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carboxylic acid amide |
| 245 | 3-[6-(Azepane-1-carbonyl)-1-propyl-1H-pyrazolo[4,3-c]pyridin-3-yl]-imidazo[1,2-a]pyridine-6-carboxylic acid amide |
| 246 | 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carboxylic acid amide |
| 247 | 3-[6-(8-Oxa-3-aza-bicyclo[3.2.1]octane-3-carbonyl)-1-(2,2,2-trifluoro-ethyl)-1H-pyrazolo[4,3-c]pyridin-3-yl]-imidazo[1,2-a]pyridine-6-carboxylic acid amide |
| 248 | Azepan-1-yl-[3-pyrazolo[1,5-a]pyrazin-3-yl-1-(2,2,2-trifluoro-ethyl)-1H-pyrazolo[4,3-c]pyridin-6-yl]-methanone |
| 249 | 1,4-Oxazepan-4-yl-[3-pyrazolo[1,5-a]pyrazin-3-yl-1-(2,2,2-trifluoro-ethyl)-1H-pyrazolo[4,3-c]pyridin-6-yl]-methanone |
| 250 | (1-Methanesulfonylmethyl-3-pyrazolo[1,5-a]pyrazin-3-yl-1H-pyrazolo[4,3-c]pyridin-6-yl)-(1,4-oxazepan-4-yl)-methanone |
| 251 | [3-(benzimidazol-1-yl)-1-(2,2,2-trifluoroethyl)pyrazolo[4,3-c]pyridin-6-yl]-(1,4-oxazepan-4-yl)methanone |
| 252 | [3-(6-fluorobenzimidazol-1-yl)-1-(2,2,2-trifluoroethyl)pyrazolo[4,3-c]pyridin-6-yl]-(1,4-oxazepan-4-yl)methanone |
| 253 | [3-furo[3,2-b]pyridin-3-yl-1-(2,2,2-trifluoroethyl)pyrazolo[4,3-c]pyridin-6-yl]-(1,4-oxazepan-4-yl)methanone |
| 254 | [1,4]Oxazepan-4-yl-[3-(1H-pyrrolo[3,2-b]pyridin-3-yl)-1-(2,2,2-trifluoroethyl)-1 H-pyrazolo[4,3-c]pyridin-6-yl]-methanone |
| 255 | [1,4]Oxazepan-4-yl-[3-(1H-pyrrolo[2,3-c]pyridin-3-yl)-1-(2,2,2-trifluoro-ethyl)-1H-pyrazolo[4,3-c]pyridin-6-yl]-methanone |
| 256 | (8-Oxa-3-aza-bicyclo[3.2.1]oct-3-yl)-[3-(1H-pyrrolo[3,2-c]pyridin-3-yl)-1-(2,2,2-trifluoro-ethyl)-1H-pyrazolo[4,3-c]pyridin-6-yl]-methanone |

and pharmaceutically acceptable salts, and solvates of any one of these compounds.

The present invention also relates to each of the intermediates described further below in the examples section of this specification, including any one of these intermediates in non-salt form or in the form of a salt (e.g., a pharmaceutically acceptable salt) of the respective compound. Such intermediates can be used, in particular, in the synthesis of the compounds of formula (I).

In a first specific embodiment, the present invention relates to a compound of formula (I) or a pharmaceutically acceptable salt, or solvate thereof, wherein the groups/variables in formula (I) have the following meanings:
The groups R^{1A} and R^{1B} are mutually linked to form, together with the nitrogen atom that they are attached to, a heterocycloalkyl which is optionally substituted with one or more groups R¹¹.

The heterocycloalkyl which is formed from R^{1A}, R^{1B} and the nitrogen atom linking R^{1A} and R^{1B} (and which is optionally substituted with one or more groups R¹¹) is preferably a monocyclic, bicyclic or tricyclic heterocycloalkyl (e.g., a bicyclic fused, bridged or spirocyclic heterocycloalkyl, or a tricyclic fused, bridged and/or spirocyclic heterocycloalkyl); more preferably, it is a monocyclic heterocycloalkyl having 3, 4, 5, 6, 7 or 8 ring atoms (particularly 5, 6, 7 or 8 ring atoms), a bicyclic heterocycloalkyl (e.g., a bicyclic fused, bridged or spirocyclic heterocycloalkyl; particularly a bicyclic bridged heterocycloalkyl) wherein each ring of said bicyclic heterocycloalkyl independently has 3, 4, 5, 6, 7 or 8 ring atoms (particularly 5, 6, 7 or 8 ring atoms), or a tricyclic heterocycloalkyl (e.g., a tricyclic fused, bridged and/or spirocyclic heterocycloalkyl; particularly a tricyclic bridged heterocycloalkyl) wherein each ring of said tricyclic heterocycloalkyl independently has 3, 4, 5, 6, 7 or 8 ring atoms (particularly 5, 6, 7 or 8 ring atoms). In the heterocycloalkyl formed from R^{1A}, R^{1B} and the nitrogen atom linking R^{1A} and R^{1B}, one or more carbon ring atoms may optionally be oxidized (to form an oxo group -C(=O)-), one or more nitrogen ring atoms (if present) may optionally be oxidized, and/or one or more sulfur ring atoms (if present) may optionally be oxidized (e.g., to form a sulfonyl group -S(=O)₂-). It is furthermore preferred that the heterocycloalkyl (including any one of the aforementioned preferred examples of the heterocycloalkyl) contains one nitrogen ring atom (i.e., the nitrogen atom linking R^{1A} and R^{1B}) and optionally one further ring heteroatom selected from nitrogen, oxygen and sulfur (wherein said optional further ring heteroatom, if present, is preferably an oxygen ring atom), wherein the remaining ring atoms are all carbon atoms.

Examples of the heterocycloalkyl, which is formed from R^{1A}, R^{1B} and the nitrogen atom linking R^{1A} and R^{1B}, include any of the respective heterocycloalkyl groups comprised in any of the specific compounds of the invention disclosed herein below in the examples section. In particular, the heterocycloalkyl formed from R^{1A}, R^{1B} and the nitrogen atom linking R^{1A} and R^{1B} may be selected, for example, from any one of the following groups:

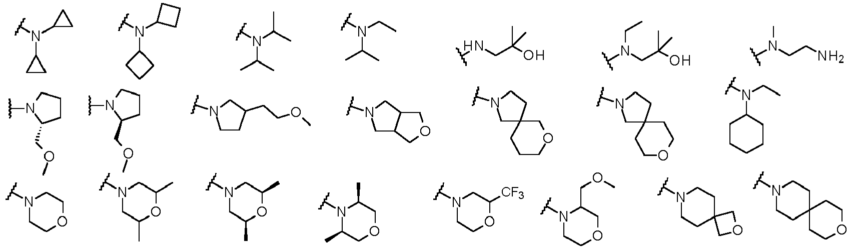

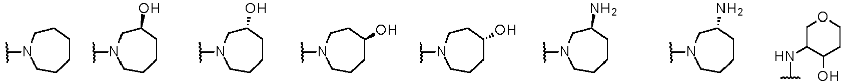

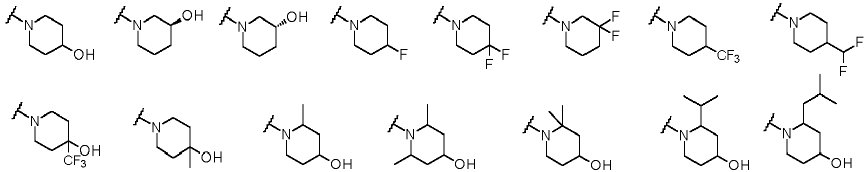

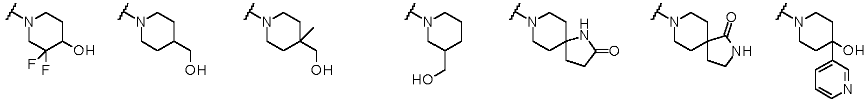

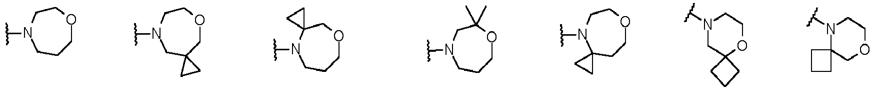

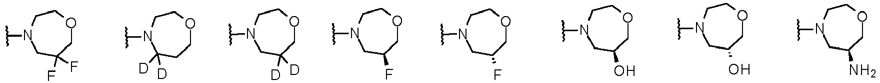

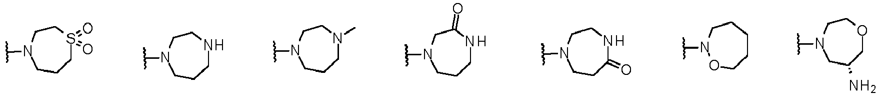

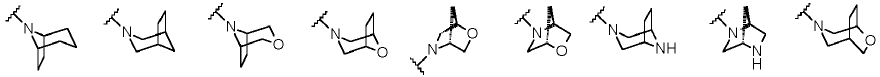

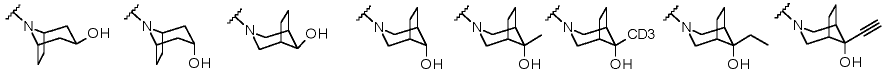

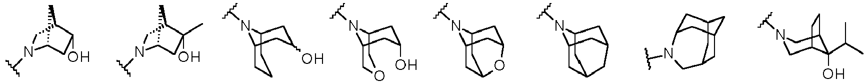

Particularly preferred examples of the heterocycloalkyl, which is formed from R^{1A}, R^{1B} and the nitrogen atom which R^{1A} and R^{1B} are attached to (and which is optionally substituted with one or more groups R¹¹), include 1,4-oxazepan-4-yl, piperidin-1-yl, 8-oxa-3-azabicyclo[3.2.1]octan-3-yl, or 8-azabicyclo[3.2.1]octan-8-yl, wherein said 1,4-oxazepan-4-yl, said piperidin-1-yl, said 8-oxa-3-azabicyclo[3.2.1]octan-3-yl, and said 8-azabicyclo[3.2.1]octan-8-yl are each optionally substituted with one or more groups R¹¹; said piperidin-1-yl is preferably substituted with -OH in position 4 (or with -OH and -CH₃ in position 4), and is optionally further substituted with one or more groups R¹¹; said 8-azabicyclo[3.2.1]octan-8-yl is preferably substituted with -OH in position 3 (particularly in endo-configuration; i.e., 3-endo-hydroxy-8-azabicyclo[3.2.1]octan-8-yl), and is optionally further substituted with one or more groups R¹¹; and said 1,4-oxazepan-4-yl may be substituted with -OH in position 6, and may optionally be further substituted with one or more groups R¹¹.

Accordingly, it is particularly preferred that R^{1A} and R^{1B} are mutually linked to form, together with the nitrogen atom that they are attached to, a group which is selected from 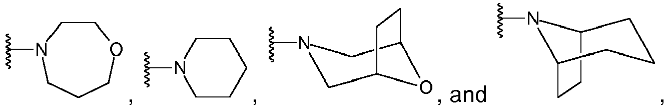 wherein each one of the above-depicted groups is optionally substituted with one or more R¹¹.

Even more preferably, R^{1A} and R^{1B} are mutually linked to form, together with the nitrogen atom that they are attached to, a group which is selected from 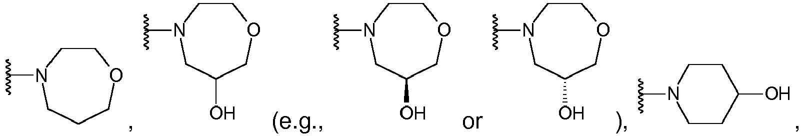 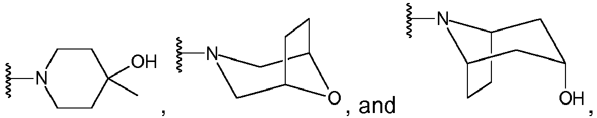 wherein each one of the above-depicted groups is optionally substituted with one or more R¹¹.

Yet even more preferably, R^{1A} and R^{1B} are mutually linked to form, together with the nitrogen atom that they are attached to, a group which is selected from 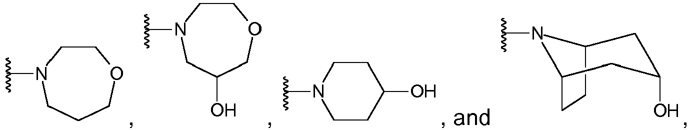 wherein each one of the above-depicted groups is optionally substituted with one or more R¹¹.

In this first embodiment, each R¹¹ is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CON(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl. Preferably, each R¹¹ is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN. More preferably, each R¹¹ is independently selected from C₁₋₄ alkyl (e.g., methyl or ethyl), -OH, -O(C₁₋₄ alkyl) (e.g., -OCH₃ or -OCH₂CH₃), -NH₂, -NH(C₁₋₄ alkyl) (e.g., -NHCH₃), -N(C₁₋₄ alkyl)(C₁₋₄ alkyl) (e.g., -N(CH₃)₂), halogen (e.g., -F, -Cl, -Br, or -I), -CF₃, and -CN.

In this first embodiment, R² and R⁴ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-cycloalkyl, and -(C₀₋₃ alkylene)-heterocycloalkyl. Preferably, R² and R⁴ are each independently selected from hydrogen, C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl. More preferably, R² and R⁴ are each independently selected from hydrogen, C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN. Even more preferably, R² and R⁴ are each independently selected from hydrogen, C₁₋₄ alkyl (e.g., methyl or ethyl), -OH, -O(C₁₋₄ alkyl) (e.g., -OCH₃ or-OCH₂CH₃), -NH₂, -NH(C₁₋₄ alkyl) (e.g., -NHCH₃), -N(C₁₋₄ alkyl)(C₁₋₄ alkyl) (e.g., -N(CH₃)₂), halogen (e.g., -F, -Cl, -Br, or -I), -CF₃, and -CN. Still more preferably, R² and R⁴ are each hydrogen.

In this first embodiment, R³ is selected from C₁₋₅ haloalkyl, -(C₁₋₅ alkylene)-CN, -(C₁₋₅ alkylene)-SO-(C₁₋₅ alkyl), and -(C₁₋₅ alkylene)-SO₂-(C₁₋₅ alkyl). Preferably, R³ is selected from C₁₋₃ haloalkyl (e.g., -CH₂-CF₃, -CH₂-CHF₂, or-CH₂-CH₂F), -(C₁₋₃ alkylene)-CN (e.g., -CH₂-CN), -(C₁₋₃ alkylene)-SO-(C₁₋₃ alkyl) (e.g., -CH₂-SO-CH₃) and -(C₁₋₃ alkylene)-SO₂-(C₁₋₃ alkyl) (e.g., -CH₂-SO₂-CH₃, -CH₂CH₂-SO₂-CH₃, or -CH₂-SO₂-CH₂CH₃). More preferably, R³ is selected from C₁₋₃ haloalkyl (particularly -CH₂-CF₃) and -(C₁₋₃ alkylene)-SO₂-(C₁₋₃ alkyl) (particularly -CH₂-SO₂-CH₃). Even more preferably, R³ is -CH₂-CF₃ or -CH₂-SO₂-CH₃.

In this first embodiment, ring A is a fused bicyclic heteroaryl, wherein said fused bicyclic heteroaryl is optionally substituted with one or more groups R^{A}. Preferably, ring A is a fused bicyclic heteroaryl, wherein each of the two fused rings (of said fused bicyclic heteroaryl) independently has 5 or 6 ring atoms, and wherein said bicyclic heteroaryl is optionally substituted with one or more groups R^{A}. More preferably, ring A is a fused bicyclic heteroaryl, wherein one of the two fused rings has 5 ring atoms and the other fused ring has 6 ring atoms, and further wherein said bicyclic heteroaryl is optionally substituted with one or more groups R^{A}. Even more preferably, ring A is a fused bicyclic heteroaryl, wherein each of the two fused rings is aromatic, wherein one of the two fused rings has 5 ring atoms and the other fused ring has 6 ring atoms, and further wherein said bicyclic heteroaryl is optionally substituted with one or more groups R^{A}; accordingly, it is particularly preferred that ring A is a fused bicyclic heteroaryl, wherein one of the two fused rings is a 5-membered aromatic ring and the other fused ring is a 6-membered aromatic ring, and further wherein said bicyclic heteroaryl is optionally substituted with one or more groups R^{A}.

If ring A is a fused bicyclic heteroaryl composed of a 5-membered ring and a 6-membered ring (e.g., as described herein above), wherein the bicyclic heteroaryl is optionally substituted with one or more groups R^{A}, it is furthermore preferred that the corresponding bicyclic heteroaryl is attached via its fused 5-membered ring to the 5-azaindazole moiety of the compound of formula (I). Even more preferably, the bicyclic heteroaryl is attached to the 5-azaindazole moiety of the compound of formula (I) via a ring atom of its fused 5-membered ring, which ring atom is adjacent to one of the two ring atoms that are shared by the two fused rings, as illustrated in the following (the heteroatoms comprised in the fused bicyclic heteroaryl are not shown):

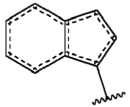

It is particularly preferred that ring A is a bicyclic heteroaryl having the following formula (II): 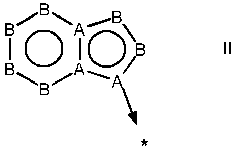 wherein each of the two fused rings (of the bicyclic heteroaryl) is aromatic, wherein each ring atom A (of the bicyclic heteroaryl) is independently a carbon or nitrogen atom, wherein each ring atom B is independently a carbon, nitrogen, oxygen or sulfur atom, wherein at least one of the ring atoms A and/or B is different from carbon, and wherein said bicyclic heteroaryl is optionally substituted with one or more groups R^{A}.

Moreover, for each of the general or preferred definitions of ring A described herein above, it is further preferred that the corresponding bicyclic heteroaryl comprises 1, 2, 3 or 4 ring heteroatoms (more preferably, 1, 2 or 3 ring heteroatoms; even more preferably, 1 or 2 ring heteroatoms) which are independently selected from nitrogen, oxygen and sulfur, wherein the remaining ring atoms are all carbon atoms; the aforementioned 1, 2, 3 or 4 ring heteroatoms may be present in only one or in both of the two fused rings.

Even more preferably, ring A is selected from any one of the following groups: 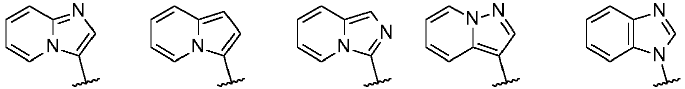 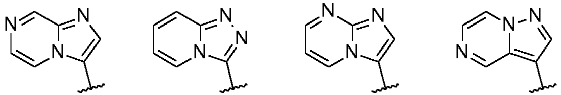 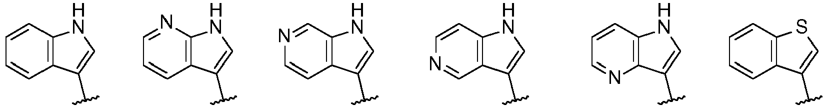 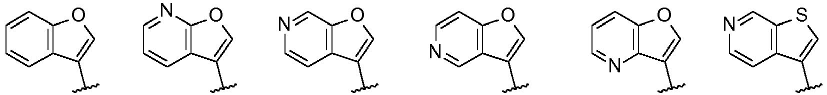 wherein each of the above-depicted groups is optionally substituted with one or more R^{A}.

Yet even more preferably, ring A is selected from any one of the following groups: 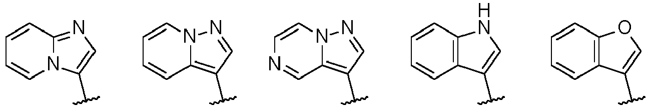 wherein each of the above-depicted groups is optionally substituted with one or more R^{A}.

Still more preferably, ring A is selected from any one of the following groups: 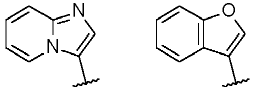 wherein each of the above-depicted groups is optionally substituted with one or more RA.

In this first embodiment, each R^{A} is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl. Preferably, each R^{A} is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN. More preferably, each R^{A} is independently selected from C₁₋₄ alkyl (e.g., methyl or ethyl), -OH, -O(C₁₋₄ alkyl) (e.g., -OCH₃ or -OCH₂CH₃), halogen (e.g., -F, -Cl, -Br, or -I; particularly -F or -CI), -CF₃, and -CN. It is particularly preferred that each R^{A} is independently halogen, and still more preferably each R^{A} is -F.

In a second specific embodiment, the present invention relates to a compound of formula (I) or a pharmaceutically acceptable salt, or solvate thereof, wherein the groups R¹¹. R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 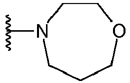 which is optionally substituted with one or more R¹¹;
the group R³ is -CH₂-CF₃; and
ring A is a group 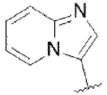
which is optionally substituted with one or more R^{A}.

In a third specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 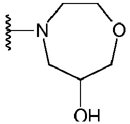
which is optionally substituted with one or more R¹¹;
the group R³ is -CH₂-CF₃; and
ring A is a group 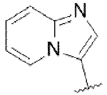
which is optionally substituted with one or more R^{A}.

In a fourth specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 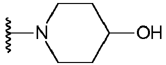
which is optionally substituted with one or more R¹¹;
the group R³ is -CH₂-CF₃; and
ring A is a group 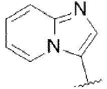 which is optionally substituted with one or more R^{A}.

In a fifth specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 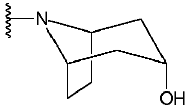
which is optionally substituted with one or more R¹¹;
the group R³ is -CH₂-CF₃; and
ring A is a group 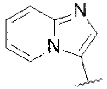
which is optionally substituted with one or more R^{A}.

In a sixth specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 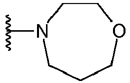
which is optionally substituted with one or more R¹¹;
the group R³ is -CH₂-SO₂-CH₃; and
ring A is a group 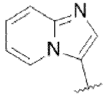
which is optionally substituted with one or more R^{A}.

In a seventh specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 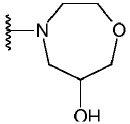
which is optionally substituted with one or more R¹¹;
the group R³ is -CH₂-SO₂-CH₃; and
ring A is a group 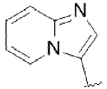 which is optionally substituted with one or more R^{A}.

In an eighth specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 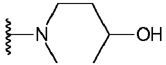
which is optionally substituted with one or more R¹¹;
the group R³ is -CH₂-SO₂-CH₃; and
ring A is a group 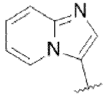
which is optionally substituted with one or more R^{A}.

In a ninth specific embodiment, the present invention relates to a compound of - pharmaceutically acceptable salt or solvate thereof, wherein formula (I) or a the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 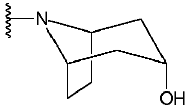
which is optionally substituted with one or more R¹¹;
the group R³ is -CH₂-SO₂-CH₃; and
ring A is a group 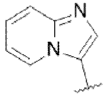
which is optionally substituted with one or more R^{A}.

In a tenth specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 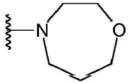
which is optionally substituted with one or more R¹¹;
the group R³ is -CH₂-CF₃; and
ring A is a group 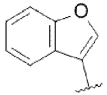
which is optionally substituted with one or more R^{A}.

In an 11^{th} specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 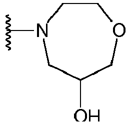
which is optionally substituted with one or more R¹¹;
the group R³ is -CH₂-CF₃; and
ring A is a group 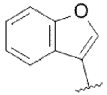
which is optionally substituted with one or more R^{A}.

In a 12^{th} specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 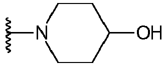
which is optionally substituted with one or more R¹¹;
the group R³ is -CH₂-CF₃; and
ring A is a group 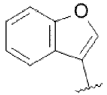
which is optionally substituted with one or more R^{A}.

In a 13^{th} specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 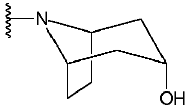
which is optionally substituted with one or more R¹¹;
the group R³ is -CH₂-CF₃; and
ring A is a group 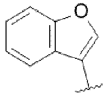
which is optionally substituted with one or more R^{A}.

In a 14^{th} specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 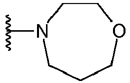
which is optionally substituted with one or more R¹¹;
the group R³ is -CH₂-SO₂-CH₃; and
ring A is a group 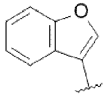
which is optionally substituted with one or more R^{A}.

In a 15^{th} specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 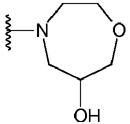
which is optionally substituted with one or more R¹¹;
the group R³ is -CH₂-SO₂-CH₃; and
ring A is a group 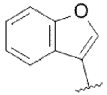
which is optionally substituted with one or more R^{A}.

In an 16^{th} specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 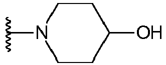
which is optionally substituted with one or more R¹¹;
the group R³ is -CH₂-SO₂-CH₃; and
ring A is a group 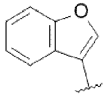
which is optionally substituted with one or more R^{A}.

In a 17^{th} specific embodiment, the present invention relates to a compound of formula (I) or a pharmaceutically acceptable salt, or solvate thereof, wherein the groups R¹¹, R², R⁴ and R^{A} are each as defined in the above-described first specific embodiment, and wherein the further groups/variables in formula (I) have the following meanings:
the groups R^{1A} and R^{1B} are mutually linked to form, together with the nitrogen atom that they are attached to, a group 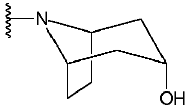
which is optionally substituted with one or more R¹¹;
the group R³ is -CH₂-SO₂-CH₃; and
ring A is a group 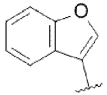
which is optionally substituted with one or more R^{A}.

The compounds of formula (I) according to the present invention can be prepared, e.g., in accordance with or in analogy to the synthetic routes described in the examples section, particularly in Example 2.

It is also possible to carry out the reactions stepwise in each case and to modify the sequence of the linking reactions of the building blocks with adaptation of the protecting-group concept.

The starting materials or starting compounds are generally known. If they are novel, they can be prepared by methods known per se.

If desired, the starting materials can also be formed in situ by not isolating them from the reaction mixture, but instead immediately converting them further into the compounds of the formula I.

The compounds of the formula I are preferably obtained by liberating them from their functional derivatives by solvolysis, in particular by hydrolysis, or by hydrogenolysis. Preferred starting materials for the solvolysis or hydrogenolysis are those which contain correspondingly protected amino, carboxyl and/or hydroxyl groups instead of one or more free amino, carboxyl and/or hydroxyl groups, preferably those which carry an amino-protecting group instead of an H atom which is connected to an N atom. Preference is furthermore given to starting materials which carry a hydroxyl-protecting group instead of the H atom of a hydroxyl group. Preference is also given to starting materials which carry a protected carboxyl group instead of a free carboxyl group. It is also possible for a plurality of identical or different protected amino, carboxyl and/or hydroxyl groups to be present in the molecule of the starting material. If the protecting groups present are different from one another, they can in many cases be cleaved off selectively.

The term "amino-protecting group" is generally known and relates to groups which are suitable for protecting (blocking) an amino group against chemical reactions, but which can easily be removed after the desired chemical reaction has been carried out elsewhere in the molecule. Typical of such groups are, in particular, unsubstituted or substituted acyl groups, furthermore unsubstituted or substituted aryl (for example 2,4-dinitophenyl) or aralkyl groups (for example benzyl, 4-nitrobenzyl, triphenylmethyl). Since the amino-protecting groups are removed after the desired reaction or reaction sequence, their type and size is, in addition, not crucial, but preference is given to those having 1-20, in particular 1-8, C atoms. The term "acyl group" is to be understood in the broadest sense in connection with the present process. It encompasses acyl groups derived from aliphatic, araliphatic, aromatic or heterocyclic carboxylic acids or sulfonic acids and, in particular, alkoxycarbonyl, aryloxycarbonyl and especially aralkoxycarbonyl groups. Examples of such acyl groups are alkanoyl, such as acteyl, propionyl, buturyl, aralkanoyl, such as phenylacetyl, aroyl, such as benzoyl or toluyl, aryoxyaklkanoyl, such as phenoxyacetyl, alkyoxycarbonyyl, such as methoxycarbonyl, ethoxycarbonyl, 2,2,2-trichloroethoxycarbonyl, BOC (tert-butoxycarbonyl), 2-iodoethoxycaronyl, aralkoxycarbonyl, such as CBZ (benzyloxycarbonyl), 4-methoxybenzyloxycarbonyl or FMOC (9-fluorenylmethoxycarbonyl). Preferred acyl groups are CBZ, FMOC, benzyl and acetyl.

The term "acid-protecting group" or "carboxyl-protecting group" is likewise generally known and relates to groups which are suitable for protecting a -COOH group against chemical reactions, but which can easily be removed after the desired chemical reaction has been carried out elsewhere in the molecule. The use of esters instead of the free acids, for example of substituted and unsubstituted alkyl esters (such as methyl, ethyl, tert-butyl and substituted derivatives thereof), of substituted and unsubstituted benzyl esters or silyl esters, is typical. The type and size of the acid-protecting groups is not crucial, but preference is given to those having 1-20, in particular 1-10, C atoms.

The term "hydroxyl-protecting group" is likewise generally known and relates to groups which are suitable for protecting a hydroxyl group against chemical reactions, but which can easily be removed after the desired chemical reaction has been carried out elsewhere in the molecule. Typical of such groups are the above-mentioned unsubstituted or substituted aryl, aralkyl or acyl groups, furthermore also alkyl groups. Their type and size of the hydroxyl-protecting groups is not crucial, but preference is given to those having 1-20, in particular 1-10, C atoms. Examples of hyrdoxyl-protecting groups are, inter alia, benzyl, p-nitrobenzoyl, p-toluenesulfonyl and acetyl, where benzyl and acetyl are preferred.

Further typical examples of amino-, acid- and hydroxyl-protecting groups are found, for example, in "Greene's Protective Groups in Organic Synthesis", fourth edition, Wiley-Interscience, 2007.

The compounds of the formula I are liberated from their functional derivatives, depending on the protecting group used, for example, with the aid of strong acids, advantageously using trifluoroacetic acid or perchloric acid, but also using other strong inorganic acids, such as hydrochloric acid or sulfuric acid, strong organic acids, such as trichloroacetic acid, or sulfonic acids, such as benzoyl- or p-toluenesulfonic acid. The presence of an additional inert solvent and/or a catalyst is possible but is not always necessary.

Depending on the respective synthetic route, the starting materials can optionally be reacted in the presence of an inert solvent.

Suitable inert solvents are, for example, heptane, hexane, petroleum ether, DMSO, benzene, toluene, xylene, trichloroethylene-, 1,2-dichloroethanecarbon tetrachloride, chloroform or dichloromethane; alcohols, such as methanol, ethanol, isopropanol, n-propanol, n-butanol or tert-butanol; ethers, such as diethyl ether, diisopropyl ether (preferably for substitution on the indole nitrogen), tetrahydrofuran (THF) or dioxane; glycol ethers, such as ethylene glycol monomethyl or monoethyl ether, ethylene glycol dimethy-l ether (diglyme); ketones, such as acetone or butanone; amides, such as acetamide, dimethylacetamide, N-methylpyrrolidone (NMP) or dimethylformamide (DMF); nitriles, such as acetonitrile; esters, such as ethyl acetate, carboxylic acids or acid anhydrides, such as, for example, such as acetic acid or acetic anhydride, nitro compounds, such as nitromethane or nitrobenzene, optionally also mixtures of the said solvents with one another or mixtures with water.

The amount of solvent is not crucial; 10 g to 500 g of solvent can preferably be added per g of the compound of the formula I to be reacted.

It may be advantageous to add an acid-binding agent, for example an alkali metal or alkaline-earth metal hydroxide, carbonate or bicarbonate or other alkali or alkaline-earth metal salts of weak acids, preferably a potassium, sodium or calcium salt, or to add an organic base, such as, for example, on triethylamine, dimethylamine, pyridine or quinoline, or an excess of the amine component.

The resultant compounds according to the invention can be separated from the corresponding solution in which they are prepared (for example by centrifugation and washing) and can be stored in another composition after separation, or they can remain directly in the preparation solution. The resultant compounds according to the invention can also be taken up in desired solvents for the particular use.

The reaction duration depends on the reaction conditions selected. In general, the reaction duration is 0.5 hour to 10 days, preferably 1 to 24 hours. On use of a microwave, the reaction time can be reduced to values of 1 to 60 minutes.

The compounds of the formula I and also the starting materials for their preparation are, in addition, prepared by known methods, as described in the literature (for example in standard works, such as Houben-Weyl, Methoden der organischen Chemie [Methods of Organic Chemistry], Georg-Thieme-Verlag, Stuttgart), for example under reaction conditions which are known and suitable for the said reactions. Use can also be made here of variants known per se, which are not described here in greater detail.

Conventional work-up steps, such as, for example, addition of water to the reaction mixture and extraction, enable the compounds to be obtained after removal of the solvent. It may be advantageous, for further purification of the product, to follow this with a distillation or crystallisation or to carry out a chromatographic purification.

An acid of the formula I can be converted into the associated addition salt using a base, for example by reaction of equivalent amounts of the acid and base in an inert solvent, such as ethanol, and inclusive evaporation. Suitable bases for this reaction are, in particular, those which give physiologically acceptable salts. Thus, the acid of the formula I can be converted into the corresponding metal salt, in particular alkali or alkaline-earth metal salt, using a base (for example sodium hydroxide, potassium hydroxide, sodium carbonate or potassium carbonate) or into the corresponding ammonium salt. Organic bases which give physiologically acceptable salts, such as, for example, ethanolamine, are also suitable for this reaction.

On the other hand, a base of the formula I can be converted into the associated acid-addition salt using an acid, for example by reaction of equivalent amounts of the base and acid in an inert solvent, such as ethanol, with subsequent evaporation. Suitable acids for this reaction are, in particular, those which give physiologically acceptable salts. Thus, it is possible to use inorganic acids, for example sulfuric acid, nitric acid, hydrohalic acids, such as hydrochloric acid or hydrobromic acid, phosphoric acids, such as orthophosphoric acid, sulfamic acid, furthermore organic acids, in particular aliphatic, alicyclic, araliphatic, aromatic or heterocyclic, mono- or polybasic carboxylic, sulfonic or sulfuric acids, for example formic acid, acetic acid, propionic acid, pivalic acid, diethylacetic acid, malonic acid, succinic acid, pimelic acid, fumaric acid, maleic acid, lactic acid, tartaric acid, malic acid, citric acid, gluconic acid, ascorbic acid, nicotinic acid, isonicotinic acid, methane- or ethanesulfonic acid, ethanedisulfonic acid, 2-hydroxysulfonic acid, benzenesulfonic acid, p-toluenesulfonic acid, naphthalenemom- and disulfonic acids or laurylsulfuric acid. Salts with physiologically unacceptable acids, for example picrates, can be used for the isolation and/or purification of the compounds of the formula I.

The following definitions apply throughout the present specification and the claims, unless specifically indicated otherwise.

The term "hydrocarbon group" refers to a group consisting of carbon atoms and hydrogen atoms.

As used herein, the term "alkyl" refers to a monovalent saturated acyclic (i.e., noncyclic) hydrocarbon group which may be linear or branched. Accordingly, an "alkyl" group does not comprise any carbon-to-carbon double bond or any carbon-to-carbon triple bond. A "C₁₋₅ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₅ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₅ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₅ alkylene" denotes an alkylene group having 1 to 5 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydro-naphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, pheno-xathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothia-diazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazo-pyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., deca-hydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thio-morpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, thiepanyl, decahydroquinolinyl, decahydro-isoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkenyl" refers to an unsaturated alicyclic (i.e., non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (i.e., non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-CI), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

The scope of the present invention embraces all pharmaceutically acceptable salt forms of the compounds of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentane-propionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. It will be understood that the present invention also relates to the compounds of formula (I), including any one of the specific compounds described herein, in non-salt form.

Moreover, the scope of the invention embraces the compounds of formula (I) in any solvated form, including, e.g., solvates with water (i.e., as a hydrate) or solvates with organic solvents such as, e.g., methanol, ethanol or acetonitrile (i.e., as a methanolate, ethanolate or acetonitrilate). All physical forms, including any amorphous or crystalline forms (i.e., polymorphs), of the compounds of formula (I) are also encompassed within the scope of the invention. It is to be understood that such solvates and physical forms of pharmaceutically acceptable salts of the compounds of the formula (I) are likewise embraced by the invention.

Furthermore, the compounds of formula (I) may exist in the form of different isomers, in particular stereoisomers (including, e.g., geometric isomers (or cis/trans isomers), enantiomers, and diastereomers) or tautomers (including, in particular, prototropic tautomers). All such isomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds provided herein (e.g., keto/enol tautomers).

The present invention also relates to mixtures of the compounds of formula (I) according to the invention, for example mixtures of two diastereomers, for example in the ratio 1:1, 1:2, 1:3, 1:4, 1:5, 1:10, 1:100 or 1:1000. These are particularly preferably mixtures of two stereoisomeric compounds. However, preference is also given to mixtures of two or more compounds of formula (I).

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., ²H; also referred to as "D"). Accordingly, the invention also embraces compounds of formula (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

As explained above, it is intended that a compound of formula (I) includes isotope-labelled forms thereof. An isotope-labelled form of a compound of formula (I) is identical to this compound apart from the fact that one or more atoms of the compound have been replaced by an atom or atoms having an atomic mass or mass number which differs from the atomic mass or mass number of the atom which usually occurs naturally. Examples of isotopes which are readily commercially available and which can be incorporated into a compound of the formula (I) by well-known methods include isotopes of hydrogen, carbon, nitrogen, oxygen, phosphorus, fluorine and chlorine, for example ²H, ³H, ¹³C, ¹⁴C, ¹⁵N, ¹⁸O, ¹⁷O, ³¹P, ³²P, ³⁵S, ¹⁸F and ³⁶Cl, respectively. A compound of formula (I) or a pharmaceutically acceptable salt, solvate or prodrug thereof, any of which contains one or more of the above-mentioned isotopes and/or other isotopes of other atoms, is intended to be part of the present invention. An isotope-labelled compound of formula (I) can be used in a number of beneficial ways. For example, an isotope-labelled compound of formula (I) into which, for example, a radioisotope, such as ³H or ¹⁴C, has been incorporated is suitable for medicament and/or substrate tissue distribution assays. These radioisotopes, i.e. tritium (³H) and carbon-14 (¹⁴C), are particularly preferred owing to their simple preparation and excellent detectability. Incorporation of heavier isotopes, for example deuterium (²H), into a compound of formula (I) may have therapeutic advantages owing to the higher metabolic stability of this isotope-labelled compound. Higher metabolic stability translates directly into an increased in-vivo half-life or lower dosages, which under most circumstances would represent a preferred embodiment of the present invention. An isotope-labelled compound of the formula (I) can usually be prepared by carrying out the procedures disclosed in the synthesis schemes and the related description, in the example section and in the preparation part in the present specification, replacing a non-isotope-labelled reactant with a readily available isotope-labelled reactant.

In order to manipulate the oxidative metabolism of the compound by way of the primary kinetic isotope effect, deuterium (²H) can also be incorporated into a compound of formula (I). The primary kinetic isotope effect is a change in the rate of a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus causes a reduction in the rate in rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom in a non-exchangeable position, rate differences of k_{M}/k_{D} = 2-7 are typical. If this rate difference is successfully applied to a compound of formula (I) that is susceptible to oxidation, the profile of this compound in vivo can thereby be drastically modified and result in improved pharmacokinetic properties.

When discovering and developing therapeutic agents, the person skilled in the art attempts to optimise pharmacokinetic parameters while retaining desirable in-vitro properties. It is reasonable to assume that many compounds with poor pharmacokinetic profiles are susceptible to oxidative metabolism. In-vitro liver microsomal assays currently available provide valuable information on the course of oxidative metabolism of this type, which in turn permits the rational design of deuterated compounds of formula (I) with improved stability through resistance to such oxidative metabolism. Significant improvements in the pharmacokinetic profiles of the compounds of the formula I are thereby obtained and can be expressed quantitatively in terms of increases in the in-vivo half-life (T/2), concentration at maximum therapeutic effect (Cₘₐₓ), area under the dose response curve (AUC), and F; and in terms of reduced clearance, dose and costs of materials.

The following is intended to illustrate the above: a compound of formula (I) which has multiple potential sites of attack for oxidative metabolism, for example benzylic hydrogen atoms and hydrogen atoms bonded to a nitrogen atom, is prepared as a series of analogues in which various combinations of hydrogen atoms are replaced by deuterium atoms, so that some, most or all of these hydrogen atoms have been replaced by deuterium atoms. Half-life determinations enable favourable and accurate determination of the extent to which the improvement in resistance to oxidative metabolism has improved. In this way, it is determined that the half-life of the parent compound can be extended by up to 100% as the result of deuterium-hydrogen exchange of this type.

The replacement of hydrogen by deuterium in a compound of formula (I) can also be used to achieve a favourable modification of the metabolite spectrum of the starting compound in order to diminish or eliminate undesired toxic metabolites. For example, if a toxic metabolite arises through oxidative carbon-hydrogen (C-H) bond cleavage, it can reasonably be assumed that the deuterated analogue will greatly diminish or eliminate production of the undesired metabolite, even if the particular oxidation is not a rate-determining step. Further information on the state of the art with respect to deuterium-hydrogen exchange is given, for example in Hanzlik et al., J. Org. Chem. 55, 3992-3997, 1990, Reider et al., J. Org. Chem. 52, 3326-3334, 1987, Foster, Adv. Drug Res. 14, 1-40, 1985, Gillette et al., Biochemistry 33(10), 2927-2937, 1994, and Jarman et al., Carcinogenesis 16(4), 683-688, 1993.

The present invention also embraces compounds of formula (I), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers, trackers or imaging probes in positron emission tomography (PET). The invention thus includes (i) compounds of formula (I), in which one or more fluorine atoms (or, e.g., all fluorine atoms) are replaced by ¹⁸F atoms, (ii) compounds of formula (I), in which one or more carbon atoms (or, e.g., all carbon atoms) are replaced by ¹¹C atoms, (iii) compounds of formula (I), in which one or more nitrogen atoms (or, e.g., all nitrogen atoms) are replaced by ¹³N atoms, (iv) compounds of formula (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula (I), in which one or more bromine atoms (or, e.g., all bromine atoms) are replaced by ⁷⁷Br atoms, (vii) compounds of formula (I), in which one or more iodine atoms (or, e.g., all iodine atoms) are replaced by ¹²⁰I atoms, and (viii) compounds of formula (I), in which one or more iodine atoms (or, e.g., all iodine atoms) are replaced by ¹²⁴I atoms. In general, it is preferred that none of the atoms in the compounds of formula (I) are replaced by specific isotopes.

It has been found that the compounds of the formula (I) are well tolerated and have valuable pharmacological properties.

Since adenosine receptors, such as A_{2A} and A_{2B}, are shown to down-regulate the immune response during inflammation and protect tissues from immune damage, inhibition of signaling through adenosine receptors can be used to intensify and prolong the immune response.

Methods are provided herein to increase an immune response. In one example, the method increases desirable and targeted tissue damage, such as damage of a tumor, for example cancer. Provided herein are methods of inhibiting one or more processes conducive to the production of extracellular adenosine and adenosine-triggered signaling through adenosine receptors. For example, enhancement of an immune response, local tissue inflammation, and targeted tissue destruction is accomplished by: inhibiting or reducing the adenosine-producing local tissue hypoxia; by degrading (or rendering inactive) accumulated extracellular adenosine; by preventing or decreasing expression of adenosine receptors on immune cells; and/or by inhibiting/antagonizing signaling by adenosine ligands through adenosine receptors. The results disclosed herein demonstrate that by in vivo administration of agents that disrupt the "hypoxia -> adenosine accumulation -> immunosuppressive adenosine receptor signaling to immune cells" pathway in subjects suffering from various diseases (e.g. cancer and sepsis) can result in *in vivo* treatment of tumors or improved immunization.

In one example, the method includes administering one or more inhibitors of extracellular adenosine and/or adenosine receptor inhibitors, such as an adenosine receptor antagonist. To increase the efficacy of a vaccine, one or more adenosine receptor inhibitors and/or inhibitors of extracellular adenosine can be administered in conjunction with the vaccine. In one example, one or more adenosine receptor inhibitors or inhibitors of extracellular adenosine are administered to increase an immune response/inflammation. In another example, a method is provided to achieve targeted tissue damage, such as for tumor destruction.

The invention therefore furthermore relates to the use of compounds according to the invention for the preparation of a medicament for the treatment and/or prophylaxis of diseases which are caused, promoted and/or propagated by adenosine or other A_{2A} and/or A_{2B} receptor agonists.

The invention thus also relates, in particular, to a medicament comprising at least one compound according to the invention and/or one of its pharmaceutically acceptable salts, solvates, and stereoisomers, including mixtures thereof in all ratios, for use in the treatment and/or prophylaxis of physiological and/or pathophysiological states.

Particular preference is given, in particular, to physiological and/or pathophysiological states which are connected to adenosine A_{2A} and/or A_{2B} receptors.

Physiological and/or pathophysiological states are taken to mean physiological and/or pathophysiological states which are medically relevant, such as, for example, diseases or illnesses and medical disorders, complaints, symptoms or complications and the like, in particular diseases.

The invention furthermore relates to a medicament comprising at least one compound according to the invention and/or one of its pharmaceutically acceptable salts, solvates, and stereoisomers, including mixtures thereof in all ratios, for use in the treatment and/or prophylaxis of physiological and/or pathophysiological states selected from the group consisting of hyperproliferative and infectious diseases or disorders.

The invention further relates to a medicament comprising at least one compound according to the invention and/or one of its pharmaceutically acceptable salts, solvates, and stereoisomers, including mixtures thereof in all ratios, for use in the treatment and/or prophylaxis of physiological and/or pathophysiological states selected from the group consisting of hyperproliferative and infectious diseases or disorders, wherein the hyperproliferative disease or disorder is cancer.

The invention thus particularly preferably relates to a medicament comprising at least one compound according to the invention and/or one of its pharmaceutically acceptable salts, solvates, and stereoisomers, including mixtures thereof in all ratios, wherein the cancer is selected from the group consisting of acute and chronic lymphocytic leukemia, acute granulocytic leukemia, adrenal cortex cancer, bladder cancer, brain cancer, breast cancer, cervical cancer, cervical hyperplasia, cervical cancer, chorio cancer, chronic granulocytic leukemia, chronic lymphocytic leukemia, colon cancer, endometrial ccancer, esophageal cancer, essential thrombocytosis, genitourinary carcinoma, glioma, glioblastoma, hairy cell leukemia, head and neck carcinoma, Hodgkin's disease, Kaposi's sarcoma, lung carcinoma, lymphoma, malignant carcinoid carcinoma, malignant hypercalcemia, malignant melanoma, malignant pancreatic insulinoma, medullary thyroid carcinoma, melanoma, multiple myeloma, mycosis fungoides, myeloid and lymphocytic leukemia, neuroblastoma, non-Hodgkin's lymphoma, non-small cell lung cancer, osteogenic sarcoma, ovarian carcinoma, pancreatic carcinoma, polycythemia vera, primary brain carcinoma, primary macroglobulinemia, prostatic cancer, renal cell cancer, rhabdomyosarcoma, skin cancer, small-cell lung cancer, soft-tissue sarcoma, squamous cell cancer, stomach cancer, testicular cancer, thyroid cancer and Wilms' tumor.

The invention further preferably relates to a medicament comprising at least one compound according to the invention and/or one of its pharmaceutically acceptable salts, solvates, and stereoisomers, including mixtures thereof in all ratios, for use in the treatment and/or prophylaxis of physiological and/or pathophysiological states selected from the group consisting of hyperproliferative and infectious diseases or disorders, wherein the hyperproliferative disease or disorder is selected from the group consisting of age-related macular degeneration, Crohn's disease, cirrhosis, chronic inflammatory-related disorders, proliferative diabetic retinopathy, proliferative vitreoretinopathy, retinopathy of prematurity, granulomatosis, immune hyperproliferation associated with organ or tissue transplantation and an immune-proliferative disease or disorder selected from the group consisting of inflammatory bowel disease, psoriasis, rheumatoid arthritis, systemic lupus erythematosus (SLE), vascular hyperproliferation secondary to retinal hypoxia and vasculitis.

The invention further preferably relates to a medicament comprising at least one compound according to the invention and/or one of its pharmaceutically acceptable salts, solvates, and stereoisomers, including mixtures thereof in all ratios, for use in the treatment and/or prophylaxis of physiological and/or pathophysiological states selected from the group consisting of hyperproliferative and infectious diseases or disorders, wherein the infectious disease or disorder is selected from the group consisting of
(a) virally induced infectious diseases which are caused by retroviruses, hepadnaviruses, herpesviruses, flaviviridae and/or adenoviruses wherein the retroviruses are selected from lentiviruses or oncoretroviruses, wherein the lentivirus is selected from the group consisting of HIV-1, HIV-2, FIV, BIV, SIVs, SHIV, CAEV, VMV and EIAV and the oncoretrovirus is selected from the group consisting of HTLV-I, HTLV-II and BLV, the hepadnavirus is selected from the group consisting of HBV, GSHV and WHV, the herpesivirus is selected from the group from the group consisting of HSV I, HSV II, EBV, VZV, HCMV or HHV 8 and the flaviviridae is selected from the group consisting of HCV, West nile and Yellow Fever,
(b) bacterial infectious diseases which are caused by Gram-positive bacteria wherein the Gram-positive bacteria are selected from the group consisting of methicillin-susceptible and methicillin-resistant staphylococci (including Staphylococcus aureus, Staphylococcus epidermidis, Staphylococcus haemolyticus, Staphylococcus hominis, Staphylococcus saprophyticus, and coagulase-negative staphylococci), glycopeptides-intermediate susceptible Staphylococcus aureus (GISA), penicillin-susceptible and penicillin-resistant streptococci (including Streptococcus pneumoniae, Streptococcus pyogenes, Streptococcus agalactiae, Streptococcus avium, Streptococcus bovis, Streptococcus lactis, Streptococcus sanguis and Streptococci Group C (GCS), Streptococci Group G (GGS) and viridans streptococci), enterococci (including vancomycinsusceptible and vancomycin-resistant strains such as Enterococcus faecalis and Enterococcus faecium), Clostridium difficile, listeria monocytogenes, Corynebacterium jeikeium, Chlamydia spp (including C. pneumoniae) and Mycobacterium tuberculosis,
(c) bacterial infectious diseases which are caused by Gram-negative bacteria wherein the Gram-negative bacteria are selected from the group consisting of the Genus Enterobacteriacae, including Escherichia spp. (including Escherichia coli), Klebsiella spp., Enterobacter spp., Citrobacter spp., Serratia spp., Proteus spp., Providencia spp., Salmonella spp., Shigella spp., the genus Pseudomonas (including P. aeruginosa), Moraxella spp. (including M. catarrhalis), Haemophilus spp. and Neisseria spp.,
(d) infectious diseases induced by intracellular active parasites selected from the group consisting of phylum Apicomplexa, or Sarcomastigophora (including Trypanosoma, Plasmodia, Leishmania, Babesia or Theileria), Cryptosporidia, Sacrocystida, Amoebia, Coccidia and Trichomonadia.

Since the compounds of the present invention are highly efficient A_{2A} receptor antagonists, the invention further preferably relates to a medicament comprising at least one compound according to the invention and/or one of its pharmaceutically acceptable salts, solvates, and stereoisomers, including mixtures thereof in all ratios, for use in the treatment and/or prophylaxis of physiological and/or pathophysiological states selected from the group consisting of movement disorders, acute and chronic pain, affective disorders, central and peripheric nervous system degeneration disorders, schizophrenia and related psychosis, cognitive disorders, attention disorders, central nervous system injury, cerebral ischemia, myocardial ischemia, muscle ischemia, sleep disorders, eye disorders, cardiovascular disorders, hepatic fibrosis, cirrhosis, fatty liver, substance abuse, Parkinson's disease, Alzheimer's disease and attention-deficit hyperactivity disorder.

It is intended that the medicaments disclosed above include a corresponding use of the compounds according to the invention for the preparation of a medicament for the treatment and/or prophylaxis of the above physiological and/or pathophysiological states.

It is additionally intended that the medicaments disclosed above include a compound of the present invention for use in the treatment and/or prophylaxis of the above physiological and/or pathophysiological states.

It is additionally intended that the medicaments disclosed above include a corresponding method for the treatment and/or prophylaxis of the above physiological and/or pathophysiological states in which at least one compound according to the invention is administered to a patient in need of such a treatment.

The compounds according to the invention preferably exhibit an advantageous biological activity which can easily be demonstrated in enzyme assays and animal experiments, as described in the examples. In such enzyme-based assays, the compounds according to the invention preferably exhibit and cause an inhibiting effect, which is usually documented by IC₅₀ values in a suitable range, preferably in the micromolar range and more preferably in the nanomolar range.

The compounds according to the invention can be administered to humans or animals, in particular mammals, such as apes, dogs, cats, rats or mice, and can be used in the therapeutic treatment of the human or animal body and in the combating of the above-mentioned diseases. They can furthermore be used as diagnostic agents or as reagents.

Furthermore, compounds according to the invention can be used for the isolation and investigation of the activity or expression of adenosine A_{2A} and/or A_{2B} receptors. In addition, they are particularly suitable for use in diagnostic methods for diseases in connection with disturbed adenosine A_{2A} and/or A_{2B} receptor activity. The invention therefore furthermore relates to the use of the compounds according to the invention for the isolation and investigation of the activity or expression of adenosine A_{2A} and/or A_{2B} receptors or as binders and inhibitors of adenosine A_{2A} and/or A_{2B} receptors.

For diagnostic purposes, the compounds according to the invention can, for example, be radioactively labelled. Examples of radioactive labels are ³H, ¹⁴C, ²³¹I and ¹²⁵I. A preferred labelling method is the iodogen method (Fraker et al., 1978). In addition, the compounds according to the invention can be labelled by enzymes, fluorophores and chemophores. Examples of enzymes are alkaline phosphatase, β-galactosidase and glucose oxidase, an example of a fluorophore is fluorescein, an example of a chemophore is luminol, and automated detection systems, for example for fluorescent colorations, are described, for example, in US 4,125,828 and US 4,207,554.

The present invention further relates to pharmaceutical compositions containing the compounds of the present invention and their use for the treatment and/or prophylaxis of diseases and disorders where the partial or total inactivation of adenosine A_{2A} and/or A_{2B} receptors could be beneficial.

The compounds of the formula (I) can be used for the preparation of pharmaceutical preparations, in particular by non-chemical methods. In this case, they are brought into a suitable dosage form together with at least one solid, liquid and/or semi-liquid excipient or adjuvant and optionally in combination with one or more further active compound(s).

The invention therefore furthermore relates to pharmaceutical preparations comprising at least one compound of the formula (I) and/or pharmaceutically acceptable salts, solvates, and stereoisomers thereof, including mixtures thereof in all ratios. In particular, the invention also relates to pharmaceutical preparations which comprise further excipients and/or adjuvants, and also to pharmaceutical preparations which comprise at least one further medicament active compound (or therapeutic agent).

In particular, the invention also relates to a process for the preparation of a pharmaceutical preparation, characterised in that a compound of the formula (I) and/or one of its pharmaceutically acceptable salts, solvates, and stereoisomers, including mixtures thereof in all ratios, is brought into a suitable dosage form together with a solid, liquid or semi-liquid excipient or adjuvant and optionally with a further medicament active compound.

The pharmaceutical preparations according to the invention can be used as medicaments in human or veterinary medicine. The patient or host can belong to any mammal species, for example a primate species, particularly humans; rodents, including mice, rats and hamsters; rabbits; horses, cattle, dogs, cats, etc. Animal models are of interest for experimental investigations, where they provide a model for the treatment of a human disease.

Suitable carrier substances are organic or inorganic substances which are suitable for enteral (for example oral), parenteral or topical administration and do not react with the novel compounds, for example water, vegetable oils (such as sunflower oil or cod-liver oil), benzyl alcohols, polyethylene glycols, gelatine, carbohydrates, such as lactose or starch, magnesium stearate, talc, lanolin or Vaseline. Owing to his expert knowledge, the person skilled in the art is familiar with which adjuvants are suitable for the desired medicament formulation. Besides solvents, for example water, physiological saline solution or alcohols, such as, for example, ethanol, propanol or glycerol, sugar solutions, such as glucose or mannitol solutions, or a mixture of the said solvents, gel formers, tablet assistants and other active-ingredient carriers, it is also possible to use, for example, lubricants, stabilisers and/or wetting agents, emulsifiers, salts for influencing the osmotic pressure, antioxidants, dispersants, antifoams, buffer substances, flavours and/or aromas or flavour correctants, preservatives, solubilisers or dyes. If desired, preparations or medicaments according to the invention may comprise one or more further active compounds, for example one or more vitamins.

If desired, preparations or medicaments according to the invention may comprise one or more further active compounds and/or one or more action enhancers (adjuvants).

The terms "pharmaceutical formulation" and "pharmaceutical preparation" are used as synonyms for the purposes of the present invention.

As used here, "pharmaceutically acceptable relates to medicaments, precipitation reagents, excipients, adjuvants, stabilisers, solvents and other agents which facilitate the administration of the pharmaceutical preparations obtained therefrom to a mammal without undesired physiological side effects, such as, for example, nausea, dizziness, digestion problems or the like.

In pharmaceutical preparations for parenteral administration, there is a requirement for isotonicity, euhydration and tolerability and safety of the formulation (low toxicity), of the adjuvants employed and of the primary packaging. Surprisingly, the compounds according to the invention preferably have the advantage that direct use is possible and further purification steps for the removal of toxicologically unacceptable agents, such as, for example, high concentrations of organic solvents or other toxicologically unacceptable adjuvants, are thus unnecessary before use of the compounds according to the invention in pharmaceutical formulations.

The invention particularly preferably also relates to pharmaceutical preparations comprising at least one compound according to the invention in precipitated non-crystalline, precipitated crystalline or in dissolved or suspended form, and optionally excipients and/or adjuvants and/or further pharmaceutical active compounds.

The compounds according to the invention preferably enable the preparation of highly concentrated formulations without unfavourable, undesired aggregation of the compounds according to the invention occurring. Thus, ready-to-use solutions having a high active-ingredient content can be prepared with the aid of compounds according to the invention with aqueous solvents or in aqueous media.

The compounds and/or physiologically acceptable salts and solvates thereof can also be lyophilised and the resultant lyophilisates used, for example, for the preparation of injection preparations.

Aqueous preparations can be prepared by dissolving or suspending compounds according to the invention in an aqueous solution and optionally adding adjuvants. To this end, defined volumes of stock solutions comprising the said further adjuvants in defined concentration are advantageously added to a solution or suspension having a defined concentration of compounds according to the invention, and the mixture is optionally diluted with water to the pre-calculated concentration. Alternatively, the adjuvants can be added in solid form. The amounts of stock solutions and/or water which are necessary in each case can subsequently be added to the aqueous solution or suspension obtained. Compounds according to the invention can also advantageously be dissolved or suspended directly in a solution comprising all further adjuvants.

The solutions or suspensions comprising compounds according to the invention and having a pH of 4 to 10, preferably having a pH of 5 to 9, and an osmolality of 250 to 350 mOsmol/kg can advantageously be prepared. The pharmaceutical preparation can thus be administered directly substantially without pain intravenously, intra-arterially, intra-articularly, subcutaneously or percutaneously. In addition, the preparation may also be added to infusion solutions, such as, for example, glucose solution, isotonic saline solution or Ringer's solution, which may also contain further active compounds, thus also enabling relatively large amounts of active compound to be administered.

Pharmaceutical preparations according to the invention may also comprise mixtures of a plurality of compounds according to the invention.

The preparations according to the invention are physiologically well tolerated, easy to prepare, can be dispensed precisely and are preferably stable with respect to assay, decomposition products and aggregates throughout storage and transport and during multiple freezing and thawing processes. They can preferably be stored in a stable manner over a period of at least three months to two years at refrigerator temperature (2-8°C) and at room temperature (23-27°C) and 60% relative atmospheric humidity (R.H.).

For example, the compounds according to the invention can be stored in a stable manner by drying and when necessary converted into a ready-to-use pharmaceutical preparation by dissolution or suspension. Possible drying methods are, for example, without being restricted to these examples, nitrogen-gas drying, vacuum-oven drying, lyophilisation, washing with organic solvents and subsequent air drying, liquid-bed drying, fluidised-bed drying, spray drying, roller drying, layer drying, air drying at room temperature and further methods.

The term "effective amount" denotes the amount of a medicament or of a pharmaceutical active compound which causes in a tissue, system, animal or human a biological or medical response which is sought or desired, for example, by a researcher or physician.

In addition, the term "therapeutically effective amount" denotes an amount which, compared with a corresponding subject who has not received this amount, has the following consequence: improved treatment, healing, prevention or elimination of a disease, syndrome, disease state, complaint, disorder or prevention of side effects or also a reduction in the progress of a disease, complaint or disorder. The term "therapeutically effective amount" also encompasses the amounts which are effective for increasing normal physiological function.

On use of preparations or medicaments according to the invention, the compounds according to the invention and/or physiologically acceptable salts and solvates thereof are generally used analogously to known, commercially available preparations or preparations, preferably in dosages of between 0.1 and 500 mg, in particular 5 and 300 mg, per use unit. The daily dose is preferably between 0.001 and 250 mg/kg, in particular 0.01 and 100 mg/kg, of body weight. The preparation can be administered one or more times per day, for example two, three or four times per day. However, the individual dose for a patient depends on a large number of individual factors, such as, for example, on the efficacy of the particular compound used, on the age, body weight, general state of health, sex, nutrition, on the time and method of administration, on the excretion rate, on the combination with other medicaments and on the severity and duration of the particular disease.

A measure of the uptake of a medicament active compound in an organism is its bioavailability. If the medicament active compound is delivered to the organism intravenously in the form of an injection solution, its absolute bioavailability, i.e. the proportion of the pharmaceutical which reaches the systemic blood, i.e. the major circulation, in unchanged form, is 100%. In the case of oral administration of a therapeutic active compound, the active compound is generally in the form of a solid in the formulation and must therefore first be dissolved in order that it is able to overcome the entry barriers, for example the gastrointestinal tract, the oral mucous membrane, nasal membranes or the skin, in particular the stratum corneum, or can be absorbed by the body. Data on the pharmacokinetics, i.e. on the bioavailability, can be obtained analogously to the method of J. Shaffer et al., J. Pharm. Sciences, 88 (1999), 313-318.

Furthermore, medicaments of this type can be prepared by means of one of the processes generally known in the pharmaceutical art.

Medicaments can be adapted for administration via any desired suitable route, for example by the oral (including buccal or sublingual), rectal, pulmonary, nasal, topical (including buccal, sublingual or transdermal), vaginal or parenteral (including subcutaneous, intramuscular, intravenous, intradermal and intra-articular routes. Medicaments of this type can be prepared by means of all processes known in the pharmaceutical art by, for example, combining the active compound with the excipient(s) or adjuvant(s).

The compounds according to the invention are also suitable for the preparation of medicaments to be administered parenterally having slow, sustained and/or controlled release of active compound. They are thus also suitable for the preparation of delayed-release formulations, which are advantageous for the patient since administration is only necessary at relatively large time intervals.

The medicaments include aqueous and non-aqueous sterile injection solutions comprising antioxidants, buffers, bacteriostatics and solutes, by means of which the formulation is rendered isotonic with the blood or synovial fluid of the recipient to be treated; as well as aqueous and non-aqueous sterile suspensions, which can comprise suspension media and thickeners. The formulations can be delivered in single-dose or multi-dose containers, for example sealed ampoules and vials, and stored in the freeze-dried (lyophilised) state, so that only the addition of the sterile carrier liquid, for example water for injection purposes, immediately before use is necessary. Injection solutions and suspensions prepared in accordance with the formulation can be prepared from sterile powders, granules and tablets.

The compounds according to the invention can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds according to the invention can also be coupled to soluble polymers as targeted medicament excipients. Such polymers can encompass polyvinylpyrrolidone, pyran copolymer, polyhydroxypropylmethacrylamidophenol, polyhydroxyethylaspartamidophenol or polyethylene oxide polylysine, substituted by palmitoyl radicals. The compounds according to the invention can furthermore be coupled to a class of biodegradable polymers which are suitable for achieving slow release of a medicament, for example polylactic acid, poly-epsilon-caprolactone, polyhydroxybutyric acid, polyorthoesters, polyacetals, polydihydroxypyrans, polycyanoacrylates, polylactic-co-glycolic acid, polymers, such as conjugates between dextran and methacrylates, polyphosphoesters, various polysaccharides and polyamines and poly-ε-caprolactone, albumin, chitosan, collagen or modified gelatine and crosslinked or amphipathic block copolymers of hydrogels.

Suitable for enteral administration (oral or rectal) are, in particular, tablets, dragees, capsules, syrups, juices, drops or suppositories, and suitable for topical use are ointments, creams, pastes, lotions, gels, sprays, foams, aerosols, solutions (for example solutions in alcohols, such as ethanol or isopropanol, acetonitrile, DMF, dimethylacetamide, 1,2-propanediol or mixtures thereof with one another and/or with water) or powders. Also particularly suitable for topical uses are liposomal preparations.

In the case of formulation to give an ointment, the active compound can be employed either with a paraffinic or a water-miscible cream base. Alternatively, the active compound can be formulated to a cream with an oil-in-water cream base or a water-in-oil base.

Medicaments adapted to transdermal administration can be delivered as independent plasters for extended, close contact with the epidermis of the recipient. Thus, for example, the active compound can be supplied from the plaster by means of iontophoresis, as described in general terms in Pharmaceutical Research, 3 (6), 318 (1986).

It goes without saying that, besides the constituents particularly mentioned above, the medicaments according to the invention may also comprise other agents usual in the art with respect to the particular type of pharmaceutical formulation.

The invention also relates to a set (or kit) comprising (or consisting of) separate packs of:
(a) a compound of formula (I) or a pharmaceutically acceptable salt or solvates thereof; and (b) a further therapeutic agent.

In particular, the present invention relates to a set (or kit) comprising (or consisting of) separate packs of:
(a) an effective amount of a compound of the formula (I) and/or pharmaceutically acceptable salts, solvates and stereoisomers thereof, including mixtures thereof in all ratios; and
(b) an effective amount of a further medicament active compound (or further therapeutic agent).

The set comprises suitable containers, such as boxes or cartons, individual bottles, bags or ampoules. The set may, for example, comprise separate ampoules each containing an effective amount of a compound of the formula I and/or solvates pharmaceutically acceptable salts, and stereoisomers thereof, including mixtures thereof in all ratios, and an effective amount of a further medicament active compound in dissolved or lyophilised form.

Furthermore, the medicaments according to the invention can be used in order to provide additive or synergistic effects in certain known therapies and/or can be used in order to restore the efficacy of certain existing therapies.

Besides the compounds according to the invention, the pharmaceutical preparations according to the invention may also comprise further medicament active compounds, for example for use in the treatment of cancer, other anti-tumor medicaments. For the treatment of the other diseases mentioned, the pharmaceutical preparations according to the invention may also, besides the compounds according to the invention, comprise further medicament active compounds which are known to the person skilled in the art in the treatment thereof. The references to methods of treatment in this description are to be interpreted as references to the compounds, pharmaceutical compositions and medicaments of the present invention for use in a method for the treatment of the human (or animal) body by therapy (or for diagnosis).

In one principal embodiment, methods are provided for enhancing an immune response in a host in need thereof. The immune response can be enhanced by reducing T cell tolerance, including by increasing IFN-γ release, by decreasing regulatory T cell production or activation, or by increasing antigen-specific memory T cell production in a host. In one embodiment, the method comprises administering a compound of the present invention to a host in combination or alternation with an antibody. In particular subembodiments, the antibody is a therapeutic antibody. In one particular embodiment, a method of enhancing efficacy of passive antibody therapy is provided comprising administering a compound of the present invention in combination or alternation with one or more passive antibodies. This method can enhance the efficacy of antibody therapy for treatment of abnormal cell proliferative disorders such as cancer or can enhance the efficacy of therapy in the treatment or prevention of infectious diseases. The compound of the present invention can be administered in combination or alternation with antibodies such as rituximab, herceptin or erbitux, for example.

In another principal embodiment, a method of treating or preventing abnormal cell proliferation is provided comprising administering a compound of the present invention to a host in need thereof substantially in the absence of another anti-cancer agent.

In another principal embodiment, a method of treating or preventing abnormal cell proliferation in a host in need thereof is provided, comprising administering a first a compound of the present invention substantially in combination with a first anti-cancer agent to the host and subsequently administering a second A_{2A} and/or A_{2B} receptor antagonist. In one subembodiment, the second antagonist is administered substantially in the absence of another anti-cancer agent. In another principal embodiment, a method of treating or preventing abnormal cell proliferation in a host in need thereof is provided, comprising administering a compound of the present invention substantially in combination with a first anti-cancer agent to the host and subsequently administering a second anti-cancer agent in the absence of the antagonist.

Thus, the cancer treatment disclosed here can be carried out as therapy with a compound of the present invention or in combination with an operation, irradiation or chemotherapy. Chemotherapy of this type can include the use of one or more active compounds of the following categories of antitumour active compounds:
(i) antiproliferative/antineoplastic/DNA-damaging active compounds and combinations thereof, as used in medical oncology, such as alkylating active compounds (for example cis-platin, parboplatin, cyclophosphamide, nitrogen mustard, melphalan, chlorambucil, busulphan and nitrosoureas); antimetabolites (for example antifolates such as fluoropyrimidines such as 5-fluorouracil and tegafur, raltitrexed, methotrexate, cytosine arabinoside, hydroxyurea and gemcitabine); antitumour antibiotics (for example anthracyclines, such as adriamycin, bleomycin, doxorubicin, daunomycin, epirubicin, idarubicin, mitomycin-C, dactinomycin and mithramycin) ; antimitotic active compounds (for example vinca alkaloids, such as vincristine, vinblastine, vindesine and vinorelbine, and taxoids, such as taxol and taxotere) ; topoisomerase inhibitors (for example epipodophyllotoxins, such as etoposide and teniposide, amsacrine, topotecan, irinotecan and camptothecin) and cell-differentiating active compounds (for example all-trans-retinoic acid, 13-cis-retinoic acid and fenretinide);
(ii) cytostatic active compounds, such as anti-oestrogens (for example tamoxifen, toremifene, raloxifene, droloxifene and iodoxyfene), oestrogen receptor regulators (for example fulvestrant), anti-androgens (for example bicalutamide, flutamide, nilutamide and cyproterone acetate), LHRH antagonists or LHRH agonists (for example goserelin, leuprorelin and buserelin), progesterones (for example megestrol acetate), aromatase inhibitors (for example anastrozole, letrozole, vorazole and exemestane) and inhibitors of 5α-reductase, such as finasteride;
(iii) active compounds which inhibit cancer invasion including for example metallo-proteinase inhibitors, like marimastat, and inhibitors of urokinase plasminogen activator receptor function;
(iv) inhibitors of growth factor function, for example growth factor antibodies, growth factor receptor antibodies, for example the anti-erbb2 antibody trastuzumab [Herceptin^{™}] and the anti-erbbl antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors, such as N-(3-chloro-4-fluorophenyl)-7-methoxy-6- (3-morpholinopropoxy) quinazolin-4-amine (gefitinib, AZD1839), N-(3-ethynylphenyl)-6,7-bis (2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (Cl 1033), for example inhibitors of the platelet-derived growth factor family and, for example, inhibitors of the hepatocyte growth factor family;
(v) anti-angiogenic active compounds, such as bevacizumab, angiostatin, endostatin, linomide, batimastat, captopril, cartilage derived inhibitor, genistein, interleukin 12, lavendustin, medroxypregesterone acetate, recombinant human platelet factor 4, tecogalan, thrombospondin, TNP-470, anti-VEGF monoclonal antibody, soluble VEGF-receptor chimaeric protein, anti-VEGF receptor antibodies, anti-PDGF receptors, inhibitors of integrins, tyrosine kinase inhibitors, serine/threonine kinase inhibitors, antisense oligonucleotides, antisense oligodexoynucleotides, siRNAs, anti-VEGF aptamers, pigment epithelium derived factor and compounds which have been published in the international patent applications WO 97/22596, WO 97/30035, WO 97/32856 and WO 98/13354);
(vi) vessel-destroying agents, such as combretastatin A4 and compounds which have been published in the international patent applications WO 99/02166, WO 00/40529, WO 00/41669, WO 01/92224, WO 02/04434 and WO 02/08213;
(vii) antisense therapies, for example those directed to the targets mentioned above, such as ISIS 2503, an anti-Ras antisense;
(viii) gene therapy approaches, including, for example, approaches for replacement of abnormal, modified genes, such as abnormal p53 or abnormal BRCA1 or BRCA2, GDEPT approaches (gene-directed enzyme pro-drug therapy), such as those which use cytosine deaminase, thymidine kinase or a bacterial nitroreductase enzyme, and approaches which increase the tolerance of a patient to chemotherapy or radiotherapy, such as multi-drug resistance therapy;
(ix) immunotherapy approaches, including, for example, ex-vivo and in-vivo approaches for increasing the immunogenicity of tumour cells of a patient, such as transfection with cytokines, such as interleukin 2, interleukin 4 or granulocyte macrophage colony stimulating factor, approaches for decreasing T-cell anergy, approaches using transfected immune cells, such as cytokine-transfected dendritic cells, approaches for use of cytokine-transfected tumour cells and approaches for use of anti-idiotypic antibodies; and
(x) chemotherapeutic agents including for example abarelix, aldesleukin, alemtuzumab, alitretinoin, allopurinol, altretamine, amifostine, anastrozole, arsenic trioxide, asparaginase, BCG live, bevaceizumab, bexarotene, bleomycin, bortezomib, busulfan, calusterone, camptothecin, capecitabine, carboplatin, carmustine, celecoxib, cetuximab, chlorambucil, cinacalcet, cisplatin, cladribine, cyclophosphamide, cytarabine, dacarbazine, dactinomycin, darbepoetin alfa, daunorubicin, denileukin diftitox, dexrazoxane, docetaxel, doxorubicin, dromostanolone, epirubicin, epoetin alfa, estramustine, etoposide, exemestane, filgrastim, floxuridine, fludarabine, fluorouracil, fulvestrant and gemcitabine.

Furthermore, the compound of formula (I) or the pharmaceutically acceptable salt, or solvate thereof, or the pharmaceutical composition comprising the compound of formula (I) or a pharmaceutically acceptable salt, or solvate thereof, can be administered in monotherapy (e.g., without concomitantly administering any further therapeutic agents, or without concomitantly administering any further therapeutic agents against the same disease/disorder that is to be treated or prevented with the compound of formula (I)).

However, as has been explained above, the compound of formula (I) or the pharmaceutically acceptable salt, or solvate thereof, or the pharmaceutical composition comprising the compound of formula (I) or a pharmaceutically acceptable salt, or solvate thereof, can also be administered in combination with one or more further therapeutic agents. If the compound of formula (I) or the pharmaceutically acceptable salt, or solvate thereof is used in combination with a second therapeutic agent active against the same disease/disorder, the dose of each compound may differ from that when the corresponding compound is used alone in particular a lower dose of each compound may be used. The combination of the compound of formula (I) or the pharmaceutically acceptable salt, or solvate thereof with one or more further therapeutic agents may comprise the simultaneous/concomitant administration of the compound of formula (I) or the pharmaceutically acceptable salt, or solvate thereof and the further therapeutic agent(s) (either in a single pharmaceutical formulation or in separate pharmaceutical formulations), or the sequential/separate administration of the compound of formula (I) or the pharmaceutically acceptable salt, or solvate thereof and the further therapeutic agent(s). If administration is sequential, either the compound of formula (I) or the pharmaceutically acceptable salt, or solvate thereof according to the present invention, or the one or more further therapeutic agents may be administered first. If administration is simultaneous, the one or more further therapeutic agents may be included in the same pharmaceutical formulation as the compound of formula (I) or the pharmaceutically acceptable salt, or solvate thereof, or they may be administered in two or more different (separate) pharmaceutical formulations. The further therapeutic agent(s) may be, for example, selected from any one of the corresponding exemplary compounds described herein above, including any of the compounds listed in table 1.

The subject or patient to be treated in accordance with the present invention may be an animal (e.g., a non-human animal). Preferably, the subject/patient is a mammal. More preferably, the subject/patient is a human (e.g., a male human or a female human) or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient to be treated in accordance with the invention is a human.

The term "treatment" of a condition, disorder or disease, as used herein, is well known in the art. "Treatment" of a condition, disorder or disease implies that a condition, disorder or disease is suspected or has been diagnosed in a patient/subject. A patient/subject suspected of suffering from a condition, disorder or disease typically shows specific clinical and/or pathological symptoms which a skilled person can easily attribute to a specific pathological condition (i.e., diagnose a condition, disorder or disease).

The "treatment" of a condition, disorder or disease may, for example, lead to a halt in the progression of the condition, disorder or disease (e.g., no deterioration of symptoms) or a delay in the progression of the condition, disorder or disease (in case the halt in progression is of a transient nature only). The "treatment" of a condition, disorder or disease may also lead to a partial response (e.g., amelioration of symptoms) or complete response (e.g., disappearance of symptoms) of the subject/patient suffering from the condition, disorder or disease. Accordingly, the "treatment" of a condition, disorder or disease may also refer to an amelioration of the condition, disorder or disease, which may, e.g., lead to a halt in the progression of the condition, disorder or disease or a delay in the progression of the condition, disorder or disease. Such a partial or complete response may be followed by a relapse. It is to be understood that a subject/patient may experience a broad range of responses to a treatment (such as the exemplary responses as described herein above). The treatment of a condition, disorder or disease may, *inter alia*, comprise curative treatment (preferably leading to a complete response and eventually to healing of the condition, disorder or disease) and palliative treatment (including symptomatic relief).

The term "prevention" of a condition, disorder or disease, as used herein, is also well known in the art. For example, a patient/subject suspected of being prone to suffer from a condition, disorder or disease may particularly benefit from a prevention of the condition, disorder or disease. The subject/patient may have a susceptibility or predisposition for a condition, disorder or disease, including but not limited to hereditary predisposition. Such a predisposition can be determined by standard methods or assays, using, e.g., genetic markers or phenotypic indicators. It is to be understood that a condition, disorder or disease to be prevented in accordance with the present invention has not been diagnosed or cannot be diagnosed in the patient/subject (for example, the patient/subject does not show any clinical or pathological symptoms). Thus, the term "prevention" comprises the use of a compound of the present invention before any clinical and/or pathological symptoms are diagnosed or determined or can be diagnosed or determined by the attending physician.

The present invention furthermore relates to the use of a compound of formula (I) or a pharmaceutically acceptable salt, or solvate thereof as an adenosine A_{2A} and/or A_{2B} receptor antagonist (particularly as a dual adenosine A_{2A} and A_{2B} receptor antagonist) in research, particularly as a research tool compound for inhibiting/ antagonizing the adenosine A_{2A} and/or A_{2B} receptor. Accordingly, the invention refers to the *in vitro* use of a compound of formula (I) or a pharmaceutically acceptable salt, or solvate thereof as an adenosine A_{2A} and/or A_{2B} receptor antagonist (particularly as a dual adenosine A_{2A} and A_{2B} receptor antagonist) and, in particular, to the *in vitro* use of a compound of formula (I) or a pharmaceutically acceptable salt, or solvate thereof as a research tool compound acting as an adenosine A_{2A} and/or A_{2B} receptor antagonist (particularly as a dual adenosine A_{2A} and A_{2B} receptor antagonist). The invention likewise relates to a method, particularly an *in vitro* method, of inhibiting the adenosine A_{2A} and/or A_{2B} receptor, the method comprising the application of a compound of formula (I) or a pharmaceutically acceptable salt, or solvate thereof. The invention further relates to a method of inhibiting the adenosine A_{2A} and/or A_{2B} receptor, the method comprising applying a compound of formula (I) or a pharmaceutically acceptable salt, or solvate thereof to a test sample (e.g., a biological sample) or a test animal (i.e., a non-human test animal).

The invention also refers to a method, particularly an *in vitro* method, of inhibiting the adenosine A_{2A} and/or A_{2B} receptor in a sample (e.g., a biological sample), the method comprising applying a compound of formula (I) or a pharmaceutically acceptable salt, or solvate thereof to said sample. The present invention further provides a method of inhibiting the adenosine A_{2A} and/or A_{2B} receptor, the method comprising contacting a test sample (e.g., a biological sample) or a test animal (i.e., a non-human test animal) with a compound of formula (I) or a pharmaceutically acceptable salt, or solvate thereof. The terms "sample", "test sample" and "biological sample" include, without being limited thereto: a cell, a cell culture or a cellular or subcellular extract; biopsied material obtained from an animal (e.g., a human), or an extract thereof; or blood, serum, plasma, saliva, urine, feces, or any other body fluid, or an extract thereof. It is to be understood that the term "*in vitro*" is used in this specific context in the sense of "outside a living human or animal body", which includes, in particular, experiments performed with cells, cellular or subcellular extracts, and/or biological molecules in an artificial environment such as an aqueous solution or a culture medium which may be provided, e.g., in a flask, a test tube, a Petri dish, a microtiter plate, etc.

It is to be understood that the present invention specifically relates to each and every combination of features and embodiments described herein, including any combination of general and/or preferred features/embodiments. In particular, the invention specifically relates to each combination of meanings (including general and/or preferred meanings) for the various groups and variables comprised in formula (I).

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

The invention will now be described by reference to the following examples which are merely illustrative and are not to be construed as a limitation of the scope of the present invention.

### Examples

The compounds described in this section are defined by their chemical formulae and their corresponding chemical names. In case of conflict between any chemical formula and the corresponding chemical name indicated herein, the present invention relates to both the compound defined by the chemical formula and the compound defined by the chemical name, and particularly relates to the compound defined by the chemical formula.

Unless indicated otherwise, per cent data denote per cent by weight. All temperatures are indicated in degrees Celsius. "Conventional work-up": water is added if necessary, the pH is adjusted, if necessary, to values between 2 and 10, depending on the constitution of the end product, the mixture is extracted with ethyl acetate or dichloromethane, the phases are separated, the organic phase is dried over sodium sulfate, filtered and evaporated, and the product is purified by chromatography on silica gel and/or by crystallisation.

Rf values on silica gel; mass spectrometry: El (electron impact ionisation): M⁺, FAB (fast atom bombardment): (M+H)⁺, THF (tetrahydrofuran), NMP (N-methlpyrrolidone), DMSO (dimethyl sulfoxide), EA (ethyl acetate), MeOH (methanol), TLC (thin-layer chromatography)

### List of Abbreviations

- AcOEt: Etyl Acetate
- DCM: Dichloromethane
- DMA: Dimethylacetamide
- DMF: Dimethylformamide
- DMSO: Dimethyl sulfoxide
- iv: Intravenous
- LC-MS: Liquid Chromatography coupled to Mass Spectrometry
- MeOH: Methanol
- NC: Not calculated
- ND: Not determined
- NMR: Nuclear Magnetic Resonance
- PEG: Polyethylene glycol
- po: Per os (oral)
- UPLC: Ultra performance liquid chromatography
- v/v: Volume to volume

### Example 1: Examples of compounds of the present invention

The present invention especially relates to the compounds of table 2 and pharmaceutically/physiologically acceptable salts, solvates and prodrugs thereof (including also stereoisomers thereof, as well as mixtures thereof in all ratios).

**Table 2 - examples of compounds of the present invention**

| **No.** | **Structure** | **IUPAC-Name** | **MW** |
|---|---|---|---|
| 1 | 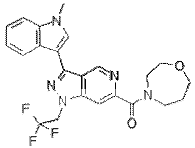 | [3-(1-Methyl-1H-indol-3-yl)-1-(2,2,2-trifluoro-ethyl)-1H-pyrazolo[4,3-c]pyridine-6-yl]-1,4-oxazepan-4-yl-methanone | 457.45 |
| 2 | 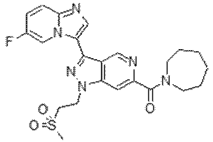 | Azepan-1-yl-[3-(6-fluoro-imidazo[1,2-a]pyridin-3-yl)-1-(2-methanesulfonyl-ethyl)-1H-pyrazolo[4,3-c]pyridin-6-yl]-methanone | 484.55 |
| 3 | 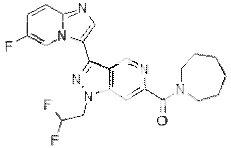 | Azepan-1-yl-[1-(2,2-difluoro-ethyl)-3-(6-fluoro-imidazo[1,2-a]pyridin-3-yl)-1H-pyrazolo[4,3-c]pyridin-6-yl]-methanone | 442.44 |
| 4 | 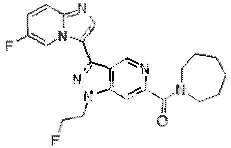 | Azepan-1-yl-[1-(2-fluoro-ethyl)-3-(6-fluoro-imidazo[1,2-a]pyridin-3-yl)-1H-pyrazolo[4,3-c]pyridin-6-yl]-methenone | 424.45 |
| 5 | 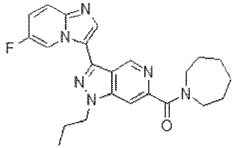 | Azepan-1-yl-[3-(6-fluoro-imidazo[1,2-a]pyridin-3-yl)-1-propyl-1H-pyrazolo[4,3-c]pyridin-6-yl]-methanone | 420.48 |
| 6 | 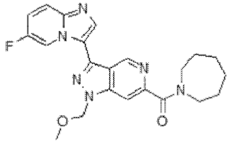 | Azepan-1-yl-[3-(6-fluoro-imidazo[1,2-a]pyridin-3-yl)-1-methoxymethyl-1H-pyrazolo[4,3-c]pyrid in-6-yl]-methanone | 422.46 |
| 7 | 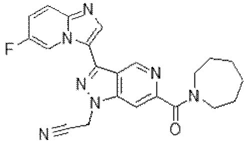 | [6-(Azepane-1-carbonyl)-3-(6-fluoro-imidazo[1,2-a]pyridin-3-yl)-pyrazolo[4,3-c]pyridin-1-yl]-acetonitrile | 417.44 |
| 8 | 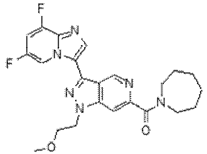 | Azepan-1-yl-[3-(6,8-difluoro-imidazo[1,2-a]pyridin-3-yl)-1-(2-methoxy-ethyl)-1H-pyrazolo[4,3-c]pyrid in-6-yl]-methanone | 454.47 |
| 9 | 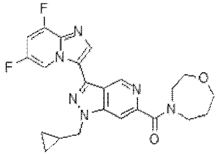 | [1-Cyclopropylmethyl-3-(6,8-difluoro-imidazo[1,2-a]pyridin-3-yl)-1H-pyrazolo[4,3-c]pyridin-6-yl]-1,4-oxazepan-4-yl-methanone | 452.46 |
| 10 | 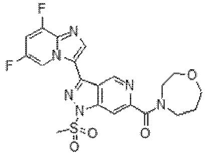 | [3-(6,8-Difluoro-imidazo[1,2-a]pyridin-3-yl)-1-methanesulfonyl-1H-pyrazolo[4,3-c]pyridin-6-yl]-1,4-oxazepan-4-yl-methanone | 476.46 |
| 11 | 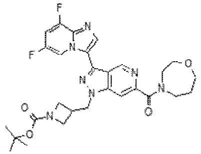 | 3-[3-(6,8-Difluoro-imidazo[1,2-a]pyridin-3-yl)-6-(1,4-oxazepane-4-carbonyl)-pyrazolo[4,3-c]pyridin-1-ylmethyl]-azetidine-1-carboxylic acid tert-butyl ester | 567.59 |
| 12 | 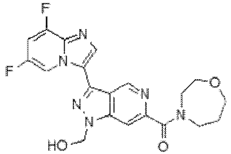 | [3-(6,8-Difluoro-imidazo[1,2-a]pyridin-3-yl)-1-hydroxymethyl-1H-pyrazolo[4,3-c]pyridin-6-yl]-1,4-oxazepan-4-yl-methanone | 428.39 |
| 13 | 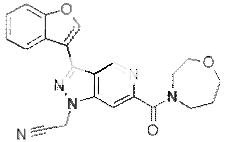 | [3-Benzofuran-3-yl-6-(1,4-oxazepane-4-carbonyl)-pyrazolo[4,3-c]pyridin-1-yl]-acetonitrile | 401.42 |
| 14 | 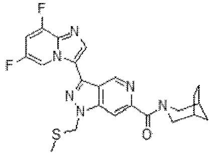 | (3-Aza-bicyclo[3.2.1]oct-3-yl)-[3-(6,8-difluoro-imidazo[1,2-a]pyridin-3-yl)-1-methylsulfanylmethyl-1H-pyrazolo[4,3-c]pyridin-6-yl]-methanone | 468.52 |
| 15 | 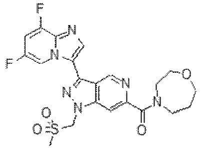 | [3-(6,8-Difluoro-imidazo[1,2-a]pyridin-3-yl)-1-methanesulfonylmethyl-1H-pyrazolo[4,3-c]pyridin-6-yl]-1,4-oxazepan-4-yl-methanone | 490.48 |
| 16 | 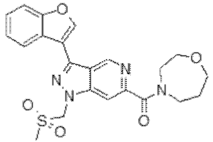 | (3-Benzofuran-3-yl-1-methanesulfonylmethyl-1H-pyrazolo[4,3-c]pyridine-6-yl)- 1,4-oxazepan-4-yl-methanone | 454.50 |
| 17 | 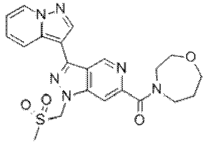 | (1-Methanesulfonylmethyl-3-pyrazolo[1,5-a]pyridin-3-yl-1H-pyrazolo[4,3-c]pyridin-6-yl)-1,4-oxazepan-4-yl-methanone | 454.50 |
| 18 | 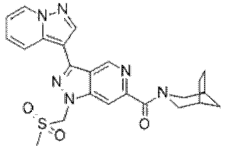 | (3-Aza-bicyclo[3.2.1]oct-3-yl)-(1-methanesulfonylmethyl-3-pyrazolo[1,5-a]pyridin-3-yl-1H-pyrazolo[4,3-c]pyridin-6-yl)-methanone | 464.54 |
| 19 | 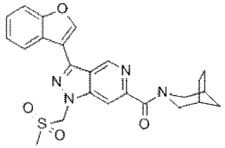 | (3-Aza-bicyclo[3.2.1]oct-3-yl)-(3-benzofuran-3-yl-1-methanesulfonylmethyl-1H-pyrazolo[4,3-c]pyridin-6-yl)-methanone | 464.54 |
| 20 | 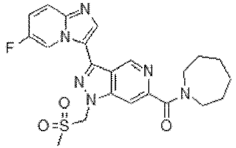 | Azepan-1-yl-[3-(6-fluoro-imidazo[1,2-a]pyridin-3-yl)-1-methanesulfonylmethyl-1H-pyrazolo[4,3-c]pyridin-6-yl]-methanone | 470.52 |
| 21 | 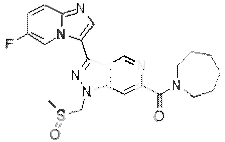 | Azepan-1-yl-[3-(6-fluoro-imidazo[1,2-a]pyridin-3-yl)-1-methanesulfinylmethyl-1H-pyrazolo[4,3-c]pyridin-6-yl]-methanone | 454.52 |
| 22 | 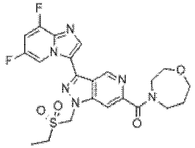 | [3-(6,8-Difluoro-imidazo[1,2-a]pyridin-3-yl)-1-ethanesulfonylmethyl-1H-pyrazolo[4,3-c]pyridin-6-yl]-1,4-oxazepan-4-yl-methanone | 504.51 |
| 23 | 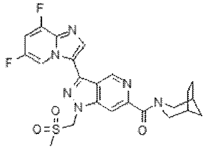 | (3-Aza-bicyclo[3.2.1]oct-3-yl)-[3-(6,8-difluoro-imidazo[1,2-a]pyridin-3-yl)-1-methanesulfonylmethyl-1H-pyrazolo[4,3-c]pyridin-6-yl]-methanone | 500.52 |
| 24 | 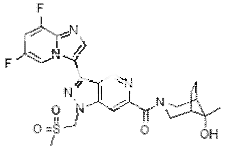 | [3-(6,8-Difluoro-imidazo[1,2-a]pyridin-3-yl)-1-methanesulfonylmethyl-1H-pyrazolo[4,3-c]pyridin-6-yl]-(8-endo-hydroxy-8-exo-methyl-3-aza-bicyclo[3.2.1]oct-3-yl)-methanone | 530.55 |
| 25 | 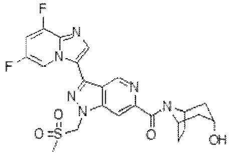 | [3-(6,8-Difluoro-imidazo[1,2-a]pyridin-3-yl)-1-methanesulfonylmethyl-1H-pyrazolo[4,3-c]pyridin-6-yl]-(3-endo-hydroxy-8-aza-bicyclo[3.2.1]oct-8-yl)-methanone | 516.52 |
| 26 | 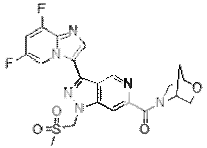 | [3-(6,8-Difluoro-imidazo[1,2-a]pyridin-3-yl)-1-methanesulfonylmethyl-1H-pyrazolo[4,3-c]pyridin-6-yl]-(2-oxa-5-aza-bicyclo[2.2.1]hept-5-yl)-methanone | 488.47 |
| 27 | 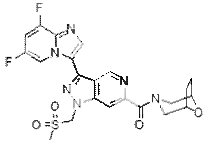 | [3-(6,8-Difluoro-imidazo[1,2-a]pyridin-3-yl)-1-methanesulfonylmethyl-1H-pyrazolo[4,3 c]pyridin-6-yl]-(8-oxa-3-aza-bicyclo[3.2.1]oct-3-yl)-methanone | 502.50 |
| 28 | 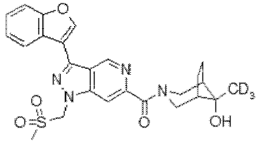 | (3-Benzofuran-3-yl-1-methanesulfonylmethyl-1H-pyrazolo[4,3-c]pyridin-6-yl)-(8-endo-hydroxy-8-exo-methyl-*d*₃-3-aza-bicyclo[3.2.1]oct-3-yl)-methanone | 497.58 |
| 29 | 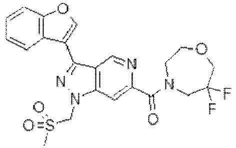 | (3-Benzofuran-3-yl-1-methanesulfonylmethyl-1H-pyrazolo[4,3-c]pyridin-6-yl)-(6,6-difluoro-1,4-oxazepan-4-yl)-methanone | 490.48 |
| 30 | 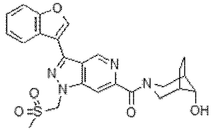 | (3-Benzofuran-3-yl-1-methanesulfonylmethyl-1H-pyrazolo[4,3-c]pyridin-6-yl)-(8-endo-hydroxy-3-aza-bicyclo[3.2.1]oct-3-yl)-methanone | 480.54 |
| 31 | 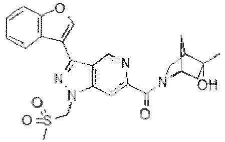 | (3-Benzofuran-3-yl-1-methanesulfonylmethyl-1H-pyrazolo[4,3-c]pyridin-6-yl)-(5-endo-hydroxy-5-exo-methyl-2-aza-bicyclo[2.2.1]hept-2-yl)-methanone | 480.54 |
| 32 | 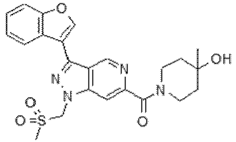 | (3-Benzofuran-3-yl-1-methanesulfonylmethyl-1H-pyrazolo[4,3-c]pyridin-6-yl)-(4-hydroxy-4-methyl-piperidin-1-yl)-methanone | 468.53 |
| 33 | 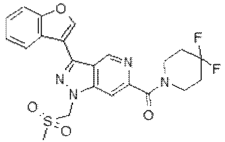 | (3-Benzofuran-3-yl-1-methanesulfonylmethyl-1H-pyrazolo[4,3-c]pyridin-6-yl)-(4,4-difluoro-piperidin-1-yl)-methanone | 474.48 |
| 34 | 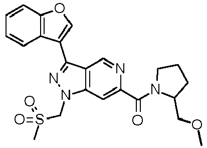 | (3-Benzofuran-3-yl-1-methanesulfonylmethyl-1H-pyrazolo[4,3-c]pyridin-6-yl)-(2-methoxymethyl-pyrrolidin-1-yl)-methanone | 468.53 |
| 35 | 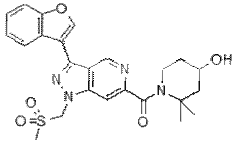 | (3-Benzofuran-3-yl-1-methanesulfonylmethyl-1H-pyrazolo[4,3-c]pyridin-6-yl)-(4-hydroxy-2,2 dimethyl-piperidin-1-yl)-methanone | 482.55 |
| 36 | 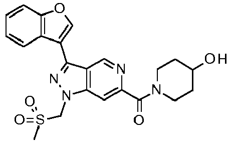 | (3-Benzofuran-3-yl-1-methanesulfonylmethyl-1H-pyrazolo[4,3-c]pyridin-6-yl)-(4-hydroxy-piperidin-1-yl)-methanone | 454.50 |
| 37 | 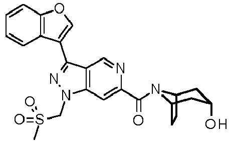 | [3-(benzofuran-3-yl)-1-(methylsulfonylmethyl)pyrazolo[4,3-c]pyridin-6-yl]-(3-endo-hydroxy-8-azabicyclo[3.2.1]octan-8-yl)methanone | 480.54 |
| 38 | 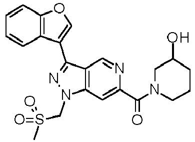 | (3-Benzofuran-3-yl-1-methanesulfonylmethyl-1H-pyrazolo[4,3-c]pyridin-6-yl)-(3-hydroxy-piperidin-1-yl)-methanone | 454.50 |
| 39 | 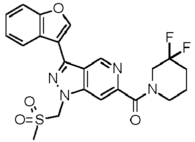 | (3-Benzofuran-3-yl-1-methanesulfonylmethyl-1H-pyrazolo[4,3-c]pyridin-6-yl)-(3,3-difluoro-piperidin-1-yl)-methanone | 474.48 |
| 40 | 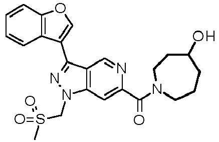 | (3-Benzofuran-3-yl-1-methanesulfonylmethyl-1H-pyrazolo[4,3-c]pyridin-6-yl)-(4-hydroxyazepan-1-yl)-methanone | 468.53 |
| 41 | 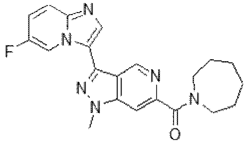 | Azepan-1-yl-[3-(6-fluoro-imidazo[1,2-a]pyridin-3-yl)-1-methyl-1H-pyrazolo[4,3-c]pyridin-6-yl]-methanone | 392.43 |
| 42 | 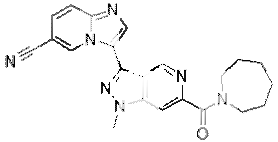 | 3-[6-(Azepane-1-carbonyl)-1-methyl-1H-pyrazolo[4,3-c]pyridin-3-yl]-imidazo[1,2-a]pyridine-6-carbonitrile | 399.45 |
| 43 | 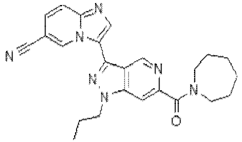 | 3-[6-(Azepane-1-carbonyl)-1-propyl-1H-pyrazolo[4,3-c]pyridin-3-yl]-imidazo[1,2-a]pyridine-6-carbonitrile | 427.50 |
| 44 | 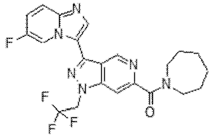 | Azepan-1-yl-[3-(6-fluoro-imidazo[1,2-a]pyridin-3-yl)-1-(2,2,2-trifluoro-ethyl)-1H-pyrazolo[4,3-c]pyrid in-6-yl]-methanone | 460.43 |
| 45 | 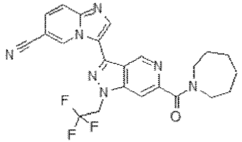 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carbonitrile | 467.45 |
| 46 | 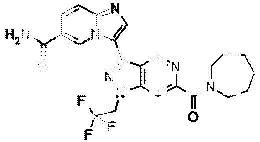 | 3-[6-(Azepane-1-carbonyl)-1-(2,2,2-trifluoro-ethyl)-1H-pyrazolo[4,3-c]-pyridin-3-yl]-imidazo[1,2-a]pyridine-6-carboxylic acid amide | 485.46 |
| 47 | 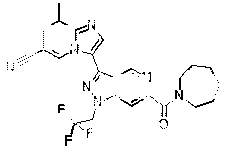 | 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carbonitrile | 481.47 |
| 48 | 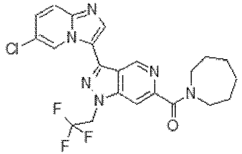 | Azepan-1-yl-[3-(6-chloro-imidazo[1,2-a]pyridin-3-yl)-1-(2,2,2-trifluoro-ethyl)-1H-pyrazolo[4,3-c]pyrid in-6-yl]-methanone | 476.88 |
| 49 | 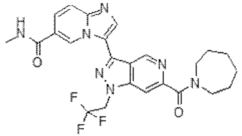 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carboxylic acid methylamide | 499.49 |
| 50 | 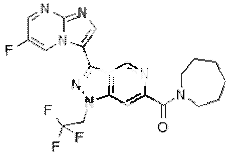 | Azepan-1-yl-[3-(6-fluoro-imidazo[1,2-a]pyrimidin-3-yl)-1-(2,2,2-trifluoro-ethyl)-1H-pyrazolo[4,3-c]pyridin-6-yl]-methanone | 461.42 |
| 51 | 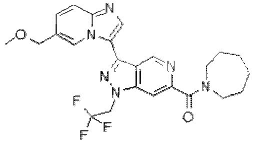 | Azepan-1-yl-[3-(6-methoxymethyl-imidazo[1,2-a]pyridin-3-yl)-1-(2,2,2-trifluoro-ethyl)-1H-pyrazolo[4,3-c]pyrid in-6-yl]-methanone | 486.49 |
| 52 | 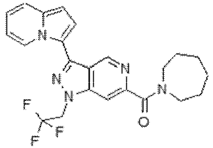 | Azepan-1-yl-[3-indolizin-3-yl-1-(2,2,2-trifluoro-ethyl)-1H-pyrazolo[4,3-c]pyridin-6-yl]-methanone | 441.45 |
| 53 | 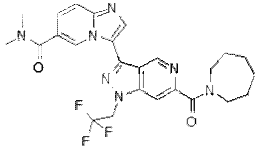 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carboxylic acid dimethylamide | 513.52 |
| 54 | 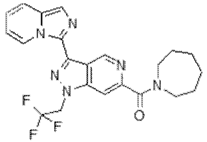 | Azepan-1-yl-[3-imidazo[1,5-a]pyridin-3-yl-1-(2,2,2-trifluoro-ethyl)-1H-pyrazolo[4,3-c]pyridin-6-yl]-methanone | 442.44 |
| 55 | 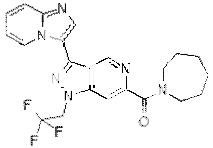 | Azepan-1-yl-[3-imidazo[1,2-a]pyridin-3-yl-1-(2,2,2-trifluoro-ethyl)-1H-pyrazolo[4,3-c]pyridin-6-yl]-methanone | 442.44 |
| 56 | 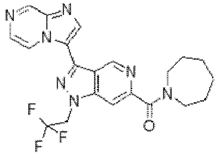 | Azepan-1 -yl-[3-imidazo[1,2-a]pyrazin-3-yl-1-(2,2,2-trifluoro-ethyl)-1H-pyrazolo[4,3-c]pyridin-6-yl]-methanone | 443.43 |
| 57 | 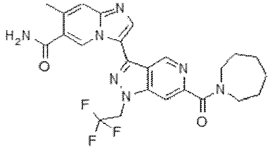 | 3-[6-(Azepane-1-carbonyl)-1-(2,2,2-trifluoro-ethyl)-1H-pyrazolo[4,3-c]pyridin-3-yl]-7-methyl-imidazo[1,2-a]pyridine-6-carboxylic acid amide | 499.49 |
| 58 | 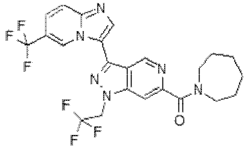 | Azepan-1-yl-[1-(2,2,2-trifluoro-ethyl)-3-(6-trifluoromethyl-imidazo[1,2-a]pyridin-3-yl)-1H-pyrazolo[4,3-c]pyridin-6-yl]-methanone | 510.44 |
| 59 | 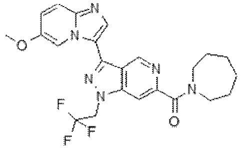 | Azepan-1-yl-[3-(6-methoxy-imidazo[1,2-a]pyridin-3-yl)-1-(2,2,2-trifluoro-ethyl)-1H-pyrazolo[4,3-c]pyrid in-6-yl]-methanone | 472.46 |
| 60 | 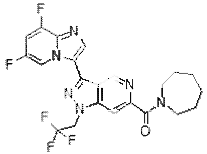 | Azepan-1-yl-[3-(6,8-difluoro-imidazo[1,2-a]pyridin-3-yl)-1-(2,2,2-trifluoro-ethyl)-1H-pyrazolo[4,3-c]pyrid in-6-yl]-methanone | 478.42 |
| 61 | 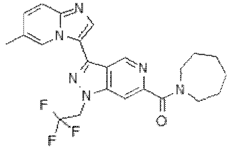 | Azepan-1-yl-[3-(6-methyl-imidazo[1,2-a]pyridin-3-yl)-1-(2,2,2-trifluoro-ethyl)-1H-pyrazolo[4,3-c]pyrid in-6-yl]-methanone | 456.46 |
| 62 | 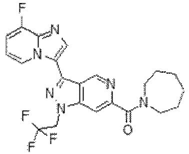 | Azepan-1-yl-[3-(8-fluoro-imidazo[1,2-a]pyridin-3-yl)-1-(2,2,2-trifluoro-ethyl)-1H-pyrazolo[4,3-c]pyrid in-6-yl]-methanone | 460.43 |
| 63 | 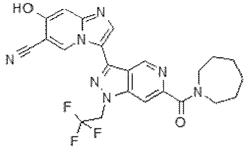 | 3-[6-(Azepane-1-carbonyl)-1-(2,2,2-trifluoro-ethyl)-1H-pyrazolo[4,3-c]pyridin-3-yl]-7-hydroxy-imidazo[1,2-a]pyridine-6-carbonitrile | 483.45 |
| 64 | 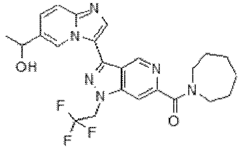 | Azepan-1-yl-[3-[6-(1-hydroxy-ethyl)-imidazo[1,2-a]pyridin-3-yl]-1-(2,2,2-trifluoro-ethyl)-1H-pyrazolo[4,3-c]pyridin-6-yl]-methanone | 486.49 |
| 65 | 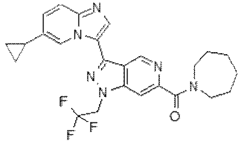 | Azepan-1-yl-[3-(6-cyclopropyl-imidazo[1,2-a]pyridin-3-yl)-1-(2,2,2-trifluoro-ethyl)-1H-pyrazolo[4,3-c]pyrid in-6-yl]-methanone | 482.50 |
| 66 | 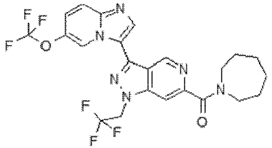 | Azepan-1-yl-[1-(2,2,2-trifluoro-ethyl)-3-(6-trifluoromethoxy-imidazo[1,2-a]pyridin-3-yl)-1H-pyrazolo[4,3-c]pyridin-6-yl]-methanone | 526.43 |
| 67 | 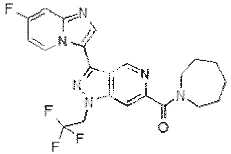 | Azepan-1-yl-[3-(7-fluoro-imidazo[1,2-a]pyridin-3-yl)-1-(2,2,2-trifluoro-ethyl)-1H-pyrazolo[4,3-c]pyrid in-6-yl]-methanone | 460.43 |
| 68 | 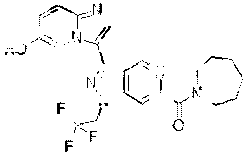 | Azepan-1-yl-[3-(6-hydroxy-imidazo[1,2-a]pyridin-3-yl)-1-(2,2,2-trifluoro-ethyl)-1H-pyrazolo[4,3-c]pyrid in-6-yl]-methanone | 458.44 |
| 69 | 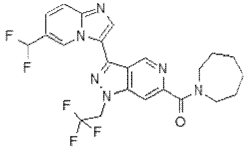 | Azepan-1-yl-[3-(6-difluoromethyl-imidazo[1,2-a]pyridin-3-yl)-1-(2,2,2-trifluoro-ethyl)-1H-pyrazolo[4,3-c]pyrid in-6-yl]-methanone | 492.44 |
| 70 | 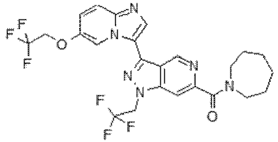 | Azepan-1-yl-[3-[6-(2,2,2-trifluoro-ethoxy)-imidazo[1,2-a]pyridin-3-yl]-1-(2,2,2-trifluoro-ethyl)-1H-pyrazolo[4,3-c]pyridin-6-yl]-methanone | 540.46 |
| 71 | 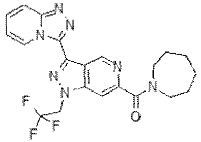 | Azepan-1-yl-[3-[1,2,4]triazolo[4,3-a]pyridin-3-yl-1-(2,2,2-trifluoro-ethyl)-1H-pyrazolo[4,3-c]pyridin-6-yl]-methanone | 443.43 |
| 72 | 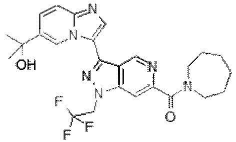 | Azepan-1-yl-[3-[6-(1-hydroxy-1-methyl-ethyl)-imidazo[1,2-a]pyridin-3-yl]-1-(2,2,2-trifluoro-ethyl)-1H-pyrazolo[4,3-c]pyridin-6-yl]-methanone | 500.52 |
| 73 | 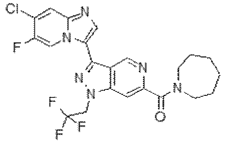 | Azepan-1-yl-[3-(7-chloro-6-fluoro-imidazo[1,2-a]pyridin-3-yl)-1-(2,2,2-trifluoro-ethyl)-1H-pyrazolo[4,3-c]pyrid in-6-yl]-methanone | 494.87 |
| 74 | 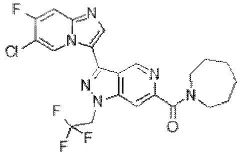 | Azepan-1-yl-[3-(6-chloro-7-fluoro-imidazo[1,2-a]pyridin-3-yl)-1-(2,2,2-trifluoro-ethyl)-1H-pyrazolo[4,3-c]pyrid in-6-yl]-methanone | 494.87 |
| 75 | 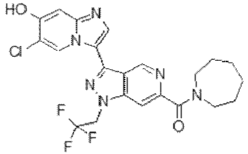 | Azepan-1-yl-[3-(6-chloro-7-hydroxy-imidazo[1,2-a]pyridin-3-yl)-1-(2,2,2-trifluoro-ethyl)-1H-pyrazolo[4,3-c]pyridin-6-yl]-methanone | 492.88 |
| 76 | 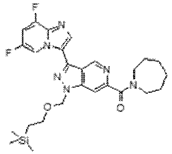 | Azepan-1-yl-[3-(6,8-difluoro-imidazo[1,2-a]pyridin-3-yl)-1-(2-trimethylsilanyl-ethoxymethyl)-1H-pyrazolo[4,3-c]pyridin-6-yl]-methanone | 526.65 |
| 77 | 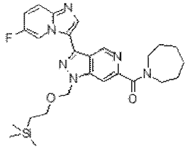 | Azepan-1-yl-[3-(6-fluoro-imidazo[1,2-a]pyridin-3-yl)-1-(2-trimethylsilanyl-ethoxymethyl)-1H-pyrazolo[4,3-c]pyridin-6-yl]-methanone | 508.66 |
| 78 | 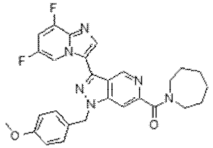 | Azepan-1-yl-[3-(6,8-difluoro-imidazo[1,2-a]pyridin-3-yl)-1-(4-methoxy-benzyl)-1H-pyrazolo[4,3-c]pyrid in-6-yl]-methanone | 516.54 |
| 79 | 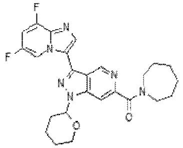 | Azepan-1-yl-[3-(6,8-difluoro-imidazo[1,2-a]pyridin-3-yl)-1-(tetrahydro-pyran-2-yl)-1H-pyrazolo[4,3-c]pyridin-6-yl]-methanone | 480.51 |
| 80 | 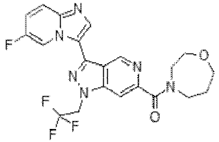 | [3-(6-Fluoro-imidazo[1,2-a]pyridin-3-yl)-1-(2,2,2-trifluoro-ethyl)-1H-pyrazolo[4,3-c]pyridin-6-yl]-1,4-oxazepan-4-yl-methanone | 462.40 |
| 81 | 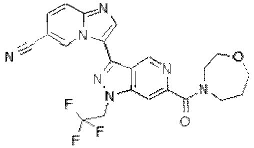 | 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carbonitrile | 469.42 |
| 82 | 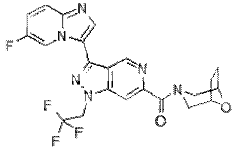 | [3-(6-Fluoro-imidazo[1,2-a]pyridin-3-yl)-1-(2,2,2-trifluoro-ethyl)-1H-pyrazolo[4,3-c]pyridin-6-yl]-(8-oxa-3-aza-bicyclo[3.2.1]oct-3-yl)-methanone | 474.41 |
| 83 | 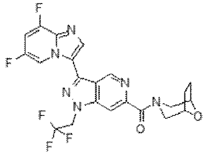 | [3-(6,8-Difluoro-imidazo[1,2-a]pyridin-3-yl)-1-(2,2,2-trifluoro-ethyl)-1H-pyrazolo[4,3c]pyridin-6-yl]-(8-oxa-3-aza-bicyclo[3.2.1]oct-3-yl)-methanone | 492.40 |
| 84 | 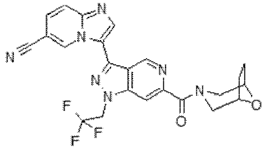 | 3-[6-(8-Oxa-3-aza-bicyclo[3.2.1]octane-3-carbonyl)-1-(2,2,2-trifluoro-ethyl)-1H-pyrazolo[4,3-c]pyridin-3-yl]-imidazo[1,2-a]pyridine-6-carbonitrile | 481.43 |
| 85 | 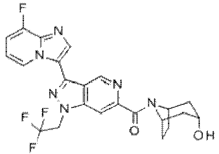 | [3-(8-fluoroimidazo[1,2-a]pyridin-3-yl)-1-(2,2,2-trifluoroethyl)pyrazolo[4,3-c]pyridin-6-yl]-(3-hydroxy-8-azabicyclo[3.2.1]octan-8-yl)methanone | 488.44 |
| 86 | 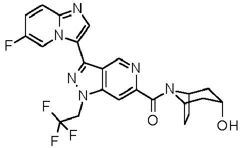 | [3-(6-fluoroimidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone | 488.44 |
| 87 | 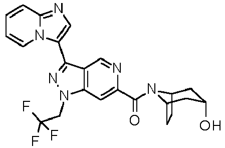 | [3-(imidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone | 470.45 |
| 88 | 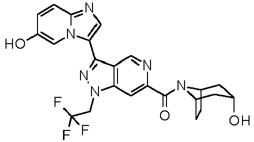 | (3-endo-hydroxy-8-azabicyclo[3.2.1]octan-8-yl)-[3-(6-hydroxyimidazo[1,2-a]pyridin-3-yl)-1-(2,2,2-trifluoroethyl)pyrazolo[4,3-c]pyrid in-6-yl]methanone | 486.45 |
| 89 | 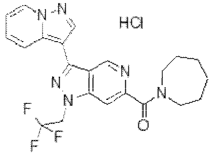 | Azepan-1 -yl-[3-pyrazolo[1,5-a]pyridin-3-yl-1-(2,2,2-trifluoro-ethyl)-1H-pyrazolo[4,3-c]pyridin-6-yl]-methanone hydrochoride | 442.44 |
| 90 | 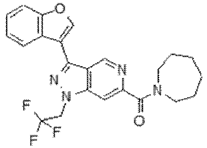 | Azepan-1-yl-[3-benzofuran-3-yl-1-(2,2,2-trifluoro-ethyl)-1H-pyrazolo[4,3-c]pyridin-6-yl]-methanone | 442.43 |
| 91 | 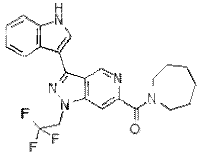 | Azepan-1-yl-[3-(1H-indol-3-yl)-1-(2,2,2-trifluoro-ethyl)-1H-pyrazolo[4,3-c]pyridin-6-yl]-methanone | 441.45 |
| 92 | 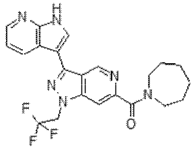 | Azepan-1-yl-[3-(1H-pyrrolo[2,3-b]pyridin-3-yl)-1-(2,2,2-trifluoro-ethyl)-1H-pyrazolo[4,3-c]pyridin-6-yl]-methanone | 442.44 |
| 93 | 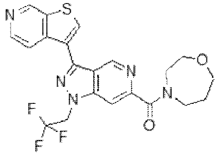 | 1,4-Oxazepan-4-yl-[3-thieno[2,3-c]pyridin-3-yl-1-(2,2,2-trifluoro-ethyl)-1H-pyrazolo[4,3-c]pyridin-6-yl]-methanone | 461.46 |
| 94 | 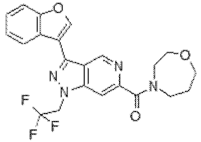 | [3-Benzofuran-3-yl-1-(2,2,2-trifluoro-ethyl)-1H-pyrazolo[4,3-c]pyridine-6-yl]-1,4-oxazepan-4-yl-methanone | 444.41 |
| 95 | 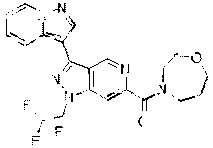 | 1,4-Oxazepan-4-yl-[3-pyrazolo[1,5-a]pyridine-3-yl-1-(2,2,2-trifluoro-ethyl)-1H-pyrazolo[4,3-c]pyridine-6-yl]-methanone | 444.41 |
| 96 | 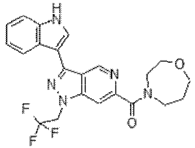 | [3-(1H-Indol-3-yl)-1-(2,2,2-trifluoro-ethyl)-1H-pyrazolo[4,3-c]pyridine-6-yl]-1,4-oxazepan-4-yl-methanone | 443.42 |
| 97 | 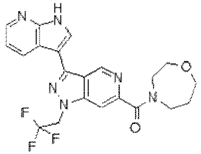 | 1,4-Oxazepan-4-yl-[3-(1H-pyrrolo[2,3-b]pyridin-3-yl)-1-(2,2,2-trifluoro-ethyl)-1H-pyrazolo[4,3-c]pyridine-6-yl]-methanone | 444.41 |
| 98 | 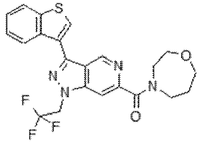 | [3-Benzo[b]thiophen-3-yl-1-(2,2,2-trifluoro-ethyl)-1H-pyrazolo[4,3-c]pyridine-6-yl]-1,4-oxazepan-4-yl-methanone | 460.47 |
| 99 | 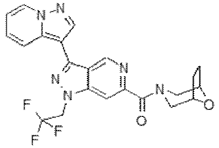 | (8-Oxa-3-aza-bicyclo[3.2.1]oct-3-yl)-[3-pyrazolo[1,5-a]pyridin-3-yl-1-(2,2,2-trifluoro-ethyl)-1H-pyrazolo[4,3-c]pyridin-6-yl]-methanone | 456.42 |
| 100 | 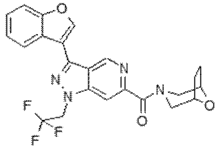 | [3-Benzofuran-3-yl-1-(2,2,2-trifluoro-ethyl)-1H-pyrazolo[4,3-c]pyridin-6-yl]-(8-oxa-3-aza-bicyclo[3.2.1]oct-3-yl)-methanone | 456.42 |
| 101 | 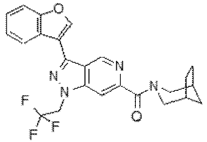 | (3-Aza-bicyclo[3.2.1]oct-3-yl)-[3-benzofuran-3-yl-1-(2,2,2-trifluoro-ethyl)-1H-pyrazolo[4,3-c]pyridin-6-yl]-methanone | 454.44 |
| 102 | 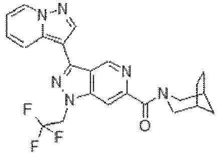 | (3-Aza-bicyclo[3.2.1]oct-3-yl)-[3-pyrazolo[1,5-a]pyridin-3-yl-1-(2,2,2-trifluoro-ethyl)-1H-pyrazolo[4,3-c]pyrid in-6-yl]-methanone | 454.45 |
| 103 | 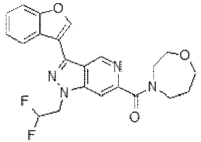 | [3-Benzofuran-3-yl-1-(2,2-difluoro-ethyl)-1H-pyrazolo[4,3-c]pyridin-6-yl]-1,4-oxazepan-4-yl-methanone | 426.42 |
| 104 | 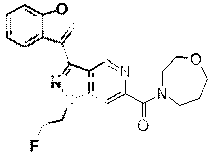 | [3-Benzofuran-3-yl-1-(2-fluoro-ethyl)-1H-pyrazolo[4,3-c]pyridin-6-yl]-1,4-oxazepan-4-yl-methanone | 408.43 |
| 105 | 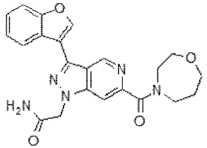 | 2-[3-Benzofuran-3-yl-6-(1,4-oxazepane-4-carbonyl)-pyrazolo[4,3-c]pyridin-1-yl]-acetamide | 419.43 |
| 106 | 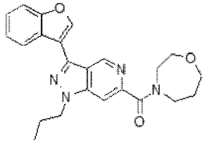 | (3-Benzofuran-3-yl-1-propyl-1H-pyrazolo[4,3-c]pyridin-6-yl)-1,4-oxazepan-4-yl-methanone | 404.46 |
| 107 | 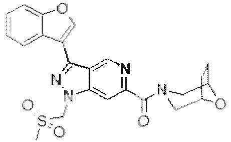 | (3-Benzofuran-3-yl-1-methanesulfonylmethyl-1H-pyrazolo[4,3-c]pyridine-6-yl)-(8-oxa-3-aza-bicyclo[3.2.1]oct-3-yl)-methanone | 466.51 |
| 108 | 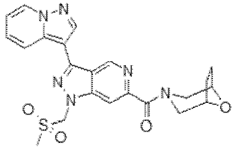 | (1-Methanesulfonylmethyl-3-pyrazolo[1,5-a]pyridine-3-yl-1H-pyrazolo[4,3-c]pyridine-6-yl)- (8-oxa-3-aza-bicyclo[3.2.1]oct-3-yl)-methanone | 466.51 |
| 109 | 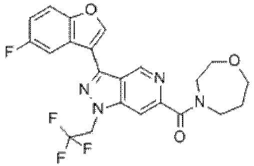 | [3-(5-fluorobenzofuran-3-yl)-1-(2,2,2-trifluoroethyl)pyrazolo[4,3-c]pyridin-6-yl]-(1,4-oxazepan-4-yl)methanone | 462.4 |
| 110 | 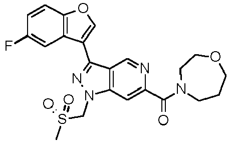 | [3-(5-fluorobenzofuran-3-yl)-1-(methylsulfonylmethyl)pyrazolo[4,3-c]pyridin-6-yl]-(1,4-oxazepan-4-yl)methanone | 472.49 |
| 111 | 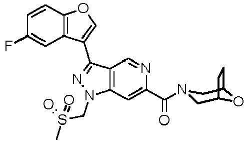 | [3-(5-fluorobenzofuran-3-yl)-1-(methylsulfonylmethyl)pyrazolo[4,3-c]pyridin-6-yl]-(8-oxa-3-azabicyclo[3.2.1]octan-3-yl)methanone | 484.50 |
| 112 | 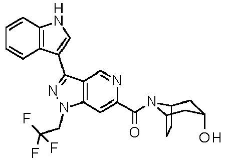 | (3-endo-hydroxy-8-azabicyclo[3.2.1]octan-8-yl)-[3-(1H-indol-3-yl)-1-(2,2,2-trifluoroethyl)pyrazolo[4,3-c]pyridin-6-yl]methanone | 469.46 |
| 113 | 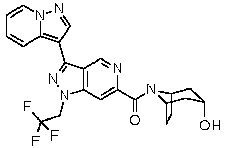 | (3-endo-hydroxy-8-azabicyclo[3.2.1]octan-8-yl)-[3-pyrazolo[1,5-a]pyridin-3-yl-1-(2,2,2-trifluoroethyl)pyrazolo[4,3-c]pyridin-6-yl]methanone | 470.45 |
| 114 | 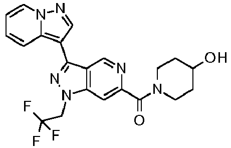 | (4-hydroxy-1-piperidyl)-[3-pyrazolo[1,5-a]pyridin-3-yl-1-(2,2,2-trifluoroethyl)pyrazolo[4,3-c]pyridin-6-yl]methanone | 444.41 |
| 115 | 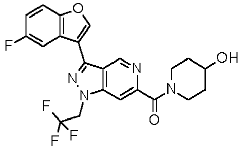 | [3-(5-fluorobenzofuran-3-yl)-1-(2,2,2-trifluoroethyl)pyrazolo[4,3-c]pyridin-6-yl]-(4-hydroxy-1-piperidyl)methanone | 462.40 |
| 116 | 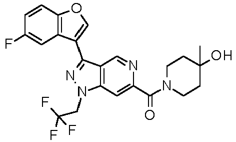 | [3-(5-fluorobenzofuran-3-yl)-1-(2,2,2-trifluoroethyl)pyrazolo[4,3-c]pyridin-6-yl]-(4-hydroxy-4-methyl-1-piperidyl)methanone | 476.42 |
| 117 | 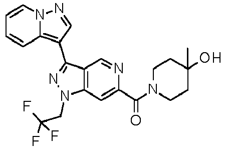 | (4-hydroxy-4-methyl-1-piperidyl)-[3-pyrazolo[1,5-a]pyridin-3-yl-1-(2,2,2-trifluoroethyl)pyrazolo[4,3-c]pyridin-6-yl]methanone | 458.44 |
| 118 | 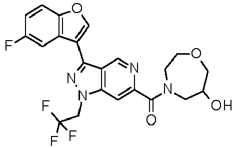 | [3-(5-fluorobenzofuran-3-yl)-1-(2,2,2-trifluoroethyl)pyrazolo[4,3-c]pyridin-6-yl]-(6-hydroxy-1,4-oxazepan-4-yl)methanone | 478.40 |
| 119 | 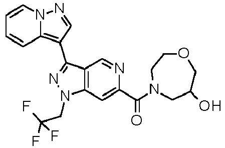 | (6-hydroxy-1,4-oxazepan-4-yl)-[3-pyrazolo[1,5-a]pyridin-3-yl-1-(2,2,2-trifluoroethyl)pyrazolo[4,3-c]pyridin-6-yl]methanone | 460.41 |
| 120 | 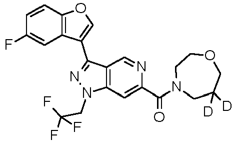 | [3-(5-fluorobenzofuran-3-yl)-1-(2,2,2-trifluoroethyl)pyrazolo[4,3-c]pyridin-6-yl]-(6,6-dideuterio-1,4-oxazepan-4-yl)methanone | 464.41 |
| 121 | 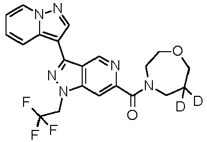 | (6,6-dideuterio-1,4-oxazepan-4-yl)-[3-pyrazolo[1,5-a]pyridin-3-yl-1-(2,2,2-trifluoroethyl)pyrazolo[4,3-c]pyrid in-6-yl]methanone | 446.42 |
| 122 | 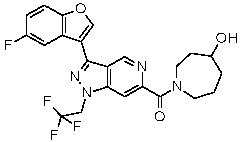 | [3-(5-fluorobenzofuran-3-yl)-1-(2,2,2-trifluoroethyl)pyrazolo[4,3-c]pyridin-6-yl]-(4-hydroxyazepan-1-yl)methanone | 476.42 |
| 123 | 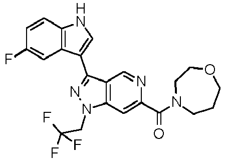 | [3-(5-fluoro-1H-indol-3-yl)-1-(2,2,2-trifluoroethyl)pyrazolo[4,3-c]pyridin-6-yl]-(1,4-oxazepan-4-yl)methanone | 461.41 |
| 124 | 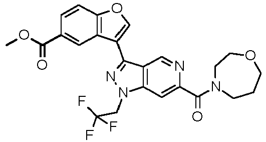 | methyl 3-[6-(1,4-oxazepane-4-carbonyl)-1-(2,2,2-trifluoroethyl)pyrazolo[4,3-c]pyridin-3-yl]benzofuran-5-carboxylate | 502.44 |
| 125 | 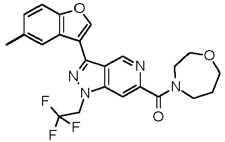 | [3-(5-methylbenzofuran-3-yl)-1-(2,2,2-trifluoroethyl)pyrazolo[4,3-c]pyridin-6-yl]-(1,4-oxazepan-4-yl)methanone | 458.43 |
| 126 | 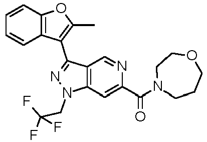 | [3-(2-methylbenzofuran-3-yl)-1-(2,2,2-trifluoroethyl)pyrazolo[4,3-c]pyridin-6-yl]-(1,4-oxazepan-4-yl)methanone | 458.43 |
| 127 | 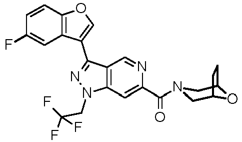 | [3-(5-fluorobenzofuran-3-yl)-1-(2,2,2-trifluoroethyl)pyrazolo[4,3-c]pyridin-6-yl]-(8-oxa-3-azabicyclo[3.2.1]octan-3-yl)methanone | 474.41 |
| 128 | 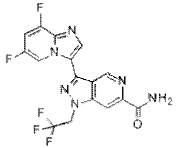 | 3-(6,8-Difluoro-imidazo[1,2-a]pyridin-3-yl)-1-(2,2,2-trifluoro-ethyl)-1H-pyrazolo[4,3-c]pyridine-6-carboxylic acid amide | 396.27 |
| 129 | 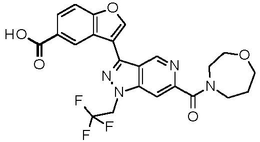 | 3-[6-(1,4-oxazepane-4-carbonyl)-1-(2,2,2-trifluoroethyl)pyrazolo[4,3-c]pyridin-3-yl]benzofuran-5-carboxylic acid | 488.42 |
| 130 | 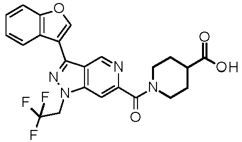 | 1-[3-(benzofuran-3-yl)-1-(2,2,2-trifluoroethyl)pyrazolo[4,3-c]pyridine-6-carbonyl]piperidine-4-carboxylic acid | 472.42 |
| 131 | 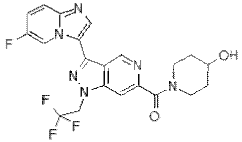 | [3-(6-Fluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone | 462.40 |
| 132 | 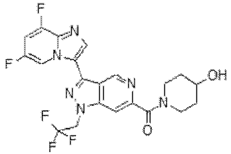 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone | 480.39 |
| 133 | 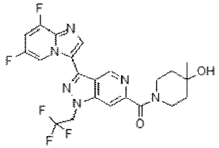 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-methyl-piperidin-1-yl)-methanone | 494.42 |
| 134 | 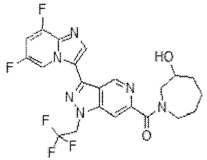 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxy-azepan-1-yl)-methanone | 494.42 |
| 135 | 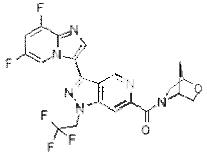 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[2.2.1]hept-5-yl)-methanone | 478.38 |
| 136 | 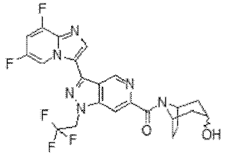 | [3-(6,8-Difluoro-[1,2,4]triazolo[4,3-a]pyridin-3-yl)-1-(2,2,2-trifluoro-ethyl)-1H-pyrazolo[4,3-c]pyridin-6-yl]-(3-hydroxy-8-aza-bicyclo[3.2.1]oct-8-yl)-methanone | 506.43 |
| 137 | 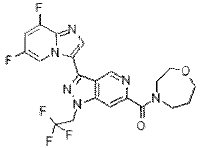 | [3-(6,8-Difluoro-[1,2,4]triazolo[4,3-a]pyridin-3-yl)-1-(2,2,2-trifluoro-ethyl)-1H-pyrazolo[4,3-c]pyridin-6-yl]-1,4-oxazepan-4-yl-methanone | 480.39 |
| 138 | 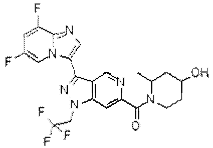 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-methyl-piperidin-1-yl)-methanone | 494.42 |
| 139 | 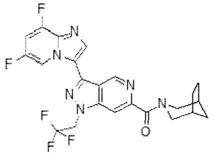 | (3-Aza-bicyclo[3.2.1]oct-3-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone | 490.43 |
| 140 | 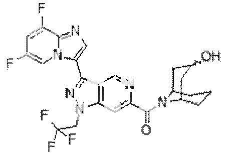 | [3-(6,8-Difluoro-imidazo[1,2-a]pyridin-3-yl)-1-(2,2,2-trifluoro-ethyl)-1H-pyrazolo[4,3-c]pyridin-6-yl]-(9-hydroxy-3-aza-bicyclo[3.3.1]non-3-yl)-methanone | 520.45 |
| 141 | 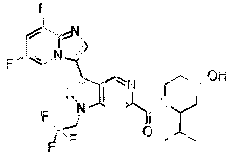 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-isopropyl-piperidin-1-yl)-methanone | 522.47 |
| 142 | 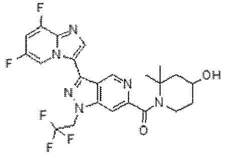 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2,2-dimethyl-piperidin-1-yl)-methanone | 508.44 |
| 143 | 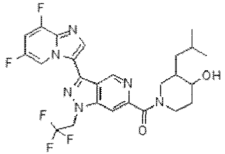 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-3-isobutyl-piperidin-1-yl)-methanone | 536.50 |
| 144 | 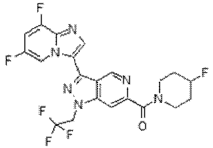 | [3-(6,8-Difluoro-imidazo[1,2-a]pyridin-3-yl)-1-(2,2,2-trifluoro-ethyl)-1H-pyrazolo[4,3-c]pyridin-6-yl]-(4-fluoro-piperidin-1-yl)-methanone | 482.38 |
| 145 | 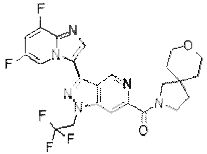 | [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-2-aza-spiro[4.5]dec-2-yl)-methanone | 520.45 |
| 146 | 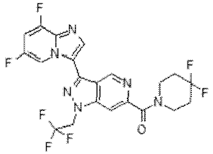 | [3-(6,8-Difluoro-imidazo[1,2-a]pyridin-3-yl)-1-(2,2,2-trifluoro-ethyl)-1H-pyrazolo[4,3-c]pyridin-6-yl]-(4,4-difluoro-piperidin-1-yl)-methanone | 500.37 |
| 147 | 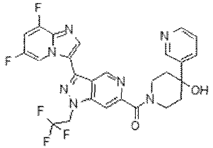 | [3-(6,8-Difluoro-imidazo[1,2-a]pyridin-3-yl)-1-(2,2,2-trifluoro-ethyl)-1H-pyrazolo[4,3-c]pyridin-6-yl]-(4'-hydroxy-3',4',5',6'-tetrahydro-2'H-[3,4']bipyridinyl-1'-yl)-methanone | 557.47 |
| 148 | 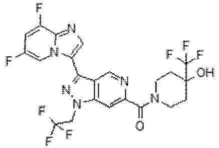 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-trifluoromethyl-piperidin-1-yl)-methanone | 548.39 |
| 149 | 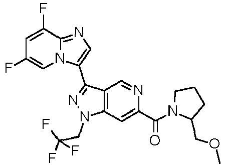 | [3-(6,8-Difluoro-imidazo[1,2-a]pyridin-3-yl)-1-(2,2,2-trifluoro-ethyl)-1H-pyrazolo[4,3-c]pyridin-6-yl]-(2-methoxymethyl-pyrrolidin-1-yl)-methanone | 494.42 |
| 150 | 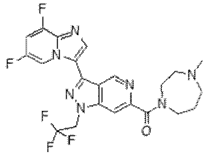 | [3-(6,8-Difluoro-imidazo[1,2-a]pyridin-3-yl)-1-(2,2,2-trifluoro-ethyl)-1H-pyrazolo[4,3-c]pyridin-6-yl]-(4-methyl-1,4-diazepan-1-yl)-methanone | 493.43 |
| 151 | 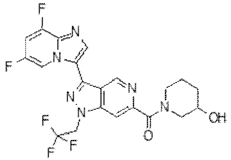 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxy-piperidin-1-yl)-methanone | 480.39 |
| 152 | 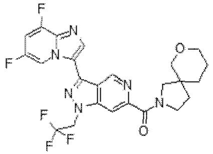 | [3-(6,8-Difluoro-imidazo[1,2-a]pyridin-3-yl)-1-(2,2,2-trifluoro-ethyl)-1H-pyrazolo[4,3-c]pyridin-6-yl]-(7-oxa-2-aza-spiro[4.5]dec-2-yl)-methanone | 520.45 |
| 153 | 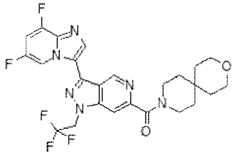 | [3-(6,8-Difluoro-imidazo[1,2-a]pyridin-3-yl)-1-(2,2,2-trifluoro-ethyl)-1H-pyrazolo[4,3-c]pyridin-6-yl]-(3-oxa-9-aza-spiro[5.5]undec-9-yl)-methanone | 534.48 |
| 154 | 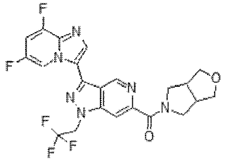 | [3-(6,8-Difluoro-imidazo[1,2-a]pyridin-3-yl)-1-(2,2,2-trifluoro-ethyl)-1H-pyrazolo[4,3-c]pyridin-6-yl]-(tetrahydro-furo[3,4-c]pyrrol-5-yl)-methanone | 492.40 |
| 155 | 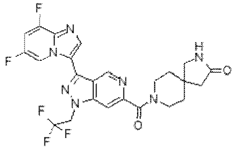 | 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-3-one | 533.45 |
| 156 | 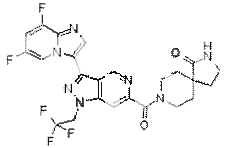 | 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-1 -one | 533.45 |
| 157 | 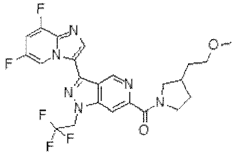 | [3-(6,8-Difluoro-imidazo[1,2-a]pyridin-3-yl)-1-(2,2,2-trifluoro-ethyl)-1H-pyrazolo[4,3-c]pyridin-6-yl]-[3-(2-methoxy-ethyl)-pyrrolidin-1-yl]-methanone | 508.44 |
| 158 | 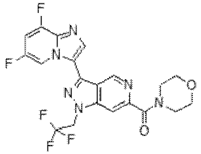 | [3-(6,8-Difluoro-imidazo[1,2-a]pyridin-3-yl)-1-(2,2,2-trifluoro-ethyl)-1H-pyrazolo[4,3-c]pyridin-6-yl]-morpholin-4-yl-methanone | 466.36 |
| 159 | 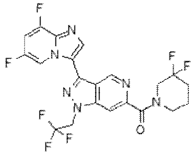 | [3-(6,8-Difluoro-imidazo[1,2-a]pyridin-3-yl)-1-(2,2,2-trifluoro-ethyl)-1H-pyrazolo[4,3-c]pyridin-6-yl]-(3,3-difluoro-piperidin-1-yl)-methanone | 500.37 |
| 160 | 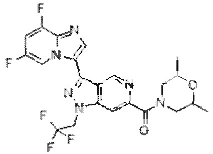 | [3-(6,8-Difluoro-imidazo[1,2-a]pyridin-3-yl)-1-(2,2,2-trifluoro-ethyl)-1H-pyrazolo[4,3-c]pyridin-6-yl]-(3,3-difluoro-piperidin-1-yl)-methanone | 494.42 |
| 161 | 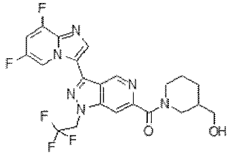 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxymethyl-piperidin-1-yl)-methanone | 494.42 |
| 162 | 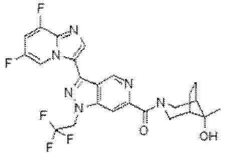 | [3-(6,8-Difluoro-imidazo[1,2-a]pyridin-3-yl)-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone | 520.45 |
| 163 | 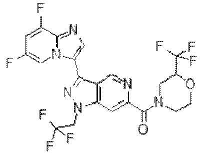 | [3-(6,8-Difluoro-imidazo[1,2-a]pyridin-3-yl)-1-(2,2,2-trifluoro-ethyl)-1H-pyrazolo[4,3-c]pyridin-6-yl]-(2-trifluoromethyl-morpholin-4-yl)-methanone | 534.36 |
| 164 | 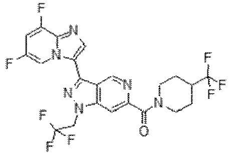 | [3-(6,8-Difluoro-imidazo[1,2-a]pyridin-3-yl)-1-(2,2,2-trifluoro-ethyl)-1H-pyrazolo[4,3-c]pyridin-6-yl]-(4-trifluoromethyl-piperidin-1-yl)-methanone | 532.39 |
| 165 | 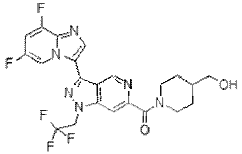 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-piperidin-1-yl)-methanone | 494.42 |
| 166 | 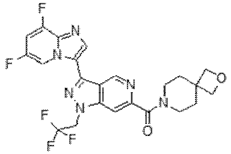 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-7-aza-spiro[3.5]non-7-yl)-methanone | 506.43 |
| 167 | 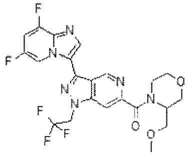 | [3-(6,8-Difluoro-imidazo[1,2-a]pyridin-3-yl)-1-(2,2,2-trifluoro-ethyl)-1H-pyrazolo[4,3-c]pyridin-6-yl]-(3-methoxymethyl-morpholin-4-yl)-methanone | 510.42 |
| 168 | 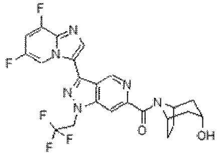 | [3-(6,8-Difluoro-imidazo[1,2-a]pyridin-3-yl)-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone | 506.43 |
| 169 | 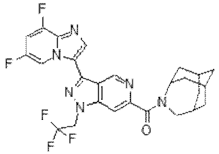 | (4-Aza-tricyclo[4.3.1.1*3,8*]undec-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone | 530.49 |
| 170 | 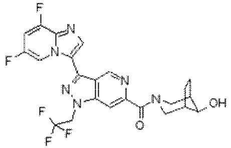 | [3-(6,8-Difluoro-imidazo[1,2-a]pyridin-3-yl)-1-(2,2,2-trifluoro-ethyl)-1H-pyrazolo[4,3-c]pyridin-6-yl]-(8-exo-hydroxy-3-aza-bicyclo[3.2.1]oct-3-yl)-methanone | 506.43 |
| 171 | 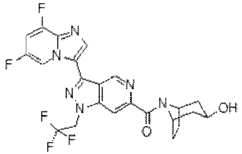 | [3-(6,8-Difluoro-imidazo[1,2-a]pyridin-3-yl)-1-(2,2,2-trifluoro-ethyl)-1H-pyrazolo[4,3-c]pyridin-6-yl]-(3-exo-hydroxy-8-aza-bicyclo[3.2.1]oct-8-yl)-methanone | 506.43 |
| 172 | 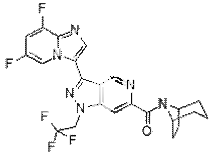 | (8-Aza-bicyclo[3.2.1]oct-8-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone | 490.43 |
| 173 | 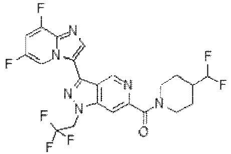 | [3-(6,8-Difluoro-imidazo[1,2-a]pyridin-3-yl)-1-(2,2,2-trifluoro-ethyl)-1H-pyrazolo[4,3-c]pyridin-6-yl]-(4-difluoromethyl-piperidin-1-yl)-methanone | 514.40 |
| 174 | 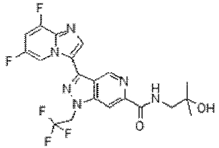 | 3-(6,8-Difluoro-imidazo[1,2-a]pyridin-3-yl)-1-(2,2,2-trifluoro-ethyl)-1H-pyrazolo[4,3-c]pyridine-6-carboxylic acid (2-hydroxy-2-methyl-propyl)-amide | 468.38 |
| 175 | 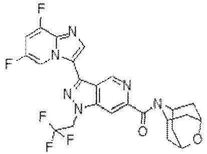 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-6-aza-tricyclo[3.3.1.1*3,7*]dec-6-yl)-methanone | 518.44 |
| 176 | 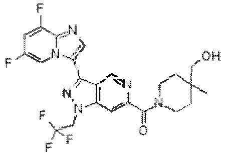 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-4-methyl-piperidin-1-yl)-methanone | 508.44 |
| 177 | 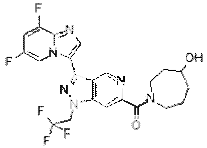 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-azepan-1-yl)-methanone | 494.42 |
| 178 | 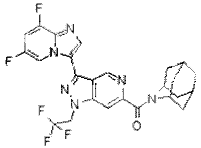 | (2-Aza-tricyclo[3.3.1.1*3,7*]dec-2-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone | 516.47 |
| 179 | 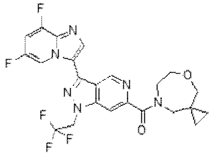 | [3-(6,8-Difluoro-imidazo[1,2-a]pyridin-3-yl)-1-(2,2,2-trifluoro-ethyl)-1H-pyrazolo[4,3-c]pyridin-6-yl]-(5-oxa-8-aza-spiro[2.6]non-8-yl)-methanone | 506.43 |
| 180 | 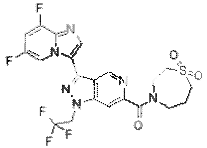 | [3-(6,8-Difluoro-imidazo[1,2-a]pyridin-3-yl)-1-(2,2,2-trifluoroethyl)-1H-pyrazolo[4,3-c]pyridin-6-yl]-1,4-thiazepan-1,1-dioxide-4-yl-methanone | 528.46 |
| 181 | 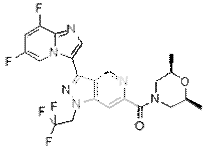 | Cis-[3-(6,8-Difluoro-imidazo[1,2-a]pyridin-3-yl)-1-(2,2,2-trifluoroethyl)-1H-pyrazolo[4,3-c]pyridin-6-yl]-(2,6-dimethyl-morpholin-4-yl)-methanone | 494.42 |
| 182 | 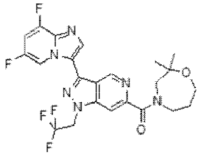 | [3-(6,8-Difluoro-imidazo[1,2-a]pyridin-3-yl)-1-(2,2,2-trifluoroethyl)-1H-pyrazolo[4,3-c]pyridin-6-yl]-(2 ,2-dimethyl-1,4-oxazepan-4-yl)-methanone | 508.44 |
| 183 | 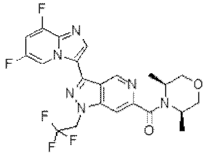 | Cis-[3-(6,8-Difluoro-imidazo[1,2-a]pyridin-3-yl)-1-(2,2,2-trifluoroethyl)-1H-pyrazolo[4,3-c]pyridin-6-yl]-(3,5-dimethyl-morpholin-4-yl)-methanone | 494.42 |
| 184 | 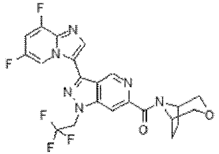 | [3-(6,8-Difluoro-imidazo[1,2-a]pyridin-3-yl)-1-(2,2,2-trifluoroethyl)-1H-pyrazolo[4,3-c]pyridin-6-yl]-(3-oxa-8-aza-bicyclo[3.2.1]oct-8-yl)-methanone | 492.40 |
| 185 | 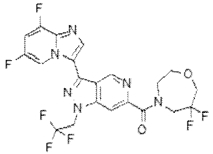 | [3-(6,8-Difluoro-imidazo[1,2-a]pyridin-3-yl)-1-(2,2,2-trifluoroethyl)-1H-pyrazolo[4,3-c]pyridin-6-yl]-(6,6-difluoro-1,4-oxazepan-4-yl)-methanone | 516.37 |
| 186 | 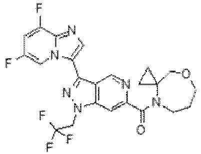 | [3-(6,8-Difluoro-imidazo[1,2-a]pyridin-3-yl)-1-(2,2,2-trifluoroethyl)-1H-pyrazolo[4,3-c]pyridin-6-yl]-(8-oxa-4-aza-spiro[2.6]non-4-yl)-methanone | 506.43 |
| 187 | 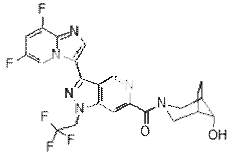 | [3-(6,8-Difluoro-imidazo[1,2-a]pyridin-3-yl)-1-(2,2,2-trifluoroethyl)-1H-pyrazolo[4,3-c]pyridin-6-yl]-(8-endo-hydroxy-3-aza-bicyclo[3.2.1]oct-3-yl)-methanone | 506.43 |
| 188 | 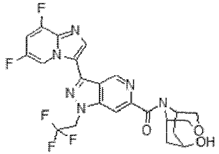 | [3-(6,8-Difluoro-imidazo[1,2-a]pyridin-3-yl)-1-(2,2,2-trifluoroethyl)-1H-pyrazolo[4,3-c]pyridin-6-yl]-(7-endo-hydroxy-3-oxa-9-aza-bicyclo[3.3.1]non-9-yl)-methanone | 522.43 |
| 189 | 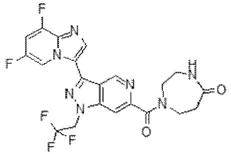 | 1-[3-(6,8-Difluoro-imidazo[1,2-a]pyridin-3-yl)-1-(2,2,2-trifluoroethyl)-1H-pyrazolo[4,3-c]pyridine-6-carbonyl]-1,4-diazepan-5-one | 493.39 |
| 190 | 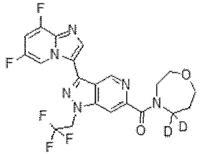 | [3-(6,8-Difluoro-imidazo[1,2-a]pyridin-3-yl)-1-(2,2,2-trifluoroethyl)-1H-pyrazolo[4,3-c]pyridin-6-yl]-1,4-oxazepan-deuterium-4-yl-methanone | 482.40 |
| 191 | 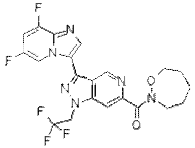 | [3-(6,8-Difluoro-imidazo[1,2-a]pyridin-3-yl)-1-(2,2,2-trifluoroethyl)-1H-pyrazolo[4,3-c]pyridin-6-yl]-[1,2]oxazepan-2-yl-methanone | 480.39 |
| 192 | 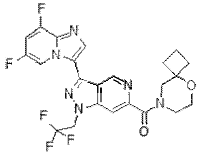 | [3-(6,8-Difluoro-imidazo[1,2-a]pyridin-3-yl)-1-(2,2,2-trifluoroethyl)-1H-pyrazolo[4,3-c]pyridin-6-yl]-(5-oxa-8-aza-spiro[3.5]non-8-yl)-methanone | 506.43 |
| 193 | 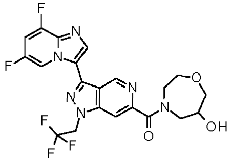 | [3-(6,8-Difluoro-imidazo[1,2-a]pyridin-3-yl)-1-(2,2,2-trifluoroethyl)-1H-pyrazolo[4,3-c]pyridin-6-yl]-(6-hydroxy-1,4-oxazepan-4-yl)-methanone | 496.39 |
| 194 | 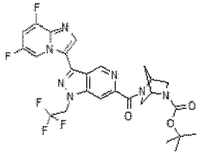 | 5-[3-(6,8-Difluoro-imidazo[1,2-a]pyridin-3-yl)-1-(2,2,2-trifluoroethyl)-1H-pyrazolo[4,3-c]pyridine-6-carbonyl]-2,5-diazabicyclo[2.2.1]heptane-2-carboxylic acid tert-butyl ester | 577.51 |
| 195 | 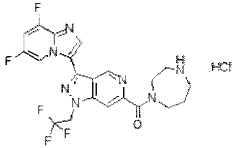 | 1,4-Diazepan-1-yl-[3-(6,8-difluoro-imidazo[1,2-a]pyridin-3-yl)-1-(2,2,2-trifluoro-ethyl)-1H-pyrazolo[4,3-c]pyridin-6-yl]-methanone hydrochloride | 479.41 |
| 196 | 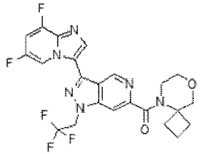 | [3-(6,8-Difluoro-imidazo[1,2-a]pyridin-3-yl)-1-(2,2,2-trifluoroethyl)-1H-pyrazolo[4,3-c]pyridin-6-yl]-(8-oxa-5-aza-spiro[3.5]non-5-yl)-methanone | 506.43 |
| 197 | 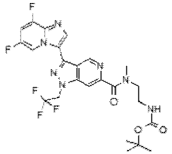 | (2-{[3-(6,8-Difluoro-imidazo[1,2-a]pyridin-3-yl)-1-(2,2,2-trifluoroethyl)-1H-pyrazolo[4,3-c]pyridine-6-carbonyl]-methyl-amino}-ethyl)-carbamic acid tert-butyl ester | 553.48 |
| 198 | 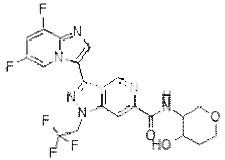 | 3-(6,8-Difluoro-imidazo[1,2-a]pyridin-3-yl)-1-(2,2,2-trifluoroethyl)-1H-pyrazolo[4,3-c]pyridine-6-carboxylic acid (4-hydroxy-tetrahydro-pyran-3-yl)-amide | 496.39 |
| 199 | 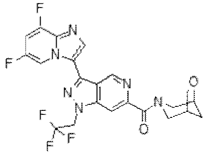 | [3-(6,8-Difluoro-imidazo[1,2-a]pyridin-3-yl)-1-(2,2,2-trifluoroethyl)-1H-pyrazolo[4,3-c]pyridin-6-yl]-(6-oxa-3-aza-bicyclo[3.2.1]oct-3-yl)-methanone | 492.40 |
| 200 | 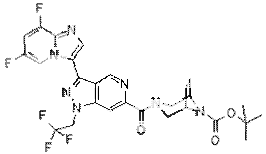 | 3-[3-(6,8-Difluoro-imidazo[1,2-a]pyridin-3-yl)-1-(2,2,2-trifluoroethyl)-1H-pyrazolo[4,3-c]pyridine-6-carbonyl]-3,8-diaza-bicyclo[3.2.1]octane-8-carboxylic acid tert-butyl ester | 591.53 |
| 201 | 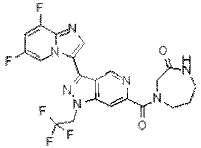 | 4-[3-(6,8-Difluoro-imidazo[1,2-a]pyridin-3-yl)-1-(2,2,2-trifluoroethyl)-1H-pyrazolo[4,3-c]pyridine-6-carbonyl]-1,4-diazepan-2-one | 493.39 |
| 202 | 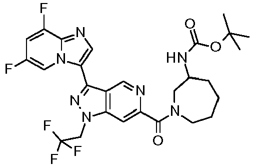 | {1 -[3-(6,8-Difluoro-imidazo[1,2-a]pyridin-3-yl)-1-(2,2,2-trifluoroethyl)-1H-pyrazolo[4,3-c]pyridine-6-carbonyl]-azepan-3-yl}-carbamic acid tert-butyl ester | 593.55 |
| 203 | 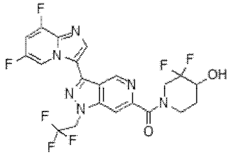 | (3,3-Difluoro-4-hydroxy-piperidin-1-yl)-[3-(6,8-difluoro-imidazo[1,2-a]pyridin-3-yl)-1-(2,2,2-trifluoroethyl)-1H-pyrazolo[4,3-c]pyridin-6-yl]-methanone | 516.37 |
| 204 | 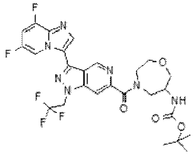 | {4-[3-(6,8-Difluoro-imidazo[1,2-a]pyridin-3-yl)-1-(2,2,2-trifluoroethyl)-1H-pyrazolo[4,3-c]pyridine-6-carbonyl]-1,4-oxazepan-6-yl}-carbamic acid tert-butyl ester | 595.52 |
| 205 | 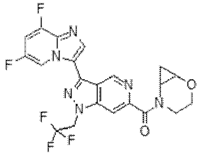 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[4.1.0]hept-5-yl)-methanone | 478.38 |
| 206 | 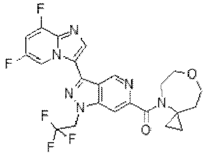 | [3-(6,8-Difluoro-imidazo[1,2-a]pyridin-3-yl)-1-(2,2,2-trifluoroethyl)-1H-pyrazolo[4,3-c]pyridin-6-yl]-(7-oxa-4-aza-spiro[2.6]non-4-yl)-methanone | 506.43 |
| 207 | 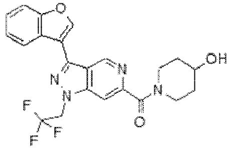 | [3-Benzofuran-3-yl-1-(2,2,2-trifluoro-ethyl)-1H-pyrazolo[4,3-c]pyridin-6-yl]-(4-hydroxy-piperidin-1-yl)-methanone | 444.41 |
| 208 | 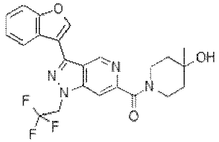 | [3-Benzofuran-3-yl-1-(2,2,2-trifluoro-ethyl)-1H-pyrazolo[4,3-c]pyridin-6-yl]-(4-hydroxy-4-methyl-piperidin-1-yl)-methanone | 458.43 |
| 209 | 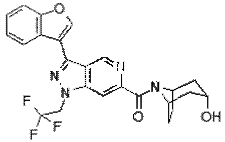 | [3-Benzofuran-3-yl-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone | 470.44 |
| 210 | 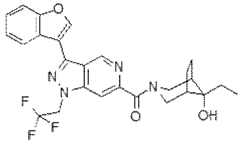 | [3-Benzofuran-3-yl-1-(2,2,2-trifluoro-ethyl)-1H-pyrazolo[4,3-c]pyridin-6-yl]-(8-exo-ethyl-8-endo-hydroxy-3-aza-bicyclo[3.2.1]oct-3-yl)-methanone | 498.50 |
| 211 | 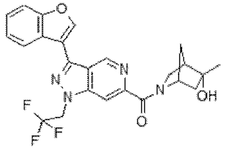 | [3-Benzofuran-3-yl-1-(2,2,2-trifluoro-ethyl)-1H-pyrazolo[4,3-c]pyridin-6-yl]-(5-endo-hydroxy-5-exo-methyl-2-aza-bicyclo[2.2.1]hept-2-yl)-methanone | 470.44 |
| 212 | 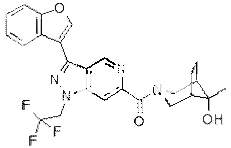 | [3-Benzofuran-3-yl-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone | 484.47 |
| 213 | 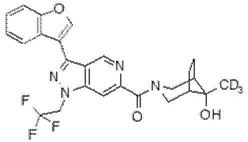 | [3-Benzofuran-3-yl-1-(2,2,2-trifluoro-ethyl)-1H-pyrazolo[4,3-c]pyridin-6-yl]-(8-endo-hydroxy-8-exo-methyl-*d₃*-3-aza-bicyclo[3.2.1]oct-3-yl)-methanone | 487.49 |
| 214 | 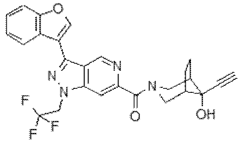 | [3-Benzofuran-3-yl-1-(2,2,2-trifluoro-ethyl)-1H-pyrazolo[4,3-c]pyridin-6-yl]-(8-exo-ethynyl-8-endo-hydroxy-3-aza-bicyclo[3.2.1]oct-3-yl)-methanone | 494.47 |
| 215 | 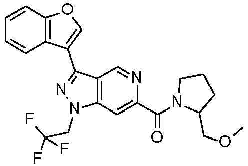 | [3-Benzofuran-3-yl-1-(2,2,2-trifluoro-ethyl)-1H-pyrazolo[4,3-c]pyridin-6-yl]-(2-methoxymethyl-pyrrolidin-1-yl)-methanone | 458.43 |
| 216 | 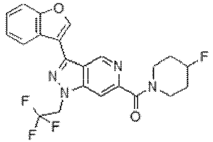 | [3-Benzofuran-3-yl-1-(2,2,2-trifluoro-ethyl)-1H-pyrazolo[4,3-c]pyridin-6-yl]-(4-fluoro-piperidin-1-yl)-methanone | 446.40 |
| 217 | 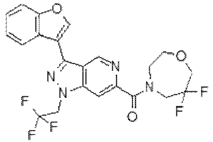 | 3-Benzofuran-3-yl-1-(2,2,2-trifluoroethyl)-1H-pyrazolo[4,3-c]pyridin-6-yl]-(6,6-difluoro-1,4-oxazepan-4-yl)-methanone | 480.39 |
| 218 | 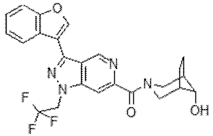 | [3-Benzofuran-3-yl-1-(2,2,2-trifluoro-ethyl)-1H-pyrazolo[4,3-c]pyridin-6-yl]-(8-endo-hydroxy-3-aza-bicyclo[3.2.1]oct-3-yl)-methanone | 470.44 |
| 219 | 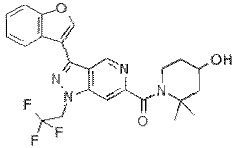 | [3-Benzofuran-3-yl-1-(2,2,2-trifluoro-ethyl)-1H-pyrazolo[4,3-c]pyridin-6-yl]-(4-hydroxy-2,2-dimethyl-piperidin-1-yl)-methanone | 472.46 |
| 220 | 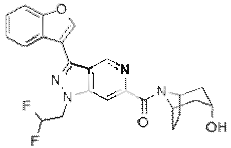 | [3-Benzofuran-3-yl-1-(2,2-difluoroethyl)-1H-pyrazolo[4,3-c]pyridin-6-yl]-(3-endo-hydroxy-8-aza-bicyclo[3.2.1]oct-8-yl)-methanone | 452.45 |
| 221 | 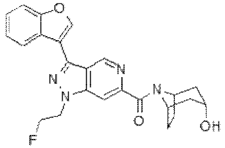 | [3-Benzofuran-3-yl-1-(2-fluoroethyl)-1 H-pyrazolo[4,3-c]pyridin-6-yl]-(3-endo-hydroxy-8-aza-bicyclo[3.2.1]oct-8-yl)-methanone | 434.46 |
| 222 | 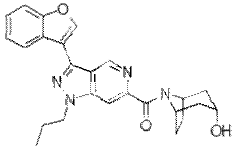 | (3-Benzofuran-3-yl-1-propyl-1H-pyrazolo[4,3-c]pyridin-6-yl)-(3-endo-hydroxy-8-aza-bicyclo[3.2.1]oct-8-yl)-methanone | 430.50 |
| 223 | 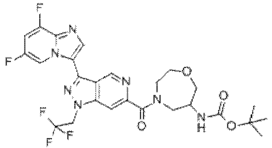 | tert-butyl N-[4-[3-(6,8-difluoroimidazo[1,2-a]pyridin-3-yl)-1-(2,2,2-trifluoroethyl)pyrazolo[4,3-c]pyridine-6-carbonyl]-1,4-oxazepan-6-yl]carbamate | 595.52 |
| 224 | 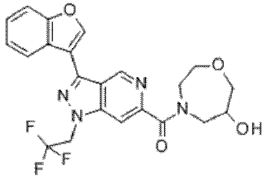 | [3-(benzofuran-3-yl)-1-(2,2,2-trifluoroethyl)pyrazolo[4,3-c]pyridin-6-yl]-(6-hydroxy-1,4-oxazepan-4-yl)methanone | 460.41 |
| 225 | 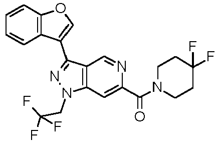 | [3-(benzofuran-3-yl)-1-(2,2,2-trifluoroethyl)pyrazolo[4,3-c]pyridin-6-yl]-(4,4-difluoro-1-piperidyl)methanone | 464.39 |
| 226 | 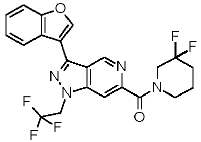 | [3-(benzofuran-3-yl)-1-(2,2,2-trifluoroethyl)pyrazolo[4,3-c]pyridin-6-yl]-(3,3-difluoro-1-piperidyl)methanone | 464.39 |
| 227 | 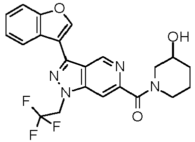 | [3-Benzofuran-3-yl-1-(2,2,2-trifluoro-ethyl)-1H-pyrazolo[4,3-c]pyridin-6-yl]-(3-hydroxy-piperidin-1-yl)-methanone | 444.41 |
| 228 | 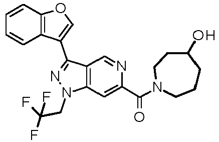 | [3-(benzofuran-3-yl)-1-(2,2,2-trifluoroethyl)pyrazolo[4,3-c]pyridin-6-yl]-(4-hydroxyazepan-1-yl)methanone | 458.43 |
| 229 | 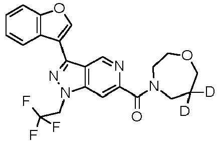 | [3-(benzofuran-3-yl)-1-(2,2,2-trifluoroethyl)pyrazolo[4,3-c]pyridin-6-yl]-(6,6-dideuterio-1,4-oxazepan-4-yl)methanone | 446.42 |
| 230 | 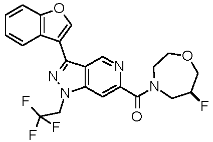 | [3-(benzofuran-3-yl)-1-(2,2,2-trifluoroethyl)pyrazolo[4,3-c]pyridin-6-yl]-[6-fluoro-1,4-oxazepan-4-yl]methanone | 462.40 |
| 231 | 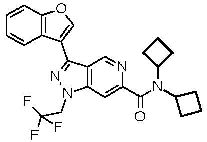 | 3-(benzofuran-3-yl)-N,N-di(cyclobutyl)-1-(2,2,2-trifluoroethyl)pyrazolo[4,3-c]pyridine-6-carboxamide | 468.47 |
| 232 | 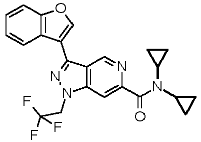 | 3-(benzofuran-3-yl)-N,N-di(cyclopropyl)-1-(2,2,2-trifluoroethyl)pyrazolo[4,3-c]pyridine-6-carboxamide | 440.42 |
| 233 | 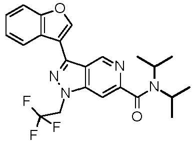 | 3-(benzofuran-3-yl)-N,N-diisopropyl-1-(2,2,2-trifluoroethyl)pyrazolo[4,3-c]pyridine-6-carboxamide | 444.45 |
| 234 | 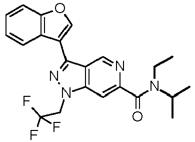 | 3-(benzofuran-3-yl)-N-ethyl-N-isopropyl-1-(2,2,2-trifluoroethyl)pyrazolo[4,3-c]pyridine-6-carboxamide | 430.43 |
| 235 | 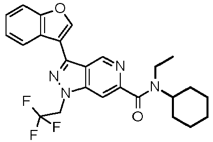 | 3-(benzofuran-3-yl)-N-cyclohexyl-N-ethyl-1-(2,2,2-trifluoroethyl)pyrazolo[4,3-c]pyridine-6-carboxamide | 470.49 |
| 236 | 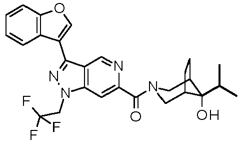 | [3-(benzofuran-3-yl)-1-(2,2,2-trifluoroethyl)pyrazolo[4,3-c]pyridin-6-yl]-(8-hydroxy-8-isopropyl-3-azabicyclo[3.2.1]octan-3-yl)methanone | 512.52 |
| 237 | 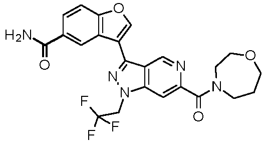 | 3-[6-(1,4-oxazepane-4-carbonyl)-1-(2,2,2-trifluoroethyl)pyrazolo[4,3-c]pyridin-3-yl]benzofuran-5-carboxamide | 487.43 |
| 238 | 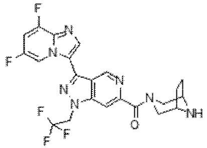 | (3,8-Diaza-bicyclo[3.2.1]oct-3-yl)-[3-(6,8-difluoro-imidazo[1,2-a]pyridin-3-yl)-1-(2,2,2-trifluoroethyl)-1H-pyrazolo[4,3-c]pyridin-6-yl]-methanone | 491.42 |
| 239 | 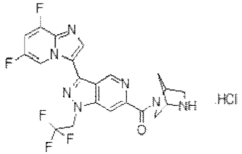 | (2,5-Diaza-bicyclo[2.2.1]hept-2-yl)-[3-(6,8-difluoro-imidazo[1,2-a]pyridin-3-yl)-1-(2,2,2-trifluoroethyl)-1H-pyrazolo[4,3-c]pyridin-6-yl]-methanone hydrochloride | 477.39 |
| 240 | 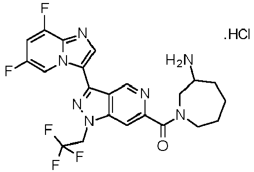 | (3-Amino-azepan-1-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone hydrochloride | 493.43 |
| 241 | 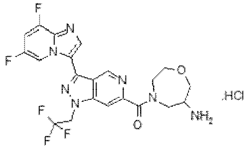 | (6-Amino-1,4-oxazepan-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone hydrochloride | 495.41 |
| 242 | 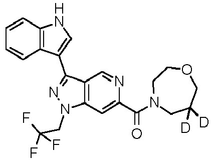 | (6,6-dideuterio-1,4-oxazepan-4-yl)-[3-(1H-indol-3-yl)-1-(2,2,2-trifluoroethyl)pyrazolo[4,3-c]pyridin-6-yl]methanone | 445.43 |
| 243 | 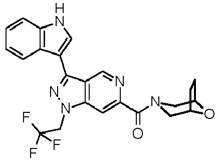 | [3-(1H-indol-3-yl)-1-(2,2,2-trifluoroethyl)pyrazolo[4,3-c]pyridin-6-yl]-(8-oxa-3-azabicyclo[3.2.1]octan-3-yl)methanone | 455.43 |
| 244 | 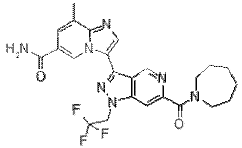 | 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carboxylic acid amide | 499.49 |
| 245 | 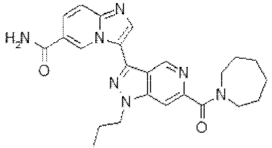 | 3-[6-(Azepane-1-carbonyl)-1-propyl-1H-pyrazolo[4,3-c]pyridin-3-yl]-imidazo[1,2-a]pyridine-6-carboxylic acid amide | 445.52 |
| 246 | 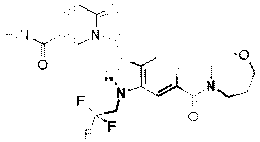 | 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carboxylic acid amide | 487.43 |
| 247 | 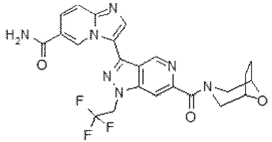 | 3-[6-(8-Oxa-3-aza-bicyclo[3.2.1]octane-3-carbonyl)-1-(2,2,2-trifluoro-ethyl)-1H-pyrazolo[4,3-c]pyridin-3-yl]-imidazo[1,2-a]pyridine-6-carboxylic acid amide | 499.45 |
| 248 | 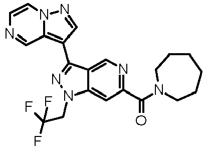 | Azepan-1 -yl-[3-pyrazolo[1,5-a]pyrazin-3-yl-1-(2,2,2-trifluoroethyl)-1H-pyrazolo[4,3-c]pyridin-6-yl]-methanone | 443.43 |
| 249 | 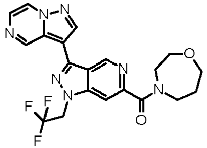 | 1,4-Oxazepan-4-yl-[3-pyrazolo[1,5-a]pyrazin-3-yl-1-(2,2,2-trifluoroethyl)-1H-pyrazolo[4,3-c]pyridin-6-yl]-methanone | 445.4 |
| 250 | 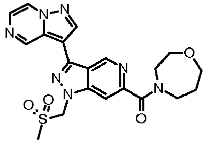 | (1-Methanesulfonylmethyl-3-pyrazolo[1,5-a]pyrazin-3-yl-1H-pyrazolo[4,3-c]pyridin-6-yl)-(1,4-oxazepan-4-yl)-methanone | 455.49 |
| 251 | 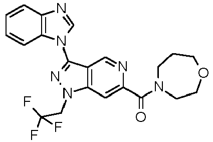 | [3-(benzimidazol-1-yl)-1-(2,2,2-trifluoroethyl)pyrazolo[4,3-c]pyridin-6-yl]-(1,4-oxazepan-4-yl)methanone | 444.41 |
| 252 | 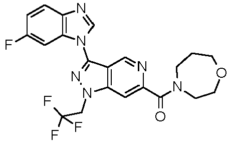 | [3-(6-fluorobenzimidazol-1-yl)-1-(2,2,2-trifluoroethyl)pyrazolo[4,3-c]pyridin-6-yl]-(1,4-oxazepan-4-yl)methanone | 462.40 |
| 253 | 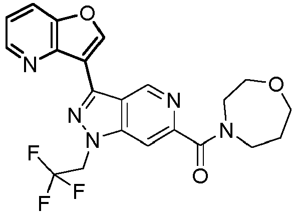 | [3-furo[3,2-b]pyridin-3-yl-1-(2,2,2-trifluoroethyl)pyrazolo[4,3-c]pyridin-6-yl]-(1,4-oxazepan-4-yl)methanone | 445.40 |
| 254 | 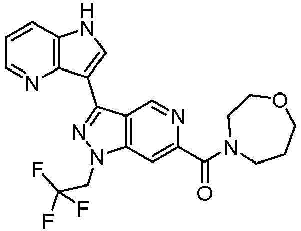 | [1,4]Oxazepan-4-yl-[3-(1H-pyrrolo[3,2-b]pyridin-3-yl)-1-(2,2,2-trifluoro-ethyl)-1H-pyrazolo[4,3-c]pyridin-6-yl]-methanone | 444.42 |
| 255 | 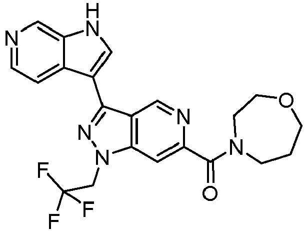 | [1,4]Oxazepan-4-yl-[3-(1H-pyrrolo[2,3-c]pyridin-3-yl)-1-(2,2,2-trifluoro-ethyl)-1H-pyrazolo[4,3-c]pyridin-6-yl]-methanone | 444.42 |
| 256 | 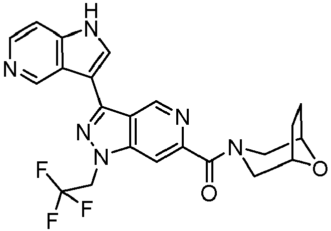 | (8-Oxa-3-aza-bicyclo[3.2.1]oct-3-yl)-[3-(1H-pyrrolo[3,2-c]pyridin-3-yl)-1-(2,2,2-trifluoro-ethyl)-1H-pyrazolo[4,3-c]pyridin-6-yl]-methanone | 456.43 |

### Example 2: Preparation of the compounds of the present invention and analytical methods

The compounds of general formula (I) and their pharmaceutically acceptable salts can be synthesized according to methods described in the following schemes:

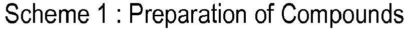

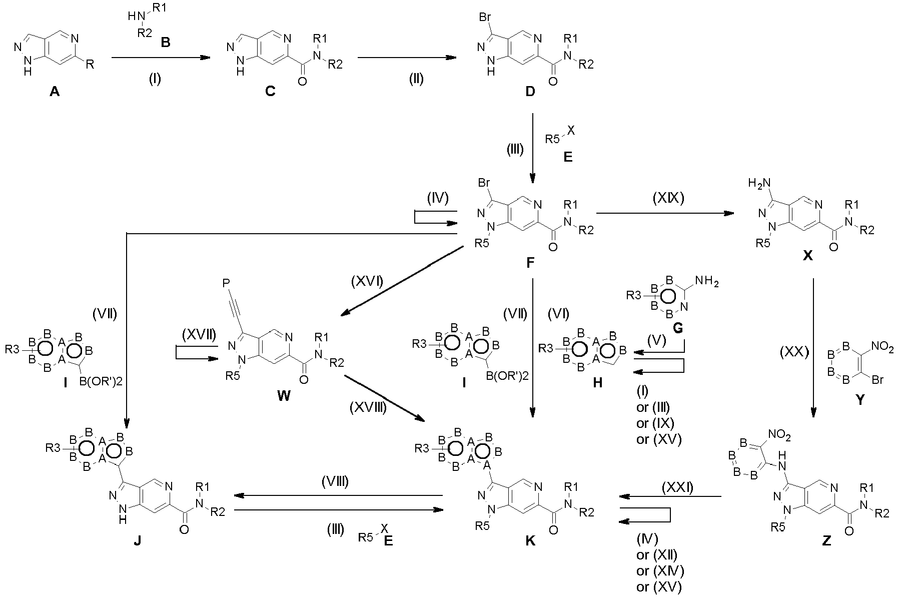

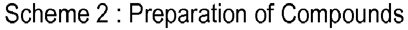

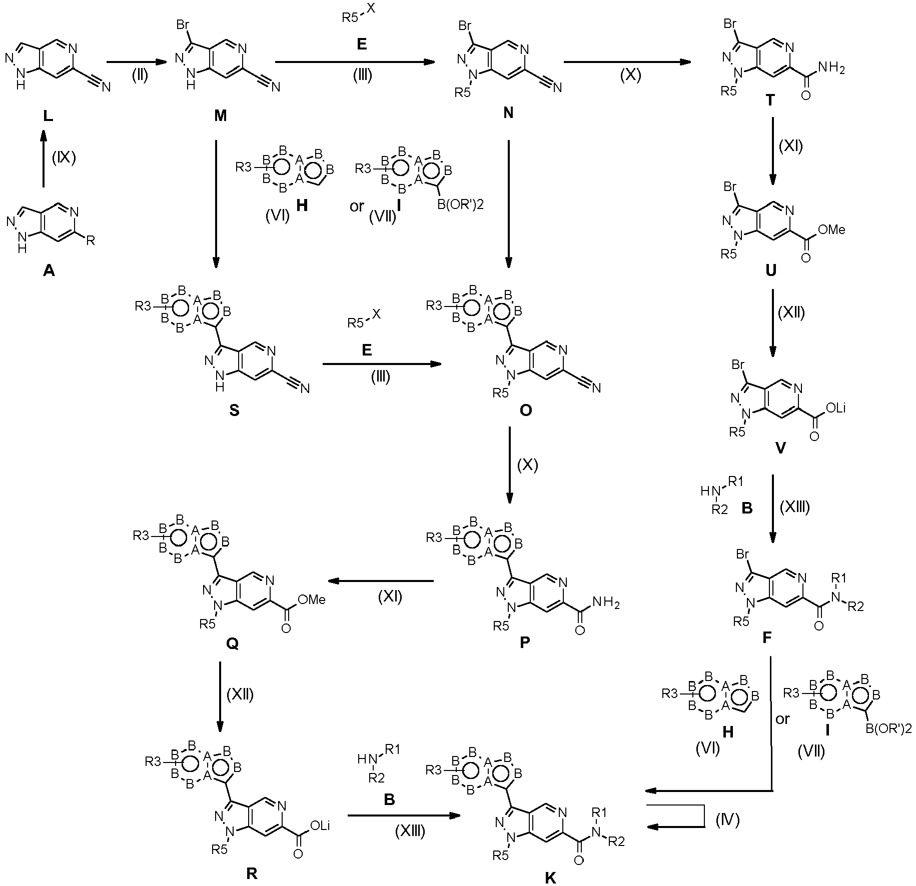

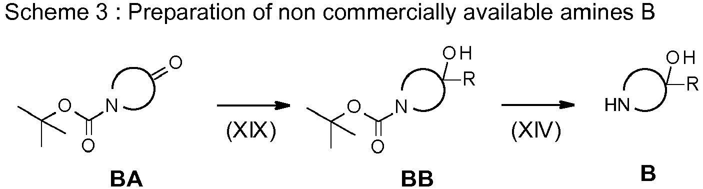

### Experimental procedures

### General conditions:

All reagents were commercial grade and used without further purification. Reactions were typically run using anhydrous solvents under argon atmosphere. Thin layer chromatography was carried out using pre-coated silica gel F-254 plate. Flash column chromatography were performed using a Biotage isolera 4 system, with the Biotage SNAP cartridge KP-SIL (40-60µm) if not specified. Other cartridges like the Biotage SNAP cartridge KP-NH (40-60µm) or the Interchim PF-15SIHP-F0025 cartridge (15µm) were punctually used. After purification by flash chromatography, final compounds were usually triturated in Et₂O or *i*Pr₂O then dried overnight under vacuum at 70°C. Final compounds were usually synthesized in 10 to 100 mg scale.

Reactions were monitored and molecules were characterized using a Waters Acquity UPLC H-class system with a photodiode array detector (190-400nm). An Acquity CSH C18 1.7µM 2.1x30mm column was used. The mobile phase consisted in a gradient of A and B: A was water with 0.025% of trifluoroacetic acid and B was acetonitrile with 0.025% of trifluoroacetic acid. Flow rate was 0.8 ml per min. All analyses were performed at 55°C. The UPLC system was coupled to a Waters SQD2 platform. All mass spectra were full-scan experiments (mass range 100-800amu). Mass spectra were obtained using positive electro spray ionisation.

Preparative HPLC were performed using a Waters HPLC system with a 2767 sample manager, a 2525 pump, a photodiode array detector (190-400nm) enabling analytical and preparative modes. An Xselect CSH C18 3.5µM 4.6x50mm column was used in analytical mode and a Xselect CSH C18 5µM 19x100mm column in preparative mode. The mobile phase consisted in both cases in a gradient of A and B : A was water with 0.1% of formic acid and B was acetonitrile with 0.1% of formic acid. Flow rate was 1 ml per min in analytical mode and 25 ml per min in preparative mode. All LCMS analysis/purification were performed at room temperature. The HPLC system was coupled with a Waters Acquity QDa detector. All mass spectra were full-scan experiments (mass range 100-800amu). Mass spectra were obtained using positive electro spray ionisation.

All NMR experiments were recorded on a Brucker AMX-400 spectrometer. Proton chemical shift are listed relative to residual DMSO (2.50ppm). Splitting patterns are designated as s (singlet), d (doublet), dd (doublet of doublet), t (triplet), dt (doublet of triplet), td (triplet of doublet), tt (triplet of triplet), q (quartet), quint (quintuplet), m (multiplet), b (broad), bs (broad singlet). When compounds were characterized as rotamer mixtures at 25°C, some signals could be specifically assigned to a rotamer.

### General Procedure I : Synthesis of compounds C and H from compounds A and H respectively (scheme 1)

In a 2-chamber glassware system, a suspension of an halogenated heteroaromatic compound A or H (1 equiv.), amine B (2 equiv.), triethylamine (2 equiv.), and XantPhos Pd G3 precatalyst (2 mol%) in anhydrous dioxane (0.3M) was degassed under argon in the chamber 1. In the chamber 2, DBU (1.5 equiv.) was added to a solution of molybdenum hexacarbonyl (0.5 equiv.) in anhydrous dioxane (0.3M). Both chambers were immediately sealed, the reaction mixtures were heated overnight at 85°C. The chamber 1 reaction mixture was diluted with a DCM/MeOH mixture. Potassium carbonate (3 equiv.) was added. Silica was added to this solution in order to prepare a solid deposit for purification by flash chromatography to afford compound C or H respectively.

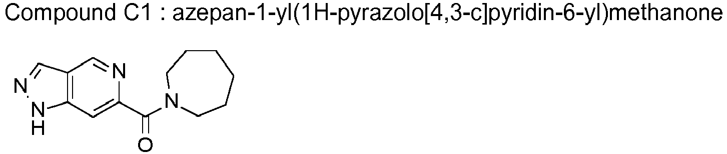

Compound C1 was obtained according to General Procedure I, starting from 6-bromo-1H-pyrazolo[4,3-c]pyridine A and azepane B1. Purification by flash chromatography (DCM/MeOH: 100/0 to 92/8) afforded C1 as a yellow powder in 80% yield. M/Z (M+H)⁺: 245

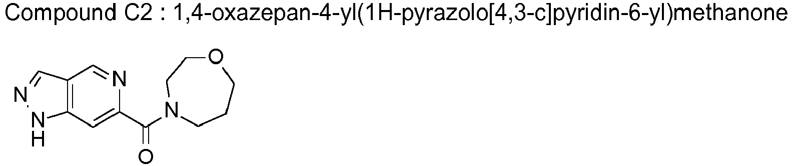

Compound C2 was obtained according to General Procedure I, starting from 6-bromo-1H-pyrazolo[4,3-c]pyridine A and 1,4-oxazepane B2. Purification by flash chromatography (DCM/MeOH: 100/0 to 94/6) afforded C2 as a light yellow powder in 65% yield. M/Z (M+H)⁺: 247

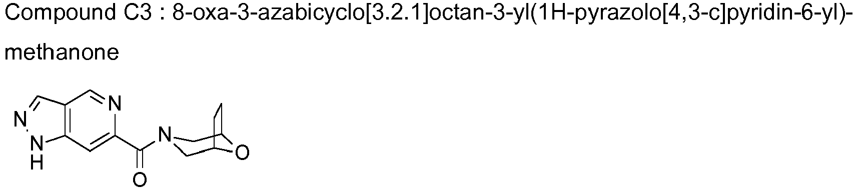

Compound C3 was obtained according to General Procedure I, starting from 6-bromo-1H-pyrazolo[4,3-c]pyridine A and 8-oxa-3-azabicyclo[3.2.1]octane hydrochloride B3. In that specific case, 3 equiv. of triethylamine and 5 equiv. of potassium carbonate were used. Purification by flash chromatography (DCM/MeOH: 10/0 to 9/1) afforded C3 as an orange oil in 89% yield. M/Z (M+H)⁺: 259

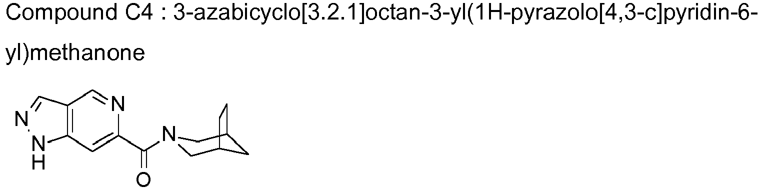

Compound C4 was obtained according to General Procedure I, starting from 6-bromo-1H-pyrazolo[4,3-c]pyridine A and 3-azabicyclo[3.2.1]octane hydrochloride B4. In that specific case, 4 equiv. of triethylamine was used. Purification by flash chromatography (DCM/MeOH: 100/0 to 94/6) afforded C4 as a yellow powder in 82% yield. M/Z (M+H)⁺: 257

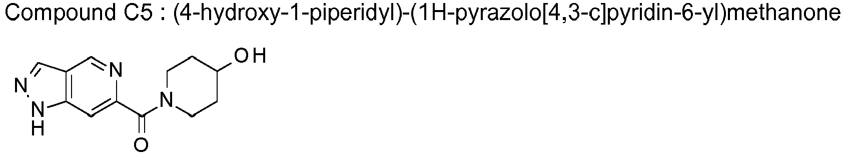

Compound C5 was obtained according to General Procedure I, using piperidin-4-ol B5. Purification by flash chromatography (DCM/MeOH: 10/0 to 9/1) afforded C5 as a yellow powder in 87% yield. M/Z (M+H)⁺: 247

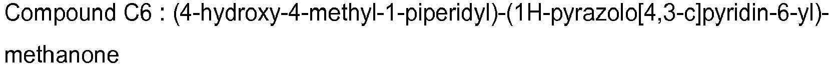

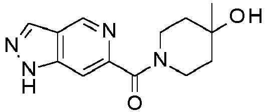

Compound C6 was obtained according to General Procedure I, using 4-methyl-piperidin-4-ol B6. Purification by flash chromatography (DCM/MeOH: 10/0 to 9/1) afforded C6 as an orange powder in 83% yield. M/Z (M+H)⁺: 261

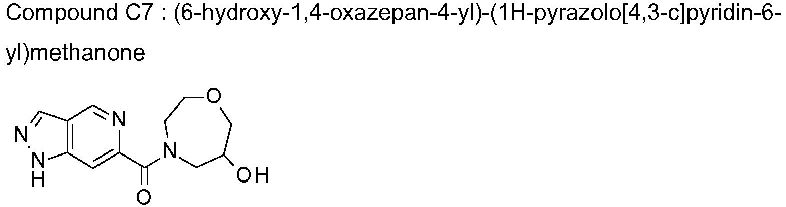

Compound C7 was obtained according to General Procedure I, using 1,4-oxazepan-6-ol B87. Purification by flash chromatography (DCM/MeOH: 10/0 to 9/1) afforded C7 in 65% yield. M/Z (M+H)⁺: 263

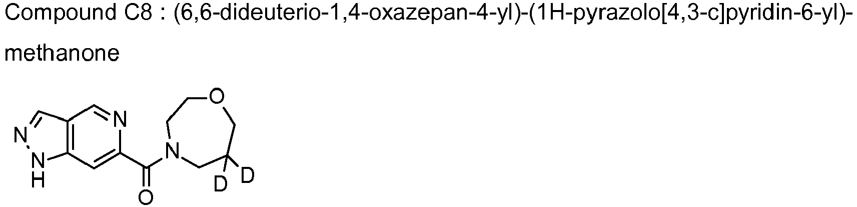

Compound C8 was obtained according to General Procedure I, using 6,6-dideuterio-1,4-oxazepane hydrochloride B91. In that specific case, 3 equiv. of triethylamine were used. Purification by flash chromatography (DCM/MeOH: 100/0 to 9/1) afforded C8 as a beige powder in quantitative yield. M/Z (M+H)⁺: 249

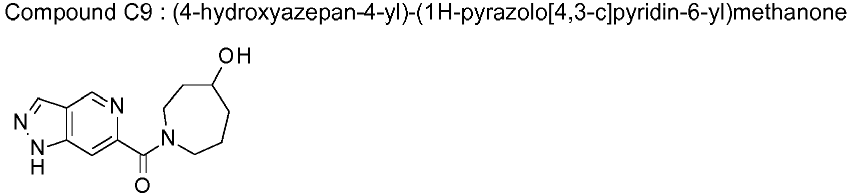

Compound C9 was obtained according to General Procedure I, using azepan-4-ol B50. Purification by flash chromatography (DCM/MeOH: 10/0 to 9/1) afforded C9 in 80% yield. M/Z (M+H)⁺: 261

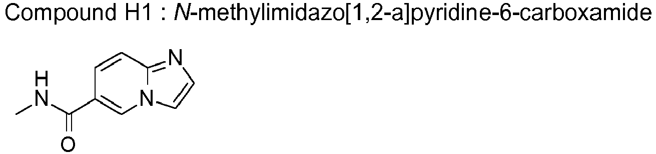

Compound H1 was obtained according to General Procedure I, starting from 6-bromoimidazo[1,2-a]pyridine H32 and methylamine, 2M in THF, B88. Purification by flash chromatography (DCM/MeOH: 10/0 to 9/1) afforded H1 in quantitative yield. M/Z (M+H)⁺: 176

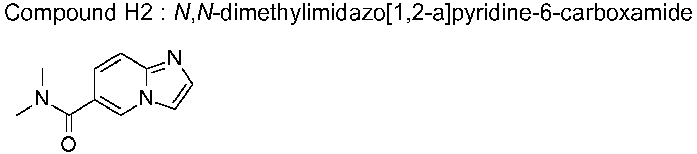

Compound H2 was obtained according to General Procedure I, starting from 6-bromoimidazo[1,2-a]pyridine H32 and dimethylamine, 2M in THF, B89. Purification by flash chromatography (DCM/MeOH: 100/0 to 95/5) afforded H2 in 96% yield. M/Z (M+H)⁺: 190

### General Procedure II : Synthesis of compounds D and M from compounds C and L respectively (schemes 1&2)

To a solution of compound C (1 equiv.) in DCM (0.2M) was added NBS (1.05 equiv.). The reaction mixture was stirred 1h at room temperature. The reaction mixture was diluted with DCM, washed with a saturated sodium bicarbonate solution and brine, dried over magnesium sulfate, concentrated then purified by flash chromatography to afford compound D.

### Alternative 1 :

To a solution of compound L (1 equiv.) in ACN (0.1M) was added NBS (1.05 equiv.). The reaction mixture was stirred 1h at 80°C. The reaction mixture was diluted with AcOEt, washed with water and brine, dried over magnesium sulfate, then concentrated. The resulting crude mixture was triturated in DCM. The precipitate was filtered to afford compound M.

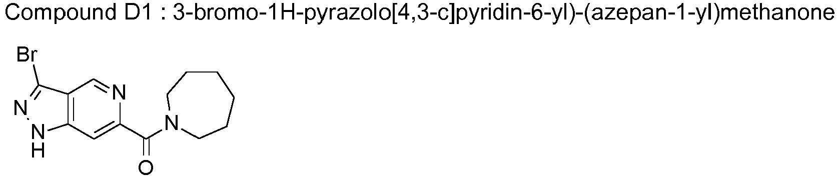

Compound D1 was obtained according to General Procedure II, starting from azepan-1-yl(1H-pyrazolo[4,3-c]pyridin-6-yl)methanone C1. Purification by flash chromatography (DCM/MeOH: 100/0 to 94/6) afforded D1 as a light yellow solid in 70% yield. M/Z (M+H)⁺: 323/325

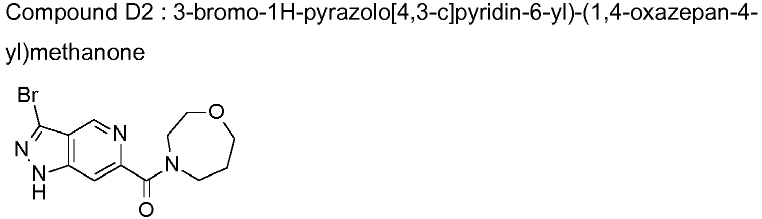

Compound D2 was obtained according to General Procedure II, starting from 1,4-oxazepan-4-yl(1H-pyrazolo[4,3-c]pyridin-6-yl)methanone C2. Purification by flash chromatography (DCM/MeOH: 10/0 to 9/1) afforded D2 as a light yellow powder in 95%. M/Z (M+H)⁺: 325/327

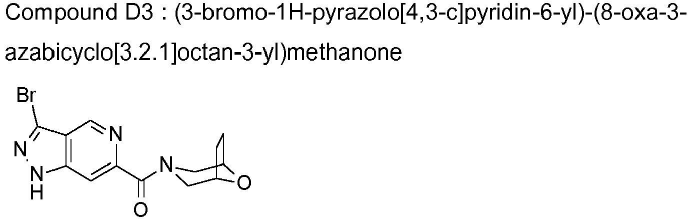

Compound D3 was obtained according to General Procedure II, starting from 8-oxa-3-azabicyclo[3.2.1]octan-3-yl(1H-pyrazolo[4,3-c]pyridin-6-yl)methanone C3. Purification by flash chromatography (DCM/MeOH: 10/0 to 9/1) afforded D3 as a white powder in 99% yield. M/Z (M+H)⁺: 337/339

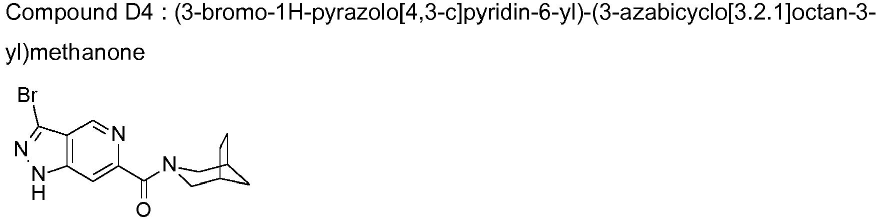

Compound D4 was obtained according to General Procedure II, starting from 3-azabicyclo[3.2.1]octan-3-yl(1H-pyrazolo[4,3-c]pyridin-6-yl)methanone C4. The aqueous work-up afforded D4 as a beige powder in 96% yield without further purification. M/Z (M+H)⁺: 335/337

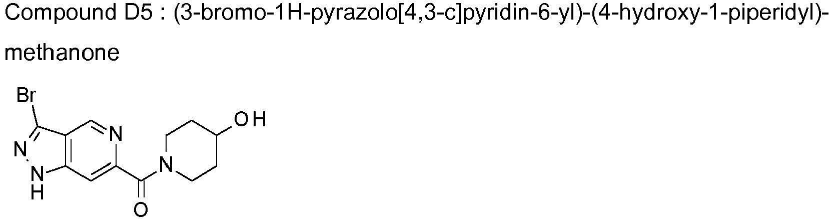

Compound D5 was obtained according to General Procedure II, starting from (4-hydroxy-1-piperidyl)-(1H-pyrazolo[4,3-c]pyridin-6-yl)methanone C5. Purification by flash chromatography (DCM/MeOH: 10/0 to 9/1) afforded D5 as a white powder in 83% yield. M/Z (M+H)⁺: 325/327

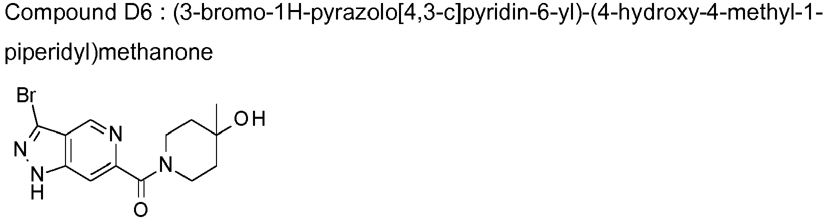

Compound D6 was obtained according to General Procedure II, starting from (4-hydroxy-4-methyl-1-piperidyl)-(1H-pyrazolo[4,3-c]pyridin-6-yl)methanone C6. Purification by flash chromatography (DCM/MeOH: 10/0 to 9/1) afforded D6 as a white powder in 95% yield. M/Z (M+H)⁺: 339/341

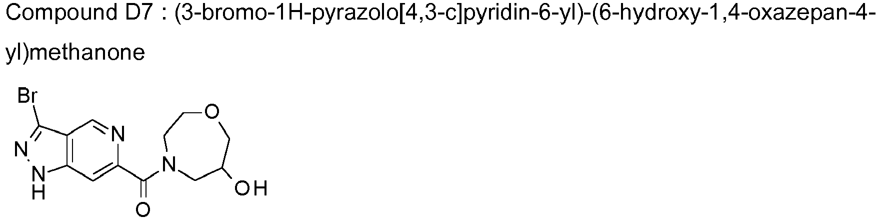

Compound D7 was obtained according to General Procedure II, starting from (6-hydroxy-1,4-oxazepan-4-yl)-(1H-pyrazolo[4,3-c]pyridin-6-yl)methanone C7. Purification by flash chromatography (DCM/MeOH: 10/0 to 9/1) afforded D7 in 86% yield. M/Z (M+H)⁺: 341/343

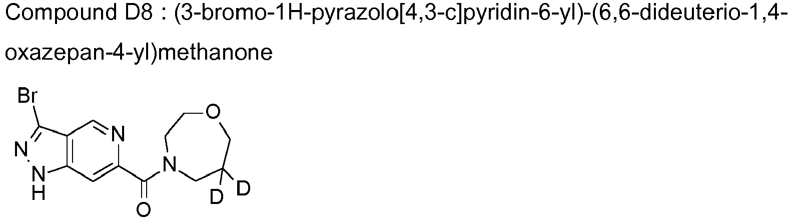

Compound D8 was obtained according to General Procedure II, starting from (6,6-dideuterio-1,4-oxazepan-4-yl)-(1H-pyrazolo[4,3-c]pyridin-6-yl)methanone C8. Purification by flash chromatography (DCM/MeOH: 10/0 to 9/1) afforded D8 as a yellow powder in 96% yield. M/Z (M+H)⁺: 327/329

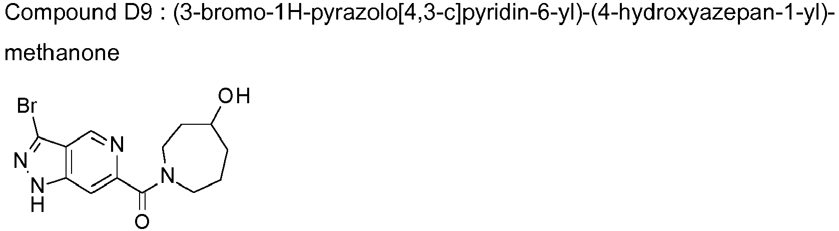

Compound D9 was obtained according to General Procedure II, starting from (4-hydroxyazepan-1-yl)-(1H-pyrazolo[4,3-c]pyridin-6-yl)methanone C9. Purification by flash chromatography (DCM/MeOH: 10/0 to 9/1) afforded D9 in 66% yield. M/Z (M+H)⁺: 339/341

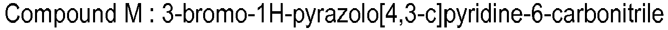

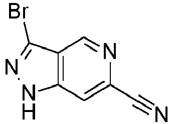

Compound M was obtained according to General Procedure II, Alternative 1, starting from 1H-pyrazolo[4,3-c]pyridine-6-carbonitrile L as a white powder in 97% yield. M/Z (M+H)⁺: 223/225

### General Procedure III : Synthesis of compounds or compounds F, H, K, N and O from compounds D, H, J, M and S respectively (schemes 1&2)

To a solution of compound D, H, J, M or S (1 equiv.) in DMF (0.15M) at 0°C was added sodium hydride (1.2 equiv.). The reaction mixture was stirred 10 min at 0°C. Compound E (1.2 equiv.) was added. The reaction mixture was stirred 4h at room temperature. The reaction mixture was quenched at 0°C with a saturated sodium bicarbonate solution and extracted with AcOEt. The organic phase was washed with brine, dried over magnesium sulfate then concentrated. The resulting oil was purified by flash chromatography to afford the compound or compound F, H, K, N or O respectively.

### Alternative 1 :

To a solution of compound D, H, J, M or S (1 equiv.) in DMF (0.15M) at 0°C was added sodium hydride (1.3 equiv.). The reaction mixture was stirred 10 min at 0°C. Compound E (1.3 equiv.) was added. The reaction mixture was stirred 15min at 150°C under microwave irradiation. The reaction mixture was quenched at 0°C with a saturated sodium bicarbonate solution and extracted with AcOEt. The organic phase was washed with brine, dried over magnesium sulfate then concentrated. The resulting oil was purified by flash chromatography to afford the compound or compound F, H, K, N or O respectively.

### Alternative 2 :

To a solution of compound D, H, J, M or S (1 equiv.) in DMF (0.15M) was added potassium carbonate (2.0 equiv.) and compound E (1.3 equiv.). The reaction mixture was stirred 4h at room temperature. The reaction mixture was diluted with a saturated sodium bicarbonate solution and extracted with AcOEt. The organic phase was washed with brine, dried over magnesium sulfate then concentrated. The resulting oil was purified by flash chromatography to afford the compound or compound F, H, K, N or O respectively.

### Alternative 3 :

To a solution of compound D, H, J, M or S (1 equiv.) in ACN (0.15M) C was added potassium carbonate (1.3 equiv.) and compound E (1.3 equiv.). The reaction mixture was 30min at 150°C under microwave irradiation. The reaction mixture was quenched by addition of a saturated ammonium chloride solution. The formed precipitate was washed with water then dried under vacuum with phosphorus pentoxide to afford the compound or compound F, H, K, N or O respectively.

### Alternative 4 :

To a solution of compound D in dioxane (0.2M) were added 3,4-dihydro-2H-pyran (20 equiv.) and PTSA (10 mol%). The reaction mixture was heated 15 min under microwave irradiation at 150°C. The reaction mixture was diluted with AcOEt, washed with a saturated sodium bicarbonate solution and brine, dried over magnesium sulfate then concentrated. The residue was purified by flash chromatography to afford compound F.

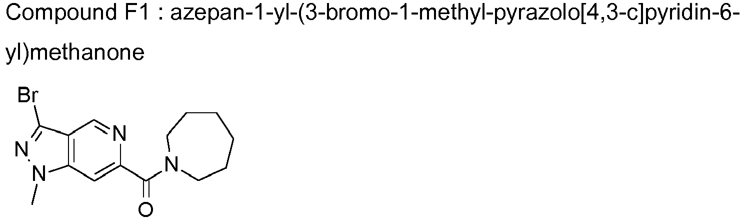

Compound F1 was obtained according to General Procedure III, starting from 3-bromo-1H-pyrazolo[4,3-c]pyridin-6-yl)-(azepan-1-yl)methanone D1 and methyl iodide E1. Purification by flash chromatography (Cyclohexane/AcOEt: 10/0 to 0/10) afforded F1 as a white powder in 75% yield. M/Z (M+H)⁺: 337/339

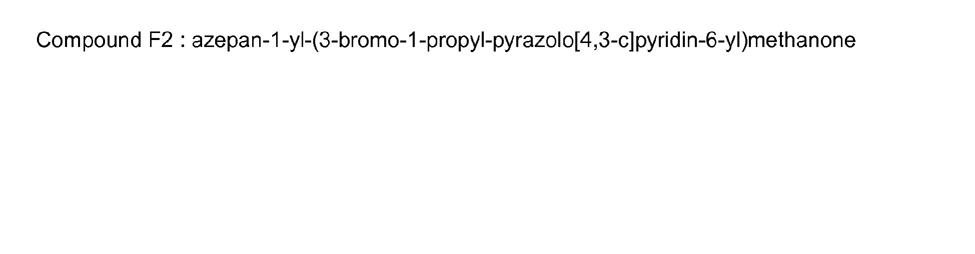

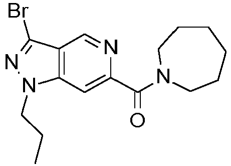

Compound F2 was obtained according to General Procedure III, starting from 3-bromo-1H-pyrazolo[4,3-c]pyridin-6-yl)-(azepan-1-yl)methanone D1 and 1-bromopropane E2 (1.5 equiv.). Purification by flash chromatography (Cyclohexane/AcOEt: 10/0 to 0/10) afforded F2 as a white powder in 56% yield. M/Z (M+H)⁺: 365/367

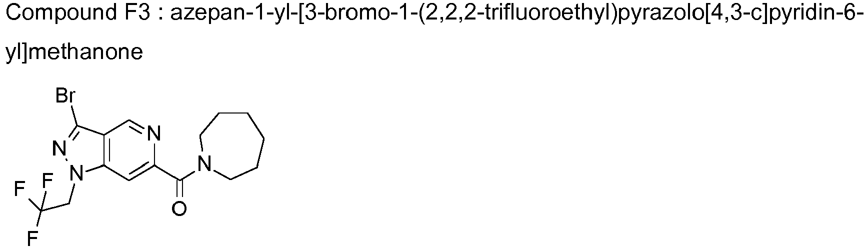

Compound F3 was obtained according to General Procedure III, Alternative 1, starting from 3-bromo-1H-pyrazolo[4,3-c]pyridin-6-yl)-(azepan-1-yl)methanone D1 and 2,2,2-trifluoroethyl *p*-toluenesulfonate E3. Purification by flash chromatography (Cyclohexane/AcOEt: 10/0 to 3/7 then DCM/MeOH : 100/0 to 95/5) afforded F3 as a light yellow solid in 47% yield. M/Z (M+H)⁺: 405/407

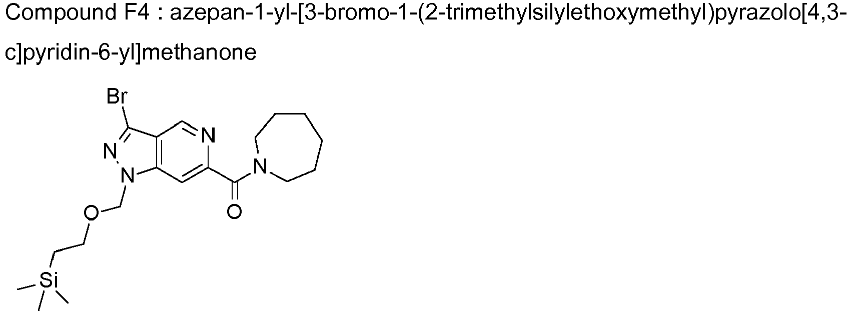

Compound F4 was obtained according to General Procedure III, starting from 3-bromo-1H-pyrazolo[4,3-c]pyridin-6-yl)-(azepan-1-yl)methanone D1 and 2-(trimethylsilyl)ethoxymethyl chloride E4. Purification by flash chromatography (Cyclohexane/AcOEt: 10/0 to 0/10) afforded F4 as a colorless oil in 86% yield. M/Z (M+H)⁺: 453/455

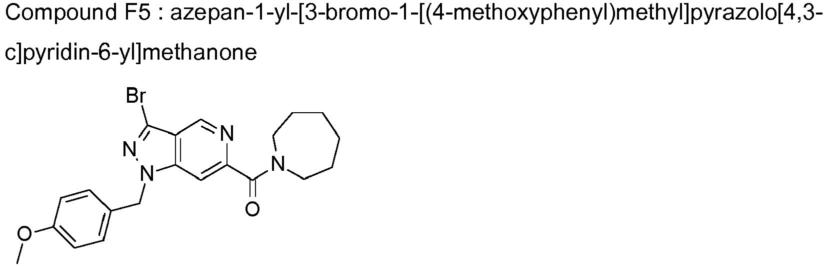

Compound F5 was obtained according to General Procedure III, starting from 3-bromo-1H-pyrazolo[4,3-c]pyridin-6-yl)-(azepan-1-yl)methanone D1 and 4-methoxybenzyl chloride E5. Purification by flash chromatography (Cyclohexane/AcOEt: 10/0 to 5/5) afforded F5 as a colorless oil in 96% yield. M/Z (M+H)⁺: 443/445

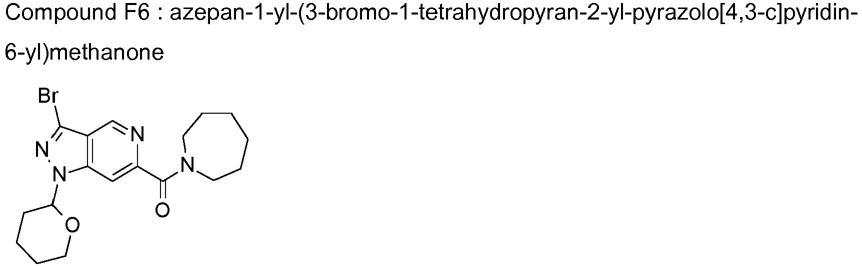

Compound F6 was obtained according to General Procedure III, Alternative 4, starting from 3-bromo-1H-pyrazolo[4,3-c]pyridin-6-yl)-(azepan-1-yl)methanone D1. Purification by flash chromatography (cyclohexane/AcOEt : 10/0 to 0/10) afforded F6 as a yellow oil in quantitative yield. M/Z (M+H)⁺: 407/409

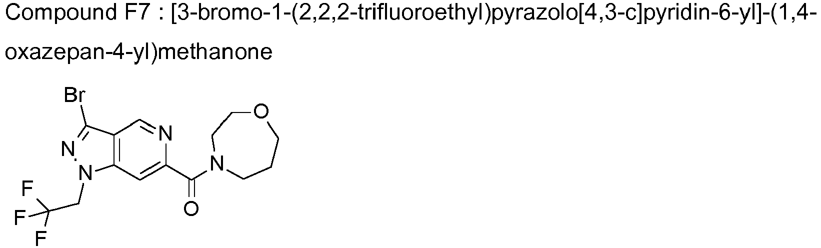

Compound F7 was obtained according to General Procedure III, Alternative 2, starting from 3-bromo-1H-pyrazolo[4,3-c]pyridin-6-yl)-(1,4-oxazepan-4-yl)methanone D2 and 2,2,2-trifluoroethyl trifluoromethanesulfonate E6. Purification by flash chromatography (DCM/MeOH: 10/0 to 9/1) afforded F7 as a white powder in 70% yield. M/Z (M+H)⁺: 407/409

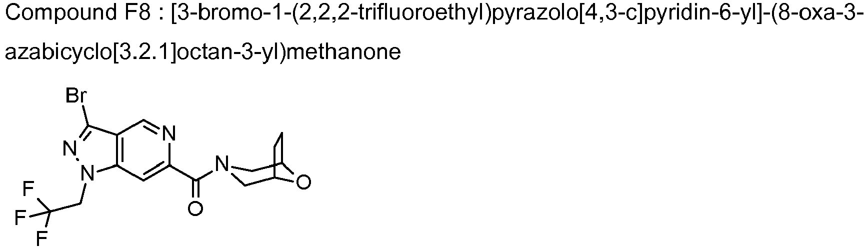

Compound F8 was obtained according to General Procedure III, starting from (3-bromo-1H-pyrazolo[4,3-c]pyridin-6-yl)-(8-oxa-3-azabicyclo[3.2.1]octan-3-yl)methanone D3 and 2,2,2-trifluoroethyl trifluoromethanesulfonate E6. Purification by flash chromatography (DCM/MeOH: 10/0 to 9/1) afforded F8 as a white powder in 75% yield. M/Z (M+H)⁺: 419/421

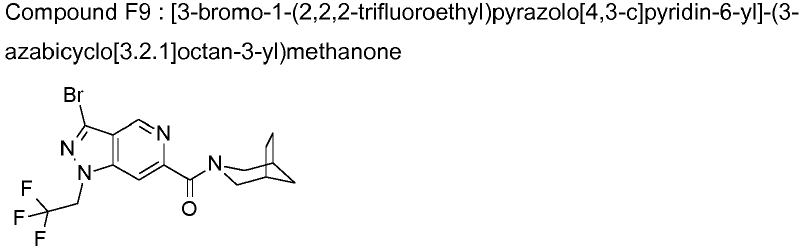

Compound F9 was obtained according to General Procedure III, starting from (3-bromo-1H-pyrazolo[4,3-c]pyridin-6-yl)-(3-azabicyclo[3.2.1]octan-3-yl)methanone D4 and 2,2,2-trifluoroethyl *p*-toluenesulfonate E3 (2 equiv.). Purification by flash chromatography (Cyclohexane/AcOEt: 10/0 to 3/7) afforded F9 as a white solid in 50% yield. M/Z (M+H)⁺: 417/419

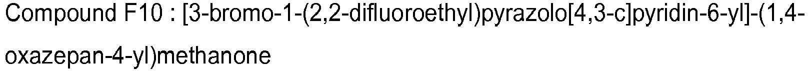

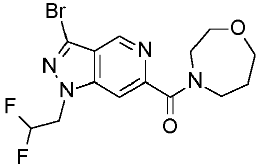

Compound F10 was obtained according to General Procedure III, Alternative 2, starting from 3-bromo-1H-pyrazolo[4,3-c]pyridin-6-yl)-(1,4-oxazepan-4-yl)methanone D2 and 1,1-difluoro-2-iodoethane E7. In that specific case, the reaction mixture was heated under microwave irradiation 30 min at 100°C then 30 min at 130°C. Purification by flash chromatography (DCM/MeOH: 100/0 to 95/5) afforded F10 as a white powder in 71% yield. M/Z (M+H)⁺: 389/391

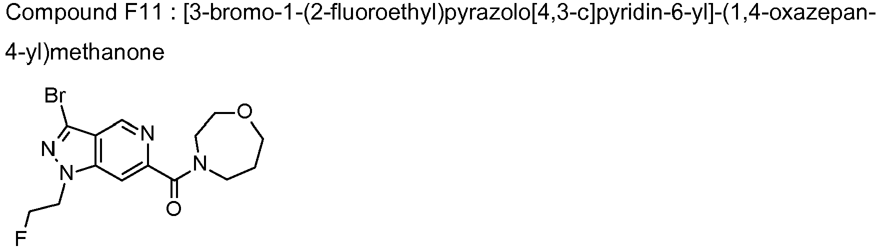

Compound F11 was obtained according to General Procedure III, Alternative 2, starting from 3-bromo-1H-pyrazolo[4,3-c]pyridin-6-yl)-(1,4-oxazepan-4-yl)methanone D2 and 1-fluoro-2-iodoethane E8 (1.5 equiv.). In that specific case, the reaction mixture was stirred 4h at rt then 30 min at 130°C under microwave irradiation. Purification by flash chromatography (DCM/MeOH: 10/0 to 9/1) afforded F11 as a colorless powder in 69% yield. M/Z (M+H)⁺: 371/373

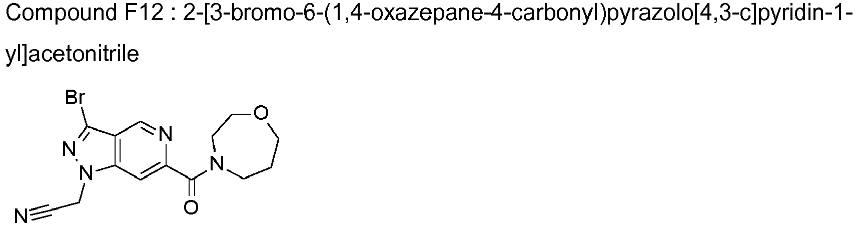

Compound F12 was obtained according to General Procedure III, starting from 3-bromo-1H-pyrazolo[4,3-c]pyridin-6-yl)-(1,4-oxazepan-4-yl)methanone D2 and bromoacetonitrile E9. Purification by flash chromatography (Cyclohexane/AcOEt: 5/5 to 0/10) afforded F12 as a beige powder in quantitative yield. M/Z (M+H)⁺: 364/366

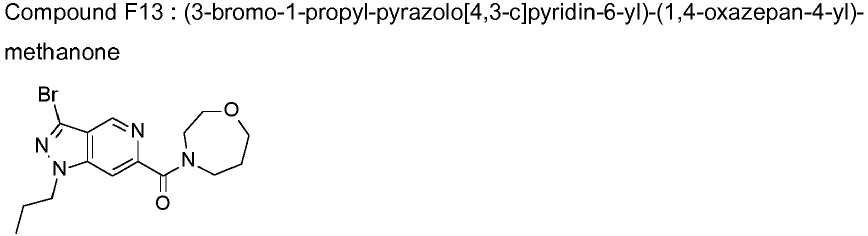

Compound F13 was obtained according to General Procedure III, starting from 3-bromo-1H-pyrazolo[4,3-c]pyridin-6-yl)-(1,4-oxazepan-4-yl)methanone D2 and 1-bromopropane E2. Purification by flash chromatography (DCM/MeOH: 10/0 to 9/1) afforded F13 as a beige powder in quantitative yield. M/Z (M+H)⁺: 367/369

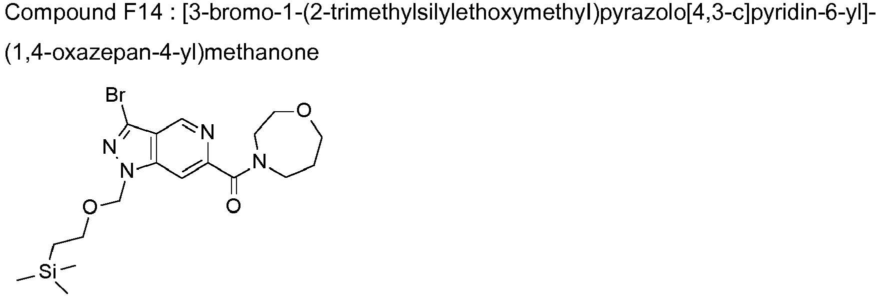

Compound F14 was obtained according to General Procedure III, starting from 3-bromo-1H-pyrazolo[4,3-c]pyridin-6-yl)-(1,4-oxazepan-4-yl)methanone D2 and 2-(trimethylsilyl)ethoxymethyl chloride E4. Purification by flash chromatography (Cyclohexane/AcOEt: 10/0 to 2/8) afforded the F14 as a colorless oil in 84% yield. M/Z (M+H)⁺: 455/457

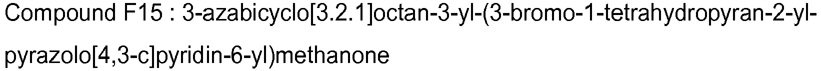

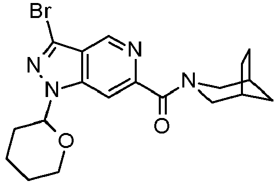

Compound F15 was obtained according to General Procedure III, Alternative 4, starting from (3-bromo-1H-pyrazolo[4,3-c]pyridin-6-yl)-(3-azabicyclo[3.2.1]octan-3-yl)methanone D4. Purification by flash chromatography (cyclohexane/AcOEt : 10/0 to 5/5) afforded the F15 as a beige powder in quantitative yield. M/Z (M+H)⁺: 419/421

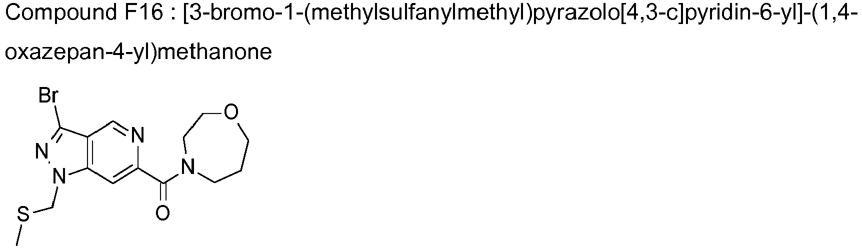

Compound F16 was obtained according to General Procedure III, starting from 3-bromo-1H-pyrazolo[4,3-c]pyridin-6-yl)-(1,4-oxazepan-4-yl)methanone D2 and chloromethyl sulfide E15. The reaction mixture was heated 2h at 85°C. Purification by flash chromatography (Cyclohexane/AcOEt: 2/8 to 0/10) afforded F16 as a yellow oil in 53% yield. M/Z (M+H)⁺: 385/387

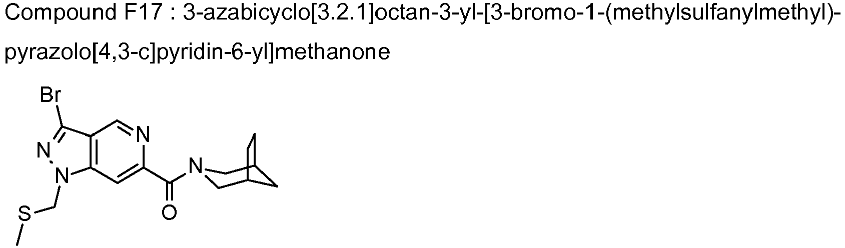

Compound F17 was obtained according to General Procedure III, starting from (3-bromo-1H-pyrazolo[4,3-c]pyridin-6-yl)-(3-azabicyclo[3.2.1]octan-3-yl)methanone D4 and chloromethyl sulfide E15. In that specific case, 1.5 equiv. of chloromethyl sulfide E15 and 1.3 equiv. of sodium hydride were used. The reaction mixture was heated 1h at 80°C. Purification by flash chromatography (DCM/MeOH: 100/0 to 95/5) afforded F17 as a yellow oil in 86% yield. M/Z (M+H)⁺: 395/397

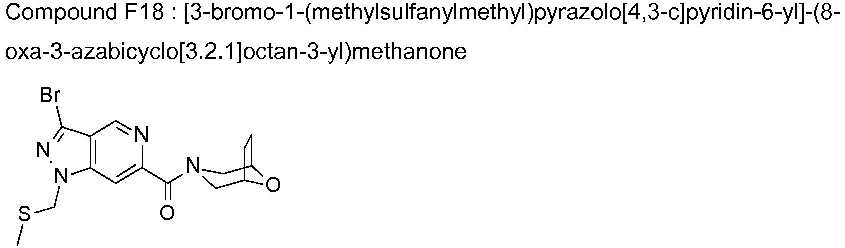

Compound F18 was obtained according to General Procedure III, starting from (3-bromo-1H-pyrazolo[4,3-c]pyridin-6-yl)-(8-oxa-3-azabicyclo[3.2.1]octan-3-yl)methanone D3 and chloromethyl sulfide E15 (1.5 equiv.). In that specific case, the reaction mixture was heated 2h at 85°C. Purification by flash chromatography (Cyclohexane/AcOEt: 7/3 to 0/10) afforded F18 as a yellow oil in 68% yield. M/Z (M+H)⁺: 375/399

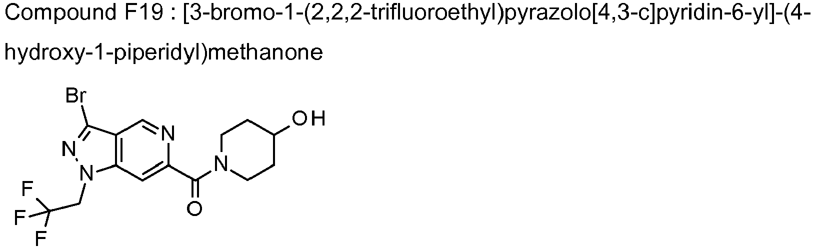

Compound F19 was obtained according to General Procedure III, Alternative 2, starting from (3-bromo-1H-pyrazolo[4,3-c]pyridin-6-yl)-(4-hydroxy-1-piperidyl)methanone D5 and 2,2,2-trifluoroethyl trifluoromethanesulfonate E6. Purification by flash chromatography (DCM/MeOH: 10/0 to 9/1) afforded F19 as a white powder in 91% yield. M/Z (M+H)⁺: 407/409

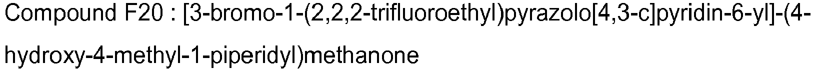

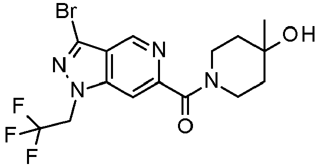

Compound F20 was obtained according to General Procedure III, Alternative 2, starting from (3-bromo-1H-pyrazolo[4,3-c]pyridin-6-yl)-(4-hydroxy-4-methyl-1-piperidyl)methanone D6 and 2,2,2-trifluoroethyl trifluoromethanesulfonate E6. Purification by flash chromatography (DCM/MeOH: 10/0 to 9/1) afforded F20 as a white powder in 68% yield. M/Z (M+H)⁺: 421/423

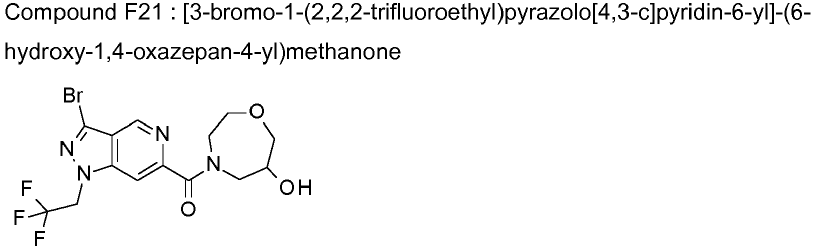

Compound F21 was obtained according to General Procedure III, Alternative 2, starting from (3-bromo-1H-pyrazolo[4,3-c]pyridin-6-yl)-(6-hydroxy-1,4-oxazepan-4-yl)methanone D7 and 2,2,2-trifluoroethyl trifluoromethanesulfonate E6. Purification by flash chromatography (DCM/MeOH: 10/0 to 9/1) afforded F21 in 76% yield. M/Z (M+H)⁺: 423/425

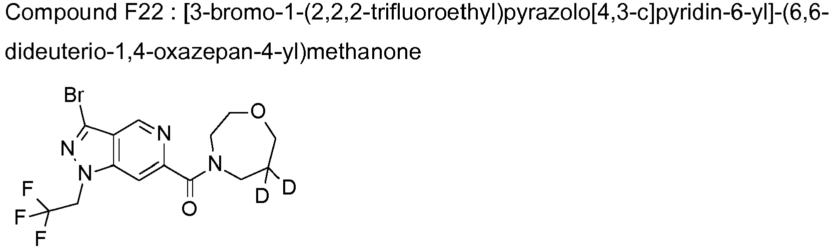

Compound F22 was obtained according to General Procedure III, Alternative 2, starting from (3-bromo-1H-pyrazolo[4,3-c]pyridin-6-yl)-(6,6-dideuterio-1,4-oxazepan-4-yl)methanone D8. Purification by flash chromatography (DCM/MeOH: 10/0 to 9/1) then on an Interchim silica cartridge (15µm) (DCM/AcOEt: 10/0 to 5/5 then DCM/AcOEt 5/5 to DCM/MeOH 9/1) afforded F22 as a white powder in 39% yield. M/Z (M+H)⁺: 409/411

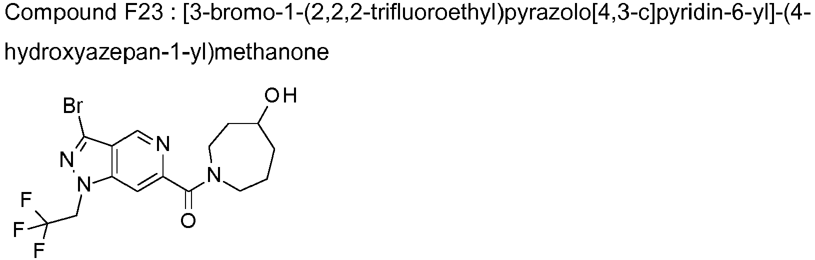

Compound F23 was obtained according to General Procedure III, Alternative 2, starting from (3-bromo-1H-pyrazolo[4,3-c]pyridin-6-yl)-(4-hydroxyazepan-1-yl)methanone D9 and 2,2,2-trifluoroethyl trifluoromethanesulfonate E6. Purification by flash chromatography (DCM/MeOH: 10/0 to 9/1) afforded F23 in 67% yield. M/Z (M+H)⁺: 421/423

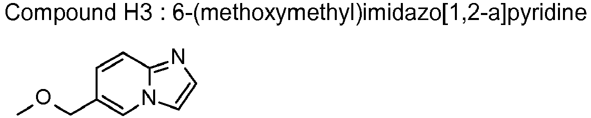

Compound H3 was obtained according to General Procedure III, starting from 6-(hydroxymethyl)imidazo[1,2-a]pyridine H37 and methyl iodide E1. Purification by flash chromatography (DCM/MeOH: 100/0 to 95/5) afforded H3 as a yellow powder in quantitative yield. M/Z (M+H)⁺: 163

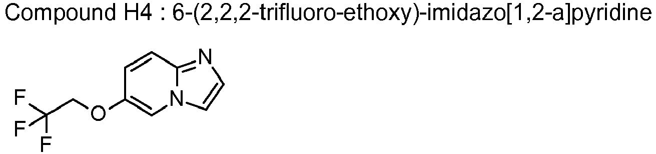

Compound H4 was obtained according to General Procedure III, Alternative 2, starting from 6-hydroxy-imidazo[1,2-a]pyridine H33 and 2,2,2-trifluoroethyl *p-*toluenesulfonate E3. In that specific case, the reaction mixture was performed in ACN. Purification by flash chromatography (DCM/MeOH: 100/0 to 95/5) afforded H4 in 23% yield. M/Z (M+H)⁺: 217

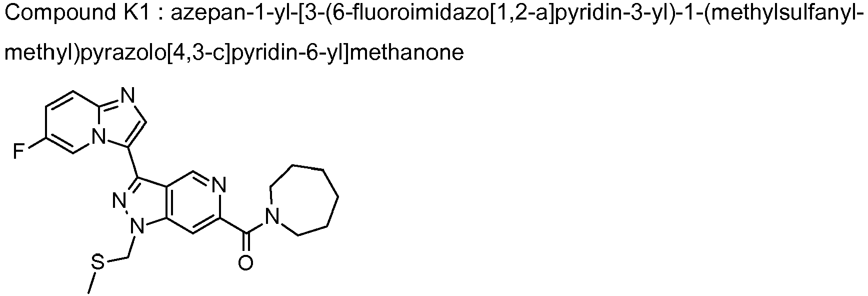

Compound K1 was obtained according to General Procedure III, starting from azepan-1-yl-[3-(6-fluoroimidazo[1,2-a]pyridin-3-yl)-1H-pyrazolo[4,3-c]pyridin-6-yl]methanone J2 and chloromethyl methyl sulfide E15. Purification by flash chromatography (DCM/MeOH: 100/0 to 95/5) afforded K1 as a beige powder in 60% yield. M/Z (M+H)⁺: 439

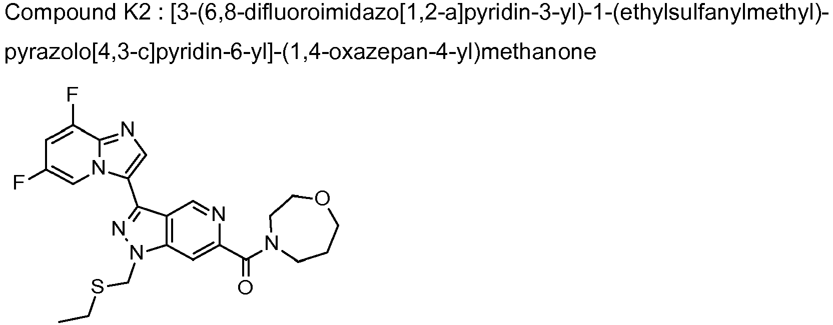

Compound K2 was obtained according to General Procedure III, starting from [3-(6,8-difluoroimidazo[1,2-a]pyridin-3-yl)-1H-pyrazolo[4,3-c]pyridin-6-yl]-(1,4-oxazepan-4-yl)methanone J4 and chloromethylsulfanyl ethane E16 (1.5 equiv.). An aqueous work-up afforded K2 as a brown solid in 73% yield without further purification. M/Z (M+H)⁺: 473

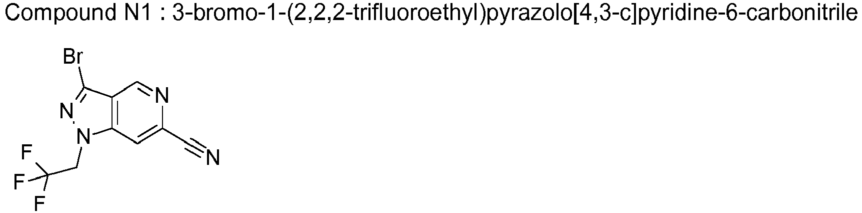

Compound N1 was obtained according to General Procedure III, starting from 3-bromo-1H-pyrazolo[4,3-c]pyridine-6-carbonitrile M and 2,2,2-trifluoroethyl *p-*toluenesulfonate E3 (1.5 equiv.). In that specific case, the reaction mixture was stirred 5h at 150°C. Purification by flash chromatography (Cyclohexane/AcOEt: 10/0 to 7/3) afforded N1 as a white powder in 50% yield. M/Z (M+H)⁺: 305/307

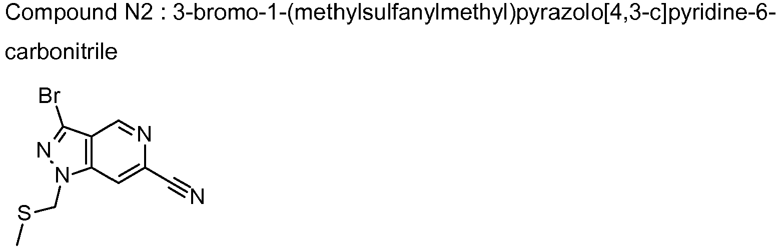

Compound N2 was obtained according to General Procedure III, starting from 3-bromo-1H-pyrazolo[4,3-c]pyridine-6-carbonitrile M and chloromethyl methyl sulfide E15 (1.5 equiv.). In that specific case, the reaction mixture was stirred 1h at 80°C. Purification by flash chromatography (Cyclohexane/AcOEt: 10/0 to 7/3) afforded N2 in 74% yield. M/Z (M+H)⁺: 283/285

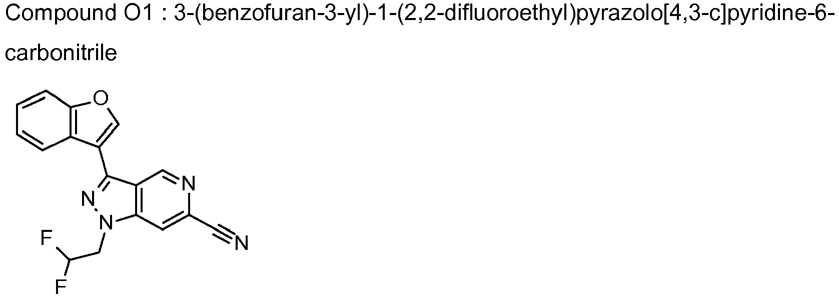

Compound O1 was obtained according to General Procedure III, Alternative 3, starting from 3-(benzofuran-3-yl)-1H-pyrazolo[4,3-c]pyridine-6-carbonitrile S and 2,2-difluoroethane E7 (1.5 equiv.). Precipitation afforded O1 as a beige powder in 86% yield. M/Z (M+H)⁺: 325

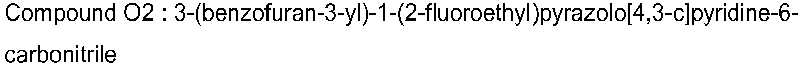

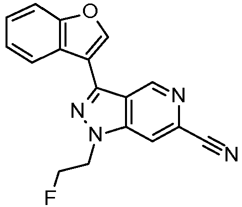

Compound O2 was obtained according to General Procedure III, starting from 3-(benzofuran-3-yl)-1H-pyrazolo[4,3-c]pyridine-6-carbonitrile S and 1-Fluoro-2-iodoethane E8 (1.3 equiv.). In that specific case, The reaction mixture was quenched by addition of a saturated ammonium chloride solution. The formed precipitate was washed with water then dried under vacuum with phosphorus pentoxide to afford O2 as a beige powder in 52% yield. M/Z (M+H)⁺: 307

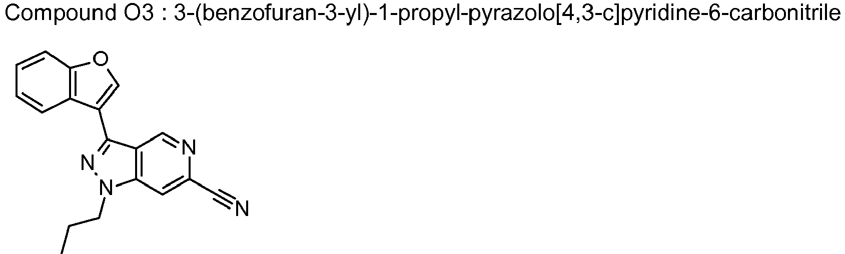

Compound O3 was obtained according to General Procedure III, starting from 3-(benzofuran-3-yl)-1H-pyrazolo[4,3-c]pyridine-6-carbonitrile S and 1-bromopropane E2 (1.5 equiv.). In that specific case, the reaction mixture was quenched by addition of a saturated ammonium chloride solution. The formed precipitate was washed with water then dried under vacuum with phosphorus pentoxide to afford O3 as a beige powder in quantitative yield. M/Z (M+H)⁺: 303

### Compound 1 : [3-(1-Methyl-1H-indol-3-yl)-1-(2,2,2-trifluoro-ethyl)-1H-pyrazolo[4,3-c]-pyridine-6-yl]-1,4-oxazepan-4-yl-methanone

Compound 1 was obtained according to General Procedure III, from [3-(1H-Indol-3-yl)-1-(2,2,2-trifluoro-ethyl)-1H-pyrazolo[4,3-c]pyridine-6-yl]-1,4-oxazepan-4-yl-methanone 96 and methyl iodide E1. Purification by flash chromatography (DCM/MeOH: 10/0 to 9/1) afforded 1 as a brown powder in 29% yield. ¹H-NMR (DMSO-*d₆*, 400MHz): 1.77-1.82 (m, 1H, CH₂); 1.92-1.98 (m, 1H, CH₂); 3.49-3.54 (m, 2H, N-CH₂); 3.66-3.69 (m, 1H, N-CH₂); 3.71-3.80 (m, 5H, N-CH₂, O-CH₂); 3.94 (s, 3H, N-CH₃); 5.60 (q, *J* 9.1 Hz, 2H, CF₃-CH₂); 7.20-7.25 (t, *J* 7.2 Hz, 1H, Ar); 7.28-7.32 (td, *J* 8.2, 0.9 Hz, 1H, Ar); 7.56 (d, *J* 8.2 Hz, 1H, Ar); 8.10 (bs, 1H, signal of a rotamer, Ar); 8.11 (bs, 1H, signal of a rotamer, Ar); 8.34-8.37 (m, 1H, Ar); 8.50 (s, 1H, signal of a rotamer, Ar); 8.51 (s, 1H, signal of a rotamer, Ar); 9.52 (d, *J* 1.0 Hz, 1H, signal of a rotamer, Ar); 9.54 (d, *J* 1.0 Hz, 1H, signal of a rotamer, Ar). M/Z (M+H)⁺: 458

### Compound 2 : Azepan-1-yl-[3-(6-fluoro-imidazo[1,2-a]pyridin-3-yl)-1-(2-methanesulfonyl-ethyl)-1H-pyrazolo[4,3-c]pyridin-6-yl]-methanone

To a solution of azepan-1-yl-[3-(6-fluoroimidazo[1,2-a]pyridin-3-yl)-1H-pyrazolo[4,3-c]-pyridin-6-yl]methanone J2 (1 equiv.) in DMA (0.1M) were added methyl vinyl sulfone (1 equiv.) and cesium carbonate (2 equiv.). The reaction mixture was stirred 48h at rt. The reaction mixture was diluted with AcOEt, washed with a saturated ammonium chloride solution and brine, dried over magnesium sulfate then concentrated. Purification by flash chromatography (DCM/MeOH: 10/0 to 8/2) afforded a mixture of starting material and expected product. Methyl vinyl sulfone (1 equiv.) and cesium carbonate (2 equiv.) were added to the resulting mixture dissolved in DMA (0.1M). The reaction mixture was stirred 48h at rt. The reaction mixture was diluted with AcOEt, washed with a saturated ammonium chloride solution and brine, dried over magnesium sulfate then concentrated. Purification by flash chromatography (DCM/MeOH: 100/0 to 85/15) afforded 2 as a beige powder in 12% yield. ¹H-NMR (DMSO-*d₆*, 400MHz): 1.54-1.64 (m, 6H, CH₂); 1.75-1.81 (m, 2H, CH₂); 3.03 (s, 3H CH₃); 3.36-3.39 (m, 2H, N-CH₂); 3.62-3.65 (m, 2H, N-CH₂); 3.95 (t, *J* 6.6 Hz, 2H, CH₂); 5.04 (t, *J* 6.6 Hz, 2H, CH₂); 7.58 (ddd, *J* 10.1, 8.2, 2.5 Hz, 1H, Ar); 7.88 (ddd, *J* 10.1, 5.2, 0.5 Hz, 1H, Ar); 8.04 (d, *J* 1.0 Hz, 1H, Ar); 8.78 (s, 1H, Ar); 9.57 (d, *J* 1.0 Hz, 1H, Ar); 9.67 (ddd, *J* 5.2, 2.5, 0.5 Hz, 1H, Ar). M/Z (M+H)⁺: 485

### Compound 3 : Azepan-1-yl-[1-(2,2-difluoro-ethyl)-3-(6-fluoro-imidazo[1,2-a]pyridin-3-yl)-1H-pyrazolo[4,3-c]pyridin-6-yl]-methanone

Compound 3 was obtained according to General Procedure III, starting from azepan-1-yl-[3-(6-fluoroimidazo[1,2-a]pyridin-3-yl)-1H-pyrazolo[4,3-c]pyridin-6-yl]methanone J2 and 2,2-difluoroiodoethane E7 (1.5 equiv.). In that specific case, the reaction mixture was stirred 1h at 70°C. Purification by flash chromatography (DCM/MeOH: 100/0 to 94/6) afforded 3 as a white powder in 45% yield. ¹H-NMR (DMSO-*d₆*, 400MHz): 1.54-1.64 (m, 6H, CH₂); 1.75-1.81 (m, 2H, CH₂); 3.36-3.38 (m, 2H, N-CH₂); 3.63-3.66 (m, 2H, N-CH₂); 5.19 (td, *J* 15.3, 3.3 Hz, 2H, CH₂-CHF₂); 6.62 (tt, *J* 54.5, 3.3 Hz, 1H, CHF₂); 7.58 (ddd, *J* 9.8, 8.1, 2.5 Hz, 1H, Ar); 7.89 (dd, *J* 9.8, 5.3 Hz, 1H, Ar); 8.05 (d, *J* 1.0 Hz, 1H, Ar); 8.80 (s, 1H, Ar); 9.60 (d, *J* 1.0 Hz, 1H, Ar); 9.63 (dd, *J* 4.9, 2.5 Hz, 1H, Ar). M/Z (M+H)⁺: 443

### Compound 4 : Azepan-1-yl-[1-(2-fluoro-ethyl)-3-(6-fluoro-imidazo[1,2-a]pyridin-3-yl)-1H-pyrazolo[4,3-c]pyridin-6-yl]-methenone

Compound 4 was obtained according to General Procedure III, starting from azepan-1-yl-[3-(6-fluoroimidazo[1,2-a]pyridin-3-yl)-1H-pyrazolo[4,3-c]pyridin-6-yl]methanone J2 and 1-fluoro-2-iodoethane E8 (1.5 equiv.). Purification by flash chromatography (DCM/MeOH: 100/0 to 94/6) afforded 4 as a white powder in 46% yield. ¹H-NMR (DMSO-*d₆*, 400MHz): 1.54-1.64 (m, 6H, CH₂); 1.75-1.81 (m, 2H, CH₂); 3.36-3.39 (m, 2H, N-CH₂); 3.62-3.65 (m, 2H, N-CH₂); 4.88-5.10 (m, 4H, CH₂-CH₂F); 7.57 (ddd, *J* 9.9, 8.2, 2.4 Hz, 1H, Ar); 7.88 (dd, *J* 9.9, 5.2 Hz, 1H, Ar); 7.99 (d, *J* 1.0 Hz, 1H, Ar); 8.78 (s, 1H, Ar); 9.58 (d, *J* 1.0 Hz, 1H, Ar); 9.62 (dd, *J* 4.9, 2.4 Hz, 1H, Ar). M/Z (M+H)⁺: 425

### Compound 5 : Azepan-1-yl-[3-(6-fluoro-imidazo[1,2-a]pyridin-3-yl)-1-propyl-1H-pyrazolo[4,3-c]pyridin-6-yl]-methanone

Compound 5 was obtained according to General Procedure III, starting from azepan-1-yl-[3-(6-fluoroimidazo[1,2-a]pyridin-3-yl)-1H-pyrazolo[4,3-c]pyridin-6-yl]methanone J2 and 1-bromopropane E2 (1.3 equiv.). Purification by precipitation from the reaction mixture (water addition then filtration) afforded 5 as a beige powder in 63% yield. ¹H-NMR (DMSO-*d₆*, 400MHz): 0.91 (t, *J* 7.4 Hz, 3H, CH₃); 1.54-1.64 (m, 6H, CH₂); 1.75-1.81 (m, 2H, CH₂); 1.92-2.00 (m, 2H, CH₂); 3.36-3.38 (m, 2H, N-CH₂); 3.62-3.65 (m, 2H, N-CH₂); 4.57 (t, *J* 6.9 Hz, 2H, CH₂); 7.53-7.61 (m, 1H, Ar); 7.88 (dd, *J* 10.0, 5.3 Hz, 1H, Ar); 8.01 (bs, 1H, Ar); 8.76 (s, 1H, Ar); 9.55-9.64 (m, 2H, Ar). M/Z (M+H)⁺: 421

### Compound 6 : Azepan-1-yl-[3-(6-fluoro-imidazo[1,2-a]pyridin-3-yl)-1-methoxymethyl-1H-pyrazolo[4,3-c]pyridin-6-yl]-methanone

Compound 6 was obtained according to General Procedure III, starting from azepan-1-yl-[3-(6-fluoroimidazo[1,2-a]pyridin-3-yl)-1H-pyrazolo[4,3-c]pyridin-6-yl]methanone J2 and chloromethyl methyl ether E10 (1.3 equiv.). Purification by flash chromatography (DCM/MeOH: 100/0 to 95/5) afforded 6 as a beige powder in 58% yield. ¹H-NMR (DMSO-*d₆*, 400MHz): 1.54-1.64 (m, 6H, CH₂); 1.75-1.81 (m, 2H, CH₂); 3.34 (s, 3H, CH₃); 3.36-3.38 (m, 2H, N-CH₂); 3.62-3.65 (m, 2H, N-CH₂); 5.96 (s, 2H, CH₂); 7.57-7.62 (m, 1H, Ar); 7.90 (dd, *J* 9.8, 5.4 Hz, 1H, Ar); 8.09 (bs, 1H, Ar); 8.83 (s, 1H, Ar); 9.61-9.63 (m, 2H, Ar). M/Z (M+H)⁺: 423

### Compound 7 : [6-(Azepane-1-carbonyl)-3-(6-fluoro-imidazo[1,2-a]pyridin-3-yl)-pyrazolo[4,3-c]pyridin-1-yl]-acetonitrile

Compound 7 was obtained according to General Procedure III, starting from azepan-1-yl-[3-(6-fluoroimidazo[1,2-a]pyridin-3-yl)-1H-pyrazolo[4,3-c]pyridin-6-yl]methanone J2 and bromoacetonitrile E9 (1.5 equiv.). Purification by flash chromatography (DCM/MeOH: 10/0 to 8/2) afforded 7 as a beige powder in 51% yield. ¹H-NMR (DMSO-*d₆*, 400MHz): 1.54-1.65 (m, 6H, CH₂); 1.75-1.81 (m, 2H, CH₂); 3.32-3.39 (m, 2H, N-CH₂); 3.63-3.66 (m, 2H, N-CH₂); 6.03 (s, 2H, CH₂); 7.59-7.64 (m, 1H, Ar); 7.92 (dd, *J* 9.7, 5.3 Hz, 1H, Ar); 8.13 (bs, 1H, Ar); 8.86 (s, 1H, Ar); 9.59-9.62 (m, 1H, Ar); 9.65 (bs, 1H, Ar). M/Z (M+H)⁺: 418

### Compound 8 : Azepan-1-yl-[3-(6,8-difluoro-imidazo[1,2-a]pyridin-3-yl)-1-(2-methoxyethyl)-1H-pyrazolo[4,3-c]pyridin-6-yl]-methanone

Compound 8 was obtained according to General Procedure III, starting from azepan-1-yl-[3-(6,8-difluoroimidazo[1,2-a]pyridin-3-yl)-1H-pyrazolo[4,3-c]pyridin-6-yl]methanone J3 and 2-bromoethyl methyl ether E11 (1.1 equiv.). Purification by flash chromatography (DCM/MeOH: 100/0 to 85/15) afforded 8 as a white powder in 81% yield. ¹H-NMR (DMSO-*d₆*, 400MHz): 1.54-1.64 (m, 6H, CH₂); 1.75-1.81 (m, 2H, CH₂); 3.22 (s, 3H, CH₃); 3.35-3.38 (m, 2H, N-CH₂); 3.61-3.67 (m, 2H, 2H, N-CH₂); 3.86 (t, *J* 5.1 Hz, 2H, N-CH₂-CH₂-O); 4.80 (t, *J* 5.1 Hz, 2H, N-CH₂-CH₂-O); 7.76 (ddd, *J* 11.1, 9.1, 2.1 Hz, 1H, Ar); 7.97 (d, *J* 1.1 Hz, 1H, Ar); 8.79 (s, 1H, Ar); 9.51 (ddd, *J* 4.8, 2.1, 0.8 Hz, 1H, Ar); 9.56 (d, *J* 1.1 Hz, 1H, Ar). M/Z (M+H)⁺: 455

### Compound 9 : [1-Cyclopropylmethyl-3-(6,8-difluoro-imidazo[1,2-a]pyridin-3-yl)-1H-pyrazolo[4,3-c]pyridin-6-yl]-1,4-oxazepan-4-yl-methanone

Compound 9 was obtained according to General Procedure III, starting from [3-(6,8-difluoroimidazo[1,2-a]pyridin-3-yl)-1H-pyrazolo[4,3-c]pyridin-6-yl]-(1,4-oxazepan-4-yl)methanone J4 and (iodomethyl)cyclopropane E12 (1.3 equiv.). Purification by flash chromatography (DCM/MeOH: 100/0 to 85/15) afforded 9 as a white powder in 48% yield. ¹H-NMR (DMSO-*d₆*, 400MHz): 0.49-0.58 (m, 4H, CH₂); 1.38-1.48 (m, 1H, CH); 1.79 (quint, *J* 5.8 Hz, 1H, CH₂); 1.95 (quint, *J* 5.8 Hz, 1H, CH₂); 3.49-3.53 (m, 2H, N-CH₂); 3.64-3.79 (m, 6H, N-CH₂ + 2 O-CH₂); 4.52 (d, *J* 7.1 Hz, 2H, N-CH₂-Cyclopropyl); 7.76 (ddd, *J* 11.0, 9.2, 2.0 Hz, 1H, Ar); 8.10 (s, 1H, Ar); 8.79 (s, 1H, signal of a rotamer, Ar); 8.80 (s, 1H, signal of a rotamer, Ar); 9.53 (m, 1H, Ar); 9.58 (d, *J* 0.9 Hz, 1H, signal of a rotamer, Ar); 9.59 (d, *J* 0.9 Hz, 1H, signal of a rotamer, Ar). M/Z (M+H)⁺: 453

### Compound 10 : [3-(6,8-Difluoro-imidazo[1,2-a]pyridin-3-yl)-1-methanesulfonyl-1H-pyrazolo[4,3-c]pyridin-6-yl]-1,4-oxazepan-4-yl-methanone

Compound 10 was obtained according to General Procedure III, starting from [3-(6,8-difluoroimidazo[1,2-a]pyridin-3-yl)-1H-pyrazolo[4,3-c]pyridin-6-yl]-(1,4-oxazepan-4-yl)methanone J4 and methanesulfonylchloride E13 (1.3 equiv.). Purification by flash chromatography (DCM/MeOH: 100/0 to 95/5) afforded 10 as a white powder in 40% yield. ¹H-NMR (DMSO-*d₆*, 400MHz): 1.81 (quint, *J* 5.7 Hz, 1H, CH₂); 1.92-1.98 (m, 1H, CH₂); 3.52-3.56 (m, 2H, N-CH₂); 3.67-3.79 (m, 9H, N-CH₂, O-CH₂, CH₃); 7.87-7.92 (m, 1H, Ar); 8.09 (bs, 1H, Ar); 9.02 (s, 1H, signal of a rotamer, Ar); 9.03 (s, 1H, signal of a rotamer, Ar); 9.46-9.47 (m, 1H, Ar); 9.78 (bs, 1H, signal of a rotamer, Ar); 9.79 (bs, 1H, signal of a rotamer, Ar). M/Z (M+H)⁺: 477

### Compound 11 : 3-[3-(6,8-Difluoro-imidazo[1,2-a]pyridin-3-yl)-6-(1,4-oxazepane-4-carbonyl)-pyrazolo[4,3-c]pyridin-1-ylmethyl]-azetidine-1-carboxylic acid tert-butyl ester

Compound 11 was obtained according to General Procedure III, starting from [3-(6,8-difluoroimidazo[1,2-a]pyridin-3-yl)-1H-pyrazolo[4,3-c]pyridin-6-yl]-(1,4-oxazepan-4-yl)methanone J4 and tert-butyl 3-(methylsulfonyloxymethyl)azetidine-1-carboxylate E14. In that specific case, 2.2 equiv. of sodium hydride were used. Purification by flash chromatography (DCM/MeOH: 100/0 to 95/5) afforded 11 as a beige powder in 26% yield. ¹H-NMR (DMSO-*d₆*, 400MHz): 1.33 (s, 9H, CH₃); 1.78 (quint, *J* 5.9 Hz, 1H, CH₂); 1.94 (quint, *J* 5.9 Hz, 1H, CH₂); 3.19-3.23 (m, 1H, CH); 3.49-3.54 (m, 2H, N-CH₂); 3.64-3.84 (m, 8H, CH₂); 3.91-4.00 (m, 2H, CH₂); 4.85 (d, *J* 7.1 Hz, 2H, N-CH₂-CH); 7.78 (ddd, *J* 11.0, 9.1, 2.0 Hz, 1H, Ar); 8.17 (bs, 1H, Ar); 8.79 (s, 1H, signal of a rotamer, Ar); 8.80 (s, 1H, signal of a rotamer, Ar); 9.46-9.47 (m, 1H, Ar); 9.59 (d, *J* 1.0 Hz, signal of a rotamer, 1H, Ar); 9.60 (d, *J* 1.0 Hz, signal of a rotamer, 1H, Ar). M/Z (M+H)⁺: 568

### Compound 12 : [3-(6,8-Difluoro-imidazo[1,2-a]pyridin-3-yl)-1-hydroxymethyl-1H-pyrazolo[4,3-c]pyridin-6-yl]-1,4-oxazepan-4-yl-methanone

A solution of [3-(6,8-difluoroimidazo[1,2-a]pyridin-3-yl)-1H-pyrazolo[4,3-c]pyridin-6-yl]-(1,4-oxazepan-4-yl)methanone J4 (1 equiv.) in an ethanol/formaldehyde 40% in water mixture (1/1, 0.15M) was stirred overnight at 85°C. The formed precipitate was filtered, then dissolved in a DCM/MeOH mixture. DCM was removed by concentration. The residual solution was cooled down to 0°C. The formed precipitate was filtered, washed with cold methanol then dried under vacuum to afford 12 as a white powder in 29% yield. ¹H-NMR (DMSO-*d₆*, 400MHz): 1.77-1.82 (m, 1H, CH₂); 1.92-1.98 (m, 1H, CH₂); 3.51-3.55 (m, 2H, N-CH₂); 3.66-3.79 (m, 6H, N-CH₂, O-CH₂); 5.93 (d, *J* 7.5 Hz, 2H, HO-CH₂); 7.03-7.13 (t, *J* 7.5 Hz, 1H, HO-CH₂); 7.78 (ddd, *J* 10.9, 9.1, 2.0 Hz, 1H, Ar); 8.09-8.10 (m, 1H, Ar); 8.81 (s, 1H, signal of a rotamer, Ar); 8.82 (s, 1H, signal of a rotamer, Ar); 9.59-9.62 (m, 2H, Ar). M/Z (M+H)⁺: 399

### Compound 13 : [3-Benzofuran-3-yl-6-(1,4-oxazepane-4-carbonyl)-pyrazolo[4,3-c]pyridin-1-yl]-acetonitrile

Compound 13 was obtained according to General Procedure III, starting from [3-(benzofuran-3-yl)-1H-pyrazolo[4,3-c]pyridin-6-yl]-(1,4-oxazepan-4-yl)methanone J1 and bromoacetonitrile E9 (1.3 equiv.). Purification by flash chromatography (DCM/MeOH: 100/0 to 97/3) afforded 13 as a beige powder in 16% yield. ¹H-NMR (DMSO-*d₆*, 400MHz): 1.79 (quint, *J* 5.7 Hz, 1H, CH₂); 1.90-1.98 (m, 2H, CH₂); 3.48-3.56 (m, 2H, CH₂); 3.64-3.69 (m, 1H, CH₂); 3.70-3.82 (m, 5H, CH₂); 6.00 (s, 2H, CH₂-CN); 7.44-7.51 (m, 2H, Ar); 7.72-7.78 (m, 1H, Ar); 8.17 (d, *J* 1.0 Hz, 1H, signal of a rotamer, Ar); 8.18 (d, *J* 1.0 Hz, 1H, signal of a rotamer, Ar); 8.35-8.40 (m, 1H, Ar); 9.19 (s, 1H, signal of a rotamer, Ar); 9.20 (s, 1H, signal of a rotamer, Ar); 9.58 (d, *J* 1.0 Hz, 1H, Ar); 9.60 (d, *J* 1.0 Hz, 1H, Ar). M/Z (M+H)⁺: 402

### Compound 14 : (3-Aza-bicyclo[3.2.1]oct-3-yl)-[3-(6,8-difluoro-imidazo[1,2-a]pyridin-3-yl)-1-methylsulfanylmethyl-1H-pyrazolo[4,3-c]pyridin-6-yl]-methanone

Compound 14 was obtained according to General Procedure III, starting from 3-azabicyclo[3.2.1]octan-3-yl-[3-(6,8-difluoroimidazo[1,2-a]pyridin-3-yl)-1H-pyrazolo[4,3-c]pyridin-6-yl]methanone J5 and chloromethyl methyl sulfide E15 (1.5 equiv.). Purification by flash chromatography (Cyclohexane/AcOEt: 5/5 to 0/10) afforded 14 as a white powder in 52% yield. ¹H-NMR (DMSO-*d₆*, 400MHz): 1.49-1.72 (m, 6H, CH₂); 2.04-2.11 (m, 1H, CH₂); 2.20 (s, 3H, SCH₃); 2.29-2.35 (m, 1H, CH₂); 2.87 (d, *J* 12.5 Hz, 1H, N-CH₂); 3.15 *(d, J* 12.5 Hz, 1H, N-CH₂); 3.32-3.38 (m, 1H, N-CH₂); 4.36 (d, *J* 12.5 Hz, 1H, N-CH₂); 5.87 (s, 2H, CH₂-SCH₃); 7.80 (ddd, *J* 11.0, 9.2, 2.2 Hz, 1H, Ar); 8.12 (d, *J* 1.0 Hz, 1H, Ar); 8.82 (s, 1H, Ar); 9.50-9.53 (m, 1H, Ar); 9.61 (d, *J* 1.0 Hz, 1H, Ar). M/Z (M+H)⁺: 469

### General Procedure IV : Synthesis of compounds and compounds F from compounds K and F respectively (scheme 1&2)

To a solution of compound F or K (1 equiv.) in DCM (0.1M) was added *m*CPBA (3 equiv.). The reaction mixture was stirred 15 min at rt.. The reaction mixture was quenched with a saturated sodium thiosulfate solution, extracted with AcOEt. The organic phase was washed with a saturated sodium bicarbonate solution and brine, dried over magnesium sulfate then concentrated. The resulting crude mixture was purified by flash chromatography to afford the compound or compound F.

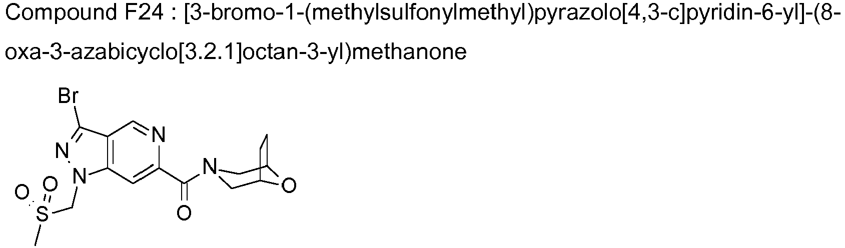

Compound F24 was obtained according to General Procedure IV, starting from [3-bromo-1-(methylsulfanylmethyl)pyrazolo[4,3-c]pyridin-6-yl]-(8-oxa-3-azabicyclo[3.2.1]octan-3-yl)methanone F18. In that specific case, the reaction mixture was stirred 15min at 150°C under microwave irradiation. Purification by flash chromatography (DCM/MeOH: 100/0 to 95/5) afforded F24 as a colorless oil in 51% yield. M/Z (M+H)⁺: 429/431

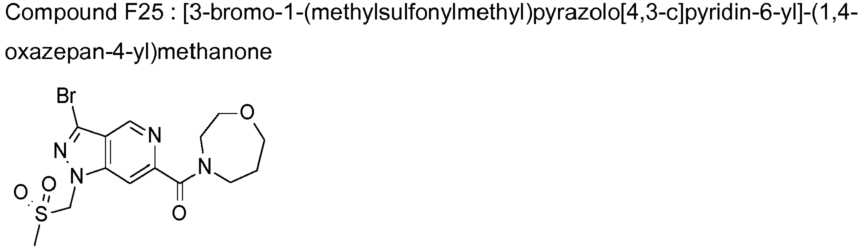

Compound F25 was obtained according to General Procedure IV, starting from [3-bromo-1-(methylsulfanylmethyl)pyrazolo[4,3-c]pyridin-6-yl]-(1,4-oxazepan-4-yl)methanone F16. Purification by flash chromatography (Cyclohexane/AcOEt: 10/0 to 0/10) afforded F25 as a white solid in 74% yield. M/Z (M+H)⁺: 417/419

### Compound 15 : [3-(6,8-Difluoro-imidazo[1,2-a]pyridin-3-yl)-1-methanesulfonylmethyl-1H-pyrazolo[4,3-c]pyridin-6-yl]-1,4-oxazepan-4-yl-methanone

Compound 15 was obtained according to General Procedure IV, starting from [3-(6,8-difluoroimidazo[1,2-a]pyridin-3-yl)-1-(methylsulfanylmethyl)pyrazolo[4,3-c]pyridin-6-yl]-(1,4-oxazepan-4-yl)methanone K7. Purification by flash chromatography (DCM/MeOH: 100/0 to 95/5) afforded 15 as a yellow solid in 8% yield. ¹H-NMR (DMSO-*d₆*, 400MHz): 1.78 (m, 1H, CH₂); 1.95 (m, 1H, CH₂); 3.14 (s, 3H, CH₃); 3.47-3.53 (m, 2H, CH₂); 3.63-3.82 (m, 6H, CH₂); 6.34 (s, 2H, SO₂-CH₂); 7.81 (ddd, *J* 11.1, 9.1, 2.2 Hz, 1H, Ar); 8.20 (d, *J* 1.0 Hz, 1H, Ar, signal of a rotamer); 8.21 (d, *J* 1.0 Hz, 1H, Ar, signal of a rotamer); 8.89 (s, 1H, Ar, signal of a rotamer); 8.90 (s, 1H, Ar, signal of a rotamer); 9.51-9.56 (m, 1H, Ar); 9.67 (d, *J* 1.0 Hz, 1H, Ar, signal of a rotamer); 9.69 (d, *J* 1.0 Hz, 1H, Ar, signal of a rotamer). M/Z (M+H)⁺: 491

### Compound 16 : (3-Benzofuran-3-yl-1-methanesulfonylmethyl-1H-pyrazolo[4,3-c]pyridine-6-yl)- 1,4-oxazepan-4-yl-methanone

Compound 16 was obtained according to General Procedure IV, starting from [3-(benzofuran-3-yl)-1-(methylsulfanylmethyl)pyrazolo[4,3-c]pyridin-6-yl]-(1,4-oxazepan-4-yl)methanone K12. Purification by flash chromatography (DCM/MeOH: 100/0 to 95/5) afforded 16 as a beige solid in 35% yield. ¹H-NMR (DMSO-*d₆*, 400MHz): 1.75-1.81 (quint, *J* 5.9 Hz, 1H, CH₂); 1.92-1.98 (quint, *J* 5.9 Hz, 1H, CH₂); 3.17 (s, 3H, SO₂-CH₃); 3.48-3.52 (m, 2H, N-CH₂); 3.65-3.67 (m, 1H, N-CH₂); 3.71-3.81 (m, 5H, N-CH₂, O-CH₂); 6.29 (s, 2H, SO₂-CH₂); 7.42-7.50 (m, 2H, Ar); 7.74-7.76 (m, 1H, Ar); 8.20 (d, *J* 1.0 Hz, 1H, signal of a rotamer, Ar); 8.21 (d, *J* 1.0 Hz, 1H, signal of a rotamer, Ar); 8.36-8.37 (m, 1H, Ar); 9.20 (s, 1H, signal of a rotamer, Ar); 9.21 (s, 1H, signal of a rotamer, Ar); 9.59 (d, *J* 1.0 Hz, 1H, signal of a rotamer, Ar); 9.60 (d, *J* 1.0 Hz, 1H, signal of a rotamer, Ar). M/Z (M+H)⁺: 455

### Compound 17 : (1-Methanesulfonylmethyl-3-pyrazolo[1,5-a]pyridin-3-yl-1H-pyrazolo[4,3-c]pyridin-6-yl)-1,4-oxazepan-4-yl-methanone

Compound 17 was obtained according to General Procedure IV, starting from [1-(methylsulfanylmethyl)-3-pyrazolo[1,5-a]pyridin-3-yl-pyrazolo[4,3-c]pyridin-6-yl]-(1,4-oxazepan-4-yl)methanone K13. Purification by flash chromatography (DCM/MeOH: 10/0 to 9/1), then by preparative HPLC afforded 17 as a white solid in 34% yield. ¹H-NMR (DMSO-*d₆*, 400MHz): 1.74-1.81 (m, 1H, CH₂); 1.91-1.97 (m, 1H, CH₂); 3.15 (s, 3H, SO₂-CH₃); 3.48-3.53 (m, 2H, N-CH₂); 3.64-3.67 (m, 1H, N-CH₂); 3.72-3.79 (m, 5H, N-CH₂, O-CH₂); 6.23 (s, 2H, SO₂-CH₂); 7.12 (td, *J* 6.9, 1.2 Hz, 1H, Ar); 7.52 (ddd, *J* 8.8, 6.9, 0.9 Hz, 1H, Ar); 8.14 (d, *J* 0.9 Hz, 1H, signal of a rotamer, Ar); 8.15 (d, *J* 0.9 Hz, 1H, signal of a rotamer, Ar); 8.34-8.38 (m, 1H, Ar); 8.87 (ddd, *J* 6.9, 1.2, 0.9 Hz, 1H, Ar); 9.05 (s, 1H, signal of a rotamer, Ar); 9.06 (s, 1H, signal of a rotamer, Ar); 9.61 (d, *J* 0.9 Hz, 1H, signal of a rotamer, Ar); 9.63 (d, *J* 0.9 Hz, 1H, signal of a rotamer, Ar). M/Z (M+H)⁺: 455

### Compound 18 : (3-Aza-bicyclo[3.2.1]oct-3-yl)-(1-methanesulfonylmethyl-3-pyrazolo[1,5-a]pyridin-3-yl-1H-pyrazolo[4,3-c]pyridin-6-yl)-methanone

Compound 18 was obtained according to General Procedure IV, starting from 3-azabicyclo[3.2.1]octan-3-yl-[1-(methylsulfanylmethyl)-3-pyrazolo[1,5-a]pyridin-3-yl-pyrazolo[4,3-c]pyridin-6-yl]methanone K14. Purification by preparative HPLC afforded 18 as a white solid in 34% yield. ¹H-NMR (DMSO-*d₆*, 400MHz): 1.49-1.73 (m, 6H, CH₂); 2.01-2.13 (m, 1H, CH); 2.26-2.36 (m, 1H, CH₂); 2.87 *(d, J* 12.7 Hz, 1H, N-CH₂); 3.09-3.19 (m, 4H, N-CH₂, CH₃); 3.35- 3.37 (m, 1H, N-CH₂); 4.32-4.40 (m, 1H, N-CH₂); 6.22 (s, 2H, SO₂-CH₂); 7.11 (td, *J* 6.9, 1.2 Hz, 1H, Ar); 7.52 (ddd, *J* 9.0, 6.9, 1.2 Hz, 1H, Ar); 8.08 (d, *J* 1.0 Hz, 1H, Ar); 8.36 (dt, *J* 9.0, 1.2 Hz, 1H, Ar); 8.87 (dt, *J* 6.9, 1.2 Hz, 1H, Ar); 9.04 (s, 1H, Ar); 9.60 (d, *J* 1.0 Hz, 1H, Ar). M/Z (M+H)⁺: 465

### Compound 19 : (3-Aza-bicyclo[3.2.1]oct-3-yl)-(3-benzofuran-3-yl-1-methanesulfonylmethyl-1H-pyrazolo[4,3-c]pyridin-6-yl)-methanone

Compound 19 was obtained according to General Procedure IV, starting from 3-azabicyclo[3.2.1]octan-3-yl-[3-(benzofuran-3-yl)-1-(methylsulfanylmethyl)pyrazolo[4,3-c]pyridin-6-yl]methanone K15. Purification by preparative HPLC afforded 19 as a white solid in 24% yield. ¹H-NMR (DMSO-*d₆*, 400MHz): 1.49-1.71 (m, 6H, CH₂); 2.04-2.10 (m, 1H, CH); 2.28-2.34 (m, 1H, CH); 2.87 *(d, J* 12.7 Hz, 1H, N-CH₂); 3.11-3.19 (m, 4H, N-CH₂, CH₃); 3.33- 3.37 (m, 1H, N-CH₂); 4.33-4.39 (m, 1H, N-CH₂); 6.28 (s, 2H, SO₂-CH₂); 7.41-7.51 (m, 2H, Ar); 7.73-7.76 (m, 1H, Ar); 8.14 (d, *J* 1.0 Hz, 1H, Ar); 8.34-8.37 (m, 1H, Ar); 9.13 (s, 1H, Ar); 9.58 (d, *J* 1.0 Hz, 1H, Ar). M/Z (M+H)⁺: 465

### Compound 20 : Azepan-1-yl-[3-(6-fluoro-imidazo[1,2-a]pyridin-3-yl)-1-methanesulfonylmethyl-1H-pyrazolo[4,3-c]pyridin-6-yl]-methanone

Compound 20 was obtained according to General Procedure IV, starting from azepan-1-yl-[3-(6-fluoroimidazo[1,2-a]pyridin-3-yl)-1-(methylsulfanylmethyl)pyrazolo[4,3-c]pyridin-6-yl]methanone K1. Purification by flash chromatography (DCM/MeOH: 100/0 to 95/5) afforded 20 as a beige solid in 15% yield. ¹H-NMR (DMSO-*d₆*, 400MHz): 1.52-1.65 (m, 6H, CH₂); 1.74-1.82 (m, 2H, CH₂); 3.15 (s, 3H, SO₂CH₃); 3.33-3.39 (m, 2H, N-CH₂); 3.62-3.67 (m, 2H, N-CH₂); 6.32 (s, 2H, CH₂-SO₂CH₃); 7.61 (ddd, *J* 10.0, 8.1, 2.5 Hz, 1H, Ar); 7.92 (dd, *J* 10.0, 5.4 Hz, 1H, Ar); 8.14 (d, *J* 1.0 Hz, 1H, Ar); 8.87 (s, 1H, Ar); 9.62-9.64 (ddd, *J* 5.2, 2.5, 0.5 Hz, 1H, Ar); 9.66 (d, *J* 1.0 Hz, 1H, Ar). M/Z (M+H)⁺: 471

### Compound 21 : Azepan-1-yl-[3-(6-fluoro-imidazo[1,2-a]pyridin-3-yl)-1-methanesulfinylmethyl-1H-pyrazolo[4,3-c]pyridin-6-yl]-methanone

To a suspension of azepan-1-yl-[3-(6-fluoroimidazo[1,2-a]pyridin-3-yl)-1-(methylsulfanylmethyl)pyrazolo[4,3-c]pyridin-6-yl]methanone K1 (1 equiv.) in ethanol (0.1M) were added H₂O₂, 30% in water, (4 equiv.) and trifluoromethanesulfonic anhydride (1 equiv.). The reaction mixture was stirred 72h at rt. The reaction mixture was diluted with DCM, washed with a saturated sodium bicarbonate solution and brine, dried over magnesium sulfate then concentrated. Purification by flash chromatography (DCM/MeOH: 100/0 to 95/5) afforded 21 as a beige solid in 26% yield. ¹H-NMR (DMSO-*d₆*, 400MHz): 1.53-1.63 (m, 6H, CH₂); 1.74-1.80 (m, 2H, CH₂); 2.76 (s, 3H CH₃); 3.36-3.40 (m, 2H, N-CH₂); 3.62-3.65 (m, 2H, N-CH₂); 5.91 (d, *J* 13.5 Hz, 1H, CH₂); 6.03 (d, *J* 13.5 Hz, 1H, CH₂); 7.60 (ddd, *J* 10.1, 8.2, 2.5 Hz, 1H, Ar); 7.91 (dd, *J* 10.1, 5.4 Hz, 1H, Ar); 8.01 (d, *J* 1.0 Hz, 1H, Ar); 8.84 (s, 1H, Ar); 9.62-9.63 (m, 2H, Ar). M/Z (M+H)⁺: 455

### Compound 22 : [3-(6,8-Difluoro-imidazo[1,2-a]pyridin-3-yl)-1-ethanesulfonylmethyl-1H-pyrazolo[4,3-c]pyridin-6-yl]-1,4-oxazepan-4-yl-methanone

Compound 22 was obtained according to General Procedure IV, starting from [3-(6,8-difluoroimidazo[1,2-a]pyridin-3-yl)-1-(ethylsulfanylmethyl)pyrazolo[4,3-c]pyridin-6-yl]-(1,4-oxazepan-4-yl)methanone K2. Purification by preparative HPLC afforded 22 as a beige solid in 33% yield. ¹H-NMR (DMSO-*d₆*, 400MHz): 1.30 (t, *J* 7.5 Hz, 3H, CH₃); 1.78 (quint, *J* 5.8 Hz, 1H, CH₂); 1.95 (quint, *J* 5.8 Hz, 1H, CH₂); 3.24 (q, *J* 7.5 Hz, 2H, CH₂-CH₃); 3.48-3.52 (m, 2H, N-CH₂); 3.64-3.80 (m, 6H, N-CH₂, O-CH₂); 6.38 (s, 2H, N-CH₂-SO₂); 7.81 (ddd, *J* 11.1, 9.2, 2.1 Hz, 1H, Ar); 8.19 (d, *J* 1.0 Hz, 1H, signal of a rotamer, Ar); 8.21 (d, *J* 1.0 Hz, 1H, signal of a rotamer, Ar); 8.89 (s, 1H, signal of a rotamer, Ar); 8.90 (s, 1H, signal of a rotamer, Ar); 9.50-9.52 (m, 1H, Ar); 9.67 (d, 1.0 Hz, 1H, Ar); 9.68 (d, 1.0 Hz, 1H, Ar). M/Z (M+H)⁺: 505

### Compound 23 : (3-Aza-bicyclo[3.2.1]oct-3-yl)-[3-(6,8-difluoro-imidazo[1,2-a]pyridin-3-yl)-1-methanesulfonylmethyl-1H-pyrazolo[4,3-c]pyridin-6-yl]-methanone

Compound 23 was obtained according to General Procedure IV, starting from Compound 14 (3-Aza-bicyclo[3.2.1]oct-3-yl)-[3-(6,8-difluoro-imidazo[1,2-a]pyridin-3-yl)-1-methylsulfanylmethyl-1H-pyrazolo[4,3-c]pyridin-6-yl]-methanone. Purification by flash chromatography (DCM/MeOH: 100/0 to 96/4) afforded 23 as a white solid in 47% yield. ¹H-NMR (DMSO-*d₆*, 400MHz): 1.49-1.72 (m, 6H, CH₂); 2.04-2.11 (m, 1H, CH₂); 2.29-2.35 (m, 1H, CH₂); 2.88 *(d, J* 12.5 Hz, 1H, N-CH₂); 3.14 (s, 3H, CH₃); 3.16-3.19 (m, 1H, N-CH₂); 3.24-3.32 (m, 1H, N-CH₂)_{;} 4.36 (d, *J* 12.5 Hz, 1H, N-CH₂); 6.34 (s, 2H, CH₂-SO₂CH₃); 7.80 (ddd, *J* 11.0, 9.2, 2.2 Hz, 1H, Ar); 8.14 (d, *J* 1.0 Hz, 1H, Ar); 8.89 (s, 1H, Ar); 9.51-9.55 (m, 1H, Ar); 9.66 (d, *J* 1.0 Hz, 1H, Ar). M/Z (M+H)⁺: 501

### Compound 24 : [3-(6,8-Difluoro-imidazo[1,2-a]pyridin-3-yl)-1-methanesulfonylmethyl-1H-pyrazolo[4,3-c]pyridin-6-yl]-(8-endo-hydroxy-8-exo-methyl-3-aza-bicyclo[3.2.1]oct-3-yl)-methanone

Compound 24 was obtained according to General Procedure IV, starting from [3-(6,8-difluoroimidazo[1,2-a]pyridin-3-yl)-1-(methylsulfanylmethyl)pyrazolo[4,3-c]pyridin-6-yl]-(8-*endo*-hydroxy-8-*exo*-methyl-3-azabicyclo[3.2.1]octan-3-yl)methanone K8. Purification by preparative HPLC afforded 24 as a beige solid in 31% yield. ¹H-NMR (DMSO-*d₆*, 400MHz): 1.18 (s, 3H, CH₃); 1.47-1.80 (m, 6H, CH, CH₂); 3.03-3.10 (m, 1H, N-CH₂); 3.14 (s, 3H, CH₃SO₂); 3.37-3.43 (m, 1H, N-CH₂); 3.62-3.69 (m, 1H, N-CH₂); 4.13-4.19 (m, 1H, N-CH₂); 4.88 (s, 1H, OH); 6.34 (s, 2H, SO₂-CH₂); 7.78-7.85 (m, 1H, Ar); 8.15 (s, 1H, Ar); 8.90 (s, 1H, Ar); 9.52-9.55 (m, 1H, Ar); 9.68 (s, 1H, Ar). M/Z (M+H)⁺: 531

### Compound 25 : [3-(6,8-Difluoro-imidazo[1,2-a]pyridin-3-yl)-1-methanesulfonylmethyl-1H-pyrazolo[4,3-c]pyridin-6-yl]-(3-endo-hydroxy-8-aza-bicyclo[3.2.1]oct-8-yl)-methanone

Compound 25 was obtained according to General Procedure IV, starting from [3-(6,8-difluoroimidazo[1,2-a]pyridin-3-yl)-1-(methylsulfanylmethyl)pyrazolo[4,3-c]pyridin-6-yl]-(3-*endo*-hydroxy-8-azabicyclo[3.2.1]octan-8-yl)methanone K9. Purification by preparative HPLC afforded 25 as a white solid in 11% yield. ¹H-NMR (DMSO-*d₆*, 400MHz): 1.67-1.71 (m, 1H, CH₂); 1.80-1.93 (m, 3H, CH₂); 2.02-2.11 (m, 2H, CH₂); 2.19-2.34 (m, 2H, CH₂); 3.14 (s, 3H, CH₃SO₂); 3.98-4.02 (m, 1H, N-CH); 4.47-4.51 (m, 1H, N-CH); 4.64-4.70 (m, 2H, CH-OH); 6.37 (s, 2H, SO₂-CH₂); 7.77-7.87 (m, 1H, Ar); 8.30 (s, 1H, Ar); 8.91 (s, 1H, Ar); 9.53-9.56 (m, 1H, Ar); 9.69 (s, 1H, Ar). M/Z (M+H)⁺: 517

### Compound 26 : [3-(6,8-Difluoro-imidazo[1,2-a]pyridin-3-yl)-1-methanesulfonylmethyl-1H-pyrazolo[4,3-c]pyridin-6-yl]-(2-oxa-5-aza-bicyclo[2.2.1]hept-5-yl)-methanone

Compound 26 was obtained according to General Procedure IV, starting from [3-(6,8-difluoroimidazo[1,2-a]pyridin-3-yl)-1-(methylsulfanylmethyl)pyrazolo[4,3-c]pyridin-6-yl]-(2-oxa-5-azabicyclo[2.2.1]heptan-5-yl)methanone K10. The crude was solubilized in a DCM/MeOH mixture. Concentration of DCM then filtration of the resulting precipitate afforded 26 as a white solid in 23% yield. ¹H-NMR (DMSO-*d₆*, 400MHz): 1.82-1.96 (m, 2H, CH₂); 3.15 (s, 3H, CH₃SO₂); 3.42-3.46 (m, 1H, major rotamer, CH₂); 3.56-3.61 (m, 1H, major rotamer, CH₂); 3.63-3.68 (m, 1H, minor rotamer, CH₂); 3.77-3.84 (m, 1H, major rotamer, 2H, minor rotamer, CH₂); 3.85-3.90 (m, 1H, minor rotamer, CH₂); 3.91-3.95 (m, 1H, major rotamer, CH₂); 4.64 (bs, 1H, minor rotamer, CH); 4.70 (bs, 1H, major rotamer, CH); 5.00 (bs, 1H, minor rotamer, CH); 5.16 (bs, 1H, major rotamer, CH); 6.37 (d, *J* 17.9 Hz, 1H, SO₂-CH₂); 6.42(d, *J* 17.9 Hz, 1H, SO₂-CH₂); 7.77-7.85 (m, 1H, Ar); 8.44 (s, 1H, major rotamer, Ar); 8.49 (s, 1H, minor rotamer, Ar); 8.90 (s, 1H, minor rotamer, Ar); 8.91 (s, 1H, major rotamer, Ar); 9.53-9.56 (m, 1H, Ar); 9.67 (s, 1H, minor rotamer, Ar); 9.72 (s, 1H, major rotamer, Ar). M/Z (M+H)⁺: 489

### Compound 27 : [3-(6,8-Difluoro-imidazo[1,2-a]pyridin-3-yl)-1-methanesulfonylmethyl-1H-pyrazolo[4,3 c]pyridin-6-yl]-(8-oxa-3-aza-bicyclo[3.2.1]oct-3-yl)-methanone

Compound 27 was obtained according to General Procedure IV, starting from [3-(6,8-difluoroimidazo[1,2-a]pyridin-3-yl)-1-(methylsulfanylmethyl)pyrazolo[4,3-c]pyridin-6-yl]-(8-oxa-3-azabicyclo[3.2.1]octan-3-yl)methanone K11. Purification by preparative HPLC afforded 27 as a yellow solid in 69% yield. ¹H-NMR (DMSO-*d₆*, 400MHz): 1.74-1.92 (m, 4H, CH₂); 3.08 (dd, *J* 13.1, 1.6 Hz, 1H, N-CH₂); 3.15 (s, 3H, CH₃SO₂); 3.34-3.42 (m, 2H, N-CH₂); 4.21-4.26 (m, 2H, N-CH₂, O-CH); 4.41-4.47 (m, 1H, O-CH); 6.35 (s, 2H, SO₂-CH₂); 7.81 (ddd, *J* 11.2, 9.3, 2.1 Hz, 1H, Ar); 8.23 (d, *J* 1.0 Hz, 1H, Ar); 8.90 (s, 1H, Ar); 9.52-9.55 (m, 1H, Ar); 9.69 (d, *J* 1.0 Hz, 1H, Ar). M/Z (M+H)⁺: 503

### Compound 28 : (3-Benzofuran-3-yl-1-methanesulfonylmethyl-1H-pyrazolo[4,3-c]pyridin-6-yl)-(8-endo-hydroxy-8-exo-methyl-d₃-3-aza-bicyclo[3.2.1]oct-3-yl)-methanone

Compound 28 was obtained according to General Procedure IV, starting from [3-(benzofuran-3-yl)-1-(methylsulfanylmethyl)pyrazolo[4,3-c]pyridin-6-yl]-(8-*endo-*hydroxy-8-*exo*-methyl-*d₃*-3-azabicyclo[3.2.1]octan-3-yl)methanone K18. Purification by preparative HPLC afforded 28 as a white solid in 7% yield. ¹H-NMR (DMSO-*d₆*, 400MHz): 1.43-1.83 (m, 6H, CH₂); 3.09 (dd, *J* 12.0, 2.5 Hz, 1H, N-CH₂); 3.17 (s, 1H, SO₂-CH₃); 3.40 *(d, J* 12.0 Hz, 1H, N-CH₂); 3.66 *(d, J* 12.0 Hz, 1H, N-CH₂); 4.16 (dd, *J* 12.0, 2.5 Hz, 1H, N-CH₂); 4.85 (s, 1H, OH); 6.28 (m, 2H, CH₂-SO₂); 7.42-7.51 (m, 2H, Ar); 7.72-7.77 (m, 1H, Ar); 8.14 (d, *J* 1.0 Hz, 1H, Ar); 8.34-8.38 (m, 1H, Ar); 9.20 (s, 1H, Ar); 9.59 (d, *J* 1.0 Hz, 1H, Ar). M/Z (M+H)⁺: 498

### Compound 29 : (3-Benzofuran-3-yl-1-methanesulfonylmethyl-1H-pyrazolo[4,3-c]pyridin-6-yl)-(6,6-difluoro-1,4-oxazepan-4-yl)-methanone

Compound 29 was obtained according to General Procedure IV, starting from [3-(benzofuran-3-yl)-1-(methylsulfanylmethyl)pyrazolo[4,3-c]pyridin-6-yl]-(6,6-difluoro-1,4-oxazepan-4-yl)methanone K19. In that specific case, 2.5 equiv. of *m*CPBA were used. Purification by preparative HPLC afforded 29 as a white solid in 45% yield. ¹H-NMR (DMSO-*d₆*, 400MHz): 3.18 (s, 3H, SO₂-CH₃); 3.62 (t, *J* 4.8 Hz, 2H, major rotamer, CH₂); 3.85-3.98 (m, 1H, major rotamer, 3H, minor rotamer, CH₂); 4.03 (t, *J* 13.1 Hz, 2H, major rotamer, CH₂); 4.30 (t, *J* 13.1 Hz, 2H, major rotamer, CH₂); 4.41 (t, *J* 13.1 Hz, 2H, minor rotamer, CH₂); 6.31 (s, 2H, CH₂-SO₂); 7.42-7.51 (m, 2H, Ar); 7.73-7.77 (m, 1H, Ar); 8.30-8.39 (m, 2H, Ar); 9.22 (s, 1H, major rotamer, Ar); 9.23 (s, 1H, minor rotamer, Ar); 9.61 (bs, 1H, Ar). M/Z (M+H)⁺: 491

### Compound 30 : (3-Benzofuran-3-yl-1-methanesulfonylmethyl-1H-pyrazolo[4,3-c]pyridin-6-yl)-(8-endo-hydroxy-3-aza-bicyclo[3.2.1]oct-3-yl)-methanone

Compound 30 was obtained according to General Procedure IV, starting from [3-(benzofuran-3-yl)-1-(methylsulfanylmethyl)pyrazolo[4,3-c]pyridin-6-yl]-(8-*endo-*hydroxy-3-azabicyclo[3.2.1]octan-3-yl)methanone K20. In that specific case, 2.3 equiv. of *m*CPBA were used. Purification by preparative HPLC afforded 30 as a white solid in 7% yield. ¹H-NMR (DMSO-*d₆*, 400MHz): 1.49-1.81 (m, 5H, CH₂); 2.00-2.05 (m, 1H, CH₂); 3.10 (dd, *J* 12.0, 2.6 Hz, 1H, N-CH₂); 3.17 (s, 3H, SO₂-CH₃); 3.31 (d, *J* 12.0 Hz, 1H, N-CH₂); 3.59 (d, *J* 12.0 Hz, 1H, N-CH₂); 3.85-3.91 (m, 1H, CH-OH); 4.16 (dd, *J* 12.0, 2.6 Hz, 1H, N-CH₂); 5.18 (d, *J* 3.1 Hz, 1H, OH); 6.28 (m, 2H, CH₂-SO₂); 7.42-7.50 (m, 2H, Ar); 7.74-7.77 (m, 1H, Ar); 8.15 (d, J0.9 Hz, 1H, Ar); 8.34-8.39 (m, 1H, Ar); 9.21 (s, 1H, Ar); 9.60 (d, *J* 0.9 Hz, 1H, Ar). M/Z (M+H)⁺: 481

### Compound 31 : (3-Benzofuran-3-yl-1-methanesulfonylmethyl-1H-pyrazolo[4,3-c]pyridin-6-yl)-(5-endo-hydroxy-5-exo-methyl-2-aza-bicyclo[2.2.1]hept-2-yl)-methanone

Compound 31 was obtained according to General Procedure IV, starting from [3-(benzofuran-3-yl)-1-(methylsulfanylmethyl)pyrazolo[4,3-c]pyridin-6-yl]-(5-*endo-*hydroxy-5-*exo*-methyl-2-azabicyclo[2.2.1]heptan-2-yl)methanone K21. In that specific case, 2.0 equiv. of *m*CPBA were used. Purification by preparative HPLC afforded 31 as a grey solid in 21% yield. ¹H-NMR (DMSO-*d₆*, 400MHz): 1.30 (s, 3H, minor rotamer CH₃); 1.32 (s, 3H, major rotamer CH₃); 1.59-1.80 (m, 4H, CH₂); 2.24-2.30 (m, 1H, CH₂); 3.15-3.20 (m, 3H, SO₂-CH₃); 3.24-3.31 (m, 1H, major rotamer N-CH₂); 3.70 (dd, *J* 10.5, 3.5 Hz, 1H, minor rotamer, N-CH₂); 3.91 (d, *J* 10.5 Hz, 1H, major rotamer, N-CH₂); 3.98 (d, *J* 10.5 Hz, 1H, minor rotamer, N-CH₂); 4.53 (bs, 1H, N-CH); 4.61 (s, 1H, minor rotamer, OH); 4.76 (s, 1H, major rotamer, OH); 6.32-6.37 (m, 2H, CH₂-SO₂); 7.42-7.51 (m, 2H, Ar); 7.74-7.79 (m, 1H, Ar); 8.33 (d, *J* 1.0 Hz, 1H, minor rotamer, Ar); 8.36-8.40 (m, 1H, Ar); 8.44 (d, *J* 1.0 Hz, 1H, major rotamer, Ar); 9.22 (s, 1H, minor rotamer, Ar); 9.24 (s, 1H, major rotamer, Ar); 9.60 (d, *J* 1.0 Hz, 1H, minor rotamer, Ar); 9.62 (d, *J* 1.0 Hz, 1H, major rotamer, Ar). M/Z (M+H)⁺: 481

### Compound 32 : (3-Benzofuran-3-yl-1-methanesulfonylmethyl-1H-pyrazolo[4,3-c]pyridin-6-yl)-(4-hydroxy-4-methyl-piperidin-1-yl)-methanone

Compound 32 was obtained according to General Procedure IV, starting from [3-(benzofuran-3-yl)-1-(methylsulfanylmethyl)pyrazolo[4,3-c]pyridin-6-yl]-(4-hydroxy-4-methyl-1-piperidyl)methanone K22. In that specific case, 2.5 equiv. of *m*CPBA were used. Purification by preparative HPLC afforded 32 as a white solid in 9% yield. ¹H-NMR (DMSO-*d₆*, 400MHz): 1.18 (s, 3H, CH₃); 1.41-1.63 (m, 4H, CH₂); 3.17 (s, 3H, CH₃-SO₂); 3.27-3.41 (m, 3H, N-CH₂); 4.12-4.18 (m, 1H, N-CH₂); 4.45 (s, 1H, OH); 6.28 (s, 2H, CH₂-SO₂); 7.42-7.50 (m, 2H, Ar); 7.73-7.77 (m, 1H, Ar); 8.15 (bs, 1H, signal of a rotamer, Ar); 8.16 (bs, 1H, signal of a rotamer, Ar); 8.35-8.37 (m, 1H, Ar); 9.20 (s, 1H, Ar); 9.58 (d, J 1.0 Hz, 1H, Ar). M/Z (M+H)⁺: 469

### Compound 33 : (3-Benzofuran-3-yl-1-methanesulfonylmethyl-1H-pyrazolo[4,3-c]pyridin-6-yl)-(4,4-difluoro-piperidin-1-yl)-methanone

Compound 33 was obtained according to General Procedure IV, starting from [3-(benzofuran-3-yl)-1-(methylsulfanylmethyl)pyrazolo[4,3-c]pyridin-6-yl]-(4,4-difluoro-1-piperidyl)methanone K23. In that specific case, 2.5 equiv. of *m*CPBA were used. Purification by preparative HPLC afforded 33 as a white solid in 10% yield. ¹H-NMR (DMSO-*d₆*, 400MHz): 1.98-2.21 (m, 4H, CH₂); 3.17 (s, 3H, SO₂-CH₃); 3.54 (t, *J* 5.6 Hz, 2H, N-CH₂); 3.83 (t, *J* 5.6 Hz, 2H, N-CH₂); 6.29 (m, 2H, CH₂-SO₂); 7.42-7.51 (m, 2H, Ar); 7.73-7.77 (m, 1H, Ar); 8.25 (d, *J* 1.0 Hz, 1H, Ar); 8.34-8.39 (m, 1H, Ar); 9.21 (s, 1H, Ar); 9.61 (d, *J* 1.0 Hz, 1H, Ar). M/Z (M+H)⁺: 475

### Compound 34 : (3-Benzofuran-3-yl-1-methanesulfonylmethyl-1H-pyrazolo[4,3-c]pyridin-6-yl)-(2-methoxymethyl-pyrrolidin-1-yl)-methanone

Compound 34 was obtained according to General Procedure IV, starting from [3-(benzofuran-3-yl)-1-(methylsulfanylmethyl)pyrazolo[4,3-c]pyridin-6-yl]-[2-(methoxymethyl)pyrrolidin-1-yl]methanone K24. In that specific case, 2.5 equiv. of *m*CPBA were used. Purification by preparative HPLC afforded 34 as a white solid in 51% yield. ¹H-NMR (DMSO-*d₆*, 400MHz): 1.74-2.02 (m, 4H, CH₂); 3.02 (s, 3H, minor rotamer, O-CH₃); 3.07 (dd, *J* 9.6, 7.5 Hz, 1H, minor rotamer, CH₂); 3.18 (s, 3H, SO₂-CH₃); 3.18-3.22 (m, 1H, major rotamer, CH₂); 3.33 (s, 3H, major rotamer, CH₃); 3.47-3.71 (m, 3H, CH₂); 4.33-4.37 (m, 1H, major rotamer, N-CH); 4.68-4.73 (m, 1H, minor rotamer, N-CH); 6.28-6.36 (m, 2H, CH₂-SO₂); 7.42-7.50 (m, 2H, Ar); 7.74-7.77 (m, 1H, Ar); 8.32 (bs, 1H, Ar); 8.35-8.38 (m, 1H, Ar); 9.20 (s, 1H, major rotamer, Ar); 9.21 (s, 1H, minor rotamer, Ar); 9.58 (d, *J* 1.0 Hz, 1H, Ar). M/Z (M+H)⁺: 469

### Compound 35 : (3-Benzofuran-3-yl-1-methanesulfonylmethyl-1H-pyrazolo[4,3-c]pyridin-6-yl)-(4-hydroxy-2,2 dimethyl-piperidin-1-yl)-methanone

Compound 35 was obtained according to General Procedure IV, starting from [3-(benzofuran-3-yl)-1-(methylsulfanylmethyl)pyrazolo[4,3-c]pyridin-6-yl]-(4-hydroxy-2,2-dimethyl-1-piperidyl)methanone K25. In that specific case, 2.5 equiv. of *m*CPBA were used. Purification by preparative HPLC afforded 35 as a white solid in 14% yield. ¹H-NMR (DMSO-*d₆*, 400MHz): 1.36-1.44 (m, 1H, CH₂); 1.48 (s, 3H, CH₃); 1.58-1.64 (m, 1H, CH₂); 1.62 (s, 3H, CH₃); 1.77-1.81 (m, 1H, CH₂); 1.84-1.91 (m, 1H, CH₂); 3.00-3.07 (m, 1H, N-CH₂); 3.17 (s, 3H, CH₃-SO₂); 3.41-3.48 (m, 1H, N-CH₂); 3.81-3.89 (m, 1H, CH); 4.72 (d, *J* 4.4 Hz, 1H, OH); 6.28 (s, 2H, CH₂-SO₂); 7.42-7.50 (m, 2H, Ar); 7.74-7.78 (m, 1H, Ar); 8.11 (d, *J* 0.8 Hz, 1H, Ar); 8.34-8.37 (m, 1H, Ar); 9.19 (s, 1H, Ar); 9.55 (d, *J* 0.8 Hz, 1H, Ar). M/Z (M+H)⁺: 483

### Compound 36 : (3-Benzofuran-3-yl-1-methanesulfonylmethyl-1H-pyrazolo[4,3-c]pyridin-6-yl)-(4-hydroxy-piperidin-1-yl)-methanone

Compound 36 was obtained according to General Procedure IV, starting from [3-(benzofuran-3-yl)-1-(methylsulfanylmethyl)pyrazolo[4,3-c]pyridin-6-yl]-(4-hydroxy-1-piperidyl)methanone K26. In that specific case, 2 equiv. of *m*CPBA were used. Purification by preparative HPLC afforded 36 as a white solid in 5% yield. ¹H-NMR (DMSO-*d₆*, 400MHz): 1.33-1.49 (m, 2H, CH₂); 1.66-1.76 (m, 1H, CH₂); 1.81-1.90 (m, 1H, CH₂); 3.11-3.20 (m, 4H, CH₃-SO₂, N-CH₂); 3.25-3.32 (m, 1H, N-CH₂); 3.51-3.60 (m, 1H, N-CH₂); 3.73-3.81 (m, 1H, CH); 4.06-4.16 (m, 1H, N-CH₂); 4.80 (d, *J* 4.0 Hz, 1H, OH); 6.28 (s, 2H, CH₂-SO₂); 7.41-7.51 (m, 2H, Ar); 7.73-7.77 (m, 1H, Ar); 8.16 (d, *J* 1.1 Hz, 1H, Ar); 8.34-8.38 (m, 1H, Ar); 9.20 (s, 1H, Ar); 9.59 (d, *J* 1.0 Hz, 1H, Ar). M/Z (M+H)⁺: 455

### Compound 37 : [3-(benzofuran-3-yl)-1-(methylsulfonylmethyl)pyrazolo[4,3-c]pyridin-6-yl]-(3-endo-hydroxy-8-azabicyclo[3.2.1]octan-8-yl)methanone

Compound 37 was obtained according to General Procedure IV, starting from [3-(benzofuran-3-yl)-1-(methylsulfanylmethyl)pyrazolo[4,3-c]pyridin-6-yl]-(3-endo-hydroxy-8-azabicyclo[3.2.1]octan-8-yl)methanone K27. In that specific case, 2 equiv. of *m*CPBA were used. Purification by recrystallisation (DMF/EtOH: 2/1) afforded 37 as a white powder in 12% yield. ¹H-NMR (DMSO-*d₆*, 400MHz): 1.67-1.72 (m, 1H, CH₂); 1.79-1.94 (m, 3H, CH₂); 2.03-2.11 (m, 2H, CH₂); 2.19-2.34 (m, 2H, CH₂); 3.17 (s, 3H, CH₃SO₂); 3.98-4.03 (m, 1H, N-CH); 4.47-4.52 (m, 1H, N-CH); 4.65 (d, *J* 2.5 Hz, 1H, OH); 4.66-4.70 (m, 1H, CH); 6.31 (s, 2H, CH₂-SO₂); 7.41-7.50 (m, 2H, Ar); 7.73-7.77 (m, 1H, Ar); 8.30 (d, *J* 1.0 Hz, 1H, Ar); 8.34-8.38 (m, 1H, Ar); 9.21 (s, 1H, Ar); 9.60 (d, *J* 1.0 Hz, 1H, Ar). M/Z (M+H)⁺: 481

### Compound 38 : (3-Benzofuran-3-yl-1-methanesulfonylmethyl-1H-pyrazolo[4,3-c]pyridin-6-yl)-(3-hydroxy-piperidin-1-yl)-methanone

Compound 38 was obtained according to General Procedure IV, starting from [3-(benzofuran-3-yl)-1-(methylsulfanylmethyl)pyrazolo[4,3-c]pyridin-6-yl]-(3-hydroxy-1-piperidyl)methanone K28. In that specific case, 2 equiv. of *m*CPBA were used. Purification by preparative HPLC afforded 38 as a white powder in 27% yield. ¹H-NMR (DMSO-*d₆*, 400MHz): 1.37-1.52 (m, 2H, CH₂); 1.59-1.97 (m, 2H, CH₂); 2.87 (dd, *J* 12.2, 9.1 Hz, 1H, N-CH₂, signal of a rotamer); 2.97-3.08 (m, 1H, N-CH₂); 3.17 (s, 3H, CH₃-SO₂); 3.19-3.25 (m, 1H, N-CH₂, signal of a rotamer); 3.43-3.60 (m, 2H, N-CH₂); 3.96-4.03 (m, 1H, CH, signal of a rotamer); 4.31 (dd, *J* 12.2, 3.9 Hz, 1H, CH, signal of a rotamer); 4.79 (d, *J* 3.9 Hz, 1H, OH, signal of a rotamer); 5.03 (d, *J* 3.9 Hz, 1H, OH, signal of a rotamer); 6.28 (s, 2H, CH₂-SO₂); 7.41-7.51 (m, 2H, Ar); 7.75 (d, *J* 8.0 Hz, 1H, Ar); 8.15 (d, *J* 1.0 Hz, 1H, Ar, signal of a rotamer); 8.18 (d, *J* 1.0 Hz, 1H, Ar, signal of a rotamer); 8.34-8.38 (m, 1H, Ar); 9.20 (s, 1H, Ar, signal of a rotamer); 9.21 (s, 1H, Ar, signal of a rotamer); 9.59 (d, *J* 1.0 Hz, 1H, Ar, signal of a rotamer); 9.60 (d, *J* 1.0 Hz, 1H, Ar, signal of a rotamer). M/Z (M+H)⁺: 455

### Compound 39 : (3-Benzofuran-3-yl-1-methanesulfonylmethyl-1H-pyrazolo[4,3-c]pyridin-6-yl)-(3,3-difluoro-piperidin-1-yl)-methanone

Compound 39 was obtained according to General Procedure IV, starting from [3-(benzofuran-3-yl)-1-(methylsulfanylmethyl)pyrazolo[4,3-c]pyridin-6-yl]-(3,3-difluoro-1-piperidyl)methanone K29. In that specific case, 2 equiv. of *m*CPBA were used. Purification by preparative HPLC afforded 39 as a white powder in 30%. ¹H-NMR (DMSO-*d₆*, 400MHz): 1.68-1.81 (m, 2H, CH₂); 2.04-2.20 (m, 2H, CH₂); 3.17 (s, 3H, CH₃-SO₂); 3.43-3.48 (m, 1H, N-CH₂); 3.73-3.78 (m, 1H, N-CH₂); 3.92-4.10 (m, 2H, N-CH₂); 6.31 (s, 2H, CH₂-SO₂); 7.41-7.51 (m, 2H, Ar); 7.74-7.77 (m, 1H, Ar); 8.23 (d, *J* 1.0 Hz, 1H, Ar, signal of a rotamer); 8.31 (d, *J* 1.0 Hz, 1H, Ar, signal of a rotamer); 8.34-8.38 (m, 1H, Ar); 9.21 (s, 1H, Ar); 9.61 (bs, 1H, Ar). M/Z (M+H)⁺: 475

### Compound 40 : (3-Benzofuran-3-yl-1-methanesulfonylmethyl-1H-pyrazolo[4,3-c]pyridin-6-yl)-(4-hydroxyazepan-1-yl)-methanone

Compound 40 was obtained according to General Procedure IV, starting from [3-(benzofuran-3-yl)-1-(methylsulfanylmethyl)pyrazolo[4,3-c]pyridin-6-yl]-(4-hydroxyazepan-1-yl)methanone K30. In that specific case, 2.5 equiv. of *m*CPBA were used. Purification by preparative HPLC afforded 40 as a white powder in 25% yield. ¹H-NMR (DMSO-*d₆*, 400MHz): 1.47-1.84 (m, 5H, CH₂); 1.87-2.02 (m, 1H, CH₂); 3.17 (s, 3H, CH₃-SO₂); 3.23-3.80 (m, 5H, N-CH₂, CH); 4.56 (d, *J* 3.9 Hz, 1H, OH, signal of a rotamer); 4.63 (d, *J* 3.9 Hz, 1H, OH, signal of a rotamer); 6.29 (s, 2H, CH₂-SO₂); 7.41-7.51 (m, 2H, Ar); 7.75 (d, *J* 7.9 Hz, 1H, Ar); 8.14 (d, *J* 1.0 Hz, 1H, Ar, signal of a rotamer); 8.16 (d, *J* 1.0 Hz, 1H, Ar, signal of a rotamer); 8.34-8.38 (m, 1H, Ar); 9.20 (s, 1H, Ar, signal of a rotamer); 9.21 (s, 1H, Ar, signal of a rotamer); 9.59 (d, *J* 1.0 Hz, 1H, Ar). M/Z (M+H)⁺: 469

### General Procedure V : Synthesis of compounds H from compounds G (scheme 1)

To a solution of compound G (1 equiv.) in ethanol (0.3M) was added chloroacetaldehyde, 50% in water, (5 equiv.). The reaction mixture was stirred 30 min at 130°C under microwave irradiation. The reaction mixture was diluted with DCM, washed with a saturated potassium carbonate solution and brine, dried over magnesium sulfate, concentrated. The resuling solid was purified by flash chromatography to afford compound H.

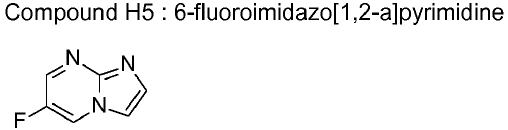

Compound H5 was obtained according to General Procedure V, starting from 2-amino-5-fluoropyrimidine G1. Purification by flash chromatography (DCM/MeOH: 10/0 to 9/1) afforded H5 in 60% yield. M/Z (M+H)⁺: 138

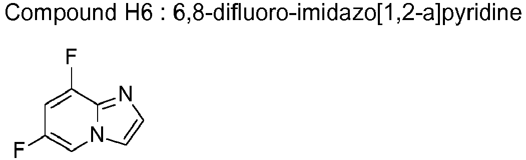

Compound H6 was obtained according to General Procedure V, starting from 3,5-difluoropyridin-2-amine G2. Purification by flash chromatography (Cyclohexane/AcOEt : 10/0 to 3/7) afforded H6 as a beige powder in 87% yield. M/Z (M+H)⁺: 155

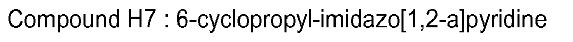

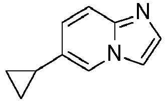

Compound H7 was obtained according to General Procedure V, starting from 5-cyclopropyl-pyridine-2-amine G3. Purification by flash chromatography (DCM/MeOH: 10/0 to 9/1) afforded the H7 in 99% yield. M/Z (M+H)⁺: 159

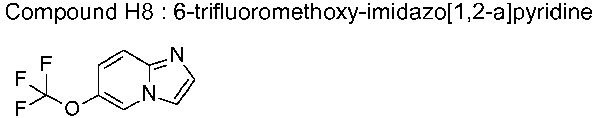

Compound H8 was obtained according to General Procedure V, starting from 5-(trifluoromethoxy)-pyridine-2-amine G4. Purification by flash chromatography (DCM/MeOH: 10/0 to 9/1) afforded H8 in 81% yield. M/Z (M+H)⁺: 203

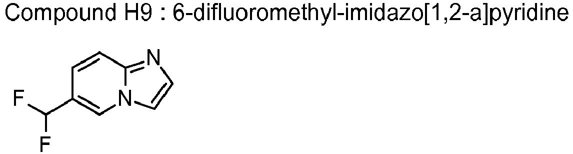

To a solution of imidazo[1,2-a]pyridine-6-carbaldehyde H38 (1 equiv.) in anhydrous DCM (0.2M) at -78°C was added dropwise a solution of deoxo-fluor, 50% in toluene, (2.1 equiv.) in DCM (0.2M). The reaction mixture was stirred 1h at 0°C, then 72h at rt. The reaction mixture was cooled down to -78°C, 2.4 additional equiv. of deoxo-fluor, 50% in toluene, were added. The reaction mixture was stirred 72h at rt, then quenched with a saturated sodium bicarbonate solution and extracted twice with DCM. The combined organic phase were dried over magnesium sulfate then concentrated. The resulting solid was purified by flash chromatography (DCM/MeOH: 100/0 to 95/5) to afford H9 in 13% yield. M/Z (M+H)⁺: 169

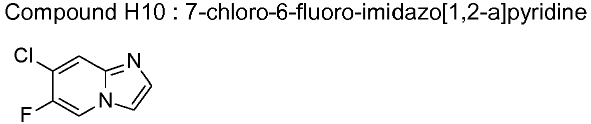

Compound H10 was obtained according to General Procedure V, starting from 4-chloro-5-fluoro-pyridine-2-amine G5. Purification by flash chromatography (DCM/MeOH: 100/0 to 96/4) afforded H10 as a beige powder in 68% yield. M/Z (M+H)⁺: 171

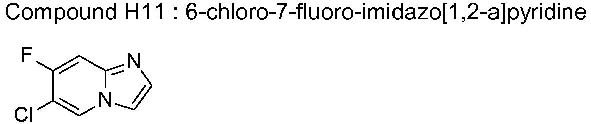

Compound H11 was obtained according to General Procedure V, starting from 5-chloro-4-fluoro-pyridine-2-amine G6. Purification by flash chromatography (Cyclohexane/AcOEt: 5/5 to 2/8) afforded H11 as a white powder in 63% yield. M/Z (M+H)⁺: 171

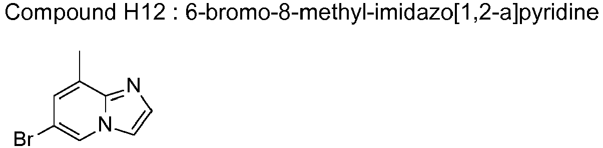

Compound H12 was obtained according to General Procedure V, starting from 5-bromo-3-methyl-pyridine-2-amine G7. Purification by flash chromatography (DCM/MeOH: 100/0 to 95/5) afforded H12 as a beige powder in 96% yield. M/Z (M+H)⁺: 211/213

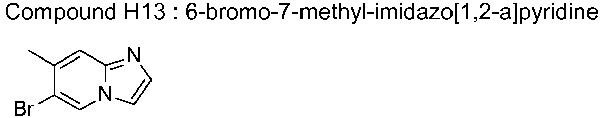

Compound H13 was obtained according to General Procedure V, starting from 5-bromo-4-methyl-pyridine-2-amine G8. Purification by flash chromatography (DCM/MeOH: 100/0 to 97/3) afforded H13 as a beige powder in 76% yield. M/Z (M+H)⁺: 211/213

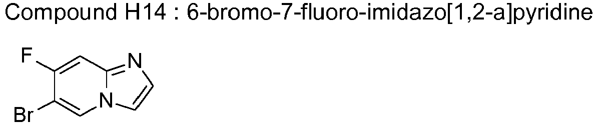

Compound H14 was obtained according to General Procedure V, starting from 5-bromo-4-fluoro-pyridine-2-amine G9. Purification by flash chromatography (DCM/MeOH: 100/0 to 95/5) afforded the compound in 63% yield. M/Z (M+H)⁺: 215/217

### General Procedure VI : Synthesis of compounds K and O from compounds F and N respectively via C-H arylation (schemes 1&2)

In a sealed vial under argon atmosphere, to a solution of compound F or N in anhydrous DMA (0.1M) were added compound H (3 equiv.) and cesium pivalate (2 equiv.). The solution was degassed with argon bubbling for 15min before addition of {[P(tBu)₃]PdBr}₂ (2.5 mol%). The reaction mixture was heated overnight at 140°C under vigorous stirring. The reaction mixture was diluted with AcOEt, washed with a saturated sodium bicarbonate solution and brine, dried over magnesium sulfate then concentrated. The resulting oil was purified by flash chromatography to afford the compound or compound K or O.

### Alternative 1 :

In a sealed vial under argon atmosphere, to a solution of compound F or N in anhydrous DMA (0.1M) were added compound H (1.3 equiv.) and cesium pivalate (1.2 equiv.). The solution was degassed with argon bubbling for 15min before addition of {[P(tBu)₃]PdBr}₂ (2.5 mol%). The reaction mixture was heated 4h at 140°C under vigorous stirring. The reaction mixture was diluted with AcOEt, washed with a saturated sodium bicarbonate solution and brine, dried over magnesium sulfate then concentrated. The resulting oil was purified by flash chromatography to afford the compound or compound K or O.

### Alternative 2 :

In a sealed vial under argon atmosphere, to a solution of compound F or N in anhydrous DMA (0.1M) were added compound H (1.3 equiv.) and cesium pivalate (2 equiv.). The solution was degassed with argon bubbling for 15min before addition of {[P(tBu)₃]PdBr}₂ (10 mol%). The reaction mixture was heated 4h at 110°C under vigorous stirring. The reaction mixture was diluted with AcOEt, washed with a saturated sodium bicarbonate solution and brine, dried over magnesium sulfate then concentrated. The resulting oil was purified by flash chromatography to afford the compound or compound K or O.

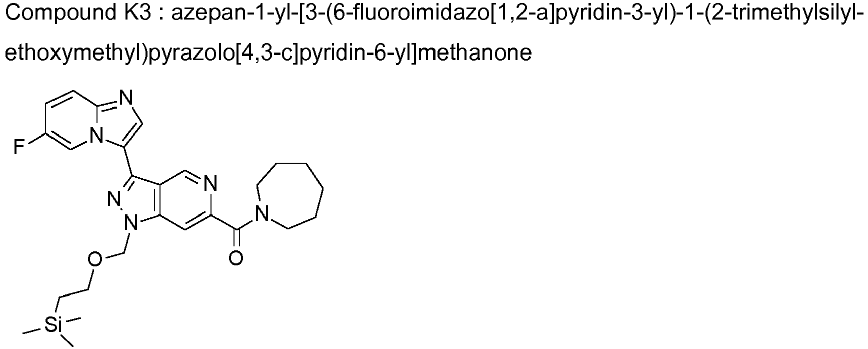

Compound K3 was obtained according to General Procedure VI, starting from azepan-1-yl-[3-bromo-1-(2-trimethylsilylethoxymethyl)pyrazolo[4,3-c]pyridin-6-yl]methanone F4 and 6-fluoroimidazo[1,2-a]pyridine H19. Purification by flash chromatography (DCM/MeOH: 10/0 to 8/2) afforded K3 as a yellow oil in 67% yield. M/Z (M+H)⁺: 509

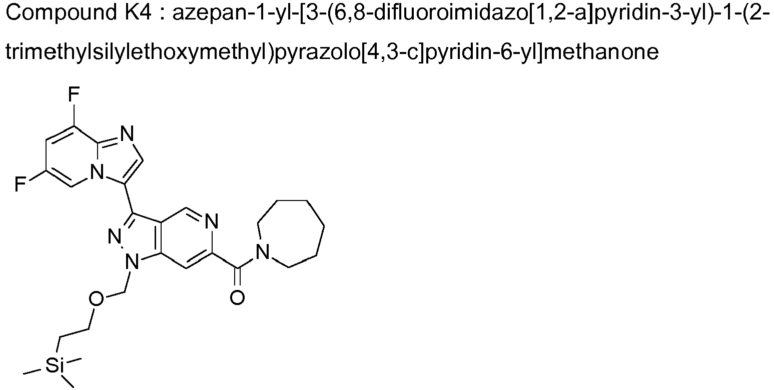

Compound K4 was obtained according to General Procedure VI, starting from azepan-1-yl-[3-bromo-1-(2-trimethylsilylethoxymethyl)pyrazolo[4,3-c]pyridin-6-yl]methanone F4 and 6,8-difluoro-imidazo[1,2-a]pyridine H6. In that specific case, 10 mol% of {[P(tBu)₃]PdBr}₂ was used. Purification by flash chromatography (Cyclohexane/AcOEt: 10/0 to 0/10) afforded K4 as a yellow oil in 73% yield. M/Z (M+H)⁺: 527

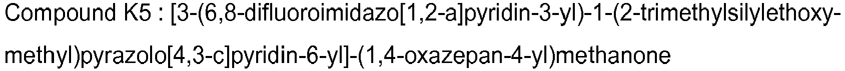

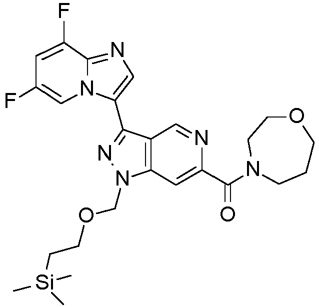

Compound K5 was obtained according to General Procedure VI, starting from [3-bromo-1-(2-trimethylsilylethoxymethyl)pyrazolo[4,3-c]pyridin-6-yl]-(1,4-oxazepan-4-yl)methanone F14 and 6,8-difluoro-imidazo[1,2-a]pyridine H6. In that specific case, 5 mol% of {[P(tBu)₃]PdBr}₂ was used. Purification by flash chromatography (DCM/MeOH: 10/0 to 8/2) afforded K5 as a yellow powder in 71% yield. M/Z (M+H)⁺: 529

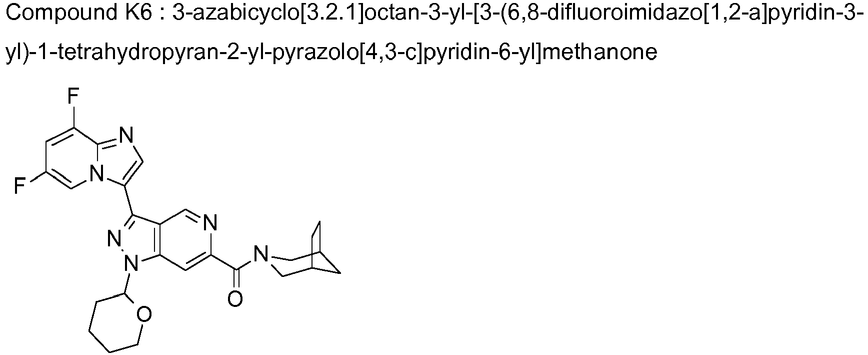

Compound K6 was obtained according to General Procedure VI, starting from 3-azabicyclo[3.2.1]octan-3-yl-(3-bromo-1-tetrahydropyran-2-yl-pyrazolo[4,3-c]pyridin-6-yl)methanone F15 and 6,8-difluoro-imidazo[1,2-a]pyridine H6. In that specific case, 5 mol% of {[P(tBu)₃]PdBr}₂ was used. Purification by flash chromatography (DCM/MeOH: 100/0 to 97/3) afforded K6 as a yellow powder in 57% yield. M/Z (M+H)⁺: 493

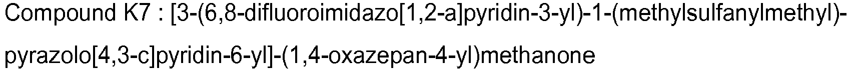

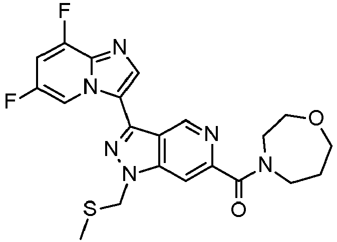

Compound K7 was obtained according to General Procedure VI, starting from [3-bromo-1-(methylsulfanylmethyl)pyrazolo[4,3-c]pyridin-6-yl]-(1,4-oxazepan-4-yl)methanone F16 and 6,8-difluoro-imidazo[1,2-a]pyridine H6 (1.3 equiv.). Purification by flash chromatography (DCM/MeOH: 100/0 to 95/5) afforded K7 in 30% yield. M/Z (M+H)⁺: 459

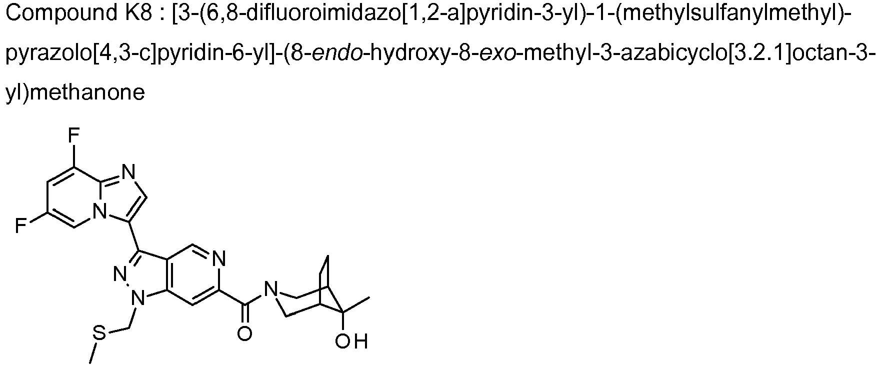

Compound K8 was obtained according to General Procedure VI, starting from [3-bromo-1-(methylsulfanylmethyl)pyrazolo[4,3-c]pyridin-6-yl]-(8-endo-hydroxy-8-exo-methyl-3-azabicyclo[3.2.1]octan-3-yl)methanone F26 and 6,8-difluoro-imidazo[1,2-a]pyridine H6. Purification by flash chromatography (DCM/MeOH: 100/0 to 95/5) afforded K8 in 28% yield. M/Z (M+H)⁺: 499

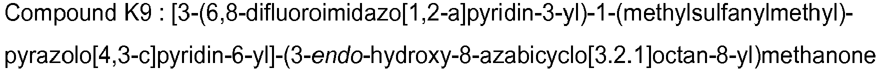

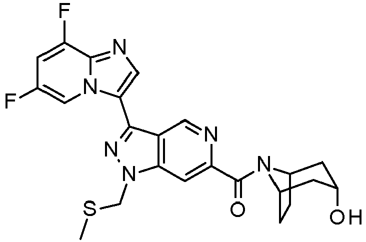

Compound K9 was obtained according to General Procedure VI, starting from [3-bromo-1-(methylsulfanylmethyl)pyrazolo[4,3-c]pyridin-6-yl]-(3-endo-hydroxy-8-azabicyclo[3.2.1]octan-8-yl)methanone F27 and 6,8-difluoro-imidazo[1,2-a]pyridine H6. Purification by flash chromatography (DCM/MeOH: 100/0 to 95/5) afforded K9 in 26% yield. M/Z (M+H)⁺: 485

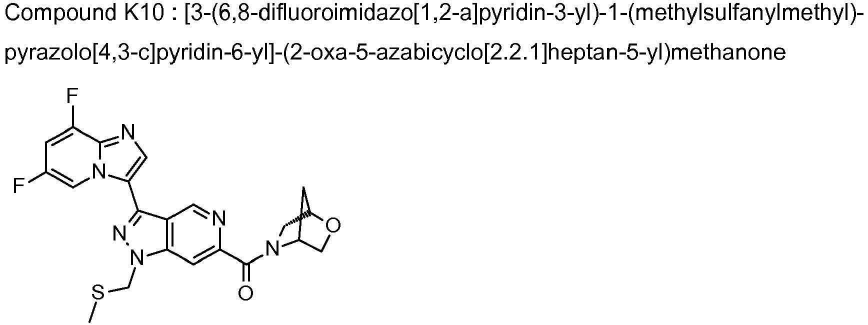

Compound K10 was obtained according to General Procedure VI, starting from [3-bromo-1-(methylsulfanylmethyl)pyrazolo[4,3-c]pyridin-6-yl]-(2-oxa-5-azabicyclo[2.2.1]heptan-5-yl)methanone F28 and 6,8-difluoro-imidazo[1,2-a]pyridine H6. Purification by flash chromatography (Cyclohexane/AcOEt: 10/0 to 0/10) afforded K10 in 38% yield. M/Z (M+H)⁺: 485

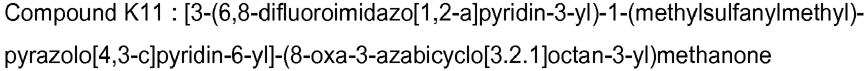

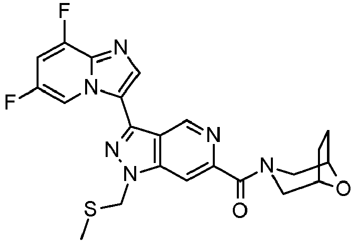

Compound K11 was obtained according to General Procedure VI, starting from [3-bromo-1-(methylsulfanylmethyl)pyrazolo[4,3-c]pyridin-6-yl]-(8-oxa-3-azabicyclo[3.2.1]octan-3-yl)methanone F29 and 6,8-difluoro-imidazo[1,2-a]pyridine H6. Purification by flash chromatography (DCM/MeOH: 100/0 to 95/5) afforded K11 in 25% yield. M/Z (M+H)⁺: 471

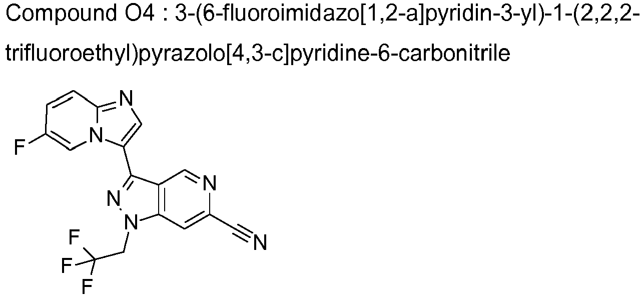

Compound O4 was obtained according to General Procedure VI, starting from 3-bromo-1-(2,2,2-trifluoroethyl)pyrazolo[4,3-c]pyridine-6-carbonitrile N1 and 6-fluoroimidazo[1,2-a]pyridine H19. Purification by flash chromatography (DCM/MeOH: 100/0 to 98/2) afforded O4 as a yellow solid in 35% yield. M/Z (M+H)⁺: 361

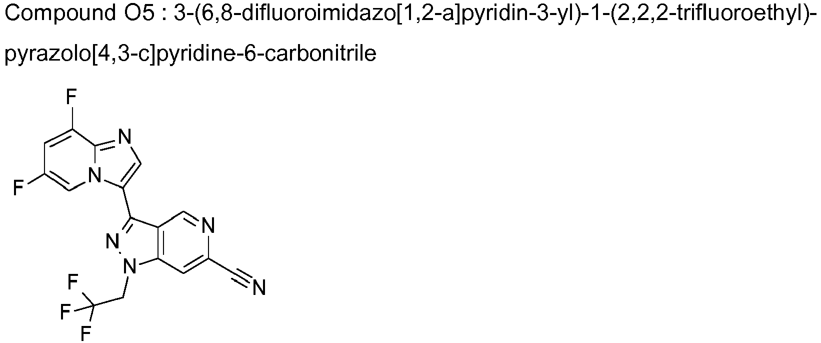

Compound O5 was obtained according to General Procedure VI, starting from 3-bromo-1-(2,2,2-trifluoroethyl)pyrazolo[4,3-c]pyridine-6-carbonitrile N1 and 6,8-difluoro-imidazo[1,2-a]pyridine H6. In that specific case, the reaction mixture was heated overnight at 110°C. Purification by flash chromatography (DCM/MeOH: 100/0 to 95/5) afforded O5 as a brown powder in 66% yield. M/Z (M+H)⁺: 397

### Compound 41 : Azepan-1-yl-[3-(6-fluoro-imidazo[1,2-a]pyridin-3-yl)-1-methyl-1H-pyrazolo[4,3-c]pyridin-6-yl]-methanone

Compound 41 was obtained according to General Procedure VI, starting from azepan-1-yl-(3-bromo-1-methyl-pyrazolo[4,3-c]pyridin-6-yl)methanone F1 and 6-fluoroimidazo[1,2-a]pyridine H19. In that specific case, {[P(tBu)₃]PdBr}₂ (2.5 mol%) was replaced by Pd-PEPPSI-IPent catalyst (5 mol%). Likewise, cesium pivalate (2 equiv.) was replaced by potassium carbonate (2 equiv.) and pivalic acid (0.3 equiv.). Purification by flash chromatography on a Biotage KP-NH cartridge (Cyclohexane/AcOEt: 50/50 to 0/100) afforded 41 as a white powder in 22% yield.¹H-NMR (DMSO-*d*₆, 400 MHz): 1.54-1.64 (m, 6H, CH₂); 1.75-1.81 (m, 2H, CH₂); 3.39-3.42 (m, 2H, N-CH₂); 3.63-3.66 (m, 2H, N-CH₂); 4.24 (s, 3H, N-CH₃); 7.57 (ddd, *J* 10.0, 8.1, 2.7 Hz, 1H, Ar); 7.88 (dd, *J* 10.0, 5.2 Hz, 1H, Ar); 7.97 (d, *J* 1.0 Hz, 1H, Ar); 8.76 (s, 1H, Ar); 9.57 (d, *J* 1.0 Hz, 1H, Ar); 9.62 (ddd, *J* 5.2, 2.7, 0.8 Hz, 1H, Ar). M/Z (M+H)⁺: 393

### Compound 42 : 3-[6-(Azepane-1-carbonyl)-1-methyl-1H-pyrazolo[4,3-c]pyridin-3-yl]-imidazo[1,2-a]pyridine-6-carbonitrile

Compound 42 was obtained according to General Procedure VI, starting from azepan-1-yl-(3-bromo-1-methyl-pyrazolo[4,3-c]pyridin-6-yl)methanone F1 and imidazo[1,2-a]pyridine-6-carbonitrile H20. Purification by flash chromatography (DCM/MeOH: 10/0 to 9/1) afforded 42 as a grey powder in 43% yield.¹H-NMR (DMSO-*d*₆, 400 MHz): 1.54-1.64 (m, 6H, CH₂); 1.75-1.81 (m, 2H, CH₂); 3.38-3.40 (m, 2H, N-CH₂); 3.62-3.65 (m, 2H, N-CH₂); 4.26 (s, 3H, N-CH₃); 7.70 (dd, *J* 9.3, 1.6 Hz, 1H, Ar); 7.95 (dd, *J* 9.3, 0.9 Hz, 1H, Ar); 7.99 (d, *J* 1.1 Hz, 1H, Ar); 8.85 (s, 1H, Ar); 9.57 (d, *J* 1.1 Hz, 1H, Ar); 10.08 (dd, *J* 1.6, 0.9 Hz, 1H, Ar). M/Z (M+H)⁺: 400

### Compound 43 : 3-[6-(Azepane-1-carbonyl)-1-propyl-1H-pyrazolo[4,3-c]pyridin-3-yl]-imidazo[1,2-a]pyridine-6-carbonitrile

Compound 43 was obtained according to General Procedure VI, starting from azepan-1-yl-(3-bromo-1-propyl-pyrazolo[4,3-c]pyridin-6-yl)methanone F2 and imidazo[1,2-a]pyridine-6-carbonitrile H20 (1.3 equiv.). Purification by flash chromatography (DCM/MeOH: 100/0 to 95/5) afforded 43 as a grey powder in 31% yield.¹H-NMR (DMSO-*d*₆,400MHz): 0.92 (t, *J* 7.3 Hz, 3H, CH₂-CH₂-CH₃); 1.54-1.65 (m, 6H, CH₂); 1.74-1.80 (m, 2H, CH₂); 1.97 (sextuplet, *J* 7.3 Hz, 2H, CH₂-CH₂-CH₃); 3.31-3.40 (m, 2H, N-CH₂); 3.61-3.65 (m, 2H, N-CH₂); 4.60 (t, *J* 7.3 Hz, 2H, CH₂-CH₂-CH₃); 7.71 (dd, *J* 9.3, 1.6 Hz, 1H, Ar); 7.96 (dd, *J* 9.3, 1.0 Hz, 1H, Ar); 8.04 (d, *J* 1.2 Hz, 1H, Ar); 8.85 (s, 1H, Ar); 9.57 (d, *J* 1.2 Hz, 1H, Ar); 10.04 (dd, *J* 1.6, 1.0 Hz, 1H, Ar). M/Z (M+H)⁺: 428

### Compound 44 : Azepan-1-yl-[3-(6-fluoro-imidazo[1,2-a]pyridin-3-yl)-1-(2,2,2-trifluoroethyl)-1H-pyrazolo[4,3-c]pyridin-6-yl]-methanone

Compound 44 was obtained according to General Procedure VI, starting from azepan-1-yl-[3-bromo-1-(2,2,2-trifluoroethyl)pyrazolo[4,3-c]pyridin-6-yl]methanone F3 and 6-fluoroimidazo[1,2-a]pyridine H19. Purification by flash chromatography (DCM/MeOH: 100/0 to 95/5) afforded 44 as a white powder in 44% yield.¹H-NMR (DMSO-*d*₆, 400 MHz): 1.54-1.64 (m, 6H, CH₂); 1.75-1.81 (m, 2H, CH₂); 3.35-3.38 (m, 2H, N-CH₂); 3.62-3.65 (m, 2H, N-CH₂); 5.74 (q, *J* 9.0 Hz, 2H, CF₃-CH₂); 7.60 (ddd, *J* 9.9, 8.0, 2.5 Hz, 1H, Ar); 7.91 (ddd, *J* 9.9, 5.5, 0.5 Hz, 1H, Ar); 8.14 (d, *J* 1.0 Hz, 1H, Ar); 8.84 (s, 1H, Ar); 9.58 (ddd, *J* 5.1, 2.5, 0.5 Hz, 1H, Ar); 9.65 (d, *J* 1.0 Hz, 1H, Ar). M/Z (M+H)⁺: 461

### Compound 45 : 3-[6-(Azepane-1-carbonyl)-1-(2,2,2-trifluoro-ethyl)-1H-pyrazolo[4,3-c]-pyridin-3-yl]-imidazo[1,2-a]pyridine-6-carbonitrile

Compound 45 was obtained according to General Procedure VI, starting from azepan-1-yl-[3-bromo-1-(2,2,2-trifluoroethyl)pyrazolo[4,3-c]pyridin-6-yl]methanone F3 and imidazo[1,2-a]pyridine-6-carbonitrile H20. Purification by flash chromatography (DCM/MeOH: 100/0 to 95/5) afforded 45 as a grey powder in 41% yield. ¹H-NMR (DMSO-*d*₆, 400 MHz): 1.54-1.64 (m, 6H, CH₂); 1.75-1.81 (m, 2H, CH₂); 3.35-3.38 (m, 2H, N-CH₂); 3.63-3.66 (m, 1H, N-CH₂); 5.79 (q, *J* 9.0 Hz, 2H, CF₃-CH₂); 7.75 (dd, *J* 9.3, 1.6 Hz, 1H, Ar); 7.99 (dd, *J* 9.3, 0.9 Hz, 1H, Ar); 8.16 (bs, 1H, Ar); 8.94 (s, 1H, Ar); 9.65 (d, *J* 1.0 Hz, 1H, Ar); 10.03 (dd, *J* 1.6, 0.9 Hz, 1H, Ar). M/Z (M+H)⁺: 468

### Compound 46 : 3-[6-(Azepane-1-carbonyl)-1-(2,2,2-trifluoro-ethyl)-1H-pyrazolo[4,3-c]-pyridin-3-yl]-imidazo[1,2-a]pyridine-6-carboxylic acid amide

Compound 46 was obtained according to General Procedure VI, starting from azepan-1-yl-[3-bromo-1-(2,2,2-trifluoroethyl)pyrazolo[4,3-c]pyridin-6-yl]methanone F3 and imidazo[1,2-a]pyridine-6-carboxamide H21. Purification by flash chromatography (DCM/MeOH: 10/0 to 9/1) afforded 46 as a white powder in 30% yield. ¹H-NMR (DMSO-*d*₆, 400 MHz): 1.54-1.64 (m, 6H, CH₂); 1.75-1.81 (m, 2H, CH₂); 3.35-3.38 (m, 2H, N-CH₂); 3.63-3.66 (m, 2H, N-CH₂); 5.70 (q, *J* 9.0 Hz, 2H, CF₃-CH₂); 7.63 (bs, 1H, NH); 7.85 (dd, *J* 9.4, 0.9 Hz, 1H, Ar); 7.90 (dd, J9.4, 1.7 Hz, 1H, Ar); 8.15 (bs, 1H, NH); 8.17 (d, *J* 1.0 Hz, 1H, Ar); 8.81 (s, 1H, Ar); 9.61 (d, *J* 1.0 Hz, 1H, Ar); 10.01 (dd, *J* 1.7, 0.9 Hz, 1H, Ar). M/Z (M+H)⁺: 486

### Compound 47 : 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carbonitrile

Compound 47 was obtained according to General Procedure VI, starting from azepan-1-yl-[3-bromo-1-(2,2,2-trifluoroethyl)pyrazolo[4,3-c]pyridin-6-yl]methanone F3 and 8-methyl-imidazo[1,2-a]pyridine-6-carbonitrile H15. (1.3 equiv.). Purification by flash chromatography (DCM/MeOH: 100/0 to 94/6) afforded 47 as a grey powder in 47% yield. ¹H-NMR (DMSO-*d*₆,400MHz): 1.54-1.64 (m, 6H, CH₂); 1.75-1.81 (m, 2H, CH₂); 2.63 (s, 3H, CH₃); 3.36-3.38 (m, 2H, N-CH₂); 3.63-3.66 (m, 2H, N-CH₂); 5.78 (q, *J* 9.0 Hz, 2H, CF₃-CH₂); 7.60 (bs, 1H, Ar); 8.16 (bs, 1H, Ar); 8.88 (s, 1H, Ar); 9.64 (d, *J* 0.9 Hz, 1H, Ar); 9.88-9.89 (m, 1H, Ar). M/Z (M+H)⁺: 482

### Compound 48 : Azepan-1-yl-[3-(6-chloro-imidazo[1,2-a]pyridin-3-yl)-1-(2,2,2-trifluoroethyl)-1H-pyrazolo[4,3-c]pyridin-6-yl]-methanone

Compound 48 was obtained according to General Procedure VI, starting from azepan-1-yl-[3-bromo-1-(2,2,2-trifluoroethyl)pyrazolo[4,3-c]pyridin-6-yl]methanone F3 and 6-chloroimidazo[1,2-a]pyridine H22 (1.3 equiv.). Purification by flash chromatography (DCM/MeOH: 100/0 to 95/5) afforded 48 as a white powder in 39% yield. ¹H-NMR (DMSO-*d*₆,400MHz): 1.52-1.66 (m, 6H, CH₂); 1.75-1.83 (m, 2H, CH₂); 3.35-3.40 (m, 2H, N-CH₂); 3.62-3.67 (m, 2H, N-CH₂); 5.75 (q, *J* 9.0 Hz, 2H, CF₃-CH₂); 7.56 (dd, *J* 9.6, 2.1 Hz, 1H, Ar); 7.88 (dd, *J* 9.6, 0.8 Hz, 1H, Ar); 8.16 (d, *J* 0.9 Hz, 1H, Ar); 8.84 (s, 1H, Ar); 9.62 (dd, *J* 2.1, 0.8 Hz, 1H, Ar); 9.64 (d, *J* 0.9 Hz, 1H, Ar). M/Z (M+H)⁺: 477/479

### Compound 49 : 3-[6-(Azepane-1-carbonyl)-1-(2,2,2-trifluoro-ethyl)-1H-pyrazolo[4,3-c]-pyridin-3-yl]-imidazo[1,2-a]pyridine-6-carboxylic acid methylamide

Compound 49 was obtained according to General Procedure VI, starting from azepan-1-yl-[3-bromo-1-(2,2,2-trifluoroethyl)pyrazolo[4,3-c]pyridin-6-yl]methanone F3 and *N*-methylimidazo[1,2-a]pyridine-6-carboxamide H1 (1.3 equiv.). Purification by flash chromatography (DCM/MeOH: 10/0 to 9/1) afforded 49 as a white powder in 46% yield. ¹H-NMR (DMSO-*d*₆,400MHz): 1.52-1.67 (m, 6H, CH₂); 1.75-1.84 (m, 2H, CH₂); 2.85 (d, *J* 4.5 Hz, 3H, CH₃); 3.35-3.39 (m, 2H, N-CH₂); 3.62-3.66 (m, 2H, N-CH₂); 5.70 (q, *J* 9.0 Hz, 2H, CF₃-CH₂); 7.86 (d, *J* 1.3 Hz, 2H, Ar); 8.17 (d, *J* 0.9 Hz, 1H, Ar); 8.65 (q, *J* 4.5 Hz, NH); 8.81 (s, 1H, Ar); 9.61 (d, J0.9 Hz, 1H, Ar); 10.01 (t, *J* 1.3 Hz, 1H, Ar). M/Z (M+H)⁺: 500

### Compound 50 : Azepan-1-yl-[3-(6-fluoro-imidazo[1,2-a]pyrimidin-3-yl)-1-(2,2,2-trifluoro-ethyl)-1H-pyrazolo[4,3-c]pyridin-6-yl]-methanone

Compound 50 was obtained according to General Procedure VI, starting from azepan-1-yl-[3-bromo-1-(2,2,2-trifluoroethyl)pyrazolo[4,3-c]pyridin-6-yl]methanone F3 and 6-fluoroimidazo[1,2-a]pyrimidine H5 (1.3 equiv.). Purification by flash chromatography (DCM/MeOH: 100/0 to 95/5) afforded 50 as a white powder in 22% yield. ¹H-NMR (DMSO-*d*₆,400MHz): 1.53-1.66 (m, 6H, CH₂); 1.73-1.83 (m, 2H, CH₂); 3.35-3.41 (m, 2H, N-CH₂); 3.62-3.66 (m, 2H, N-CH₂); 5.75 (q, J9.0 Hz, 2H, CF₃-CH₂); 8.15 (bs, 1H, Ar); 8.95 (d, *J* 3.0 Hz, 1H, Ar); 9.01 (s, 1H, Ar); 9.68 (d, *J* 1.0 Hz, 1H, Ar); 9.86 (dd, *J* 4.5, 3.0 Hz, 1H, Ar). M/Z (M+H)⁺: 462

### Compound 51 : Azepan-1-yl-[3-(6-methoxymethyl-imidazo[1,2-a]pyridin-3-yl)-1-(2,2,2-trifluoro-ethyl)-1H-pyrazolo[4,3-c]pyridin-6-yl]-methanone

Compound 51 was obtained according to General Procedure VI, starting from azepan-1-yl-[3-bromo-1-(2,2,2-trifluoroethyl)pyrazolo[4,3-c]pyridin-6-yl]methanone F3 and 6-(methoxymethyl)imidazo[1,2-a]pyridine H3 (1.3 equiv.). Purification by flash chromatography (DCM/MeOH: 100/0 to 94/6) afforded 51 as a white powder in 45% yield. ¹H-NMR (DMSO-*d*₆,400MHz): 1.54-1.64 (m, 6H, CH₂); 1.75-1.81 (m, 2H, CH₂); 3.36-3.39 (m, 5H, CH₂, O-CH₃); 3.63-3.66 (m, 2H, N-CH₂); 4.55 (s, 2H, O-CH₂); 5.70 (q, *J* 9.0 Hz, 2H, CF₃-CH₂); 7.44 (dd, *J* 9.2, 1.7 Hz, 1H, Ar); 7.80 (d, *J* 9.2 Hz, 1H, Ar); 8.15 (s, 1H, Ar); 8.75 (s, 1H, Ar); 9.49 (bs, 1H, Ar); 9.60 (s, 1H, Ar). M/Z (M+H)+: 487

### Compound 52 : Azepan-1-yl-[3-indolizin-3-yl-1-(2,2,2-trifluoro-ethyl)-1H-pyrazolo[4,3-c]pyridin-6-yl]-methanone

Compound 52 was obtained according to General Procedure VI, starting from azepan-1-yl-[3-bromo-1-(2,2,2-trifluoroethyl)pyrazolo[4,3-c]pyridin-6-yl]methanone F3 and indolizine H23 (1.3 equiv.). Purification by flash chromatography (DCM/MeOH: 100/0 to 98/2) afforded 52 as a green powder in 32% yield. ¹H-NMR (DMSO-*d*₆, 400MHz): 1.53-1.65 (m, 6H, CH₂); 1.75-1.82 (m, 2H, CH₂); 3.35-3.40 (m, 2H, N-CH₂); 3.62-3.66 (m, 2H, N-CH₂); 5.66 (q, *J* 9.1 Hz, 2H, CF₃-CH₂); 6.75 (d, *J* 4.3 Hz, 1H, Ar); 6.92 (td, *J* 6.7, 1.4 Hz, 1H, Ar); 6.99 (ddd, *J* 8.8, 6.7, 1.0 Hz, 1H, Ar); 7.68 (dt, *J* 8.8, 1.4 Hz, 1H, Ar); 7.91 (d, J4.3 Hz, 1H, Ar); 8.10 (d, *J* 0.9 Hz, 1H, Ar); 9.52 (ddd, *J* 6.7, 1.4, 1.0 Hz, 1H, Ar); 9.55 (d, *J* 0.9 Hz, 1H, Ar). M/Z (M+H)⁺: 442

### Compound 53 : 3-[6-(Azepane-1-carbonyl)-1-(2,2,2-trifluoro-ethyl)-1H-pyrazolo[4,3-c]-pyridin-3-yl]-imidazo[1,2-a]pyridine-6-carboxylic acid dimethylamide

Compound 53 was obtained according to General Procedure VI, starting from azepan-1-yl-[3-bromo-1-(2,2,2-trifluoroethyl)pyrazolo[4,3-c]pyridin-6-yl]methanone F3 and *N*,*N*-dimethylimidazo[1,2-a]pyridine-6-carboxamide H2 (1.3 equiv.). Purification by flash chromatography (DCM/MeOH: 100/0 to 95/5) afforded 53 as a beige powder in 41% yield. ¹H-NMR (DMSO-*d*₆,400MHz): 1.52-1.67 (m, 6H, CH₂); 1.74-1.83 (m, 2H, CH₂); 3.07 (s, 6H, N-CH₃); 3.35-3.41 (m, 2H, N-CH₂); 3.62-3.66 (m, 2H, N-CH₂); 5.73 (q, *J* 9.1 Hz, 2H, CF₃-CH₂); 7.54 (dd, *J* 9.3, 1.6 Hz, 1H, Ar); 7.86 (dd, *J* 9.3, 1.0 Hz, 1H, Ar); 8.16 (d, *J* 0.9 Hz, 1H, Ar); 8.84 (s, 1H, Ar); 9.64 (d, *J* 0.9 Hz, 1H, Ar); 9.66 (dd, *J* 1.6, 1.0 Hz, 1H, Ar). M/Z (M+H)⁺: 514

### Compound 54 : Azepan-1-yl-[3-imidazo[1,5-a]pyridin-3-yl-1-(2,2,2-trifluoro-ethyl)-1H-pyrazolo[4,3-c]pyridin-6-yl]-methanone

Compound 54 was obtained according to General Procedure VI, starting from azepan-1-yl-[3-bromo-1-(2,2,2-trifluoroethyl)pyrazolo[4,3-c]pyridin-6-yl]methanone F3 and imidazo[1,5-a]pyridine H24 (1.3 equiv.). Purification by flash chromatography (DCM/MeOH: 10/0 to 9/1) afforded 54 as a yellow powder in 14% yield. ¹H-NMR (DMSO-*d*₆,400MHz): 1.53-1.64 (m, 6H, CH₂); 1.75-1.81 (m, 2H, CH₂); 3.34-3.37 (m, 2H, CH₂); 3.63-3.65 (m, 2H, N-CH₂); 5.71 (q, *J* 9.0 Hz, 2H, CF₃-CH₂); 7.06-7.11 (m, 2H, Ar); 7.82 (d, *J* 0.9 Hz, 1H, Ar); 7.83-7.87 (m, 1H, Ar); 8.14 (d, *J* 1.0 Hz, 1H, Ar); 9.34-9.38 (m, 1H, Ar); 9.77 (d, *J* 1.0 Hz, 1H, Ar). M/Z (M+H)⁺: 443

### Compound 55 : Azepan-1-yl-[3-imidazo[1,2-a]pyridin-3-yl-1-(2,2,2-trifluoro-ethyl)-1H-pyrazolo[4,3-c]pyridin-6-yl]-methanone

Compound 55 was obtained according to General Procedure VI, starting from azepan-1-yl-[3-bromo-1-(2,2,2-trifluoroethyl)pyrazolo[4,3-c]pyridin-6-yl]methanone F3 and imidazo[1,2-a]pyridine H25 (1.3 equiv.). Purification by flash chromatography (DCM/MeOH: 100/0 to 95/5) then by preparative HPLC afforded 55 as a beige powder in 14% yield. ¹H-NMR (DMSO-*d*₆,400MHz): 1.53-1.64 (m, 6H, CH₂); 1.75-1.81 (m, 2H, CH₂); 3.35-3.38 (m, 2H, N-CH₂); 3.63-3.65 (m, 2H, N-CH₂); 5.70 (q, J9.1 Hz, 2H, CF₃-CH₂); 7.25 (td, *J* 6.8, 1.0 Hz, 1H, Ar); 7.50 (ddd, *J* 9.1, 6.8, 1.0 Hz, 1H, Ar); 7.83 (dt, *J* 9.1, 1.0 Hz, 1H, Ar); 8.14 (d, *J* 1.0 Hz, 1H, Ar); 8.78 (s, 1H, Ar); 9.53 (dt, *J* 6.8, 1.0 Hz, 1H, Ar); 9.63 (d, *J* 1.0 Hz, 1H, Ar). M/Z (M+H)⁺: 443

### Compound 56 : Azepan-1-yl-[3-imidazo[1,2-a]pyrazin-3-yl-1-(2,2,2-trifluoro-ethyl)-1H-pyrazolo[4,3-c]pyridin-6-yl]-methanone

Compound 56 was obtained according to General Procedure VI, starting from azepan-1-yl-[3-bromo-1-(2,2,2-trifluoroethyl)pyrazolo[4,3-c]pyridin-6-yl]methanone F3 and imidazo[1,2-a]pyrazine H26 (1.3 equiv.). Purification by flash chromatography (DCM/MeOH: 100/0 to 95/5) afforded 56 as a yellow powder in 8% yield. ¹H-NMR (DMSO-*d*₆,400MHz): 1.53-1.67 (m, 6H, CH₂); 1.74-1.82 (m, 2H, CH₂); 3.39-3.40 (m, 2H, N-CH₂); 3.62-3.66 (m, 2H, N-CH₂); 5.74 (q, *J* 9.0 Hz, 2H, CF₃-CH₂); 8.18 (bs, 1H, Ar); 8.23 (d, *J* 4.7 Hz, 1H, Ar); 8.98 (s, 1H, Ar); 9.31 (d, *J* 1.4 Hz, 1H, Ar); 9.39 (dd, *J* 4.7, 1.4 Hz, 1H, Ar); 9.69 (bs, 1H, Ar). M/Z (M+H)⁺: 444

### Compound 57 : 3-[6-(Azepane-1-carbonyl)-1-(2,2,2-trifluoro-ethyl)-1H-pyrazolo[4,3-c]-pyridin-3-yl]-7-methyl-imidazo[1,2-a]pyridine-6-carboxylic acid amide

Compound 57 was obtained according to General Procedure VI, starting from azepan-1-yl-[3-bromo-1-(2,2,2-trifluoroethyl)pyrazolo[4,3-c]pyridin-6-yl]methanone F3 and 7-methyl-imidazo[1,2-a]pyridine-6-carboxamide H18 (1.3 equiv.). Purification by flash chromatography (DCM/MeOH: 10/0 to 9/1) afforded 57 as a white powder in 32% yield. ¹H-NMR (DMSO-*d*₆,400MHz): 1.54-1.64 (m, 6H, CH₂); 1.75-1.81 (m, 2H, CH₂); 2.48 (s, 3H, CH₃); 3.35-3.38 (m, 2H, CH₂); 3.63-3.65 (m, 2H, N-CH₂); 5.70 (q, *J* 8.9 Hz, 2H, CF₃-CH₂); 7.64 (bs, 1H, NH); 7.66 (s, 1H, Ar); 8.01 (bs, 1H, NH); 8.15 (s, 1H, Ar); 8.74 (s, 1H, Ar); 9.54 (s, 1H, Ar); 9.61 (s, 1H, Ar). M/Z (M+H)+: 500

### Compound 58 : Azepan-1-yl-[1-(2,2,2-trifluoro-ethyl)-3-(6-trifluoromethyl-imidazo[1,2-a]pyridin-3-yl)-1H-pyrazolo[4,3-c]pyridin-6-yl]-methanone

Compound 58 was obtained according to General Procedure VI, starting from azepan-1-yl-[3-bromo-1-(2,2,2-trifluoroethyl)pyrazolo[4,3-c]pyridin-6-yl]methanone F3 and 6-(trifluoromethyl)imidazo[1,2-a]pyridine H27 (1.3 equiv.). Purification by flash chromatography (DCM/MeOH: 100/0 to 95/5) afforded 58 as a white powder in 39% yield. ¹H-NMR (DMSO-*d*₆,400MHz): 1.54-1.66 (m, 6H, CH₂); 1.74-1.83 (m, 2H, CH₂); 3.34-3.39 (m, 2H, N-CH₂); 3.61-3.65 (m, 2H, N-CH₂); 5.73 (q, *J* 9.1 Hz, 2H, CF₃-CH₂); 7.74 (dd, *J* 9.5, 1.8 Hz, 1H, Ar); 8.03 (br d, *J* 9.5 Hz, 1H, Ar); 8.20 (d, *J* 0.9 Hz, 1H, Ar); 8.94 (s, 1H, Ar); 9.66 (d, *J* 0.9 Hz, 1H, Ar); 9.96-9.98 (m, 1H, Ar). M/Z (M+H)⁺: 511

### Compound 59 : Azepan-1-yl-[3-(6-methoxy-imidazo[1,2-a]pyridin-3-yl)-1-(2,2,2-trifluoro-ethyl)-1H-pyrazolo[4,3-c]pyridin-6-yl]-methanone

Compound 59 was obtained according to General Procedure VI, starting from azepan-1-yl-[3-bromo-1-(2,2,2-trifluoroethyl)pyrazolo[4,3-c]pyridin-6-yl]methanone F3 and 6-methoxy-imidazo[1,2-a]pyridine H28 (1.3 equiv.). Purification by flash chromatography (DCM/MeOH: 100/0 to 95/5) afforded 59 as a white powder in 43% yield. ¹H-NMR (DMSO-*d*₆,400MHz): 1.52-1.66 (m, 6H, CH₂); 1.73-1.82 (m, 2H, CH₂); 3.34-3.39 (m, 2H, N-CH₂); 3.61-3.66 (m, 2H, N-CH₂); 5.71 (q, J9.1 Hz, 2H, CF₃-CH₂); 7.31 (dd, *J* 9.8, 2.4 Hz, 1H, Ar); 7.75 (dd, *J* 9.8, 0.5 Hz, 1H, Ar); 8.15 (d, *J* 1.0 Hz, 1H, Ar); 8.70 (s, 1H, Ar); 9.23 (dd, *J* 2.4, 0.5 Hz, 1H, Ar); 9.61 (d, *J* 1.0 Hz, 1H, Ar). M/Z (M+H)⁺: 473

### Compound 60 : Azepan-1-yl-[3-(6,8-difluoro-imidazo[1,2-a]pyridin-3-yl)-1-(2,2,2-trifluoro-ethyl)-1H-pyrazolo[4,3-c]pyridin-6-yl]-methanone

Compound 60 was obtained according to General Procedure VI, starting from azepan-1-yl-[3-bromo-1-(2,2,2-trifluoroethyl)pyrazolo[4,3-c]pyridin-6-yl]methanone F3 and 6,8-difluoro-imidazo[1,2-a]pyridine H6 (1.3 equiv.). Purification by flash chromatography (DCM/MeOH: 100/0 to 95/5) afforded 60 as a white powder in 24% yield. ¹H-NMR (DMSO-*d*₆,400MHz): 1.52-1.66 (m, 6H, CH₂); 1.74-1.82 (m, 2H, CH₂); 3.34-3.39 (m, 2H, N-CH₂); 3.62-3.66 (m, 2H, N-CH₂); 5.76 (q, *J* 9.2 Hz, 2H, CF₃-CH₂); 7.81 (ddd, *J* 11.1, 9.2, 2.0 Hz, 1H, Ar); 8.16 (d, *J* 0.8 Hz, 1H, Ar); 8.86 (s, 1H, Ar); 9.47-9.50 (ddd, *J* 4.7, 2.0, 0.5 Hz, 1H, Ar); 9.66 (d, *J* 0.8 Hz, 1H, Ar). M/Z (M+H)⁺: 479

### Compound 61 : Azepan-1-yl-[3-(6-methyl-imidazo[1,2-a]pyridin-3-yl)-1-(2,2,2-trifluoroethyl)-1H-pyrazolo[4,3-c]pyridin-6-yl]-methanone

Compound 61 was obtained according to General Procedure VI, starting from azepan-1-yl-[3-bromo-1-(2,2,2-trifluoroethyl)pyrazolo[4,3-c]pyridin-6-yl]methanone F3 and 6-methyl-imidazo[1,2-a]pyridine H29 (1.3 equiv.). Purification by flash chromatography (DCM/MeOH: 100/0 to 95/5) afforded 61 as a white powder in 32% yield. ¹H-NMR (DMSO-*d*₆, 400MHz): 1.53-1.66 (m, 6H, CH₂); 1.75-1.83 (m, 2H, CH₂); 2.41 (s, 3H, CH₃); 3.34-3.39 (m, 2H, N-CH₂); 3.62-3.66 (m, 2H, N-CH₂); 5.71 (q, *J* 9.0 Hz, 2H, CF₃-CH₂); 7.36 (dd, *J* 9.2, 1.7 Hz, 1H, Ar); 7.73 (dd, *J* 9.2, 0.4 Hz, 1H, Ar); 8.13 (d, *J* 0.9 Hz, 1H, Ar); 8.69 (s, 1H, Ar); 9.31-9.34 (m, 1H, Ar); 9.59 (d, J0.9 Hz, 1H, Ar). M/Z (M+H)⁺: 457

### Compound 62 : Azepan-1-yl-[3-(8-fluoro-imidazo[1,2-a]pyridin-3-yl)-1-(2,2,2-trifluoroethyl)-1H-pyrazolo[4,3-c]pyridin-6-yl]-methanone

Compound 62was obtained according to General Procedure VI, starting from azepan-1-yl-[3-bromo-1-(2,2,2-trifluoroethyl)pyrazolo[4,3-c]pyridin-6-yl]methanone F3 and 8-fluoro-imidazo[1,2-a]pyridine H30 (1.3 equiv.). Purification by flash chromatography (DCM/MeOH: 10/0 to 9/1) afforded 62 as a white powder in 34% yield. ¹H-NMR (DMSO-*d*₆,400MHz): 1.51-1.66 (m, 6H, CH₂); 1.75-1.83 (m, 2H, CH₂); 3.35-3.39 (m, 2H, N-CH₂); 3.62-3.66 (m, 2H, N-CH₂); 5.71 (q, *J* 9.0 Hz, 2H, CF₃-CH₂); 7.23 (ddd, *J* 7.7, 7.0, 4.9 Hz, 1H, Ar); 7.41 (ddd, *J* 11.1, 7.7, 0.7 Hz, 1H, Ar); 8.16 (d, *J* 0.9 Hz, 1H, Ar); 8.80 (s, 1H, Ar); 9.36 (dd, *J* 7.0, 0.7 Hz, 1H, Ar); 9.64 (d, *J* 0.9 Hz, 1H, Ar). M/Z (M+H)⁺: 461

### Compound 63 : 3-[6-(Azepane-1-carbonyl)-1-(2,2,2-trifluoro-ethyl)-1H-pyrazolo[4,3-c]pyridin-3-yl]-7-hydroxy-imidazo[1,2-a]pyridine-6-carbonitrile

Compound 63 was obtained according to General Procedure VI, starting from azepan-1-yl-[3-bromo-1-(2,2,2-trifluoroethyl)pyrazolo[4,3-c]pyridin-6-yl]methanone F3 and 7-fluoro-imidazo[1,2-a]pyridine-6-carbonitrile H16 (1.3 equiv.). Purification by flash chromatography (DCM/MeOH: 10/0 to 9/1) afforded 63 as a white powder in 3% yield. ¹H-NMR (DMSO-*d*₆, 400MHz): 1.50-1.66 (m, 6H, CH₂); 1.74-1.82 (m, 2H, CH₂); 3.34-3.39 (m, 2H, N-CH₂); 3.62-3.66 (m, 2H, N-CH₂); 5.74 (q, J9.1 Hz, 2H, CF₃-CH₂); 6.91 (bs, 1H, Ar); 8.12 (d, *J* 0.7 Hz, 1H, Ar); 8.69 (s, 1H, Ar); 9.58 (d, *J* 0.7 Hz, 1H, Ar); 9.82 (bs, 1H, Ar); 12.02 (bs, 1H, OH). M/Z (M+H)⁺: 484

Compound 64 : Azepan-1-yl-[3-[6-(1-hydroxy-ethyl)-imidazo[1,2-a]pyridin-3-yl]-1-(2,2,2-trifluoro-ethyl)-1H-pyrazolo[4,3-c]pyridin-6-yl]-methanone Compound 64 was obtained according to General Procedure VI, starting from azepan-1-yl-[3-bromo-1-(2,2,2-trifluoroethyl)pyrazolo[4,3-c]pyridin-6-yl]methanone F3 and 6-(1-hydroxy-ethyl)-imidazo[1,2-a]pyridine H31 (1.3 equiv.). In that specific case, 2.5 equiv. of cesium pivalate and 10 mol% of {[P(tBu)₃]PdBr}₂ were used. Purification by flash chromatography (DCM/MeOH: 100/0 to 95/5) afforded 64 as a white powder in 42% yield. ¹H-NMR (DMSO-*d*₆,400MHz): 1.45 (d, *J* 6.3 Hz, 3H, CH₃); 1.55-1.64 (m, 6H, CH₂); 1.75-1.81 (m, 2H, CH₂); 3.36-3.38 (m, 2H, N-CH₂); 3.63-3.66 (m, 2H, N-CH₂); 4.86 (qd, *J* 6.3, 4.2 Hz, 1H, CH); 5.45 (d, *J* 4.2 Hz, 1H, OH), 5.68 (q, *J* 8.8 Hz, 2H, CF₃-CH₂); 7.50 (dd, *J* 9.2, 1.7 Hz, 1H, Ar); 7.77 (dd, *J* 9.2, 0.7 Hz, 1H, Ar); 8.15 (d, *J* 1.0 Hz, 1H, Ar); 8.71 (s, 1H, Ar); 9.49 (dd, *J* 1.7, 0.7 Hz, 1H, Ar); 9.59 (d, *J* 1.0 Hz, 1H, Ar). M/Z (M+H)⁺: 487

### Compound 65 : Azepan-1-yl-[3-(6-cyclopropyl-imidazo[1,2-a]pyridin-3-yl)-1-(2,2,2-trifluoro-ethyl)-1H-pyrazolo[4,3-c]pyridin-6-yl]-methanone

Compound 675 was obtained according to General Procedure VI, starting from azepan-1-yl-[3-bromo-1-(2,2,2-trifluoroethyl)pyrazolo[4,3-c]pyridin-6-yl]methanone F3 and 6-cyclopropyl-imidazo[1,2-a]pyridine H7 (1.3 equiv.). Purification by flash chromatography (DCM/MeOH: 100/0 to 95/5) afforded 65 as a beige powder in 17% yield. ¹H-NMR (DMSO-*d*₆,400MHz): 0.74-0.79 (m, 2H, CH₂); 1.00 -1.06 (m, 2H, CH₂); 1.53-1.66 (m, 6H, CH₂); 1.74-1.83 (m, 2H, CH₂); 2.03-2.11 (m, 1H, CH); 3.34-3.40 (m, 2H, N-CH₂); 3.62-3.66 (m, 2H, N-CH₂); 5.71 (q, *J* 9.0 Hz, 2H, CF₃-CH₂); 7.26 (dd, *J* 9.4, 1.6 Hz, 1H, Ar); 7.72 (d, *J* 9.4 Hz, 1H, Ar); 8.16 (d, *J* 0.9 Hz, 1H, Ar); 8.70 (s, 1H, Ar); 9.33-9.36 (m, 1H, Ar); 9.59 (d, *J* 0.9 Hz, 1H, Ar). M/Z (M+H)⁺: 483

### Compound 66 : Azepan-1-yl-[1-(2,2,2-trifluoro-ethyl)-3-(6-trifluoromethoxy-imidazo[1,2-a]pyridin-3-yl)-1H-pyrazolo[4,3-c]pyridin-6-yl]-methanone

Compound 66 was obtained according to General Procedure VI, starting from azepan-1-yl-[3-bromo-1-(2,2,2-trifluoroethyl)pyrazolo[4,3-c]pyridin-6-yl]methanone F3 and 6-trifluoromethoxy-imidazo[1,2-a]pyridine H8 (1.3 equiv.). Purification by flash chromatography (DCM/MeOH: 100/0 to 95/5) afforded 66 as a beige powder in 31% yield. ¹H-NMR (DMSO-*d*₆,400MHz): 1.52-1.65 (m, 6H, CH₂); 1.74-1.83 (m, 2H, CH₂); 3.34-3.40 (m, 2H, N-CH₂); 3.62-3.66 (m, 2H, N-CH₂); 5.73 (q, J9.0 Hz, 2H, CF₃-CH₂); 7.62 (dd, *J* 9.8, 1.5 Hz, 1H, Ar); 7.98 (dd, *J* 9.8, 0.7 Hz, 1H, Ar); 8.18 (d, *J* 0.9 Hz, 1H, Ar); 8.90 (s, 1H, Ar); 9.65 (d, *J* 0.9 Hz, 1H, Ar); 9.70-9.72 (m, 1H, Ar). M/Z (M+H)⁺: 527

### Compound 67 : Azepan-1-yl-[3-(7-fluoro-imidazo[1,2-a]pyridin-3-yl)-1-(2,2,2-trifluoroethyl)-1H-pyrazolo[4,3-c]pyridin-6-yl]-methanone

Compound 67 was obtained according to General Procedure VI, starting from azepan-1-yl-[3-bromo-1-(2,2,2-trifluoroethyl)pyrazolo[4,3-c]pyridin-6-yl]methanone F3 and 7-fluoro-imidazo[1,2-a]pyridine H36 (1.3 equiv.). Purification by flash chromatography (DCM/MeOH: 100/0 to 95/5) afforded 67 as a white powder in 24% yield. ¹H-NMR (DMSO-*d*₆,400MHz): 1.52-1.65 (m, 6H, CH₂); 1.75-1.83 (m, 2H, CH₂); 3.34-3.39 (m, 2H, N-CH₂); 3.62-3.66 (m, 2H, N-CH₂); 5.69 (q, *J* 9.0 Hz, 2H, CF₃-CH₂); 7.34 (td, *J* 7.6, 2.6 Hz, 1H, Ar); 7.71 (dd, *J* 9.9, 2.6 Hz, 1H, Ar); 8.14 (d, *J* 1.0 Hz, 1H, Ar); 8.76 (s, 1H, Ar); 9.54 (ddd, *J* 7.6, 5.8, 0.5 Hz, 1H, Ar); 9.62 (d, *J* 1.0 Hz, 1H, Ar). M/Z (M+H)⁺: 461

### Compound 68 : Azepan-1-yl-[3-(6-hydroxy-imidazo[1,2-a]pyridin-3-yl)-1-(2,2,2-trifluoro-ethyl)-1H-pyrazolo[4,3-c]pyridin-6-yl]-methanone

Compound 68 was obtained according to General Procedure VI, Alternative 1, starting from azepan-1-yl-[3-bromo-1-(2,2,2-trifluoroethyl)pyrazolo[4,3-c]pyridin-6-yl]methanone F3 and 6-hydroxy-imidazo[1,2-a]pyridine H33. Purification by flash chromatography (DCM/MeOH: 10/0 to 9/1) afforded 68 as a beige powder in 59% yield. ¹H-NMR (DMSO-*d*₆,400MHz): 1.52-1.65 (m, 6H, CH₂); 1.75-1.82 (m, 2H, CH₂); 3.35-3.40 (m, 2H, N-CH₂); 3.62-3.66 (m, 2H, N-CH₂); 5.66 (q, *J* 9.0 Hz, 2H, CF₃-CH₂); 7.20 (dd, J9.5, 2.3 Hz, 1H, Ar); 7.68 (d, J9.5 Hz, 1H, Ar); 8.13 (d, *J* 0.8 Hz, 1H, Ar); 8.64 (s, 1H, Ar); 9.10 (dd, *J* 2.3, 0.6 Hz, 1H, Ar); 9.59 (d, *J* 0.8 Hz, 1H, Ar); 9.87 (s, 1H, OH). M/Z (M+H)⁺: 459

### Compound 69 : Azepan-1-yl-[3-(6-difluoromethyl-imidazo[1,2-a]pyridin-3-yl)-1-(2,2,2-trifluoro-ethyl)-1H-pyrazolo[4,3-c]pyridin-6-yl]-methanone

Compound 69 was obtained according to General Procedure VI, Alternative 1, starting from azepan-1-yl-[3-bromo-1-(2,2,2-trifluoroethyl)pyrazolo[4,3-c]pyridin-6-yl]methanone F3 and 6-difluoromethyl-imidazo[1,2-a]pyridine H9. Purification by flash chromatography (DCM/MeOH: 100/0 to 95/5) afforded 69 as a beige powder in 19% yield. ¹H-NMR (DMSO-*d*₆,400MHz): 1.51-1.67 (m, 6H, CH₂); 1.74-1.83 (m, 2H, CH₂); 3.34-3.40 (m, 2H, N-CH₂); 3.61-3.68 (m, 2H, N-CH₂); 5.70 (q, *J* 9.2 Hz, 2H, CF₃-CH₂); 7.28 (t, *J* 55.3 Hz, 1H, CH-F₂); 7.62 (dd, *J* 9.3, 1.5 Hz, 1H, Ar); 7.95 (d, *J* 9.3 Hz, 1H, Ar); 8.17 (d, *J* 0.8 Hz, 1H, Ar); 8.86 (s, 1H, Ar); 9.64 (d, *J* 0.8 Hz, 1H, Ar); 9.78-9.81 (m, 1H, Ar). M/Z (M+H)⁺: 493

### Compound 70 : Azepan-1-yl-[3-[6-(2,2,2-trifluoro-ethoxy)-imidazo[1,2-a]pyridin-3-yl]-1-(2,2,2-trifluoro-ethyl)-1H-pyrazolo[4,3-c]pyridin-6-yl]-methanone

Compound 70 was obtained according to General Procedure VI, Alternative 1, starting from azepan-1-yl-[3-bromo-1-(2,2,2-trifluoroethyl)pyrazolo[4,3-c]pyridin-6-yl]methanone F3 and 6-(2,2,2-trifluoro-ethoxy)-imidazo[1,2-a]pyridine H4. Purification by flash chromatography (DCM/MeOH: 100/0 to 95/5) afforded 70 as a white powder in 22% yield. ¹H-NMR (DMSO-*d*₆,400MHz): 1.53-1.66 (m, 6H, CH₂); 1.75-1.83 (m, 2H, CH₂); 3.345-3.39 (m, 2H, N-CH₂); 3.62-3.68 (m, 2H, N-CH₂); 4.88 (q, *J* 8.8 Hz, 2H, O-CH₂-CF₃); 5.72 (q, *J* 9.0 Hz, 2H, CF₃-CH₂); 7.48 (dd, *J* 9.8, 2.5 Hz, 1H, Ar); 7.84 (dd, *J* 9.8, 0.5 Hz, 1H, Ar); 8.15 (d, J0.9 Hz, 1H, Ar); 8.76 (s, 1H, Ar); 9.35 (dd, J2.5, 0.5 Hz, 1H, Ar); 9.62 (d, *J* 0.9Hz, 1H, Ar). M/Z (M+H)⁺: 541

### Compound 71 : Azepan-1-yl-[3-[1,2,4]triazolo[4,3-a]pyridin-3-yl-1-(2,2,2-trifluoroethyl)-1H-pyrazolo[4,3-c]pyridin-6-yl]-methanone

Compound 71 was obtained according to General Procedure VI, Alternative 1, starting from azepan-1-yl-[3-bromo-1-(2,2,2-trifluoroethyl)pyrazolo[4,3-c]pyridin-6-yl]methanone F3 and [1,2,4]triazolo[4,3-a]pyridine H34. Purification by flash chromatography (DCM/MeOH: 100/0 to 95/5) afforded 71 as a white powder in 5% yield. ¹H-NMR (DMSO-*d*₆,400MHz): 1.50-1.69 (m, 6H, CH₂); 1.76-1.83 (m, 2H, CH₂); 3.34-3.39 (m, 2H, N-CH₂); 3.62-3.69 (m, 2H, N-CH₂); 5.78 (q, *J* 9.2 Hz, 2H, CF₃-CH₂); 7.35 (t, *J* 6.9 Hz, 1H, Ar); 7.62 (dd, *J* 8.8, 6.9 Hz, 1H, Ar); 8.05 (d, *J* 8.8, Hz, 1H, Ar); 8.23 (bs, 1H, Ar); 9.32 (d, *J* 6.9 Hz, 1H, Ar); 9.75 (bs, 1H, Ar). M/Z (M+H)⁺: 444

### Compound 72 : Azepan-1-yl-[3-[6-(1-hydroxy-1-methyl-ethyl)-imidazo[1,2-a]pyridin-3-yl]-1-(2,2,2-trifluoro-ethyl)-1H-pyrazolo[4,3-c]pyridin-6-yl]-methanone

Compound 72 was obtained according to General Procedure VI, Alternative 1, starting from azepan-1-yl-[3-bromo-1-(2,2,2-trifluoroethyl)pyrazolo[4,3-c]pyridin-6-yl]methanone F3 and 6-(1-hydroxy-1-methyl-ethyl)-imidazo[1,2-a]pyridine H35. Purification by flash chromatography (DCM/MeOH: 10/0 to 9/1) afforded 72 as a white powder in 27% yield. ¹H-NMR (DMSO-*d*₆,400MHz): 1.54 (s, 6H, CH₃); 1.55-1.64 (m, 6H, CH₂); 1.75-1.81 (m, 2H, CH₂); 3.36-3.38 (m, 2H, N-CH₂); 3.63-3.66 (m, 2H, N-CH₂); 5.31 (s, 1H, OH); 5.66 (q, *J* 9.0 Hz, 2H, CF₃-CH₂); 7.62 (dd, *J* 9.4, 1.6 Hz, 1H, Ar); 7.75 (dd, *J* 9.4, 0.8 Hz, 1H, Ar); 8.16 (d, J0.8 Hz, 1H, Ar); 8.70 (s, 1H, Ar); 9.59 (d, *J* 0.8 Hz, 1H, Ar); 9.59-9.61 (m, 1H, Ar). M/Z (M+H)⁺: 501

### Compound 73: Azepan-1-yl-[3-(7-chloro-6-fluoro-imidazo[1,2-a]pyridin-3-yl)-1-(2,2,2-trifluoro-ethyl)-1H-pyrazolo[4,3-c]pyridin-6-yl]-methanone

Compound 73 was obtained according to General Procedure VI, Alternative 2, starting from azepan-1-yl-[3-bromo-1-(2,2,2-trifluoroethyl)pyrazolo[4,3-c]pyridin-6-yl]methanone F3 and 7-chloro-6-fluoro-imidazo[1,2-a]pyridine H10. Purification by flash chromatography (DCM/MeOH: 10/0 to 9/1) afforded 73 as a yellow powder in 27% yield. ¹H-NMR (DMSO-*d*₆,400MHz): 1.54-1.64 (m, 6H, CH₂); 1.75-1.81 (m, 2H, CH₂); 3.35-3.38 (m, 2H, N-CH₂), 3.63-3.66 (m, 2H, N-CH₂); 5.75 (q, J9.0 Hz, 2H, CF₃-CH₂); 8.15 (d, *J* 1.0 Hz, 1H, Ar); 8.26 (d, *J* 7.2 Hz, 1H, Ar); 8.87 (s, 1H, Ar); 9.65 (d, *J* 1.0 Hz, 1H, Ar); 9.71 (d, *J* 5.4 Hz, 1H, Ar). M/Z (M+H)⁺: 495/497

### Compound 74 : Azepan-1-yl-[3-(6-chloro-7-fluoro-imidazo[1,2-a]pyridin-3-yl)-1-(2,2,2-trifluoro-ethyl)-1H-pyrazolo[4,3-c]pyridin-6-yl]-methanone

Compound 74 was obtained according to General Procedure VI, Alternative 2, starting from azepan-1-yl-[3-bromo-1-(2,2,2-trifluoroethyl)pyrazolo[4,3-c]pyridin-6-yl]methanone F3 and 6-chloro-7-fluoro-imidazo[1,2-a]pyridine H11. Purification by flash chromatography (DCM/MeOH: 10/0 to 9/1) afforded 74 as a white powder in 22% yield. ¹H-NMR (DMSO-*d*₆,400MHz): 1.52-1.65 (m, 6H, CH₂); 1.72-1.82 (m, 2H, CH₂); 3.33-3.40 (m, 2H, CH₂); 3.60-3.68 (m, 2H, CH₂); 5.70-5.82 (m, 2H, CF₃-CH₂); 8.00 (d, *J* 10.0 Hz, 1H, Ar); 8.15 (bs, 1H, Ar); 8.82 (s, 1H, Ar); 9.63 (bs, 1H, Ar); 9.72 (d, *J* 7.2 Hz, 1H, Ar). M/Z (M+H)⁺: 495/497

### Compound 75 : Azepan-1-yl-[3-(6-chloro-7-hydroxy-imidazo[1,2-a]pyridin-3-yl)-1-(2,2,2-trifluoro-ethyl)-1H-pyrazolo[4,3-c]pyridin-6-yl]-methanone

Compound 75 was obtained according to General Procedure VI, Alternative 2, starting from azepan-1-yl-[3-bromo-1-(2,2,2-trifluoroethyl)pyrazolo[4,3-c]pyridin-6-yl]methanone F3 and 6-chloro-7-fluoro-imidazo[1,2-a]pyridine H11. Purification by flash chromatography (DCM/MeOH: 10/0 to 9/1) afforded 75 as a yellow powder in 17% yield. ¹H-NMR (DMSO-*d*₆,400MHz): 1.50-1.66 (m, 6H, CH₂); 1.72-1.83 (m, 2H, CH₂); 3.22-3.40 (m, 2H, CH₂); 3.58-3.70 (m, 2H, CH₂); 5.72 (q, *J* 9.0 Hz, 2H, CF₃-CH₂); 6.90-7.10 (m, 1H, Ar); 8.12 (s, 1H, Ar); 8.61 (s, 1H, Ar); 9.53 (s, 1H, Ar); 9.57 (s, 1H, Ar); 11.61 (bs, 1H, OH). M/Z (M+H)⁺: 493/495

### Compound 76 : Azepan-1-yl-[3-(6,8-difluoro-imidazo[1,2-a]pyridin-3-yl)-1-(2-trimethylsilanyl-ethoxymethyl)-1H-pyrazolo[4,3-c]pyridin-6-yl]-methanone

Compound 76 was obtained according to General Procedure VI, Alternative 2, starting from azepan-1-yl-[3-bromo-1-(2-trimethylsilylethoxymethyl)pyrazolo[4,3-c]pyridin-6-yl]methanone F4 and 6,8-difluoro-imidazo[1,2-a]pyridine H6. Purification by flash chromatography (Cyclohexane/AcOEt : 10/0 to 0/10) afforded 76 as a beige powder in 73% yield. ¹H-NMR (DMSO-*d*₆,400MHz): -0.12 (s, 9H, CH₃); 0.87 (dd, *J* 8.0, 2.8 Hz, 2H, N-CH₂-O-CH₂-CH₂-SiMe₃); 1.54-1.64 (m, 6H, CH₂); 1.75-1.81 (m, 2H, CH₂); 3.36-3.40 (m, 2H, N-CH₂); 3.62-3.69 (m, 4H, N-CH₂ + N-CH₂-O-CH₂-CH₂-SiMe₃); 5.99 (s, 2H, N-CH₂-O-CH₂-CH₂-SiMe₃); 7.79 (ddd, *J* 11.0, 9.1, 2.0 Hz, 1H, Ar); 8.09 (d, *J* 0.9 Hz, 1H, Ar); 8.85 (s, 1H, Ar); 9.51-9.53 (m, 1H, Ar); 9.63 (d, J0.9 Hz, 2H, Ar). M/Z (M+H)⁺: 527

### Compound 77 : Azepan-1-yl-[3-(6-fluoro-imidazo[1,2-a]pyridin-3-yl)-1-(2-trimethylsilanyl-ethoxymethyl)-1H-pyrazolo[4,3-c]pyridin-6-yl]-methanone

Compound 77 was obtained according to General Procedure VI, starting from azepan-1 -yl-[3-bromo-1 -(2-trimethylsilylethoxymethyl)pyrazolo[4,3-c]pyridin-6-yl]methanone F4 and 6-fluoro-imidazo[1,2-a]pyridine H19 (1.3 equiv.). Purification by flash chromatography (DCM/MeOH : 100/0 to 93/7) afforded 77 as a beige powder in 67% yield. ¹H-NMR (DMSO-*d*₆,400MHz): 0.11 (s, 9H, 3CH₃); 0.87 (t, J8.1 Hz, 2H, O-CH₂-CH₂-SiMe₃) ; 1.54-1.64 (m, 6H, CH₂); 1.75-1.81 (m, 2H, CH₂); 3.36-341 (m, 2H, N-CH₂); 3.62-3.69 (m, 4H, N-CH₂, O-CH₂-CH₂-SiMe₃); 5.99 (s, 2H, N-CH₂ O-CH₂-CH₂-SiMe₃); 7.59 (ddd, *J* 10.0, 8.1, 2.5 Hz, 1H, Ar); 7.90 (dd, *J* 10.0, 5.4 Hz, 1H, Ar); 8.07 (d, *J* 1.1 Hz, 1H, Ar); 8.83 (s, 1H, Ar) ; 9.61-9.63 (m, 2H, Ar). M/Z (M+H)⁺: 509

### Compound 78 : Azepan-1-yl-[3-(6,8-difluoro-imidazo[1,2-a]pyridin-3-yl)-1-(4-methoxybenzyl)-1H-pyrazolo[4,3-c]pyridin-6-yl]-methanone

Compound 78 was obtained according to General Procedure VI, Alternative 2, starting from azepan-1-yl-[3-bromo-1-[(4-methoxyphenyl)methyl]pyrazolo[4,3-c]pyridin-6-yl]methanone F5 and 6,8-difluoro-imidazo[1,2-a]pyridine H6. Purification by flash chromatography (DCM/MeOH : 100/0 to 97/3) afforded 78 as a white powder in 77% yield. ¹H-NMR (DMSO-*d*₆,400MHz): 1.50-1.64 (m, 6H, CH₂); 1.72-1.82 (m, 2H, CH₂); 3.31-3.36 (m, 2H, N-CH₂); 3.59-3.65 (m, 2H, N-CH₂); 3.70 (s, 3H, CH₃); 5.80 (bs, 2H, CH₂); 6.88-6.92 (m, 2H, Ar); 7.36-7.40 (m, 2H, Ar); 7.76 (ddd, *J* 11.1, 9.1, 2.1 Hz); 8.05 (d, *J* 1.0 Hz, 1H, Ar); 8.79 (s, 1H, Ar); 9.49 (ddd, *J* 4.8, 2.1, 0.6 Hz); 9.58 (d, *J* 1.0 Hz, 1H, Ar). M/Z (M+H)⁺: 517

### Compound 79 : Azepan-1-yl-[3-(6,8-difluoro-imidazo[1,2-a]pyridin-3-yl)-1-(tetrahydropyran-2-yl)-1H-pyrazolo[4,3-c]pyridin-6-yl]-methanone

Compound 79 was obtained according to General Procedure VI, Alternative 2, starting from azepan-1-yl-(3-bromo-1-tetrahydropyran-2-yl-pyrazolo[4,3-c]pyridin-6-yl)methanone F6 and 6,8-difluoro-imidazo[1,2-a]pyridine H6. Purification by flash chromatography (DCM/MeOH : 100/0 to 95/5) afforded 79 as a white powder in 57% yield. ¹H-NMR (DMSO-*d*₆,400MHz): 1.53-1.74 (m, 8H, CH₂); 1.74-1.89 (m, 3H, CH₂); 2.06-2.19 (m, 2H, CH₂); 2.38-2.64 (m, 1H, CH₂); 3.35-3.44 (m, 2H, N-CH₂); 3.58-3.71 (m, 2H, N-CH₂); 3.81-3.89 (m, 1H, O-CH₂O); 3.93-3.99 (m, 1H, O-CH₂); 6.13 (dd, *J* 9.3, 1.9Hz, 1H, CH); 7.79 (ddd, *J* 11.1, 9.1, 2.0 Hz); 8.04 (d, *J* 1.0 Hz, 1H, Ar); 8.84 (s, 1H, Ar); 9.49 (ddd, *J* 4.8, 2.0, 0.8 Hz); 9.62 (d, *J* 1.0 Hz, 1H, Ar). M/Z (M+H)⁺: 481

### Compound 80 : [3-(6-Fluoro-imidazo[1,2-a]pyridin-3-yl)-1-(2,2,2-trifluoro-ethyl)-1H-pyrazolo[4,3-c]pyridin-6-yl]-1,4-oxazepan-4-yl-methanone

Compound 80 was obtained according to General Procedure VI, starting from [3-bromo-1-(2,2,2-trifluoroethyl)pyrazolo[4,3-c]pyridin-6-yl]-(1,4-oxazepan-4-yl)methanone F7 and 6-fluoro-imidazo[1,2-a]pyridine H19 (1.3 equiv.). Purification by flash chromatography (DCM/MeOH : 10/0 to 9/1) afforded 80 as a white powder in 26% yield. ¹H-NMR (DMSO-*d*₆,400MHz): 1.74-1.82 (m, 1H, CH₂); 1.91-1.99 (m, 1H, CH₂); 3.48-3.55 (m, 2H, CH₂); 3.63-3.69 (m, 1H, CH₂); 3.70-3.83 (m, 5H, CH₂); 5.76 (q, *J* 9.0 Hz, 2H, CF₃-CH₂); 7.61 (ddd, *J* 9.9, 8.2, 2.5 Hz, 1H, Ar); 7.92 (dd, *J* 9.9, 5.3 Hz, 1H, Ar); 8.20 (bs, 1H, Ar); 8.85 (s, 1H, Ar, signal of a rotamer); 8.86 (s, 1H, Ar, signal of a rotamer); 9.59 (ddd, *J* 4.9, 2.5, 1.5 Hz, 1H, Ar); 9.65 (bs, 1H, Ar, signal of a rotamer); 9.67 (bs, 1H, Ar, signal of a rotamer). M/Z (M+H)⁺: 463

### Compound 81 : 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carbonitrile

Compound 81 was obtained according to General Procedure VI, Alternative 2, starting from [3-bromo-1-(2,2,2-trifluoroethyl)pyrazolo[4,3-c]pyridin-6-yl]-(1,4-oxazepan-4-yl)methanone F7 and 6-fluoro-imidazo[1,2-a]pyridine H20. Purification by flash chromatography (DCM/MeOH : 100/0 to 93/7) afforded 81 as a beige powder in 59% yield. ¹H-NMR (DMSO-*d*₆, 400MHz): 1.75-1.80 (m, 1H, CH₂); 1.92-1.98 (m, 1H, CH₂); 3.49-3.53 (m, 2H, N-CH₂); 3.64-3.67 (m, 1H, N-CH₂); 3.71-3.80 (m, 5H, N-CH₂, O-CH₂); 5.79 (q, *J* 9.1 Hz, 2H, CH₂-CF₃); 7.75 (dd, *J* 9.3, 1.7 Hz, 1H, Ar); 7.99 (dd, *J* 9.3, 0.8 Hz, 1H, Ar); 8.21 (d, *J* 1.0 Hz, 1H, signal of a rotamer, Ar); 8.22 (d, *J* 1.0 Hz, 1H, signal of a rotamer, Ar); 8.93 (s, 1H, signal of a rotamer, Ar); 8.94 (s, 1H, signal of a rotamer, Ar); 9.65 (d, *J* 1.0 Hz, 1H, signal of a rotamer, Ar); 9.67 (d, *J* 1.0 Hz, 1H, signal of a rotamer, Ar); 10.02 (dd, *J* 1.7, 0.8 Hz, 1H, signal of a rotamer, Ar); 10.03 (dd, *J* 1.7, 0.8 Hz, 1H, signal of a rotamer, Ar). M/Z (M+H)⁺: 470

### Compound 82 : [3-(6-Fluoro-imidazo[1,2-a]pyridin-3-yl)-1-(2,2,2-trifluoro-ethyl)-1H-pyrazolo[4,3-c]pyridin-6-yl]-(8-oxa-3-aza-bicyclo[3.2.1]oct-3-yl)-methanone

Compound 82 was obtained according to General Procedure VI, Alternative 2, starting from [3-bromo-1-(2,2,2-trifluoroethyl)pyrazolo[4,3-c]pyridin-6-yl]-(8-oxa-3-azabicyclo[3.2.1]octan-3-yl)methanone F8 and 6-fluoro-imidazo[1,2-a]pyridine H19. Purification by flash chromatography (DCM/MeOH : 100/0 to 92/8) afforded 82 as a beige powder in 48% yield. ¹H-NMR (DMSO-*d*₆,400MHz): 1.73-1.93 (m, 4H, CH₂); 3.03-3.11 (m, 1H, CH); 3.32-3.44 (m, 2H, N-CH₂); 4.18-4.27 (m, 2H, N-CH₂); 4.41-4.47 (m, 1H, CH); 5.70-5.82 (m, 2H, CF₃-CH₂); 7.60 (ddd, *J* 10.1, 8.1, 2.4 Hz, 1H, Ar); 7.91 (dd, *J* 10.1, 5.3 Hz, 1H, Ar); 8.21 (bs, 1H, Ar); 8.84 (s, 1H, Ar); 9.58 (dd, *J* 5.3, 2.4 Hz, 1H, Ar); 9.62-9.67 (m, 1H, Ar). M/Z (M+H)⁺: 475

### Compound 83 : [3-(6,8-Difluoro-imidazo[1,2-a]pyridin-3-yl)-1-(2,2,2-trifluoro-ethyl)-1H-pyrazolo[4,3c]pyridin-6-yl]-(8-oxa-3-aza-bicyclo[3.2.1]oct-3-yl)-methanone

Compound 83 was obtained according to General Procedure VI, starting from [3-bromo-1-(2,2,2-trifluoroethyl)pyrazolo[4,3-c]pyridin-6-yl]-(8-oxa-3-azabicyclo[3.2.1]octan-3-yl)methanone F8 and 6,8-difluoro-imidazo[1,2-a]pyridine H6 (1.3 equiv.). In that specific case, 10 mol% of {[P(tBu)₃]PdBr}₂ was used. Purification by flash chromatography (DCM/MeOH : 100/0 to 94/6) afforded 83 as a beige powder in 51% yield. ¹H-NMR (DMSO-*d*₆, 400MHz): 1.73-1.94 (m, 4H, CH₂); 3.04-3.11 (m, 1H, CH); 3.33-3.43 (m, 2H, N-CH₂); 4.19-4.26 (m, 2H, N-CH₂); 4.40-4.47 (m, 1H, CH); 5.70-5.82 (m, 2H, CF₃-CH₂); 7.81 (ddd, *J* 11.1, 9.1, 2.1 Hz, 1H, Ar); 8.23 (br s, 1H, Ar); 8.87 (s, 1H, Ar); 9.46-9.50 (ddd, *J* 4.7, 2.1, 0.6 Hz, 1H, Ar); 9.68 (d, *J* 1.0 Hz, 1H, Ar). M/Z (M+H)⁺: 493

### Compound 84 : 3-[6-(8-Oxa-3-aza-bicyclo[3.2.1]octane-3-carbonyl)-1-(2,2,2-trifluoroethyl)-1H-pyrazolo[4,3-c]pyridin-3-yl]-imidazo[1,2-a]pyridine-6-carbonitrile

Compound 84 was obtained according to General Procedure VI, Alternative 2, starting from [3-bromo-1-(2,2,2-trifluoroethyl)pyrazolo[4,3-c]pyridin-6-yl]-(8-oxa-3-azabicyclo[3.2.1]octan-3-yl)methanone F8 and imidazo[1,2-a]pyridine-6-carbonitrile H20. Purification by flash chromatography (DCM/MeOH : 100/0 to 93/7) afforded 84 as a beige powder in 61% yield. ¹H-NMR (DMSO-*d*₆,400MHz): 1.74-1.92 (m, 4H, CH₂); 3.06-3.09 (m, 1H, N-CH₂); 3.35-3.42 (m, 2H, N-CH₂); 4.21-4.26 (m, 2H, N-CH₂, O-CH); 4.43-4.45 (m, 1H, O-CH); 5.74-5.85 (m, 2H, CH₂-CF₃); 7.75 (dd, *J* 9.3, 1.7 Hz, 1H, Ar); 7.99 (dd, *J* 9.3, 0.9 Hz, 1H, Ar); 8.23 (bs, 1H, Ar); 8.94 (s, 1H, Ar); 9.66 (d, *J* 1.0 Hz, 1H, Ar); 10.02 (dd, *J* 1.7, 0.9 Hz, 1H, Ar). M/Z (M+H)⁺: 482

### Compound 85 : [3-(8-fluoroimidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone

Compound 85 was obtained according to General Procedure VI, Alternative 2, starting from [3-bromo-1-(2,2,2-trifluoroethyl)pyrazolo[4,3-c]pyridin-6-yl]-(3-*endo-*hydroxy-8-azabicyclo[3.2.1]octan-8-yl)methanone F30 and 8-fluoro-imidazo[1,2-a]pyridine H30. Purification by flash chromatography (DCM/MeOH : 10/0 to 9/1) afforded 85 as a beige powder in 20% yield. ¹H-NMR (DMSO-d6, 400MHz): 1.66-1.73 (m, 1H, CH₂); 1.79-1.95 (m, 3H, CH₂); 2.04-2.13 (m, 2H, CH₂); 2.19-2.33 (m, 2H, CH₂); 3.97-4.03 (m, 1H, CH); 4.50-4.56 (m, 1H, CH); 4.66-4.72 (m, 2H, OH, CH); 5.75 (q, *J* 9.0 Hz, 2H, CH₂-CF₃); 7.19-7.26 (m, 1H, Ar); 7.42 (dd, *J* 11.0, 7.7 Hz, 1H, Ar); 8.31 (bs, 1H, Ar); 8.81 (s, 1H, Ar); 9.36 (dd, *J* 6.9, 0.6 Hz, 1H, Ar); 9.65 (d, *J* 1.0 Hz, 1H, Ar). M/Z (M+H)+: 489

### Compound 86 : [3-(6-fluoroimidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone

Compound 86 was obtained according to General Procedure VI, Alternative 2, starting from [3-bromo-1-(2,2,2-trifluoroethyl)pyrazolo[4,3-c]pyridin-6-yl]-(3-*endo-*hydroxy-8-azabicyclo[3.2.1]octan-8-yl)methanone F30 and 6-fluoro-imidazo[1,2-a]pyridine H19. Purification by flash chromatography (DCM/MeOH : 10/0 to 9/1) afforded 86 as a white powder in 53% yield. ¹H-NMR (DMSO-d6, 400MHz): 1.66-1.73 (m, 1H, CH₂); 1.79-1.95 (m, 3H, CH₂); 2.02-2.12 (m, 2H, CH₂); 2.18-2.34 (m, 2H, CH₂); 3.97-4.03 (m, 1H, CH); 4.46-4.51 (m, 1H, CH); 4.64-4.71 (m, 2H, OH, CH); 5.77 (q, *J* 9.0 Hz, 2H, CH₂-CF₃); 7.60 (ddd, *J* 10.0, 8.1, 2.4 Hz, 1H, Ar); 7.91 (dd, *J* 10.0, 5.3 Hz, 1H, Ar); 8.29 (bs, 1H, Ar); 8.84 (s, 1H, Ar); 9.58 (dd, *J* 5.1, 2.4, 0.4 Hz, 1H, Ar); 9.65 (d, *J* 1.0 Hz, 1H, Ar). M/Z (M+H)+: 489

### Compound 87 : [3-(imidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone

Compound 87 was obtained according to General Procedure VI, Alternative 2, starting from [3-bromo-1-(2,2,2-trifluoroethyl)pyrazolo[4,3-c]pyridin-6-yl]-(3-*endo-*hydroxy-8-azabicyclo[3.2.1]octan-8-yl)methanone F30 and imidazo[1,2-a]pyridine H25. Purification by flash chromatography (DCM/MeOH : 10/0 to 9/1) afforded 87 as a white powder in 54% yield. ¹H-NMR (DMSO-d6, 400MHz): 1.66-1.73 (m, 1H, CH₂); 1.80-1.97 (m, 3H, CH₂); 2.02-2.12 (m, 2H, CH₂); 2.18-2.34 (m, 2H, CH₂); 3.97-4.03 (m, 1H, CH); 4.46-4.51 (m, 1H, CH); 4.64-4.71 (m, 2H, OH, CH); 5.73 (q, *J* 9.0 Hz, 2H, CH₂-CF₃); 7.25 (td, *J* 6.8, 1.0 Hz, 1H, Ar); 7.50 (ddd, *J* 8.0, 6.7, 1.1 Hz, 1H, Ar); 7.82 (d, *J* 9.0 Hz, 1H, Ar); 8.29 (bs, 1H, Ar); 8.77 (s, 1H, Ar); 9.53 (d, *J* 6.9 Hz, 1H, Ar); 9.63 (d, *J* 0.9 Hz, 1H, Ar). M/Z (M+H)+: 471

### Compound 88 : (3-endo-hydroxy-8-azabicyclo[3.2.1]octan-8-yl)-[3-(6-hydroxyimidazo[1,2-a]pyridin-3-yl)-1-(2,2,2-trifluoroethyl)pyrazolo[4,3-c]pyridin-6-yl]methanone

Compound 88 was obtained according to General Procedure VI, Alternative 1, starting from [3-bromo-1-(2,2,2-trifluoroethyl)pyrazolo[4,3-c]pyridin-6-yl]-(3-*endo-*hydroxy-8-azabicyclo[3.2.1]octan-8-yl)methanone F30 and 6-hydroxy-imidazo[1,2-a]pyridine H33. Purification by flash chromatography (DCM/MeOH : 10/0 to 9/1) afforded 88 as a white powder in 42% yield. ¹H-NMR (DMSO-d6, 400MHz): 1.66-1.73 (m, 1H, CH₂); 1.80-1.97 (m, 3H, CH₂); 2.01-2.13 (m, 2H, CH₂); 2.18-2.34 (m, 2H, CH₂); 3.97-4.03 (m, 1H, CH); 4.46-4.51 (m, 1H, CH); 4.64-4.71 (m, 2H, OH, CH); 5.68 (q, *J* 9.0 Hz, 2H, CH₂-CF₃); 7.20 (dd, *J* 9.6, 2.0 Hz, 1H, Ar); 7.68 (d, *J* 9.6 Hz, 1H, Ar); 8.28 (bs, 1H, Ar); 8.65 (s, 1H, Ar); 9.10 (d, *J* 2.0 Hz, 1H, Ar); 9.60 (d, *J* 0.9 Hz, 1H, Ar); 9.88 (bs, 1H, OH). M/Z (M+H)+: 487

### General Procedure VII : Synthesis of compounds J, K, O and S from compounds D, F, N and M respectively (schemes 1&2)

Under argon atmosphere, to a solution of compound D, F, N or M in anhydrous dioxane (0.1M) were added compound I (1.2 equiv.) and a 1.2M potassium carbonate solution (3 equiv.). The solution was degassed with argon bubbling for 15min before addition of PdCl₂(dppf).CH₂Cl₂ (10 mol%). The reaction mixture was heated 2h at 100°C. The reaction mixture was diluted with AcOEt, washed with a saturated sodium bicarbonate solution and brine, dried over magnesium sulfate then concentrated. The resulting yellow oil was purified by flash chromatography to afford the compound or compound J, K, O or S, respectively.

### Alternative 1 :

Under argon atmosphere, to a solution of compound D, F, N or M in anhydrous DMA (0.1M) were added compound l (1.2 equiv.) and a 1.2M potassium carbonate solution (3 equiv.). The solution was degassed with argon bubbling for 15min before addition of PdCl₂(dppf).CH₂Cl₂ (10 mol%). The reaction mixture was heated 4h at 70°C. The reaction mixture was diluted with AcOEt, washed with a saturated sodium bicarbonate solution and brine, dried over magnesium sulfate then concentrated. The resulting yellow oil was purified by flash chromatography to afford the compound or J, K, O or S, respectively.

### Alternative 2 :

Under argon atmosphere, to a solution of the corresppnding bromo-aryl (1.5 equiv.) in anhydrous dioxane (0.1M) were added bispinacolatodiboron (1.7 equiv.) and potassium acetate (4 equiv.). The solution was degassed with argon bubbling for 5min before addition of Pd(PPh₃)₄ (15 mol%). The reaction mixture was heated overnight at 100°C. Compound F (1 equiv.) and a 1.2M potassium carbonate solution (3 equiv.) were added. The reaction mixture was heated 2h at 100°C. The reaction mixture was diluted with AcOEt, filtered on an hydrophobic cartridge then concentrated. The resulting yellow oil was purified by flash chromatography to afford the compound.

### Alternative 3 :

Under argon atmosphere, to a solution of compound F in anhydrous dioxane (0.1M) were added compound l (2.0 equiv.) and a 1.2M potassium carbonate solution (3 equiv.). The solution was degassed with argon bubbling for 15min before addition of XPhos PdG3 precatalyst (5 mol%). The reaction mixture was heated 1h at 60°C. The reaction mixture was diluted with AcOEt, washed with a saturated sodium bicarbonate solution and brine, dried over magnesium sulfate then concentrated. The resulting oil was purified by flash chromatography to afford the compound.

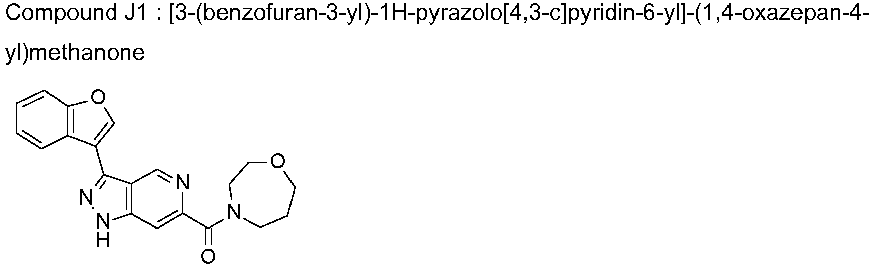

Compound J1 was obtained according to General Procedure VII, starting from 3-bromo-1H-pyrazolo[4,3-c]pyridin-6-yl)-(1,4-oxazepan-4-yl)methanone D2 and benzofuran-3-yl-boronic acid pinacol ester l2. In that specific case, the reaction mixture was heated 15min at 150°C under microwave irradiation. Purification by flash chromatography (DCM/MeOH: 100/0 to 95/5) afforded J1 as a beige powder in 72% yield. M/Z (M+H)⁺: 363

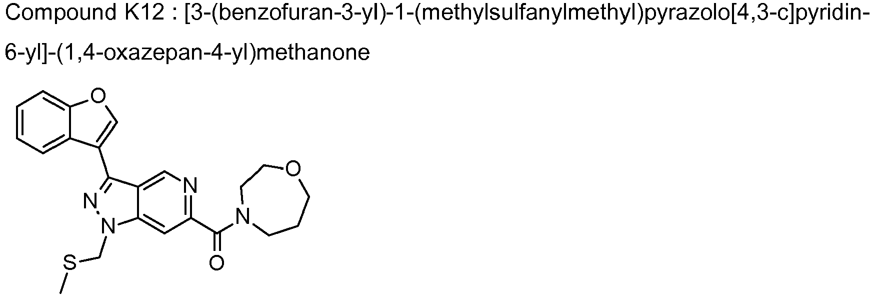

Compound K12 was obtained according to General Procedure VII, starting from [3-bromo-1-(methylsulfanylmethyl)pyrazolo[4,3-c]pyridin-6-yl]-(1,4-oxazepan-4-yl)methanone F16 and benzofuran-3-yl-boronic acid pinacol ester l2. Purification by flash chromatography (DCM/MeOH: 10/0 to 9/1) afforded K12 in 88% yield. M/Z (M+H)⁺: 423

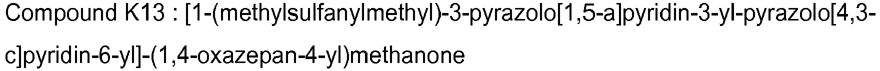

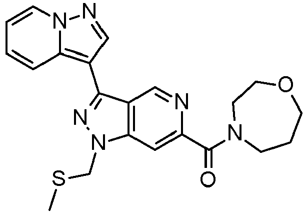

Compound K13 was obtained according to General Procedure VII, starting from [3-bromo-1-(methylsulfanylmethyl)pyrazolo[4,3-c]pyridin-6-yl]-(1,4-oxazepan-4-yl)methanone F16 and pyrazolo[1,5-a]pyridine-3-yl-boronic acid pinacol ester l1. Purification by flash chromatography (DCM/MeOH: 100/0 to 95/5) afforded K13 in 82% yield. M/Z (M+H)⁺: 423

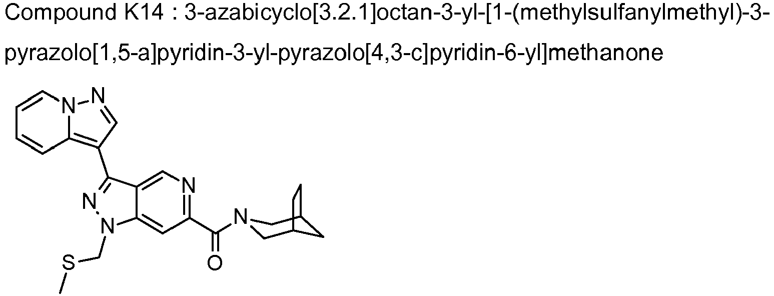

Compound K14 was obtained according to General Procedure VII, starting from 3-azabicyclo[3.2.1]octan-3-yl-[3-bromo-1-(methylsulfanylmethyl)pyrazolo[4,3-c]pyridin-6-yl]methanone F17 and pyrazolo[1,5-a]pyridine-3-yl-boronic acid pinacol ester l1. In that specific case, the reaction mixture was stirred 15min at 150°C under microwave irradiation. Purification by flash chromatography (DCM/MeOH: 10/0 to 9/1) afforded K14 in 68% yield. M/Z (M+H)⁺: 433

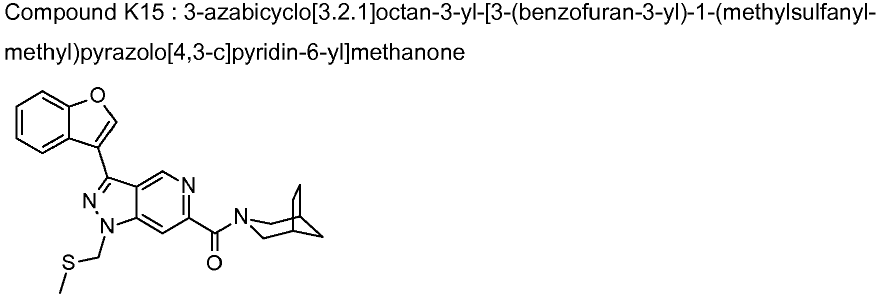

Compound K15 was obtained according to General Procedure VII, starting from 3-azabicyclo[3.2.1]octan-3-yl-[3-bromo-1-(methylsulfanylmethyl)pyrazolo[4,3-c]pyridin-6-yl]methanone F17 and benzofuran-3-yl-boronic acid pinacol ester l2. In that specific case, the reaction mixture was stirred 15min at 150°C under microwave irradiation. Purification by flash chromatography (Cyclohexane/AcOEt: 10/0 to 0/10) afforded K15 in 50% yield. M/Z (M+H)⁺: 433

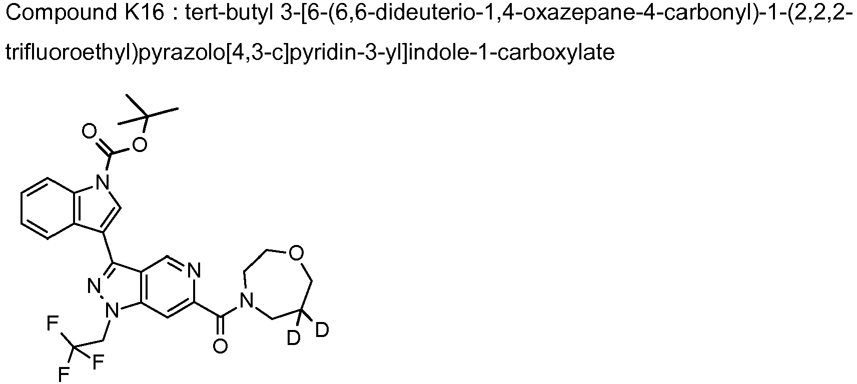

Compound K16 was obtained according to General Procedure VII, starting from [3-bromo-1-(2,2,2-trifluoroethyl)pyrazolo[4,3-c]pyridin-6-yl]-(6,6-dideuterio-1,4-oxazepan-4-yl)methanone F22 and tert-butyl 3-(4,4,5,5-tetramethyl-1,3,2-dioxaborolan-2-yl)indole-1-carboxylate l9. Purification by flash chromatography (DCM/MeOH: 10/0 to 9/1) afforded K16 as powder in 74% yield. M/Z (M+H)⁺: 546

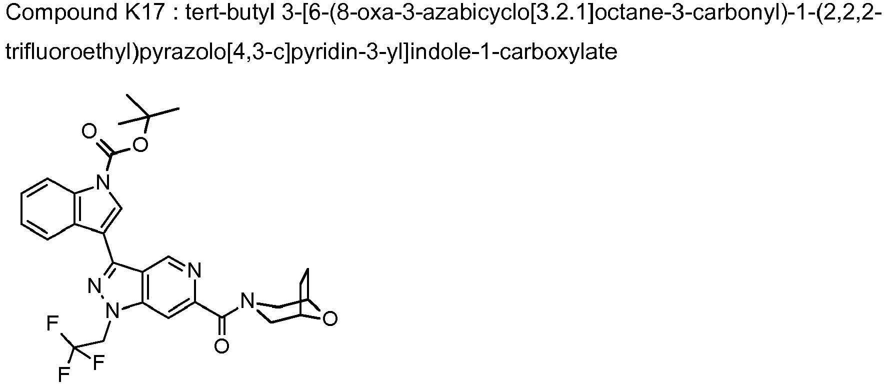

Compound K17 was obtained according to General Procedure VII, starting from [3-bromo-1-(2,2,2-trifluoroethyl)pyrazolo[4,3-c]pyridin-6-yl]-(8-oxa-3-azabicyclo[3.2.1]octan-3-yl)methanone F8 and tert-butyl 3-(4,4,5,5-tetramethyl-1,3,2-dioxaborolan-2-yl)indole-1-carboxylate l9. Purification by flash chromatography (DCM/MeOH: 10/0 to 9/1) afforded K17 as an orange powder in 78% yield. M/Z (M+H)⁺: 556

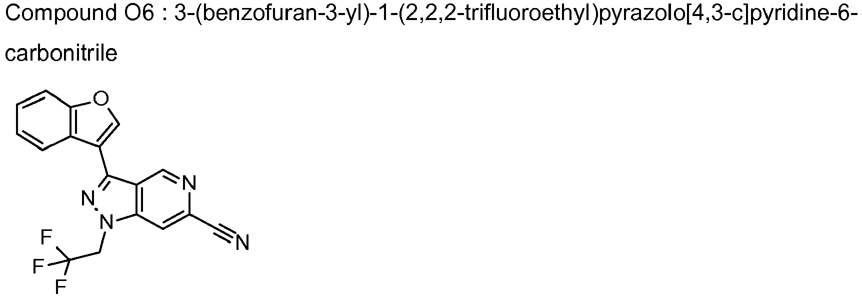

Compound O6 was obtained according to General Procedure VII, starting from 3-bromo-1-(2,2,2-trifluoroethyl)pyrazolo[4,3-c]pyridine-6-carbonitrile N1 and benzofuran-3-yl-boronic acid pinacol ester l2. Purification by flash chromatography (Cyclohexane/AcOEt: 10/0 to 5/5) afforded O6 in quantitative yield. M/Z (M+H)⁺: 343

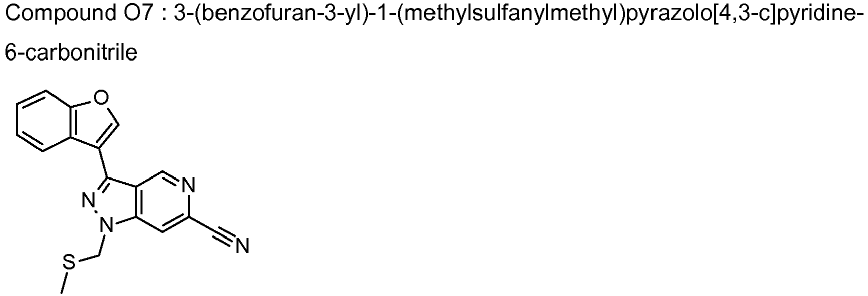

Compound O7 was obtained according to General Procedure VII, starting from 3-bromo-1-(methylsulfanylmethyl)pyrazolo[4,3-c]pyridine-6-carbonitrile N2 and benzofuran-3-yl-boronic acid pinacol ester l2. Purification by flash chromatography (Cyclohexane/AcOEt: 10/0 to 5/5) afforded O7 in quantitative yield without further purification. M/Z (M+H)⁺: 321

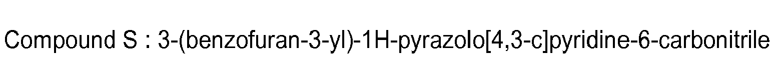

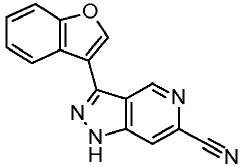

Compound S was obtained according to General Procedure VII, starting from 3-bromo-1H-pyrazolo[4,3-c]pyridine-6-carbonitrile M and benzofuran-3-yl-boronic acid pinacol ester l2. In that specific case, DMA was used instead of dioxane, the reaction mixture was heated 1.5h at 150°C. Purification by flash chromatography (DCM/MeOH: 100/0 to 99/1) afforded S as a brown powder in 63% yield. M/Z (M+H)⁺: 343.

### Compound 89 : Azepan-1-yl-[3-pyrazolo[1,5-a]pyridin-3-yl-1-(2,2,2-trifluoro-ethyl)-1H-pyrazolo[4,3-c]pyridin-6-yl]-methanone hydrochoride

Compound 89 was obtained according to General Procedure VII, starting from azepan-1-yl-[3-bromo-1-(2,2,2-trifluoroethyl)pyrazolo[4,3-c]pyridin-6-yl]methanone F3 and pyrazolo[1,5-a]pyridine-3-yl-boronic acid pinacol ester l1. Purification by flash chromatography (DCM/MeOH: 100/0 to 96/4) afforded 89 as a yellow powder in 83% yield. ¹H-NMR (DMSO-*d*₆,400MHz): 1.53-1.64 (m, 6H, CH₂); 1.75-1.81 (m, 2H, CH₂); 3.36-3.39 (m, 2H, N-CH₂); 3.62-3.66 (m, 2H, N-CH₂); 5.62 (q, *J* 9.0 Hz, 2H, CF₃-CH₂); 7.11 (td, *J* 6.8, 1.2 Hz, 1H, Ar), 7.53 (ddd, *J* 8.8, 6.8, 1.2 Hz, 1H, Ar); 8.11 (bs, 1H, Ar), 8.34 (dt, *J* 8.8, 1.2 Hz, 1H, Ar); 8.86 (dt, *J* 6.8, 1.2 Hz, 1H, Ar); 9.04 (s, 1H Ar), 9.63 (d, *J* 0.9 Hz, 1H, Ar). M/Z (M+H)⁺: 443

### Compound 90 : Azepan-1-yl-[3-benzofuran-3-yl-1-(2,2,2-trifluoro-ethyl)-1H-pyrazolo[4,3-c]pyridin-6-yl]-methanone

Compound 90 was obtained according to General Procedure VII, starting from azepan-1-yl-[3-bromo-1-(2,2,2-trifluoroethyl)pyrazolo[4,3-c]pyridin-6-yl]methanone F3 and benzofuran-3-yl-boronic acid pinacol ester l2. Purification by flash chromatography (DCM/MeOH: 100/0 to 96/4) afforded 90 as a beige powder in 80% yield. ¹H-NMR (DMSO-*d*₆,400MHz): 1.53-1.66 (m, 6H, CH₂); 1.74-1.83 (m, 2H, CH₂); 3.36-3.38 (m, 2H, N-CH₂); 3.63-3.66 (m, 2H, N-CH₂); 5.67 (q, *J* 9.0 Hz, 2H, CF₃-CH₂); 7.42-7.50 (m, 2H, Ar); 7.73-7.75 (m, 1H, Ar); 8.13 (d, *J* 0.9 Hz, 1H, Ar); 8.34-8.36 (m, 1H, Ar); 9.17 (s, 1H, Ar); 9.58 (d, *J* 0.9 Hz, 1H, Ar). M/Z (M+H)⁺: 443

### Compound 91 : Azepan-1-yl-[3-(1H-indol-3-yl)-1-(2,2,2-trifluoro-ethyl)-1H-pyrazolo[4,3-c]pyridin-6-yl]-methanone

Compound 91 was obtained according to General Procedure VII, starting from azepan-1-yl-[3-bromo-1-(2,2,2-trifluoroethyl)pyrazolo[4,3-c]pyridin-6-yl]methanone F3 and 1H-indol-3-yl-boronic acid pinacol ester l3. Purification by flash chromatography (DCM/MeOH: 100/0 to 95/5) afforded 91 as a beige powder in 68% yield. ¹H-NMR (DMSO-*d*₆, 400MHz): 1.52-1.65 (m, 6H, CH₂); 1.74-1.83 (m, 2H, CH₂); 3.35-3.42 (m, 2H, CH₂); 3.61-3.66 (m, 2H, CH₂); 5.59 (q, 2H, CF₃-CH₂); 7.15-7.25 (m, 2H, Ar); 7.49 (d, *J* 7.8 Hz, 1H, Ar); 8.04 (bs, 1H, Ar); 8.34 (d, *J* 7.8 Hz, 1H, Ar); 8.43 (d, *J* 2.7 Hz, 1H, Ar); 9.53 (d, *J* 0.9 Hz, 1H, Ar); 11.70 (bs, 1H, NH). M/Z (M+H)⁺: 442

### Compound 92 : Azepan-1-yl-[3-(1H-pyrrolo[2,3-b]pyridin-3-yl)-1-(2,2,2-trifluoro-ethyl)-1H-pyrazolo[4,3-c]pyridin-6-yl]-methanone

Compound 92 was obtained according to General Procedure VII, starting from azepan-1-yl-[3-bromo-1-(2,2,2-trifluoroethyl)pyrazolo[4,3-c]pyridin-6-yl]methanone F3 and 1H-pyrrolo[2,3-b]pyridin-3-yl-boronic acid pinacol ester l4. Purification by flash chromatography (DCM/MeOH: 100/0 to 95/5) afforded 92 as a beige powder in 12% yield. ¹H-NMR (DMSO-*d*₆,400MHz): 1.52-1.65 (m, 6H, CH₂); 1.74-1.83 (m, 2H, CH₂); 3.35-3.42 (m, 2H, N-CH₂); 3.61-3.66 (m, 2H, N-CH₂); 5.59 (q, 2H, CF₃-CH₂); 7.25 (dd, *J* 8.0, 4.7 Hz, 1H, Ar); 8.05 (bs, 1H, Ar); 8.35 (dd, *J* 4.7, 1.5 Hz, 1H, Ar); 8.57 (d, *J* 2.8 Hz, 1H, Ar); 8.63 (dd, *J* 8.0, 1.5 Hz, 1H, Ar); 9.58 (d, *J* 1.0 Hz, 1H, Ar); 12.24 (bs, 1H, NH). M/Z (M+H)⁺: 443

### Compound 93 : 1,4-Oxazepan-4-yl-[3-thieno[2,3-c]pyridin-3-yl-1-(2,2,2-trifluoro-ethyl)-1H-pyrazolo[4,3-c]pyridin-6-yl]-methanone

Compound 93 was obtained according to General Procedure VII, starting from [3-bromo-1-(2,2,2-trifluoroethyl)pyrazolo[4,3-c]pyridin-6-yl]-(1,4-oxazepan-4-yl)methanone F7 and thieno[2,3-c]pyridin-3-yl-boronic acid pinacol ester l5. In that specific case, DMA was used instead of dioxane and the reaction mixture was heated under microwave irradiation 15min at 150°C. Purification by flash chromatography (DCM/MeOH: 100/0 to 95/5) afforded 93 as a grey powder in 21% yield. ¹H-NMR (DMSO-*d*₆,400MHz): 1.78 (quint, *J* 5.8 Hz, 1H, CH₂); 1.95 (quint, *J* 5.8 Hz, 1H, CH₂); 3.47-3.54 (m, 2H, CH₂); 3.70-3.82 (m, 6H, CH₂); 5.73 (q, *J* 8.0 Hz, 2H, CF₃-CH₂); 8.21-8.24 (m, 1H, Ar); 8.58-8.61 (m, 1H, Ar); 8.64 (d, *J* 5.6 Hz, 1H, Ar); 9.10 (bs, 1H, signal of a rotamer, Ar); 9.11 (bs, 1H, signal of a rotamer, Ar); 9.41 (s, 1H, Ar), 9.56 (d, *J* 1.0 Hz, 1H, signal of a rotamer, Ar); 9.58 (d, *J* 1.0 Hz, 1H, signal of a rotamer, Ar). M/Z (M+H)⁺: 462

### Compound 94 : [3-Benzofuran-3-yl-1-(2,2,2-trifluoro-ethyl)-1H-pyrazolo[4,3-c]pyridine-6-yl]-1,4-oxazepan-4-yl-methanone

Compound 94 was obtained according to General Procedure VII, Alternative 1, starting from [3-bromo-1-(2,2,2-trifluoroethyl)pyrazolo[4,3-c]pyridin-6-yl]-(1,4-oxazepan-4-yl)methanone F7 and benzofuran-3-yl-boronic acid pinacol ester l2. Purification by flash chromatography (DCM/MeOH: 100/0 to 95/5) afforded 94 as a beige powder in 55% yield. ¹H-NMR (DMSO-*d*₆, 400MHz): 1.75-1.81 (d, *J* 5.6 Hz, 1H, CH₂); 1.92-1.98 (d, *J* 5.8 Hz, 1H, CH₂); 3.49-3.53 (m, 2H, N-CH₂); 3.65-3.67 (m, 1H, N-CH₂); 3.71-3.80 (m, 5H, N-CH₂, O-CH₂); 5.69 (q, *J* 9.0 Hz, 2H, CF₃-CH₂); 7.43-7.50 (m, 2H, Ar); 7.73-7.76 (m, 1H, Ar); 8.19 (bs, 1H, signal of a rotamer, Ar); 8.20 (bs, 1H, signal of a rotamer, Ar); 8.34-8.36 (m, 1H, Ar); 9.17 (s, 1H, signal of a rotamer, Ar); 9.18 (s, 1H, signal of a rotamer, Ar); 9.57 (d, *J* 1.0 Hz, 1H, signal of a rotamer, Ar); 9.59 (d, *J* 1.0 Hz, 1H, signal of a rotamer, Ar). M/Z (M+H)⁺: 445

### Compound 95 : 1,4-Oxazepan-4-yl-[3-pyrazolo[1,5-a]pyridine-3-yl-1-(2,2,2-trifluoroethyl)-1H-pyrazolo[4,3-c]pyridine-6-yl]-methanone

Compound 95 was obtained according to General Procedure VII, Alternative 1, starting from [3-bromo-1-(2,2,2-trifluoroethyl)pyrazolo[4,3-c]pyridin-6-yl]-(1,4-oxazepan-4-yl)methanone F7 and pyrazolo[1,5-a]pyridine-3-yl-boronic acid pinacol ester l1. Purification by flash chromatography (DCM/MeOH: 100/0 to 95/5) afforded 95 as a white powder in 27% yield. ¹H-NMR (DMSO-*d*₆,400MHz): 1.75-1.81 (d, J5.6 Hz, 1H, CH₂); 1.91-1.97 (d, J5.8 Hz, 1H, CH₂); 3.49-3.54 (m, 2H, N-CH₂); 3.64-3.67 (m, 1H, N-CH₂); 3.70-3.78 (m, 5H, N-CH₂, O-CH₂); 5.62 (q, *J* 9.0 Hz, 2H, CF₃-CH₂); 7.11 (td, *J* 6.8, 0.8 Hz, 1H, Ar); 7.52 (dd, *J* 8.7, 6.8 Hz, 1H, Ar); 8.12 (s, 1H, signal of a rotamer, Ar); 8.13 (s, 1H, signal of a rotamer, Ar); 8.34 (dd, *J* 8.7, 0.8 Hz, 1H, Ar); 8.86 (d, *J* 6.8 Hz, 1H, Ar); 9.02 (s, 1H, signal of a rotamer, Ar); 9.03 (s, 1H, signal of a rotamer, Ar); 9.60 (s, 1H, signal of a rotamer, Ar); 9.61 (s, 1H, signal of a rotamer, Ar). M/Z (M+H)⁺: 445

### Compound 96 : [3-(1H-lndol-3-yl)-1-(2,2,2-trifluoro-ethyl)-1H-pyrazolo[4,3-c]pyridine-6-yl]-1,4-oxazepan-4-yl-methanone

Compound 96 was obtained according to General Procedure VII, starting from [3-bromo-1-(2,2,2-trifluoroethyl)pyrazolo[4,3-c]pyridin-6-yl]-(1,4-oxazepan-4-yl)methanone F7 and 1H-indol-3-yl-boronic acid pinacol ester l3. Purification by flash chromatography (DCM/MeOH: 10/0 to 9/1) afforded 96 as a brown powder in 62% yield. ¹H-NMR (DMSO-*d*₆, 400MHz): 1.76-1.81 (m, 1H, CH₂); 1.92-1.97 (m, 1H, CH₂); 3.49-3.54 (m, 2H, N-CH₂); 3.65-3.67 (m, 1H, N-CH₂); 3.71-3.80 (m, 5H, N-CH₂, O-CH₂); 5.60 (q, *J* 9.0 Hz, 2H, CF₃-CH₂); 7.15-7.25 (m, 2H, Ar); 7.49 (d, *J* 7.9 Hz, 1H, Ar); 8.09 (bs, 1H, signal of a rotamer, Ar); 8.10 (bs, 1H, signal of a rotamer, Ar); 8.34 (d, *J* 7.9 Hz, 1H, Ar); 8.44 (d, *J* 3.0 Hz, 1H, signal of a rotamer, Ar); 8.45 (d, *J* 3.0 Hz, 1H, signal of a rotamer, Ar); 9.53 (d, *J* 0.8 Hz, 1H, signal of a rotamer, Ar); 9.54 (d, *J* 0.8 Hz, 1H, signal of a rotamer, Ar); 11.71 (bs, 1H, NH). M/Z (M+H)⁺: 444

### Compound 97 : 1,4-Oxazepan-4-yl-[3-(1H-pyrrolo[2,3-b]pyridin-3-yl)-1-(2,2,2-trifluoroethyl)-1H-pyrazolo[4,3-c]pyridine-6-yl]-methanone

Compound 97 was obtained according to General Procedure VII, starting from [3-bromo-1-(2,2,2-trifluoroethyl)pyrazolo[4,3-c]pyridin-6-yl]-(1,4-oxazepan-4-yl)methanone F7 and 1H-pyrrolo[2,3-b]pyridin-3-yl-boronic acid pinacol ester l4. Purification by flash chromatography (DCM/MeOH: 10/0 to 8/2) afforded 97 as a white powder in 7% yield. ¹H-NMR (DMSO-*d*₆, 400MHz): 1.75-1.81 (m, 1H, CH₂); 1.92-1.97 (m, 1H, CH₂); 3.49-3.54 (m, 2H, N-CH₂); 3.65-3.67 (m, 1H, N-CH₂); 3.71-3.79 (m, 5H, N-CH₂, O-CH₂); 5.61 (q, *J* 9.0 Hz, 2H, CF₃-CH₂); 7.25 (dd, *J* 7.8, 4.7 Hz, 1H, Ar); 8.10 (bs, 1H, signal of a rotamer, Ar); 8.11 (bs, 1H, signal of a rotamer, Ar); 8.35 (dd, *J* 4.7, 1.4 Hz, 1H, Ar); 8.57 (s, 1H, signal of a rotamer, Ar); 8.58 (s, 1H, signal of a rotamer, Ar); 8.63 (dt, *J* 7.8, 1.4 Hz, 1H, signal of a rotamer, Ar); 9.58 (d, *J* 0.9 Hz, 1H, signal of a rotamer, Ar); 9.60 (d, *J* 0.9 Hz, 1H, signal of a rotamer, Ar); 12.25 (s, 1H, NH). M/Z (M+H)⁺: 445

### Compound 98 : [3-Benzo[b]thiophen-3-yl-1-(2,2,2-trifluoro-ethyl)-1H-pyrazolo[4,3-c]-pyridine-6-yl]-1,4-oxazepan-4-yl-methanone

Compound 98 was obtained according to General Procedure VII, starting from [3-bromo-1-(2,2,2-trifluoroethyl)pyrazolo[4,3-c]pyridin-6-yl]-(1,4-oxazepan-4-yl)methanone F7 and benzo[b]thiophen-3-yl-boronic acid pinacol ester l6. Purification by flash chromatography (DCM/MeOH: 100/0 to 95/5) afforded 98 as a beige powder in 54% yield. ¹H-NMR (DMSO-*d*₆,400MHz): 1.75-1.81 (m, 1H, CH₂); 1.92-1.98 (m, 1H, CH₂); 3.49-3.53 (m, 2H, N-CH₂); 3.64-3.67 (m, 1H, N-CH₂); 3.71-3.81 (m, 5H, N-CH₂, O-CH₂); 5.72 (q, *J* 9.1 Hz, 2H, CF₃-CH₂); 7.48-7.57 (m, 2H, Ar); 8.12-8.16 (m, 1H, Ar); 8.19-8.22 (m, 1H, Ar); 8.70-8.73 (m, 1H, Ar); 8.76 (s, 1H, signal of a rotamer, Ar); 8.77 (s, 1H, signal of a rotamer, Ar); 9.51 (d, *J* 1.1 Hz, 1H, signal of a rotamer, Ar); 9.53 (d, *J* 1.1 Hz, 1H, signal of a rotamer, Ar). M/Z (M+H)⁺: 461

### Compound 99 : (8-Oxa-3-aza-bicyclo[3.2.1]oct-3-yl)-[3-pyrazolo[1,5-a]pyridin-3-yl-1-(2,2,2-trifluoro-ethyl)-1H-pyrazolo[4,3-c]pyridin-6-yl]-methanone

Compound 99 was obtained according to General Procedure VII, Alternative 1, starting from [3-bromo-1-(2,2,2-trifluoroethyl)pyrazolo[4,3-c]pyridin-6-yl]-(8-oxa-3-azabicyclo[3.2.1]octan-3-yl)methanone F8 and pyrazolo[1,5-a]pyridine-3-yl-boronic acid pinacol ester l1. Purification by flash chromatography (DCM/MeOH: 100/0 to 95/5) afforded 99 as a white powder in 39% yield. ¹H-NMR (DMSO-*d*₆, 400MHz): 1.71-1.96 (m, 4H, CH₂); 3.06 (dd, *J* 13.2, 1.9 Hz, 1H, N-CH₂); 3.30-3.33 (m, 1H, N-CH₂); 3.39-3.47 (m, 1H, N-CH₂); 4.19-4.27 (m, 2H, N-CH₂, O-CH); 4.39-4.46 (m, 1H, O-CH); 5.58-5.66 (m, 2H, CF₃-CH₂); 7.11 (td, *J* 6.8, 1.1 Hz, 1H, Ar); 7.52 (ddd, *J* 9.0, 6.8, 1.1 Hz, 1H, Ar); 8.15 (d, *J* 0.8 Hz, 1H, Ar); 8.34 (dt, J9.0, 1.1 Hz, 1H, Ar); 8.86 (dt, *J* 6.8, 1.1 Hz, 1H, Ar); 9.02 (s, 1H, Ar); 9.61 (d, *J* 0.8 Hz, 1H, Ar). M/Z (M+H)⁺: 457

### Compound 100 : [3-Benzofuran-3-yl-1-(2,2,2-trifluoro-ethyl)-1H-pyrazolo[4,3-c]pyridin-6-yl]-(8-oxa-3-aza-bicyclo[3.2.1]oct-3-yl)-methanone

Compound 100 was obtained according to General Procedure VII, Alternative 1, starting from [3-bromo-1-(2,2,2-trifluoroethyl)pyrazolo[4,3-c]pyridin-6-yl]-(8-oxa-3-azabicyclo[3.2.1]octan-3-yl)methanone F8 and benzofuran-3-yl-boronic acid pinacol ester l2. Purification by flash chromatography (DCM/MeOH: 100/0 to 95/5) afforded 100 as a white powder in 24% yield. ¹H-NMR (DMSO-*d*₆,400MHz): 1.74-1.93 (m, 4H, CH₂); 3.07 (dd, *J* 13.4, 1.7 Hz, 1H, N-CH₂); 3.32-3.35 (m, 1H, N-CH₂); 3.39-3.46 (m, 1H, N-CH₂); 4.18-4.27 (m, 2H, N-CH₂, O-CH); 4.40-4.46 (m, 1H, O-CH); 5.63-5.74 (m, 2H, CF₃-CH₂); 7.42-7.50 (m, 2H, Ar); 7.72-7.76 (m, 1H, Ar); 8.20 (bs, 1H, Ar); 8.32-8.37 (m, 1H, Ar); 9.16 (s, 1H, Ar); 9.59 (d, *J* 1.0Hz, 1H, Ar). M/Z (M+H)⁺: 457

### Compound 101 : (3-Aza-bicyclo[3.2.1]oct-3-yl)-[3-benzofuran-3-yl-1-(2,2,2-trifluoroethyl)-1H-pyrazolo[4,3-c]pyridin-6-yl]-methanone

Compound 101 was obtained according to General Procedure VII, Alternative 1, starting from [3-bromo-1-(2,2,2-trifluoroethyl)pyrazolo[4,3-c]pyridin-6-yl]-(3-azabicyclo[3.2.1]octan-3-yl)methanone F9 and benzofuran-3-yl-boronic acid pinacol ester I2. In that specific case, the reaction mixture was heated under microwave irradiation 15 min at 150°C. Purification by flash chromatography (Cyclohexane/AcOEt: 10/0 to 5/5) afforded 101 as a white powder in 26% yield. ¹H-NMR (DMSO-*d_{6,}* 400MHz): 1.47-1.72 (m, 6H, CH₂); 2.05-2.12 (m, 1H, CH₂); 2.28-2.36 (m, 1H, CH₂); 2.84-2.91 (m, 1H, N-CH₂); 3.12-3.19 (m, 1H, N-CH₂); 3.26-3.31 (m, 1H, N-CH₂); 4.32-4.41 (m, 1H, N-CH₂); 5.70 (q, *J* 9.1 Hz, 2H, CF₃-CH₂); 7.42-7.51 (m, 2H, Ar); 7.75 (d, *J* 7.8 Hz, 1H, Ar); 8.14 (s, 1H, Ar); 8.35 (d, *J* 6.9 Hz, 1H, Ar); 9.18 (s, 1H, Ar); 9.58 (s, 1H, Ar). M/Z (M+H)⁺: 455

### Compound 102 : (3-Aza-bicyclo[3.2.1]oct-3-yl)-[3-pyrazolo[1,5-a]pyridin-3-yl-1-(2,2,2-trifluoro-ethyl)-1H-pyrazolo[4,3-c]pyridin-6-yl]-methanone

Compound 102 was obtained according to General Procedure VII, Alternative 1, starting from [3-bromo-1-(2,2,2-trifluoroethyl)pyrazolo[4,3-c]pyridin-6-yl]-(3-azabicyclo[3.2.1]octan-3-yl)methanone F9 and pyrazolo[1,5-a]pyridine-3-yl-boronic acid pinacol ester I1. In that specific case, the reaction mixture was heated under microwave irradiation 15 min at 150°C. Purification by flash chromatography (Cyclohexane/AcOEt: 10/0 to 4/6) afforded 102 as a white powder in 34% yield. ¹H-NMR (DMSO-*d_{6,}* 400MHz): 1.45-1.77 (m, 6H, CH₂); 2.04-2.13 (m, 1H, CH₂); 2.26-2.34 (m, 1H, CH₂); 2.83-2.93 (m, 1H, N-CH₂); 3.11-3.20 (m, 1H, N-CH₂); 3.21-3.30 (m, 1H, N-CH₂); 4.31-4.42 (m, 1H, N-CH₂); 5.62 (q, *J* 9.0 Hz, 2H, CF₃-CH₂); 7.06-7.14 (m, 1H, Ar); 7.49-7.56 (m, 1H, Ar); 8.07 (s, 1H, Ar); 8.33 (d, *J* 8.8 Hz, 1H, Ar); 8.86 (d, *J* 6.7 Hz, 1H, Ar); 9.03 (s, 1H, Ar); 9.60 (s, 1H, Ar). M/Z (M+H)⁺: 455

### Compound 103 : [3-Benzofuran-3-yl-1-(2,2-difluoro-ethyl)-1H-pyrazolo[4,3-c]pyridin-6-yl]-1,4-oxazepan-4-yl-methanone

Compound 103 was obtained according to General Procedure VII, starting from [3-bromo-1-(2,2-difluoroethyl)pyrazolo[4,3-c]pyridin-6-yl]-(1,4-oxazepan-4-yl)methanone F10 and benzofuran-3-yl-boronic acid pinacol ester I2. Purification by flash chromatography (DCM/MeOH: 100/0 to 97/3) afforded 103 as a brown powder in 37% yield. ¹H-NMR (DMSO-*d_{6,}* 400MHz): 1.78 (quint, *J* 5.7 Hz, 1H, CH₂); 1.94 (quint, *J* 5.7 Hz, 1H, CH₂); 3.47-3.55 (m, 2H, CH₂); 3.64-3.69 (m, 1H, CH₂); 3.70-3.82 (m, 5H, CH₂); 5.15 (dt, *J* 15.3, 3.3 Hz, 2H, CH₂-CHF₂); 6.59 (tt, *J* 54.4, 3.3 Hz, 1H, CHF₂); 7.41-7.50 (m, 2H, Ar); 7.71-7.75 (m, 1H, Ar); 8.08 (s, 1H, signal of a rotamer, Ar); 8.09 (s, 1H, signal of a rotamer, Ar); 8.37-8.41 (m, 1H, Ar); 9.13 (s, 1H, signal of a rotamer, Ar); 9.14 (s, 1H, signal of a rotamer, Ar); 9.54 (d, *J* 1.0 Hz, 1H, signal of a rotamer, Ar); 9.55 (d, *J* 1.0 Hz, 1H, signal of a rotamer, Ar). M/Z (M+H)⁺: 427

### Compound 104 : [3-Benzofuran-3-yl-1-(2-fluoro-ethyl)-1H-pyrazolo[4,3-c]pyridin-6-yl]-1,4-oxazepan-4-yl-methanone

Compound 104 was obtained according to General Procedure VII, starting from [3-bromo-1-(2-fluoroethyl)pyrazolo[4,3-c]pyridin-6-yl]-(1,4-oxazepan-4-yl)methanone F11 and benzofuran-3-yl-boronic acid pinacol ester I2. Purification by flash chromatography (DCM/MeOH: 100/0 to 97/3) afforded 104 as a white powder in 49% yield. ¹H-NMR (DMSO-*d_{6,}* 400MHz): 1.79 (quint, *J* 5.7 Hz, 1H, CH₂); 1.95 (quint, *J* 5.7 Hz, 1H, CH₂); 3.48-3.56 (m, 2H, CH₂); 3.64-3.69 (m, 1H, CH₂); 3.70-3.82 (m, 5H, CH₂); 4.86-5.04 (m, 4H, CH₂-CH₂-F); 7.40-7.50 (m, 2H, Ar); 7.71-7.75 (m, 1H, Ar); 8.03 (bs, 1H, signal of a rotamer, Ar); 8.04 (bs, 1H, signal of a rotamer, Ar); 8.36-8.41 (m, 1H, Ar); 9.11 (s, 1H, signal of a rotamer, Ar); 9.12 (s, 1H, signal of a rotamer, Ar); 9.51 (d, *J* 1.0 Hz, 1H, signal of a rotamer, Ar), 9.53 (d, *J* 1.0 Hz, 1H, signal of a rotamer, Ar). M/Z (M+H)⁺: 409

### Compound 105 : 2-[3-Benzofuran-3-yl-6-(1,4-oxazepane-4-carbonyl)-pyrazolo[4,3-c]pyridin-1-yl]-acetamide

Compound 105 was obtained according to General Procedure VII, starting from 2-[3-bromo-6-(1,4-oxazepane-4-carbonyl)pyrazolo[4,3-c]pyridin-1-yl]acetonitrile F12 and benzofuran-3-yl-boronic acid pinacol ester I2. Purification by flash chromatography (DCM/MeOH: 100/0 to 93/7) afforded 105 as a beige powder in 8% yield. ¹H-NMR (DMSO-*d_{6,}* 400MHz): 1.79 (quint, *J* 5.7 Hz, 1H, CH₂); 1.95 (quint, *J* 5.7 Hz, 1H, CH₂); 3.48-3.56 (m, 2H, CH₂); 3.64-3.69 (m, 1H, CH₂); 3.70-3.82 (m, 5H, CH₂); 5.27 (s, 2H CH₂₋CONH₂); 7.37 (bs, 1H, NH₂); 7.40-7.50 (m, 2H, Ar); 7.70-7.78 (m, 2H, Ar, NH₂); 7.95 (d, *J* 0.9 Hz, 1H, signal of a rotamer, Ar); 7.96 (d, *J* 0.9 Hz, 1H, signal of a rotamer, Ar); 8.36-8.41 (m, 1H, Ar); 9.11 (s, 1H, signal of a rotamer, Ar); 9.12 (s, 1H, signal of a rotamer, Ar); 9.50 (d, *J* 1.0 Hz, 1H, signal of a rotamer, Ar); 9.52 (d, *J* 1.0 Hz, 1H, signal of a rotamer, Ar). M/Z (M+H)⁺: 420

### Compound 106 : (3-Benzofuran-3-yl-1-propyl-1H-pyrazolo[4,3-c]pyridin-6-yl)-1,4-oxazepan-4-yl-methanone

Compound 106 was obtained according to General Procedure VII, starting from (3-bromo-1-propyl-pyrazolo[4,3-c]pyridin-6-yl)-(1,4-oxazepan-4-yl)methanone F13 and benzofuran-3-yl-boronic acid pinacol ester I2. Purification by flash chromatography (DCM/MeOH: 100/0 to 95/5) afforded 106 as a beige powder in 36% yield. ¹H-NMR (DMSO-*d₆*,400MHz): 0.91 (t, *J* 7.0 Hz, 3H, CH₃); 1.79 (quint, *J* 5.7 Hz, 1H, CH₂); 1.90-2.02 (m, 3H, CH₂, CH₂-CH₂-CH₃); 3.48-3.56 (m, 2H, CH₂); 3.64-3.69 (m, 1H, CH₂); 3.70-3.82 (m, 5H, CH₂); 4.54 (t, *J* 6.9 Hz, 2H, CH₂-CH₂-CH₃); 7.40-7.50 (m, 2H, Ar); 7.70-7.78 (m, 1H, Ar); 8.02-8.05 (m, 1H, Ar); 8.35-8.40 (m, 1H, Ar); 9.08 (s, 1H, signal of a rotamer, Ar); 9.09 (s, 1H, signal of a rotamer, Ar); 9.49 (d, *J* 1.0 Hz, 1H, signal of a rotamer, Ar); 9.51 (d, *J* 1.0 Hz, 1H, signal of a rotamer, Ar). M/Z (M+H)⁺: 405

### Compound 107 : (3-Benzofuran-3-yl-1-methanesulfonylmethyl-1H-pyrazolo[4,3-c]pyridine-6-yl)-(8-oxa-3-aza-bicyclo[3.2.1]oct-3-yl)-methanone

Compound 107 was obtained according to General Procedure VII, starting from [3-bromo-1-(methylsulfonylmethyl)pyrazolo[4,3-c]pyridin-6-yl]-(8-oxa-3-azabicyclo[3.2.1]octan-3-yl)methanone F24 and benzofuran-3-yl-boronic acid pinacol ester I2. Purification by flash chromatography (Cyclohexane/AcOEt: 10/0 to 0/10) afforded 107 as a beige powder in 37% yield. ¹H-NMR (DMSO-*d_{6,}* 400MHz): 1.74-1.93 (m, 4H, CH₂); 3.05-3.09 (m, 1H, N-CH₂); 3.17 (s, 3H, SO₂-CH₃); 3.32-3.34 (m, 1H, N-CH₂); 3.40-3.43 (m, 1H, N-CH₂); 4.21-4.25 (m, 2H, N-CH₂, O-CH); 4.43- 4.44 (m, 1H, O-CH); 6.30 (s, 2H, SO₂-CH₂); 7.42-7.50 (m, 2H, Ar); 7.74-7.76 (m, 1H, Ar); 8.22 (d, *J* 0.7 Hz, 1H, Ar); 8.35-8.37 (m, 1H, Ar); 9.2 (s, 1H, Ar); 9.6 (d, *J* 0.7 Hz, 1H, Ar). M/Z (M+H)⁺: 467

### Compound 108 : (1-Methanesulfonylmethyl-3-pyrazolo[1,5-a]pyridine-3-yl-1H-pyrazolo[4,3-c]pyridine-6-yl)- (8-oxa-3-aza-bicyclo[3.2.1]oct-3-yl)-methanone

Compound 108 was obtained according to General Procedure VII, starting from [3-bromo-1-(methylsulfonylmethyl)pyrazolo[4,3-c]pyridin-6-yl]-(8-oxa-3-azabicyclo[3.2.1]octan-3-yl)methanone F24 and pyrazolo[1,5-a]pyridine-3-yl-boronic acid pinacol ester I1. Purification by flash chromatography (DCM/MeOH: 100/0 to 95/5) afforded 108 as a beige powder in 40% yield. ¹H-NMR (DMSO-*d_{6,}* 400MHz): 1.74-1.93 (m, 4H, CH₂); 3.05-3.08 (m, 1H, N-CH₂); 3.15 (s, 3H, SO₂-CH₃); 3.33-3.35 (m, 1H, N-CH₂); 3.41-3.44 (m, 1H, N-CH₂); 4.20-4.25 (m, 2H, N-CH₂, O-CH); 4.43-4.44 (m, 1H, O-CH); 6.23 (s, 2H, SO₂-CH₂); 7.12 (td, *J* 6.8, 1.0 Hz, 1H, Ar); 7.52 (ddd, *J* 8.9, 6.8, 0.8 Hz, 1H, Ar); 8.16 (d, *J* 0.8 Hz, 1H, Ar); 8.36 (dt, *J* 8.9, 1.0 Hz, 1H, Ar); 8.87 (ddd, *J* 6.8, 1.0, 0.8 Hz, 1H, Ar); 9.05 (s, 1H, Ar); 9.63 (d, *J* 0.8 Hz, 1H, Ar). M/Z (M+H)⁺: 467

### Compound 109 : [3-(5-fluorobenzofuran-3-yl)-1-(2,2,2-trifluoroethyl)pyrazolo[4,3-c]pyridin-6-yl]-(1,4-oxazepan-4-yl)methanone

Compound 109 was obtained according to General Procedure VII, Alternative 2, starting from [3-bromo-1-(2,2,2-trifluoroethyl)pyrazolo[4,3-c]pyridin-6-yl]-(1,4-oxazepan-4-yl)methanone F7 and 5-fluorobenzofuran. Purification by flash chromatography (DCM/MeOH: 100/0 to 98/2) afforded 109 as a white powder in 67% yield. ¹H-NMR (DMSO-*d_{6,}* 400MHz): 1.74-1.82 (m, 1H, CH₂); 1.91-1.98 (m, 1H, CH₂); 3.46-3.53 (m, 2H, CH₂); 3.63-3.68 (m, 1H, N-CH₂); 3.69-3.81 (m, 5H, N-CH₂, O-CH₂); 5.72 (d, *J* 9.0 Hz, 2H, CF₃-CH₂); 7.33 (td, *J* 9.1, 2.8 Hz, 1H, Ar); 7.79 (dd, *J* 9.1, 4.2 Hz, 1H, Ar); 8.04 (dd, *J* 8.7, 2.8 Hz, 1H, Ar); 8.18 (bs, 1H, Ar, signal of a rotamer); 8.19 (bs, 1H, Ar, signal of a rotamer); 9.25 (s, 1H, Ar, signal of a rotamer); 9.26 (s, 1H, Ar, signal of a rotamer); 9.58 (d, *J* 1.0 Hz, 1H, Ar, signal of a rotamer); 9.59 (d, *J* 1.0 Hz, 1H, Ar, signal of a rotamer). M/Z (M+H)⁺: 463

### Compound 110 : [3-(5-fluorobenzofuran-3-yl)-1-(methylsulfonylmethyl)pyrazolo[4,3-c]pyridin-6-yl]-(1,4-oxazepan-4-yl)methanone

Compound 110 was obtained according to General Procedure VII, Alternative 3, starting from 3-bromo-1-(methylsulfonylmethyl)pyrazolo[4,3-c]pyridin-6-yl]-(1,4-oxazepan-4-yl)methanone F25 and 5-fluorobenzofuran-3-yl-boronic acid pinacol ester I7. Purification by flash chromatography (DCM/MeOH: 10/0 to 9/1) afforded 110 as a pink powder in 78% yield. ¹H-NMR (DMSO-*d_{6,}* 400MHz): 1.78 (quint, *J* 5.6 Hz, 1H, CH₂); 1.94 (quint, *J* 5.6 Hz, 1H, CH₂); 3.16 (s, 3H, SO₂-CH₃); 3.46-3.53 (m, 2H, CH₂); 3.63-3.68 (m, 1H, N-CH₂); 3.69-3.81 (m, 5H, N-CH₂, O-CH₂); 6.30 (s, 2H, SO₂-CH₂); 7.33 (td, *J* 9.1, 2.3 Hz, 1H, Ar); 7.80 (dd, *J* 9.1, 4.0 Hz, 1H, Ar); 8.04 (dd, *J* 8.7, 2.3 Hz, 1H, Ar); 8.19 (d, *J* 0.8 Hz, 1H, Ar, signal of a rotamer); 8.20 (d, *J* 0.8 Hz, 1H, Ar, signal of a rotamer); 9.29 (s, 1H, Ar, signal of a rotamer); 9.30 (s, 1H, Ar, signal of a rotamer); 9.59 (d, *J* 1.0 Hz, 1H, Ar, signal of a rotamer); 9.61 (d, *J* 1.0 Hz, 1H, Ar, signal of a rotamer). M/Z (M+H)⁺: 473

### Compound 111 : [3-(5-fluorobenzofuran-3-yl)-1-(methylsulfonylmethyl)pyrazolo[4,3-c]-pyridin-6-yl]-(8-oxa-3-azabicyclo[3.2.1]octan-3-yl)methanone

Compound 111 was obtained according to General Procedure VII, Alternative 3, starting from [3-bromo-1-(methylsulfonylmethyl)pyrazolo[4,3-c]pyridin-6-yl]-(8-oxa-3-azabicyclo[3.2.1]octan-3-yl)methanone F24 and 5-fluorobenzofuran-3-yl-boronic acid pinacol ester I7. Purification by preparative HPLC afforded 111 as a grey powder in 33% yield. ¹H-NMR (DMSO-*d_{6,}* 400MHz): 1.73-1.94 (m, 4H, CH₂); 3.04-3.09 (m, 1H, N-CH₂); 3.15 (s, 3H, SO₂-CH₃); 3.33-3.42 (m, 2H, N-CH₂); 4.18-4.25 (m, 2H, N-CH₂, O-CH); 4.41- 4.46 (m, 1H, O-CH); 6.31 (s, 2H, SO₂-CH₂); 7.34 (td, *J* 9.1, 2.8 Hz, 1H, Ar); 7.80 (dd, *J* 9.1, 4.2 Hz, 1H, Ar); 8.04 (dd, *J* 8.7, 2.8 Hz, 1H, Ar); 8.21 (d, *J* 1.0 Hz, 1H, Ar); 9.29 (s, 1H, Ar); 9.61 (d, *J* 1.0 Hz, 1H, Ar). M/Z (M+H)⁺: 485

### Compound 112 : (3-endo-hydroxy-8-azabicyclo[3.2.1]octan-8-yl)-[3-(1H-indol-3-yl)-1-(2,2,2-trifluoroethyl)pyrazolo[4,3-c]pyridin-6-yl]methanone

Compound 112 was obtained according to General Procedure VII, starting from [3-bromo-1-(2,2,2-trifluoroethyl)pyrazolo[4,3-c]pyridin-6-yl]-(3-*endo*-hydroxy-8-azabicyclo[3.2.1]octan-8-yl)methanone F30 and 1H-indol-3-yl-boronic acid pinacol ester I3. Purification by flash chromatography (DCM/MeOH : 10/0 to 95/5) afforded 112 as a brown powder in 63% yield. ¹H-NMR (DMSO-*d₆*, 400MHz): 1.66-1.73 (m, 1H, CH₂); 1.80-1.97 (m, 3H, CH₂); 2.04-2.13 (m, 2H, CH₂); 2.18-2.34 (m, 2H, CH₂); 3.97-4.03 (m, 1H, CH); 4.50-4.56 (m, 1H, CH); 4.64 (d, *J* 2.3 Hz, 1H, OH); 4.65-4.71 (m, 1H, CH); 5.62 (q, *J* 9.1 Hz, 2H, CH₂-CF₃); 7.17 (td, *J* 7.7, 1.0 Hz, 1H, Ar); 7.23 (td, *J* 7.7, 1.0 Hz, 1H, Ar); 7.49 (d, J7.7 Hz, 1H, Ar); 8.19 (bs, 1H, Ar); 8.34 (d, J7.7 Hz, 1H, Ar); 8.45 (d, *J* 2.7 Hz, 1H, Ar); 9.54 (d, *J* 0.8 Hz, 1H, Ar); 11.71 (bs, 1H, NH). M/Z (M+H)+: 470

### Compound 113 : (3-endo-hydroxy-8-azabicyclo[3.2.1]octan-8-yl)-[3-pyrazolo[1,5-a]pyridin-3-yl-1-(2,2,2-trifluoroethyl)pyrazolo[4,3-c]pyridin-6-yl]methanone

Compound 113 was obtained according to General Procedure VII, starting from [3-bromo-1-(2,2,2-trifluoroethyl)pyrazolo[4,3-c]pyridin-6-yl]-(3-endo-hydroxy-8-azabicyclo[3.2.1]octan-8-yl)methanone F30 and pyrazolo[1,5-a]pyridine-3-yl-boronic acid pinacol ester I1. Purification by flash chromatography (DCM/MeOH : 10/0 to 95/5) then by preparative HPLC afforded 113 as a white powder in 39% yield. ¹H-NMR (DMSO-*d₆*, 400MHz): 1.66-1.73 (m, 1H, CH₂); 1.80-1.97 (m, 3H, CH₂); 2.03-2.11 (m, 2H, CH₂); 2.19-2.31 (m, 2H, CH₂); 3.97-4.03 (m, 1H, CH); 4.49-4.54 (m, 1H, CH); 4.64 (d, J2.3 Hz, 1H, OH); 4.65-4.71 (m, 1H, CH); 5.64 (q, J9.1 Hz, 2H, CH₂-CF₃); 7.11 (td, *J* 6.8, 1.3 Hz, 1H, Ar); 7.52 (ddd, *J* 9.0, 6.8, 1.0 Hz, 1H, Ar); 8.23 (bs, 1H, Ar); 8.34 (dt, *J* 9.0, 1.0 Hz, 1H, Ar); 8.86 (d, *J* 6.8 Hz, 1H, Ar); 9.03 (s, 1H, Ar); 9.60 (d, *J* 1.0 Hz, 1H, Ar). M/Z (M+H)+: 471

### Compound 114 : (4-hydroxy-1-piperidyl)-[3-pyrazolo[1,5-a]pyridin-3-yl-1-(2,2,2-trifluoroethyl)pyrazolo[4,3-c]pyridin-6-yl]methanone

Compound 114 was obtained according to General Procedure VII, starting from [3-bromo-1-(2,2,2-trifluoroethyl)pyrazolo[4,3-c]pyridin-6-yl]-(4-hydroxy-1-piperidyl)methanone F19 and pyrazolo[1,5-a]pyridine-3-yl-boronic acid pinacol ester I1. Purification by flash chromatography (DCM/MeOH : 10/0 to 9/1) afforded 1164 as a white powder in 14% yield. ¹H-NMR (DMSO-*d₆*, 400MHz): 1.33-1.49 (m, 2H, CH₂); 1.66-1.76 (m, 1H, CH₂); 1.81-1.89 (m, 1H, CH₂); 3.10-3.19 (m, 1H, N-CH₂); 3.23-3.30 (m, 1H, N-CH₂); 3.52-3.61 (m, 1H, N-CH₂); 3.72-3.80 (m, 1H, CH); 4.07-4.15 (m, 1H, N-CH₂); 4.80 (d, J4.2 Hz, 1H, OH); 5.62 (q, J9.1 Hz, 2H, CH₂-CF₃); 7.11 (td, J6.9, 1.4 Hz, 1H, Ar); 7.52 (ddd, *J* 8.8, 6.8, 1.0 Hz, 1H, Ar); 8.09 (bs, 1H, Ar); 8.34 (dt, *J* 8.8, 1.0 Hz, 1H, Ar); 8.86 (dd, *J* 6.8, 1.0 Hz, 1H, Ar); 9.03 (s, 1H, Ar); 9.60 (d, *J* 1.1 Hz, 1H, Ar). M/Z (M+H)+: 445

### Compound 115 : [3-(5-fluorobenzofuran-3-yl)-1-(2,2,2-trifluoroethyl)pyrazolo[4,3-c]pyridin-6-yl]-(4-hydroxy-1-piperidyl)methanone

Compound 115 was obtained according to General Procedure VII, starting from [3-bromo-1-(2,2,2-trifluoroethyl)pyrazolo[4,3-c]pyridin-6-yl]-(4-hydroxy-1-piperidyl)methanone F19 and 5-fluorobenzofuran-3-yl-boronic acid pinacol ester I7. In that specific case, Pd(PPh₃)₄ was used instead of PdCl₂(dppf).CH₂Cl₂. Purification by flash chromatography (DCM/MeOH : 100/0 to 95/5) afforded 115 as a white powder in 29% yield. ¹H-NMR (DMSO-*d₆*, 400MHz): 1.33-1.49 (m, 2H, CH₂); 1.67-1.75 (m, 1H, CH₂); 1.81-1.89 (m, 1H, CH₂); 3.10-3.19 (m, 1H, N-CH₂); 3.25-3.30 (m, 1H, N-CH₂); 3.51-3.58 (m, 1H, N-CH₂); 3.73-3.82 (m, 1H, CH); 4.07-4.14 (m, 1H, N-CH₂); 4.80 (d, J4.0 Hz, 1H, OH); 5.70 (q, J9.1 Hz, 2H, CH₂-CF₃); 7.33 (td, *J* 9.1, 2.8 Hz, 1H, Ar); 7.80 (dd, *J* 9.1, 4.2 Hz, 1H, Ar); 8.04 (dd, *J* 8.7, 2.8 Hz, 1H, Ar); 8.15 (bs, 1H, Ar); 9.26 (s, 1H, Ar); 9.58 (d, *J* 1.0 Hz, 1H, Ar). M/Z (M+H)+: 463

### Compound 116 : [3-(5-fluorobenzofuran-3-yl)-1-(2,2,2-trifluoroethyl)pyrazolo[4,3-c]pyridin-6-yl]-(4-hydroxy-4-methyl-1-piperidyl)methanone

Compound 116 was obtained according to General Procedure VII, starting from [3-bromo-1-(2,2,2-trifluoroethyl)pyrazolo[4,3-c]pyridin-6-yl]-(4-hydroxy-4-methyl-1-piperidyl)methanone F20 and 5-fluorobenzofuran-3-yl-boronic acid pinacol ester I7. In that specific case, Pd(PPh₃)₄ was used instead of PdCl₂(dppf).CH₂Cl₂. Purification by flash chromatography (DCM/MeOH : 10/0 to 9/1) afforded 116 as a white powder in 47% yield. ¹H-NMR (DMSO-*d₆*, 400MHz): 1.18 (s, 3H, CH₃); 1.39-1.65 (m, 4H, CH₂); 3.25-3.41 (m, 3H, N-CH₂); 4.12-4.19 (m, 1H, N-CH₂); 4.46 (s, 1H, OH); 5.70 (q, J9.1 Hz, 2H, CH₂-CF₃); 7.34 (td, *J* 9.1, 2.8 Hz, 1H, Ar); 7.80 (dd, *J* 9.1, 4.2 Hz, 1H, Ar); 8.04 (dd, *J* 8.7, 2.8 Hz, 1H, Ar); 8.14 (bs, 1H, Ar); 9.26 (s, 1H, Ar); 9.58 (d, *J* 1.0 Hz, 1H, Ar). M/Z (M+H)+: 477

### Compound 117 : (4-hydroxy-4-methyl-1-piperidyl)-[3-pyrazolo[1,5-a]pyridin-3-yl-1-(2,2,2-trifluoroethyl)pyrazolo[4,3-c]pyridin-6-yl]methanone

Compound 117 was obtained according to General Procedure VII, starting from [3-bromo-1-(2,2,2-trifluoroethyl)pyrazolo[4,3-c]pyridin-6-yl]-(4-hydroxy-4-methyl-1-piperidyl)methanone F20 and pyrazolo[1,5-a]pyridine-3-yl-boronic acid pinacol ester I1. Purification by flash chromatography (DCM/MeOH : 10/0 to 9/1) afforded 117 as a white powder in 57% yield. ¹H-NMR (DMSO-*d₆*, 400MHz): 1.18 (s, 3H, CH₃); 1.39-1.65 (m, 4H, CH₂); 3.25-3.41 (m, 3H, N-CH₂); 4.11-4.18 (m, 1H, N-CH₂); 4.46 (s, 1H, OH); 5.61 (q, *J* 9.0 Hz, 2H, CH₂-CF₃); 7.11 (td, *J* 6.8, 1.3 Hz, 1H, Ar); 7.52 (ddd, *J* 8.8, 6.8, 1.0 Hz, 1H, Ar); 8.08 (bs, 1H, Ar); 8.34 (dt, *J* 8.8, 1.0 Hz, 1H, Ar); 8.86 (dd, *J* 6.8, 1.0 Hz, 1H, Ar); 9.03 (s, 1H, Ar); 9.60 (d, *J* 1.0 Hz, 1H, Ar). M/Z (M+H)+: 459

### Compound 118 : [3-(5-fluorobenzofuran-3-yl)-1-(2,2,2-trifluoroethyl)pyrazolo[4,3-c]pyridin-6-yl]-(6-hydroxy-1,4-oxazepan-4-yl)methanone

Compound 118 was obtained according to General Procedure VII, starting from [3-bromo-1-(2,2,2-trifluoroethyl)pyrazolo[4,3-c]pyridin-6-yl]-(6-hydroxy-1,4-oxazepan-4-yl)methanone F21 and 5-fluorobenzofuran-3-yl-boronic acid pinacol ester I7. In that specific case, Pd(PPh₃)₄ was used instead of PdCl₂(dppf).CH₂Cl₂. Purification by flash chromatography (DCM/MeOH : 10/0 to 9/1) afforded 118 as a white powder in 35% yield. ¹H-NMR (DMSO-*d₆*, 400MHz): 3.22-3.47 (m, 1H, CH₂); 3.49-3.82 (m, 6H, CH₂); 3.83-3.90 (m, 1H, CH₂, signal of a rotamer); 3.96-4.08 (m, 1H, CH₂); 4.22-4.28 (m, 1H, CH₂, signal of a rotamer); 5.00 (d, *J* 4.4 Hz, 1H, OH, signal of a rotamer); 5.14 (d, *J* 4.9 Hz, 1H, OH, signal of a rotamer); 5.66-5.76 (m, 2H, CH₂-CF₃); 7.30-7.37 (m, 1H, Ar); 7.77-7.82 (m, 1H, Ar); 8.02-8.07 (m, 1H, Ar); 8.19-8.22 (m, 1H, Ar); 9.26 (s, 1H, Ar, signal of a rotamer); 9.28 (s, 1H, Ar, signal of a rotamer); 9.58 (d, *J* 1.0 Hz, 1H, Ar, signal of a rotamer); 9.60 (d, *J* 1.0 Hz, 1H, Ar, signal of a rotamer). M/Z (M+H)+: 479

### Compound 119 : (6-hydroxy-1,4-oxazepan-4-yl)-[3-pyrazolo[1,5-a]pyridin-3-yl-1-(2,2,2-trifluoroethyl)pyrazolo[4,3-c]pyridin-6-yl]methanone

Compound 119 was obtained according to General Procedure VII, starting from [3-bromo-1-(2,2,2-trifluoroethyl)pyrazolo[4,3-c]pyridin-6-yl]-(6-hydroxy-1,4-oxazepan-4-yl)methanone F21 and pyrazolo[1,5-a]pyridine-3-yl-boronic acid pinacol ester I1. Purification by flash chromatography (DCM/MeOH : 10/0 to 9/1) afforded 119 as a white powder in 83% yield. ¹H-NMR (DMSO-*d₆*, 400MHz): 3.22-3.47 (m, 1H, CH₂); 3.49-3.82 (m, 6H, CH₂); 3.83-3.90 (m, 1H, CH₂, signal of a rotamer); 3.96-4.08 (m, 1H, CH₂); 4.22-4.28 (m, 1H, CH₂, signal of a rotamer); 5.02 (d, *J* 4.4 Hz, 1H, OH, signal of a rotamer); 5.14 (d, *J* 4.9 Hz, 1H, OH, signal of a rotamer); 5.58-5.66 (m, 2H, CH₂-CF₃); 7.08-7.13 (m, 1H, Ar); 7.49-7.55 (m, 1H, Ar); 8.15 (bs, 1H, Ar, signal of a rotamer); 8.16 (bs, 1H, Ar, signal of a rotamer); 8.32-8.36 (m, 1H, Ar); 8.84-8.88 (m, 1H, Ar); 9.02 (s, 1H, Ar, signal of a rotamer); 9.04 (s, 1H, Ar, signal of a rotamer); 9.60 (d, *J* 1.0 Hz, 1H, Ar, signal of a rotamer); 9.62 (d, *J* 1.0 Hz, 1H, Ar, signal of a rotamer). M/Z (M+H)+: 461

### Compound 120 : [3-(5-fluorobenzofuran-3-yl)-1-(2,2,2-trifluoroethyl)pyrazolo[4,3-c]pyridin-6-yl]-(6,6-dideuterio-1,4-oxazepan-4-yl)methanone

Compound 120 was obtained according to General Procedure VII, starting from [3-bromo-1-(2,2,2-trifluoroethyl)pyrazolo[4,3-c]pyridin-6-yl]-(6,6-dideuterio-1,4-oxazepan-4-yl)methanone F22 and 5-fluorobenzofuran-3-yl-boronic acid pinacol ester I7. In that specific case, Pd(PPh₃)₄ was used instead of PdCl₂(dppf).CH₂Cl₂. Purification by flash chromatography (DCM/MeOH : 10/0 to 9/1) afforded 120 as a white powder in 66% yield. ¹H-NMR (DMSO-*d₆*, 400MHz): 3.47-3.52 (m, 2H, CH₂); 3.63-3.67 (m, 1H, CH₂); 3.70-3.72 (m, 1H, CH₂); 3.73-3.81 (m, 4H, CH₂); 5.70 (q, *J* 9.0 Hz, 2H, CH₂-CF₃); 7.33 (td, *J* 9.1, 2.8 Hz, 1H, Ar); 7.80 (dd, *J* 9.1, 4.2 Hz, 1H, Ar); 8.04 (dd, *J* 8.7, 2.8 Hz, 1H, Ar); 8.17-8.20 (m, 1H, Ar); 9.25 (s, 1H, Ar, signal of a rotamer); 9.26 (s, 1H, Ar, signal of a rotamer); 9.58 (d, *J* 1.0 Hz, 1H, Ar, signal of a rotamer); 9.59 (d, *J* 1.0 Hz, 1H, Ar, signal of a rotamer). M/Z (M+H)+: 465

### Compound 121 : (6,6-dideuterio-1,4-oxazepan-4-yl)-[3-pyrazolo[1,5-a]pyridin-3-yl-1-(2,2,2-trifluoroethyl)pyrazolo[4,3-c]pyridin-6-yl]methanone

Compound 121 was obtained according to General Procedure VII, starting from [3-bromo-1-(2,2,2-trifluoroethyl)pyrazolo[4,3-c]pyridin-6-yl]-(6,6-dideuterio-1,4-oxazepan-4-yl)methanone F22 and pyrazolo[1,5-a]pyridine-3-yl-boronic acid pinacol ester 11. Purification by flash chromatography (DCM/MeOH : 10/0 to 9/1) then by preparative HPLC afforded 121 as a white powder in 34% yield. ¹H-NMR (DMSO-*d₆*, 400MHz): 3.47-3.53 (m, 2H, CH₂); 3.63-3.67 (m, 1H, CH₂); 3.69-3.73 (m, 1H, CH₂); 3.73-3.81 (m, 4H, CH₂); 5.70 (q, *J* 9.1 Hz, 2H, CH₂-CF₃); 7.11 (td, *J* 6.8, 1.3 Hz, 1H, Ar); 7.52 (ddd, *J* 8.8, 6.8, 0.6 Hz, 1H, Ar); 8.12 (bs, 1H, Ar, signal of a rotamer); 8.13 (bs, 1H, Ar, signal of a rotamer); 8.32-8.36 (m, 1H, Ar); 8.86 (d, *J* 6.8 Hz, 1H, Ar); 9.02 (s, 1H, Ar, signal of a rotamer); 9.03 (s, 1H, Ar, signal of a rotamer); 9.59 (d, *J* 1.0 Hz, 1H, Ar, signal of a rotamer); 9.61 (d, *J* 1.0 Hz, 1H, Ar, signal of a rotamer). M/Z (M+H)+: 447

### Compound 122 : [3-(5-fluorobenzofuran-3-yl)-1-(2,2,2-trifluoroethyl)pyrazolo[4,3-c]pyridin-6-yl]-(4-hydroxyazepan-1-yl)methanone

Compound 122 was obtained according to General Procedure VII, Alternative 2, starting from [3-bromo-1-(2,2,2-trifluoroethyl)pyrazolo[4,3-c]pyridin-6-yl]-(4-hydroxyazepan-1-yl)methanone F23 and 3-bromo-5-fluorobenzofuran. Purification by flash chromatography (DCM/MeOH : 100/0 to 95/5) then on an Interchim cartridge (15µm) (DCM/MeOH : 100/0 to 95/5) afforded 122 as a white powder in 20% yield. ¹H-NMR (DMSO-*d₆*, 400MHz): 1.48-1.83 (m, 5H, CH₂); 1.89-2.01 (m, 1H, CH₂); 3.22-3.47 (m, 2H, N-CH₂); 3.49-3.81 (m, 3H, CH, N-CH₂); 4.55 (d, *J* 3.7 Hz, 1H, OH, signal of a rotamer); 4.61 (d, *J* 3.7 Hz, 1H, OH, signal of a rotamer); 5.70 (q, *J* 9.1 Hz, 2H, CH₂-CF₃); 7.33 (td, *J* 9.1, 2.8 Hz, 1H, Ar); 7.80 (dd, *J* 9.1, 4.2 Hz, 1H, Ar); 8.05 (dd, *J* 8.8, 2.7 Hz, 1H, Ar); 8.12 (bs, 1H, Ar, signal of a rotamer); 8.14 (bs, 1H, Ar, signal of a rotamer); 9.25 (s, 1H, Ar, signal of a rotamer); 9.26 (s, 1H, Ar, signal of a rotamer); 9.58 (d, *J* 1.0 Hz, 1H, Ar). M/Z (M+H)+: 477

### Compound 123 : [3-(5-fluoro-1H-indol-3-yl)-1-(2,2,2-trifluoroethyl)pyrazolo[4,3-c]pyridin-6-yl]-(1,4-oxazepan-4-yl)methanone

Compound 123 was obtained according to General Procedure VII, starting from [3-bromo-1-(2,2,2-trifluoroethyl)pyrazolo[4,3-c]pyridin-6-yl]-(1,4-oxazepan-4-yl)methanone F7 and tert-butyl 5-fluoro-3-(4,4,5,5-tetramethyl-1,3,2-dioxaborolan-2-yl)indole-1-carboxylate I8. Purification by flash chromatography (DCM/MeOH: 100/0 to 92/8) afforded 123 as a white powder in 42% yield. ¹H-NMR (DMSO-*d₆*, 400MHz): 1.78 (quint, *J* 5.6 Hz, 1H, CH₂); 1.94 (quint, *J* 5.6 Hz, 1H, CH₂); 3.48-3.54 (m, 2H, N-CH₂); 3.64-3.68 (m, 1H, N-CH₂); 3.70-3.80 (m, 5H, N-CH₂, O-CH₂); 5.62 (q, J9.1 Hz, 2H, CF₃-CH₂); 7.08 (td, *J* 9.0, 2.4 Hz, 1H, Ar); 7.50 (dd, *J* 9.0, 4.5 Hz, 1H, Ar); 8.04 (dd, *J* 10.0, 2.4 Hz, 1H, Ar); 8.09 (bs, 1H, signal of a rotamer, Ar); 8.10 (bs, 1H, signal of a rotamer, Ar); 8.55 (d, *J* 2.9 Hz, 1H, Ar), 9.54 (d, *J* 0.9 Hz, 1H, signal of a rotamer, Ar); 9.56 (d, *J* 0.9 Hz, 1H, signal of a rotamer, Ar); 11.84 (bs, 1H, NH). M/Z (M+H)⁺: 463

### Compound 124 : methyl 3-[6-(1,4-oxazepane-4-carbonyl)-1-(2,2,2-trifluoroethyl)pyrazolo[4,3-c]pyridin-3-yl]benzofuran-5-carboxylate

Compound 124 was obtained according to General Procedure VII, Alternative 2, starting from [3-bromo-1-(2,2,2-trifluoroethyl)pyrazolo[4,3-c]pyridin-6-yl]-(1,4-oxazepan-4-yl)methanone F7 and 3-bromobenzofuran-5-carboxylic acid methyl ester. Purification by flash chromatography (Cyclohexane/AcOEt : 10/0 to 0/10) afforded 124 as a white powder in 53% yield. ¹H-NMR (DMSO-*d_{6,}* 400MHz): 1.77 (quint, *J* 5.8 Hz, 1H, CH₂); 1.95 (quint, *J* 5.8 Hz, 1H, CH₂); 3.46-3.53 (m, 2H, CH₂); 3.63-3.68 (m, 1H, N-CH₂); 3.69-3.81 (m, 5H, N-CH₂, O-CH₂); 3.92 (s, 3H, CH₃); 5.72 (q, *J* 9.0 Hz, 2H, CF₃-CH₂); 7.88 (d, *J* 8.7 Hz, 1H, Ar); 8.09 (dd, *J* 8.7, 1.8 Hz, 1H, Ar); 8.22 (bs, 1H, Ar, signal of a rotamer); 8.23 (bs, 1H, Ar, signal of a rotamer); 8.98-8.99 (m, 1H, Ar); 9.30 (d, *J* 1.8 Hz, 1H, Ar); 9.58 (d, *J* 1.0 Hz, 1H, Ar, signal of a rotamer); 9.60 (d, *J* 1.0 Hz, 1H, Ar, signal of a rotamer). M/Z (M+H)⁺: 303

### Compound 125 : [3-(5-methylbenzofuran-3-yl)-1-(2,2,2-trifluoroethyl)pyrazolo[4,3-c]pyridin-6-yl]-(1,4-oxazepan-4-yl)methanone

Compound 125 was obtained according to General Procedure VII, Alternative 2, starting from [3-bromo-1-(2,2,2-trifluoroethyl)pyrazolo[4,3-c]pyridin-6-yl]-(1,4-oxazepan-4-yl)methanone F7 and 3-bromo-5-methyl-benzofuran. Purification by flash chromatography (DCM/MeOH : 100/0 to 97/3 then on a 15µm cartridge with DCM/MeOH : 100/0 to 98/2) afforded 125 as a white powder in 37% yield. ¹H-NMR (DMSO-*d_{6,}* 400MHz): 1.78 (quint, *J* 5.8 Hz, 1H, CH₂); 1.94 (quint, *J* 5.8 Hz, 1H, CH₂); 2.48 (s, 3H, CH₃); 3.47-3.54 (m, 2H, CH₂); 3.63-3.68 (m, 1H, N-CH₂); 3.69-3.81 (m, 5H, N-CH_{2,} O-CH₂); 5.69 (q, *J* 9.1 Hz, 2H, CF₃-CH₂); 7.28 (dd, *J* 8.5, 1.5 Hz, 1H, Ar); 7.61 (d, *J* 8.5 Hz, 1H, Ar); 8.12 (d, *J* 1.5 Hz, 1H, Ar); 8.18 (bs, 1H, Ar, signal of a rotamer); 8.19 (bs, 1H, Ar, signal of a rotamer); 9.10 (bs, 1H, Ar, signal of a rotamer); 9.11 (bs, 1H, Ar, signal of a rotamer); 9.55 (d, *J* 1.0 Hz, 1H, Ar, signal of a rotamer); 9.57 (d, *J* 1.0 Hz, 1H, Ar, signal of a rotamer). M/Z (M+H)⁺: 459

### Compound 126 : [3-(2-methylbenzofuran-3-yl)-1-(2,2,2-trifluoroethyl)pyrazolo[4,3-c]pyridin-6-yl]-(1,4-oxazepan-4-yl)methanone

Compound 126 was obtained according to General Procedure VII, Alternative 2, starting from [3-bromo-1-(2,2,2-trifluoroethyl)pyrazolo[4,3-c]pyridin-6-yl]-(1,4-oxazepan-4-yl)methanone F7 and 3-bromo-2-methyl-benzofuran. Purification by flash chromatography (DCM/MeOH : 100/0 to 98/2) then by preparative HPLC afforded 126 as a white powder in 17% yield. ¹H-NMR (OMSO-*d_{6,}* 400MHz): 1.78 (quint, J5.8 Hz, 1H, CH₂); 1.94 (quint, *J* 5.8 Hz, 1H, CH₂); 2.69 (s, 3H, CH₃, signal of a rotamer); 2.70 (s, 3H, CH₃, signal of a rotamer); 3.47-3.54 (m, 2H, CH₂); 3.62-3.66 (m, 1H, N-CH₂); 3.69-3.81 (m, 5H, N-CH₂, O-CH₂); 5.70 (q, *J* 9.0 Hz, 2H, CF₃-CH₂); 7.31-7.40 (m, 2H, Ar); 7.63-7.67 (m, 1H, Ar); 7.70-7.73 (m, 1H, Ar); 8.19-8.21 (m, 1H, Ar); 9.12 (d, *J* 1.0 Hz, 1H, Ar, signal of a rotamer); 9.14 (d, *J* 1.0 Hz, 1H, Ar, signal of a rotamer). M/Z (M+H)⁺: 459

### Compound 127 : [3-(5-fluorobenzofuran-3-yl)-1-(2,2,2-trifluoroethyl)pyrazolo[4,3-c]pyridin-6-yl]-(8-oxa-3-azabicyclo[3.2.1]octan-3-yl)methanone

Compound 127 was obtained according to General Procedure VII, Alternative 2, starting from 3-bromo-1-(2,2,2-trifluoroethyl)pyrazolo[4,3-c]pyridin-6-yl]-(1,4-oxazepan-4-yl)methanone F7 and 3-bromo-5-fluoro-benzofuran. Purification by flash chromatography (DCM/MeOH : 100/0 to 97/3 then Cyclohexane/AcOEt : 10/0 to 0/10) afforded 127 as a white powder in 67% yield. ¹H-NMR (DMSO-*d_{6,}* 400MHz): 1.74-1.94 (m, 4H, CH₂); 3.04-3.09 (m, 1H, N-CH₂); 3.33-3.42 (m, 2H, N-CH₂); 4.18-4.26 (m, 2H, N-CH₂, O-CH); 4.41-4.46 (m, 1H, O-CH); 5.66-5.77 (m, 2H, CF₃-CH₂); 7.33 (td, J9.1, 2.8 Hz, 1H, Ar); 7.80 (dd, *J* 9.1, 4.2 Hz, 1H, Ar); 8.04 (dd, *J* 8.7, 2.7 Hz, 1H, Ar); 8.21 (bs, 1H, Ar); 9.26 (s, 1H, Ar); 9.60 (d, *J* 1.0 Hz, 1H, Ar). M/Z (M+H)⁺: 475

### General Procedure VIII : Synthesis of compounds J from compounds K (scheme 1)

To a solution of compound K (1 equiv.) in THF (0.1M) was added TBAF, 1M in THF (2 equiv.). The reaction mixture was heated 4h at 70°C. The reaction mixture was quenched with water. The resulting precipitate was filtered, then purified by flash chromatography to afford compound J.

### Alternative 1 :

To a solution of compound K (1 equiv.) in methanol was added HCl, 4N in dioxane (40 equiv.). The reaction mixture was heated at 5min at 130°C under microwave irradiation. The reaction mixture was diluted with AcOEt, washed with a saturated sodium bicarbonate solution and brine, dried over magnesium sulfate then concentrated. The resulting oil was purified by flash chromatography to afford compound J.

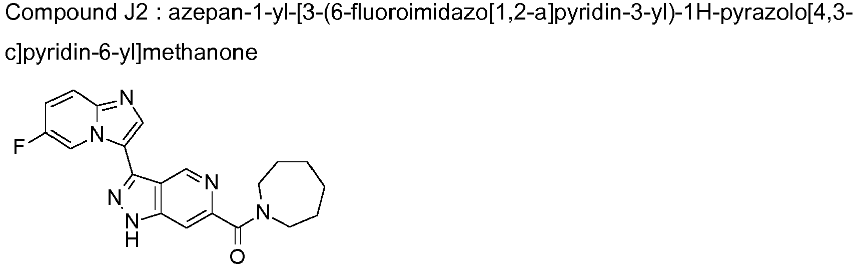

Compound J2 was obtained according to General Procedure VIII, starting from azepan-1-yl-[3-(6-fluoroimidazo[1,2-a]pyrid in-3-yl)-1-(2-trimethylsilylethoxymethyl)pyrazolo[4,3-c]pyridin-6-yl]methanone K3. Purification by flash chromatography (DCM/MeOH: 100/0 to 95/5) afforded J2 as a white powder in 70% yield. M/Z (M+H)⁺: 379

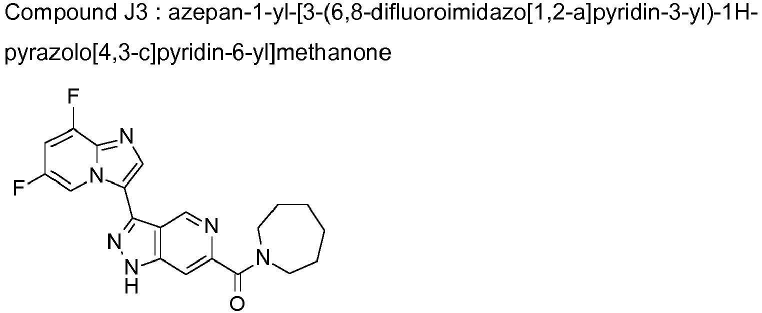

Compound J3 was obtained according to General Procedure VIII, starting from azepan-1-yl-[3-(6,8-difluoroimidazo[1,2-a]pyridin-3-yl)-1-(2-trimethylsilylethoxymethyl)pyrazolo[4,3-c]pyridin-6-yl]methanone K4. Purification by flash chromatography (Cyclohexane/AcOEt: 10/0 to 0/10) afforded J3 as a beige powder in 49% yield. M/Z (M+H)⁺: 397

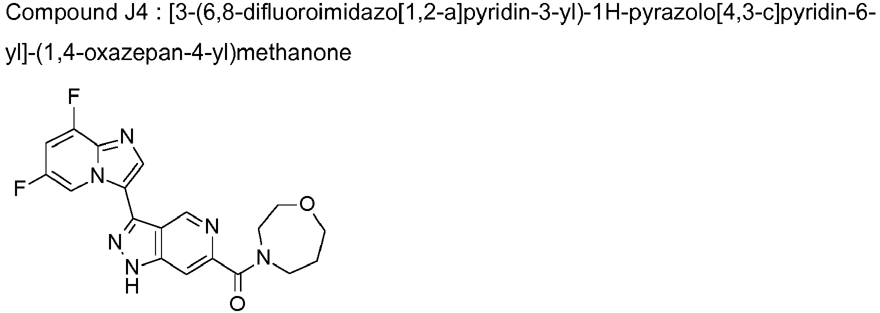

Compound J4 was obtained according to General Procedure VIII, starting from [3-(6,8-difluoroimidazo[1,2-a]pyridin-3-yl)-1-(2-trimethylsilylethoxymethyl)pyrazolo[4,3-c]pyridin-6-yl]-(1,4-oxazepan-4-yl)methanone K5. Purification by flash chromatography (DCM/MeOH: 100/0 to 95/5) afforded J4 as a white powder in 30% yield. M/Z (M+H)⁺: 399

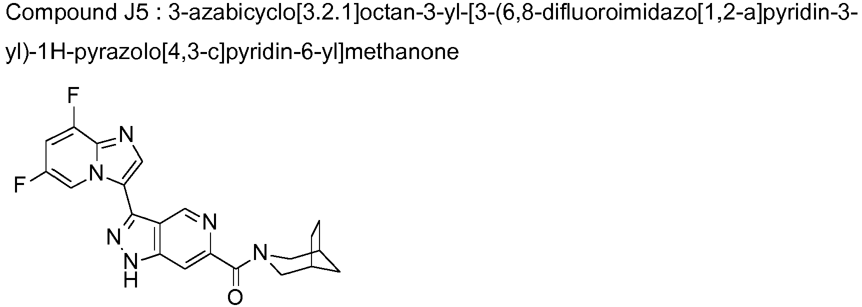

Compound J5 was obtained according to the General Procedure VIII, Alternative 1, starting from 3-azabicyclo[3.2.1]octan-3-yl-[3-(6,8-difluoroimidazo[1,2-a]pyridin-3-yl)-1-tetrahydropyran-2-yl-pyrazolo[4,3-c]pyridin-6-yl]methanone K6. Purification by flash chromatography (DCM/MeOH: 100/0 to 95/5) afforded J5 as a beige powder in 72% yield. M/Z (M+H)⁺: 409

### General Procedure IX : Synthesis of compounds H and L from compound H and A respectively (schemes 1&2)

Under argon atmosphere, to a solution of compound H (1 equiv.) in anhydrous DMA (0.2M) was added zinc cyanide (2 equiv.). The reaction mixture was degassed with argon. Pd(PPh₃)₄ (5 mol%) was added. The reaction mixture was heated 10 min at 130°C under microwave irradiation. The reaction mixture was diluted with AcOEt, washed with a saturated sodium bicarbonate solution and brine, dried over magnesium sulfate then concentrated. The resulting crude mixture was purified by flash chromatography to afford the compound H.

### Alternative 1

Under argon atmosphere, to a solution of compound A (1 equiv.) in anhydrous DMA (0.2M) was added zinc cyanide (1.5 equiv.). The reaction mixture was degassed with argon. Pd(PPh₃)₄ (10 mol%) was added. The reaction mixture was heated 3h at 130°C. The reaction mixture was diluted with AcOEt, washed with water and brine, dried over magnesium sulfate then concentrated. The resulting crude mixture was triturated in DCM. The precipitate was filtered, then purified by flash chromatography to afford the compound L.

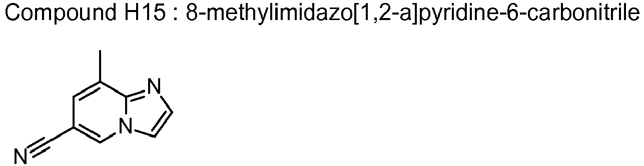

Compound H15 was obtained according to General Procedure IX, starting from 6-bromo-8-methyl-imidazo[1,2-a]pyridine H12. Purification by flash chromatography (DCM/MeOH: 100/0 to 95/5) afforded H15 as a beige powder in 77% yield. M/Z (M+H)⁺: 158

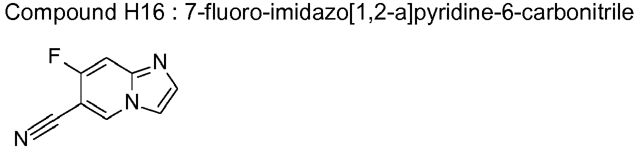

Compound H16 was obtained according to General Procedure IX, starting from 6-bromo-7-fluoro-imidazo[1,2-a]pyridine H14. Purification by flash chromatography (DCM/MeOH: 100/0 to 95/5) afforded H16 as a beige powder in 67% yield. M/Z (M+H)⁺: 162

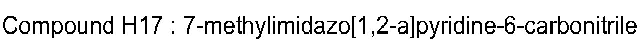

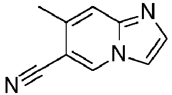

Compound H17 was obtained according to General Procedure IX, starting from 6-bromo-8-methyl-imidazo[1,2-a]pyridine H13. Purification by flash chromatography (DCM/MeOH: 100/0 to 95/5) afforded H17 as a beige powder in 79% yield. M/Z (M+H)⁺: 158

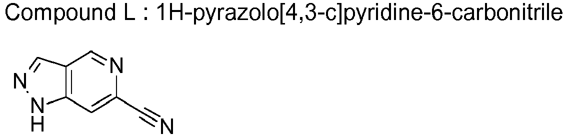

Compound L was obtained according to the General Procedure IX, Alternative 1, starting from 6-bromo-1H-pyrazolo[4,3-c]pyridine A. Purification by flash chromatography (DCM/MeOH: 10/0 to 0/10) afforded L as a white powder in 61% yield. M/Z (M+H)⁺: 145

### General Procedure X : Synthesis of compounds P and T from compounds O and N respectively (scheme 2)

To a solution of compound O or N (1 equiv.) in DMSO (0.1M) were added a solution of H₂O₂, 30% in water, (1.5 equiv.) and potassium carbonate (0.2 equiv.). The reaction mixture was stirred overnight at rt. Water was added to the reaction mixture. The resulting precipitate was filtered then dried under vacuum at 70°C with phosphorus pentoxide to afford compound P or T respectively.

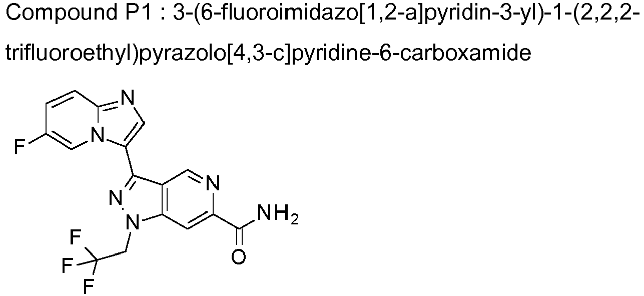

Compound P1 was obtained according to General Procedure X, starting from 3-(6-fluoroimidazo[1,2-a]pyridin-3-yl)-1-(2,2,2-trifluoroethyl)pyrazolo[4,3-c]pyridine-6-carbonitrile O4 (2 equiv.). Precipitation afforded P1 as a beige powder without further purification. M/Z (M+H)⁺: 379

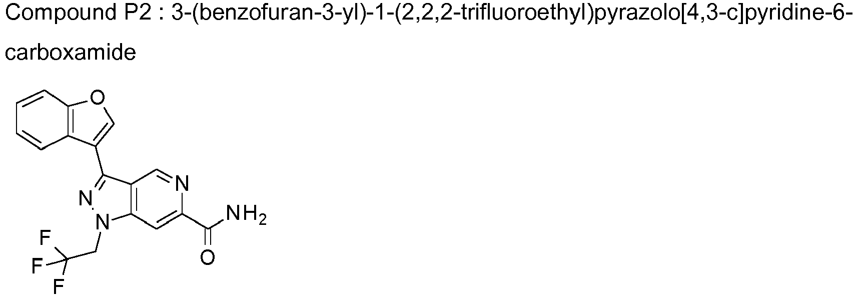

Compound P2 was obtained according to General Procedure X, starting from 3-(benzofuran-3-yl)-1-(2,2,2-trifluoroethyl)pyrazolo[4,3-c]pyridine-6-carbonitrile O6. Precipitation afforded P2 as a beige powder without further purification. M/Z (M+H)⁺: 361

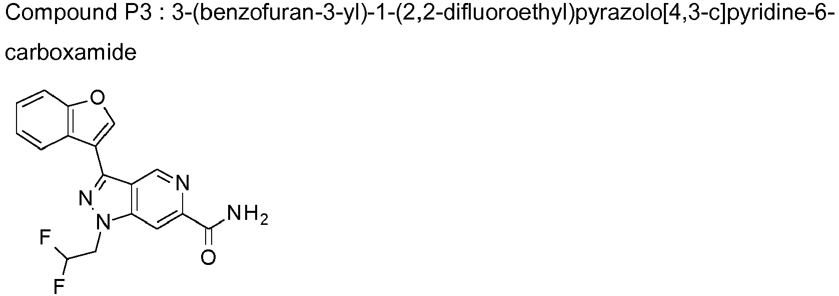

Compound P3 was obtained according to General Procedure X, starting from 3-(benzofuran-3-yl)-1-(2,2-difluoroethyl)pyrazolo[4,3-c]pyridine-6-carbonitrile O1. Precipitation afforded P3 as a beige powder without further purification. M/Z (M+H)⁺: 343

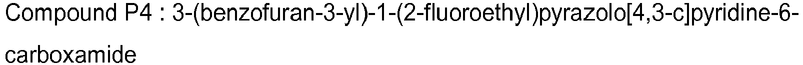

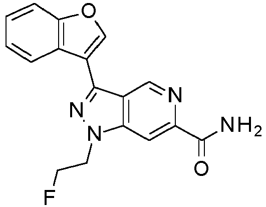

Compound P4 was obtained according to General Procedure X, starting from 3-(benzofuran-3-yl)-1-(2-fluoroethyl)pyrazolo[4,3-c]pyridine-6-carbonitrile O2. Precipitation afforded P4 as a beige powder without further purification. M/Z (M+H)⁺: 325

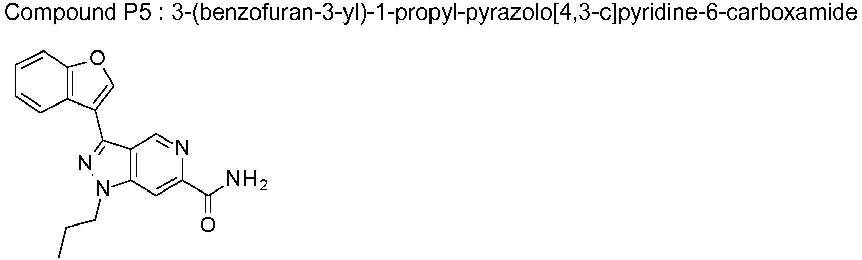

Compound P5 was obtained according to General Procedure X, starting from 3-(benzofuran-3-yl)-1-propyl-pyrazolo[4,3-c]pyridine-6-carbonitrile O3. Precipitation afforded P5 as a beige powder without further purification. M/Z (M+H)⁺: 321

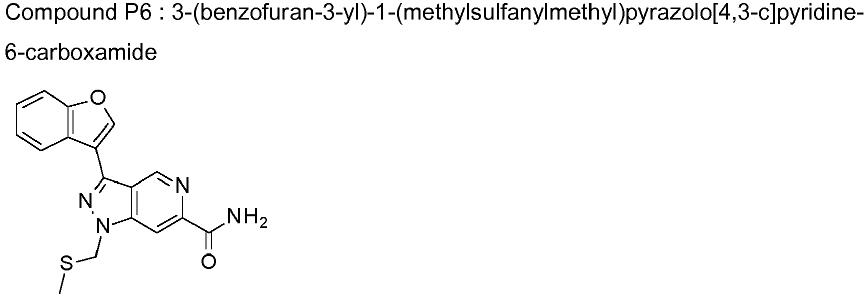

Compound P6 was obtained according to General Procedure X, starting from 3-(benzofuran-3-yl)-1-(methylsulfanylmethyl)pyrazolo[4,3-c]pyridine-6-carbonitrile O7. In that specific case, 4.5 equiv. of H₂O₂, 30% in water and 0.6 equiv. of potassium carbonate were used. Precipitation afforded P6 without further purification. M/Z (M+H)⁺: 339

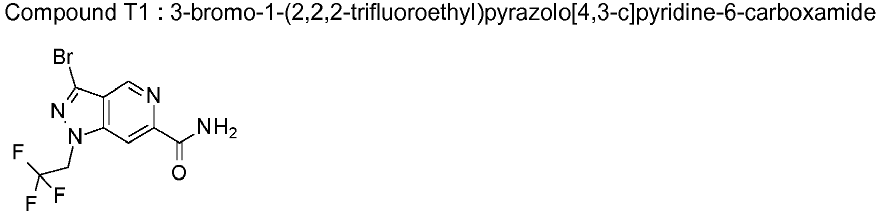

Compound T1 was obtained according to General Procedure X, starting from 3-bromo-1-(2,2,2-trifluoroethyl)pyrazolo[4,3-c]pyridine-6-carbonitrile N1. Precipitation afforded T1 without further purification. M/Z (M+H)⁺: 323/325

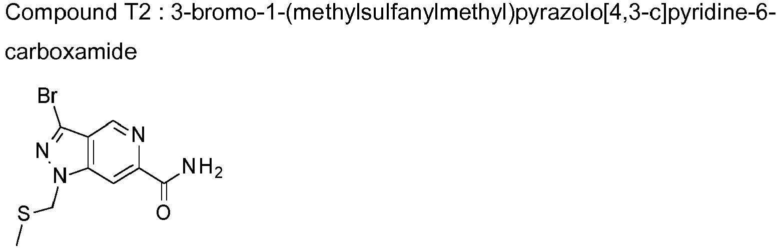

Compound T2 was obtained according to General Procedure X, starting from 3-bromo-1-(methylsulfanylmethyl)pyrazolo[4,3-c]pyridine-6-carbonitrile N2. In that specific case, the filtrate was extracted with AcOEt. The resulting solution was dried over magnesium sulfate, concentrated, combined to the precipitate to afford T2 without further purification. M/Z (M+H)⁺: 301/303

### Compound 128 : 3-(6,8-difluoroimidazo[1,2-a]pyridin-3-yl)-1-(2,2,2-trifluoroethyl)pyrazolo[4,3-c]pyridine-6-carboxamide

Compound 128 was obtained according to General Procedure X, starting from 3-(6,8-difluoroimidazo[1,2-a]pyridin-3-yl)-1-(2,2,2-trifluoroethyl)pyrazolo[4,3-c]pyridine-6-carbonitrile O5. Precipitation afforded 128 as a beige powder in 67% yield without further purification. ¹H-NMR (DMSO-*d₆*,400MHz): 5.86 (q, *J* 9.0 Hz, 2H, CF₃-CH₂); 7.79 (t, *J* 10.3 Hz, 1H, Ar); 7.87 (bs, 1H, NH); 8.29 (br s, 1H, NH); 8.67 (s, 1H, Ar); 8.89 (s, 1H, Ar); 9.44-9.49 (m, 1H, Ar); 9.64 (s, 1H, Ar). M/Z (M+H)⁺: 397

### General Procedure XI : Synthesis of compounds Q and U from compounds P and T respectively (scheme 2)

To a solution of compound P or T (1 equiv.) in MeOH (0.1M) was added DMF-DMA (6 equiv.). The reaction mixture was heated overnight at 50°C. The reaction mixture was concentrated to dryness, then purified by flash chromatgraphy to afford compound Q or U respectively.

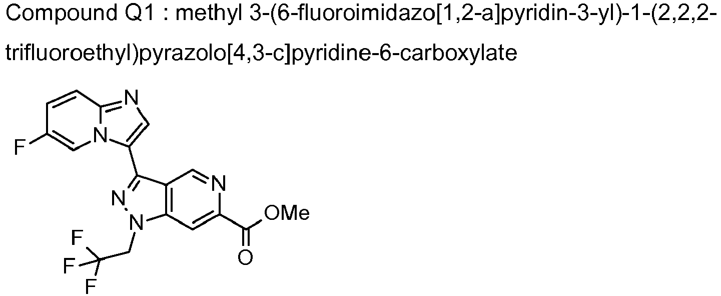

Compound Q1 was obtained according to General Procedure XI, starting from 3-(6-fluoroimidazo[1,2-a]pyridin-3-yl)-1-(2,2,2-trifluoroethyl)pyrazolo[4,3-c]pyridine-6-carboxamide P1. In that specific case, 3 equiv. of DMF-DMA were used. Purification by flash chromatography (DCM/MeOH: 100/0 to 96/2) afforded Q1 as a beige powder in 78% yield. M/Z (M+H)⁺: 394

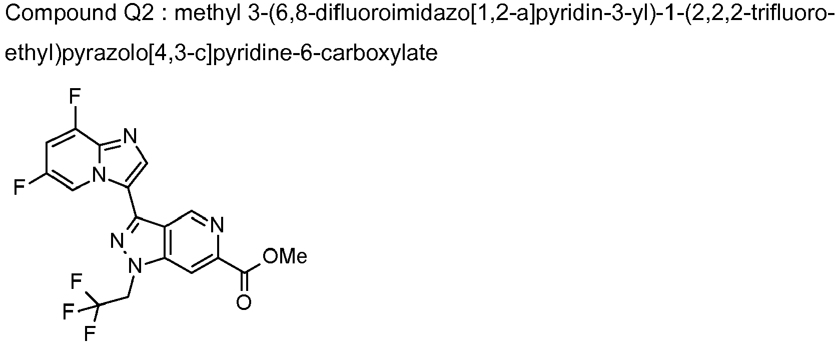

Compound Q2 was obtained according to General Procedure XI, starting from 3-(6,8-difluoroimidazo[1,2-a]pyridin-3-yl)-1-(2,2,2-trifluoroethyl)pyrazolo[4,3-c]pyridine-6-carboxamide 133. Purification by flash chromatography (DCM/MeOH: 100/0 to 97/3) afforded Q2 as a white powder in 71% yield. M/Z (M+H)⁺: 412

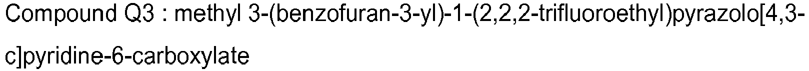

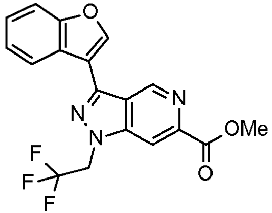

Compound Q3 was obtained according to General Procedure XI, starting from 3-(benzofuran-3-yl)-1-(2,2,2-trifluoroethyl)pyrazolo[4,3-c]pyridine-6-carboxamide P2. Purification by flash chromatography (DCM/MeOH: 100/0 to 95/5) afforded Q3 as a white powder in 47% yield. M/Z (M+H)⁺: 376

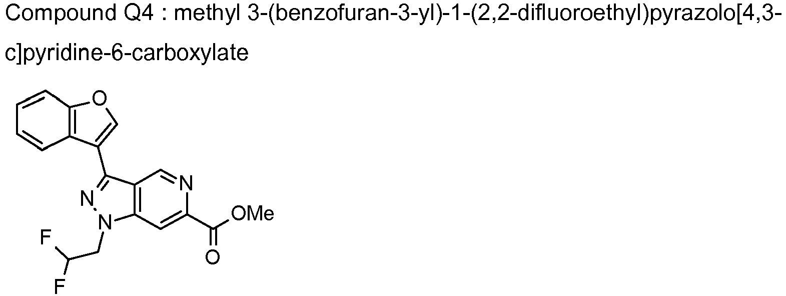

Compound Q4 was obtained according to General Procedure XI, starting from 3-(benzofuran-3-yl)-1-(2,2-difluoroethyl)pyrazolo[4,3-c]pyridine-6-carboxamide P3. Purification by flash chromatography (DCM/MeOH: 100/0 to 98/2) afforded Q4 as a white powder in 72% yield. M/Z (M+H)⁺: 358

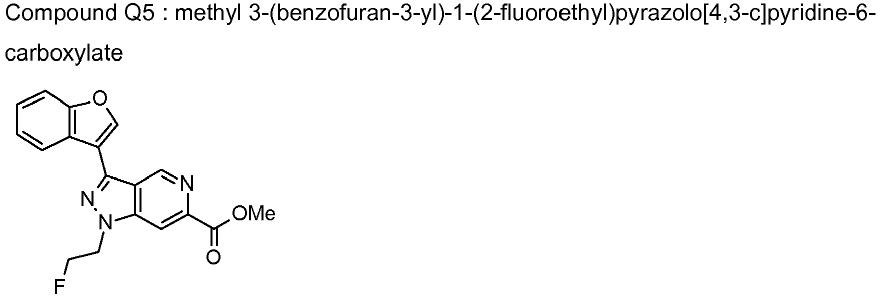

Compound Q5 was obtained according to General Procedure XI, starting from 3-(benzofuran-3-yl)-1-(2-fluoroethyl)pyrazolo[4,3-c]pyridine-6-carboxamide P4. Purification by flash chromatography (DCM/MeOH: 100/0 to 99/1) afforded Q5 as a yellow powder in 71% yield. M/Z (M+H)⁺: 340

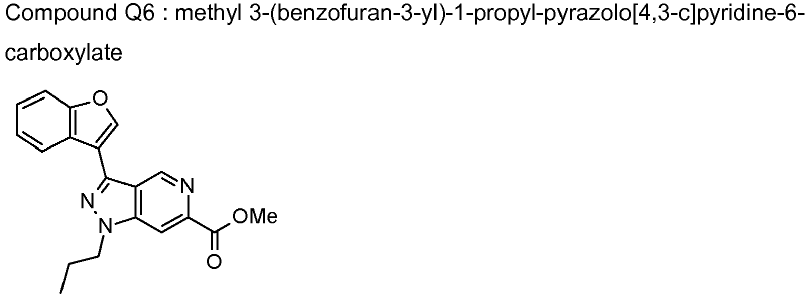

Compound Q6 was obtained according to General Procedure XI, starting from 3-(benzofuran-3-yl)-1-propyl-pyrazolo[4,3-c]pyridine-6-carboxamide P5. Purification by flash chromatography (DCM/MeOH: 100/0 to 98/2) afforded Q6 as a beige powder in 82% yield. M/Z (M+H)⁺: 336

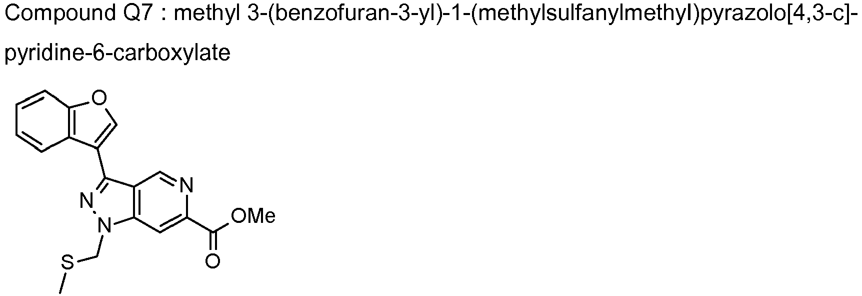

Compound Q7 was obtained according to General Procedure XI, starting from 3-(benzofuran-3-yl)-1-(methylsulfanylmethyl)pyrazolo[4,3-c]pyridine-6-carboxamide P6. Purification by flash chromatography (DCM/MeOH: 100/0 to 95/5) afforded Q7 in 67% yield. M/Z (M+H)⁺: 354

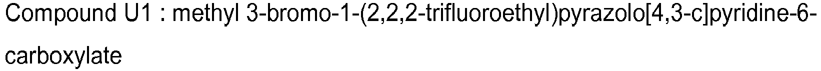

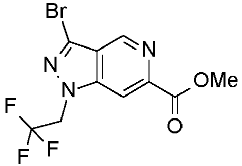

Compound U1 was obtained according to General Procedure XI, starting from 3-bromo-1-(2,2,2-trifluoroethyl)pyrazolo[4,3-c]pyridine-6-carboxamide T1. Purification by flash chromatography (Cyclohexane/AcOEt: 5/5 to 2/8) afforded U1 in 80% yield. M/Z (M+H)⁺: 338/340

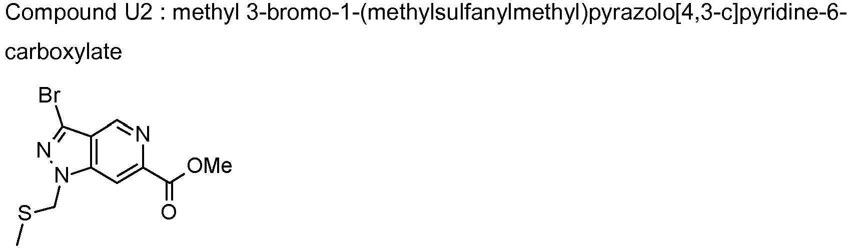

Compound U2 was obtained according to General Procedure XI, starting from 3-bromo-1-(methylsulfanylmethyl)pyrazolo[4,3-c]pyridine-6-carboxamide T2. Concentration of the reaction mixture afforded U2 without further purification. M/Z (M+H)⁺: 316/318

### General Procedure XII : Synthesis of compounds R and V from compounds Q and U respectively (scheme 2)

To a solution of compound Q or U (1 equiv.) in THF (0.1M) were added LiOH, 1N in water (1.1 equiv.). The reaction mixture was stirred 2h at rt. The reaction mixture was concentrated to dryness to afford compound R or V respectively.

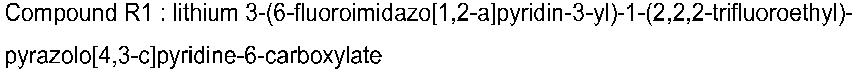

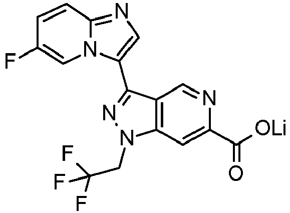

Compound R1 was obtained according to General Procedure XII, starting from methyl 3-(6-fluoroimidazo[1,2-a]pyridin-3-yl)-1-(2,2,2-trifluoroethyl)pyrazolo[4,3-c]pyridine-6-carboxylate Q1. Concentration of the reaction mixture afforded R1 as a beige powder in quantitative yield. M/Z (M+H)⁺: 380

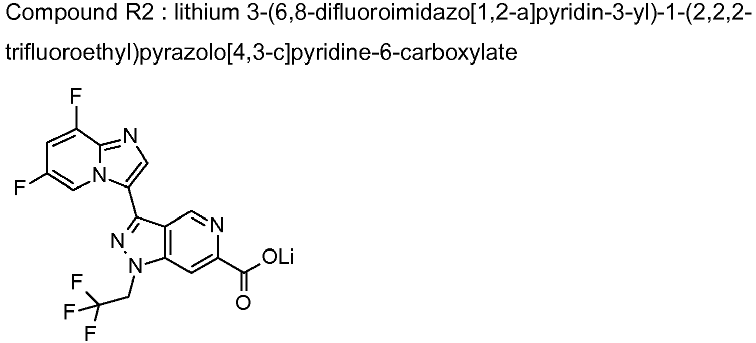

Compound R2 was obtained according to General Procedure XII, starting from methyl 3-(6,8-difluoroimidazo[1,2-a]pyridin-3-yl)-1-(2,2,2-trifluoroethyl)pyrazolo[4,3-c]pyridine-6-carboxylate Q2. In that specific case, 2.3 equiv. of LiOH, 1N in water were used. Concentration of the reaction mixture afforded R2 as a beige powder in quantitative yield. M/Z (M+H)⁺: 398

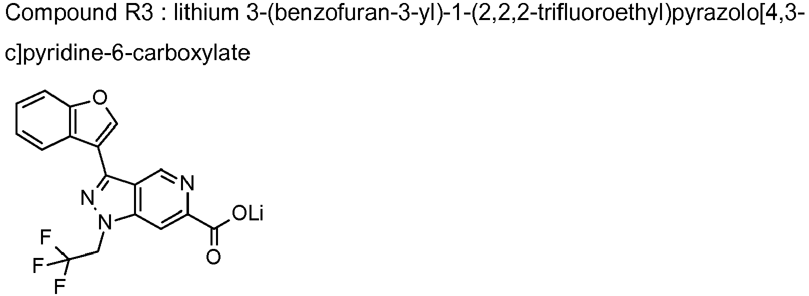

Compound R3 was obtained according to General Procedure XII, starting from methyl 3-(benzofuran-3-yl)-1-(2,2,2-trifluoroethyl)pyrazolo[4,3-c]pyridine-6-carboxylate Q3. In that specific case, 1.5 equiv. of LiOH, 1N in water were used. Concentration of the reaction mixture afforded R3 in quantitative yield. M/Z (M+H)⁺: 362

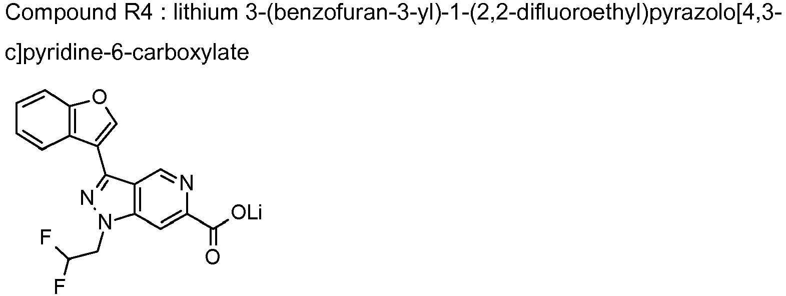

Compound R4 was obtained according to General Procedure XII, starting from methyl 3-(benzofuran-3-yl)-1-(2,2-difluoroethyl)pyrazolo[4,3-c]pyridine-6-carboxylate Q4. In that specific case, 2.0 equiv. of LiOH, 1N in water were used. Concentration of the reaction mixture afforded R4 as a yellow powder in quantitative yield. M/Z (M+H)⁺: 344

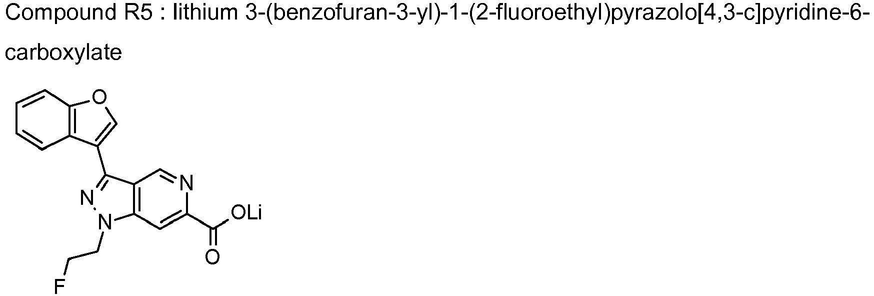

Compound R5 was obtained according to General Procedure XII, starting from methyl 3-(benzofuran-3-yl)-1-(2-fluoroethyl)pyrazolo[4,3-c]pyridine-6-carboxylate Q5. In that specific case, 2.0 equiv. of LiOH, 1N in water were used. Concentration of the reaction mixture afforded R5 as a yellow powder in quantitative yield. M/Z (M+H)⁺: 326

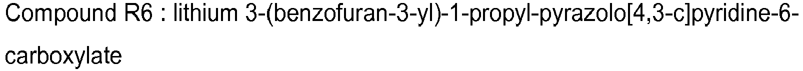

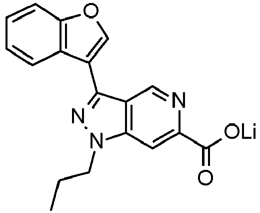

Compound R6 was obtained according to General Procedure XII, starting from methyl 3-(benzofuran-3-yl)-1-propyl-pyrazolo[4,3-c]pyridine-6-carboxylate Q6. In that specific case, 2.0 equiv. of LiOH, 1N in water were used. Concentration of the reaction mixture afforded R6 as a yellow powder in quantitative yield. M/Z (M+H)⁺: 322

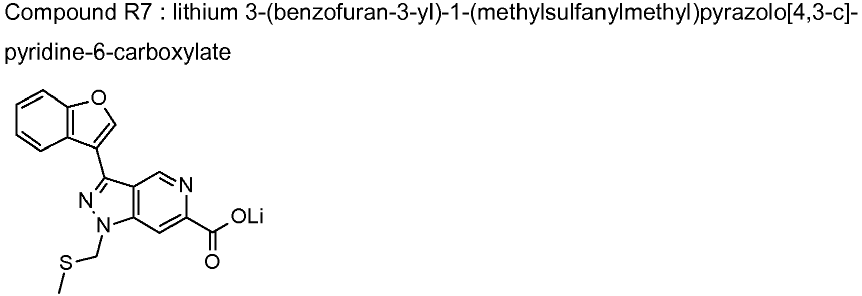

Compound R7 was obtained according to General Procedure XII, starting from methyl 3-(benzofuran-3-yl)-1-(methylsulfanylmethyl)pyrazolo[4,3-c]pyridine-6-carboxylate Q7. Concentration of the reaction mixture afforded R7 in quantitative yield. M/Z (M+H)⁺: 340

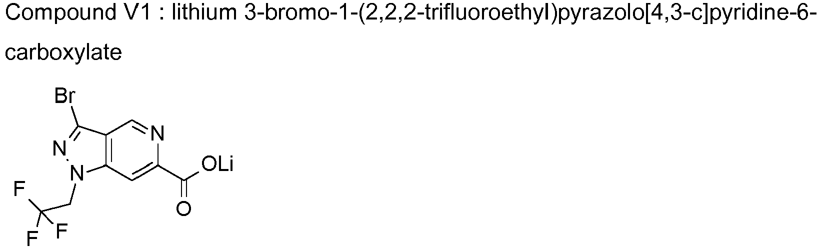

Compound V1 was obtained according to General Procedure XII, starting from methyl 3-bromo-1-(2,2,2-trifluoroethyl)pyrazolo[4,3-c]pyridine-6-carboxylate U1. In that specific case, 2.0 equiv. of LiOH, 1N in water were used. Concentration of the reaction mixture afforded V1 in quantitative yield. M/Z (M+H)⁺: 324/326

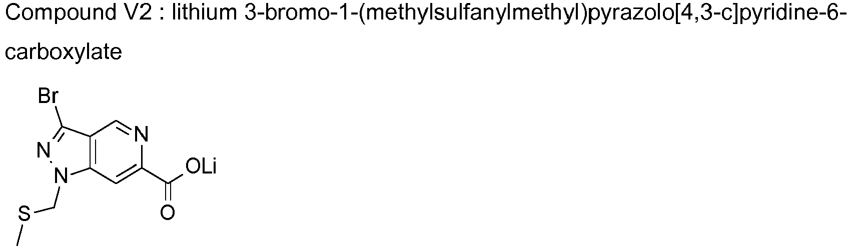

Compound V2 was obtained according to General Procedure XII, starting from methyl 3-bromo-1-(methylsulfanylmethyl)pyrazolo[4,3-c]pyridine-6-carboxylate U2. Concentration of the reaction mixture afforded V2 in quantitative yield. M/Z (M+H)⁺: 283/285

### Compound 129 : 3-[6-(1,4-oxazepane-4-carbonyl)-1-(2,2,2-trifluoroethyl)pyrazolo[4,3-c]pyridin-3-yl]benzofuran-5-carboxylic acid

Compound 129 was obtained according to General Procedure XII, starting from methyl 3-[6-(1,4-oxazepane-4-carbonyl)-1-(2,2,2-trifluoroethyl)pyrazolo[4,3-c]pyridin-3-yl]benzofuran-5-carboxylate 129. In that specific case, the reaction mixture was diluted with water, acidified with HCl, 1N in water, then extracted with ethyl acetate. The combined organic layers were filtered on a hydrophobic cartridge, then contentrated to afford 129 as a white powder in 56% yield. ¹H-NMR (DMSO-*d_{6,}* 400MHz): 1.78 (quint, *J* 5.9 Hz, 1H, CH₂); 1.95 (quint, *J* 5.9 Hz, 1H, CH₂); 3.48-3.53 (m, 2H, CH₂); 3.64-3.68 (m, 1H, N-CH₂); 3.70-3.81 (m, 5H, N-CH₂, O-CH₂); 5.72 (d, *J* 9.0 Hz, 2H, CF₃-CH₂); 7.82-7.86 (m, 1H, Ar); 8.04-8.09 (m, 1H, Ar); 8.21 (bs, 1H, Ar, signal of a rotamer); 8.22 (bs, 1H, Ar, signal of a rotamer); 8.96-8.99 (m, 1H, Ar); 9.28-9.30 (m, 1H, Ar); 9.59 (d, *J* 1.0 Hz, 1H, Ar, signal of a rotamer); 9.60 (d, *J* 1.0 Hz, 1H, Ar, signal of a rotamer). M/Z (M+H)⁺: 489

### Exemple 130 : 1-[3-(benzofuran-3-yl)-1-(2,2,2-trifluoroethyl)pyrazolo[4,3-c]pyridine-6-carbonyl]piperidine-4-carboxylic acid

Exemple 130 was obtained according to General Procedure XII, starting from ethyl 1-[3-(benzofuran-3-yl)-1-(2,2,2-trifluoroethyl)pyrazolo[4,3-c]pyridine-6-carbonyl]piperidine-4-carboxylate K31. In that specific case, the reaction mixture was cooled down to 0°C, a 1N HCl solution was added dropwise. The obtained precipitate was filtered. Purification by preparative HPLC afforded 130 in 23% yield. ¹H-NMR (DMSO-*d_{6,}* 400MHz): 1.50-1.63 (m, 2H, CH₂); 1.74-1.83 (m, 1H, CH₂); 1.92-2.01 (m, 1H, CH₂); 2.55-2.64 (m, 1H, CH); 2.97-3.07 (m, 1H, CH₂); 3.01-3.19 (m, 1H, CH₂); 3.59-3.67 (m, 1H, CH₂); 4.38-4.46 (m, 1H, CH₂); 5.63-5.74 (m, 2H, CH₂-CF₃); 7.42-7.50 (m, 2H, Ar); 7.72-7.76 (m, 1H, Ar); 8.16 (bs, 1H, Ar); 8.33-8.36 (m, 1H, Ar); 9.17 (s, 1H, Ar); 9.58 (d, *J* 1.0 Hz, 1H, Ar); 12.30 (s, 1H, CO₂H). M/Z (M+H)⁺: 473

### General Procedure XIII : Synthesis of compounds F and K from compounds V and R (scheme 2)

To a solution of compound R or V (1 equiv.) in DMA (0.1M) were added compound B (1.3 equiv.), diisopropylethylamine (3 equiv.) and BOP (1.5 equiv.). The reaction mixture was stirred 30min at rt. The reaction mixture was diluted with AcOEt, washed with water and brine, dried over magnesium sulfate, then concentrated. The resulting crude mixture was purified by flash chromatography to afford compound F or K.

### Alternative 1:

To a solution of compound R or V (1 equiv.) in NMP (0.1M) were added compound B (1.3 equiv.), diisopropylethylamine (4 equiv.) and HATU (1.5 equiv.). The reaction mixture was stirred 1h at rt. The reaction mixture was diluted with AcOEt, washed with a saturated ammonium chloride solution and brine, dried over magnesium sulfate, then concentrated. The resulting crude mixture was purified by flash chromatography to afford compound F or K.

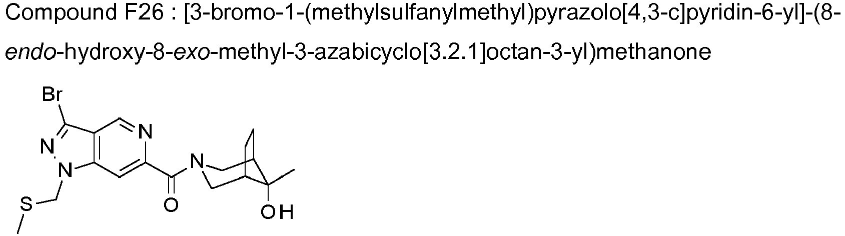

Compound F26 was obtained according to General Procedure XIII, starting from lithium 3-bromo-1-(methylsulfanylmethyl)pyrazolo[4,3-c]pyridine-6-carboxylate V2 and *endo*-(8-exo-methyl)-3-azabicyclo[3.2.1]octan-8-ol B35. Purification by flash chromatography (Cyclohexane/AcOEt: 10/0 to 6/4) afforded F26 in 74% yield. M/Z (M+H)⁺: 425/427

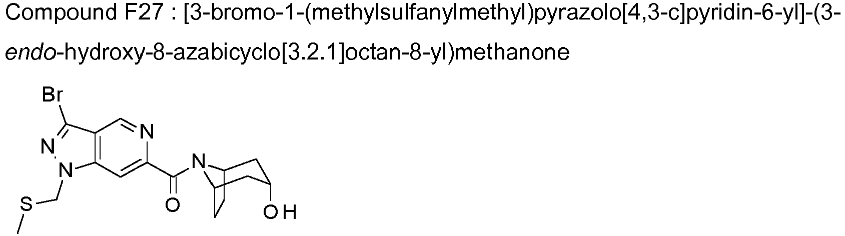

Compound F27 was obtained according to General Procedure XIII, starting from lithium 3-bromo-1-(methylsulfanylmethyl)pyrazolo[4,3-c]pyridine-6-carboxylate V2 and nortropine B41. Purification by flash chromatography (Cyclohexane/AcOEt: 10/0 to 0/10) afforded F27 in 88% yield. M/Z (M+H)⁺: 411/413

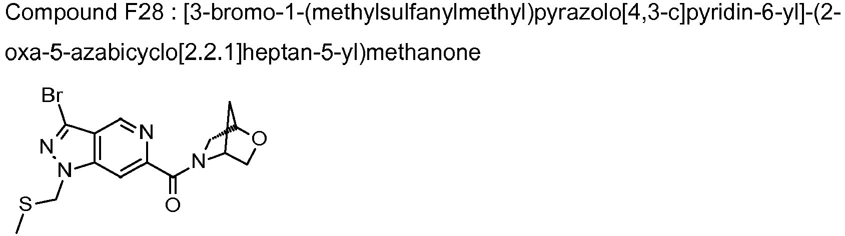

Compound F28 was obtained according to General Procedure XIII, starting from lithium 3-bromo-1-(methylsulfanylmethyl)pyrazolo[4,3-c]pyridine-6-carboxylate V2 and 2-oxa-5-azabicyclo[2.2.1]heptane hydrochloride B8. Purification by flash chromatography (Cyclohexane/AcOEt: 10/0 to 0/10) afforded F28 in 88% yield. M/Z (M+H)⁺: 383/385

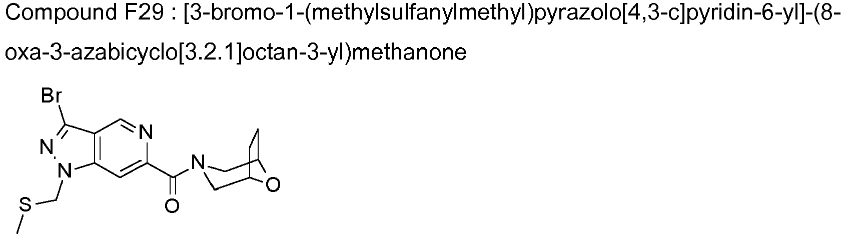

Compound F29 was obtained according to General Procedure XIII, starting from lithium 3-bromo-1-(methylsulfanylmethyl)pyrazolo[4,3-c]pyridine-6-carboxylate V2 and 8-oxa-3-azabicyclo[3.2.1]octane hydrochloride B3. Purification by flash chromatography (Cyclohexane/AcOEt: 10/0 to 5/5) afforded F29 in 71% yield. M/Z (M+H)⁺: 397/399

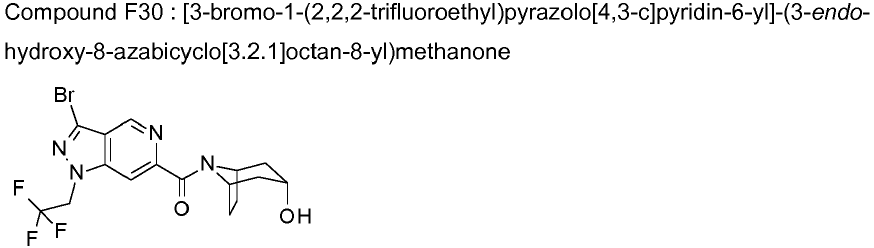

Compound F30 was obtained according to General Procedure XIII, starting from lithium 3-bromo-1-(2,2,2-trifluoroethyl)pyrazolo[4,3-c]pyridine-6-carboxylate V1 and nortropine B41. Purification by flash chromatography (DCM/MeOH : 100/0 to 95/5) afforded F30 as a brown powder in 65% yield. M/Z (M+H)⁺: 433/435

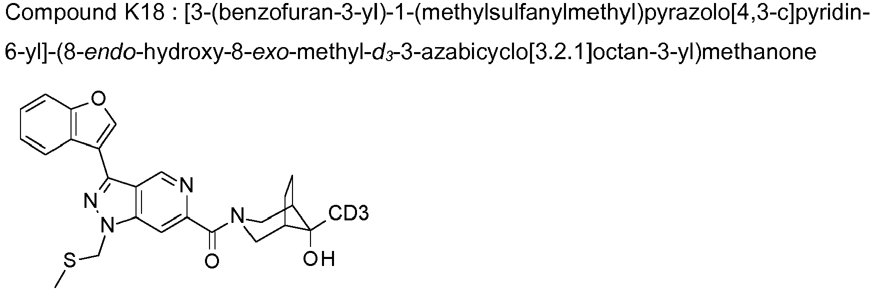

Compound K18 was obtained according to General Procedure XIII, starting from lithium 3-(benzofuran-3-yl)-1-(methylsulfanylmethyl)pyrazolo[4,3-c]pyridine-6-carboxylate R7 and *endo*-(8-exo-methyl-d₃)-3-aza-bicyclo[3.2.1]octan-8-ol B83. Purification by flash chromatography (DCM/MeOH : 100/0 to 95/5) afforded K18 in 61% yield. M/Z (M+H)⁺: 466

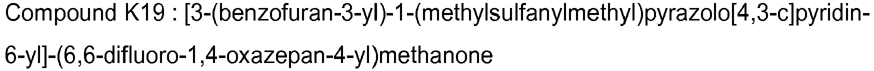

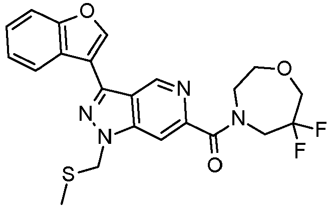

Compound K19 was obtained according to General Procedure XIII, starting from lithium 3-(benzofuran-3-yl)-1-(methylsulfanylmethyl)pyrazolo[4,3-c]pyridine-6-carboxylate R7 and 6,6-difluoro-1,4-oxazepane hydrochloride B58. In that specific case, 4 equiv. of diisopropylethylamine were used. Purification by flash chromatography (DCM/MeOH : 100/0 to 95/5) afforded K19 in 56% yield. M/Z (M+H)⁺: 459

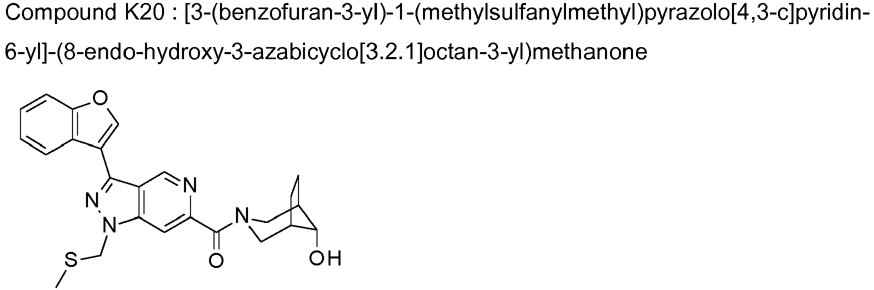

Compound K20 was obtained according to General Procedure XIII, starting from lithium 3-(benzofuran-3-yl)-1-(methylsulfanylmethyl)pyrazolo[4,3-c]pyridine-6-carboxylate R7 and *endo*-3-aza-bicyclo[3.2.1]octan-8-ol B85. Purification by flash chromatography (DCM/MeOH : 100/0 to 95/5) afforded K20 in 67% yield. M/Z (M+H)⁺: 449

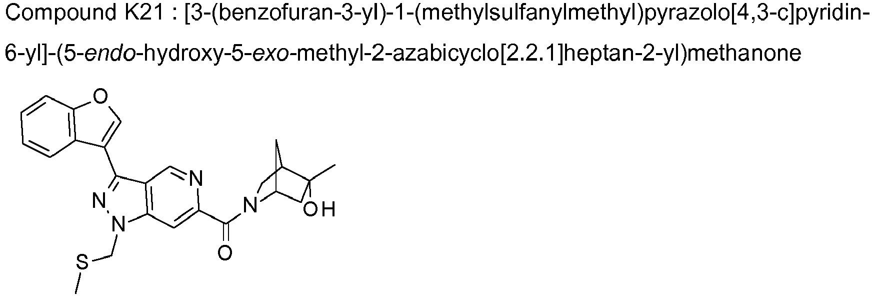

Compound K21 was obtained according to General Procedure XIII, starting from lithium 3-(benzofuran-3-yl)-1-(methylsulfanylmethyl)pyrazolo[4,3-c]pyridine-6-carboxylate R7 and *endo*-(5-exo-methyl)-2-azabicyclo[2.2.1]heptan-5-ol B82. Purification by flash chromatography (DCM/MeOH : 100/0 to 95/5) afforded K21 in 52% yield. M/Z (M+H)⁺: 449

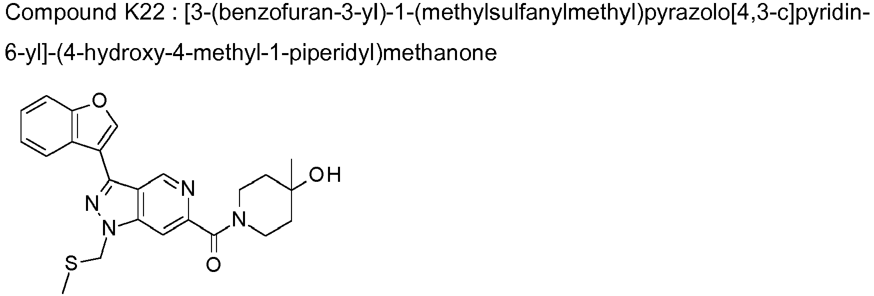

Compound K22 was obtained according to General Procedure XIII, starting from lithium 3-(benzofuran-3-yl)-1-(methylsulfanylmethyl)pyrazolo[4,3-c]pyridine-6-carboxylate R7 and 4-methylpiperidin-4-ol B6. Purification by flash chromatography (DCM/MeOH : 100/0 to 95/5) afforded K22 in 58% yield. M/Z (M+H)⁺: 437

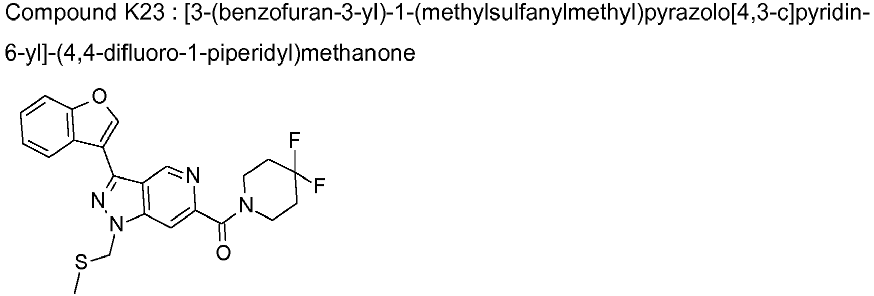

Compound K23 was obtained according to General Procedure XIII, starting from lithium 3-(benzofuran-3-yl)-1-(methylsulfanylmethyl)pyrazolo[4,3-c]pyridine-6-carboxylate R7 and 4,4-difluoropiperidine hydrochloride B18. In that specific case, 4 equiv. of diisopropylethylamine were used. Purification by flash chromatography (DCM/MeOH : 100/0 to 95/5) afforded K23 in 49% yield. M/Z (M+H)⁺: 444

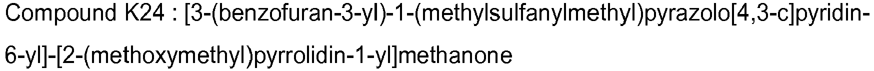

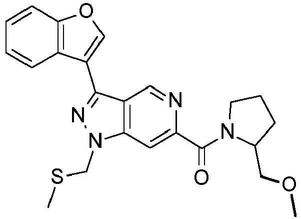

Compound K24 was obtained according to General Procedure XIII, starting from lithium 3-(benzofuran-3-yl)-1-(methylsulfanylmethyl)pyrazolo[4,3-c]pyridine-6-carboxylate R7 and 2-(methoxymethyl)pyrrolidine B22. Purification by flash chromatography (DCM/MeOH : 100/0 to 95/5) afforded K24 in 86% yield. M/Z (M+H)⁺: 437

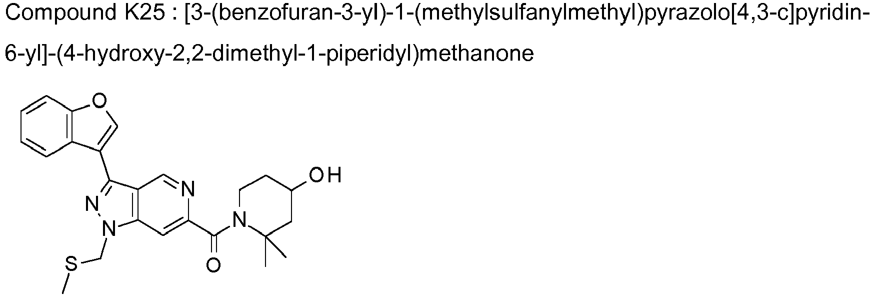

Compound K25 was obtained according to General Procedure XIII, starting from lithium 3-(benzofuran-3-yl)-1-(methylsulfanylmethyl)pyrazolo[4,3-c]pyridine-6-carboxylate R7 and 2,2-dimethylpiperidin-4-ol B14. Purification by flash chromatography (DCM/MeOH : 100/0 to 95/5) afforded K25 in 73% yield. M/Z (M+H)⁺: 451

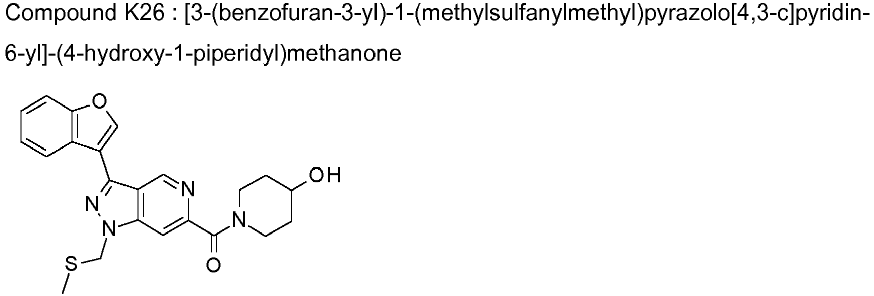

Compound K26 was obtained according to General Procedure XIII, Alternative 1, starting from lithium 3-(benzofuran-3-yl)-1-(methylsulfanylmethyl)pyrazolo[4,3-c]pyridine-6-carboxylate R7 and piperidin-4-ol B5. Purification by flash chromatography (DCM/MeOH : 100/0 to 95/5) afforded K26 in 63% yield. M/Z (M+H)⁺: 423

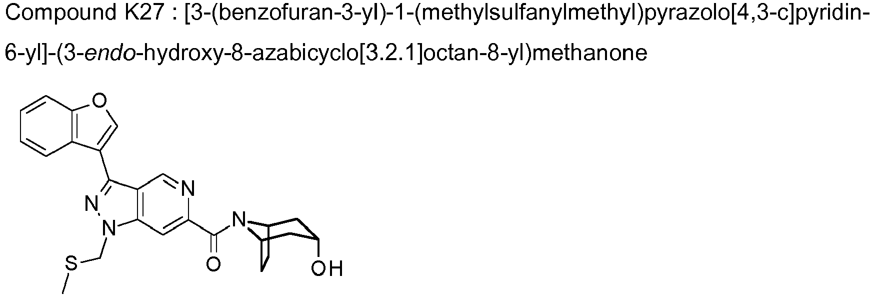

Compound K27 was obtained according to General Procedure XIII, Alternative 1, starting from lithium 3-(benzofuran-3-yl)-1-(methylsulfanylmethyl)pyrazolo[4,3-c]pyridine-6-carboxylate R7 and nortropine B41. Purification by flash chromatography (DCM/MeOH : 100/0 to 95/5) afforded K27 in 48% yield. M/Z (M+H)⁺: 449

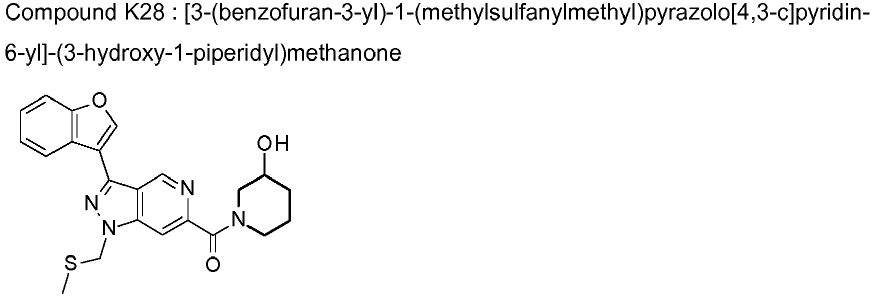

Compound K28 was obtained according to General Procedure XIII, Alternative 1, starting from lithium 3-(benzofuran-3-yl)-1-(methylsulfanylmethyl)pyrazolo[4,3-c]pyridine-6-carboxylate R7 and piperidin-3-ol B24. Purification by flash chromatography (DCM/MeOH : 100/0 to 95/5) afforded K28 in 65% yield. M/Z (M+H)⁺: 423

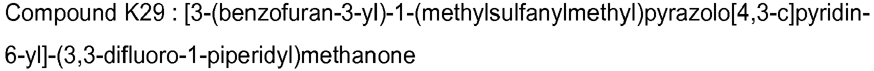

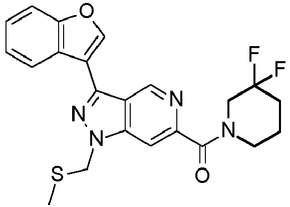

Compound K29 was obtained according to General Procedure XIII, Alternative 1, starting from lithium 3-(benzofuran-3-yl)-1-(methylsulfanylmethyl)pyrazolo[4,3-c]pyridine-6-carboxylate R7 and 3,3-difluoro-piperidine B32. Purification by flash chromatography (DCM/MeOH : 100/0 to 95/5) afforded K29 in 70% yield. M/Z (M+H)⁺: 443

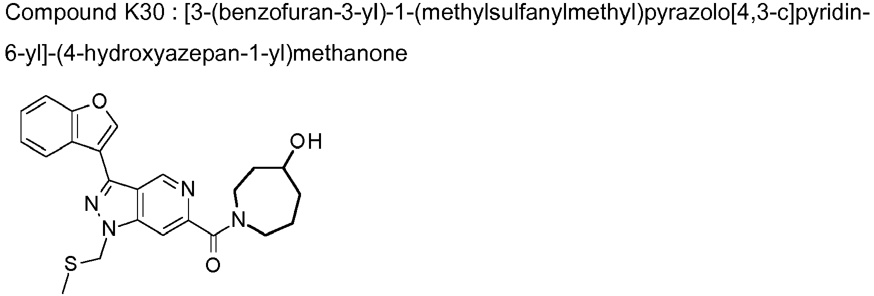

Compound K30 was obtained according to General Procedure XIII, Alternative 1, starting from lithium 3-(benzofuran-3-yl)-1-(methylsulfanylmethyl)pyrazolo[4,3-c]pyridine-6-carboxylate R7 and azepan-4-ol B50. Purification by flash chromatography (DCM/MeOH : 100/0 to 95/5) afforded K30 in 82% yield. M/Z (M+H)⁺: 437

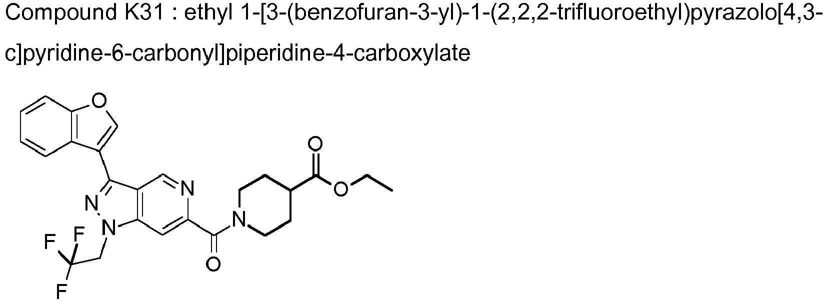

Compound K31 was obtained according to General Procedure XIII, Alternative 1, starting from lithium 3-(benzofuran-3-yl)-1-(2,2,2-trifluoroethyl)pyrazolo[4,3-c]pyridine-6-carboxylate R3 and ethyl isonipecotate B99. Purification by flash chromatography (Cyclohexane/AcOEt: 10/0 to 5/5) afforded K31 in 36% yield. M/Z (M+H)⁺: 501

### Compound 131 : [3-(6-Fluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone

Compound 131 was obtained according to General Procedure XIII, starting from lithium 3-(6-fluoroimidazo[1,2-a]pyridin-3-yl)-1-(2,2,2-trifluoroethyl)pyrazolo[4,3-c]pyridine-6-carboxylate R1 and piperidin-4-ol B5. Purification by flash chromatography (DCM/MeOH: 10/0 to 9/1) afforded 131 as a white powder in 72% yield. ¹H-NMR (DMSO-*d₆*,400MHz): 1.33-1.50 (m, 2H, CH₂); 1.66-1.74 (m, 1H, CH₂); 1.81-1.90 (m, 1H, CH₂); 3.11-3.20 (m, 1H, CH₂); 3.25-3.30 (m, 1H, CH₂); 3.50-3.60 (m, 1H, CH₂); 3.73-3.82 (m, 1H, CH₂); 4.06-4.15 (m, 1H, CH₂); 4.82 (d, *J* 4.0 Hz, 1H, OH); 5.74 (q, *J* 9.0 Hz, 2H, CH₂); 7.60 (ddd, *J* 10.0, 8.2, 2.5 Hz, 1H, Ar); 7.91 (dd, *J* 10.0, 5.3 Hz, 1H, Ar); 8.16 (bs, 1H, Ar); 8.84 (s, 1H, Ar); 9.57 (ddd, *J* 5.1, 2.5, 0.5 Hz, 1H, Ar); 9.65 (d, *J* 1.0 Hz, 1H, Ar). M/Z (M+H)⁺: 463

### Compound 132 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone

Compound 132 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and piperidin-4-ol B5. Purification by flash chromatography (DCM/MeOH: 10/0 to 9/1) afforded 132 as a white powder in 63% yield. ¹H-NMR (DMSO-*d_{6,}* 400MHz): 1.33-1.50 (m, 2H, CH₂); 1.67-1.76 (m, 1H, CH₂); 1.82-1.90 (m, 1H, CH₂); 3.11-3.20 (m, 1H, CH); 3.26-3.36 (m, 1H, CH₂); 3.50-3.59 (m, 1H, CH₂); 3.74-3.82 (m, 1H, CH₂); 4.06-4.16 (m, 1H, CH₂); 4.80 (d, *J* 4.0 Hz, 1H, OH); 5.75 (q, *J* 9.0 Hz, 2H, CF₃-CH₂); 7.77 (ddd, *J* 11.0, 9.1, 2.0 Hz, 1H, Ar); 8.17 (bs, 1H, Ar); 8.87 (s, 1H, Ar); 9.48 (ddd, *J* 4.7, 2.0, 0.5 Hz, 1H, Ar); 9.66 (d, *J* 1.0 Hz, 1H, Ar). M/Z (M+H)⁺: 481

### Compound 133 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-methyl-piperidin-1-yl)-methanone

Compound 133 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-methylpiperidin-4-ol B6. Purification by flash chromatography (DCM/MeOH: 10/0 to 9/1) afforded 133 as a white powder in 68% yield. ¹H-NMR (DMSO-*d_{6,}* 400MHz): 1.18 (s, 3H, CH₃); 1.40-1.65 (m, 4H, CH₂); 3.32-3.42 (m, 3H, CH₂); 4.11-4.19 (m, 1H, CH₂); 4.46 (s, 1H, OH); 5.75 (q, *J* 9.0 Hz, 2H, CH₂-CF₃); 7.77 (ddd, *J* 11.1, 9.2, 2.0 Hz 1H, Ar); 8.17 (bs, 1H, Ar); 8.87 (s, 1H, Ar); 9.46-9.50 (m, 1H, Ar); 9.66 (d, *J* 1.0 Hz, 1H, Ar). M/Z (M+H)⁺: 495

### Compound 134 : [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxy-azepan-1-yl)-methanone

Compound 134 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and azepan-3-ol B7. Purification by flash chromatography (DCM/MeOH: 100/0 to 95/5) afforded 134 as a white powder in 71% yield (racemic mixture). ¹H-NMR (DMSO-*d_{6,}* 400MHz): 1.35-1.48 (m, 1H, CH₂); 1.48-1.65 (m, 2H, CH₂); 1.65-1.92 (m, 3H, CH₂); 3.04 (dd, *J* 13.1, 9.0 Hz, 1H, signal of a rotamer, CH₂); 3.14 (dd, *J* 14.3, 7.8 Hz, 1H, signal of a rotamer, CH₂); 3.23-3.31 (m, 1H, signal of a rotamer, CH₂); 3.34-3.45 (m, 2H, signal of a rotamer, CH₂); 3.57-3.65 (m, 1H, signal of a rotamer, CH2); 3.65-3.74 (m, 1H, signal of a rotamer, CH); 3.87-3.96 (m, 2H, signal of a rotamer, CH, CH₂); 4.19 (dd, *J* 13.1, 4.9 Hz, 1H, signal of a rotamer, CH₂); 4.69 (d, *J* 4.4 Hz, 1H, signal of a rotamer, OH); 4.88 (d, *J* 4.4 Hz, 1H, signal of a rotamer, OH); 5.70-5.81 (m, 2H, CF₃-CH₂); 7.77-7.84 (m, 1H, Ar); 8.17 (bs, 1H, signal of a rotamer, Ar); 8.18 (bs, 1H, signal of a rotamer, Ar); 8.86 (s, 1H, signal of a rotamer, Ar); 8.88 (s, 1H, signal of a rotamer, Ar); 9.46-9.51 (m, 1H, Ar); 9.66 (d, *J* 0.9 Hz, 1H, signal of a rotamer, Ar); 9.67 (d, *J* 0.9 Hz, 1H, signal of a rotamer, Ar). M/Z (M+H)⁺: 495

### Compound 135 : [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[2.2.1]hept-5-yl)-methanone

Compound 135 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-oxa-5-azabicyclo[2.2.1]heptane hydrochloride B8. In that specific case, 5 equiv. of diisopropylethylamine were used. Purification by flash chromatography (DCM/MeOH: 100/0 to 95/5) afforded 135 as a beige powder in 67% yield. ¹H-NMR (DMSO-*d_{6,}* 400MHz): 1.80-1.97 (m, 2H, CH₂); 3.42-3.47 (m, 1H, major rotamer, CH₂); 3.56-3.61 (m, 1H, major rotamer, CH₂); 3.63-3.68 (m, 1H, minor rotamer, CH₂); 3.77-3.84 (m, 2H, minor rotamer, 1H, major rotamer, CH₂); 3.85-3.89 (m, 1H minor rotamer, CH₂); 3.91-3.96 (m, 1H major rotamer, CH₂); 4.64 (bs, 1H, minor rotamer, CH); 4.70 (bs, 1H, major rotamer, CH); 5.00 (bs, 1H, minor rotamer, CH); 5.17 (bs, 1H, major rotamer, CH); 5.75-5.87 (m, 2H, CH₂-CF₃); 7.77-7.84 (m, 1H, Ar); 8.43 (bs, 1H, major rotamer, Ar); 8.48 (bs, 1H, minor rotamer, Ar); 8.86 (s, 1H, minor rotamer, Ar); 8.88 (s, 1H, major rotamer, Ar); 9.46-9.51 (m, 1H, Ar); 9.65 (d, *J* 0.9 Hz, 1H, minor rotamer, Ar); 9.70 (d, *J 0.9* Hz, 1H, major rotamer, Ar). M/Z (M+H)⁺: 479

### Compound 136 : [3-(6,8-Difluoro-[1,2,4]triazolo[4,3-a]pyridin-3-yl)-1-(2,2,2-trifluoroethyl)-1H-pyrazolo[4,3-c]pyridin-6-yl]-(3-hydroxy-8-aza-bicyclo[3.2.1]oct-8-yl)-methanone

Compound 136 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 8-azabicyclo[3.2.1]octan-3-ol B10. Purification by flash chromatography (DCM/MeOH: 100/0 to 95/5) afforded 136 as a white powder in 63% yield (mixture of *endolexo* isomers). ¹H-NMR (DMSO-*d_{6,}* 400MHz): 1.70 (d, *J* 14.3 Hz, 1H, CH₂); 1.80-1.96 (m, 3H, CH₂); 2.03-2.13 (m, 2H, CH₂); 2.18-2.32 (m, 2H, CH₂); 4.00 (m, 1H, N-CH); 4.46-4.51 (m, 1H, N-CH); 4.61 -4.70 (m, 2H, CH-OH); 5.78 (q, *J* 9.0 Hz, 2H, CF₃-CH₂); 7.80 (ddd, *J* 11.0, 9.2, 2.2 Hz, 1H, Ar); 8.31 (d, *J* 0.9 Hz, 1H, Ar); 8.86 (s, 1H, Ar); 9.48 (ddd, *J* 4.7, 2.2, 0.8 Hz, 1H, Ar); 9.66 (d, *J* 0.9 Hz, 1H, Ar). M/Z (M+H)⁺: 507

### Compound 137 : [3-(6,8-Difluoro-[1,2,4]triazolo[4,3-a]pyridin-3-yl)-1-(2,2,2-trifluoroethyl)-1H-pyrazolo[4,3-c]pyridin-6-yl]-1,4-oxazepan-4-yl-methanone

Compound 137 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 1,4-oxazepane B2. Purification by flash chromatography (DCM/MeOH: 100/0 to 95/5) afforded 137 as a white powder in 43% yield. ¹H-NMR (DMSO-*d_{6,}* 400MHz): 1.78 (quint, J5.9 Hz, 1H, CH₂); 1.95 (quint, *J* 5.9 Hz, 1H, CH₂); 3.47-3.55 (m, 2H, N-CH₂); 3.62-3.69 (m, 1H, CH₂); 3.70-3.83 (m, 5H, CH₂); 5.80 (q, *J* 8.9 Hz, 2H, CF₃-CH₂); 7.80 (ddd, *J* 11.1, 9.1, 2.3 Hz, 1H, Ar); 8.21-8.22 (m, 1H, Ar); 8.86 (s, 1H, signal of a rotamer, Ar); 8.87 (s, 1H, signal of a rotamer, Ar); 9.46-9.49 (m, 1H, Ar); 9.66 (d, *J* 1.0 Hz, 1H, signal of a rotamer, Ar); 9.67 (d, *J* 1.0 Hz, 1H, signal of a rotamer, Ar). M/Z (M+H)⁺: 481

### Compound 138 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-methyl-piperidin-1-yl)-methanone

Compound 138 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-methylpiperidin-4-ol B11. Purification by flash chromatography (DCM/MeOH: 100/0 to 95/5) afforded 138 as a white powder in 49% yield. ¹H-NMR (DMSO-*d_{6,}* 400MHz): 1.16-2.00 (m, 7H, CH₂); 2.92-3.02 (m, 1H, one diastereoisomer, CH₂); 3.10-3.23 (m, 1H, one diastereoisomer, CH₂); 3.38-3.52 (m, 1H, one diastereoisomer, CH₂); 3.80-3.97 (m, 1H, CH); 4.03-4.08 (m, 1H, one diastereoisomer, CH₂); 4.46-4.54 (m, 1H, one diastereoisomer, CH₂); 4.69-4.78 (m, 1H, OH); 4.92-5.01 (m, 1H, one diastereoisomer, CH₂); 5.75 (q, *J* 8.9 Hz, 2H, CF₃-CH₂); 7.80 (ddd, *J* 11.2, 9.3, 2.0 Hz, 1H, Ar); 8.14 (s, 1H, one diastereoisomer, Ar); 8.16 (s, 1H, one diastereoisomer, Ar); 8.86 (s, 1H, Ar); 9.45-9.50 (m, 1H, Ar); 9.63-9.67 (m, 1H, Ar). M/Z (M+H)⁺: 495

### Compound 139 : (3-Aza-bicyclo[3.2.1]oct-3-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone

Compound 139 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-azabicyclo[3.2.1]octane hydrochloride B4. In that specific case, 4 equiv. of diisopropylethylamine were used. Purification by flash chromatography (DCM/MeOH: 100/0 to 95/5) afforded 139 as a white powder in 42% yield. ¹H-NMR (DMSO-*d_{6,}* 400MHz): 1.49-1.72 (m, 6H, CH₂); 2.06-2.11 (m, 1H, CH₂); 2.29-2.36 (m, 1H, CH₂); 2.87 (bs, 1H, signal of a rotamer, N-CH₂); 2.90 (bs, 1H, signal of a rotamer, N-CH₂); 3.15 (bs, 1H, signal of a rotamer, N-CH₂); 2.18 (bs, 1H, signal of a rotamer, N-CH₂); 3.32-3.35 (m, 1H, N-CH₂); 4.33-4.39 (m, 1H, N-CH₂); 5.70-5.82 (m, 2H, CF₃-CH₂); 7.80 (ddd, *J* 11.1, 9.2, 2.0 Hz, 1H, Ar); 8.16 (d, *J* 0.9 Hz, 1H, Ar); 8.86 (s, 1H, Ar); 9.46-9.50 (m, 1H, Ar); 9.66 (d, *J* 0.9 Hz, 1H, Ar). M/Z (M+H)⁺: 491

### Compound 140 : [3-(6,8-Difluoro-imidazo[1,2-a]pyridin-3-yl)-1-(2,2,2-trifluoro-ethyl)-1H-pyrazolo[4,3-c]pyridin-6-yl]-(9-hydroxy-3-aza-bicyclo[3.3.1]non-3-yl)-methanone

Compound 140 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 9-azabicyclo[3.3.1]nonan-3-ol B12. In that specific case, 4 equiv. of diisopropylethylamine were used. Purification by flash chromatography (DCM/MeOH: 100/0 to 95/5) afforded 140 as a white powder in 23% yield. ¹H-NMR (DMSO-*d_{6,}* 400MHz): 1.26-1.70 (m, 7H, CH₂); 2.08-2.18 (m, 1H, CH); 2.19-2.29 (m, 2H, CH₂); 3.64-3.76 (m, 1H, CH); 3.86-3.94 (m, 1H, CH); 4.74 (d, J4.7 Hz, 1H, OH); 4.85-4.92 (m, 1H, CH); 5.76 (q, *J* 9.1 Hz, 2H, CF₃-CH₂); 7.81 (ddd, *J* 11.0, 9.1, 2.2 Hz, 1H, Ar); 8.16 (d, *J* 0.8 Hz, 1H, Ar); 8.86 (s, 1H, Ar); 9.47-9.50 (m, 1H, Ar); 9.66 (d, *J* 0.8 Hz, 1H, Ar). M/Z (M+H)⁺: 521

### Compound 141 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-isopropyl-piperidin-1-yl)-methanone

Compound 141 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-isopropylpiperidin-4-ol B13. Purification by flash chromatography (DCM/MeOH: 100/0 to 95/5) afforded 141 as a white powder in 70% yield. ¹H-NMR (DMSO-*d_{6,}* 400MHz): 0.70 (d, J6.6 Hz, 3H, minor rotamer, CH₃); 0.73 (d, *J* 6.6 Hz, 3H, minor rotamer, CH₃); 0.93 (d, *J* 6.6 Hz, 3H, major rotamer, CH₃); 1.02 (d, *J* 6.6 Hz, 3H, major rotamer, CH₃); 1.21-1.55 (m, 2H, CH₂); 1.67-1.76 (m, 2H, minor rotamer, CH₂); 1.89-2.17 (m, 3H, both rotamers, CH, major rotamer, CH₂); 2.75-2.85 (m, 1H, minor rotamer, N-CH₂); 3.01-3.13 (m, 1H, major rotamer, N-CH₂); 3.34-3.42 (m, 1H, N-CH₂); 3.74-3.87 (m, 1H, CH-OH); 4.38-4.56 (m, 1H, N-CH); 4.72 (d, *J* 4.5 Hz, 1H, major rotamer, OH); 4.75 (d, J4.5 Hz, 1H, minor rotamer, OH); 5.69-5.83 (m, 2H, CH₂-CF₃); 7.76-7.84 (m, 1H, Ar); 8.11 (bs, 1H, minor rotamer, Ar); 8.14 (bs, 1H, major rotamer, Ar); 8.86 (s, 1H, major rotamer, Ar); 8.88 (s, 1H, minor rotamer, Ar); 9.46-9.50 (m, 1H, Ar); 9.66 (d, *J* 0.9 Hz, 1H, minor rotamer, Ar); 9.67 (d, *J* 0.9 Hz, 1H, major rotamer, Ar). M/Z (M+H)⁺: 523

### Compound 142 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2,2-dimethyl-piperidin-1-yl)-methanone

Compound 142 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2,2-dimethylpiperidin-4-ol B14. Purification by flash chromatography (DCM/MeOH: 100/0 to 95/5) afforded 142 as a beige powder in 11% yield. ¹H-NMR (DMSO-*d_{6,}*400MHz): 1.36-1.45 (m, 1H, CH₂); 1.48 (s, 3H, CH₃); 1.56-1.68 (m, 4H, CH₃, CH₂); 1.75-1.83 (m, 1H, CH₂); 1.84-1.93 (m, 1H, CH₂); 2.99-3.10 (m, 1H, N-CH₂); 3.40-3.49 (m, 1H, N-CH₂); 3.80-3.90 (m, 1H, CH); 4.73 (d, *J* 4.0 Hz, 1H, OH); 5.74 (q, *J* 9.0 Hz, 2H, CH₂-CF₃); 7.80 (ddd, *J* 11.0, 9.2, 2.2 Hz, 1H, Ar); 8.12 (bs, 1H, Ar); 8.86 (s, 1H, Ar); 9.46-9.50 (m, 1H, Ar); 9.63 (bs, 1H, Ar). M/Z (M+H)⁺: 509

### Compound 143 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-3-isobutyl-piperidin-1-yl)-methanone

Compound 143 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-isobutylpiperidin-4-ol B15. Purification by flash chromatography (DCM/MeOH: 100/0 to 95/5) afforded 143 as a beige powder in 26% yield. ¹H-NMR (DMSO-*d_{6,}* 400MHz): 0.57-0.70 (m, 3H, CH₃); 0.78-0.89 (m, 1H, CH); 0.89-0.97 (m, 3H, CH₃); 1.05-1.93 (m, 5H, CH₂, CH); 2.73-2.85 (m, 1H, CH-OH); 3.05-3.18 (m, 1H, N-CH₂); 3.19-3.39 (m, 1H, N-CH₂); 3.53-3.63 (m, 1H, major rotamer, N-CH₂); 3.79-3.85 (m, 1H, minor rotamer, N-CH₂); 3.99-4.12 (m, 1H, minor rotamer, N-CH₂); 4.23-4.34 (m, 1H, major rotamer, N-CH₂); 4.66-4.70 (m, 1H, minor rotamer, OH); 4.75-4.80 (m, 1H, minor rotamer, OH); 5.70-5.81 (m, 2H, CH₂-CF₃); 7.77-7.85 (m, 1H, Ar); 8.16-8.22 (m, 1H, Ar); 8.87 (s, 1H, Ar); 9.46-9.50 (m, 1H, Ar); 9.63 (bs, 1H, one isomer, Ar); 9.66 (bs, 1H, one isomer, Ar). M/Z (M+H)⁺: 537

### Compound 144 : [3-(6,8-Difluoro-imidazo[1,2-a]pyridin-3-yl)-1-(2,2,2-trifluoro-ethyl)-1H-pyrazolo[4,3-c]pyridin-6-yl]-(4-fluoro-piperidin-1-yl)-methanone

Compound 144 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-fluoropiperidine hydrochloride B16. In that specific case, 4 equiv. of diisopropylethylamine were used. Purification by flash chromatography (DCM/MeOH: 100/0 to 95/5) afforded 144 as a white powder in 45% yield. ¹H-NMR (DMSO-*d₆*, 400MHz): 1.66-2.08 (m, 4H, CH₂); 3.34-3.43 (m, 1H, N-CH₂); 3.46-3.57 (m, 1H, N-CH₂); 3.69-3.84 (m, 2H, N-CH₂); 4.87-5.05 (m, 1H, CH-F); 5.76 (q, *J* 9.1 Hz, 2H, CF₃-CH₂); 7.80 (ddd, *J* 11.0, 9.0, 2.1 Hz, 1H, Ar); 8.20 (d, *J* 0.9 Hz, 1H, Ar); 8.87 (s, 1H, Ar); 9.46-9.50 (m, 1H, Ar); 9.67 (d, *J* 0.9 Hz, 1H, Ar). M/Z (M+H)⁺: 483

### Compound 145 : [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-2-aza-spiro[4.5]dec-2-yl)-methanone

Compound 145 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 8-oxa-2-azaspiro[4.5]decane B17 (1.2 equiv.). Purification by flash chromatography (DCM/MeOH: 100/0 to 95/5) afforded 145 as a white powder in 68% yield. ¹H-NMR (DMSO-*d_{6,}* 400MHz): 1.42-1.64 (m, 4H, CH₂); 1.80-1.90 (m, 2H, CH₂); 3.44-3.52 (m, 2H, CH₂); 3.53-3.70 (m, 5H, CH₂); 3.72-3.78 (m, 1H, CH₂); 5.79 (q, *J* 9.0 Hz, 2H, CF₃-CH₂); 7.76-7.85 (m, 1H, Ar); 8.35 (bs, 1H, signal of a rotamer, Ar); 8.36 (bs, 1H, signal of a rotamer, Ar); 8.87 (s, 1H, signal of a rotamer, Ar); 8.89 (s, 1H, signal of a rotamer, Ar); 9.46-9.50 (m, 1H, Ar); 9.67 (bs, 1H, signal of a rotamer, Ar), 9.70 (bs, 1H, signal of a rotamer, Ar). M/Z (M+H)⁺: 521

### Compound 146 : [3-(6,8-Difluoro-imidazo[1,2-a]pyridin-3-yl)-1-(2,2,2-trifluoro-ethyl)-1H-pyrazolo[4,3-c]pyridin-6-yl]-(4,4-difluoro-piperidin-1-yl)-methanone

Compound 146 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4,4-difluoropiperidine hydrochloride B18 (1.2 equiv.). In that specific case, 4 equiv. of diisopropylethylamine were used. Purification by flash chromatography (DCM/MeOH: 100/0 to 98/2) afforded 146 as a white powder in 70% yield. ¹H-NMR (DMSO-*d₆*,400MHz): 2.00-2.20 (m, 4H, CH₂); 3.50-3.60 (m, 2H, N-CH₂); 3.80-3.88 (m, 2H, N-CH₂); 5.76 (q, J9.0 Hz, 2H, CF₃-CH₂); 7.76-7.84 (m, 1H, Ar); 8.26 (bs, 1H, Ar); 8.88 (s, 1H, Ar); 9.46-9.50 (m, 1H, Ar); 9.68 (bs, 1H, Ar). M/Z (M+H)⁺: 501

### Compound 147 : [3-(6,8-Difluoro-imidazo[1,2-a]pyridin-3-yl)-1-(2,2,2-trifluoro-ethyl)-1H-pyrazolo[4,3-c]pyridin-6-yl]-(4'-hydroxy-3',4',5',6'-tetrahydro-2'H-[3,4']bipyridinyl-1'-yl)-methanone

Compound 147 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-(3-pyridyl)piperidin-4-ol B19 (1.2 equiv.). Purification by flash chromatography (DCM/MeOH: 100/0 to 9/1) afforded 147 as a white powder in 54% yield. ¹H-NMR (DMSO-*d₆*, 400MHz): 1.60-1.67 (m, 1H, CH₂); 1.80-1.88 (m, 1H, CH₂); 1.93-2.08 (m, 2H, CH₂); 3.22-3.31 (m, 1H, N-CH₂); 3.46-3.56 (m, 1H, N-CH₂); 3.58-3.65 (m, 1H, N-CH₂); 4.51-4.58 (m, 1H, N-CH₂); 5.44 (s, 1H, OH); 5.76 (q, *J* 9.0 Hz, 2H, CF₃-CH₂); 7.37 (ddd, *J* 8.0, 4.8, 0.6 Hz, 1H, Ar); 7.81 (ddd, *J* 11.0, 9.4, 2.2 Hz, 1H, Ar); 7.89 (ddd, *J* 8.0, 2.3, 1.8 Hz, 1H, Ar); 8.23 (bs, 1H, Ar); 8.47 (dd, *J* 4.8, 1.8 Hz, 1H, Ar); 8.73-8.76 (m, 1H, Ar); 8.87 (s, 1H, Ar); 9.46-9.50 (ddd, *J* 4.7, 2.4, 0.8 Hz, 1H, Ar); 9.68 (bs, 1H, Ar). M/Z (M+H)⁺: 558

### Compound 148 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-trifluoromethyl-piperidin-1-yl)-methanone

Compound 148 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-(trifluoromethyl)piperidin-4-ol B20 (1.2 equiv.). Purification by flash chromatography (DCM/MeOH: 100/0 to 95/5) afforded 148 as a white powder in 60% yield. ¹H-NMR (DMSO-*d_{6,}* 400MHz): 1.60-1.89 (m, 4H, CH₂); 3.03-3.14 (m, 1H, N-CH₂); 3.25-3.36 (m, 1H, N-CH₂); 3.64-3.73 (m, 1H, N-CH₂); 4.51-4.60 (m, 1H, N-CH₂); 5.71-5.80 (m, 2H, CH₂-CF₃); 6.18 (s, 1H, OH); 7.80 (ddd, *J* 11.1, 9.2, 2.1 Hz, 1H, Ar); 8.23 (bs, 1H, Ar); 8.87 (s, 1H, Ar); 9.46-9.50 (m, 1H, Ar); 9.68 (d, *J* 1.0 Hz, 1H, Ar). M/Z (M+H)⁺: 549

### Compound 149 : [3-(6,8-Difluoro-imidazo[1,2-a]pyridin-3-yl)-1-(2,2,2-trifluoro-ethyl)-1H-pyrazolo[4,3-c]pyridin-6-yl]-(2-methoxymethyl-pyrrolidin-1-yl)-methanone

Compound 149 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-(methoxymethyl)pyrrolidine B21 (1.2 equiv.). Purification by flash chromatography (DCM/MeOH: 100/0 to 95/5) afforded 149 as a white powder in 48% yield. ¹H-NMR (DMSO-*d_{6,}* 400MHz): 1.72-2.04 (m, 4H, CH₂); 2.99 (s, 3H, signal of a rotamer, CH₃); 3.03-3.10 (dd, *J* 9.8, 7.3 Hz, 1H, signal of a rotamer, CH₂); 3.19 (dd, *J* 9.8, 5.1 Hz, 1H, signal of a rotamer, CH₂); 3.33 (s, 3H, signal of a rotamer, CH₃); 3.45-3.60 (m, 3H, signal of a rotamer, CH₂); 3.62-3.72 (m, 3H, signal of a rotamer, CH₂); 4.31-4.38 (m, 1H, signal of a rotamer, CH₂); 4.68-4.75 (m, 1H, signal of a rotamer, CH₂); 5.70-5.86 (m, 2H, CF₃-CH₂); 7.80 (ddd, *J* 11.0, 9.2, 2.2 Hz, 1H, Ar); 8.31 (s, 1H, signal of a rotamer, Ar); 8.32 (s, 1H, signal of a rotamer, Ar); 8.86 (s, 1H, signal of a rotamer, Ar); 8.88 (s, 1H, signal of a rotamer, Ar); 9.46-9.50 (m, 1H, Ar); 9.65 (s, 1H, signal of a rotamer, Ar); 9.66 (s, 1H, signal of a rotamer, Ar). M/Z (M+H)⁺: 495

### Compound 150 : [3-(6,8-Difluoro-imidazo[1,2-a]pyridin-3-yl)-1-(2,2,2-trifluoro-ethyl)-1H-pyrazolo[4,3-c]pyridin-6-yl]-(4-methyl-1,4-diazepan-1-yl)-methanone

Compound 150 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-methyl-1,4-diazepane B23 (1.2 equiv.). Purification by flash chromatography (DCM/MeOH: 100/0 to 97/3) afforded 150 as a white powder in 35% yield. ¹H-NMR (DMSO-*d_{6,}* 400MHz): 1.73-1.82 (m, 1H, CH₂); 1.89-1.98 (m, 1H, CH₂); 2.25-2.37 (m, 3H, CH₃); 2.53-2.66 (m, 3H, N-CH₂); 2.70-2.76 (m, 1H, N-CH₂); 3.40-3.45 (m, 1H, N-CH₂); 3.45-3.50 (m, 1H, N-CH₂); 3.66-3.76 (m, 2H, N-CH₂); 5.76 (q, *J* 9.0 Hz, 2H, CH₂-CF₃); 7.80 (ddd, *J* 11.0, 9.2, 2.2 Hz, 1H, Ar); 8.19 (bs, 1H, Ar); 8.87 (s, 1H, Ar); 9.46-9.50 (m, 1H, Ar); 9.65-9.67 (m, 1H, Ar). M/Z (M+H)⁺: 494

Compound 151 : [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxy-piperidin-1-yl)-methanone Compound 151 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and piperidin-3-ol B24. Purification by flash chromatography (DCM/MeOH: 10/0 to 9/1) afforded 151 as a white powder in 34% yield. ¹H-NMR (DMSO-*d_{6,}* 400MHz): 1.37-1.51 (m, 2H, CH₂); 1.60-1.71 (m, 1H, signal of a rotamer, CH₂); 1.77-1.97 (m, 3H, signal of a rotamer, CH₂); 2.89 (dd, *J* 12.3, 9.0 Hz, 1H, signal of a rotamer, N-CH₂); 2.99-3.08 (m, 1H, N-CH₂); 3.21-3.28 (m, 1H, signal of a rotamer, N-CH₂); 3.42-3.62 (m, 2H, N-CH₂); 3.91-4.00 (m, 1H, signal of a rotamer, CH-OH); 4.30 (dd, *J* 12.4, 3.7 Hz, 1H, signal of a rotamer, CH-OH); 4.77 (d, *J* 3.7 Hz, 1H, signal of a rotamer, OH); 5.04 (d, J4.1 Hz, 1H, signal of a rotamer, OH); 5.71-5.81 (m, 2H, CF₃-CH₂); 7.76 (m, 1H, Ar); 8.16 (s, 1H, signal of a rotamer, Ar); 8.18 (s, 1H, signal of a rotamer, Ar); 8.86 (s, 1H, signal of a rotamer, Ar); 8.87 (s, 1H, signal of a rotamer, Ar); 9.45-9.51 (m, 1H, Ar); 9.66 (s, 1H, signal of a rotamer, Ar); 9.67 (s, 1H, signal of a rotamer, Ar). M/Z (M+H)⁺: 481

### Compound 152 : [3-(6,8-Difluoro-imidazo[1,2-a]pyridin-3-yl)-1-(2,2,2-trifluoro-ethyl)-1H-pyrazolo[4,3-c]pyridin-6-yl]-(7-oxa-2-aza-spiro[4.5]dec-2-yl)-methanone

Compound 152 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 7-oxa-2-azaspiro[4.5]decane B25 (1.2 equiv.). Purification by flash chromatography (DCM/MeOH: 100/0 to 95/5) afforded 152 as a white powder in 62% yield. ¹H-NMR (DMSO-*d₆*,400MHz): 1.32-1.72 (m, 5H, CH₂); 1.80-1.90 (m, 1H, CH₂); 3.30-3.57 (m, 4H, CH₂); 3.60-3.80 (m, 4H, CH₂); 5.75-5.85 (m, 2H, CH₂-CF₃); 7.80 (ddd, *J* 11.0, 9.2, 2.2 Hz, 1H, Ar); 8.36 (bs, 1H, Ar); 8.86 (s, 1H, one isomer, Ar); 8.89 (s, 1H, one isomer, Ar); 9.46-9.50 (m, 1H, Ar); 9.66 (d, *J* 1.0 Hz, 1H, one isomer, Ar); 9.69 (d, *J* 1.0 Hz, 1H, one isomer, Ar). M/Z (M+H)⁺: 521

### Compound 153 : [3-(6,8-Difluoro-imidazo[1,2-a]pyridin-3-yl)-1-(2,2,2-trifluoro-ethyl)-1H-pyrazolo[4,3-c]pyridin-6-yl]-(3-oxa-9-aza-spiro[5.5]undec-9-yl)-methanone

Compound 153 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-oxa-9-azaspiro[5.5]undecane B26 (1.2 equiv.). Purification by flash chromatography (DCM/MeOH: 100/0 to 93/7) afforded 153 as a white powder in 58% yield. ¹H-NMR (DMSO-*d*_{*6*,} 400MHz): 1.42-1.55 (m, 6H, CH₂); 1.56-1.62 (m, 2H, CH₂); 3.32-3.39 (m, 2H, CH₂); 3.50-3.63 (m, 4H, CH₂); 3.66-3.73 (m, 2H; CH₂); 5.76 (q, *J* 9.0 Hz, 2H, CH₂-CF₃); 7.80 (ddd, *J* 11.0, 9.2, 2.2 Hz, 1H, Ar); 8.17 (bs, 1H, Ar); 8.87 (s, 1H, Ar); 9.46-9.50 (m, 1H, Ar); 9.66 (d, *J* 1.0 Hz, 1H, Ar). M/Z (M+H)⁺: 535

### Compound 154 : [3-(6,8-Difluoro-imidazo[1,2-a]pyridin-3-yl)-1-(2,2,2-trifluoro-ethyl)-1H-pyrazolo[4,3-c]pyridin-6-yl]-(tetrahydro-furo[3,4-c]pyrrol-5-yl)-methanone

Compound 154 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and tetrahydro-furo[3,4-c]pyrrole B27 (1.2 equiv.). Purification by flash chromatography (DCM/MeOH: 100/0 to 95/5) afforded 154 as a white powder in 50% yield. ¹H-NMR (DMSO-*d*_{*6*,} 400MHz): 2.91-3.02 (m, 2H, CH₂); 3.47-3.52 (m, 1H, CH); 3.56-3.65 (m, 3H, CH+CH₂); 3.72-3.92 (m, 4H; N-CH₂); 5.79 (q, *J* 9.0 Hz, 2H, CF₃-CH₂); 7.80 (ddd, *J* 11.0, 9.2, 2.2 Hz, 1H, Ar); 8.34 (bs, 1H, Ar); 8.87 (s, 1H, Ar); 9.46-9.50 (m, 1H, Ar); 9.67 (d, *J* 1.0 Hz, 1H, Ar). M/Z (M+H)⁺: 493

### Compound 155 : 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-3-one

Compound 155 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2,8-diaza-spiro[4.5]decan-3-one B28 (1.2 equiv.). Purification by flash chromatography (DCM/MeOH: 10/0 to 9/1) afforded 155 as a white powder in 53% yield. ¹H-NMR (DMSO-*d*_{*6*,} 400MHz): 1.52-1.59 (m, 2H, CH₂); 1.62-1.70 (m, 2H, CH₂); 2.12 (d, *J* 16.5 Hz, 1H, CH₂); 2.18 (d, *J* 16.5 Hz, 1H, CH₂); 3.09 (d, *J* 9.6 Hz, 2H; CH₂); 3.14 (d, *J* 9.6 Hz, 2H; CH₂); 3.33-3.45 (m, 2H, N-CH₂); 3.55-3.65 (m, 1H, N-CH₂); 3.78-3.86 (m, 1H, N-CH₂); 5.70-5.82 (q, *J* 9.1 Hz 2H, CF₃-CH₂); 7.55 (s, 1H, NH); 7.80 (ddd, *J* 11.0, 9.2, 2.2 Hz, 1H, Ar); 8.18 (bs, 1H, Ar); 8.87 (s, 1H, Ar); 9.46-9.50 (m, 1H, Ar); 9.66 (d, *J* 1.0 Hz, 1H, Ar). M/Z (M+H)⁺: 534

### Compound 156 : 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-1-one

Compound 156 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2,8-diaza-spiro[4.5]decan-1-one B29 (1.2 equiv.). Purification by flash chromatography (DCM/MeOH: 10/0 to 9/1) afforded 156 as a beige powder in 55% yield. ¹H-NMR (DMSO-*d*_{*6*,} 400MHz): 1.30-1.37 (m, 1H, CH₂); 1.47-1.57 (m, 1H, CH₂); 1.65-1.78 (m, 2H, CH₂); 1.95-2.12 (m, 2H, CH₂); 3.11-3.26 (m, 4H, N-CH₂); 3.60-3.70 (m, 1H, N-CH₂); 4.34-4.42 (m, 1H, N-CH₂); 5.70-5.82 (m, 2H, CF₃-CH₂); 7.61 (s, 1H, NH); 7.80 (ddd, *J* 11.0, 9.2, 2.2 Hz, 1H, Ar); 8.20 (bs, 1H, Ar); 8.87 (s, 1H, Ar); 9.46-9.50 (m, 1H, Ar); 9.66 (d, *J* 1.0 Hz, 1H, Ar). M/Z (M+H)⁺: 534

### Compound 157 : [3-(6,8-Difluoro-imidazo[1,2-a]pyridin-3-yl)-1-(2,2,2-trifluoro-ethyl)-1H-pyrazolo[4,3-c]pyridin-6-yl]-[3-(2-methoxy-ethyl)-pyrrolidin-1-yl]-methanone

Compound 157 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-(2-methoxy-ethyl)-pyrrolidine B30 (1.2 equiv.). Purification by flash chromatography (DCM/MeOH: 100/0 to 95/5) afforded 157 as a white powder in 68% yield. ¹H-NMR (DMSO-*d*_{*6*,} 400MHz): 1.50-1.63 (m, 2H, CH₂); 1.63-1.73 (m, 1H, CH); 1.97-2.11 (m, 1H, CH₂); 2.16-2.30 (m, 1H, CH₂); 3.12-3.17 (m, 1H, one isomer, N-CH₂); 3.18 (s, 3H, one isomer, CH₃); 3.24 (s, 3H, one isomer, CH₃); 3.25-3.31 (m, 1H, one isomer, N-CH₂); 3.38-3.43 (m, 1H, N-CH₂); 3.44-3.54 (m, 1H, N-CH₂); 3.60-3.75 (m, 2H, CH₂); 3.79 (dd, *J* 11.4, 7.2 Hz, 1H, CH₂); 5.79 (q, *J* 9.0 Hz, 2H, CF₃-CH₂); 7.80 (ddd, *J* 11.0, 9.2, 2.2 Hz, 1H, Ar); 8.35 (s, 1H, Ar); 8.87 (s, 1H, one isomer, Ar); 8.87 (s, 1H, one isomer, Ar); 9.46-9.51 (m, 1H, Ar); 9.66 (dd, *J* 1.0 Hz, 1H, one isomer, Ar); 9.68 (dd, *J* 1.0 Hz, 1H, one isomer, Ar). M/Z (M+H)⁺: 509

### Compound 158 : [3-(6,8-Difluoro-imidazo[1,2-a]pyridin-3-yl)-1-(2,2,2-trifluoro-ethyl)-1H-pyrazolo[4,3-c]pyridin-6-yl]-morpholin-4-yl-methanone

Compound 158 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and morpholine B31. Purification by flash chromatography (DCM/MeOH: 100/0 to 95/5) afforded 158 as a white powder in 42% yield. ¹H-NMR (DMSO-*d*_{*6*,} 400MHz): 3.46-3.52 (m, 2H, N-CH₂); 3.55-3.62 (m, 2H, N-CH₂); 3.72 (bs, 4H, O-CH₂); 5.77 (q, *J* 9.0 Hz, 2H, CF₃-CH₂); 7.80 (ddd, *J* 11.0, 8.9, 2.0 Hz, 1H, Ar); 8.25 (d, *J* 0.8 Hz, 1H, Ar); 8.87 (s, 1H, Ar); 9.46-9.50 (m, 1H, Ar); 9.67 (d, *J* 0.8 Hz, 1H, Ar). M/Z (M+H)⁺: 467

### Compound 159 : [3-(6,8-Difluoro-imidazo[1,2-a]pyridin-3-yl)-1-(2,2,2-trifluoro-ethyl)-1H-pyrazolo[4,3-c]pyridin-6-yl]-(3,3-difluoro-piperidin-1-yl)-methanone

Compound 159 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3,3-difluoro-piperidine B32. Purification by flash chromatography (DCM/MeOH: 100/0 to 95/5) afforded 159 as a white powder in 42% yield. ¹H-NMR (DMSO-*d*_{*6*,} 400MHz): 1.67-1.87 (m, 2H, CH₂); 2.03-2.22 (m, 2H, CH₂); 3.44-3.52 (m, 1H, CH₂); 3.73-3.79 (m, 1H, CH₂); 3.88-3.99 (m, 1H, N-CH₂); 4.02-4.10 (m, 1H, N-CH₂); 5.74-5.84 (m, 2H, CF₃-CH₂); 7.81 (ddd, *J* 11.2, 9.2, 2.2 Hz, 1H, Ar); 8.24 (s, 1H, signal of a rotamer, Ar); 8.30 (s, 1H, signal of a rotamer, Ar); 8.87 (s, 1H, Ar); 9.45-9.50 (m, 1H, Ar); 9.67 (bs, 1H, signal of a rotamer, Ar); 9.68 (bs, 1H, signal of a rotamer, Ar). M/Z (M+H)⁺: 501

### Compound 160 : [3-(6,8-Difluoro-imidazo[1,2-a]pyridin-3-yl)-1-(2,2,2-trifluoro-ethyl)-1H-pyrazolo[4,3-c]pyridin-6-yl]-(2,6-dimethyl-morpholin-1-yl)-methanone

Compound 160 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2,6-dimethyl-morpholine B33. Purification by flash chromatography (DCM/MeOH: 100/0 to 95/5) afforded 160 as a white powder in 22% yield. ¹H-NMR (DMSO-*d_{6,}* 400MHz): 0.99 (d, *J* 6.4 Hz, 3H, major isomer, CH₃); 1.03 (d, *J* 6.2 Hz, 3H, minor isomer, CH₃); 1.19 (d, *J* 6.2 Hz, 3H, major isomer, CH₃); 1.22 (d, *J* 6.4 Hz, 3H, minor isomer, CH₃); 2.57 (dd, *J* 12.9, 10.7 Hz, 1H, major isomer, CH); 2.81 (dd, *J* 13.1, 10.7 Hz, 1H, major isomer, CH); 3.11-3.18 (m, 1H, minor isomer, CH₂); 3.46-3.79 (m, 3H, minor isomer, 3H, major isomer, CH₂); 3.88-3.96 (m, 1H, minor isomer, CH); 4.03-4.10 (m, 1H, minor isomer, CH); 4.43-4.49 (m, 1H, major isomer, CH); 5.77 (q, *J* 9.2 Hz, 2H, CF₃-CH₂); 7.80 (ddd, *J* 11.0, 9.1, 2.2 Hz, 1H, Ar); 8.23 (d, *J* 0.9 Hz, 1H, major isomer Ar); 8.24 (d, *J* 1.0 Hz, minor isomer Ar); 8.87 (s, 1H, Ar); 9.47-9.50 (m, 1H, Ar); 9.68 (d, *J* 1.0 Hz, 1H, Ar). M/Z (M+H)⁺: 495

### Compound 161 : [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxymethyl-piperidin-1-yl)-methanone

Compound 161 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-hydroxymethyl-piperidine B34. Purification by flash chromatography (DCM/MeOH: 100/0 to 95/5) afforded 161 as a white powder in 20% yield. ¹H-NMR (DMSO-*d_{6,}* 400MHz): 1.20-1.81 (m, 5H, CH, CH₂); 2.62-3.69 (m, 5H, CH₂); 4.35 (t, *J* 5.2 Hz, 1H, signal of a rotamer, OH); 4.36-4.57 (m, 1H, CH₂); 4.62 (t, *J* 5.2 Hz, 1H, signal of a rotamer, OH); 5.75 (q, *J* 9.0 Hz, 2H, CF₃-CH₂); 7.76 (ddd, *J* 11.0, 9.1, 2.0 Hz, 1H, Ar); 8.16 (bs, 1H, Ar); 8.87 (bs, 1H, Ar); 9.46-9.50 (m, 1H, Ar); 9.66 (bs, 1H, Ar). M/Z (M+H)⁺: 495

### Compound 162 : [3-(6,8-Difluoro-imidazo[1,2-a]pyridin-3-yl)-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone

Compound 162 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and *endo*-(8-*exo*-methyl)-3-azabicyclo[3.2.1]octan-8-ol B35 (1.2 equiv.). Purification by flash chromatography (DCM/MeOH: 100/0 to 93/7) afforded 162 as a white powder in 73% yield. ¹H-NMR (DMSO-*d_{6,}* 400MHz): 1.18 (s, 3H, CH₃); 1.45-1.82 (m, 6H, CH, CH₂); 3.07 (dd, *J* 12.0, 2.4 Hz, 1H, CH₂); 3.40 (d, *J* 12.0 Hz, 1H, CH₂); 3.67 (d, *J* 12.0 Hz, 1H, CH₂); 4.16 (dd, *J* 12.0, 2.4 Hz, 1H, CH₂); 4.87 (s, 1H, OH); 5.75 (q, *J* 9.0 Hz, 2H, CF₃-CH₂); 7.80 (ddd, *J* 11.0, 9.2, 2.2 Hz, 1H, Ar); 8.15 (bs, 1H, Ar); 8.86 (s, 1H, Ar); 9.46-9.50 (m, 1H, Ar); 9.66 (d, *J* 1.0 Hz, 1H, Ar). M/Z (M+H)⁺: 521

### Compound 163 : [3-(6,8-Difluoro-imidazo[1,2-a]pyridin-3-yl)-1-(2,2,2-trifluoro-ethyl)-1H-pyrazolo[4,3-c]pyridin-6-yl]-(2-trifluoromethyl-morpholin-4-yl)-methanone

Compound 163 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-trifluoromethyl-morpholine B36. Purification by flash chromatography (DCM/MeOH: 100/0 to 95/5) afforded 163 as a white powder in 46% yield. ¹H-NMR (DMSO-*d_{6,}* 400MHz, 80°C): 3.30-3.43 (m, 2H, O-CH₂); 3.75 (td, *J* 11.2, 2.8 Hz, 1H, CH-CF₃); 3.97-4.19 (m, 2H, N-CH₂); 4.18-4.44 (m, 2H, N-CH₂); 5.68 (q, *J* 9.0 Hz, 2H, CF₃-CH₂); 7.62 (ddd, *J* 11.0, 9.0, 2.1 Hz, 1H, Ar); 8.26 (bs, 1H, Ar); 8.76 (s, 1H, Ar); 9.39-9.44 (m, 1H, Ar); 9.60 (d, *J* 1.0 Hz, 1H, Ar). M/Z (M+H)⁺: 535

### Compound 164 : [3-(6,8-Difluoro-imidazo[1,2-a]pyridin-3-yl)-1-(2,2,2-trifluoro-ethyl)-1H-pyrazolo[4,3-c]pyridin-6-yl]-(4-trifluoromethyl-piperidin-1-yl)-methanone

Compound 164 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-trifluoromethyl-piperidine hydrochloride B37. In that specific case, 4 equiv. of diisopropylethylamine were used. Purification by flash chromatography (DCM/MeOH: 10/0 to 9/1) afforded 164 as a grey powder in 28% yield. ¹H-NMR (DMSO-*d_{6,}* 400MHz): 1.40-1.56 (m, 2H, CH₂); 1.75-1.83 (m, 1H, CH₂); 1.94-2.04 (m, 1H, CH₂); 2.65-2.78 (m, 1H, CH); 2.91 (td, *J* 13.0, 2.4 Hz, 1H, CH₂); 3.09-3.20 (m, 1H, CH₂); 3.74-3.83 (m, 1H, CH₂); 4.62-4.71 (m, 1H, CH₂); 5.72-5.81 (m, 2H, CF₃-CH₂); 7.81 (ddd, *J* 11.1, 9.1, 2.2 Hz, 1H, Ar); 8.22 (d, *J* 1.0 Hz, 1H, Ar); 8.87 (s, 1H, Ar); 9.47-9.50 (m, 1H, Ar); 9.68 (d, *J* 1.0 Hz, 1H, Ar). M/Z (M+H)⁺: 533

### Compound 165 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-piperidin-1-yl)-methanone

Compound 165 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-hydroxymethyl-piperidine B38. Purification by flash chromatography (DCM/MeOH: 10/0 to 9/1) afforded 165 as a white powder in 38% yield. ¹H-NMR (DMSO-*d_{6,}* 400MHz): 1.08-1.18 (m, 2H, CH₂); 1.57-1.75 (m, 2H, CH₂); 1.77-1.86 (m, 1H, CH₂); 2.84 (td, *J* 12.7, 2.4 Hz, 1H, CH₂); 3.04 (td, *J* 12.7, 2.4 Hz, 1H, CH₂); 3.27-3.30 (m, 2H, CH₂); 3.64-3.67 (m, 1H, CH₂); 4.54 (t, *J* 5.3 Hz, 1H, OH); 4.53-4.60 (m, 1H, CH₂); 5.76 (q, *J* 9.3 Hz, 2H, CF₃-CH₂); 7.80 (ddd, *J* 11.2, 9.2, 2.0 Hz, 1H, Ar); 8.16 (bs, 1H, Ar); 8.87 (s, 1H, Ar); 9.46-9.50 (m, 1H, Ar); 9.66 (d, *J* 1.0 Hz, 1H, Ar). M/Z (M+H)⁺: 495

### Compound 166 : [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-7-aza-spiro[3.5]non-7-yl)-methanone

Compound 166 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-oxa-7-aza-spiro[3.5]nonane oxalate B39. In that specific case, 4 equiv. of diisopropylethylamine were used. Purification by flash chromatography (DCM/MeOH: 10/0 to 9/1) afforded 166 as a white powder in 38% yield. ¹H-NMR (DMSO-*d_{6,}* 400MHz): 1.80 (t, *J* 5.3 Hz, 2H, CH₂); 1.89 (t, *J* 5.3 Hz, 2H, CH₂); 3.27-3.30 (m, 2H, N-CH₂); 3.60-3.65 (m, 2H, N-CH₂); 4.33 (d, *J* 5.8 Hz, 2H, O-CH₂); 4.38 (d, *J* 5.8 Hz, 2H, O-CH₂); 5.75 (q, *J* 9.1 Hz, 2H, CF₃-CH₂); 7.81 (ddd, *J* 11.2, 9.1, 2.0 Hz, 1H, Ar); 8.17 (d, *J* 0.9 Hz, 1H, Ar); 8.87 (s, 1H, Ar); 9.46-9.50 (m, 1H, Ar); 9.66 (d, *J* 0.9 Hz, 1H, Ar). M/Z (M+H)⁺: 507

### Compound 167 : [3-(6,8-Difluoro-imidazo[1,2-a]pyridin-3-yl)-1-(2,2,2-trifluoro-ethyl)-1H-pyrazolo[4,3-c]pyridin-6-yl]-(3-methoxymethyl-morpholin-4-yl)-methanone

Compound 167 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-methoxymethyl-morpholine hydrochloride B40. In that specific case, 4 equiv. of diisopropylethylamine were used. Purification by flash chromatography (DCM/MeOH: 100/0 to 95/5) afforded 167 as a white powder in 2% yield. ¹H-NMR (DMSO-*d*_{*6*,} 400MHz): 3.06 (s, 3H, signal of a rotamer, CH₃); 3.10-3.23 (m, 1H, CH₂); 3.35-3.80 (m, 8H, CH₂, CH₃); 3.91-4.02 (m, 1H, CH₂); 4.04-4.12 (m, 1H, signal of a rotamer, CH₂); 4.15-4.24 (m, 1H, signal of a rotamer, CH₂); 4.60-4.69 (m, 1H, signal of a rotamer, CH₂); 5.70-5.88 (m, 2H, CF₃-CH₂); 7.81 (ddd, *J* 10.7, 9.3, 2.3 Hz, 1H, Ar); 8.22 (bs, 1H, Ar); 8.87 (s, 1H, Ar); 9.45-9.51 (m, 1H, Ar); 9.63-9.69 (m, 1H, Ar). M/Z (M+H)⁺: 511

### Compound 168 : [3-(6,8-Difluoro-imidazo[1,2-a]pyridin-3-yl)-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone

Compound 168 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and nortropine B41. Purification by flash chromatography (DCM/MeOH: 10/0 to 9/1) afforded 168 as a white powder in 52% yield. ¹H-NMR (DMSO-*d*_{*6*,} 400MHz): 1.70 (d, *J* 13.9 Hz, 1H, CH₂); 1.80-1.95 (m, 3H, CH₂); 2.03-2.12 (m, 2H, CH₂); 2.22-2.35 (m, 2H, CH₂); 3.97-4.03 (m, 1H, CH-OH); 4.47-4.52 (m, 1H, N-CH₂); 4.65 (d, *J* 2.4 Hz, 1H, OH); 4.66-4.72 (m, 1H, N-CH₂); 5.78 (q, *J* 9.0 Hz, 2H, CF₃-CH₂); 7.80 (ddd, *J* 11.0, 9.2, 2.2 Hz, 1H, Ar); 8.30 (d, *J* 0.8 Hz, 1H, Ar); 8.86 (s, 1H, Ar); 9.46-9.50 (m, 1H, Ar); 9.66 (d, *J* 0.8 Hz, 1H, Ar). M/Z (M+H)⁺: 507

### Compound 169 : (4-Aza-tricyclo[4.3.1.1*3,8*]undec-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone

Compound 169 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-Aza-tricyclo[4.3.1.1*3,8*]undecane hydrochloride B42. In that specific case, 4 equiv. of diisopropylethylamine were used. Purification by flash chromatography (DCM/MeOH: 100/0 to 95/5) afforded 169 as a white powder in 23% yield. ¹H-NMR (DMSO-*d*_{*6*,} 400MHz): 1.38-2.13 (m, 12H, CH₂); 2.06-2.12 (m, 1H, minor isomer, CH); 2.34-2.40 (m, 1H, major isomer, CH); 3.42 (d, *J* 3.7 Hz, 2H, minor isomer, N-CH₂); 3.69 (d, *J* 3.7 Hz, 2H, major isomer, N-CH₂); 3.95-4.00 (m, 1H, major isomer, N-CH); 4.91-4.99 (m, 1H, minor isomer, N-CH); 5.68-5.80 (m, 2H, CF₃-CH₂); 7.81 (ddd, *J* 11.0, 9.3, 1.8 Hz, 1H, Ar); 8.10 (d, J0.4 Hz, 1H, major isomer, Ar); 8.13 (d, *J* 0.4 Hz, 1H, minor isomer, Ar); 8.86 (s, 1H, minor isomer, Ar); 8.87 (s, 1H, major isomer, Ar); 9.46-9.50 (m, 1H, Ar); 9.65 (bs, 1H, Ar). M/Z (M+H)⁺: 531

### Compound 170 : [3-(6,8-Difluoro-imidazo[1,2-a]pyridin-3-yl)-1-(2,2,2-trifluoro-ethyl)-1H-pyrazolo[4,3-c]pyridin-6-yl]-(8-exo-hydroxy-3-aza-bicyclo[3.2.1]oct-3-yl)-methanone

Compound 170 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and *exo*-3-aza-bicyclo[3.2.1]octan-8-ol hydrochloride B43. In that specific case, 4 equiv. of diisopropylethylamine were used. Purification by flash chromatography (DCM/MeOH: 100/0 to 95/5) afforded 170 as a white powder in 55% yield. ¹H-NMR (DMSO-*d*_{*6*,} 400MHz): 1.39-1.56 (m, 2H, CH₂); 1.69-1.87 (m, 2H, CH₂); 1.87-1.97 (m, 1H, CH₂); 2.12-2.18 (m, 1H, CH₂); 2.89 (d, *J* 12.9 Hz, 1H, N-CH₂); 3.14-3.21 (m, 1H, N-CH₂); 3.31-3.38 (m, 1H, N-CH₂); 3.87 (bs, 1H, CH); 4.35-4.42 (m, 1H, N-CH₂); 4.74 (d, *J* 2.7 Hz, 1H, OH); 5.69-5.83 (m, 2H, CF₃-CH₂); 7.80 (ddd, *J* 11.0, 9.0, 2.0 Hz, 1H, Ar); 8.17 (d, *J* 0.9 Hz, 1H, Ar); 8.86 (s, 1H, Ar); 9.46-9.51 (m, 1H, Ar); 9.65 (d, *J* 0.9 Hz, 1H, Ar). M/Z (M+H)⁺: 507

### Compound 171 : [3-(6,8-Difluoro-imidazo[1,2-a]pyridin-3-yl)-1-(2,2,2-trifluoro-ethyl)-1H-pyrazolo[4,3-c]pyridin-6-yl]-(3-exo-hydroxy-8-aza-bicyclo[3.2.1]oct-8-yl)-methanone

Compound 171 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-*exo*-hydroxy-8-aza-bicyclo[3.2.1]octane hydrochloride B44. In that specific case, 4 equiv. of diisopropylethylamine were used. Purification by flash chromatography (DCM/MeOH: 100/0 to 95/5) afforded 171 as a white powder in 32% yield. ¹H-NMR (DMSO-*d_{6,}* 400MHz): 1.51-1.64 (m, 2H, CH₂); 1.69-2.01 (m, 6H, CH₂); 3.96-4.07 (m, 1H, CH-OH); 4.54-4.60 (m, 1H, N-CH); 4.63 (d, *J* 5.4 Hz, 1H, OH); 4.69-4.74 (m, 1H, N-CH); 5.80 (q, *J* 9.0 Hz, 2H, CF₃-CH₂); 7.80 (ddd, *J* 11.1, 8.7, 1.9 Hz, 1H, Ar); 8.33 (d, *J* 0.8 Hz, 1H, Ar); 8.87 (s, 1H, Ar); 9.46-9.51 (m, 1H, Ar); 9.67 (d, *J* 0.8 Hz, 1H, Ar). M/Z (M+H)⁺: 507

### Compound 172 : (8-Aza-bicyclo[3.2.1]oct-8-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone

Compound 172 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 8-aza-bicyclo[3.2.1]octane hydrochloride B45. In that specific case, 4 equiv. of diisopropylethylamine were used. Purification by flash chromatography (DCM/MeOH: 100/0 to 95/5) afforded 172 as a white powder in 30% yield. ¹H-NMR (DMSO-*d*_{*6*,} 400MHz): 1.40-1.47 (m, 1H, CH₂); 1.55-1.64 (m, 2H, CH₂); 1.71-1.87 (m, 5H, CH₂); 1.92-2.00 (m, 2H, CH₂); 4.45-4.52 (m, 1H, N-CH); 4.66-4.75 (m, 1H, N-CH); 5.79 (q, *J* 9.3 Hz, 2H, CF₃-CH₂); 7.80 (ddd, *J* 11.1, 9.2, 2.1 Hz, 1H, Ar); 8.31 (d, *J* 0.9 Hz, 1H, Ar); 8.86 (s, 1H, Ar); 9.46-9.50 (m, 1H, Ar); 9.66 (d, *J* 0.9 Hz, 1H, Ar). M/Z (M+H)⁺: 491

### Compound 173 : [3-(6,8-Difluoro-imidazo[1,2-a]pyridin-3-yl)-1-(2,2,2-trifluoro-ethyl)-1H-pyrazolo[4,3-c]pyridin-6-yl]-(4-difluoromethyl-piperidin-1-yl)-methanone

Compound 173 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 4-difluoromethyl-piperidine hydrochloride B46. In that specific case, 4 equiv. of diisopropylethylamine were used. Purification by flash chromatography (DCM/MeOH: 100/0 to 95/5) afforded 173 as a beige powder in 39% yield. ¹H-NMR (DMSO-*d_{6,}* 400MHz): 1.31-1.46 (m, 2H, CH₂); 1.60-1.70 (m, 1H, CH₂); 1.80-1.91 (m, 1H, CH₂); 2.08-2.26 (m, 1H, CH₂); 2.88 (td, *J* 13.0, 2.6 Hz, 1H, N-CH₂); 3.1 (td, *J* 13.0, 2.6 Hz, 1H, N-CH₂); 3.69-3.79 (m, 1H, N-CH₂); 4.58-4.67 (m, 1H, N-CH₂); 5.76 (q, *J* 9.2 Hz, 2H, CF₃-CH₂); 5.98 (td, *J* 56.6, 4.7 Hz, CH-CF₂); 7.80 (ddd, *J* 11.1, 9.0, 2.2 Hz, 1H, Ar); 8.19 (d, *J* 0.9 Hz, 1H, Ar); 8.86 (s, 1H, Ar); 9.46-9.50 (m, 1H, Ar); 9.66 (d, *J* 0.9 Hz, 1H, Ar). M/Z (M+H)⁺: 515

### Compound 174 : 3-(6,8-Difluoro-imidazo[1,2-a]pyridin-3-yl)-1-(2,2,2-trifluoro-ethyl)-1H-pyrazolo[4,3-c]pyridine-6-carboxylic acid (2-hydroxy-2-methyl-propyl)-amide

Compound 174 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 1-amino-2-methyl-2-propanol B47. Purification by flash chromatography (DCM/MeOH: 10/0 to 9/1) afforded 174 as a white powder in 63% yield. ¹H-NMR (DMSO-*d*_{*6*,} 400MHz): 1.15 (s, 6H, CH₃); 3.37 (d, J6.1 Hz, 2H, NH-CH₂); 4.77 (s, 1H, OH); 5.87 (q, J9.0 Hz, 2H, CF₃-CH₂); 7.80 (ddd, *J* 11.2, 9.1, 2.0 Hz, 1H, Ar); 8.68 (d, *J* 0.8 Hz, 1H, Ar); 8.70 (t, *J* 6.1 Hz, 1H, NH); 8.87 (s, 1H, Ar); 9.44-9.48 (m, 1H, Ar); 9.68 (d, *J* 0.8 Hz, 1H, Ar). M/Z (M+H)⁺: 469

### Compound 175 : [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-6-aza-tricyclo[3.3.1.1*3,7*]dec-6-yl)-methanone

Compound 175 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-oxa-6-aza-tricyclo[3.3.1.1*3,7*]decane B48. Purification by flash chromatography (DCM/MeOH: 100/0 to 95/5) afforded 175 as a white powder in 42% yield. ¹H-NMR (DMSO-*d*_{*6*,} 400MHz): 1.79-1.94 (m, 6H, CH₂); 2.06-2.14 (m, 2H, CH₂); 4.05-4.10 (m, 1H, N-CH); 4.13-4.18 (m, 2H, O-CH); 4.97-5.02 (m, 1H, N-CH); 5.76 (q, *J* 9.0 Hz, 2H, CF₃-CH₂); 7.81 (ddd, *J* 11.2, 9.1, 2.0 Hz, 1H, Ar); 8.22 (d, *J* 0.9 Hz, 1H, Ar); 8.87 (s, 1H, Ar); 9.46-9.50 (m, 1H, Ar); 9.67 (d, *J* 0.9 Hz, 1H, Ar). M/Z (M+H)⁺: 519

### Compound 176 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-4-methyl-piperidin-1-yl)-methanone

Compound 176 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and (4-methyl-4-piperidyl)methanol B49 (1.2 equiv.). Purification by flash chromatography (DCM/MeOH: 100/0 to 95/5), then by preparative HPLC afforded 176 as a white powder in 64% yield. ¹H-NMR (DMSO-*d_{6,}* 400MHz): 0.94 (s, 3H, CH₃); 1.14-1.22 (m, 1H, CH₂); 1.28-1.37 (m, 1H, CH₂); 1.42-1.60 (m, 2H, CH₂); 3.21 (d, *J* 5.4 Hz, 2H, CH₂-OH); 3.22-3.29 (m, 1H, N-CH₂); 3.32-3.47 (m, 2H, N-CH₂); 3.98-4.06 (m, 1H, N-CH₂); 4.59 (t, *J* 5.4 Hz, 1H, OH); 5.77 (q, *J* 9.0 Hz, 2H, CH₂-CF₃); 7.98 (ddd, *J* 11.0, 9.2, 2.2 Hz, 1H, Ar); 8.16 (bs, 1H, Ar); 8.86 (s, 1H, Ar); 9.46-9.49 (m, 1H, Ar); 9.65 (d, *J* 1.0 Hz, 1H, Ar). M/Z (M+H)⁺: 509

### Compound 177 : [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-azepan-1-yl)-methanone

Compound 177 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and azepan-4-ol B50. Purification by flash chromatography (DCM/MeOH: 100/0 to 94/6), then by preparative HPLC afforded 177 as a white powder in 31% yield. ¹H-NMR (DMSO-*d*_{*6*,} 400MHz): 1.46-1.83 (m, 5H, CH₂); 1.90-2.04 (m, 1H, CH₂); 3.22-3.31 (m, 1H, N-CH₂); 3.50-3.82 (m, 5H, N-CH₂, OH); 5.75 (q, *J9.0* Hz, 2H, CF₃-CH₂); 7.81 (ddd, *J* 10.9, 9.2, 2.0 Hz, 1H, Ar); 8.15 (d, *J* 1.0 Hz, 1H, signal of a rotamer, Ar); 8.17 (d, *J* 1.0 Hz, 1H, signal of a rotamer, Ar); 8.86 (s, 1H, signal of a rotamer, Ar); 8.87 (s, 1H, signal of a rotamer, Ar); 9.46-9.50 (m, 1H, Ar); 9.66 (d, *J* 1.0 Hz, 1H, Ar). M/Z (M+H)⁺: 495

### Compound 178 : (2-Aza-tricyclo[3.3.1.1*3,7*]dec-2-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone

Compound 178 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-aza-adamantane hydrochloride B51. In that specific case, 4 equiv. of diisopropylethylamine were used. Purification by flash chromatography (DCM/MeOH: 100/0 to 95/5), then by preparative HPLC afforded 178 as a beige powder in 16% yield. ¹H-NMR (DMSO-*d_{6,}* 400MHz): 1.62-1.70 (m, 2H, CH₂); 1.78-1.93 (m, 8H, CH₂); 2.07-2.13 (m, 2H, CH₂); 3.79-3.85 (m, 1H, N-CH₂); 4.79-4.83 (m, 1H, N-CH₂); 5.75 (q, *J* 8.9 Hz, 2H, CF₃-CH₂); 7.79 (ddd, *J* 11.1, 9.1, 2.1 Hz, 1H, Ar); 8.17 (d, *J*0.9 Hz, 1H, Ar); 8.86 (s, 1H, Ar); 9.46-9.49 (m, 1H, Ar); 9.65 (d, *J* 0.9 Hz, 1H, Ar). M/Z (M+H)⁺: 517

### Compound 179 : [3-(6,8-Difluoro-imidazo[1,2-a]pyridin-3-yl)-1-(2,2,2-trifluoro-ethyl)-1H-pyrazolo[4,3-c]pyridin-6-yl]-(5-oxa-8-aza-spiro[2.6]non-8-yl)-methanone

Compound 179 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 5-oxa-8-aza-spiro[2.6]nonane B52. Purification by flash chromatography (DCM/MeOH: 100/0 to 95/5) afforded 179 as a white powder in 52% yield. ¹H-NMR (DMSO-*d_{6,}* 400MHz): 0.26 (dd, *J* 6.0, 4.5 Hz, 1H, CH₂); 0.44 (dd, *J* 6.0, 4.5 Hz, 1H, CH₂); 0.59 (dd, *J* 6.0, 4.5 Hz, 1H, CH₂); 0.70 (dd, *J* 6.0, 4.5 Hz, 1H, CH₂); 3.44 (bs, 2H, CH₂); 3.52 (bs, 1H, CH₂); 3.58-3.63 (m, 1H, CH₂); 3.65 (bs, 1H, CH₂); 3.72-3.78 (m, 1H, CH₂); 3.83-3.90 (m, 2H, CH₂); 5.76 (q, *J* 9.1 Hz, 2H, CF₃-CH₂); 7.80 (ddd, *J* 11.1, 9.2, 2.3 Hz, 1H, Ar); 8.20 (d, *J* 1.0 Hz, 1H, signal of a rotamer, Ar); 8.23 (d, *J* 1.0 Hz, 1H, signal of a rotamer, Ar); 8.86 (s, 1H, signal of a rotamer, Ar); 8.87 (s, 1H, signal of a rotamer, Ar); 9.46-9.49 (m, 1H, Ar); 9.64 (d, *J* 1.0 Hz, 1H, signal of a rotamer, Ar); 9.66 (d, *J* 1.0 Hz, 1H, signal of a rotamer, Ar). M/Z (M+H)⁺: 507

### Compound 180 : [3-(6,8-Difluoro-imidazo[1,2-a]pyridin-3-yl)-1-(2,2,2-trifluoro-ethyl)-1H-pyrazolo[4,3-c]pyridin-6-yl]-1,4-thiazepan-1,1-dioxide-4-yl-methanone

Compound 180 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 1,4-thiazepan-1,1-dioxide hydrochloride B53. In that specific case, 4 equiv. of diisopropylethylamine were used. Purification by flash chromatography (DCM/MeOH: 100/0 to 95/5) afforded 180 as a white powder in 36% yield. ¹H-NMR (DMSO-*d_{6,}* 400MHz): 1.99-2.15 (m, 2H, CH₂); 3.35-3.41 (m, 2H, CH₂); 3.47-3.54 (m, 2H, CH₂); 3.60 (t, *J* 6.1 Hz, 1H, CH₂); 3.67-3.73 (m, 1H, CH₂); 3.83 (t, *J* 6.1 Hz, 1H, CH₂); 3.88-3.96 (m, 1H, CH₂); 5.69-5.84 (m, 2H, CF₃-CH₂); 7.81 (ddd, *J* 10.8, 9.2, 1.8 Hz, 1H, Ar); 8.27 (bs, 1H, signal of a rotamer, Ar); 8.28 (bs, 1H, signal of a rotamer, Ar); 8.88 (s, 1H, Ar); 9.47-9.50 (m, 1H, Ar); 9.68 (d, *J* 1.0 Hz, 1H, signal of a rotamer, Ar); 9.69 (d, *J* 1.0 Hz, 1H, signal of a rotamer, Ar). M/Z (M+H)⁺: 529

### Compound 181 : Cis-[3-(6,8-Difluoro-imidazo[1,2-a]pyridin-3-yl)-1-(2,2,2-trifluoroethyl)-1H-pyrazolo[4,3-c]pyridin-6-yl]-(2,6-dimethyl-morpholin-4-yl)-methanone

Compound 181 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and cis-[2,6-dimethyl-morpholine B54. Purification by flash chromatography (Cyclohexane/AcOEt: 10/0 to 3/7) afforded 181 as a white powder in 73% yield. ¹H-NMR (DMSO-*d*_{*6*,} 400MHz): 0.99 (d, *J* 6.1 Hz, 3H, CH₃); 1.19 (d, *J* 6.1 Hz, 3H, CH₃); 2.50-2.61 (m, 1H, N-CH₂); 2.77-2.86 (m, 1H, N-CH₂); 3.54-3.64 (m, 2H, O-CH); 3.69 (d, *J* 13.2 Hz, 1H, N-CH₂); 4.46 (d, *J* 12.7 Hz, 1H, N-CH₂); 5.77 (q, *J* 9.3 Hz, 2H, CF₃-CH₂); 7.76-7.85 (m, 1H, Ar); 8.24 (s, 1H, Ar); 8.88 (s, 1H, Ar); 9.47-9.50 (m, 1H, Ar); 9.68 (s, 1H, Ar). M/Z (M+H)⁺: 495

### Compound 1892 : [3-(6,8-Difluoro-imidazo[1,2-a]pyridin-3-yl)-1-(2,2,2-trifluoro-ethyl)-1H-pyrazolo[4,3-c]pyridin-6-yl]-(2,2-dimethyl-1,4-oxazepan-4-yl)-methanone

Compound 182 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2,2-dimethyl-1,4-oxazepane B55. Purification by flash chromatography (Cyclohexane/AcOEt: 10/0 to 2/8) afforded 182 as a white powder in 60% yield. ¹H-NMR (DMSO-*d*_{*6*,} 400MHz): 0.87-1.37 (m, 6H, CH₃); 1.77-1.90 (m, 2H, CH₂); 3.37-3.49 (m, 2H, CH₂); 3.72-3.79 (m, 4H, CH₂); 5.69 (q, *J* 8.9 Hz, 2H, CH₂-CF₃); 7.59-7.68 (m, 1H, Ar); 8.10-8.19 (m, 1H, Ar); 8.76 (bs, 1H, Ar); 9.41-9.44 (m, 1H, Ar); 9.59 (bs, 1H, Ar). M/Z (M+H)⁺: 509

### Compound 183 : Cis-[3-(6,8-Difluoro-imidazo[1,2-a]pyridin-3-yl)-1-(2,2,2-trifluoroethyl)-1H-pyrazolo[4,3-c]pyridin-6-yl]-(3,5-dimethyl-morpholin-4-yl)-methanone

Compound 183 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and cis-3,5-dimethyl-morpholine hydrochloride B56 (1.2 equiv). In that specific case, 4 equiv. of diisopropylethylamine were used. Purification by flash chromatography (DCM/MeOH: 100/0 to 95/5) afforded 183 as a white powder in 71% yield. ¹H-NMR (DMSO-*d*_{*6*,} 400MHz): 1.04-1.39 (m, 6H, CH₃); 3.44-3.52 (m, 2H, CH₂); 3.82-3.92 (m, 2H, CH₂); 3.95-4.05 (m, 2H; CH₂); 5.77 (q, *J* 9.0 Hz, 2H, CH₂-CF₃); 7.80 (ddd, *J* 11.0, 9.2, 2.2 Hz, 1H, Ar); 8.24 (s, 1H, Ar); 8.86 (s, 1H, Ar); 9.46-9.50 (m, 1H, Ar); 9.66 (d, *J* 1.0 Hz, 1H, Ar). M/Z (M+H)⁺: 495

### Compound 184 : [3-(6,8-Difluoro-imidazo[1,2-a]pyridin-3-yl)-1-(2,2,2-trifluoro-ethyl)-1H-pyrazolo[4,3-c]pyridin-6-yl]-(3-oxa-8-aza-bicyclo[3.2.1]oct-8-yl)-methanone

Compound 184 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-oxa-8-aza-bicyclo[3.2.1]octane hydrochloride B57 (1.2 equiv.). In that specific case, 4 equiv. of diisopropylethylamine were used. Purification by flash chromatography (DCM/MeOH: 100/0 to 95/5) afforded 184 as a white powder in 58% yield. ¹H-NMR (DMSO-*d_{6,}* 400MHz): 1.88-2.01 (m, 4H, CH₂); 3.56-3.62 (m, 1H, N-CH); 3.68-3.74 (m, 3H, N-CH, O-CH₂); 4.62-4.70 (m, 2H, O-CH₂); 5.77 (q, *J* 9.0 Hz, 2H, CH₂-CF₃); 7.80 (ddd, *J* 11.0, 9.2, 2.2 Hz, 1H, Ar); 8.41 (bs, 1H, Ar); 8.87 (s, 1H, Ar); 9.46-9.50 (m, 1H, Ar); 9.68 (d, *J* 1.0 Hz, 1H, Ar). M/Z (M+H)⁺: 493

### Compound 185 : [3-(6,8-Difluoro-imidazo[1,2-a]pyridin-3-yl)-1-(2,2,2-trifluoro-ethyl)-1H-pyrazolo[4,3-c]pyridin-6-yl]-(6,6-difluoro-1,4-oxazepan-4-yl)-methanone

Compound 185 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 6,6-difluoro-1,4-oxazepane hydrochloride B58 (1.2 equiv). In that specific case, 4 equiv. of diisopropylethylamine were used. Purification by flash chromatography (DCM/MeOH: 100/0 to 97/3) afforded 185 as a white powder in 54% yield. ¹H-NMR (DMSO-*d*_{*6*,} 400MHz): 3.60-3.67 (m, 1H, CH₂); 3.84-3.97 (m, 4H, CH₂); 3.98-4.08 (m, 1H, CH₂); 4.25-4.42 (m, 2H, CH₂); 5.72-5.84 (m, 2H, CH₂-CF₃); 7.80 (ddd, *J* 11.0, 9.2, 2.2 Hz, 1H, Ar); 8.32 (bs, 1H, signal of a rotamer, Ar); 8.33 (bs, 1H, signal of a rotamer, Ar); 8.87 (bs, 1H, signal of a rotamer, Ar); 8.88 (bs, 1H, signal of a rotamer, Ar); 9.46-9.50 (m, 1H, Ar); 9.68 (bs, 1H, Ar). M/Z (M+H)⁺: 517

### Compound 186 : [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-4-aza-spiro[2.6]non-4-yl)-methanone

Compound 186 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 8-oxa-4-aza-spiro[2.6]nonane hydrochloride B59. In that specific case, 4 equiv. of diisopropylethylamine were used. Purification by flash chromatography (DCM/MeOH: 100/0 to 95/5), then by preparative HPLC afforded 186 as a white powder in 16% yield. ¹H-NMR (DMSO-*d_{6,}* 400MHz): 0.49-0.53 (m, 2H, major rotamer, CH₂); 0.65-0.69 (m, 2H, major rotamer, CH₂); 0.93-096 (m, 2H, minor rotamer, CH₂); 1.02-1.05 (m, 2H, minor rotamer, CH₂); 1.74-1.79 (m, 1H, minor rotamer, CH₂); 2.02-2.08 (m, 1H, major rotamer, CH₂); 3.39-3.42 (m, 1H, N-CH₂); 3.66-3.89 (m, 5H, N-CH₂, O-CH₂); 5.77 (q, *J* 9.1 Hz, 2H, CH₂-CF₃); 7.81 (ddd, *J* 10.8, 9.3, 1.8 Hz, 1H, Ar); 8.12 (s, 1H, major rotamer, Ar); 8.22 (s, 1H, minor rotamer, Ar); 8.85 (s, 1H, minor rotamer, Ar); 8.88 (s, 1H, major rotamer, Ar); 9.48 (ddd, *J* 8.9, 5.1, 1.8 Hz, 1H, Ar); 9.63 (s, 1H, minor rotamer, Ar); 9.66 (s, 1H, major rotamer, Ar). M/Z (M+H)⁺: 507

### Compound 187 : [3-(6,8-Difluoro-imidazo[1,2-a]pyridin-3-yl)-1-(2,2,2-trifluoro-ethyl)-1H-pyrazolo[4,3-c]pyridin-6-yl]-(8-endo-hydroxy-3-aza-bicyclo[3.2.1]oct-3-yl)-methanone

Compound 187 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and *endo*-3-aza-bicyclo[3.2.1]oct-8-ol hydrochloride B60 (1.2 equiv.). In that specific case, 4 equiv. of diisopropylethylamine were used. Purification by flash chromatography (DCM/MeOH: 100/0 to 92/8) afforded 187 as a white powder in 51% yield. ¹H-NMR (DMSO-*d_{6,}* 400MHz): 1.48-1.82 (m, 5H, CH₂); 2.00-2.06 (m, 1H, CH₂); 3.05-3.12 (m, 1H, N-CH₂); 3.32-3.37 (m, 1H, N-CH₂); 3.60 (d, *J* 12.1 Hz, 1H, N-CH₂); 3.85-3.90 (m, 1H, CH-OH); 4.13-4.20 (m, 1H, N-CH₂); 5.17 (d, *J* 1.8 Hz, 1H, OH); 5.72-5.84 (q, *J* 8.8 Hz, 2H, CF₃-CH₂); 7.80 (ddd, *J* 11.0, 9.2, 2.2 Hz, 1H, Ar); 8.16 (bs, 1H, Ar); 8.87 (s, 1H, Ar); 9.46-9.50 (m, 1H, Ar); 9.66 (d, *J* 1.0 Hz, 1H, Ar).M/Z (M+H)⁺: 507

### Compound 188 : [3-(6,8-Difluoro-imidazo[1,2-a]pyridin-3-yl)-1-(2,2,2-trifluoro-ethyl)-1H-pyrazolo[4,3-c]pyridin-6-yl]-(7-endo-hydroxy-3-oxa-9-aza-bicyclo[3.3.1]non-9-yl)-methanone

Compound 188 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and *endo*-3-oxa-9-azabicyclo[3.3.1]nonan-7-ol trifluoroacetate B61 (1.2 equiv.). In that specific case, 4 equiv. of diisopropylethylamine were used. Purification by flash chromatography (DCM/MeOH: 100/0 to 95/5) afforded 188 as a white powder in 58% yield. ¹H-NMR (DMSO-*d_{6,}* 400MHz): 1.62-1.70 (m, 1H, CH₂); 1.79-1.87 (m, 1H, CH₂); 2.18-2.27 (m, 2H, CH₂); 3.66-3.78 (m, 3H, N-CH₂, O-CH₂); 3.80-3.89 (m, 1H, CH-OH); 3.91-3.97 (m, 1H, O-CH₂); 3.99-4.05 (m, 1H, O-CH₂); 4.62-4.67 (m, 1H, O-CH₂); 5.07 (*d, J* 10.9 Hz, 1H, OH); 5.77 (q, *J* 9.0 Hz, 2H, CH₂-CF₃); 7.81 (ddd, *J* 11.0, 9.2, 2.2 Hz, 1H, Ar); 8.28 (bs, 1H, Ar); 8.87 (s, 1H, Ar); 9.46-9.50 (m, 1H, Ar); 9.67 (d, *J* 1.0 Hz, 1H, Ar). M/Z (M+H)⁺: 523

### Compound 189 : 1-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-diazepan-5-one

Compound 189 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and homopiperazin-5-one B62. Purification by flash chromatography (DCM/MeOH: 10/0 to 9/1) afforded 189 as a white powder in 52% yield. ¹H-NMR (DMSO-*d*_{*6*,} 400MHz, 80°C): 2.57-2.63 (m, 2H, CH₂); 3.26-3.36 (m, 2H, CH₂); 3.51-3.88 (m, 4H, N-CH₂); 5.66 (q, *J* 9.0 Hz, 2H, CH₂-CF₃); 7.32 (bs, 1H, NH); 7.62 (ddd, *J* 11.0, 9.0, 2.0 Hz, 1H, Ar); 8.16 (bs, 1H, Ar); 8.75 (s, 1H, Ar); 9.40-9.44 (m, 1H, Ar); 9.58 (d, *J* 1.1 Hz, 1H, Ar). M/Z (M+H)⁺: 494

### Compound 190 : [3-(6,8-Difluoro-imidazo[1,2-a]pyridin-3-yl)-1-(2,2,2-trifluoro-ethyl)-1H-pyrazolo[4,3-c]pyridin-6-yl]-1,4-oxazepan-5,5-dideuterio-4-yl-methanone

Compound 190 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 5,5-dideuterio-1,4-oxazepane hydrochloride B63. In that specific case, 4 equiv. of diisopropylethylamine were used. Purification by flash chromatography (DCM/MeOH: 100/0 to 95/5) afforded 190 as a white powder in 61% yield. ¹H-NMR (DMSO-*d*_{*6*,} 400MHz): 1.76 (t, *J* 5.5 Hz, 1H, CH₂); 1.93 (t, *J* 5.5 Hz, 1H, CH₂); 3.48-3.53 (m, 1H, N-CH₂); 3.64-3.67 (m, 1H, N-CH₂); 3.69-3.81 (m, 4H, O-CH₂); 5.76 (q, *J* 8.9 Hz, 2H, CH₂-CF₃); 7.80 (ddd, *J* 11.1, 9.1, 2.3 Hz, 1H, Ar); 8.22 (bs, 1H, Ar); 8.87 (d, *J* 2.3 Hz, 1H, Ar); 9.47-9.49 (m, 1H, Ar); 9.66 (d, *J* 1.1 Hz, 1H, signal of a rotamer, Ar); 9.67 (d, *J* 1.1 Hz, 1H, signal of a rotamer, Ar). M/Z (M+H)⁺: 483

### Compound 191 : [3-(6,8-Difluoro-imidazo[1,2-a]pyridin-3-yl)-1-(2,2,2-trifluoro-ethyl)-1H-pyrazolo[4,3-c]pyridin-6-yl]-[1,2]oxazepan-2-yl-methanone

Compound 191 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and [1,2]oxazepane hydrochloride B64. In that specific case, 4 equiv. of diisopropylethylamine were used. Purification by flash chromatography (DCM/MeOH: 100/0 to 95/5), then by preparative HPLC afforded 191 as a white powder in 38% yield. ¹H-NMR (DMSO-*d*_{*6*,} 400MHz): 1.67-1.91 (m, 6H, CH₂); 3.65-3.87 (m, 2H, CH₂); 3.87-4.20 (m, 2H, CH₂); 5.77 (q, *J* 9.1 Hz, 2H, CH₂-CF₃); 7.80 (ddd, *J* 11.1, 9.2, 2.1 Hz, 1H, Ar); 8.23 (bs, 1H, Ar); 8.87 (s, 1H, Ar); 9.46-9.50 (m, 1H, Ar); 9.67 (d, *J* 1.0 Hz, 1H, Ar). M/Z (M+H)⁺: 481

### Compound 192 : [3-(6,8-Difluoro-imidazo[1,2-a]pyridin-3-yl)-1-(2,2,2-trifluoro-ethyl)-1H-pyrazolo[4,3-c]pyridin-6-yl]-(5-oxa-8-aza-spiro[3.5]non-8-yl)-methanone

Compound 192 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 5-oxa-8-aza-spiro[3.5]nonane B65. Purification by flash chromatography (DCM/MeOH: 100/0 to 95/5) afforded 192 as a white powder in 56% yield. ¹H-NMR (DMSO-*d_{6,}* 400MHz): 1.24-1.42 (m, 1H, signal of a rotamer, CH₂); 1.55-2.10 (m, 5H, signal of a rotamer, 1H, signal of a rotamer, CH₂); 3.39-3.44 (m, 1H, N-CH₂); 3.45 (bs, 1H, N-CH₂); 3.48-3.54 (m, 1H, N-CH₂), 3.58-3.69 (m, 2H, O-CH₂); 3.73 (bs, 1H, N-CH₂); 5.77 (q, *J* 8.9 Hz, 2H, CH₂-CF₃); 7.81 (ddd, *J* 10.9, 9.0, 2.2 Hz, 1H, Ar); 8.25 (s, 1H, signal of a rotamer, Ar); 8.27 (s, 1H, signal of a rotamer, Ar); 8.86 (s, 1H, signal of a rotamer, Ar); 8.90 (s, 1H, signal of a rotamer, Ar); 9.48-9.49 (m, 1H, Ar); 9.67 (s, 1H, signal of a rotamer, Ar); 9.70 (s, 1H, signal of a rotamer, Ar). M/Z (M+H)⁺: 507

### Compound 193 : [3-(6,8-Difluoro-imidazo[1,2-a]pyridin-3-yl)-1-(2,2,2-trifluoro-ethyl)-1H-pyrazolo[4,3-c]pyridin-6-yl]-(6-hydroxy-1,4-oxazepan-4-yl)-methanone

Compound 193 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 1,4-oxazepan-6-ol B66. Purification by flash chromatography (DCM/MeOH: 10/0 to 9/1), then by preparative HPLC afforded 193 as a yellow powder in 13% yield. ¹H-NMR (DMSO-*d*_{*6*,} 400MHz): 3.23-4.28 (m, 10H, CH, CH₂, OH); 5.70-5.82 (m, 2H, CH₂-CF₃); 7.77-7.85 (m, 1H, Ar); 8.22-8.25 (m, 1H, Ar); 8.87 (s, 1H, signal of a rotamer, Ar); 8.89 (s, 1H, signal of a rotamer, Ar); 9.45-9.50 (m, 1H, Ar); 9.66 (d, *J* 1.0 Hz, 1H, Ar); 9.68 (d, *J* 1.0 Hz, 1H, Ar). M/Z (M+H)⁺: 497

### Compound 194 : 5-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,5-diaza-bicyclo[2.2.1]heptane-2-carboxylic acid tert-butyl ester

Compound 194 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and (1S,4S)-2-Boc-2,5-diaza-bicyclo[2.2.1]heptane B67. Purification by flash chromatography (DCM/MeOH: 100/0 to 95/5), then by preparative HPLC afforded 194 as a white powder in 75% yield. ¹H-NMR (DMSO-*d_{6,}* 400MHz): 1.32-1.50 (m, 9H, CH₃); 1.71-1.99 (m, 2H, CH₂); 3.27-3.72 (m, 3H, CH₂); 3.90-4.54 (m, 2H, CH₂); 4.95 (bs, 1H, signal of a rotamer, N-CH₂), 5.00 (bs, 1H, signal of a rotamer, N-CH₂), 5.74-5.90 (m, 2H, CH₂-CF₃); 7.81 (ddd, *J* 11.1, 9.1, 2.1 Hz, 1H, Ar); 8.43 (bs, 1H, major rotamer, Ar); 8.49 (bs, 1H, minor rotamer, Ar); 8.86 (s, 1H, minor rotamer, Ar); 8.89 (s, 1H, major rotamer, Ar); 9.45-9.51 (m, 1H, Ar); 9.65 (d, *J* 1.0 Hz, 1H, minor rotamer, Ar); 9.71 (bs, 1H, major rotamer, Ar). M/Z (M+H)⁺: 578

### Compound 195 : 1,4-Diazepan-1-yl-[3-(6,8-difluoro-imidazo[1,2-a]pyridin-3-yl)-1-(2,2,2-trifluoro-ethyl)-1H-pyrazolo[4,3-c]pyridin-6-yl]-methanone hydrochloride

Compound 195 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and homopiperazine B68 (5 equiv.). Purification by flash chromatography (DCM/MeOH: 10/0 to 9/1), then by preparative HPLC afforded 195 as a white powder in 19% yield. ¹H-NMR (DMSO-*d_{6,}* 400MHz): 2.02-2.09 (m, 1H, CH₂); 2.10-2.18 (m, 1H, CH₂); 3.18-3.42 (m, 4H, N-CH₂); 3.55 (t, *J* 6.0 Hz, 1H, N-CH₂), 3.37-3.74 (m, 1H, N-CH₂); 3.79 (d, *J* 6.0 Hz, 1H, N-CH₂); 3.39-3.67 (m, 1H, N-CH₂); 5.73-5.84 (m, 2H, CH₂-CF₃); 7.82 (ddd, *J* 10.9, 8.9, 2.1 Hz, 1H, Ar); 8.32 (bs, 1H, signal of a rotamer, Ar); 8.34 (bs, 1H, signal of a rotamer, Ar); 8.89 (s, 1H, signal of a rotamer, Ar); 8.90 (s, 1H, signal of a rotamer, Ar); 9.31-9.42 (m, 2H, NH₂⁺); 9.48-9.51 (m, 1H, Ar); 9.69 (bs, 1H, Ar). M/Z (M+H)⁺: 480

### Compound 196 : [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-5-aza-spiro[3.5]non-5-yl)-methanone

Compound 196 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 8-oxa-5-aza-spiro[3.5]nonane B69. Purification by flash chromatography (DCM/MeOH: 100/0 to 95/5), then by preparative HPLC afforded 196 as a white powder in 40% yield. ¹H-NMR (DMSO-*d_{6,}* 400MHz): 1.53-2.26 (m, 4H, CH₂); 2.51-2.61 (m, 2H, CH₂); 3.35-3.63 (m, 4H, O-CH₂); 3.79 (bs, 2H, N-CH₂), 5.75 (q, *J* 9.1 Hz, 2H, CH₂-CF₃); 7.80 (ddd, *J* 11.0, 9.2, 2.3 Hz, 1H, Ar); 8.24 (bs, 1H, Ar); 8.85 (s, 1H, Ar); 9.44-9.50 (m, 1H, Ar); 9.64 (d, *J* 1.0 Hz, 1H, Ar). M/Z (M+H)⁺: 507

### Compound 197 : (2-{[3-(6,8-Difluoro-imidazo[1,2-a]pyridin-3-yl)-1-(2,2,2-trifluoroethyl)-1H-pyrazolo[4,3-c]pyridine-6-carbonyl]-methyl-amino}-ethyl)-carbamic acid tert-butyl ester

Compound 197 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and *tert*-butyl-2-(methylamino)ethylcarbamate B70. Purification by flash chromatography (DCM/MeOH: 100/0 to 95/5), then by preparative HPLC afforded 197 as a beige powder in 60% yield. ¹H-NMR (DMSO-*d_{6,}* 400MHz, 80°C:) 1.37 (bs, 9H, CH₃); 3.07 (s, 3H, N-CH₃); 3.24 (bs, 2H, N-CH₂); 3.52 (bs, 2H, N-CH₂), 5.65 (q, *J* 9.0 Hz, 2H, CH₂-CF₃); 6.44 (bs, 1H, NH); 7.62 (ddd, *J* 11.0, 9.2, 2.2 Hz, 1H, Ar); 8.10 (bs, 1H, Ar); 8.73 (s, 1H, Ar); 9.39-9.47 (m, 1H, Ar); 9.56 (d, *J* 0.9 Hz, 1H, Ar). M/Z (M+H)⁺: 554

### Compound 198 : 3-(6,8-Difluoro-imidazo[1,2-a]pyridin-3-yl)-1-(2,2,2-trifluoro-ethyl)-1H-pyrazolo[4,3-c]pyridine-6-carboxylic acid (4-hydroxy-tetrahydro-pyran-3-yl)-amide

Compound 198 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-oxa-7-aza-bicyclo[4.1.0]heptane B71 (1.2 equiv.). Purification by flash chromatography (DCM/MeOH: 100/0 to 95/5), then by preparative HPLC afforded 198 as a white powder in 45% yield. ¹H-NMR (DMSO-*d_{6,}* 400MHz): 1.47-1.58 (m, 1H, CH₂); 1.87-1.96 (m, 1H, CH₂); 3.25-3.46 (m, 2H, CH₂); 3.76-3.95 (m, 4H, CH, CH₂); 5.09 (d, *J* 2.7 Hz, OH); 5.87 (q, *J* 9.1 Hz, 2H, CH₂-CF₃); 7.81 (ddd, *J* 11.1, 9.2, 2.0 Hz, 1H, Ar); 8.63-8.69 (m, 2H, Ar, NH); 8.87 (s, 1H, Ar); 9.45-9.48 (m, 1H, Ar); 9.65 (d, *J* 0.9 Hz, 1H, Ar). M/Z (M+H)⁺: 497

### Compound 199 : [3-(6,8-Difluoro-imidazo[1,2-a]pyridin-3-yl)-1-(2,2,2-trifluoro-ethyl)-1H-pyrazolo[4,3-c]pyridin-6-yl]-(6-oxa-3-aza-bicyclo[3.2.1]oct-3-yl)-methanone

Compound 199 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 6-oxa-3-aza-bicyclo[3.2.1]octane hydrochloride B72 (1.2 equiv.). In that specific case, 4 equiv. of diisopropylethylamine were used. Purification by flash chromatography (DCM/MeOH: 100/0 to 95/5) afforded 199 as a white powder in 60% yield. ¹H-NMR (DMSO-*d_{6,}* 400MHz): 1.81-1.95 (m, 2H, CH₂); 2.36-2.41 (m, 1H, signal of a rotamer, CH); 2.58-2.63 (m, 1H, signal of a rotamer, CH); 2.89 (d, *J* 13.1 Hz, 1H, signal of a rotamer, CH₂); 3.10 (d, *J* 13.1 Hz, 1H, signal of a rotamer, CH₂); 3.21 (d, *J* 13.1 Hz, 1H, signal of a rotamer, CH₂); 3.34-3.44 (m, 1H, CH₂); 3.49-3.56 (m, 1H, signal of a rotamer, CH₂); 3.64-3.70 (m, 1H, signal of a rotamer, CH₂); 3.70-3.76 (m, 1H, signal of a rotamer, CH₂); 3.80 (d, *J* 7.7 Hz, 1H, signal of a rotamer, CH); 3.90 (d, *J* 7.7 Hz, 1H, signal of a rotamer, CH); 4.10-4.13 (m, 1H, signal of a rotamer, CH₂); 4.27-4.35 (m, 1H, signal of a rotamer, CH₂); 4.34-4.39 (m, 1H, signal of a rotamer, CH₂); 4.44-4.51 (m, 1H, signal of a rotamer, CH₂); 5.68-5.83 (m, 2H, CF₃-CH₂); 7.80 (ddd, *J* 11.0, 9.2, 1.9 Hz, 1H, Ar); 8.18 (bs, 1H, signal of a rotamer, Ar); 8.19 (bs, 1H, signal of a rotamer, Ar); 8.86 (s, 1H, signal of a rotamer, Ar); 8.87 (s, 1H, signal of a rotamer, Ar); 9.45-9.49 (m, 1H, Ar); 9.65 (d, *J* 0.9 Hz, 1H, signal of a rotamer, Ar); 9.68 (d, *J* 0.9 Hz, 1H, signal of a rotamer, Ar). M/Z (M+H)⁺: 493

### Compound 200 : 3-[3-(6,8-Difluoro-imidazo[1,2-a]pyridin-3-yl)-1-(2,2,2-trifluoro-ethyl)-1H-pyrazolo[4,3-c]pyridine-6-carbonyl]-3,8-diaza-bicyclo[3.2.1]octane-8-carboxylic acid tert-butyl ester

Compound 200 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 8-Boc-3,8-diaza-bicyclo[3.2.1]octane B73. Purification by flash chromatography (DCM/MeOH: 100/0 to 95/5) afforded 200 as a white powder in 65% yield. ¹H-NMR (DMSO-*d*_{*6*,} 400MHz): 1.42 (s, 9H, O-CH₃); 1.66-1.99 (m, 4H, CH₂); 3.00 (d, *J* 12.4 Hz, 1H, N-CH₂); 3.27 (d, *J* 12.8 Hz, 1H, N-CH); 3.49 (d, *J* 12.4 Hz, 1H, N-CH); 4.03 (s, 1H, N-CH); 4.25 (d, *J* 5.7 Hz, 1H, N-CH); 4.39 (d, *J* 12.4 Hz, 1H, N-CH); 5.69-5.83 (m, 2H, CH₂-CF₃); 7.81 (ddd, *J* 11.0, 9.1, 2.2Hz, 1H, Ar); 8.23 (bs, 1H, Ar); 8.87 (s, 1H, Ar); 9.46-9.50 (m, 1H, Ar); 9.68 (d, *J* 0.9 Hz, 1H, Ar). M/Z (M+H)⁺: 592

### Compound 201 : 4-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-diazepan-2-one

Compound 201 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 1,4-diazepan-2-one B74. Purification by flash chromatography (DCM/MeOH: 10/0 to 9/1) afforded 201 as a white powder in 44% yield. ¹H-NMR (DMSO-*d_{6,}* 400MHz): 1.72-1.80 (m, 2H, major rotamer, CH₂); 1.85-1.93 (m, 2H, minor rotamer, CH₂); 3.18-3.27 (m, 2H, CH₂); 3.54 (t, *J* 5.7 Hz, 2H, major rotamer, N-CH₂); 3.84 (t, *J* 5.7 Hz, 2H, minor rotamer, N-CH₂); 4.25 (s, 2H, minor rotamer, N-CH₂); 4.30 (s, 2H, major rotamer, N-CH₂); 5.76 (q, *J* 9.0 Hz, 2H, CH₂-CF₃); 7.62 (t, *J* 4.9 Hz, 1H, minor rotamer, NH); 7.66 (t, *J* 4.9 Hz, 1H, major rotamer, NH); 7.80 (ddd, *J* 10.9, 9.1, 2.0 Hz, 1H, Ar); 8.21 (bs, 1H, minor rotamer, Ar); 8.24 (bs, 1H, major rotamer, Ar); 8.87 (s, 1H, major rotamer, Ar); 8.88 (s, 1H, minor rotamer, Ar); 9.45-9.49 (m, 1H, Ar); 9.64 (d, *J* 1.0 Hz, 1H, minor rotamer, Ar); 9.67 (d, *J* 1.0 Hz, 1H, major rotamer, Ar). M/Z (M+H)⁺: 494

### Compound 202 : {1-[3-(6,8-Difluoro-imidazo[1 ,2-a]pyridin-3-yl)-1-(2,2,2-trifluoro-ethyl)-1H-pyrazolo[4,3-c]pyridine-6-carbonyl]-azepan-3-yl}-carbamic acid tert-butyl ester

Compound 202 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3-(Boc-amino)-azepane B75. Purification by flash chromatography (DCM/MeOH: 100/0 to 95/5) afforded 202 as a white powder in 44% yield. ¹H-NMR (DMSO-*d*_{*6*,} 400MHz): 0.88-1.95 (m, 15H, CH₃, CH₂); 3.12-4.10 (m, 5H, N-CH, N-CH₂); 5.70-5.83 (m, 2H, CH₂-CF₃); 6.61 (d, *J* 7.5 Hz, 1H, signal of a rotamer, NH); 6.87 (d, *J* 7.5 Hz, 1H, signal of a rotamer, NH); 7.80 (ddd, *J* 11.1, 9.2, 2.1 Hz, 1H, Ar); 8.17 (bs, 1H, signal of a rotamer, Ar); 8.21 (bs, 1H, signal of a rotamer, Ar); 8.84 (s, 1H, signal of a rotamer, Ar); 8.87 (s, 1H, signal of a rotamer, Ar); 9.44-9.50 (m, 1H, Ar); 9.62 (bs, 1H, signal of a rotamer, Ar); 9.66 (d, *J* 1.0 Hz, 1H, signal of a rotamer, Ar). M/Z (M+H)⁺: 594

### Compound 203 : (3,3-Difluoro-4-hydroxy-piperidin-1-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone

Compound 203 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 3,3-difluoro-4-hydroxy-piperidine B77. Purification by flash chromatography (DCM/MeOH: 100/0 to 95/5) afforded 203 as a white powder in 53% yield. ¹H-NMR (DMSO-*d_{6,}* 400MHz): 1.63-1.78 (m, 1H, CH₂); 1.80-2.00 (m, 1H, CH₂); 3.38-3.47 (m, 1H, CH₂); 3.52-3.69 (m, 1H, CH₂); 3.79-4.20 (m, 3H, CH₂); 5.73-5.87 (m, 3H, OH, CH₂-CF₃); 7.81 (ddd, *J* 11.1, 9.3, 2.1 Hz, 1H, Ar); 8.24 (s, 1H, major rotamer, Ar); 8.31 (s, 1H, minor rotamer Ar); 8.87 (s, 1H, Ar); 9.44-9.52 (m, 1H, Ar); 9.66-9.69 (m, 1H, Ar). M/Z (M+H)+: 517

### Compound 204 : {4-[3-(6,8-Difluoro-imidazo[1 ,2-a]pyridin-3-yl)-1-(2,2,2-trifluoro-ethyl)-1H-pyrazolo[4,3-c]pyridine-6-carbonyl]-1,4-oxazepan-6-yl}-carbamic acid tert-butyl ester

Compound 204 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 1,4-oxazepan-6-yl-carbamic acid *tert*-butyl ester B78. Purification by flash chromatography (DCM/MeOH: 100/0 to 95/5) afforded 204 as a white powder in 62% yield. ¹H-NMR (DMSO-*d*_{*6*,} 400MHz): 0.89-1.24 (m, 9H, signal of a rotamer, CH₃); 1.41 (m, 9H, signal of a rotamer, CH₃); 3.38-4.22 (m, 9H, CH, CH₂); 5.70-5.86 (m, 2H, CH₂-CF₃); 6.68 (d, *J* 7.8 Hz, 1H, signal of a rotamer, NH); 6.90 (d, *J* 7.8 Hz, 1H, signal of a rotamer, NH); 7.81 (ddd, *J* 11.1, 8.9, 2.2 Hz, 1H, Ar); 8.26 (bs, 1H, signal of a rotamer, Ar); 8.27 (bs, 1H, signal of a rotamer, Ar); 8.51 (s, 1H, signal of a rotamer, Ar); 8.86 (s, 1H, signal of a rotamer, Ar); 9.45-9.51 (m, 1H, Ar); 9.64 (bs, 1H, signal of a rotamer, Ar); 9.66 (d, *J* 0.9 Hz, 1H, signal of a rotamer, Ar). M/Z (M+H)⁺: 596

### Compound 205 : [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[4.1.0]hept-5-yl)-methanone

Compound 205 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 2-oxa-5-aza-bicyclo[4.1.0]heptane hydrochloride B79 (1.2 equiv.). In that specific case, 4 equiv. of diisopropylethylamine were used. Purification by flash chromatography (DCM/MeOH: 100/0 to 97/3) afforded 205 as a white powder in 75% yield. ¹H-NMR (DMSO-*d_{6,}* 400MHz): 0.45-0.52 (m, 1H, major rotamer, CH₂); 0.57-0.64 (m, 1H, major rotamer, CH₂); 0.82-0.87 (m, 1H, minor rotamer, CH₂); 0.94-1.01 (m, 1H, minor rotamer, CH₂); 2.93-2.99 (m, 1H, major rotamer, CH₂); 3.11-3.19 (m, 2H, minor rotamer, CH₂); 3.26-3.47 (m, 2H, major rotamer, 1H, minor rotamer, CH₂); 3.57-3.75 (m, 3H, CH, CH₂); 3.78-3.85 (m, 1H, CH); 5.77 (q, *J* 9.0 Hz, 2H, CF₃-CH₂); 7.81 (ddd, *J* 11.0, 9.5, 2.0 Hz, 1H, Ar); 8.26 (bs, 1H, major rotamer, Ar); 8.29 (bs, 1H, minor rotamer, Ar); 8.87 (s, 1H, minor rotamer, Ar); 8.88 (s, 1H, major rotamer, Ar); 9.45-9.49 (m, 1H, Ar); 9.68 (d, *J* 0.8 Hz, 1H, minor rotamer, Ar); 9.69 (d, *J* 0.8 Hz, 1H, major rotamer, Ar). M/Z (M+H)⁺: 479

### Compound 206: [3-(6,8-Difluoro-imidazo[1,2-a]pyridin-3-yl)-1-(2,2,2-trifluoro-ethyl)-1H-pyrazolo[4,3-c]pyridin-6-yl]-(7-oxa-4-aza-spiro[2.6]non-4-yl)-methanone

Compound 206 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and 7-oxa-4-azaspiro[2.6]nonane B80 (1.2 equiv.). In that specific case, 6 equiv. of diisopropylethylamine were used. Purification by flash chromatography (DCM/MeOH: 100/0 to 94/6) afforded 206 as a beige powder in 20% yield. ¹H-NMR (DMSO-*d*_{*6*,} 400MHz): 0.38-0.51 (m, 2H, major rotamer, CH₂); 0.58-0.71 (m, 2H, major rotamer, CH₂); 0.88-0.93 (m, 2H, minor rotamer, CH₂); 0.94-1.01 (m, 2H, minor rotamer, CH₂); 1.81-2.11 (m, 2H, CH₂); 3.40-3.46 (m, 1H, CH₂); 3.63-3.93 (m, 5H, CH₂); 5.77 (q, *J* 9.0 Hz, 4H, CH₂- CF₃); 7.80 (ddd, *J* 11.0, 9.1, 2.0 Hz, 1H, Ar); 8.13 (bs, 1H, major rotamer, Ar); 8.24 (bs, 1H, minor rotamer, Ar); 8.84 (s, 1H, minor rotamer, Ar); 8.88 (s, 1H, major rotamer, Ar); 9.45-9.51 (m, 1H, Ar) ; 9.61 (d, *J* 0.9 Hz, 1H, minor rotamer, Ar), 9.68 (d, *J* 0.9 Hz, 1H, major rotamer, Ar). M/Z (M+H)⁺: 507

### Compound 207 : [3-Benzofuran-3-yl-1-(2,2,2-trifluoro-ethyl)-1H-pyrazolo[4,3-c]pyridin-6-yl]-(4-hydroxy-piperidin-1-yl)-methanone

Compound 207 was obtained according to General Procedure XIII, starting from lithium 3-(benzofuran-3-yl)-1-(2,2,2-trifluoroethyl)pyrazolo[4,3-c]pyridine-6-carboxylate R3 and piperidin-4-ol B5. Purification by flash chromatography (DCM/MeOH: 100/0 to 95/5) afforded 207 as a white powder in 28% yield. ¹H-NMR (DMSO-*d_{6,}* 400MHz): 1.34-1.48 (m, 2H, CH₂); 1.69-1.74 (m, 1H, CH₂); 1.83-1.88 (m, 1H, CH₂); 3.12-3.19 (m, 1H, N-CH₂); 3.26-3.33 (m, 1H, N-CH₂); 3.51-3.59 (m, 1H, N-CH₂); 3.74-3.81 (m, 1H, N-CH₂); 4.06-4.14 (m, 1H, CH); 4.80 (d, *J* 4.1 Hz, 1H, OH); 5.68 (q, *J* 8.8 Hz, 2H, CH₂-CF₃); 7.42-7.50 (m, 2H, Ar); 7.73-7.76 (m, 1H, Ar); 8.15 (bs, 1H, Ar); 8.33-8.36 (m, 1H, Ar); 9.17 (s, 1H, Ar); 9.57 (d, *J* 0.9 Hz, 1H, Ar). M/Z (M+H)⁺: 445

### Compound 208 : [3-Benzofuran-3-yl-1-(2,2,2-trifluoro-ethyl)-1H-pyrazolo[4,3-c]pyridin-6-yl]-(4-hydroxy-4-methyl-piperidin-1-yl)-methanone

Compound 208 was obtained according to General Procedure XIII, starting from lithium 3-(benzofuran-3-yl)-1-(2,2,2-trifluoroethyl)pyrazolo[4,3-c]pyridine-6-carboxylate R3 and 4-methylpiperidin-4-ol B6. Purification by flash chromatography (DCM/MeOH: 100/0 to 95/5) afforded 208 as a white powder in 14% yield. ¹H-NMR (DMSO-*d_{6,}* 400MHz): 1.18 (s, 3H, CH₃); 1.41-1.63 (m, 4H, CH₂); 3.27-3.41 (m, 3H, CH₂); 4.12-4.18 (m, 1H, CH₂); 4.45 (s, 1H, OH); 5.67 (q, *J* 9.1 Hz, 2H, CH₂-CF₃); 7.42-7.50 (m, 2H, Ar); 7.73-7.76 (m, 1H, Ar); 8.14 (s, 1H, Ar); 8.33-8.36 (m, 1H, Ar); 9.17 (s, 1H, Ar); 9.57 (d, *J* 1.0 Hz, 1H, Ar). M/Z (M+H)⁺: 459

### Compound 209 : [3-Benzofuran-3-yl-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone

Compound 209 was obtained according to General Procedure XIII, starting from lithium 3-(benzofuran-3-yl)-1-(2,2,2-trifluoroethyl)pyrazolo[4,3-c]pyridine-6-carboxylate R3 and nortropine B41. Purification by flash chromatography (DCM/MeOH: 100/0 to 95/5) afforded 209 as a white powder in 22% yield. ¹H-NMR (DMSO-*d_{6,}* 400MHz): 1.70 (d, *J* 13.8 Hz, 1H, CH₂); 1.81-1.92 (m, 3H, CH₂); 2.05-2.11 (m, 2H, CH₂); 2.19-2.33 (m, 2H, CH₂); 3.98-4.02 (m, 1H, CH); 4.49-4.52 (m, 1H, CH); 4.65 (d, *J* 2.4 Hz, 1H, OH); 4.66-4.70 (m, 1H, CH); 5.70 (q, *J* 9.0 Hz, 2H, CH₂-CF₃); 7.43-7.50 (m, 2H, Ar); 7.73-7.76 (m, 1H, Ar); 8.28 (bs, 1H, Ar); 8.33-8.36 (m, 1H, Ar); 9.17 (s, 1H, Ar); 9.58 (d, *J* 1.0 Hz, 1H, Ar). M/Z (M+H)⁺: 471

### Compound 210 : [3-Benzofuran-3-yl-1-(2,2,2-trifluoro-ethyl)-1H-pyrazolo[4,3-c]pyridin-6-yl]-(8-exo-ethyl-8-endo-hydroxy-3-aza-bicyclo[3.2.1]oct-3-yl)-methanone

Compound 210 was obtained according to General Procedure XIII, starting from lithium 3-(benzofuran-3-yl)-1-(2,2,2-trifluoroethyl)pyrazolo[4,3-c]pyridine-6-carboxylate R3 and *endo*-(8-*exo*-ethyl)-3-azabicyclo[3.2.1]octan-8-ol B81. Purification by flash chromatography (DCM/MeOH: 100/0 to 95/5) afforded 210 as a beige powder in 62% yield. ¹H-NMR (DMSO-*d*_{*6*,} 400MHz): 0.86 (t, *J* 7.3 Hz, CH₃); 1.38-1.73 (m, 7H, CH, CH₂); 1.83-1.89 (m, 1H, CH); 3.10 (d, *J* 12.1 Hz, 1H, N-CH₂); 3.39 (d, *J* 12.1 Hz, 1H, N-CH₂); 3.66 (d, *J* 12.1, 1H, N-CH₂); 4.18 (dd, *J* 12.1, 2.8 Hz, 1H, N-CH₂); 4.63 (s, 1H, OH); 5.68 (q, *J* 9.1 Hz, 2H, CH₂-CF₃); 7.42-7.50 (m, 2H, Ar); 7.72-7.76 (m, 1H, Ar); 8.13 (d, *J* 0.8 Hz, 1H, Ar); 8.32-8.36 (m, 1H, Ar); 9.16 (s, 1H, Ar); 9.58 (d, *J* 0.8 Hz, 1H, Ar). M/Z (M+H)⁺: 499

### Compound 211 : [3-Benzofuran-3-yl-1-(2,2,2-trifluoro-ethyl)-1H-pyrazolo[4,3-c]pyridin-6-yl]-(5-endo-hydroxy-5-exo-methyl-2-aza-bicyclo[2.2.1]hept-2-yl)-methanone

Compound 211 was obtained according to General Procedure XIII, starting from lithium 3-(benzofuran-3-yl)-1-(2,2,2-trifluoroethyl)pyrazolo[4,3-c]pyridine-6-carboxylate R3 and *endo*-(5-*exo*-methyl)-2-azabicyclo[2.2.1]heptan-5-ol B82. Purification by flash chromatography (DCM/MeOH: 100/0 to 95/5) afforded 211 as a white powder in 26% yield. ¹H-NMR (DMSO-*d_{6,}* 400MHz): 1.29 (s, 3H, minor rotamer, CH₃); 1.31 (s, 3H, major rotamer, CH₃); 1.57-1.81 (m, 4H, CH₂); 2.23-2.26 (m, 1H, minor rotamer, CH); 2.28-2.31 (m, 1H, major rotamer, CH); 3.25 (d, *J* 3.3 Hz, 1H, minor rotamer, N-CH₂); 3.27-3.32 (m, 2H, major rotamer, N-CH₂); 3.68 (d, *J* 10.0, 3.3 Hz, 1H, minor rotamer, N-CH₂); 3.91 (dd, *J* 10.0, 1.0 Hz, 1H, major rotamer, CH₂); 3.97 (d, *J* 10.0 Hz, 1H, minor rotamer, CH₂); 4.52 (s, 1H, N-CH); 4.58 (s, 1H, minor rotamer, OH); 4.73 (s, 1H, major rotamer, OH); 5.67-5.79 (m, 2H, CH₂-CF₃); 7.42-7.50 (m, 2H, Ar); 7.71-7.77 (m, 1H, Ar); 8.30 (bs, 1H, major rotamer, Ar); 8.34-8.37 (m, 1H, Ar); 8.40 (bs, 1H, minor rotamer, Ar); 9.16 (s, 1H, minor rotamer, Ar); 9.19 (s, 1H, major rotamer, Ar); 9.57 (d, *J* 1.0 Hz, 1H, minor rotamer, Ar); 9.59 (d, *J* 1.0 Hz, 1H, major rotamer, Ar). M/Z (M+H)⁺: 471

### Compound 212 : [3-Benzofuran-3-yl-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone

Compound 212 was obtained according to General Procedure XIII, starting from lithium 3-(benzofuran-3-yl)-1-(2,2,2-trifluoroethyl)pyrazolo[4,3-c]pyridine-6-carboxylate R3 and *endo*-(8-*exo*-methyl)-3-azabicyclo[3.2.1]octan-8-ol B35. Purification by flash chromatography (DCM/MeOH: 100/0 to 95/5) afforded 212 as a white powder in 35% yield. ¹H-NMR (DMSO-*d_{6,}* 400MHz): 1.18 (s, 3H, CH₃); 1.45-1.84 (m, 6H, CH, CH₂); 3.09 (dd, *J* 12.1, 3.1 Hz, 1H, N-CH₂); 3.40 (d, *J* 12.1 Hz, 1H, N-CH₂); 3.66 (d, *J* 12.1, 1H, N-CH₂); 4.16 (dd, *J* 12.1, 3.1 Hz, 1H, N-CH₂); 4.86 (s, 1H, OH); 5.68 (q, *J* 9.2 Hz, 2H, CH₂-CF₃); 7.42-7.50 (m, 2H, Ar); 7.72-7.76 (m, 1H, Ar); 8.12 (bs, 1H, Ar); 8.33-8.36 (m, 1H, Ar); 9.16 (s, 1H, Ar); 9.57 (d, *J* 1.0 Hz, 1H, Ar). M/Z (M+H)⁺: 485

### Compound 213 : [3-Benzofuran-3-yl-1-(2,2,2-trifluoro-ethyl)-1H-pyrazolo[4,3-c]pyridin-6-yl]-(8-endo-hydroxy-8-exo-methyl-exo-d₃-aza-bicyclo[3.2.1]oct-3-yl)-methanone

Compound 213 was obtained according to General Procedure XIII, starting from lithium 3-(benzofuran-3-yl)-1-(2,2,2-trifluoroethyl)pyrazolo[4,3-c]pyridine-6-carboxylate R3 and *endo*-(8-*exo*-methyl-*d₃)*-3-aza-bicyclo[3.2.1]octan-8-ol B83. Purification by flash chromatography (DCM/MeOH: 100/0 to 95/5) afforded 213 as a white powder in 26% yield. ¹H-NMR (DMSO-*d_{6,}* 400MHz): 1.45-1.80 (m, 6H, CH, CH₂); 3.09 (dd, *J* 12.1, 3.0 Hz, 1H, N-CH₂); 3.40 (d, *J* 12.1 Hz, 1H, N-CH₂); 3.67 (d, *J* 12.1*,* 1H, N-CH₂); 4.16 (dd, *J* 12.1, 3.0 Hz, 1H, N-CH₂); 4.85 (s, 1H, OH); 5.68 (q, *J* 9.1 Hz, 2H, CH₂-CF₃); 7.42-7.50 (m, 2H, Ar); 7.71-7.78 (m, 1H, Ar); 8.13 (d, *J* 0.9 Hz, 1H, Ar); 8.32-8.38 (m, 1H, Ar); 9.16 (s, 1H, Ar); 9.58 (d, *J* 0.9 Hz, 1H, Ar). M/Z (M+H)⁺: 488

### Compound 214 : [3-Benzofuran-3-yl-1-(2,2,2-trifluoro-ethyl)-1H-pyrazolo[4,3-c]pyridin-6-yl]-(8-exo-ethynyl-8-endo-hydroxy-3-aza-bicyclo[3.2.1]oct-3-yl)-methanone Compound 214 was obtained according to General Procedure XIII, starting from

lithium 3-(benzofuran-3-yl)-1-(2,2,2-trifluoroethyl)pyrazolo[4,3-c]pyridine-6-carboxylate R3 and *endo*-(8-*exo*-ethynyl)-3-aza-bicyclo[3.2.1]octan-8-ol B84. Purification by flash chromatography (DCM/MeOH: 100/0 to 95/5) afforded 214 as a white powder in 51% yield. ¹H-NMR (DMSO-*d*_{*6*,} 400MHz): 1.52-1.71 (m, 2H, CH₂); 1.78-1.98 (m, 3H, CH₂); 2.13-2.18 (m, 1H, CH₂); 3.20 (dd, *J* 12.8, 2.7 Hz, 1H, N-CH₂); 3.31-3.35 (m, 1H, N-CH₂); 3.36 (s, 1H, CH); 3.59 (d, *J* 12.1, 1H, N-CH₂); 4.22 (dd, *J* 12.8, 2.7 Hz, 1H, N-CH₂); 5.62-5.73 (m, 2H, CH₂-CF₃); 6.09 (d, 1H, OH); 7.42-7.50 (m, 2H, Ar); 7.72-7.76 (m, 1H, Ar); 8.65 (s, 1H, Ar); 8.32-8.37 (m, 1H, Ar); 9.16 (s, 1H, Ar); 9.58 (d, *J* 1.0 Hz, 1H, Ar). M/Z (M+H)⁺: 495

### Compound 215 : [3-Benzofuran-3-yl-1-(2,2,2-trifluoro-ethyl)-1H-pyrazolo[4,3-c]pyridin-6-yl]-(2-methoxymethyl-pyrrolidin-1-yl)-methanone

Compound 215 was obtained according to General Procedure XIII, starting from lithium 3-(benzofuran-3-yl)-1-(2,2,2-trifluoroethyl)pyrazolo[4,3-c]pyridine-6-carboxylate R3 and 2-(methoxymethyl)pyrrolidine B22. Purification by flash chromatography (Cyclohexane/AcOEt: 10/0 to 3/7) afforded 215 as an orange powder in 30% yield. ¹H-NMR (DMSO-*d*_{*6*,} 400MHz): 1.74-2.01 (m, 4H, CH₂); 3.00 (s, 3H, minor rotamer, CH₃); 3.07 (dd, *J* 9.7, 7.2 Hz, 1H, minor rotamer, N-CH₂); 3.20 (dd, *J* 9.7, 4.8 Hz, 1H, minor rotamer, N-CH₂); 3.33 (s, 3H, major rotamer, CH₃); 3.49 (dd, *J* 9.7, 7.2 Hz, 1H, major rotamer, N-CH₂); 3.51-3.60 (m, 1H, O-CH₂); 3.64 (dd, *J* 9.7, 3.3 Hz, 1H, major rotamer, N-CH₂); 3.66-3.72 (m, 1H, O-CH₂); 4.31-4.39 (m, 1H, major rotamer, CH); 4.67-4.77 (m, 1H, minor rotamer, CH); 5.63-5.80 (m, 2H, CH₂-CF₃); 7.41-7.50 (m, 2H, Ar); 7.72-7.77 (m, 1H, Ar); 8.30 (bs, 1H, Ar); 8.33-8.38 (m, 1H, Ar); 9.16 (s, 1H, major rotamer, Ar); 9.18 (s, 1H, minor rotamer, Ar); 9.57 (d, *J* 0.9 Hz, 1H, Ar). M/Z (M+H)⁺: 459

### Compound 216 : [3-Benzofuran-3-yl-1-(2,2,2-trifluoro-ethyl)-1H-pyrazolo[4,3-c]pyridin-6-yl]-(4-fluoro-piperidin-1-yl)-methanone

Compound 216 was obtained according to General Procedure XIII, starting from lithium 3-(benzofuran-3-yl)-1-(2,2,2-trifluoroethyl)pyrazolo[4,3-c]pyridine-6-carboxylate R3 and 4-fluoropiperidine B16. Purification by flash chromatography (Cyclohexane/AcOEt: 10/0 to 3/7) afforded 216 as a white powder in 51% yield. ¹H-NMR (DMSO-*d_{6,}* 400MHz): 1.68-2.07 (m, 4H, CH₂); 3.35-3.41 (m, 1H, CH₂); 3.49-3.56 (m, 1H, CH₂); 3.71-3.83 (m, 2H, CH₂); 4.90-5.05 (m, 1H, CH-F); 5.69 (q, *J* 8.9 Hz, 2H, CH₂-CF₃); 7.43-7.50 (m, 2H, Ar); 7.73-7.76 (m, 1H, Ar); 8.19 (d, *J* 0.9 Hz, 1H, Ar); 8.33-8.36 (m, 1H, Ar); 9.17 (s, 1H, Ar); 9.59 (d, *J* 0.9 Hz, 1H, Ar). M/Z (M+H)⁺: 447

### Compound 217 : 3-Benzofuran-3-yl-1-(2,2,2-trifluoro-ethyl)-1H-pyrazolo[4,3-c]pyridin-6-yl]-(6,6-difluoro-1,4-oxazepan-4-yl)-methanone

Compound 217 was obtained according to General Procedure XIII, starting from lithium 3-(benzofuran-3-yl)-1-(2,2,2-trifluoroethyl)pyrazolo[4,3-c]pyridine-6-carboxylate R3 and 6,6-difluoro-1,4-oxazepane hydrochloride B58. In that specific case, 4 equiv. of diisopropylethylamine were used. Purification by flash chromatography (DCM/MeOH: 100/0 to 97/3) afforded 217 as a white powder in 31% yield. ¹H-NMR (DMSO-*d_{6,}* 400MHz): 3.61-3.66 (m, 1H, CH₂); 3.86-4.08 (m, 5H, CH₂); 4.25-4.42 (m, 2H, CH₂); 5.71 (q, *J* 8.9 Hz, 2H, CH₂-CF₃); 7.42-7.51 (m, 2H, Ar); 7.72-7.76 (m, 1H, Ar); 8.28-8.32 (m, 1H, Ar); 8.33-8.37 (m, 1H, Ar); 9.18 (s, 1H, Ar); 9.59 (bs, 1H, Ar). M/Z (M+H)+: 481

### Compound 218: [3-Benzofuran-3-yl-1-(2,2,2-trifluoro-ethyl)-1H-pyrazolo[4,3-c]pyridin-6-yl]-(8-endo-hydroxy-3-aza-bicyclo[3.2.1]oct-3-yl)-methanone

Compound 218 was obtained according to General Procedure XIII, starting from lithium 3-(benzofuran-3-yl)-1-(2,2,2-trifluoroethyl)pyrazolo[4,3-c]pyridine-6-carboxylate R3 and endo-3-aza-bicyclo[3.2.1]octan-8-ol B85. Purification by flash chromatography (DCM/MeOH: 100/0 to 95/5) afforded 218 as a white powder in 31% yield. ¹H-NMR (DMSO-*d_{6,}* 400MHz): 1.48-1.84 (m, 5H, CH, CH₂); 2.04 (bs, 1H, CH); 3.11 (dd, *J* 12.5, 2.5 Hz, 1H, N-CH₂); 3.30-3.35 (m, 1H, N-CH₂); 3.60 (d, *J* 12.1, 1H, N-CH₂); 3.86-3.91 (m, 1H, CH-OH); 4.17 (dd, *J* 12.5, 2.5 Hz, 1H, N-CH₂); 5.19 (d, *J*2.8 Hz, 1H, OH); 5.68 (q, *J* 9.0 Hz, 2H, CH₂-CF₃); 7.43-7.51 (m, 2H, Ar); 7.73-7.79 (m, 1H, Ar); 8.15 (d, *J* 1.0 Hz, 1H, Ar); 8.33-8.38 (m, 1H, Ar); 9.18 (s, 1H, Ar); 9.59 (d, *J* 1.0 Hz, 1H, Ar). M/Z (M+H)⁺: 471

### Compound 219 : [3-Benzofuran-3-yl-1-(2,2,2-trifluoro-ethyl)-1H-pyrazolo[4,3-c]pyridin-6-yl]-(4-hydroxy-2,2-dimethyl-piperidin-1-yl)-methanone

Compound 232 was obtained according to General Procedure XIII, starting from lithium 3-(benzofuran-3-yl)-1-(2,2,2-trifluoroethyl)pyrazolo[4,3-c]pyridine-6-carboxylate R3 and 2,2-dimethylpiperidin-4-ol B14. Purification by flash chromatography (DCM/MeOH: 100/0 to 95/5) afforded 219 as a white powder in 15% yield. ¹H-NMR (DMSO-*d_{6,}* 400MHz): 1.22-1.27 (m, 1H, CH₂); 1.35-1.44 (m, 1H, CH₂); 1.48 (s, 3H, CH₃); 1.62 (s, 3H, CH₃); 1.76-1.81 (m, 1H, CH₂); 1.84-1.92 (m, 1H, CH₂); 3.00-3.07 (m, 1H, CH₂); 3.41-3.49 (m, 1H, CH₂); 3.81-3.88 (m, 1H, CH-OH); 4.73 (d, *J*4.4 Hz, 1H, OH); 5.67 (q, *J* 9.0 Hz, 2H, CH₂-CF₃); 7.42-7.50 (m, 2H, Ar); 7.73-7.76 (m, 1H, Ar); 8.10 (bs, 1H, Ar); 8.32-8.35 (m, 1H, Ar); 9.15 (s, 1H, Ar); 9.54 (d, *J* 0.9 Hz, 1H, Ar). M/Z (M+H)⁺: 473

### Compound 220 : [3-Benzofuran-3-yl-1-(2,2-difluoro-ethyl)-1H-pyrazolo[4,3-c]pyridin-6-yl]-(3-endo-hydroxy-8-aza-bicyclo[3.2.1]oct-8-yl)-methanone

Compound 220 was obtained according to General Procedure XIII, starting from lithium 3-(benzofuran-3-yl)-1-(2,2-difluoroethyl)pyrazolo[4,3-c]pyridine-6-carboxylate R4 and nortropine B41 (1.2 equiv.). In that specific case, 1.5 equiv. of HATU was used intead of BOP. Purification by flash chromatography (DCM/MeOH: 100/0 to 94/6) afforded 220 as a white powder in 68% yield. ¹H-NMR (DMSO-*d_{6,}* 400MHz): 1.66-1.73 (m, 1H, CH₂); 1.79-1.95 (m, 3H, CH₂); 2.04-2.13 (m, 2H, CH₂); 2.19-2.32 (m, 2H, CH₂); 3.97-4.03 (m, 1H, CH); 4.49-4.56 (m, 1H, CH); 4.64 (d, *J* 2.3 Hz, 1H, OH); 4.65-4.71 (m, 1H, CH); 5.16 (dt, *J* 15.3, 3.2 Hz, 2H, CH₂-CHF₂); 6.59 (tt, *J* 54.4, 3.3 Hz, 1H, CHF₂); 7.40-7.48 (m, 2H, Ar); 7.70-7.76 (m, 1H, Ar); 8.18 (bs, 1H, Ar); 8.37-8.41 (m, 1H, Ar); 9.14 (s, 1H, Ar); 9.55 (d, *J* 1.0 Hz, 1H, Ar). M/Z (M+H)⁺: 453

### Compound 221 : [3-Benzofuran-3-yl-1-(2-fluoro-ethyl)-1H-pyrazolo[4,3-c]pyridin-6-yl]-(3-endo-hydroxy-8-aza-bicyclo[3.2.1]oct-8-yl)-methanone

Compound 221 was obtained according to General Procedure XIII, starting from lithium 3-(benzofuran-3-yl)-1-(2-fluoroethyl)pyrazolo[4,3-c]pyridine-6-carboxylate R5 and nortropine B41 (1.2 equiv.). In that specific case, 1.5 equiv. of HATU was used intead of BOP. Purification by preparative HPLC afforded 221 as a yellow powder in 47% yield. ¹H-NMR (DMSO-*d_{6,}* 400MHz): 1.65-1.73 (m, 1H, CH₂); 1.79-1.96 (m, 3H, CH₂); 2.04-2.13 (m, 2H, CH₂); 2.18-2.32 (m, 2H, CH₂); 3.97-4.03 (m, 1H, CH); 4.50-4.56 (m, 1H, CH); 4.65-4.71 (m, 1H, CH); 4.86-5.04 (m, 4H, CH₂-CH₂-F); 7.40-7.51 (m, 2H, Ar); 7.71-7.76 (m, 1H, Ar); 8.13 (bs, 1H, Ar); 8.37-8.41 (m, 1H, Ar); 9.12 (s, 1H, Ar); 9.54 (d, *J* 1.0 Hz, 1H, Ar). M/Z (M+H)⁺: 435

### Compound 222 : (3-Benzofuran-3-yl-1-propyl-1H-pyrazolo[4,3-c]pyridin-6-yl)-(3-endo-hydroxy-8-aza-bicyclo[3.2.1]oct-8-yl)-methanone

Compound 222 was obtained according to General Procedure XIII, starting from lithium 3-(benzofuran-3-yl)-1-propyl-pyrazolo[4,3-c]pyridine-6-carboxylate R6 and nortropine B41 (1.2 equiv.). In that specific case, 1.5 equiv. of HATU was used intead of BOP. Purification by flash chromatography (DCM/MeOH: 100/0 to 94/6) afforded 222 as a white powder in 33% yield. ¹H-NMR (DMSO-*d_{6,}* 400MHz): 0.92 (t, *J*7.2 Hz, 3H, CH₃); 1.65-1.73 (m, 1H, CH₂); 1.78-1.92 (m, 3H, CH₂); 1.97 (q, *J* 7.2 Hz, 2H, CH₂-CH₃); 2.05-2.13 (m, 2H, CH₂); 2.18-2.32 (m, 2H, CH₂); 4.00 (bs, 1H, CH); 4.49-4.58 (m, 3H, CH, CH₂); 4.62-4.65 (d, *J*2.1 Hz, 1H, OH); 4.65-4.71 (m, 1H, CH); 7.40-7.48 (m, 2H, Ar); 7.70-7.75 (m, 1H, Ar); 8.11 (bs, 1H, Ar); 8.35-8.39 (m, 1H, Ar); 9.09 (s, 1H, Ar); 9.51 (d, *J* 1.0 Hz, 1H, Ar). M/Z (M+H)⁺: 431

### Compound 223 : tert-butyl N-[4-[3-(6,8-difluoroimidazo[1,2-a]pyridin-3-yl)-1-(2,2,2-trifluoroethyl)pyrazolo[4,3-c]pyridine-6-carbonyl]-1,4-oxazepan-6-yl]carbamate

Compound 223 was obtained according to General Procedure XIII, starting from lithium 3-(6,8-difluoroimidazo[1,2-a]pyridin-3-yl)-1-(2,2,2-trifluoroethyl)pyrazolo[4,3-c]pyridine-6-carboxylate R2 and tert-butyl N-(1,4-oxazepan-6-yl)carbamate B86. Purification by flash chromatography (DCM/MeOH: 100/0 to 95/5) afforded 223 as a white powder in 62% yield. 1H-NMR (DMSO-d6, 400MHz): 0.89-1.24 (m, 9H, signal of a rotamer, CH₃); 1.41 (m, 9H, signal of a rotamer, CH₃); 3.38-4.22 (m, 9H, CH, CH₂); 5.70-5.86 (m, 2H, CH₂-CF₃); 6.68 (d, *J* 7.8 Hz, 1H, signal of a rotamer, NH); 6.90 (d, *J* 7.8 Hz, 1H, signal of a rotamer, NH); 7.81 (ddd, *J* 11.1, 8.9, 2.2 Hz, 1H, Ar); 8.26 (bs, 1H, signal of a rotamer, Ar); 8.27 (bs, 1H, signal of a rotamer, Ar); 8.51 (s, 1H, signal of a rotamer, Ar); 8.86 (s, 1H, signal of a rotamer, Ar); 9.45-9.51 (m, 1H, Ar); 9.64 (bs, 1H, signal of a rotamer, Ar); 9.66 (d, J 0.9 Hz, 1H, signal of a rotamer, Ar). M/Z (M+H)+: 596

### Compound 224 : [3-(benzofuran-3-yl)-1-(2,2,2-trifluoroethyl)pyrazolo[4,3-c]pyridin-6-yl]-(6-hydroxy-1,4-oxazepan-4-yl)methanone

Compound 224 was obtained according to General Procedure XIII, starting from lithium 3-(benzofuran-3-yl)-1-(2,2,2-trifluoroethyl)pyrazolo[4,3-c]pyridine-6-carboxylate R3 and 1,4-oxazepan-6-ol B87 (1.2 equiv.). Purification by flash chromatography (DCM/MeOH: 100/0 to 95/5) afforded 224 as a beige powder in 58% yield. ¹H-NMR (DMSO-*d_{6,}* 400MHz): 3.22-3.47 (m, 1H, CH₂); 3.49-3.82 (m, 6H, CH₂); 3.83-3.90 (m, 1H, CH₂, signal of a rotamer); 3.96-4.08 (m, 1H, CH₂); 4.22-4.28 (m, 1H, CH₂, signal of a rotamer); 5.00 (d, *J* 3.8 Hz, 1H, OH, major rotamer); 5.14 (d, *J* 3.8 Hz, 1H, OH, minor rotamer); 5.69 (m, 2H, CH₂-CF₃); 7.42-7.50 (m, 2H, Ar); 7.73-7.76 (m, 1H, Ar); 8.21 (bs, 1H, Ar, minor rotamer); 8.22 (bs, 1H, Ar, major rotamer); 8.33-8.36 (m, 1H, Ar); 9.17 (s, 1H, Ar, minor rotamer); 9.19 (s, 1H, Ar, major rotamer); 9.57 (d, *J* 1.0 Hz, 1H, Ar, minor rotamer); 9.59 (d, *J* 1.0 Hz, 1H, Ar, major rotamer). M/Z (M+H)⁺: 461

### Compound 225 : [3-(benzofuran-3-yl)-1-(2,2,2-trifluoroethyl)pyrazolo[4,3-c]pyridin-6-yl]-(4,4-difluoro-1-piperidyl)methanone

Compound 225 was obtained according to General Procedure XIII, starting from lithium 3-(benzofuran-3-yl)-1-(2,2,2-trifluoroethyl)pyrazolo[4,3-c]pyridine-6-carboxylate R3 and 4,4-difluoropiperidine B18. Purification by flash chromatography (Cyclohexane/AcOEt: 10/0 to 3/7) afforded 225 as a white powder in 63% yield. ¹H-NMR (DMSO-*d_{6,}* 400MHz): 1.99-2.18 (m, 4H, CH₂); 3.51-3.58 (m, 2H, CH₂); 3.79-3.88 (m, 2H, CH₂); 5.69 (q, *J* 8.9 Hz, 2H, CH₂-CF₃); 7.42-7.50 (m, 2H, Ar); 7.72-7.76 (m, 1H, Ar); 8.24 (d, *J* 0.9 Hz, 1H, Ar); 8.33-8.37 (m, 1H, Ar); 9.18 (s, 1H, Ar); 9.60 (d, *J* 0.9 Hz, 1H, Ar). M/Z (M+H)⁺: 465

### Compound 226 : [3-(benzofuran-3-yl)-1-(2,2,2-trifluoroethyl)pyrazolo[4,3-c]pyridin-6-yl]-(3,3-difluoro-1-piperidyl)methanone

Compound 226 was obtained according to General Procedure XIII, starting from lithium 3-(benzofuran-3-yl)-1-(2,2,2-trifluoroethyl)pyrazolo[4,3-c]pyridine-6-carboxylate R3 and 3,3-difluoropiperidine B90. Purification by flash chromatography (Cyclohexane/AcOEt: 10/0 to 3/7) afforded 226 as a white powder in 79% yield. ¹H-NMR (DMSO-*d_{6,}* 400MHz): 1.68-1.81 (m, 2H, CH₂); 2.04-2.19 (m, 2H, CH₂); 3.44-3.48 (m, 1H, N-CH₂); 3.72-3.79 (m, 1H, N-CH₂); 3.93 (t, *J* 11.9 Hz, 2H, signal of a rotamer); 4.05 (t, *J* 11.9 Hz, 2H, signal of a rotamer); 5.71 (q, *J* 8.9 Hz, 2H, CH₂-CF₃); 7.42-7.50 (m, 2H, Ar); 7.72-7.76 (m, 1H, Ar); 8.22 (bs, 1H, Ar, signal of a rotamer); 8.28 (bs, 1H, Ar, signal of a rotamer); 8.33-8.37 (m, 1H, Ar); 9.18 (s, 1H, Ar); 9.59-9.61 (m, 1H, Ar). M/Z (M+H)⁺: 465

### Compound 227 : [3-Benzofuran-3-yl-1-(2,2,2-trifluoro-ethyl)-1H-pyrazolo[4,3-c]pyridin-6-yl]-(3-hydroxy-piperidin-1-yl)-methanone

Compound 227 was obtained according to General Procedure XIII, starting from lithium 3-(benzofuran-3-yl)-1-(2,2,2-trifluoroethyl)pyrazolo[4,3-c]pyridine-6-carboxylate R3 and piperidin-3-ol B24. Purification by flash chromatography (DCM/MeOH: 100/0 to 95/5) afforded 227 as a white powder in 84% yield. ¹H-NMR (DMSO-*d_{6,}* 400MHz): 1.37-1.51 (m, 2H, CH₂); 1.60-1.69 (m, 1H, signal of a rotamer, CH₂); 1.77-1.95 (m, 3H, signal of a rotamer, CH₂); 2.87 (dd, *J* 12.2, 9.0 Hz, 1H, signal of a rotamer, N-CH₂); 2.97-3.08 (m, 1H, N-CH₂); 3.21-3.28 (m, 1H, signal of a rotamer, N-CH₂); 3.42-3.62 (m, 2H, N-CH₂); 3.91-4.00 (m, 1H, signal of a rotamer, CH-OH); 4.31 (dd, *J* 12.2, 3.8 Hz, 1H, signal of a rotamer, CH-OH); 4.78 (d, *J* 3.7 Hz, 1H, signal of a rotamer, OH); 5.03 (d, *J* 4.1 Hz, 1H, signal of a rotamer, OH); 5.64-5.73 (m, 2H, CF₃-CH₂); 7.42-7.50 (m, 2H, Ar); 7.73-7.76 (m, 1H, Ar); 8.14 (bs, 1H, Ar, signal of a rotamer); 8.16 (bs, 1H, Ar, signal of a rotamer); 8.33-8.37 (m, 1H, Ar); 9.17 (s, 1H, Ar, signal of a rotamer); 9.18 (s, 1H, Ar, signal of a rotamer); 9.57 (d, *J* 0.9 Hz, 1H, Ar, signal of a rotamer); 9.58 (d, *J* 0.9 Hz, 1H, Ar, signal of a rotamer). M/Z (M+H)⁺: 445

### Compound 228 : [3-(benzofuran-3-yl)-1-(2,2,2-trifluoroethyl)pyrazolo[4,3-c]pyridin-6-yl]-(4-hydroxyazepan-1-yl)methanone

Compound 228 was obtained according General Procedure XIII, starting from lithium 3-(benzofuran-3-yl)-1-(2,2,2-trifluoroethyl)pyrazolo[4,3-c]pyridine-6-carboxylate R3 and azepan-4-ol B50. Purification by flash chromatography (DCM/MeOH: 100/0 to 95/5) then by preparative HPLC afforded 228 as a white powder in 41% yield. ¹H-NMR (DMSO-*d_{6,}* 400MHz): 1.49-1.83 (m, 5H, CH₂); 1.90-2.03 (m, 1H, CH₂); 3.23-3.80 (m, 5H, N-CH₂, CH); 4.55 (d, *J* 3.9 Hz, 1H, OH, signal of a rotamer); 4.60 (d, *J* 3.9 Hz, 1H, OH, signal of a rotamer); 5.68 (q, *J* 8.9 Hz, 2H, CH₂-CF₃); 7.42-7.50 (m, 2H, Ar); 7.72-7.76 (m, 1H, Ar); 8.12 (bs, 1H, Ar, signal of a rotamer); 8.14 (bs, 1H, Ar, signal of a rotamer); 8.33-8.37 (m, 1H, Ar); 9.16 (s, 1H, Ar, signal of a rotamer); 9.17 (s, 1H, Ar, signal of a rotamer); 9.57 (d, *J* 1.0 Hz, 1H, Ar). M/Z (M+H)⁺: 459

### Compound 229 : [3-(benzofuran-3-yl)-1-(2,2,2-trifluoroethyl)pyrazolo[4,3-c]pyridin-6-yl]-(6,6-dideuterio-1,4-oxazepan-4-yl)methanone

Compound 229 was obtained according to General Procedure XIII, starting from lithium 3-(benzofuran-3-yl)-1-(2,2,2-trifluoroethyl)pyrazolo[4,3-c]pyridine-6-carboxylate R3 and 6,6-dideuterio-1,4-oxazepane hydrochloride B91. In that specific case, 4 equiv. of diisopropylethylamine were used. Purification by flash chromatography (DCM/MeOH: 100/0 to 95/5) afforded 229 as a white powder in 36% yield. ¹H-NMR (DMSO-*d_{6,}* 400MHz): 3.47-3.54 (m, 2H, CH₂); 3.64-3.68 (m, 1H, CH₂); 3.70-3.73 (m, 1H, CH₂); 3.73-3.81 (m, 4H, CH₂); 5.69 (q, *J* 9.1 Hz, 2H, CH₂-CF₃); 7.42-7.49 (m, 2H, Ar); 7.72-7.76 (m, 1H, Ar); 8.19 (bs, 1H, Ar, signal of a rotamer); 8.20 (bs, 1H, Ar, signal of a rotamer); 8.33-8.37 (m, 1H, Ar); 9.17 (s, 1H, Ar, signal of a rotamer); 9.18 (s, 1H, Ar, signal of a rotamer); 9.57 (d, *J* 1.1 Hz, 1H, Ar, signal of a rotamer); 9.59 (d, *J* 1.1 Hz, 1H, Ar, signal of a rotamer). M/Z (M+H)+: 447

### Compound 230 : [3-(benzofuran-3-yl)-1-(2,2,2-trifluoroethyl)pyrazolo[4,3-c]pyridin-6-yl]-[6-fluoro-1,4-oxazepan-4-yl]methanone

Compound 230 was obtained according to General Procedure XIII, starting from lithium 3-(benzofuran-3-yl)-1-(2,2,2-trifluoroethyl)pyrazolo[4,3-c]pyridine-6-carboxylate R3 and 6-fluoro-1,4-oxazepane hydrochloride B92. In that specific case, 4 equiv. of diisopropylethylamine were used. Purification by flash chromatography (DCM/MeOH: 100/0 to 95/5) then by preparative HPLC afforded 230 as a white powder in 22% yield. ¹H-NMR (DMSO-*d_{6,}* 400MHz): 3.46-3.55 (m, 1H, CH₂, signal of a rotamer); 3.59-3.69 (m, 2H, CH₂); 3.75-3.95 (m, 3H, CH₂); 3.97-4.15 (m, 2H, CH₂); 4.16-4.27 (m, 1H, CH₂, signal of a rotamer); 4.86-5.17 (m, 1H, CH-F); 5.61-5.80 (m, 2H, CH₂-CF₃); 7.42-7.50 (m, 2H, Ar); 7.72-7.77 (m, 1H, Ar); 8.24 (bs, 1H, Ar, signal of a rotamer); 8.25 (bs, 1H, Ar, signal of a rotamer); 8.33-8.37 (m, 1H, Ar); 9.16-9.18 (m, 1H, Ar); 9.59 (bs, 1H, Ar). M/Z (M+H)+: 463

### Compound 231 : 3-(benzofuran-3-yl)-N,N-di(cyclobutyl)-1-(2,2,2-trifluoroethyl)pyrazolo[4,3-c]pyridine-6-carboxamide

Compound 231 was obtained according to General Procedure XIII, starting from lithium 3-(benzofuran-3-yl)-1-(2,2,2-trifluoroethyl)pyrazolo[4,3-c]pyridine-6-carboxylate R3 and dicyclobutylamine B94. Purification by flash chromatography (DCM/MeOH: 100/0 to 95/5) afforded 231 as a white powder in 34% yield. ¹H-NMR (DMSO-*d_{6,}* 400MHz): 1.34-2.28 (m, 9H, CH₂); 2.50-3.09 (m, 3H, CH₂); 4.09-4.33 (m, 2H, N-CH); 5.68 (q, *J* 9.0 Hz, 2H, CH₂-CF₃); 7.42-7.50 (m, 2H, Ar); 7.72-7.77 (m, 1H, Ar); 8.10 (bs, 1H, Ar); 8.32-8.37 (m, 1H, Ar); 9.17 (s, 1H, Ar); 9.55 (d, *J* 1.0 Hz, 1H, Ar). M/Z (M+H)+: 469

### Compound 232 : 3-(benzofuran-3-yl)-N,N-di(cyclopropyl)-1-(2,2,2-trifluoroethyl)-pyrazolo[4,3-c]pyridine-6-carboxamide

Compound 232 was obtained according to General Procedure XIII, starting from lithium 3-(benzofuran-3-yl)-1-(2,2,2-trifluoroethyl)pyrazolo[4,3-c]pyridine-6-carboxylate R3 and dicyclopropylamine B95. Purification by flash chromatography (DCM/MeOH: 100/0 to 95/5) then by preparative HPLC afforded 232 as a white powder in 30% yield. ¹H-NMR (DMSO-*d_{6,}* 400MHz): 0.32-0.87 (m, 8H, CH₂); 2.75-2.83 (m, 2H, N-CH); 5.68 (q, *J* 9.0 Hz, 2H, CH₂-CF₃); 7.42-7.50 (m, 2H, Ar); 7.72-7.77 (m, 1H, Ar); 8.13 (bs, 1H, Ar); 8.32-8.37 (m, 1H, Ar); 9.15 (s, 1H, Ar); 9.53 (d, *J* 1.1 Hz, 1H, Ar). M/Z (M+H)+: 441

### Compound 233 : 3-(benzofuran-3-yl)-N,N-diisopropyl-1-(2,2,2-trifluoroethyl)pyrazolo[4,3-c]pyridine-6-carboxamide

Compound 233 was obtained according to General Procedure XIII, starting from lithium 3-(benzofuran-3-yl)-1-(2,2,2-trifluoroethyl)pyrazolo[4,3-c]pyridine-6-carboxylate R3 and diisopropylamine B96. Purification by flash chromatography (DCM/MeOH: 100/0 to 95/5) then by preparative HPLC afforded 233 as a white powder in 24% yield. ¹H-NMR (DMSO-*d_{6,}* 400MHz): 1.12 (d, 6.7 Hz, 6H, CH₃); 1.50 (d, 6.7 Hz, 6H, CH₃); 3.64 (heptuplet, *J* 6.7 Hz, 2H, N-CH); 5.67 (q, *J* 9.1 Hz, 2H, CH₂-CF₃); 7.42-7.50 (m, 2H, Ar); 7.72-7.77 (m, 1H, Ar); 8.04 (bs, 1H, Ar); 8.32-8.37 (m, 1H, Ar); 9.16 (s, 1H, Ar); 9.55 (d, *J* 1.1 Hz, 1H, Ar). M/Z (M+H)+: 445

### Compound 234 : 3-(benzofuran-3-yl)-N-ethyl-N-isopropyl-1-(2,2,2-trifluoroethyl)-pyrazolo[4,3-c]pyridine-6-carboxamide

Compound 234 was obtained according to General Procedure XIII, starting from lithium 3-(benzofuran-3-yl)-1-(2,2,2-trifluoroethyl)pyrazolo[4,3-c]pyridine-6-carboxylate R3 and N-ethylisopropylamine B97. Purification by preparative HPLC afforded 234 as a white powder in 13% yield. ¹H-NMR (DMSO-*d_{6,}* 400MHz): 1.00 (t, *J*6.9 Hz, 3H, CH₃, signal of a rotamer); 1.14 (d, *J* 6.6 Hz, 6H, CH₃, signal of a rotamer); 1.25 (t, *J* 6.9 Hz, 3H, CH₃, signal of a rotamer); 1.30 (d, *J* 6.6 Hz, 6H, CH₃, signal of a rotamer); 3.24 (q, *J* 6.9 Hz, 2H, CH₂, signal of a rotamer); 3.42 (q, *J* 6.9 Hz, 2H, CH₂, signal of a rotamer); 3.81 (heptuplet, 6.6 Hz, 1H, CH, signal of a rotamer); 4.53 (heptuplet, 6.6 Hz, 1H, CH, signal of a rotamer); 5.67 (q, *J* 9.1 Hz, 2H, CH₂-CF₃); 7.42-7.50 (m, 2H, Ar); 7.72-7.77 (m, 1H, Ar); 8.09 (bs, 1H, Ar, signal of a rotamer); 8.11 (bs, 1H, Ar, signal of a rotamer); 8.32-8.37 (m, 1H, Ar); 9.17 (s, 1H, Ar); 9.55 (bs, 1H, Ar, signal of a rotamer); 9.58 (bs, 1H, Ar, signal of a rotamer). M/Z (M+H)+: 431

### Compound 235: 3-(benzofuran-3-yl)-N-cyclohexyl-N-ethyl-1-(2,2,2-trifluoroethyl)pyrazolo[4,3-c]pyridine-6-carboxamide

Compound 235 was obtained according to General Procedure XIII, starting from lithium 3-(benzofuran-3-yl)-1-(2,2,2-trifluoroethyl)pyrazolo[4,3-c]pyridine-6-carboxylate R3 and N-cyclohexyl-N-ethylamine B98. Purification by flash chromatography (DCM/MeOH: 100/0 to 95/5) then by preparative HPLC afforded 235 as a white powder in 23% yield. ¹H-NMR (DMSO-*d_{6,}* 400MHz): 0.85-1.09 (m, 3H, CH₂, CH₃); 1.14-1.26 (m, 2H, CH₂, CH₃); 1.31-1.87 (m, 8H, CH₂); 3.20-3.39 (m, 3H, N-CH, N-CH₂, signal of a rotamer); 3.44 (q, *J* 7.1 Hz, 2H, N-CH₂, signal of a rotamer); 4.13-4.24 (m, 1H, N-CH, signal of a rotamer); 5.67 (q, *J* 9.1 Hz, 2H, CH₂-CF₃); 7.42-7.50 (m, 2H, Ar); 7.72-7.77 (m, 1H, Ar); 8.08 (bs, 1H, Ar); 8.32-8.37 (m, 1H, Ar); 9.16 (s, 1H, Ar, signal of a rotamer); 9.18 (s, 1H, Ar, signal of a rotamer); 9.55 (bs, 1H, Ar, signal of a rotamer); 9.58 (bs, 1H, Ar, signal of a rotamer). M/Z (M+H)+: 471

### Compound 236 : [3-(benzofuran-3-yl)-1-(2,2,2-trifluoroethyl)pyrazolo[4,3-c]pyridin-6-yl]-(8-hydroxy-8-isopropyl-3-azabicyclo[3.2.1]octan-3-yl)methanone

Compound 236 was obtained according to General Procedure XIII, starting from lithium 3-(benzofuran-3-yl)-1-(2,2,2-trifluoroethyl)pyrazolo[4,3-c]pyridine-6-carboxylate R3 and *endo*-(8-*exo*-isopropyl)-3-azabicyclo[3.2.1]octan-8-ol B100. Purification by flash chromatography (DCM/MeOH: 100/0 to 95/5) afforded 236 as a white powder in 13% yield. ¹H-NMR (DMSO-*d_{6,}* 400MHz): 0.81 (t, *J* 6.8 Hz, 3H, CH₃); 0.87 (t, *J* 6.8 Hz, 3H, CH₃); 1.45-1.72 (m, 5H, CH, CH₂); 1.75-1.80 (m, 1H, CH); 2.01-2.07 (m, 1H, CH); 3.11 (dd, *J* 12.2, 2.7 Hz, 1H, N-CH₂); 3.39 (d, *J* 12.2 Hz, 1H, N-CH₂); 3.64 (d, *J* 12.2 Hz, 1H, N-CH₂); 4.18 (dd, *J* 12.2, 2.7 Hz, 1H, N-CH₂); 4.49 (s, 1H, OH); 5.63-5.74 (m, 2H, CH₂-CF₃); 7.42-7.50 (m, 2H, Ar); 7.72-7.76 (m, 1H, Ar); 8.14 (bs, 1H, Ar); 8.33-8.36 (m, 1H, Ar); 9.18 (s, 1H, Ar); 9.58 (d, *J* 1.0 Hz, 1H, Ar). M/Z (M+H)⁺: 513

### Compound 237 : 3-[6-(1,4-oxazepane-4-carbonyl)-1-(2,2,2-trifluoroethyl)pyrazolo[4,3-c]pyridin-3-yl]benzofuran-5-carboxamide

Compound 237 was obtained according to General Procedure XIII, starting from 3-[6-(1,4-oxazepane-4-carbonyl)-1-(2,2,2-trifluoroethyl)pyrazolo[4,3-c]pyridin-3-yl]benzofuran-5-carboxylic acid 129 and ammonia, 0.5N in dioxane. Purification by flash chromatography (DCM/MeOH: 100/0 to 98/2, KP-NH silica) afforded 237 as a white powder in 56% yield. ¹H-NMR (DMSO-*d_{6,}* 400MHz): 1.78 (quint, *J* 5.9 Hz, 1H, CH₂); 1.95 (quint, *J* 5.9 Hz, 1H, CH₂); 3.48-3.53 (m, 2H, CH₂); 3.64-3.68 (m, 1H, N-CH₂); 3.70-3.81 (m, 5H, N-CH_{2,} O-CH₂); 5.72 (d, *J*9.0 Hz, 2H, CF₃-CH₂); 7.40 (bs, 1H, NH); 7.79 (d, *J* 8.7 Hz, 1H, Ar); 7.98 (dd, *J* 8.7, 1.8 Hz, 1H, Ar); 8.03 (bs, 1H, NH); 8.19 (bs, 1H, Ar, signal of a rotamer); 8.20 (bs, 1H, Ar, signal of a rotamer); 8.84 (bs, 1H, Ar); 9.22 (s, 1H, Ar, signal of a rotamer); 9.23 (s, 1H, Ar, signal of a rotamer); 9.56 (d, *J* 1.0 Hz, 1H, Ar, signal of a rotamer); 9.58 (d, *J* 1.0 Hz, 1H, Ar, signal of a rotamer). M/Z (M+H)⁺: 488

### General Procedure XIV : Synthesis of compounds or compounds B from compounds BB (scheme 1&3)

To a solution of compound BB or of the corresponding Compound (1 equiv.) in DCM (0.1M) was added TFA (6 equiv.). The reaction mixture was stirred 72h at rt. After concentration, the crude was dissolved in MeOH, then HCl, 0.5M in Et₂O, was added. The obtained precipitate was triturated in Et₂O to afford the target compound.

### Alternative 1 :

To a solution of compound BB or of the corresponding Compound (1 equiv.) in DCM (0.1M) was added TFA (6 equiv.). The reaction mixture was stirred 72h at rt. After concentration, the crude was dissolved in a minimum amount of MeOH, absorbed on a SCX-2 resin (5g cartridge). The resin was washed 3 times with MeOH. The compound was then released by addition of a ammonia solution, 3.5M in MeOH, concentration, and was used as such in the next step.

### Alternative 2 :

To a solution of compound K (1 equiv.) in DCM (0.1M) was added TFA (20 equiv.). The reaction mixture was stirred 1h at rt. The reaction mixture was poured at 0°C on a saturated sodium bicarbonate solution, then extracted with DCM. The organic phase was filtered on a hydrophobic cartridge then purified by flash chromatography to afford the compound.

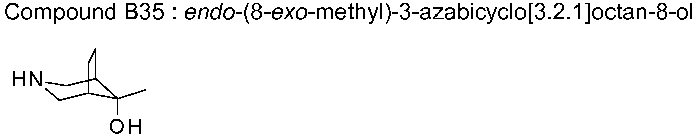

Compound B35 was obtained according to General Procedure XIV, Alternative 1, starting from tert-butyl 8-*endo*-hydroxy-8-exo-methyl-3-azabicyclo[3.2.1]octane-3-carboxylate BB1. Trituration in Et₂O afforded B35 as a white powder in quantitative yield. M/Z (M+H)⁺: 142

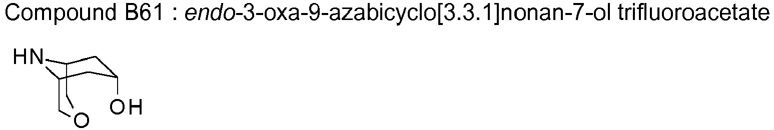

Compound B61 was obtained according to General Procedure XIV, Alternative 1, starting from tert-butyl 7-*endo*-hydroxy-3-oxa-9-azabicyclo[3.3.1]nonane-9-carboxylate BB6. Trituration in Et₂O afforded B61 as a beige powder in quantitative yield. M/Z (M+H)⁺: 144

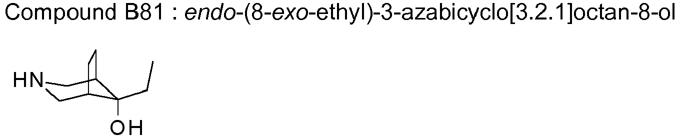

Compound B81 was obtained according to General Procedure XIV, Alternative 1, starting from tert-butyl 8-*endo*-hydroxy-8-*exo*-ethyl-3-azabicyclo[3.2.1]octane-3-carboxylate BB2. Trituration in Et₂O afforded B81 as a white powder in 88% yield. M/Z (M+H)⁺: 155

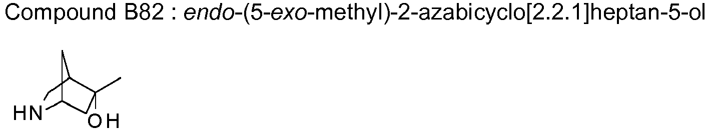

Compound B82 was obtained according to General Procedure XIV, Alternative 1, starting from tert-butyl 8-hydroxy-8-*exo*-methyl-3-azabicyclo[3.2.1]octane-3-carboxylate BB5. Trituration in Et₂O afforded B82 in 79% yield. M/Z (M+H)⁺: 128

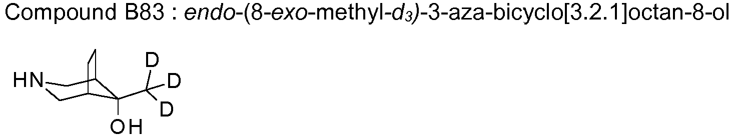

Compound B83 was obtained according to General Procedure XIV, Alternative 1, starting from tert-butyl 8-*endo*-hydroxy-8-*exo*-methyl-*d₃*-3-azabicyclo[3.2.1]octane-3-carboxylate BB3. Trituration in Et₂O afforded B83 in quantitative yield. M/Z (M+H)⁺: 145

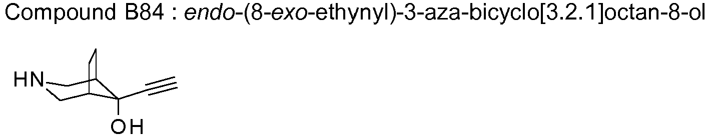

Compound B84 was obtained according to General Procedure XIV, Alternative 1, starting from tert-butyl 8-*endo*-hydroxy-8-*exo*-ethynyl-3-azabicyclo[3.2.1]octane-3-carboxylate BB4. Trituration in Et₂O afforded B84 as a beige powder in quantitative yield. M/Z (M+H)⁺: 152

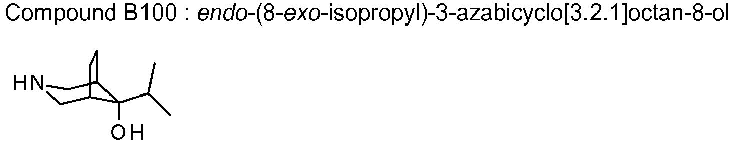

Compound B100 was obtained according to General Procedure XIV, Alternative 1, starting from tert-butyl 8-*endo*-hydroxy-8-*exo*-isopropyl-3-azabicyclo[3.2.1]octane-3-carboxylate BB7. Trituration in Et₂O afforded B100 as a yellow powder in quantitative yield. M/Z (M+H)⁺: 170

### Compound 238 : (3,8-Diaza-bicyclo[3.2.1]oct-3-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone

Compound 238 was obtained according to the following procedure : to a solution of 3-[3-(6,8-Difluoro-imidazo[1,2-a]pyridin-3-yl)-1-(2,2,2-trifluoro-ethyl)-1H-pyrazolo[4,3-c]pyridine-6-carbonyl]-3,8-diaza-bicyclo[3.2.1]octane-8-carboxylic acid tert-butyl ester 200 in DCM (0.1M) was added TFA (15 equiv.). The reaction mixture was stirred 72h at rt. After completion of the reaction, the reaction mixture was diluted with DCM, washed with a saturated sodium bicarbonate solution. The aqueous phase was basified with NaOH 6N, then extracted with DCM. The combined organic phases were dried over magnesium sulfate then concentrated. The obtained solid was triturated in diethyl ether to afford 256 as a beige powder in 45% yield. ¹H-NMR (DMSO-*d_{6,}* 400MHz): 1.47-1.71 (m, 4H, CH₂); 2.85 (dd, *J* 12.7, 1.4 Hz, 1H, CH₂); 3.09-3.19 (m, 3H, CH₂); 3.36-3.42 (m, 1H, CH₂), 4.16 (dd, *J* 12.7, 1.9 Hz, 1H, CH₂); 5.67 (m, *J* 8.4 Hz, 2H, CH₂-CF₃); 7.72 (ddd, *J* 11.1, 9.1, 2.1 Hz, 1H, Ar); 8.08 (d, *J* 0.9 Hz, 1H, Ar); 8.78 (s, 1H, Ar); 9.37-9.41 (m, 1H, Ar); 9.57 (d, *J*0.9 Hz, 1H, Ar). M/Z (M+H)⁺: 492

### Compound 239 : (2,5-Diaza-bicyclo[2.2.1]hept-2-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone hydrochloride

Compound 239 was obtained according to General Procedure XIV, starting from 5-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,5-diaza-bicyclo[2.2.1]heptane-2-carboxylic acid tert-butyl ester 194. After completion of the reaction, the reaction mixture was diluted with DCM, washed with a saturated sodium bicarbonate solution, dried over magnesium sulfate. The crude was dissolved in MeOH, then HCl, 0.5M in Et₂O, was added. The obtained precipitate was triturated in Et₂O to afford 239 as a white powder in 27% yield. ¹H-NMR (DMSO-*d_{6,}* 400MHz): 1.92 (t, *J* 10.5 Hz, 1H, CH₂); 2.15 (dd, *J* 11.2, 10.5 Hz, 1H, CH₂); 3.50-3.58 (m, 1H, CH₂); 3.70 (d, *J* 12.5 Hz, 1H, signal of a rotamer, CH₂); 3.78 (d, *J* 12.5 Hz, 1H, signal of a rotamer, CH₂); 4.11 (bs, 1H, CH₂); 4.45 (bs, 1H, signal of a rotamer, CH₂); 4.50 (bs, 1H, signal of a rotamer CH₂); 5.00 (s, 1H, signal of a rotamer, CH₂); 5.24 (s, 1H, signal of a rotamer, CH₂); 5.77-5.97 (m, 2H, CH₂-CF₃); 7.82 (ddd, *J* 11.0, 9.1, 1.8 Hz, 1H, Ar); 8.48 (s, 1H, signal of a rotamer, Ar); 8.60 (s, 1H, signal of a rotamer, Ar); 8.88 (s, 1H, signal of a rotamer, Ar); 8.90 (s, 1H, signal of a rotamer, Ar); 8.97 (bs, 1H, signal of a rotamer, NH₂⁺); 9.19 (s, 1H, signal of a rotamer, NH₂⁺); 9.46-9.51 (m, 1H, Ar); 9.51-9.61 (bs, 1H, NH₂⁺); 9.67 (d, *J* 1.0 Hz, 1H, signal of a rotamer, Ar) 9.70 (d, *J* 1.0 Hz, 1H, signal of a rotamer, Ar). M/Z (M+H)⁺: 478

### Compound 240 : (3-Amino-azepan-1-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone hydrochloride

Compound 240 was obtained according to General Procedure XIV, starting from {1-[3-(6,8-Difluoro-imidazo[1,2-a]pyridin-3-yl)-1-(2,2,2-trifluoro-ethyl)-1H-pyrazolo[4,3-c]pyridine-6-carbonyl]-azepan-3-yl}-carbamic acid tert-butyl ester 211. Trituration in Et₂O afforded 240 as a yellow powder in 94% yield. ¹H-NMR (DMSO-*d_{6,}* 400MHz): 1.39-2.15 (m, 6H, CH₂); 3.26-3.65 (m, 4H, CH₂); 3.76-4.18 (m, (m, 1H, CH₂); 5.72-5.87 (m, 2H, CH₂-CF₃); 7.79-7.87 (m, 1H, Ar); 8.00-8.21 (m, 3H, NH₃⁺); 8.23 (bs, 1H, major rotamer, Ar); 8.29 (bs, 1H, minor rotamer, Ar); 8.86 (s, 1H, minor rotamer, Ar); 8.89 (s, 1H, major rotamer, Ar); 9.44-9.52 (m, 1H, Ar); 9.68 (d, *J* 1.0 Hz, 1H, major rotamer, Ar); 9.71 (d, *J* 1.0 Hz, 1H, minor rotamer, Ar). M/Z (M+H)⁺: 494

### Compound 241 : (6-Amino-1,4-oxazepan-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone hydrochloride

Compound 241 was obtained according to General Procedure XIV, starting from tert-butyl N-[4-[3-(6,8-difluoroimidazo[1,2-a]pyridin-3-yl)-1-(2,2,2-trifluoroethyl)pyrazolo[4,3-c]pyridine-6-carbonyl]-1,4-oxazepan-6-yl]carbamate 223. Trituration in Et₂O afforded 241 as a yellow powder in 57% yield. ¹H-NMR (DMSO-*d_{6,}* 400MHz): 3.16-3.65 (m, 3H, CH₂); 3.82-4.23 (m, 6H, CH₂); 5.72-5.87 (m, 2H, CH₂-CF₃); 7.78-1.88 (m, 1H, Ar); 8.22-8.32 (m, 3H, NH₃⁺); 8.31 (s, 1H, signal of a rotamer, Ar); 8.35 (s, 1H, signal of a rotamer, Ar); 8.84 (s, 1H, signal of a rotamer, Ar); 8.89 (s, 1H, signal of a rotamer, Ar); 9.47-9.51 (m, 1H, Ar); 9.68 (bs, 1H, signal of a rotamer, Ar); 9.73 (bs, 1H, signal of a rotamer, Ar). M/Z (M+H)⁺: 496

### Exemple 242 : (6,6-dideuterio-1,4-oxazepan-4-yl)-[3-(1H-indol-3-yl)-1-(2,2,2-trifluoroethyl)pyrazolo[4,3-c]pyridin-6-yl]methanone

Exemple 242 was obtained according to General Procedure XIV, Alternative 2, starting from [tert-butyl 3-[6-(6,6-dideuterio-1,4-oxazepane-4-carbonyl)-1-(2,2,2-trifluoroethyl)pyrazolo[4,3-c]pyridin-3-yl]indole-1-carboxylate K16. Purification by flash chromatography (DCM/MeOH: 10/0 to 9/1) afforded 242 as a beige powder in 44% yield. ¹H-NMR (DMSO-*d_{6,}* 400MHz): 3.47-3.54 (m, 2H, CH₂); 3.64-3.68 (m, 1H, CH₂); 3.70-3.73 (m, 1H, CH₂); 3.73-3.81 (m, 4H, CH₂); 5.60 (q, *J* 9.0 Hz, 2H, CH₂-CF₃); 7.15-7.25 (m, 2H, Ar); 7.50 (d, *J* 7.9 Hz, 1H, Ar); 8.08 (bs, 1H, signal of a rotamer, Ar); 8.09 (bs, 1H, signal of a rotamer, Ar); 8.34 (d, *J* 7.9 Hz, 1H, Ar); 8.44 (d, *J* 2.9 Hz, 1H, signal of a rotamer, Ar); 8.45 (d, *J* 2.9 Hz, 1H, signal of a rotamer, Ar); 9.52 (d, *J* 0.9 Hz, 1H, signal of a rotamer, Ar); 9.54 (d, *J* 0.9 Hz, 1H, signal of a rotamer, Ar); 11.78 (bs, 1H, NH). M/Z (M+H)⁺: 446

### Exemple 243 : [3-(1H-indol-3-yl)-1-(2,2,2-trifluoroethyl)pyrazolo[4,3-c]pyridin-6-yl]-(8-oxa-3-azabicyclo[3.2.1]octan-3-yl)methanone

Exemple 263 was obtained according to General Procedure XIV, Alternative 2, starting from tert-butyl 3-[6-(8-oxa-3-azabicyclo[3.2.1]octane-3-carbonyl)-1-(2,2,2-trifluoroethyl)pyrazolo[4,3-c]pyridin-3-yl]indole-1-carboxylate K17. Purification by flash chromatography (DCM/MeOH: 10/0 to 9/1) afforded 243 as an brown oil in 38% yield. ¹H-NMR (DMSO-*d_{6,}* 400MHz): 1.73-1.94 (m, 4H, CH₂); 3.03-3.09 (m, 1H, N-CH₂); 3.30-3.35 (m, 1H, N-CH₂); 3.42-3.48 (m, 1H, N-CH₂); 4.20-4.27 (m, 2H, N-CH₂, O-CH); 4.41-4.45 (m, 1H, O-CH); 7.17 (td, *J*7.9, 1.3 Hz, 1H, Ar); 7.23 (td, *J*7.9, 1.3 Hz, 1H, Ar); 7.49 (d, *J* 7.9 Hz, 1H, Ar); 8.12 (bs, 1H, Ar); 8.34 (d, *J* 7.9 Hz, 1H, Ar); 8.44 (d, *J* 2.8 Hz, 1H, Ar); 9.55 (d, *J* 1.0 Hz, 1H, Ar); 11.71 (bs, 1H, NH). M/Z (M+H)⁺: 456

### General Procedure XV : Synthesis of compounds H from compounds H (scheme1) ; Synthesis of final compounds (scheme 1)

A solution of compound H or of the corresponding Compound in a dioxane/NaOH, 1N in water, mixture (1/1, 0.1M) was heated 20 min at 80°C. The reaction mixture was cooled down at 0°C, then diluted with water. The resulting precipitate was filtered, then purified by flash chromatography to afford the target compound.

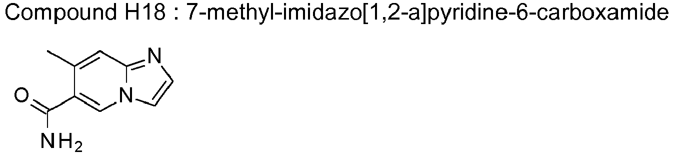

Compound H18 was obtained according to General Procedure XV, starting from 7-methyl-imidazo[1,2-a]pyridine-6-carbonitrile H17. Purification by flash chromatography (DCM/MeOH: 10/0 to 9/1) afforded H18 as a white powder in 66% yield. M/Z (M+H)⁺: 176

### Compound 244 : 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carboxylic acid amide

Compound 244 was obtained according to General Procedure XV, starting from 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carbonitrile 47. Purification by flash chromatography (DCM/MeOH: 10/0 to 9/1) afforded 244 as a white powder in 41% yield. ¹H-NMR (DMSO-*d_{6,}* 400MHz): 1.54-1.64 (m, 6H, CH₂); 1.75-1.81 (m, 2H, CH₂); 2.63 (s, 3H, CH₃); 3.36-3.38 (m, 2H, N-CH₂); 3.63-3.66 (m, 2H, N-CH₂); 5.70 (q, *J*8.9 Hz, 2H, CF₃-CH₂); 7.58 (bs, 1H, NH); 7.75 (bs, 1H, Ar); 8.09 (bs, 1H, NH); 8.16 (bs, 1H, Ar); 8.76 (s, 1H, Ar); 9.60 (d, *J* 1.0 Hz, 1H, Ar); 9.86 (bs, 1H, Ar). M/Z (M+H)⁺: 500

### Compound 245 : 3-[6-(Azepane-1-carbonyl)-1-propyl-1H-pyrazolo[4,3-c]pyridin-3-yl]-imidazo[1,2-a]pyridine-6-carboxylic acid amide

Compound 245 was obtained according to General Procedure XV, starting from 3-[6-(Azepane-1-carbonyl)-1-propyl-1H-pyrazolo[4,3-c]pyridin-3-yl]-imidazo[1,2-a]pyridine-6-carbonitrile 43. Purification by flash chromatography (DCM/MeOH: 10/0 to 9/1) afforded 245 as a white powder in 36% yield. ¹H-NMR (DMSO-*d_{6,}* 400MHz): 0.92 (t, *J* 7.3 Hz, 3H, CH₂-CH₂-CH₃); 1.53-1.65 (m, 6H, CH₂); 1.74-1.81 (m, 2H, CH₂); 1.99 (sextuplet, *J*7.3 Hz, 2H, CH₂-CH₂-CH₃); 3.35-3.41 (m, 2H, N-CH₂); 3.61-3.65 (m, 2H, N-CH₂); 4.57 (t, *J* 7.3 Hz, 2H, CH₂-CH₂-CH₃); 7.60 (bs, 1H, NH); 7.82 (dd, *J* 9.4, 0.9 Hz, 1H, Ar); 7.87 (dd, *J* 9.4, 1.7 Hz, 1H, Ar); 8.03 (d, *J* 1.1 Hz, 1H, Ar); 8.20 (bs, 1H, NH); 8.73 (s, 1H, Ar); 9.54 (d, *J* 1.1 Hz, 1H, Ar); 10.07 (dd, *J* 1.7, 0.9 Hz, 1H, Ar). M/Z (M+H)⁺: 446

### Compound 246 : 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carboxylic acid amide

Compound 246 was obtained according to General Procedure XV, starting from 3-[6-(1,4-Oxazepane-4-carbonyl)-1 -(2,2,2-trifluoro-ethyl)-1H-pyrazolo[4,3-c]pyridin-3-yl]-imidazo[1,2-a]pyridine-6-carbonitrile 81. No further purification was necessary to afford 246 as a white powder in 34% yield. ¹H-NMR (DMSO-*d_{6,}* 400MHz): 1.75-1.81 (m, 1H, CH₂); 1.92-1.98 (m, 1H, CH₂); 3.49-3.53 (m, 2H, N-CH₂); 3.64-3.67 (m, 1H, N-CH₂); 3.71-3.80 (m, 5H, N-CH₂, O-CH₂); 5.71 (q, *J*9.0 Hz, 2H, CH₂-CF₃); 7.63 (bs, 1H, NH); 7.85 (dd, *J* 9.3, 0.8 Hz, 1H, Ar); 7.90 (dd, *J* 9.3, 1.6 Hz, 1H, Ar); 8.15 (bs, 1H, NH); 8.22 (d, *J* 1.0 Hz, 1H, signal of a rotamer, Ar); 8.22 (d, *J* 1.0 Hz, 1H, signal of a rotamer, Ar); 8.80 (s, 1H, signal of a rotamer, Ar); 8.81 (s, 1H, signal of a rotamer, Ar); 9.61 (d, *J* 1.0 Hz, 1H, signal of a rotamer, Ar); 9.63 (d, *J* 1.0 Hz, 1H, signal of a rotamer, Ar); 10.00 (dd, *J* 1.6, 0.8 Hz, 1H, signal of a rotamer, Ar); 10.01 (dd, *J* 1.6, 0.8 Hz, 1H, signal of a rotamer, Ar). M/Z (M+H)⁺: 488

### Compound 247 : 3-[6-(8-Oxa-3-aza-bicyclo[3.2.1]octane-3-carbonyl)-1-(2,2,2-trifluoroethyl)-1H-pyrazolo[4,3-c]pyridin-3-yl]-imidazo[1,2-a]pyridine-6-carboxylic acid amide

Compound 247 was obtained according to General Procedure XV, starting from 3-[6-(8-Oxa-3-aza-bicyclo[3.2.1]octane-3-carbonyl)-1-(2,2,2-trifluoro-ethyl)-1H-pyrazolo[4,3-c]pyridin-3-yl]-imidazo[1,2-a]pyridine-6-carbonitrile 84. Purification by preparative HPLC afforded 247 as a white powder in 27% yield. ¹H-NMR (DMSO-*d_{6,}* 400MHz): 1.75-1.92 (m, 4H, CH₂); 3.06-3.09 (m, 1H, N-CH₂); 3.31-3.42 (m, 2H, N-CH₂); 4.21-4.26 (m, 2H, N-CH₂, O-CH); 4.43-4.45 (m, 1H, O-CH); 5.71 (q, *J*9.0 Hz, 2H, signal of a rotamer, CH₂-CF₃); 5.72 (q, *J* 9.0 Hz, 2H, signal of a rotamer, CH₂-CF₃); 7.63 (bs, 1H, NH); 7.85 (dd, *J* 9.4, 0.8 Hz, 1H, Ar); 7.90 (dd, *J* 9.4, 1.6 Hz, 1H, Ar); 8.15 (bs, 1H, NH); 8.24 (bs, 1H, Ar); 8.81 (s, 1H, Ar); 9.63 (d, *J*0.9 Hz, 1H, Ar); 10.01 (dd, *J* 1.6, 0.8 Hz, 1H, Ar). M/Z (M+H)⁺: 500

### General Procedure XVI : Synthesis of compounds W from F (scheme 1)

Under argon atmosphere, to a solution of compound F (1 equiv.) in a mixture anhydrous DMF/triethylamine (0.1M, 5/2) was added ethynyltrimethylsilane (1.2 equiv.). The solution was degassed with argon bubbling for 5min, then Cul (10 mol%) and PdCl₂(PPH₃)₂ (5 mol%) were added. The reaction mixture was heated overnight at 80°C. The reaction mixture was diluted with AcOEt, washed with a saturated sodium bicarbonate solution and brine. The organic phase was filtered on an hydrophobic cartridge then concentrated. The resulting oil was purified by flash chromatography to afford compound W.

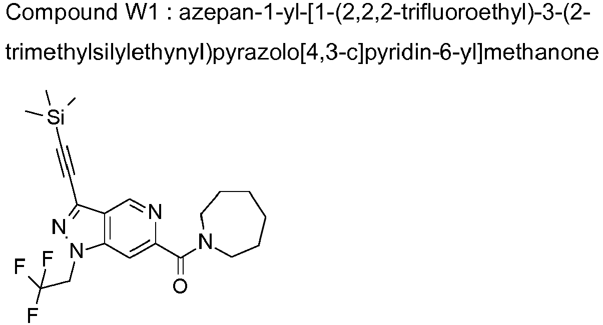

Compound W1 was obtained according to General Procedure XVI, starting from azepan-1-yl-[3-bromo-1-(2,2,2-trifluoroethyl)pyrazolo[4,3-c]pyridin-6-yl]methanone F3. Purification by flash chromatography (Cyclohexane/AcOEt: 10/0 to 4/6) afforded W1 as an orange oil in 95% yield. M/Z (M+H)⁺: 423

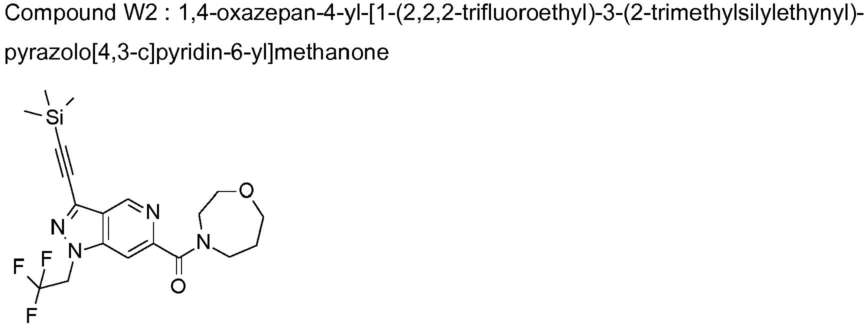

Compound W2 was obtained according to General Procedure XVI, starting from 1,4-oxazepan-4-yl -[3-bromo-1-(2,2,2-trifluoroethyl)pyrazolo[4,3-c]pyridin-6-yl]methanone F7. Purification by flash chromatography (Cyclohexane/AcOEt: 10/0 to 3/7) afforded W2 as an orange oil in 47% yield. M/Z (M+H)⁺: 425

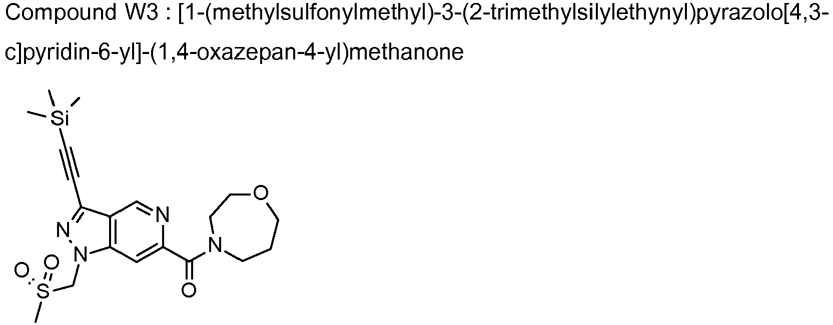

Compound W3 was obtained according to General Procedure XVI, starting from 3-bromo-1-(methylsulfonylmethyl)pyrazolo[4,3-c]pyridin-6-yl]-(1,4-oxazepan-4-yl)methanone F25. Purification by flash chromatography (Cyclohexane/AcOEt: 10/0 to 3/7) afforded W3 as an orange oil in 47% yield. M/Z (M+H)⁺: 425

### General Procedure XVII : Synthesis of compounds W from W (scheme 1)

To a solution of compound W (1 equiv.) in MeOH (0.15M) was added potassium carbonate (25 mol%). The reaction mixture was stirred overnight at rt. The reaction mixture was concentrated, the resulting solid was dissolved in DCM, washed with water. The organic phase was filtered on an hydrophobic cartridge then concentrated. The resulting solid was purified by flash chromatography to afford compound W.

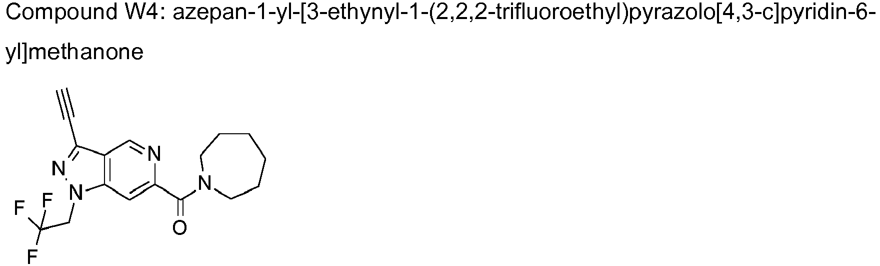

Compound W4 was obtained according to General Procedure XVII, starting from azepan-1-yl-[1-(2,2,2-trifluoroethyl)-3-(2-trimethylsilylethynyl)pyrazolo[4,3-c]pyridin-6-yl]methanone W1. Purification by flash chromatography (Cyclohexane/AcOEt: 10/0 to 5/5) afforded W4 as a white powder in 88% yield. M/Z (M+H)⁺: 351

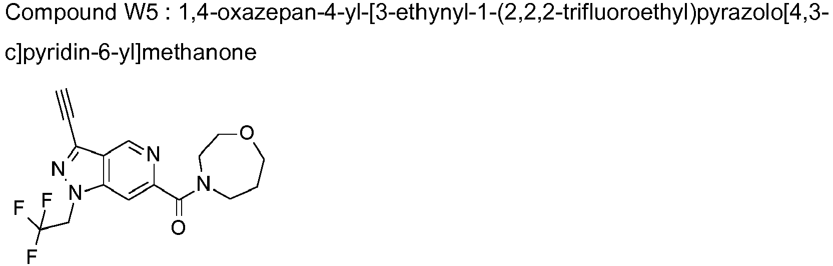

Compound W5 was obtained according to General Procedure XVII, starting from 1,4-oxazepan-4-yl-[1-(2,2,2-trifluoroethyl)-3-(2-trimethylsilylethynyl)pyrazolo[4,3-c]pyridin-6-yl]methanone W2. Purification by flash chromatography (DCM/MeOH: 100/0 to 95/5) afforded W5 as a white powder in 55% yield. M/Z (M+H)⁺: 353

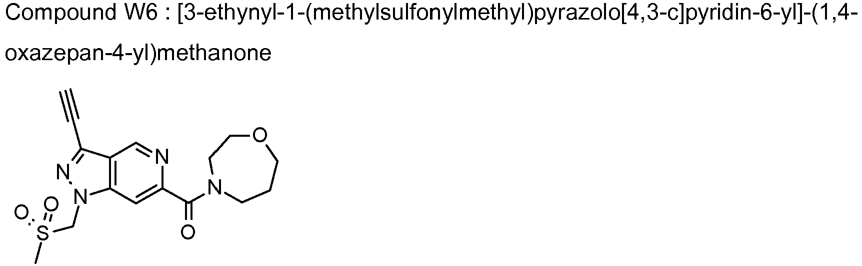

Compound W6 was obtained according to General Procedure XVII, starting from [1-(methylsulfonylmethyl)-3-(2-trimethylsilylethynyl)pyrazolo[4,3-c]pyridin-6-yl]-(1,4-oxazepan-4-yl)methanone W3. Purification by flash chromatography (DCM/MeOH: 100/0 to 95/5) afforded W6 as a white powder in 74% yield. M/Z (M+H)⁺: 363

### General Procedure XVIII : Synthesis of compounds from W (scheme 1)

To a solution of compound W (1 equiv.) in DMF (0.1M) was added potassium carbonate (1.5 equiv.). 1-Aminopyrazin-1-ium 2,4,6-trimethylbenzenesulfonate (2 equiv.) was added by portions over 1h at 0°C. The reaction mixture was heated for a defined time at a defined temperature. If needed, 2 additional equiv. of 1-aminopyrazin-1-ium 2,4,6-trimethylbenzenesulfonate were added. The reaction mixture was heated again. The reaction mixture was diluted with AcOEt, washed with a saturated sodium bicarbonate solution. The aqueous phase was extracted 2 times with AcOEt. The combined organic phase were washed with brine and filtered on an hydrophobic cartridge, then concentrated. The resulting solid was purified by flash chromatography to afford the compound.

### Compound 248 : Azepan-1-yl-[3-pyrazolo[1,5-a]pyrazin-3-yl-1-(2,2,2-trifluoro-ethyl)-1H-pyrazolo[4,3-c]pyridin-6-yl]-methanone

Compound 248 was obtained according to General Procedure XVIII, starting from azepan-1-yl-[3-ethynyl-1-(2,2,2-trifluoroethyl)pyrazolo[4,3-c]pyridin-6-yl]methanone W4 (1 addition of 1-Aminopyrazin-1-ium 2,4,6-trimethylbenzenesulfonate, overnight, 90°C). Purification by flash chromatography (DCM/MeOH: 100/0 to 95/5) afforded 248 as a beige powder in 23% yield. ¹H-NMR (DMSO-*d_{6,}* 400MHz): 1.52-1.65 (m, 6H, CH₂); 1.74-1.81 (m, 2H, CH₂); 3.35-3.39 (m, 2H, N-CH₂); 3.60-3.67 (m, 2H, N-CH₂); 5.68 (q, *J* 9.1 Hz, 2H, CF₃-CH₂); 8.11 (m, 2H, Ar); 8.95 (dd, *J*4.7, 1.4 Hz, 1H, Ar); 9.19 (s, 1H, Ar); 9.65 (d, *J* 1.0 Hz); 9.73 (d, *J* 1.4 Hz, 1H, Ar). M/Z (M+H)⁺: 444

### Compound 249 : 1,4-Oxazepan-4-yl-[3-pyrazolo[1,5-a]pyrazin-3-yl-1-(2,2,2-trifluoroethyl)-1H-pyrazolo[4,3-c]pyridin-6-yl]-methanone

Compound 249 was obtained according to General Procedure XVIII, starting from 1,4-oxazepan-4-yl-[3-ethynyl-1-(2,2,2-trifluoroethyl)pyrazolo[4,3-c]pyridin-6-yl]methanone X2 (2 additions of 1-Aminopyrazin-1-ium 2,4,6-trimethylbenzenesulfonate, overnight, 50°C). Purification by flash chromatography (DCM/MeOH: 100/0 to 95/5) afforded 249 as a beige powder in 42% yield. ¹H-NMR (DMSO-*d_{6,}* 400MHz): 1.78 (quint, *J* 5.8 Hz, 1H, CH₂); 1.95 (quint, *J* 5.8 Hz, 1H, CH₂); 3.47-3.55 (m, 2H, N-CH₂); 3.63-3.69 (m, 1H, N-CH₂); 3.69-3.81 (m, 5H, N-CH₂, O-CH₂); 5.69 (q, *J*9.1 Hz, 2H, CF₃-CH₂); 8.12 (d, *J*4.8 Hz, 1H, Ar); 8.16-8.18 (m, 1H, Ar); 8.96 (dd, *J* 4.8, 1.5 Hz, 1H, Ar); 9.19 (s, 1H, signal of a rotamer, Ar); 9.20 (s, 1H, signal of a rotamer, Ar); 9.66 (d, *J* 1.1 Hz, 1H, signal of a rotamer, Ar); 9.67 (d, *J* 1.1 Hz, 1H, signal of a rotamer, Ar); 9.72-9.74 (m, 1H, Ar). M/Z (M+H)⁺: 446

### Compound 250 : (1-Methanesulfonylmethyl-3-pyrazolo[1,5-a]pyrazin-3-yl-1H-pyrazolo[4,3-c]pyridin-6-yl)-(1,4-oxazepan-4-yl)methanone

Compound 250 was obtained according to General Procedure XVIII, starting from [3-ethynyl-1-(methylsulfonylmethyl)pyrazolo[4,3-c]pyridin-6-yl]-(1,4-oxazepan-4-yl)methanone X3 (3 additions of 1-Aminopyrazin-1-ium 2,4,6-trimethylbenzenesulfonate, overnight, 80°C). The reaction mixture was heated overnight at 80°C. Purification by flash chromatography (DCM/MeOH: 100/0 to 94/6) afforded 250 as a beige powder in 48% yield. ¹H-NMR (DMSO-*d_{6,}* 400MHz): 1.78 (quint, *J* 5.9 Hz, 1H, CH₂); 1.95 (quint, *J* 5.9 Hz, 1H, CH₂); 3.16 (s, 3H, CH₃); 3.47-3.53 (m, 2H, N-CH₂); 3.63-3.68 (m, 1H, N-CH₂); 3.69-3.81 (m, 5H, N-CH₂, O-CH₂); 6.28 (s, 2H, SO₂-CH₂); 8.12 (d, *J* 4.8 Hz, 1H, Ar); 8.17 (d, *J* 1.1 Hz, 1H, signal of a rotamer, Ar); 8.18 (d, *J* 1.1 Hz, 1H, signal of a rotamer, Ar); 8.96 (dd, *J* 4.8, 1.5 Hz, 1H, Ar); 9.22 (s, 1H, signal of a rotamer, Ar); 9.23 (s, 1H, signal of a rotamer, Ar); 9.67 (d, *J* 1.1 Hz, 1H, signal of a rotamer, Ar); 9.69 (d, *J* 1.1 Hz, 1H, signal of a rotamer, Ar); 9.77 (d, *J* 1.5 Hz, 1H, signal of a rotamer, Ar); 9.78 (d, *J* 1.5 Hz, 1H, signal of a rotamer, Ar). M/Z (M+H)⁺: 456

### General Procedure XIX : Synthesis of compounds X from compounds F (scheme 1)

To a solution of compound F (1 equiv.) in dioxane (0.15M) were added benzophenone imine (1.3 equiv.), cesium carbonate (2.4 equiv.), and XantPhos Pd G3 precatalyst (10 mol%). The reaction mixture was stirred overnight at 90°C. The reaction mixture was cooled down, diluted with AcOEt, washed with water. The aqueous layer was extracted twice with DCM. The combined organic layers were dried over sodium sulfate and concentrated.

The resulting oil was dissolved in dioxane (0.05M). HCl 4N in dioxane (5 equiv.) was added dropwise at 0°C. The reaction mixture was stirred 1h at 0°C. The reaction mixture was diluted with water, extracted twice with diethyl ether. The aqueous layer was basified with solid sodium bicarbonate to pH 9-10 then extracted 3 times with DCM. The combined organic layers were dried over magnesium sulfate and concentrated. The resulting oil was purified by flash chromatography to afford compound X.

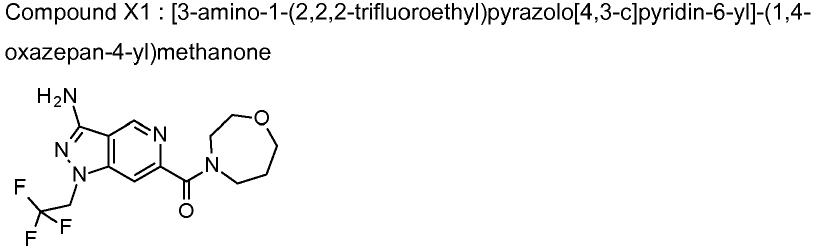

Compound X1 was obtained according to General Procedure XIX, starting from [3-bromo-1-(2,2,2-trifluoroethyl)pyrazolo[4,3-c]pyridin-6-yl]-(1,4-oxazepan-4-yl)methanone F7. Purification by flash chromatography (DCM/MeOH: 10/0 to 9/1) afforded X1 as a yellow solid in 64% yield. M/Z (M+H)⁺: 344

### General Procedure XX : Synthesis of compounds Z from compounds X (scheme 1)

To a degassed suspension of compound X (1 equiv.) in dioxane (0.1M) were added a halogeno-nitrobenzene, cesium carbonate Y (2.4 equiv.) and BrettPhos Pd G1 precatalyst (10 mol%). The reaction mixture was stirred 24h at 90°C. The reaction mixture was diluted with DCM, washed with water, the aqueous layer was extracted with DCM. The combined organic layers were dried over sodium sulfate, concentrated. The resulting residue was purified by flash chromatography to afford the compound Z.

### Alternative 1 :

To a degassed suspension of compound Y (1 equiv.) in dioxane (0.1M) were added a 2-bromo-nitrobenzene, cesium carbonate (2.4 equiv.) and XantPhos Pd G3 precatalyst (10 mol%). The reaction mixture was stirred 2h at 90°C. The reaction mixture was diluted with DCM, washed with water, the aqueous layer was extracted with DCM. The combined organic layers were dried over sodium sulfate, concentrated. The resulting residue was purified by flash chromatography to afford the compound Z.

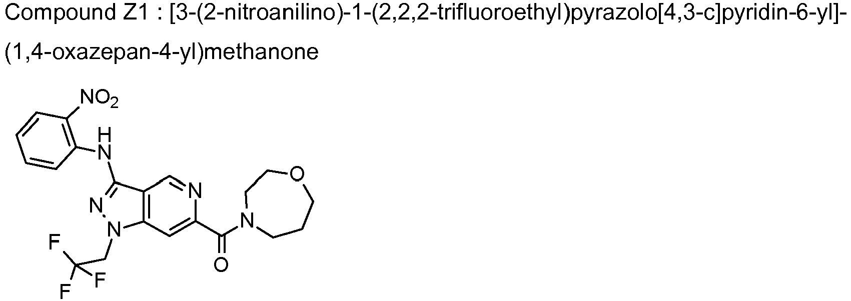

Compound Z1 was obtained according to General Procedure XX, starting from [3-amino-1-(2,2,2-trifluoroethyl)pyrazolo[4,3-c]pyridin-6-yl]-(1,4-oxazepan-4-yl)methanone X1 and 2-bromonitrobenzene Y1. Purification by flash chromatography (DCM/AcOEt: 10/0 to 5/5) afforded the compound Z1 as an orange powder in 75% yield. M/Z (M+H)⁺: 465

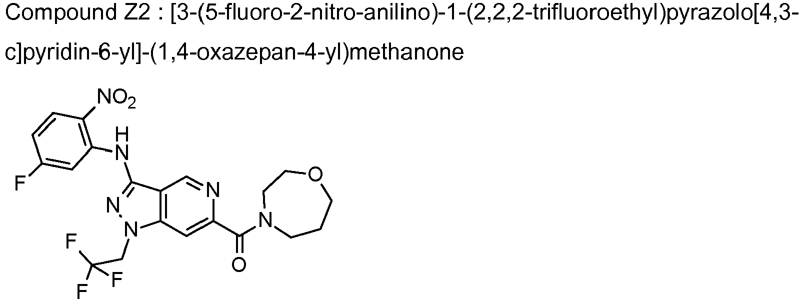

Compound Z2 was obtained according to General Procedure XX, Alternative 1, starting from [3-amino-1-(2,2,2-trifluoroethyl)pyrazolo[4,3-c]pyridin-6-yl]-(1,4-oxazepan-4-yl)methanone X1 and 2-bromo-4-fluoronitrobenzene Y2. Purification by flash chromatography (DCM/MeOH: 10/0 to 9/1) afforded the compound Z2 as an orange powder in 59% yield. M/Z (M+H)⁺: 483

### General Procedure XXI : Synthesis of compounds from compounds Z (scheme 1)

To a suspension of compound Z (1 equiv.), iron powder (10 equiv.), and ammonium chloride (10 equiv.) in isopropanol (0.1M) was added formic acid (final concentration :0.05M, isopropanol/formic acid 1/1). The reaction mixture was stirred 2h at 90°C. The reaction mixture was diluted with isopropanol, filtered on a glass filter, then quenched with water and extracted with DCM. The resulting residue was purified by flash chromatography to afford the compound.

### Compound 251 : [3-(benzimidazol-1-yl)-1-(2,2,2-trifluoroethyl)pyrazolo[4,3-c]pyridin-6-yl]-(1,4-oxazepan-4-yl)methanone

Compound 251 was obtained according to General Procedure XXI, starting from [3-(2-nitroanilino)-1-(2,2,2-trifluoroethyl)pyrazolo[4,3-c]pyridin-6-yl]-(1,4-oxazepan-4-yl)methanone Z1. Purification by flash chromatography (DCM/MeOH: 100/0 to 95/5) afforded 251 as a pale pink powder in 64% yield. ¹H-NMR (DMSO-*d_{6,}* 400MHz): 1.77 (quint, *J*5.8 Hz, 1H, CH₂); 1.94 (quint, *J*5.8 Hz, 1H, CH₂); 3.47-3.53 (m, 2H, N-CH₂); 3.63-3.66 (m, 1H, N-CH₂); 3.69-3.81 (m, 5H, N-CH_{2,} O-CH₂); 5.70 (q, *J*9.0 Hz, 2H, CF₃-CH₂); 7.41 (td, *J* 7.8, 1.0 Hz, 1H, Ar); 7.46 (td, *J* 7.8, 1.0 Hz, 1H, Ar); 7.86 (d, *J* 7.8 Hz, 1H, Ar); 8.12 (d, *J* 7.8 Hz, 1H, Ar); 8.24 (bs, 1H, Ar); 9.14 (s, 1H, signal of a rotamer, Ar); 9.15 (s, 1H, signal of a rotamer, Ar); 9.46 (d, *J* 1.0 Hz, 1H, signal of a rotamer, Ar); 9.48 (d, *J* 1.0 Hz, 1H, signal of a rotamer, Ar). M/Z (M+H)⁺: 445

### Compound 252 : [3-(6-fluorobenzimidazol-1-yl)-1-(2,2,2-trifluoroethyl)pyrazolo[4,3-c]pyridin-6-yl]-(1,4-oxazepan-4-yl)methanone

Compound 252 was obtained according to General Procedure XXI, starting from [3-(5-fluoro-2-nitro-anilino)-1-(2,2,2-trifluoroethyl)pyrazolo[4,3-c]pyridin-6-yl]-(1,4-oxazepan-4-yl)methanone Z2. Purification by flash chromatography (DCM/MeOH: 10/0 to 9/1) afforded 252 as a white powder in 23% yield. ¹H-NMR (DMSO-*d_{6,}* 400MHz): 1.77 (quint, *J* 5.8 Hz, 1H, CH₂); 1.94 (quint, *J*5.8 Hz, 1H, CH₂); 3.47-3.53 (m, 2H, N-CH₂); 3.62-3.66 (m, 1H, N-CH₂); 3.69-3.81 (m, 5H, N-CH_{2,} O-CH₂); 5.72 (q, *J* 9.0 Hz, 2H, CF₃-CH₂); 7.28 (td, *J* 9.3, 2.5 Hz, 1H, Ar); 7.88 (dd, *J*8.8, 4.9 Hz, 1H, Ar); 8.12 (dt, *J*9.3, 2.1 Hz, 1H, Ar); 8.23 (bs, 1H, Ar); 9.18 (s, 1H, signal of a rotamer, Ar); 9.19 (s, 1H, signal of a rotamer, Ar); 9.49 (d, *J* 1.0 Hz, 1H, signal of a rotamer, Ar); 9.51 (d, *J* 1.0 Hz, 1H, signal of a rotamer, Ar). M/Z (M+H)⁺: 463

### General Procedure XXII : Synthesis of compounds BB from compounds BA (scheme 3)

To a solution of compound BA (1 equiv.) in THF (0.1M) at 0°C was added a solution of the corresponding Grignard reagent RMgX (1.5 equiv.) was added dropwise. The reaction mixture was stirred 30 min at rt. The reaction mixture was quenched by addition of a saturated ammonium chloride solution, then extracted with DCM. The organic phase was filtered on an hydrophobic cartridge, then concentrated. The resulting solid was purified by flash chromatography to afford compound BB.

### Alternative 1 :

To a solution of compound BA (1 equiv.) in MeOH (0.1M) at 0°C was added NaBH₄ (1.5 equiv.). The reaction mixture was stirred overnight at rt. The reaction mixture was quenched by addition of water, then extracted with DCM. The organic phase was fried over magnesium sulfate then concentrated. The resulting solid was purified by flash chromatography to afford compound BB.

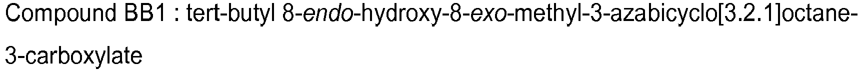

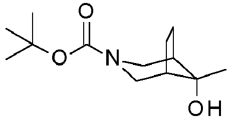

Compound BB1 was obtained according to General Procedure XXII, starting from tert-butyl 8-oxo-3-azabicyclo[3.2.1]octane-3-carboxylate BA1 and a solution of MeMgBr, 3M in Et₂O. Purification by flash chromatography (Cyclohexane/AcOEt: 10/0 to 5/5) afforded BB1 as a white powder in 86% yield. M/Z (M+H-*t*Bu)⁺: 186

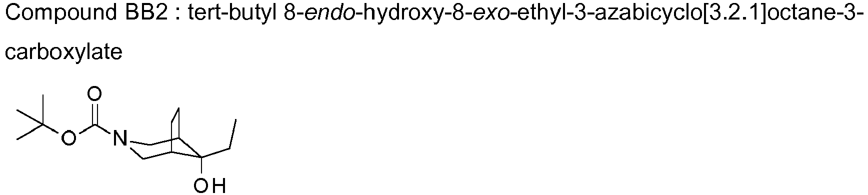

Compound BB2 was obtained according to General Procedure XXII, starting from tert-butyl 8-oxo-3-azabicyclo[3.2.1]octane-3-carboxylate BA1 and a solution of EtMgBr, 1M in THF. Purification by flash chromatography (Cyclohexane/AcOEt: 10/0 to 5/5) afforded BB2 as a white powder in 29% yield. M/Z (M+H-*t*Bu)⁺: 200

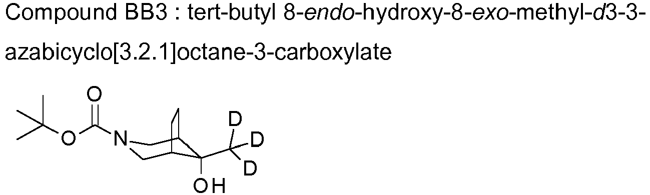

Compound BB3 was obtained according to General Procedure XXII, starting from tert-butyl 8-oxo-3-azabicyclo[3.2.1]octane-3-carboxylate BA1 and a solution of CD₃MgI, 1M in Et₂O. Purification by flash chromatography (Cyclohexane/AcOEt: 10/0 to 5/5) afforded BB3 as a white powder in 87% yield. M/Z (M+H-*t*Bu)⁺: 189

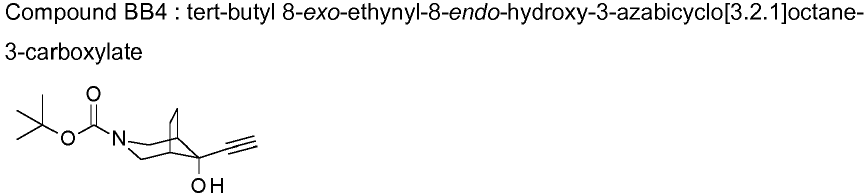

Compound BB4 was obtained according to General Procedure XXII, starting from tert-butyl 8-oxo-3-azabicyclo[3.2.1]octane-3-carboxylate BA1 and a solution of ethynyl magnesium bromide, 0.5M in THF. Purification by flash chromatography (Cyclohexane/AcOEt: 10/0 to 8/2) afforded BB4 as a white powder in quantitative yield. M/Z (M+H-*t*Bu)⁺: 196

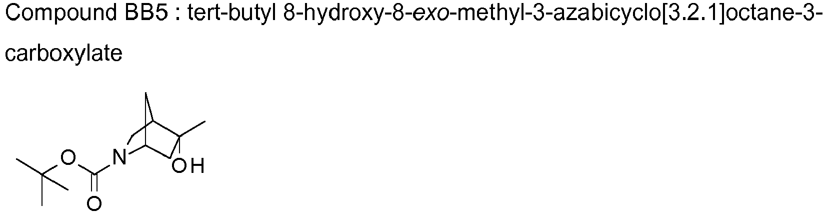

Compound BB5 was obtained according to General Procedure XXII, starting from tert-butyl 5-oxo-2-azabicyclo[2.2.1]heptane-2-carboxylate BA2 and a solution of MeMgBr, 3M in Et₂O. Purification by flash chromatography (Cyclohexane/AcOEt: 10/0 to 5/5) afforded BB5 as a colorless oil in 81% yield. M/Z (M+H)⁺: 228

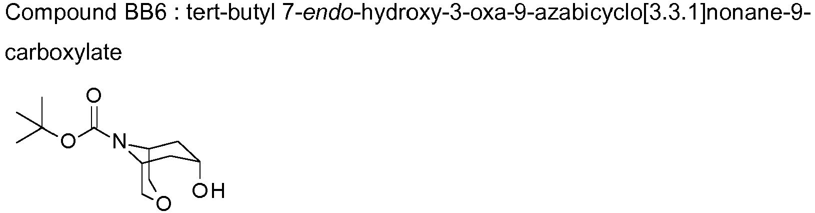

Compound BB6 was obtained according to General Procedure XXII, Alternative 1, starting from tert-butyl 7-oxo-3-oxa-9-azabicyclo[3.3.1]nonane-9-carboxylate BA3. Purification by flash chromatography (Cyclohexane/AcOEt: 10/0 to 5/5) afforded BB6 as a colorless oil in 84% yield. M/Z (M+H-*t*Bu)⁺: 188

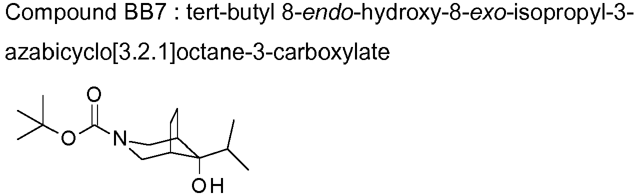

Compound BB7 was obtained according to General Procedure XXII, starting from tert-butyl 8-oxo-3-azabicyclo[3.2.1]octane-3-carboxylate BA1 and a solution of isopropyl magnesium chloride, 2M in THF. In that specific case, isopropyl magnesium chloride, 2M in THF, and zinc chloride, 0.5M in THF, (10mol%) were pre-mixed and stirred for 3h at 0°C before adding compound BA1. Purification by flash chromatography (Cyclohexane/AcOEt: 10/0 to 4/6) afforded BB7 as a beige powder in 24% yield. M/Z (M+H-*t*Bu)⁺: 214

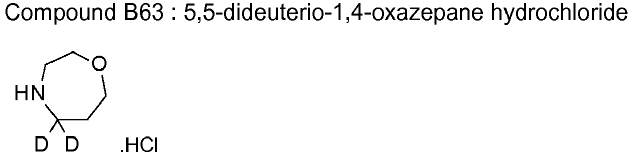

To a solution of 1,4-oxazepan-5-one (1 equiv.) in DMF (0.1M) at 0°C, was added sodium hydride (1.2 equiv.). The reaction mixture was stirred 15 min at 0°C, then p-methoxybenzyl chloride (1.1 equiv.) was added. The reaction mixture was stirred 3h at rt, then quenched with a saturated bicarbonate solution. The reaction mixture was extracted with AcOEt, the organic phase was washed with brine, dried over magnesium sulfate then concentrated. The resulting solid was purified by flash chromatography (DCM/MeOH: 100/0 to 95/5) to afford 4-[(4-methoxyphenyl)methyl]-1,4-oxazepan-5-one as a colorless oil in 93% yield. M/Z (M+H)⁺: 236

To a solution of 4-[(4-methoxyphenyl)methyl]-1,4-oxazepan-5-one (1 equiv.) in THF (0.1M) at 0°C, was added LiAID4 (3 equiv.). The reaction mixture was stirred overnight at rt. The reaction mixture was quenched with x µl of water (x is the number of mg of LiAID4), 3x µl of a 3N NaOH solution, x µl of water, then diluted with Et2O. The resulting white suspension was filtered off, the filtrate was concentrated then purified by flash chromatography (DCM/MeOH: 10/0 to 9/1) to afford 5,5-dideuterio-4-[(4-methoxyphenyl)methyl]-1,4-oxazepane as a colorless oil in quantitative yield. M/Z (M+H)⁺: 224

To a solution of 5,5-dideuterio-4-[(4-methoxyphenyl)methyl]-1,4-oxazepane (1 equiv.) in DCE (0.2M) at 0°C, was added 1-chloroethyl chloroformate (2.5 equiv.). The reaction mixture was refluxed overnight, then concentrated. The resulting solid was dissolved in MeOH (0.1M). The reaction mixture was refluxed 3h. HCl, 1.25N in MeOH, (1 equiv.) was added, then the reaction mixture was concentrated. The resulting residue was triturated in Et₂O, then dried overnight under vacuum to afford 5,5-dideuterio-1,4-oxazepane hydrochloride B63 as a light brown powder in 90% yield. M/Z (M+H)⁺: 104

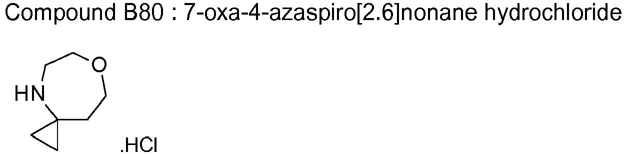

To a solution of 1,4-oxazepan-5-one (1 equiv.) in DMF (0.1M) at 0°C, was added sodium hydride (1.2 equiv.). The reaction mixture was stirred 15 min at 0°C, then p-methoxybenzyl chloride (1.1 equiv.) was added. The reaction mixture was stirred 3h at rt, then quenched with a saturated bicarbonate solution. The reaction mixture was extracted with AcOEt, the organic phase was washed with brine, dried over magnesium sulfate then concentrated. The resulting solid was purified by flash chromatography (DCM/MeOH: 100/0 to 95/5) to afford 4-[(4-methoxyphenyl)methyl]-1,4-oxazepan-5-one as a colorless oil in 93% yield. M/Z (M+H)⁺: 236

To a solution of 4-[(4-methoxyphenyl)methyl]-1,4-oxazepan-5-one (1 equiv.) in THF (0.1M) were added dropwise Ti(OiPr)4 (2 equiv.) followed by EtMgBr, 1M in THF, (4 equiv.). The reaction mixture was stirred overnight at rt. 4 additional equiv. of EtMgBr, 1M in THF, were added. The reaction mixture was stirred 72h at rt. The reaction mixture was quenched with water. The resulting precipitate was filtered, dissolved in AcOEt. The organic phase was washed with a saturated sodium bicarbonate solution and brine, dried over magnesium sulfate, concentrated. The resulting solid was purified by flash chromatography (Cyclohexane/AcOEt: 10/0 to 4/6) to afford 4-[(4-methoxyphenyl)methyl]-7-oxa-4-azaspiro[2.6]nonane as a colorless oil in 60% yield. M/Z (M+H)⁺: 248

To a solution of 4-[(4-methoxyphenyl)methyl]-7-oxa-4-azaspiro[2.6]nonane (1 equiv.) in DCE (0.2M) at 0°C, was added 1-chloroethyl chloroformate (2.5 equiv.). The reaction mixture was refluxed overnight, then concentrated. The resulting solid was dissolved in MeOH (0.1M). The reaction mixture was refluxed 3h. HCl, 1.25N in MeOH, (1 equiv.) was added, then the reaction mixture was concentrated. The resulting residue was triturated in Et₂O, then dried overnight under vacuum to afford 7-oxa-4-azaspiro[2.6]nonane hydrochloride B80 as an orange oil in 67% yield. M/Z (M+H)⁺: 128

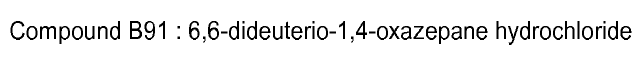

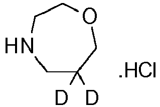

To a solution of 1,4-oxazepan-5-one (1 equiv.) in DMF (0.1M) at 0°C, was added sodium hydride (1.2 equiv.). The reaction mixture was stirred 15 min at 0°C, then p-methoxybenzyl chloride (1.1 equiv.) was added. The reaction mixture was stirred 3h at rt, then quenched with a saturated bicarbonate solution. The reaction mixture was extracted with AcOEt, the organic phase was washed with brine, dried over magnesium sulfate then concentrated. The resulting solid was purified by flash chromatography (DCM/MeOH: 100/0 to 95/5) to afford 4-[(4-methoxyphenyl)methyl]-1,4-oxazepan-5-one as a colorless oil in 93% yield. M/Z (M+H)⁺: 236

To a suspension of 4-[(4-methoxyphenyl)methyl]-1,4-oxazepan-5-one (1 equiv.) in D₂O (0.25M) was added potassium carbonate (2 equiv.). The reaction mixture was heated 72h at reflux. The reaction mixture was diluted with a saturated sodium bicarbonate solution then extracted with DCM. The combined organic layers were filtered on an hydrophobic cartridge. Purification by flash chromatography (Cyclohexane/AcOEt : 8/2 to 0/10 then DCM/MeOH : 10/0 to 97/3) afforded 6,6-dideuterio-4-[(4-methoxyphenyl)methyl]-1,4-oxazepan-5-one as a colorless oil in 68% yield. M/Z (M+H)⁺: 238

To a solution of 6,6-dideuterio-4-[(4-methoxyphenyl)methyl]-1,4-oxazepan-5-one (1 equiv.) in THF (0.1M) at 0°C, was added LiAIH4 (2 equiv.). The reaction mixture was stirred overnight at rt. The reaction mixture was quenched with x µl of water (x is the number of mg of Li Al D4), 3x µl of a 3N NaOH solution, x µl of water, then diluted with Et2O. The resulting white suspension was filtered off, the filtrate was concentrated then purified by flash chromatography (DCM/MeOH: 100/0 to 92/8) to afford 6,6-dideuterio-4-[(4-methoxyphenyl)methyl]-1,4-oxazepane as a colorless oil in quantitative yield. M/Z (M+H)⁺: 224

To a solution of 6,6-dideuterio-4-[(4-methoxyphenyl)methyl]-1,4-oxazepane (1 equiv.) in DCE (0.2M) at 0°C, was added 1-chloroethyl chloroformate (2.5 equiv.). The reaction mixture was refluxed overnight, then concentrated. The resulting solid was dissolved in MeOH (0.1M). The reaction mixture was refluxed 3h. HCl, 1.25N in MeOH, (1 equiv.) was added, then the reaction mixture was concentrated. The resulting residue was triturated in Et₂O, then dried overnight under vacuum to afford 6,6-dideuterio-1,4-oxazepane hydrochloride B91 as a beige powder in 84% yield. M/Z (M+H)⁺: 104

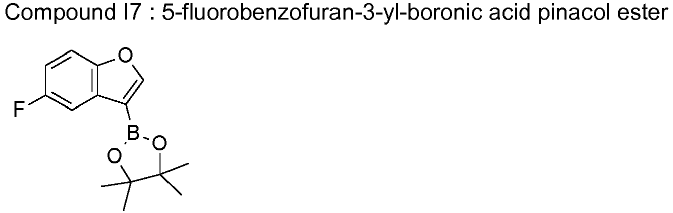

To a solution of 3-bromo-5-fluorobenzofuran (1 equiv.) in THF (0.2M) at -78°C was added dropwise n-BuLi, 1.6M in hexanes (1.1 equiv.). The resulting solution was stirred 1h at -78°C. 2-Isopropoxy-4,4,5,5-tetramethyl-1,3,2-dioxaborolane (3 equiv.) was added dropwise. The resulting solution was stirred 1h at -78°C. The reaction mixture was quenched with a saturated ammonium chloride solution then extracted with ethyl acetate. The combined organic phase were filtered on an hydrophobic cartridge, then concentrated. Purification by flash chromatography (Cyclohexane/AcOEt : 10/0 to 8/2 then 8/2 to 4/6) afforded I7 in 56% yield. M/Z (M+H-dioxolan)⁺: 149

### Compound 253: [3-furo[3,2-b]pyridin-3-yl-1-(2,2,2-trifluoroethyl)pyrazolo[4,3-c]pyridin-6-yl]-(1,4-oxazepan-4-yl)methanone

Under argon atmosphere, to a solution of compound F7 (1 equiv.) in anhydrous dioxane (0.1M) were added 3-(tributylstannyl)furo[3,2-b]pyridine (1.5 equiv.) and Pd(PPh₃)₄ (10 mol%). The reaction mixture was heated overnight at 100°C. The reaction mixture was diluted with a KF 1M aqueous solution, then stirred 15 min at rt. The reaction mixture was extracted with DCM (3 times). The combined organic layers were filtered on an hydrophobic cartridge, concentrated, then purified by flash chromatography (DCM/MeOH: 100/0 to 95/5) to afford 253 as a white solid in 64% yield. ¹H-NMR (DMSO-*d_{6,}* 400MHz) : 1.78 (quint, *J* 6.0 Hz, 1H, CH₂); 1.94 (quint, *J* 6.0 Hz, 1H, CH₂); 3.49-3.53 (m, 2H, N-CH₂); 3.64-3.66 (m, 1H, N-CH₂); 3.70-3.79 (m, 5H, CH₂); 5.66 (q, *J*9.0 Hz, 2H, CH₂-CF₃); 7.53 (dd, *J*8.5, 4.8 Hz, 1H, Ar); 8.17-8.19 (m, 1H, Ar); 8.22 (d, *J* 1.0 Hz, 1H, one rotamer, Ar); 8.24 (d, *J* 1.0 Hz, 1H, one rotamer, Ar); 8.73-8.74 (m, 1H, Ar); 9.03 (s, 1H, one rotamer, Ar); 9.04 (s, 1H, one rotamer, Ar); 9.79 (d, *J* 1.0 Hz, 1H, one rotamer, Ar); 9.82 (d, *J* 1.0 Hz, 1H, one rotamer, Ar). M/Z (M+H)⁺: 446

### Compound 254: [1,4]Oxazepan-4-yl-[3-(1H-pyrrolo[3,2-b]pyridin-3-yl)-1-(2,2,2-trifluoro-ethyl)-1H-pyrazolo[4,3-c]pyridin-6-yl]-methanone

Under argon atmosphere, to a solution of compound F7 (1 equiv.) in anhydrous dioxane (0.1M) were added 3-(tributylstannyl)furo[3,2-b]pyridine (1.5 equiv.) and Pd(PPh₃)₄ (5 mol%). The reaction mixture was heated overnight at 100°C. The reaction mixture was diluted with water. The reaction mixture was extracted with ethyl acetate (3 times). The combined organic layers were washed with brine, filtered on an hydrophobic cartridge, concentrated, then purified by flash chromatography (DCM/MeOH: 100/0 to 92/8) to afford 254 as a white solid in 7% yield. ¹H-NMR (DMSO-*d₆*, 400 MHz): 1.72-1.82 (m, 1H, CH₂); 1.88-1.98 (m, 1H, CH₂); 3.48-3.58 (m, 2H, N-CH₂); 3.61-3.68 (m, 1H, N-CH₂); 3.68-3.84 (m, 5H, CH₂); 5.58 (q, *J*9.1 Hz, 2H, CH₂-CF₃); 7.26 (dd, *J*8.4, 4.5 Hz, 1H, Ar); 7.92 (dd, *J*8.4, 1.2 Hz, 1H, Ar); 8.06-8.09 (m, 1H, Ar); 8.27 (bs, 1H, Ar, signal of a rotamer); 8.29 (bs, 1H, Ar, signal of a rotamer); 8.53 (d, *J*4.5 Hz, 1H, Ar); 9.97 (d, *J*1.2 Hz, 1H, Ar, signal of a rotamer); 9.99 (d, *J* 1.2 Hz, 1H, Ar, signal of a rotamer); 11.89 (s, 1H, NH). M/Z (M+H)⁺: 445

### Compound 255: [1,4]Oxazepan-4-yl-[3-(1H-pyrrolo[2,3-c]pyridin-3-yl)-1-(2,2,2-trifluoro-ethyl)-1H-pyrazolo[4,3-c]pyridin-6-yl]-methanone

Compound 255 was obtained according to General Procedure VII, Alternative 2, starting from [3-bromo-1-(2,2,2-trifluoroethyl)pyrazolo[4,3-c]pyridin-6-yl]-(1,4-oxazepan-4-yl)methanone F7 and tert-butyl 3-bromo-1H-pyrrolo[2,3-c]pyridine-1-carboxylate. Purification by flash chromatography (DCM/MeOH: 100/0 to 95/5) afforded 255 as a white powder in 50% yield. ¹H-NMR (DMSO-*d₆*, 400 MHz): 1.75-1.81 (m, 1H, CH₂); 1.90-1.97 (m, 1H, CH₂); 3.48-3.54 (m, 2H, N-CH₂); 3.64-3.68 (m, 1H, N-CH₂); 3.69-3.82 (m, 5H, CH₂); 5.62 (q, 2H, *J* 9.3 Hz, CH₂-CF₃); 8.11-8.14 (m, 2H, Ar); 8.20-8.23 (m, 1H, Ar); 8.27-8.31 (m, 1H, Ar); 8.69 (m, 1H, Ar, signal of a rotamer); 8.70 (m, 1H, Ar, signal of a rotamer); 8.87 (bs, 1H, NH); 9.57 (m, 1H, Ar, signal of a rotamer); 9.58 (m, 1H, Ar, signal of a rotamer). M/Z (M+H)⁺: 445

### Compound 256: (8-Oxa-3-aza-bicyclo[3.2.1]oct-3-yl)-[3-(1H-pyrrolo[3,2-c]pyridin-3-yl)-1-(2,2,2-trifluoro-ethyl)-1H-pyrazolo[4,3-c]pyridin-6-yl]-methanone

Compound 256 was obtained according to General Procedure VII, starting from [3-bromo-1-(2,2,2-trifluoroethyl)pyrazolo[4,3-c]pyridin-6-yl]-(1,4-oxazepan-4-yl)methanone F7 and tert-butyl 3-(4,4,5,5-tetramethyl-1,3,2-dioxaborolan-2-yl)-1H-pyrrolo[3,2-c]pyridine-1-carboxylate. Purification by flash chromatography (DCM/MeOH: 100/0 to 90/10) then by preparative LC-MS afforded 256 as a white powder in 14% yield. ¹H-NMR (DMSO-*d₆*, 400 MHz): 1.69-1.98 (m, 4H, CH₂); 3.07 (m, 1H, N-CH₂); 3.29 (m, 1H, N-CH₂); 3.39-3.49 (m, 1H, N-CH₂); 4.16-4.29 (m, 2H, N-CH₂, O-CH); 4.44 (m, 1H, O-CH); 5.56-5.76 (m, 2H, CH₂-CF₃); 7.52 (d, *J* 5.6 Hz, 1H, Ar); 8.15 (bs, 1H, Ar); 8.32 (d, *J*5.6 Hz, 1H, Ar); 8.59 (s, 1H, Ar); 9.55 (s, 1H, Ar); 9.60 (d, *J* 1.0 Hz, 1H, Ar); 12.16 (bs, 1H, NH). M/Z (M+H)⁺: 457

### Example 3: Testing compounds of the present invention for inhibitory activities against human adenosine receptors in recombinant cells.

The functional activities of human A_{2A} and A_{2B} receptors were determined by quantification of cAMP, being the second messenger for adenosine receptors.

For this purpose, recombinant HEK293 cells, expressing either human A_{2A} or A_{2B} receptors (both Gs coupled) were seeded into 394-well microtiter plates, test compounds and agonist (NECA) were added. After a 15min incubation, HTRF reagents (cAMP dynamic 2, Cis Bio) were added and the cellular cAMP levels were determined using the ENVISION (Perkin Elmer) plate reader.

The compounds of the present invention show a high selectivity for adenosine A_{2A} and A_{2B} receptors, as also reflected by the IC₅₀ data of the exemplary compounds of formula (I) according to the present invention shown in table 3 below.

Particularly, in contrast to the known adenosine A_{2A} receptor antagonists, the compounds of the present invention surprisingly show an A_{2A}/A_{2B} dual activity (see table 3) which is highly advantageous for the treatment and/or prevention of hyperproliferative and infectious diseases and disorders as it is disclosed above.

**Table 3**

| **No.** | **A2A (Category)** | **A2B (Category)** |
|---|---|---|
| 3 | A | A |
| 4 | A | B |
| 5 | A | B |
| 6 | A | B |
| 7 | A | B |
| 9 | A | B |
| 10 | A | B |
| 13 | A | B |
| 14 | A | A |
| 15 | A | A |
| 16 | A | A |
| 17 | A | A |
| 18 | A | B |
| 19 | A | A |
| 20 | A | A |
| 23 | A | A |
| 24 | A | A |
| 25 | A | A |
| 27 | A | B |
| 28 | A | A |
| 29 | A | B |
| 30 | A | A |
| 31 | A | B |
| 32 | A | B |
| 33 | A | B |
| 34 | A | B |
| 35 | A | B |
| 36 | A | A |
| 37 | A | A |
| 38 | A | B |
| 39 | A | A |
| 40 | A | A |
| 41 | A | B |
| 44 | A | A |
| 46 | A | A |
| 48 | A | B |
| 50 | A | B |
| 52 | A | B |
| 55 | A | B |
| 56 | A | B |
| 59 | A | B |
| 60 | A | A |
| 61 | A | B |
| 62 | A | A |
| 68 | A | B |
| 71 | A | B |
| 73 | A | A |
| 74 | A | B |
| 80 | A | B |
| 81 | A | B |
| 82 | A | B |
| 83 | A | B |
| 85 | A | A |
| 86 | A | A |
| 87 | A | B |
| 88 | A | B |
| 89 | A | A |
| 90 | A | A |
| 91 | A | A |
| 92 | A | B |
| 94 | A | A |
| 95 | A | B |
| 96 | A | B |
| 99 | A | B |
| 100 | A | A |
| 101 | A | A |
| 102 | A | A |
| 103 | A | B |
| 104 | A | B |
| 105 | A | B |
| 106 | A | B |
| 107 | A | A |
| 108 | A | B |
| 109 | A | A |
| 110 | A | A |
| 111 | A | A |
| 112 | A | A |
| 113 | A | A |
| 114 | A | A |
| 115 | A | A |
| 116 | A | A |
| 117 | A | B |
| 118 | A | A |
| 119 | A | B |
| 120 | A | A |
| 121 | A | A |
| 122 | A | A |
| 123 | A | A |
| 125 | A | B |
| 126 | A | B |
| 127 | A | A |
| 131 | A | B |
| 132 | A | A |
| 133 | A | B |
| 134 | A | B |
| 135 | A | B |
| 136 | A | A |
| 137 | A | A |
| 138 | A | A |
| 139 | A | A |
| 140 | A | B |
| 141 | A | B |
| 142 | A | B |
| 143 | A | B |
| 144 | A | B |
| 146 | A | B |
| 147 | A | B |
| 148 | B | B |
| 149 | A | B |
| 151 | A | B |
| 154 | A | B |
| 159 | A | B |
| 161 | A | B |
| 162 | A | B |
| 165 | A | B |
| 166 | A | B |
| 168 | A | A |
| 169 | A | A |
| 170 | A | B |
| 171 | A | B |
| 172 | A | A |
| 173 | A | B |
| 176 | A | B |
| 177 | A | A |
| 180 | A | B |
| 182 | A | B |
| 183 | A | B |
| 185 | A | B |
| 187 | A | B |
| 190 | A | A |
| 191 | A | B |
| 192 | A | B |
| 193 | A | B |
| 194 | A | A |
| 197 | A | B |
| 199 | A | B |
| 201 | A | B |
| 203 | A | B |
| 204 | A | B |
| 205 | A | B |
| 207 | A | A |
| 208 | A | A |
| 209 | A | A |
| 210 | A | B |
| 211 | A | B |
| 212 | A | B |
| 213 | A | A |
| 214 | A | B |
| 215 | A | B |
| 216 | A | B |
| 217 | A | A |
| 218 | A | A |
| 219 | A | B |
| 220 | A | B |
| 221 | A | A |
| 222 | A | B |
| 224 | A | A |
| 225 | A | B |
| 226 | A | B |
| 227 | A | A |
| 228 | A | A |
| 229 | A | A |
| 230 | A | A |
| 232 | A | B |
| 233 | A | B |
| 234 | A | B |
| 235 | A | B |
| 237 | A | B |
| 242 | A | B |
| 243 | A | B |
| 244 | A | B |
| 245 | A | B |
| 246 | A | B |
| 247 | A | B |
| 248 | A | B |
| 249 | A | B |
| 250 | A | B |
| 251 | A | B |
| 252 | A | A |

| | | |
|---|---|---|
| "A" means IC₅₀ value is < 100 nM, "B" means IC₅₀ value is < 1 µM. | | |

Furthermore, in contrast to analogous indazole compounds, including e.g. the A_{2A} inhibitors taught in WO 2010/084425, the 5-azaindazole compounds of formula (I) according to the present invention show a surprising and advantageous A_{2A}/A_{2B} dual inhibitory activity, as demonstrated in table 4.

**Table 4**

| **Compound No.** | **Structure** | **Functional A2A receptor activity, HEK293, cAMP, IC50 [µM]** | **Functional A2B receptor activity, HEK293, cAMP, IC50 [µM]** |
|---|---|---|---|
| 44 | 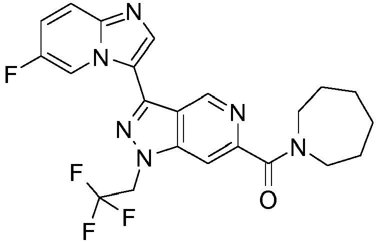 | A | A |
| analogue to No. 44 | 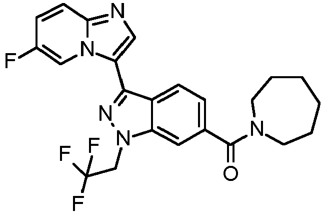 | A | B |
| 60 | 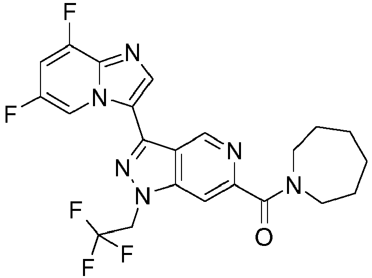 | A | A |
| analogue to No. 60 | 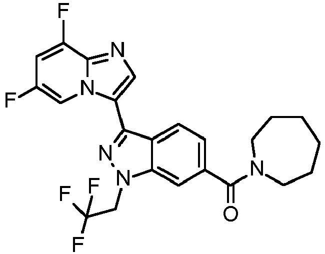 | A | B |
| 48 | 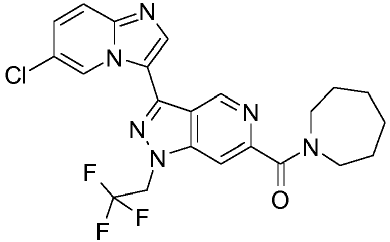 | A | B |
| analogue to No. 48 | 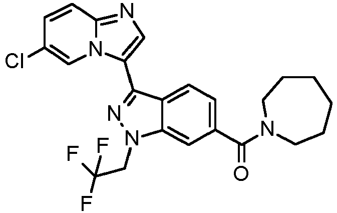 | A | C |

| | | | |
|---|---|---|---|
| "A" means IC₅₀ value is < 100 nM; "B" means IC₅₀ value is < 1 µM; "C" means IC₅₀ value is > 1 µM. | | | |

### Example 4: Testing the effects of the compounds of the present invention against endogenous human A_{2A} receptor - human T cell assay

The endogenous functional activity of the Gs-coupled human A_{2A} receptor was measured in T cells, where this receptor is highly expressed. Determination of receptor activity was done by quantification of cAMP, which is a second messenger for adenosine receptors.

In short, human pan T cells were isolated from human PBMC (MACS Pan T Cell Isolation Kit, Miltenyi Biotec) that have been derived from fresh whole blood. The T cells were seeded in 384-well microtiter plates and treated with test compounds. After 10min incubation at room temperature, the A_{2A} adenosine receptor agonist NECA was added, and the plates were incubated for another 45min. Finally, HTRF reagents (cAMP Femto Kit, CisBio) were added to the wells, and after 1h cellular cAMP levels were determined using the ENVISION (Perkin Elmer) plate reader.

The obtained raw data were normalized against the inhibitor control and the neutral control (DMSO) and the normalized data were fitted using Genedata Screener software.

The compounds of the present invention show that they are able to inhibit the A_{2A} receptor expressed in human T cells which incubated with the A_{2A} adenosine receptor agonist NECA (as measured by quantification of cAMP), which is preferred for the treatment and/or prevention of hyperproliferative and infectious diseases and disorders as it is disclosed above. Therefore, the compounds of the present invention surprisingly are able to prevent immunosuppression and thus are able to support antitumor T cell induced inhibition of tumor growth, reduction or destruction of metastases and prevention of neovascularization.

**Table 5**

| **No.** | **Structure** | **Human T cell cAMP IC₅₀ (nM)** | **Human T cell IL-2 IC₅₀ (nM)** |
|---|---|---|---|
| 85 | 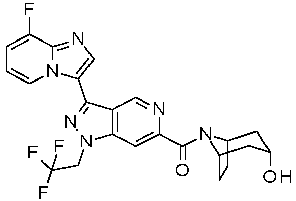 | A | B |
| 109 | 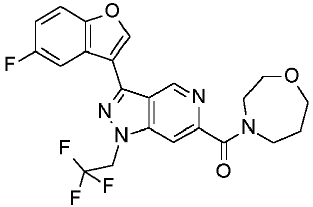 | A | A |
| 224 | 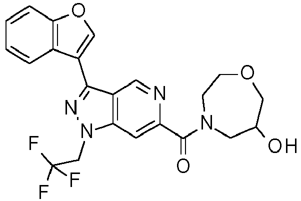 | A | A |

| | | | |
|---|---|---|---|
| "A" means IC₅₀ value is < 100 nM, "B" means IC₅₀ value is < 1 µM. | | | |

### Example 5: Testing the effect of the compounds of the present invention on mouse T cells

### Background:

Adenosine (Ado) in tumor microenvironment can inhibit T cell activity by signaling through A_{2A} receptors and suppress cytokine secretion by T cells. A_{2A} specific agonists like NECA does similar job of inhibition of T cell cytokine secretion in vitro and in vivo. Potential A_{2A} antagonists or A_{2A}/A_{2B} dual antagonists can rescue T cells from this inhibition. Herein, we describe the in vitro system we established using Pan T cells from mouse spleens to screen potential A_{2A} antagonists or A_{2A}/A_{2B} dual antagonists for their activity. The method described involves the use of CD3/CD28 pre-coated beads to stimulate Pan T cells purified from mouse splenocytes, combined with the addition of A_{2A} agonist along with potential A_{2A} or A_{2A}/A_{2B} dual antagonists to evaluate potentiation of T cell cytokine production.

### Assay description:

### Mouse T cells

Briefly, mouse Pan T cells are purified from spleens of BALB/c mice using Pan T cell isolation kit Mouse II (MACS Miltenyi biotech Cat# Order no. 130-095-130) according to manufacturer's protocol. The purified T cells are seeded in Nunc^{™} 96-Well Polystyrene Round Bottom Microwell Plates in RPMI medium with 10% heat inactivated fetal bovine serum. The cells are rested at 37°C for 1 h before activating with CD3/CD28 pre-coated beads (Dynabeads^{™} Mouse T-Activator CD3/CD28; Cat# 11456D). After 30 min the cells are treated with varying doses of test antagonist(s). The cells are incubated for additional 30 min at 37°C before treating with A_{2A} agonist NECA (1 µM) or neutral control (DMSO). After 24 h incubation IL-2 levels in the supernatants are measured by ELISAs according to manufacturer's protocol (R&D systems Cat# DY402 (IL-2)). Once the concentrations are calculated, the difference of cytokine concentration of DMSO control and agonist alone control is calculated and the percentage of rescue by each concentration of antagonist is calculated by using Microsoft Excel. These percentages of cytokine rescue in a dose dependent manner of antagonist is plotted in GraphPad Prism software and IC₅₀ is calculated.

The compounds of the present invention show that they are able to rescue T cells from inhibition and are able to prevent the suppression of cytokine secretion as induced by adenosine or A_{2A} specific agonists like NECA, which is preferred for the treatment and/or prevention of hyperproliferative and infectious diseases and disorders as it is disclosed above. Therefore, the compounds of the present invention surprisingly are able to prevent immunosuppression and thus are able to support antitumor T cell induced inhibition of tumor growth, reduction or destruction of metastases and prevention of neovascularization.

**Table 6**

| **No.** | **Structure** | **Mouse T cell IL-2 IC₅₀ (nM)** |
|---|---|---|
| 85 | 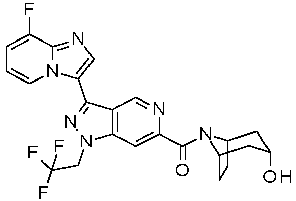 | A |
| 109 | 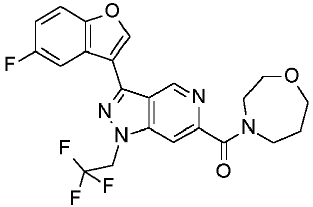 | B |
| 224 | 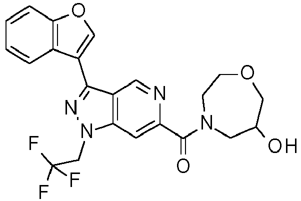 | B |

| | | |
|---|---|---|
| "A" means IC₅₀ value is < 100 nM, "B" means IC₅₀ value is < 1 µM. | | |

The following examples relate to pharmaceutical compositions comprising an active ingredient according to the invention, i.e. a compound of formula (I) or a pharmaceutically acceptable salt, or solvate thereof:

### Example 6: Injection vials

A solution of 100 g of an active ingredient according to the invention and 5 g of disodium hydrogenphosphate in 3 l of bidistilled water is adjusted to pH 6.5 using 2N hydrochloric acid, sterile filtered, transferred into injection vials, lyophilised under sterile conditions and sealed under sterile conditions. Each injection vial contains 5 mg of active ingredient.

### Example 7: Suppositories

A mixture of 20 g of an active ingredient according to the invention with 100 g of soya lecithin and 1400 g of cocoa butter is melted, poured into moulds and allowed to cool. Each suppository contains 20 mg of active ingredient.

### Example 8: Solution

A solution is prepared from 1 g of an active ingredient according to the invention, 9.38 g of NaH₂PO₄ · 2 H₂O, 28.48 g of Na₂HPO₄ · 12 H₂O and 0.1 g of benzalkonium chloride in 940 ml of bidistilled water. The pH is adjusted to 6.8, and the solution is made up to 1 l and sterilised by irradiation. This solution can be used in the form of eye drops.

### Example 9: Ointment

500 mg of an active ingredient according to the invention are mixed with 99.5 g of Vaseline under aseptic conditions.

### Example 10: Tablets

A mixture of 1 kg of active ingredient, 4 kg of lactose, 1.2 kg of potato starch, 0.2 kg of talc and 0.1 kg of magnesium stearate is pressed to give tablets in a conventional manner in such a way that each tablet contains 10 mg of active ingredient.

### Example 11: Coated tablets

Tablets are pressed analogously to Example 10 and subsequently coated in a conventional manner with a coating of sucrose, potato starch, talc, tragacanth and dye.

### Example 12: Capsules

2 kg of active ingredient are introduced into hard gelatine capsules in a conventional manner in such a way that each capsule contains 20 mg of the active ingredient.

### Example 13: Ampoules

A solution of 1 kg of an active ingredient according to the invention in 60 l of bidistilled water is sterile filtered, transferred into ampoules, lyophilised under sterile conditions and sealed under sterile conditions. Each ampoule contains 10 mg of active ingredient.

## Claims

1. A compound of formula (I) 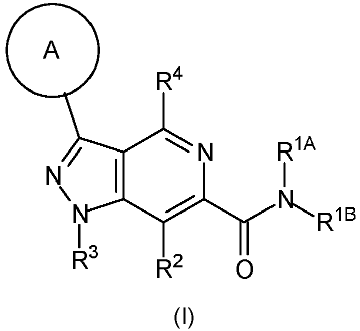 wherein:
R^{1A} and R^{1B} are mutually linked to form, together with the nitrogen atom that they are attached to, a heterocycloalkyl which is optionally substituted with one or more groups R¹¹;
or, alternatively, R^{1A} and R^{1B} are each independently selected from hydrogen, C₁₋₅ alkyl, C₂₋₅ alkenyl, C₂₋₅ alkynyl, -CO(C₁₋₅ alkyl), carbocyclyl, and heterocyclyl, wherein said alkyl, said alkenyl, said alkynyl, and the alkyl moiety of said -CO(C₁₋₅ alkyl) are each optionally substituted with one or more groups R¹², and further wherein said carbocyclyl and said heterocyclyl are each optionally substituted with one or more groups R¹³.
each R¹¹ is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl;
each R¹² is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl;
each R¹³ is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-cycloalkyl, and -(C₀₋₃ alkylene)-heterocycloalkyl;
R² and R⁴ are each independently selected from hydrogen, C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl;
R³ is selected from hydrogen, C₁₋₅ alkyl, C₂₋₅ alkenyl, C₂₋₅ alkynyl, -(C₁₋₅ alkylene)-OH, -(C₁₋₅ alkylene)-O(C₁₋₅ alkyl), -(C₁₋₅ alkylene)-O(C₁₋₅ alkylene)-OH, -(C₁₋₅ alkylene)-O(C₁₋₅ alkylene)-O(C₁₋₅ alkyl), -(C₁₋₅ alkylene)-SH, -(C₁₋₅ alkylene)-S(C₁₋₅ alkyl), -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -(C₁₋₅ alkylene)-halogen, C₁₋₅ haloalkyl, -(C₁₋₅ alkylene)-O-(C₁₋₅ haloalkyl), -(C₁₋₅ alkylene)-CF₃, -(C₁₋₅ alkylene)-CN, -(C₁₋₅ alkylene)-NO₂, -(C₁₋₅ alkylene)-Si(C₁₋₅ alkyl)₃, -(C₁₋₅ alkylene)-O(C₁₋₅ alkylene)-Si(C₁₋₅ alkyl)₃, -(C₁₋₅ alkylene)-CHO, -(C₁₋₅ alkylene)-CO-(C₁₋₅ alkyl), -(C₁₋₅ alkylene)-COOH, -(C₁₋₅ alkylene)-CO-O-(C₁₋₅ alkyl), -(C₁₋₅ alkylene)-O-CO-(C₁₋₅ alkyl), -(C₁₋₅ alkylene)-CO-NH₂, -(C₁₋₅ alkylene)-CO-NH(C₁₋₅ alkyl), -(C₁₋₅ alkylene)-CO-N(C₁₋₅ alkyl)(C₁₋₅ alkyl), -(C₁₋₅ alkylene)-NH-CO-(C₁₋₅ alkyl), -(C₁₋₅ alkylene)-N(C₁₋₅ alkyl)-CO-(C₁₋₅ alkyl), -(C₁₋₅ alkylene)-NH-CO-O-(C₁₋₅ alkyl), -(C₁₋₅ alkylene)-N(C₁₋₅ alkyl)-CO-O-(C₁₋₅ alkyl), -(C₁₋₅ alkylene)-O-CO-NH-(C₁₋₅ alkyl), -(C₁₋₅ alkylene)-O-CO-N(C₁₋₅ alkyl)-(C₁₋₅ alkyl), -(C₁₋₅ alkylene)-SO₂-NH₂, -(C₁₋₅ alkylene)-SO₂-NH(C₁₋₅ alkyl), -(C₁₋₅ alkylene)-SO₂-N(C₁₋₅ alkyl)(C₁₋₅ alkyl), -(C₁₋₅ alkylene)-NH-SO₂-(C₁₋₅ alkyl), -(C₁₋₅ alkylene)-N(C₁₋₅ alkyl)-SO₂-(C₁₋₅ alkyl), -(C₁₋₅ alkylene)-SO-(C₁₋₅ alkyl), -(C₁₋₅ alkylene)-SO₂-(C₁₋₅ alkyl), -(C₀₋₅ alkylene)-carbocyclyl, and -(C₀₋₅ alkylene)-heterocyclyl, wherein the carbocyclyl moiety of said -(C₀₋₅ alkylene)-carbocyclyl and the heterocyclyl moiety of said -(C₀₋₅ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl;
ring A is a bicyclic heteroaryl, wherein said bicyclic heteroaryl is optionally substituted with one or more groups R^{A}; and
each R^{A} is independently selected from C₁₋₅ alkyl, C₂₋₅ alkenyl, C₂₋₅ alkynyl, -(C₀₋₃ alkylene)-OH, -(C₀₋₃ alkylene)-O(C₁₋₅ alkyl), -(C₀₋₃ alkylene)-O(C₁₋₅ alkylene)-OH, -(C₀₋₃ alkylene)-O(C₁₋₅ alkylene)-O(C₁₋₅ alkyl), -(C₀₋₃ alkylene)-SH, -(C₀₋₃ alkylene)-S(C₁₋₅ alkyl), -(C₀₋₃ alkylene)-NH₂, -(C₀₋₃ alkylene)-NH(C₁₋₅ alkyl), -(C₀₋₃ alkylene)-N(C₁₋₅ alkyl)(C₁₋₅ alkyl), -(C₀₋₃ alkylene)-halogen, -(C₀₋₃ alkylene)-(C₁₋₅ haloalkyl), -(C₀₋₃ alkylene)-O-(C₁₋₅ haloalkyl), -(C₀₋₃ alkylene)-CF₃, -(C₀₋₃ alkylene)-CN, -(C₀₋₃ alkylene)-NO₂, -(C₀₋₃ alkylene)-CHO, -(C₀₋₃ alkylene)-CO-(C₁₋₅ alkyl), -(C₀₋₃ alkylene)-COOH, -(C₀₋₃ alkylene)-CO-O-(C₁₋₅ alkyl), -(C₀₋₃ alkylene)-O-CO-(C₁₋₅ alkyl), -(C₀₋₃ alkylene)-CO-NH₂, -(C₀₋₃ alkylene)-CO-NH(C₁₋₅ alkyl), -(C₀₋₃ alkylene)-CO-N(C₁₋₅ alkyl)(C₁₋₅ alkyl), -(C₀₋₃ alkylene)-NH-CO-(C₁₋₅ alkyl), -(C₀₋₃ alkylene)-N(C₁₋₅ alkyl)-CO-(C₁₋₅ alkyl), -(C₀₋₃ alkylene)-NH-CO-O-(C₁₋₅ alkyl), -(C₀₋₃ alkylene)-N(C₁₋₅ alkyl)-CO-O-(C₁₋₅ alkyl), -(C₀₋₃ alkylene)-O-CO-NH-(C₁₋₅ alkyl), -(C₀₋₃ alkylene)-O-CO-N(C₁₋₅ alkyl)-(C₁₋₅ alkyl), -(C₀₋₃ alkylene)-SO₂-NH₂, -(C₀₋₃ alkylene)-SO₂-NH(C₁₋₅ alkyl), -(C₀₋₃ alkylene)-SO₂-N(C₁₋₅ alkyl)(C₁₋₅ alkyl), -(C₀₋₃ alkylene)-NH-SO₂-(C₁₋₅ alkyl), -(C₀₋₃ alkylene)-N(C₁₋₅ alkyl)-SO₂-(C₁₋₅ alkyl), -(C₀₋₃ alkylene)-SO-(C₁₋₅ alkyl), -(C₀₋₃ alkylene)-SO₂-(C₁₋₅ alkyl), -(C₀₋₃ alkylene)-carbocyclyl, and -(C₀₋₃ alkylene)-heterocyclyl, wherein the carbocyclyl moiety of said -(C₀₋₃ alkylene)-carbocyclyl and the heterocyclyl moiety of said -(C₀₋₃ alkylene)-heterocyclyl are each optionally substituted with one or more groups independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyl, and heterocycloalkyl; or a pharmaceutically acceptable salt or solvate thereof.

2. The compound of claim 1, wherein R^{1A} and R^{1B} are mutually linked to form, together with the nitrogen atom that they are attached to, a heterocycloalkyl which is optionally substituted with one or more groups R¹¹.

3. The compound of claim 1 or 2, R^{1A} and R^{1B} are mutually linked to form, together with the nitrogen atom that they are attached to, a group which is selected from 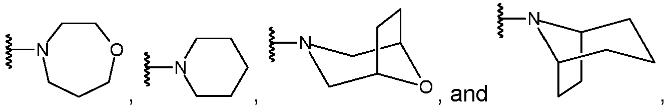 wherein each one of the above-depicted groups is optionally substituted with one or more R¹¹.

4. The compound of any one of claims 1 to 3, wherein R² and R⁴ are each independently selected from hydrogen, C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN.

5. The compound of any one of claims 1 to 4, wherein R³ is selected from C₁₋₅ haloalkyl, -(C₁₋₅ alkylene)-CN, -(C₁₋₅ alkylene)-SO-(C₁₋₅ alkyl), and -(C₁₋₅ alkylene)-SO₂-(C₁₋₅ alkyl).

6. The compound of any one of claims 1 to 5, wherein R³ is -CH₂-CF₃ or -CH₂-SO₂-CH₃.

7. The compound of any one of claims 1 to 6, wherein ring A is a fused bicyclic heteroaryl, wherein one of the two fused rings of said fused bicyclic heteroaryl has 5 ring atoms and the other fused ring has 6 ring atoms, and further wherein said bicyclic heteroaryl is optionally substituted with one or more groups R^{A}.

8. The compound of any one of claims 1 to 7, wherein ring A is a bicyclic heteroaryl having the following formula (II): 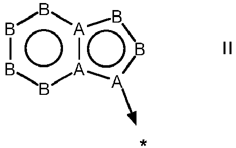 wherein each of the two fused rings of said bicyclic heteroaryl is aromatic, wherein each ring atom A of the bicyclic heteroaryl is independently a carbon or nitrogen atom, wherein each ring atom B is independently a carbon, nitrogen, oxygen or sulfur atom, wherein at least one of the ring atoms A and/or B is different from carbon, and wherein said bicyclic heteroaryl is optionally substituted with one or more groups R^{A}.

9. The compound of any one of claims 1 to 8, wherein ring A is selected from any one of the following groups: 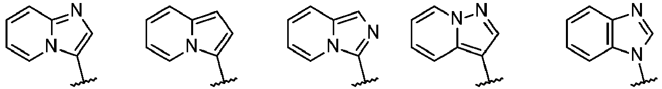 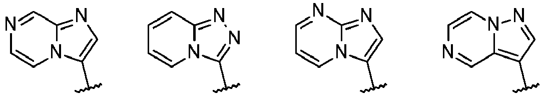 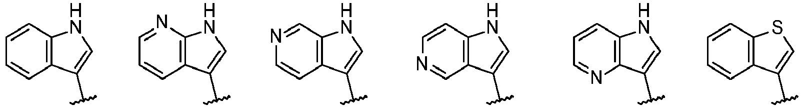 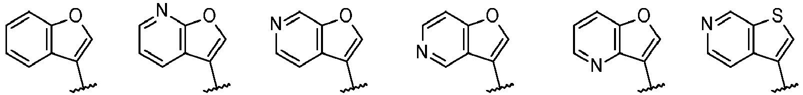
wherein each of the above-depicted groups is optionally substituted with one or more groups R^{A};
and preferably wherein ring A is selected from any one of the following groups: 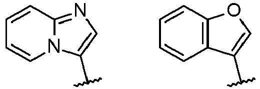 wherein each of the above-depicted groups is optionally substituted with one or more groups R^{A}.

10. The compound of any one of claims 1 to 9, wherein each R^{A} is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogen, C₁₋₅ haloalkyl, -CF₃, and -CN; preferably wherein each R^{A} is independently halogen; and more preferably wherein each R^{A} is -F.

11. The compound of claim 1, wherein said compound is selected from:
| | |
|---|---|
| 1 | [3-(1-Methyl-1H-indol-3-yl)-1-(2,2,2-trifluoro-ethyl)-1H-pyrazolo[4,3-c]pyridine-6-yl]-1,4-oxazepan-4-yl-methanone |
| 2 | Azepan-1-yl-[3-(6-fluoro-imidazo[1,2-a]pyridin-3-yl)-1-(2-methanesulfonyl-ethyl)-1H-pyrazolo[4,3-c]pyridin-6-yl]-methanone |
| 3 | Azepan-1-yl-[1-(2,2-difluoro-ethyl)-3-(6-fluoro-imidazo[1,2-a]pyridin-3-yl)-1H-pyrazolo[4,3-c]pyridin-6-yl]-methanone |
| 4 | Azepan-1-yl-[1-(2-fluoro-ethyl)-3-(6-fluoro-imidazo[1,2-a]pyridin-3-yl)-1H-pyrazolo[4,3-c]pyridin-6-yl]-methenone |
| 5 | Azepan-1-yl-[3-(6-fluoro-imidazo[1,2-a]pyridin-3-yl)-1-propyl-1H-pyrazolo[4,3-c]pyridin-6-yl]-methanone |
| 6 | Azepan-1-yl-[3-(6-fluoro-imidazo[1,2-a]pyridin-3-yl)-1-methoxymethyl-1H-pyrazolo[4,3-c]pyridin-6-yl]-methanone |
| 7 | [6-(Azepane-1-carbonyl)-3-(6-fluoro-imidazo[1,2-a]pyridin-3-yl)-pyrazolo[4,3-c]pyridin-1-yl]-acetonitrile |
| 8 | Azepan-1-yl-[3-(6,8-difluoro-imidazo[1,2-a]pyridin-3-yl)-1-(2-methoxy-ethyl)-1H-pyrazolo[4,3-c]pyridin-6-yl]-methanone |
| 9 | [1-Cyclopropylmethyl-3-(6,8-difluoro-imidazo[1,2-a]pyridin-3-yl)-1H-pyrazolo[4,3-c]pyridin-6-yl]-1,4-oxazepan-4-yl-methanone |
| 10 | [3-(6,8-Difluoro-imidazo[1,2-a]pyridin-3-yl)-1-methanesulfonyl-1H-pyrazolo[4,3-c]pyridin-6-yl]-1,4-oxazepan-4-yl-methanone |
| 11 | 3-[3-(6,8-Difluoro-imidazo[1,2-a]pyridin-3-yl)-6-(1,4-oxazepane-4-carbonyl)-pyrazolo[4,3-c]pyridin-1-ylmethyl]-azetidine-1-carboxylic acid tert-butyl ester |
| 12 | [3-(6,8-Difluoro-imidazo[1,2-a]pyridin-3-yl)-1-hydroxymethyl-1H-pyrazolo[4,3-c]pyridin-6-yl]-1,4-oxazepan-4-yl-methanone |
| 13 | [3-Benzofuran-3-yl-6-(1,4-oxazepane-4-carbonyl)-pyrazolo[4,3-c]pyridin-1-yl]-acetonitrile |
| 14 | (3-Aza-bicyclo[3.2.1]oct-3-yl)-[3-(6,8-difluoro-imidazo[1,2-a]pyridin-3-yl)-1-methylsulfanylmethyl-1H-pyrazolo[4,3-c]pyridin-6-yl]-methanone |
| 15 | [3-(6,8-Difluoro-imidazo[1,2-a]pyridin-3-yl)-1-methanesulfonylmethyl-1H-pyrazolo[4,3-c]pyridin-6-yl]-1,4-oxazepan-4-yl-methanone |
| 16 | (3-Benzofuran-3-yl-1-methanesulfonylmethyl-1H-pyrazolo[4,3-c]pyridine-6-yl)- 1,4-oxazepan-4-yl-methanone |
| 17 | (1-Methanesulfonylmethyl-3-pyrazolo[1,5-a]pyridin-3-yl-1H-pyrazolo[4,3-c]pyridin-6-yl)-1,4-oxazepan-4-yl-methanone |
| 18 | (3-Aza-bicyclo[3.2.1]oct-3-yl)-(1-methanesulfonylmethyl-3-pyrazolo[1,5-a]pyridin-3-yl-1H-pyrazolo[4,3-c]pyridin-6-yl)-methanone |
| 19 | (3-Aza-bicyclo[3.2.1]oct-3-yl)-(3-benzofuran-3-yl-1-methanesulfonylmethyl-1H-pyrazolo[4,3-c]pyridin-6-yl)-methanone |
| 20 | Azepan-1-yl-[3-(6-fluoro-imidazo[1,2-a]pyridin-3-yl)-1-methanesulfonylmethyl-1H-pyrazolo[4,3-c]pyridin-6-yl]-methanone |
| 21 | Azepan-1-yl-[3-(6-fluoro-imidazo[1,2-a]pyridin-3-yl)-1-methanesulfinylmethyl-1H-pyrazolo[4,3-c]pyridin-6-yl]-methanone |
| 22 | [3-(6,8-Difluoro-imidazo[1,2-a]pyridin-3-yl)-1-ethanesulfonylmethyl-1H-pyrazolo[4,3-c]pyridin-6-yl]-1,4-oxazepan-4-yl-methanone |
| 23 | (3-Aza-bicyclo[3.2.1]oct-3-yl)-[3-(6,8-difluoro-imidazo[1,2-a]pyridin-3-yl)-1-methanesulfonylmethyl-1H-pyrazolo[4,3-c]pyridin-6-yl]-methanone |
| 24 | [3-(6,8-Difluoro-imidazo[1,2-a]pyridin-3-yl)-1-methanesulfonylmethyl-1H-pyrazolo[4,3-c]pyridin-6-yl]-(8-endo-hydroxy-8-exo-methyl-3-aza-bicyclo[3.2.1]oct-3-yl)-methanone |
| 25 | [3-(6,8-Difluoro-imidazo[1 ,2-a]pyridin-3-yl)-1-methanesulfonylmethyl-1H-pyrazolo[4,3-c]pyridin-6-yl]-(3-endo-hydroxy-8-aza-bicyclo[3.2.1]oct-8-yl)-methanone |
| 26 | [3-(6,8-Difluoro-imidazo[1,2-a]pyridin-3-yl)-1-methanesulfonylmethyl-1H-pyrazolo[4,3-c]pyridin-6-yl]-(2-oxa-5-aza-bicyclo[2.2.1]hept-5-yl)-methanone |
| 27 | [3-(6,8-Difluoro-imidazo[1,2-a]pyridin-3-yl)-1-methanesulfonylmethyl-1H-pyrazolo[4,3c]pyridin-6-yl]-(8-oxa-3-aza-bicyclo[3.2.1]oct-3-yl)-methanone |
| 28 | (3-Benzofuran-3-yl-1-methanesulfonylmethyl-1H-pyrazolo[4,3-c]pyridin-6-yl)-(8-endo-hydroxy-8-exo-methyl-d3-3-aza-bicyclo[3.2.1]oct-3-yl)-methanone |
| 29 | (3-Benzofuran-3-yl-1-methanesulfonylmethyl-1H-pyrazolo[4,3-c]pyridin-6-yl)-(6,6-difluoro-1,4-oxazepan-4-yl)-methanone |
| 30 | (3-Benzofuran-3-yl-1-methanesulfonylmethyl-1H-pyrazolo[4,3-c]pyridin-6-yl)-(8-endo-hydroxy-3-aza-bicyclo[3.2.1]oct-3-yl)-methanone |
| 31 | (3-Benzofuran-3-yl-1-methanesulfonylmethyl-1H-pyrazolo[4,3-c]pyridin-6-yl)-(5-endo-hydroxy-5-exo-methyl-2-aza-bicyclo[2.2.1]hept-2-yl)-methanone |
| 32 | (3-Benzofuran-3-yl-1-methanesulfonylmethyl-1H-pyrazolo[4,3-c]pyridin-6-yl)-(4-hydroxy-4-methyl-piperidin-1-yl)-methanone |
| 33 | (3-Benzofuran-3-yl-1-methanesulfonylmethyl-1H-pyrazolo[4,3-c]pyridin-6-yl)-(4,4-difluoro-piperidin-1-yl)-methanone |
| 34 | (3-Benzofuran-3-yl-1-methanesulfonylmethyl-1H-pyrazolo[4,3-c]pyridin-6-yl)-(2-methoxymethyl-pyrrolidin-1-yl)-methanone |
| 35 | (3-Benzofuran-3-yl-1-methanesulfonylmethyl-1H-pyrazolo[4,3-c]pyridin-6-yl)-(4-hydroxy-2,2 dimethyl-piperidin-1-yl)-methanone |
| 36 | (3-Benzofuran-3-yl-1-methanesulfonylmethyl-1H-pyrazolo[4,3-c]pyridin-6-yl)-(4-hydroxy-piperidin-1-yl)-methanone |
| 37 | [3-(benzofuran-3-yl)-1-(methylsulfonylmethyl)pyrazolo[4,3-c]pyridin-6-yl]-(3-endo-hydroxy-8-azabicyclo[3.2.1]octan-8-yl)methanone |
| 38 | (3-Benzofuran-3-yl-1-methanesulfonylmethyl-1H-pyrazolo[4,3-c]pyridin-6-yl)-(3-hydroxy-piperidin-1-yl)-methanone |
| 39 | (3-Benzofuran-3-yl-1-methanesulfonylmethyl-1H-pyrazolo[4,3-c]pyridin-6-yl)-(3,3-difluoro-piperidin-1-yl)-methanone |
| 40 | (3-Benzofuran-3-yl-1-methanesulfonylmethyl-1H-pyrazolo[4,3-c]pyridin-6-yl)-(4-hydroxyazepan-1-yl)-methanone |
| 41 | Azepan-1-yl-[3-(6-fluoro-imidazo[1,2-a]pyridin-3-yl)-1-methyl-1H-pyrazolo[4,3-c]pyridin-6-yl]-methanone |
| 42 | 3-[6-(Azepane-1-carbonyl)-1-methyl-1H-pyrazolo[4,3-c]pyridin-3-yl]-imidazo[1,2-a]pyridine-6-carbonitrile |
| 43 | 3-[6-(Azepane-1-carbonyl)-1-propyl-1H-pyrazolo[4,3-c]pyridin-3-yl]-imidazo[1,2-a]pyridine-6-carbonitrile |
| 44 | Azepan-1-yl-[3-(6-fluoro-imidazo[1,2-a]pyridin-3-yl)-1-(2,2,2-trifluoro-ethyl)-1H-pyrazolo[4,3-c]pyridin-6-yl]-methanone |
| 45 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carbonitrile |
| 46 | 3-[6-(Azepane-1-carbonyl)-1-(2,2,2-trifluoro-ethyl)-1H-pyrazolo[4,3-c]-pyridin-3-yl]-imidazo[1,2-a]pyridine-6-carboxylic acid amide |
| 47 | 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carbonitrile |
| 48 | Azepan-1-yl-[3-(6-chloro-imidazo[1,2-a]pyridin-3-yl)-1-(2,2,2-trifluoro-ethyl)-1H-pyrazolo[4,3-c]pyridin-6-yl]-methanone |
| 49 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carboxylic acid methylamide |
| 50 | Azepan-1-yl-[3-(6-fluoro-imidazo[1,2-a]pyrimidin-3-yl)-1-(2,2,2-trifluoro-ethyl)-1H-pyrazolo[4,3-c]pyridin-6-yl]-methanone |
| 51 | Azepan-1-yl-[3-(6-methoxymethyl-imidazo[1,2-a]pyridin-3-yl)-1-(2,2,2-trifluoro-ethyl)-1H-pyrazolo[4,3-c]pyridin-6-yl]-methanone |
| 52 | Azepan-1-yl-[3-indolizin-3-yl-1-(2,2,2-trifluoro-ethyl)-1H-pyrazolo[4,3-c]pyridin-6-yl]-methanone |
| 53 | 3-[6-(Azepane-1-carbonyl)-1-(2,2,2-trifluoro-ethyl)-1H-pyrazolo[4,3-c]pyridin-3-yl]-imidazo[1,2-a]pyridine-6-carboxylic acid dimethylamide |
| 54 | Azepan-1-yl-[3-imidazo[1,5-a]pyridin-3-yl-1-(2,2,2-trifluoro-ethyl)-1H-pyrazolo[4,3-c]pyridin-6-yl]-methanone |
| 55 | Azepan-1-yl-[3-imidazo[1,2-a]pyridin-3-yl-1-(2,2,2-trifluoro-ethyl)-1H-pyrazolo[4,3-c]pyridin-6-yl]-methanone |
| 56 | Azepan-1-yl-[3-imidazo[1,2-a]pyrazin-3-yl-1-(2,2,2-trifluoro-ethyl)-1H-pyrazolo[4,3-c]pyridin-6-yl]-methanone |
| 57 | 3-[6-(Azepane-1-carbonyl)-1-(2,2,2-trifluoro-ethyl)-1H-pyrazolo[4,3-c]pyridin-3-yl]-7-methyl-imidazo[1,2-a]pyridine-6-carboxylic acid amide |
| 58 | Azepan-1-yl-[1-(2,2,2-trifluoro-ethyl)-3-(6-trifluoromethyl-imidazo[1,2-a]pyridin-3-yl)-1H-pyrazolo[4,3-c]pyridin-6-yl]-methanone |
| 59 | Azepan-1-yl-[3-(6-methoxy-imidazo[1,2-a]pyridin-3-yl)-1-(2,2,2-trifluoro-ethyl)-1H-pyrazolo[4,3-c]pyridin-6-yl]-methanone |
| 60 | Azepan-1-yl-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 61 | Azepan-1-yl-[3-(6-methyl-imidazo[1,2-a]pyridin-3-yl)-1-(2,2,2-trifluoro-ethyl)-1H-pyrazolo[4,3-c]pyridin-6-yl]-methanone |
| 62 | Azepan-1-yl-[3-(8-fluoro-imidazo[1,2-a]pyridin-3-yl)-1-(2,2,2-trifluoro-ethyl)-1H-pyrazolo[4,3-c]pyridin-6-yl]-methanone |
| 63 | 3-[6-(Azepane-1-carbonyl)-1-(2,2,2-trifluoro-ethyl)-1H-pyrazolo[4,3-c]pyridin-3-yl]-7-hydroxy-imidazo[1,2-a]pyridine-6-carbonitrile |
| 64 | Azepan-1-yl-[3-[6-(1-hydroxy-ethyl)-imidazo[1,2-a]pyridin-3-yl]-1-(2,2,2-trifluoro-ethyl)-1H-pyrazolo[4,3-c]pyridin-6-yl]-methanone |
| 65 | Azepan-1-yl-[3-(6-cyclopropyl-imidazo[1,2-a]pyridin-3-yl)-1-(2,2,2-trifluoro-ethyl)-1H-pyrazolo[4,3-c]pyridin-6-yl]-methanone |
| 66 | Azepan-1-yl-[1-(2,2,2-trifluoro-ethyl)-3-(6-trifluoromethoxy-imidazo[1,2-a]pyridin-3-yl)-1H-pyrazolo[4,3-c]pyridin-6-yl]-methanone |
| 67 | Azepan-1-yl-[3-(7-fluoro-imidazo[1,2-a]pyridin-3-yl)-1-(2,2,2-trifluoro-ethyl)-1H-pyrazolo[4,3-c]pyridin-6-yl]-methanone |
| 68 | Azepan-1-yl-[3-(6-hydroxy-imidazo[1,2-a]pyridin-3-yl)-1-(2,2,2-trifluoro-ethyl)-1H-pyrazolo[4,3-c]pyridin-6-yl]-methanone |
| 69 | Azepan-1-yl-[3-(6-difluoromethyl-imidazo[1,2-a]pyridin-3-yl)-1-(2,2,2-trifluoro-ethyl)-1H-pyrazolo[4,3-c]pyridin-6-yl]-methanone |
| 70 | Azepan-1-yl-[3-[6-(2,2,2-trifluoro-ethoxy)-imidazo[1,2-a]pyridin-3-yl]-1-(2,2,2-trifluoro-ethyl)-1H-pyrazolo[4,3-c]pyridin-6-yl]-methanone |
| 71 | Azepan-1-yl-[3-[1,2,4]triazolo[4,3-a]pyridin-3-yl-1-(2,2,2-trifluoro-ethyl)-1H-pyrazolo[4,3-c]pyridin-6-yl]-methanone |
| 72 | Azepan-1-yl-[3-[6-(1-hydroxy-1-methyl-ethyl)-imidazo[1,2-a]pyridin-3-yl]-1-(2,2,2-trifluoro-ethyl)-1H-pyrazolo[4,3-c]pyridin-6-yl]-methanone |
| 73 | Azepan-1-yl-[3-(7-chloro-6-fluoro-imidazo[1,2-a]pyridin-3-yl)-1-(2,2,2-trifluoro-ethyl)-1H-pyrazolo[4,3-c]pyridin-6-yl]-methanone |
| 74 | Azepan-1-yl-[3-(6-chloro-7-fluoro-imidazo[1,2-a]pyridin-3-yl)-1-(2,2,2-trifluoro-ethyl)-1H-pyrazolo[4,3-c]pyridin-6-yl]-methanone |
| 75 | Azepan-1-yl-[3-(6-chloro-7-hydroxy-imidazo[1,2-a]pyridin-3-yl)-1-(2,2,2-trifluoro-ethyl)-1H-pyrazolo[4,3-c]pyridin-6-yl]-methanone |
| 76 | Azepan-1-yl-[3-(6,8-difluoro-imidazo[1,2-a]pyridin-3-yl)-1-(2-trimethylsilanyl-ethoxymethyl)-1H-pyrazolo[4,3-c]pyridin-6-yl]-methanone |
| 77 | Azepan-1-yl-[3-(6-fluoro-imidazo[1,2-a]pyridin-3-yl)-1-(2-trimethylsilanyl-ethoxymethyl)-1H-pyrazolo[4,3-c]pyridin-6-yl]-methanone |
| 78 | Azepan-1-yl-[3-(6,8-difluoro-imidazo[1,2-a]pyridin-3-yl)-1-(4-methoxy-benzyl)-1H-pyrazolo[4,3-c]pyridin-6-yl]-methanone |
| 79 | Azepan-1-yl-[3-(6,8-difluoro-imidazo[1,2-a]pyridin-3-yl)-1-(tetrahydro-pyran-2-yl)-1H-pyrazolo[4, 3-c]pyridin-6-yl]-methanone |
| 80 | [3-(6-Fluoro-imidazo[1,2-a]pyridin-3-yl)-1-(2,2,2-trifluoro-ethyl)-1H-pyrazolo[4,3-c]pyridin-6-yl]-1,4-oxazepan-4-yl-methanone |
| 81 | 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carbonitrile |
| 82 | [3-(6-Fluoro-imidazo[1,2-a]pyridin-3-yl)-1-(2,2,2-trifluoro-ethyl)-1H-pyrazolo[4,3-c]pyridin-6-yl]-(8-oxa-3-aza-bicyclo[3.2.1]oct-3-yl)-methanone |
| 83 | [3-(6,8-Difluoro-imidazo[1,2-a]pyridin-3-yl)-1-(2,2,2-trifluoro-ethyl)-1H-pyrazolo[4,3c]pyridin-6-yl]-(8-oxa-3-aza-bicyclo[3.2.1]oct-3-yl)-methanone |
| 84 | 3-[6-(8-Oxa-3-aza-bicyclo[3.2.1]octane-3-carbonyl)-1-(2,2,2-trifluoro-ethyl)-1H-pyrazolo[4,3-c]pyridin-3-yl]-imidazo[1,2-a]pyridine-6-carbonitrile |
| 85 | [3-(8-fluoroimidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone |
| 86 | [3-(6-fluoroimidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone |
| 87 | [3-(imidazo[1,2-a]pyridin-3-yl)-1-(2,2,2-trifluoroethyl)pyrazolo[4,3-c]pyridin-6-yl]-(3-endo-hydroxy-8-azabicyclo[3.2.1]octan-8-yl)methanone |
| 88 | (3-endo-hydroxy-8-azabicyclo[3.2.1]octan-8-yl)-[3-(6-hydroxyimidazo[1,2-a]pyridin-3-yl)-1-(2,2,2-trifluoroethyl)pyrazolo[4,3-c]pyridin-6-yl]methanone |
| 89 | Azepan-1-yl-[3-pyrazolo[1,5-a]pyridin-3-yl-1-(2,2,2-trifluoro-ethyl)-1H-pyrazolo[4,3-c]pyridin-6-yl]-methanone hydrochloride |
| 90 | Azepan-1-yl-[3-benzofuran-3-yl-1-(2,2,2-trifluoro-ethyl)-1H-pyrazolo[4,3-c]pyridin-6-yl]-methanone |
| 91 | Azepan-1-yl-[3-(1H-indol-3-yl)-1-(2,2,2-trifluoro-ethyl)-1H-pyrazolo[4,3-c]pyridin-6-yl]-methanone |
| 92 | Azepan-1-yl-[3-(1H-pyrrolo[2,3-b]pyridin-3-yl)-1-(2,2,2-trifluoro-ethyl)-1H-pyrazolo[4,3-c]pyridin-6-yl]-methanone |
| 93 | 1,4-Oxazepan-4-yl-[3-thieno[2,3-c]pyridin-3-yl-1-(2,2,2-trifluoro-ethyl)-1H-pyrazolo[4,3-c]pyridin-6-yl]-methanone |
| 94 | [3-Benzofuran-3-yl-1-(2,2,2-trifluoro-ethyl)-1H-pyrazolo[4,3-c]pyridine-6-yl]-1,4-oxazepan-4-yl-methanone |
| 95 | 1,4-Oxazepan-4-yl-[3-pyrazolo[1,5-a]pyridine-3-yl-1-(2,2,2-trifluoro-ethyl)-1H-pyrazolo[4,3-c]pyridine-6-yl]-methanone |
| 96 | [3-(1H-Indol-3-yl)-1-(2,2,2-trifluoro-ethyl)-1H-pyrazolo[4,3-c]pyridine-6-yl]-1,4-oxazepan-4-yl-methanone |
| 97 | 1,4-Oxazepan-4-yl-[3-(1H-pyrrolo[2,3-b]pyridin-3-yl)-1-(2,2,2-trifluoro-ethyl)-1H-pyrazolo[4,3-c]pyridine-6-yl]-methanone |
| 98 | [3-Benzo[b]thiophen-3-yl-1-(2,2,2-trifluoro-ethyl)-1H-pyrazolo[4,3-c]pyridine-6-yl]-1,4-oxazepan-4-yl-methanone |
| 99 | (8-Oxa-3-aza-bicyclo[3.2.1]oct-3-yl)-[3-pyrazolo[1,5-a]pyridin-3-yl-1-(2,2,2-trifluoro-ethyl)-1H-pyrazolo[4,3-c]pyridin-6-yl]-methanone |
| 100 | [3-Benzofuran-3-yl-1-(2,2,2-trifluoro-ethyl)-1H-pyrazolo[4,3-c]pyridin-6-yl]-(8-oxa-3-aza-bicyclo[3.2. 1]oct-3-yl)-methanone |
| 101 | (3-Aza-bicyclo[3.2.1]oct-3-yl)-[3-benzofuran-3-yl-1-(2,2,2-trifluoro-ethyl)-1H-pyrazolo[4,3-c]pyridin-6-yl]-methanone |
| 102 | (3-Aza-bicyclo[3.2.1]oct-3-yl)-[3-pyrazolo[1,5-a]pyridin-3-yl-1-(2,2,2-trifluoro-ethyl)-1H-pyrazolo[4,3-c]pyridin-6-yl]-methanone |
| 103 | [3-Benzofuran-3-yl-1-(2,2-difluoro-ethyl)-1H-pyrazolo[4,3-c]pyridin-6-yl]-1,4-oxazepan-4-yl-methanone |
| 104 | [3-Benzofuran-3-yl-1-(2-fluoro-ethyl)-1H-pyrazolo[4,3-c]pyridin-6-yl]-1,4-oxazepan-4-yl-methanone |
| 105 | 2-[3-Benzofuran-3-yl-6-(1,4-oxazepane-4-carbonyl)-pyrazolo[4,3-c]pyridin-1-yl]-acetamide |
| 106 | (3-Benzofuran-3-yl-1-propyl-1H-pyrazolo[4,3-c]pyridin-6-yl)-1,4-oxazepan-4-yl-methanone |
| 107 | (3-Benzofuran-3-yl-1-methanesulfonylmethyl-1H-pyrazolo[4,3-c]pyridine-6-yl)-(8-oxa-3-aza-bicyclo[3.2.1]oct-3-yl)-methanone |
| 108 | (1-Methanesulfonylmethyl-3-pyrazolo[1,5-a]pyridine-3-yl-1H-pyrazolo[4,3-c]pyridine-6-yl)- (8-oxa-3-aza-bicyclo[3.2.1]oct-3-yl)-methanone |
| 109 | [3-(5-fluorobenzofuran-3-yl)-1-(2,2,2-trifluoroethyl)pyrazolo[4,3-c]pyridin-6-yl]-(1,4-oxazepan-4-yl)methanone |
| 110 | [3-(5-fluorobenzofuran-3-yl)-1-(methylsulfonylmethyl)pyrazolo[4,3-c]pyridin-6-yl]-(1,4-oxazepan-4-yl)methanone |
| 111 | [3-(5-fluorobenzofuran-3-yl)-1-(methylsulfonylmethyl)pyrazolo[4,3-c]pyridin-6-yl]-(8-oxa-3-azabicyclo[3.2.1]octan-3-yl)methanone |
| 112 | (3-endo-hydroxy-8-azabicyclo[3.2.1]octan-8-yl)-[3-(1H-indol-3-yl)-1-(2,2,2-trifluoroethyl)pyrazolo[4,3-c]pyridin-6-yl]methanone |
| 113 | (3-endo-hydroxy-8-azabicyclo[3.2.1]octan-8-yl)-[3-pyrazolo[1,5-a]pyridin-3-yl-1-(2,2,2-trifluoroethyl)pyrazolo[4,3-c]pyridin-6-yl]methanone |
| 114 | (4-hydroxy-1-piperidyl)-[3-pyrazolo[1,5-a]pyridin-3-yl-1-(2,2,2-trifluoroethyl)pyrazolo[4,3-c]pyridin-6-yl]methanone |
| 115 | [3-(5-fluorobenzofuran-3-yl)-1-(2,2,2-trifluoroethyl)pyrazolo[4,3-c]pyridin-6-yl]-(4-hydroxy-1-piperidyl)methanone |
| 116 | [3-(5-fluorobenzofuran-3-yl)-1-(2,2,2-trifluoroethyl)pyrazolo[4,3-c]pyridin-6-yl]-(4-hydroxy-4-methyl-1-piperidyl)methanone |
| 117 | (4-hydroxy-4-methyl-1-piperidyl)-[3-pyrazolo[1,5-a]pyridin-3-yl-1-(2,2,2-trifluoroethyl)pyrazolo[4,3-c]pyridin-6-yl]methanone |
| 118 | [3-(5-fluorobenzofuran-3-yl)-1-(2,2,2-trifluoroethyl)pyrazolo[4,3-c]pyridin-6-yl]-(6-hydroxy-1,4-oxazepan-4-yl)methanone |
| 119 | (6-hydroxy-1,4-oxazepan-4-yl)-[3-pyrazolo[1,5-a]pyridin-3-yl-1-(2,2,2-trifluoroethyl)pyrazolo[4,3-c]pyridin-6-yl]methanone |
| 120 | [3-(5-fluorobenzofuran-3-yl)-1-(2,2,2-trifluoroethyl)pyrazolo[4,3-c]pyridin-6-yl]-(6,6-dideuterio-1,4-oxazepan-4-yl)methanone |
| 121 | (6,6-dideuterio-1,4-oxazepan-4-yl)-[3-pyrazolo[1,5-a]pyridin-3-yl-1-(2,2,2-trifluoroethyl)pyrazolo[4,3-c]pyridin-6-yl]methanone |
| 122 | [3-(5-fluorobenzofuran-3-yl)-1-(2,2,2-trifluoroethyl)pyrazolo[4,3-c]pyridin-6-yl]-(4-hydroxyazepan-1-yl)methanone |
| 123 | [3-(5-fluoro-1H-indol-3-yl)-1-(2,2,2-trifluoroethyl)pyrazolo[4,3-c]pyridin-6-yl]-(1,4-oxazepan-4-yl)methanone |
| 124 | methyl 3-[6-(1 ,4-oxazepane-4-carbonyl)-1-(2,2,2-trifluoroethyl)pyrazolo[4,3-c]pyridin-3-yl]benzofuran-5-carboxylate |
| 125 | [3-(5-methylbenzofuran-3-yl)-1-(2,2,2-trifluoroethyl)pyrazolo[4,3-c]pyridin-6-yl]-(1,4-oxazepan-4-yl)methanone |
| 126 | [3-(2-methylbenzofuran-3-yl)-1-(2,2,2-trifluoroethyl)pyrazolo[4,3-c]pyridin-6-yl]-(1,4-oxazepan-4-yl)methanone |
| 127 | [3-(5-fluorobenzofuran-3-yl)-1-(2,2,2-trifluoroethyl)pyrazolo[4,3-c]pyridin-6-yl]-(8-oxa-3-azabicyclo[3.2.1]octan-3-yl)methanone |
| 128 | 3-(6,8-Difluoro-imidazo[1,2-a]pyridin-3-yl)-1-(2,2,2-trifluoro-ethyl)-1H-pyrazolo[4,3-c]pyridine-6-carboxylic acid amide |
| 129 | 3-[6-(1,4-oxazepane-4-carbonyl)-1-(2,2,2-trifluoroethyl)pyrazolo[4,3-c]pyridin-3-yl]benzofuran-5-carboxylic acid |
| 130 | 1-[3-(benzofuran-3-yl)-1-(2,2,2-trifluoroethyl)pyrazolo[4,3-c]pyridine-6-carbonyl]piperidine-4-carboxylic acid |
| 131 | [3-(6-Fluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone |
| 132 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-piperidin-1-yl)-methanone |
| 133 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-methyl-piperidin-1-yl)-methanone |
| 134 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxy-azepan-1-yl)-methanone |
| 135 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[2.2.1]hept-5-yl)-methanone |
| 136 | [3-(6,8-Difluoro-[1,2,4]triazolo[4,3-a]pyridin-3-yl)-1-(2,2,2-trifluoro-ethyl)-1H-pyrazolo[4,3-c]pyridin-6-yl]-(3-hydroxy-8-aza-bicyclo[3.2.1]oct-8-yl)-methanone |
| 137 | [3-(6,8-Difluoro-[1,2,4]triazolo[4,3-a]pyridin-3-yl)-1-(2,2,2-trifluoro-ethyl)-1H-pyrazolo[4,3-c]pyridin-6-yl]-1,4-oxazepan-4-yl-methanone |
| 138 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-methyl-piperidin-1-yl)-methanone |
| 139 | (3-Aza-bicyclo[3.2.1]oct-3-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 140 | [3-(6,8-Difluoro-imidazo[1,2-a]pyridin-3-yl)-1-(2,2,2-trifluoro-ethyl)-1H-pyrazolo[4,3-c]pyridin-6-yl]-(9-hydroxy-3-aza-bicyclo[3.3.1]non-3-yl)-methanone |
| 141 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2-isopropyl-piperidin-1-yl)-methanone |
| 142 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-2,2-dimethyl-piperidin-1-yl)-methanone |
| 143 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-3-isobutyl-piperidin-1-yl)-methanone |
| 144 | [3-(6,8-Difluoro-imidazo[1,2-a]pyridin-3-yl)-1-(2,2,2-trifluoro-ethyl)-1H-pyrazolo[4,3-c]pyridin-6-yl]-(4-fluoro-piperidin-1-yl)-methanone |
| 145 | [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-2-aza-spiro[4.5]dec-2-yl)-methanone |
| 146 | [3-(6,8-Difluoro-imidazo[1,2-a]pyridin-3-yl)-1-(2,2,2-trifluoro-ethyl)-1H-pyrazolo[4,3-c]pyridin-6-yl]-(4,4-difluoro-piperidin-1-yl)-methanone |
| 147 | [3-(6,8-Difluoro-imidazo[1,2-a]pyridin-3-yl)-1-(2,2,2-trifluoro-ethyl)-1H-pyrazolo[4,3-c]pyridin-6-yl]-(4'-hydroxy-3',4',5',6'-tetrahydro-2'H-[3,4']bipyridinyl-1'-yl)-methanone |
| 148 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-4-trifluoromethyl-piperidin-1-yl)-methanone |
| 149 | [3-(6,8-Difluoro-imidazo[1,2-a]pyridin-3-yl)-1-(2,2,2-trifluoro-ethyl)-1H-pyrazolo[4,3-c]pyridin-6-yl]-(2-methoxymethyl-pyrrolidin-1-yl)-methanone |
| 150 | [3-(6,8-Difluoro-imidazo[1,2-a]pyridin-3-yl)-1-(2,2,2-trifluoro-ethyl)-1H-pyrazolo[4,3-c]pyridin-6-yl]-(4-methyl-1,4-diazepan-1-yl)-methanone |
| 151 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxy-piperidin-1-yl)-methanone |
| 152 | [3-(6,8-Difluoro-imidazo[1,2-a]pyridin-3-yl)-1-(2,2,2-trifluoro-ethyl)-1H-pyrazolo[4,3-c]pyridin-6-yl]-(7-oxa-2-aza-spiro[4.5]dec-2-yl)-methanone |
| 153 | [3-(6,8-Difluoro-imidazo[1,2-a]pyridin-3-yl)-1-(2,2,2-trifluoro-ethyl)-1H-pyrazolo[4,3-c]pyridin-6-yl]-(3-oxa-9-aza-spiro[5.5]undec-9-yl)-methanone |
| 154 | [3-(6,8-Difluoro-imidazo[1,2-a]pyridin-3-yl)-1-(2,2,2-trifluoro-ethyl)-1H-pyrazolo[4,3-c]pyridin-6-yl]-(tetrahydro-furo[3,4-c]pyrrol-5-yl)-methanone |
| 155 | 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-3-one |
| 156 | 8-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,8-diaza-spiro[4.5]decan-1-one |
| 157 | [3-(6,8-Difluoro-imidazo[1,2-a]pyridin-3-yl)-1-(2,2,2-trifluoro-ethyl)-1H-pyrazolo[4,3-c]pyridin-6-yl]-[3-(2-methoxy-ethyl)-pyrrolidin-1-yl]-methanone |
| 158 | [3-(6,8-Difluoro-imidazo[1,2-a]pyridin-3-yl)-1-(2,2,2-trifluoro-ethyl)-1H-pyrazolo[4,3-c]pyridin-6-yl]-morpholin-4-yl-methanone |
| 159 | [3-(6,8-Difluoro-imidazo[1,2-a]pyridin-3-yl)-1-(2,2,2-trifluoro-ethyl)-1H-pyrazolo[4,3-c]pyridin-6-yl]-(3,3-difluoro-piperidin-1-yl)-methanone |
| 160 | [3-(6,8-Difluoro-imidazo[1,2-a]pyridin-3-yl)-1-(2,2,2-trifluoro-ethyl)-1H-pyrazolo[4,3-c]pyridin-6-yl]-(3,3-difluoro-piperidin-1-yl)-methanone |
| 161 | [3-(6,8-Difluoro-imidazo[1,2-a]pyridin-3-yl)-1-(2,2,2-trifluoro-ethyl)-1H-pyrazolo[4,3-c]pyridin-6-yl]-(3-hydroxymethyl-piperidin-1-yl)-methanone |
| 162 | [3-(6,8-Difluoro-imidazo[1,2-a]pyridin-3-yl)-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone |
| 163 | [3-(6,8-Difluoro-imidazo[1,2-a]pyridin-3-yl)-1-(2,2,2-trifluoro-ethyl)-1H-pyrazolo[4,3-c]pyridin-6-yl]-(2-trifluoromethyl-morpholin-4-yl)-methanone |
| 164 | [3-(6,8-Difluoro-imidazo[1,2-a]pyridin-3-yl)-1-(2,2,2-trifluoro-ethyl)-1H-pyrazolo[4,3-c]pyridin-6-yl]-(4-trifluoromethyl-piperidin-1-yl)-methanone |
| 165 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-piperidin-1-yl)-methanone |
| 166 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-7-aza-spiro[3.5]non-7-yl)-methanone |
| 167 | [3-(6,8-Difluoro-imidazo[1,2-a]pyridin-3-yl)-1-(2,2,2-trifluoro-ethyl)-1H-pyrazolo[4,3-c]pyridin-6-yl]-(3-methoxymethyl-morpholin-4-yl)-methanone |
| 168 | [3-(6,8-Difluoro-imidazo[1,2-a]pyridin-3-yl)-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone |
| 169 | (4-Aza-tricyclo[4.3.1.1*3,8*]undec-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 170 | [3-(6,8-Difluoro-imidazo[1,2-a]pyridin-3-yl)-1-(2,2,2-trifluoro-ethyl)-1H-pyrazolo[4,3-c]pyridin-6-yl]-(8-exo-hydroxy-3-aza-bicyclo[3.2.1]oct-3-yl)-methanone |
| 171 | [3-(6,8-Difluoro-imidazo[1,2-a]pyridin-3-yl)-1-(2,2,2-trifluoro-ethyl)-1H-pyrazolo[4,3-c]pyridin-6-yl]-(3-exo-hydroxy-8-aza-bicyclo[3.2.1]oct-8-yl)-methanone |
| 172 | (8-Aza-bicyclo[3.2.1]oct-8-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 173 | [3-(6,8-Difluoro-imidazo[1,2-a]pyridin-3-yl)-1-(2,2,2-trifluoro-ethyl)-1H-pyrazolo[4,3-c]pyridin-6-yl]-(4-difluoromethyl-piperidin-1-yl)-methanone |
| 174 | 3-(6,8-Difluoro-imidazo[1,2-a]pyridin-3-yl)-1-(2,2,2-trifluoro-ethyl)-1H-pyrazolo[4,3-c]pyridine-6-carboxylic acid (2-hydroxy-2-methyl-propyl)-amide |
| 175 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-6-aza-tricyclo[3.3.1.1*3,7*]dec-6-yl)-methanone |
| 176 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxymethyl-4-methyl-piperidin-1-yl)-methanone |
| 177 | [3-(6,8-Difluoro-imidazo[1,2-a]pyridin-3-yl)-1-(2,2,2-trifluoro-ethyl)-1H-pyrazolo[4,3-c]pyridin-6-yl]-(4-hydroxy-azepan-1-yl)-methanone |
| 178 | (2-Aza-tricyclo[3.3.1.1*3,7*]dec-2-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 179 | [3-(6,8-Difluoro-imidazo[1,2-a]pyridin-3-yl)-1-(2,2,2-trifluoro-ethyl)-1H-pyrazolo[4,3-c]pyridin-6-yl]-(5-oxa-8-aza-spiro[2.6]non-8-yl)-methanone |
| 180 | [3-(6,8-Difluoro-imidazo[1,2-a]pyridin-3-yl)-1-(2,2,2-trifluoro-ethyl)-1H-pyrazolo[4,3-c]pyridin-6-yl]-1,4-thiazepan-1,1-dioxide-4-yl-methanone |
| 181 | Cis-[3-(6,8-Difluoro-imidazo[1,2-a]pyridin-3-yl)-1-(2,2,2-trifluoro-ethyl)-1H-pyrazolo[4,3-c]pyridin-6-yl]-(2,6-dimethyl-morpholin-4-yl)-methanone |
| 182 | [3-(6,8-Difluoro-imidazo[1,2-a]pyridin-3-yl)-1-(2,2,2-trifluoro-ethyl)-1H-pyrazolo[4,3-c]pyridin-6-yl]-(2 ,2-dimethyl-1,4-oxazepan-4-yl)-methanone |
| 183 | Cis-[3-(6,8-Difluoro-imidazo[1,2-a]pyridin-3-yl)-1-(2,2,2-trifluoro-ethyl)-1H-pyrazolo[4,3-c]pyridin-6-yl]-(3,5-dimethyl-morpholin-4-yl)-methanone |
| 184 | [3-(6,8-Difluoro-imidazo[1,2-a]pyridin-3-yl)-1-(2,2,2-trifluoro-ethyl)-1H-pyrazolo[4,3-c]pyridin-6-yl]-(3-oxa-8-aza-bicyclo[3.2.1]oct-8-yl)-methanone |
| 185 | [3-(6,8-Difluoro-imidazo[1,2-a]pyridin-3-yl)-1-(2,2,2-trifluoro-ethyl)-1H-pyrazolo[4,3-c]pyridin-6-yl]-(6,6-difluoro-1,4-oxazepan-4-yl)-methanone |
| 186 | [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-4-aza-spiro[2.6]non-4-yl)-methanone |
| 187 | [3-(6,8-Difluoro-imidazo[1,2-a]pyridin-3-yl)-1-(2,2,2-trifluoro-ethyl)-1H-pyrazolo[4,3-c]pyridin-6-yl]-(8-endo-hydroxy-3-aza-bicyclo[3.2.1]oct-3-yl)-methanone |
| 188 | [3-(6,8-Difluoro-imidazo[1,2-a]pyridin-3-yl)-1-(2,2,2-trifluoro-ethyl)-1H-pyrazolo[4,3-c]pyridin-6-yl]-(7-endo-hydroxy-3-oxa-9-aza-bicyclo[3.3.1]non-9-yl)-methanone |
| 189 | 1-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-diazepan-5-one |
| 190 | [3-(6,8-Difluoro-imidazo[1,2-a]pyridin-3-yl)-1-(2,2,2-trifluoro-ethyl)-1H-pyrazolo[4,3-c]pyridin-6-yl]-1,4-oxazepan-deuterium-4-yl-methanone |
| 191 | [3-(6,8-Difluoro-imidazo[1,2-a]pyridin-3-yl)-1-(2,2,2-trifluoro-ethyl)-1H-pyrazolo[4,3-c]pyridin-6-yl]-[1,2]oxazepan-2-yl-methanone |
| 192 | [3-(6,8-Difluoro-imidazo[1,2-a]pyridin-3-yl)-1-(2,2,2-trifluoro-ethyl)-1H-pyrazolo[4,3-c]pyridin-6-yl]-(5-oxa-8-aza-spiro[3.5]non-8-yl)-methanone |
| 193 | [3-(6,8-Difluoro-imidazo[1,2-a]pyridin-3-yl)-1-(2,2,2-trifluoro-ethyl)-1H-pyrazolo[4, 3-c] pyrid i n-6-yl]-(6-hyd roxy-1, 4-oxazepan-4-yl)-methanone |
| 194 | 5-[3-(6,8-Difluoro-imidazo[1,2-a]pyridin-3-yl)-1-(2,2,2-trifluoro-ethyl)-1H-pyrazolo[4,3-c]pyridine-6-carbonyl]-2,5-diaza-bicyclo[2.2.1]heptane-2-carboxylic acid tert-butyl ester |
| 195 | 1,4-Diazepan-1-yl-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 196 | [3-(6,8-Difluoro-imidazo[1,2-a]pyridin-3-yl)-1-(2,2,2-trifluoro-ethyl)-1H-pyrazolo[4,3-c]pyridin-6-yl]-(8-oxa-5-aza-spiro[3.5]non-5-yl)-methanone |
| 197 | (2-{[3-(6,8-Difluoro-imidazo[1,2-a]pyridin-3-yl)-1-(2,2,2-trifluoro-ethyl)-1H-pyrazolo[4,3-c]pyridine-6-carbonyl]-methyl-amino}-ethyl)-carbamic acid tert-butyl ester |
| 198 | 3-(6,8-Difluoro-imidazo[1,2-a]pyridin-3-yl)-1-(2,2,2-trifluoro-ethyl)-1H-pyrazolo[4,3-c]pyridine-6-carboxylic acid (4-hydroxy-tetrahydro-pyran-3-yl)-amide |
| 199 | [3-(6,8-Difluoro-imidazo[1,2-a]pyridin-3-yl)-1-(2,2,2-trifluoro-ethyl)-1H-pyrazolo[4,3-c]pyridin-6-yl]-(6-oxa-3-aza-bicyclo[3.2.1]oct-3-yl)-methanone |
| 200 | 3-[3-(6,8-Difluoro-imidazo[1,2-a]pyridin-3-yl)-1-(2,2,2-trifluoro-ethyl)-1H-pyrazolo[4,3-c]pyridine-6-carbonyl]-3,8-diaza-bicyclo[3.2.1]octane-8-carboxylic acid tert-butyl ester |
| 201 | 4-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-diazepan-2-one |
| 202 | {1-[3-(6,8-Difluoro-imidazo[1,2-a]pyridin-3-yl)-1-(2,2,2-trifluoro-ethyl)-1H-pyrazolo[4,3-c]pyridine-6-carbonyl]-azepan-3-yl}-carbamic acid tert-butyl ester |
| 203 | (3,3-Difluoro-4-hydroxy-piperidin-1-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 204 | {4-[3-(6,8-Difluoro-imidazo[1,2-a]pyridin-3-yl)-1-(2,2,2-trifluoro-ethyl)-1H-pyrazolo[4,3-c]pyridine-6-carbonyl]-1,4-oxazepan-6-yl}-carbamic acid tert-butyl ester |
| 205 | [3-(6,8-Difluoro-imidazo[1,2-a]pyridin-3-yl)-1-(2,2,2-trifluoro-ethyl)-1H-pyrazolo[4,3-c]pyridin-6-yl]-(2-oxa-5-aza-bicyclo[4.1.0]hept-5-yl)-methanone |
| 206 | [3-(6,8-Difluoro-imidazo[1,2-a]pyridin-3-yl)-1-(2,2,2-trifluoro-ethyl)-1H-pyrazolo[4,3-c]pyridin-6-yl]-(7-oxa-4-aza-spiro[2.6]non-4-yl)-methanone |
| 207 | [3-Benzofuran-3-yl-1-(2,2,2-trifluoro-ethyl)-1H-pyrazolo[4,3-c]pyridin-6-yl]-(4-hydroxy-piperidin-1-yl)-methanone |
| 208 | [3-Benzofuran-3-yl-1-(2,2,2-trifluoro-ethyl)-1H-pyrazolo[4,3-c]pyridin-6-yl]-(4-hydroxy-4-methyl-piperidin-1-yl)-methanone |
| 209 | [3-Benzofuran-3-yl-1-(2,2,2-trifluoro-ethyl)-1H-pyrazolo[4,3-c]pyridin-6-yl]-(3-endo-hydroxy-8-aza-bicyclo[3.2.1]oct-8-yl)-methanone |
| 210 | [3-Benzofuran-3-yl-1-(2,2,2-trifluoro-ethyl)-1H-pyrazolo[4,3-c]pyridin-6-yl]-(8-exo-ethyl-8-endo-hydroxy-3-aza-bicyclo[3.2.1]oct-3-yl)-methanone |
| 211 | [3-Benzofuran-3-yl-1-(2,2,2-trifluoro-ethyl)-1H-pyrazolo[4,3-c]pyridin-6-yl]-(5-endo-hydroxy-5-exo-methyl-2-aza-bicyclo[2.2.1]hept-2-yl)-methanone |
| 212 | [3-Benzofuran-3-yl-1-(2,2,2-trifluoro-ethyl)-1H-pyrazolo[4,3-c]pyridin-6-yl]-(8-endo-hydroxy-8-exo-methyl-3-aza-bicyclo[3.2.1]oct-3-yl)-methanone |
| 213 | [3-Benzofuran-3-yl-1-(2,2,2-trifluoro-ethyl)-1H-pyrazolo[4,3-c]pyridin-6-yl]-(8-endo-hydroxy-8-exo-methyl-d3-3-aza-bicyclo[3.2.1]oct-3-yl)-methanone |
| 214 | [3-Benzofuran-3-yl-1-(2,2,2-trifluoro-ethyl)-1H-pyrazolo[4,3-c]pyridin-6-yl]-(8-exo-ethynyl-8-endo-hydroxy-3-aza-bicyclo[3.2.1]oct-3-yl)-methanone |
| 215 | [3-Benzofuran-3-yl-1-(2,2,2-trifluoro-ethyl)-1H-pyrazolo[4,3-c]pyridin-6-yl]-(2-methoxymethyl-pyrrolidin-1-yl)-methanone |
| 216 | [3-Benzofuran-3-yl-1-(2,2,2-trifluoro-ethyl)-1H-pyrazolo[4,3-c]pyridin-6-yl]-(4-fluoro-piperidin-1-yl)-methanone |
| 217 | 3-Benzofuran-3-yl-1-(2,2,2-trifluoro-ethyl)-1H-pyrazolo[4,3-c]pyridin-6-yl]-(6,6-difluoro-1,4-oxazepan-4-yl)-methanone |
| 218 | [3-Benzofuran-3-yl-1-(2,2,2-trifluoro-ethyl)-1H-pyrazolo[4,3-c]pyridin-6-yl]-(8-endo-hydroxy-3-aza-bicyclo[3.2.1]oct-3-yl)-methanone |
| 219 | [3-Benzofuran-3-yl-1-(2,2,2-trifluoro-ethyl)-1H-pyrazolo[4,3-c]pyridin-6-yl]-(4-hydroxy-2,2-dimethyl-piperidin-1-yl)-methanone |
| 220 | [3-Benzofuran-3-yl-1-(2,2-difluoro-ethyl)-1H-pyrazolo[4,3-c]pyridin-6-yl]-(3-endo-hydroxy-8-aza-bicyclo[3.2.1]oct-8-yl)-methanone |
| 221 | [3-Benzofuran-3-yl-1-(2-fluoro-ethyl)-1H-pyrazolo[4,3-c]pyridin-6-yl]-(3-endo-hydroxy-8-aza-bicyclo[3.2.1]oct-8-yl)-methanone |
| 222 | (3-Benzofuran-3-yl-1-propyl-1H-pyrazolo[4,3-c]pyridin-6-yl)-(3-endo-hydroxy-8-aza-bicyclo[3.2.1]oct-8-yl)-methanone |
| 223 | tert-butyl N-[4-[3-(6,8-difluoroimidazo[1,2-a]pyridin-3-yl)-1-(2,2,2-trifluoroethyl)pyrazolo[4,3-c]pyridine-6-carbonyl]-1,4-oxazepan-6-yl]carbamate |
| 224 | [3-(benzofuran-3-yl)-1-(2,2,2-trifluoroethyl)pyrazolo[4,3-c]pyridin-6-yl]-(6-hydroxy-1,4-oxazepan-4-yl)methanone |
| 225 | [3-(benzofuran-3-yl)-1-(2,2,2-trifluoroethyl)pyrazolo[4,3-c]pyridin-6-yl]-(4,4-difluoro-1-piperidyl)methanone |
| 226 | [3-(benzofuran-3-yl)-1-(2,2,2-trifluoroethyl)pyrazolo[4,3-c]pyridin-6-yl]-(3,3-difluoro-1-piperidyl)methanone |
| 227 | [3-Benzofuran-3-yl-1-(2,2,2-trifluoro-ethyl)-1H-pyrazolo[4,3-c]pyridin-6-yl]-(3-hydroxy-piperidin-1-yl)-methanone |
| 228 | [3-(benzofuran-3-yl)-1-(2,2,2-trifluoroethyl)pyrazolo[4,3-c]pyridin-6-yl]-(4-hydroxyazepan-1-yl)methanone |
| 229 | [3-(benzofuran-3-yl)-1-(2,2,2-trifluoroethyl)pyrazolo[4,3-c]pyridin-6-yl]-(6,6-dideuterio-1,4-oxazepan-4-yl)methanone |
| 230 | [3-(benzofuran-3-yl)-1-(2,2,2-trifluoroethyl)pyrazolo[4,3-c]pyridin-6-yl]-[6-fluoro-1,4-oxazepan-4-yl]methanone |
| 231 | 3-(benzofuran-3-yl)-N,N-di(cyclobutyl)-1-(2,2,2-trifluoroethyl)pyrazolo[4,3-c]pyridine-6-carboxamide |
| 232 | 3-(benzofuran-3-yl)-N, N-di(cyclopropyl)-1-(2,2,2-trifluoroethyl)pyrazolo[4,3-c]pyridine-6-carboxamide |
| 233 | 3-(benzofuran-3-yl)-N, N-diisopropyl-1-(2,2,2-trifluoroethyl)pyrazolo[4,3-c]pyridine-6-carboxamide |
| 234 | 3-(benzofuran-3-yl)-N-ethyl-N-isopropyl-1-(2,2,2-trifluoroethyl)pyrazolo[4,3-c]pyridine-6-carboxamide |
| 235 | 3-(benzofuran-3-yl)-N-cyclohexyl-N-ethyl-1-(2,2,2-trifluoroethyl)pyrazolo[4,3-c]pyridine-6-carboxamide |
| 236 | [3-(benzofuran-3-yl)-1-(2,2,2-trifluoroethyl)pyrazolo[4,3-c]pyridin-6-yl]-(8-hydroxy-8-isopropyl-3-azabicyclo[3.2.1]octan-3-yl)methanone |
| 237 | 3-[6-(1,4-oxazepane-4-carbonyl)-1-(2,2,2-trifluoroethyl)pyrazolo[4,3-c]pyridin-3-yl]benzofuran-5-carboxamide |
| 238 | (3,8-Diaza-bicyclo[3.2.1]oct-3-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 239 | (2,5-Diaza-bicyclo[2.2.1]hept-2-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 240 | (3-Amino-azepan-1-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 241 | (6-Amino-1,4-oxazepan-4-yl)-[3-(6,8-difluoro-imidazo[1,2-a]pyridin-3-yl)-1-(2,2,2-trifluoro-ethyl)-1H-pyrazolo[4,3-c]pyridin-6-yl]-methanone |
| 242 | (6,6-dideuterio-1,4-oxazepan-4-yl)-[3-(1H-indol-3-yl)-1-(2,2,2-trifluoroethyl)pyrazolo[4,3-c]pyridin-6-yl]methanone |
| 243 | [3-(1H-indol-3-yl)-1-(2,2,2-trifluoroethyl)pyrazolo[4,3-c]pyridin-6-yl]-(8-oxa-3-azabicyclo[3.2.1]octan-3-yl)methanone |
| 244 | 3-[6-(Azepane-1-carbonyl)-1-(2,2,2-trifluoro-ethyl)-1H-pyrazolo[4,3-c]pyridin-3-yl]-8-methyl-imidazo[1,2-a]pyridine-6-carboxylic acid amide |
| 245 | 3-[6-(Azepane-1-carbonyl)-1-propyl-1H-pyrazolo[4,3-c]pyridin-3-yl]-imidazo[1,2-a]pyridine-6-carboxylic acid amide |
| 246 | 3-[6-(1,4-Oxazepane-4-carbonyl)-1-(2,2,2-trifluoro-ethyl)-1H-pyrazolo[4,3-c]pyridin-3-yl]-imidazo[1,2-a]pyridine-6-carboxylic acid amide |
| 247 | 3-[6-(8-Oxa-3-aza-bicyclo[3.2.1]octane-3-carbonyl)-1-(2,2,2-trifluoro-ethyl)-1H-pyrazolo[4,3-c]pyridin-3-yl]-imidazo[1,2-a]pyridine-6-carboxylic acid amide |
| 248 | Azepan-1-yl-[3-pyrazolo[1,5-a]pyrazin-3-yl-1-(2,2,2-trifluoro-ethyl)-1H-pyrazolo[4,3-c]pyridin-6-yl]-methanone |
| 249 | 1,4-Oxazepan-4-yl-[3-pyrazolo[1,5-a]pyrazin-3-yl-1-(2,2,2-trifluoro-ethyl)-1H-pyrazolo[4,3-c]pyridin-6-yl]-methanone |
| 250 | (1-Methanesulfonylmethyl-3-pyrazolo[1,5-a]pyrazin-3-yl-1H-pyrazolo[4,3-c]pyridin-6-yl)-(1,4-oxazepan-4-yl)-methanone |
| 251 | [3-(benzimidazol-1-yl)-1-(2,2,2-trifluoroethyl)pyrazolo[4,3-c]pyridin-6-yl]-(1,4-oxazepan-4-yl)methanone |
| 252 | [3-(6-fluorobenzimidazol-1-yl)-1-(2,2,2-trifluoroethyl)pyrazolo[4,3-c]pyridin-6-yl]-(1,4-oxazepan-4-yl)methanone |
| 253 | [3-furo[3,2-b]pyridin-3-yl-1-(2,2,2-trifluoroethyl)pyrazolo[4,3-c]pyridin-6-yl]-(1,4-oxazepan-4-yl)methanone |
| 254 | [1,4]Oxazepan-4-yl-[3-(1H-pyrrolo[3,2-b]pyridin-3-yl)-1-(2,2,2-trifluoro-ethyl)-1H-pyrazolo[4,3-c]pyridin-6-yl]-methanone |
| 255 | [1,4]Oxazepan-4-yl-[3-(1H-pyrrolo[2,3-c]pyridin-3-yl)-1-(2,2,2-trifluoro-ethyl)-1H-pyrazolo[4,3-c]pyridin-6-yl]-methanone |
| 256 | (8-Oxa-3-aza-bicyclo[3.2.1]oct-3-yl)-[3-(1H-pyrrolo[3,2-c]pyridin-3-yl)-1-(2,2,2-trifluoro-ethyl)-1H-pyrazolo[4,3-c]pyridin-6-yl]-methanone |
and pharmaceutically acceptable salts or solvates of any one of the aforementioned compounds.

12. The compound of any one of claims 1 to 11 for use as a medicament.

13. A pharmaceutical composition comprising the compound of any one of claims 1 to 11 and a pharmaceutically acceptable excipient.

14. The compound of any one of claims 1 to 11 or the pharmaceutical composition of claim 13 for use in the treatment or prevention of a hyperproliferative disease or disorder or an infectious disease or disorder.

15. Use of the compound of any one of claims 1 to 11 in the preparation of a medicament for the treatment or prevention of a hyperproliferative disease or disorder or an infectious disease or disorder.

16. The compound for use according to claim 14 or the pharmaceutical composition for use according to claim 14 or the use of claim 15 wherein the disease or disorder to be treated or prevented is a hyperproliferative disease or disorder, and wherein said hyperproliferative disease or disorder is cancer.

17. The compound for use according to claim 16 or the pharmaceutical composition for use according to claim 16 or the use of claim 16 wherein the cancer is selected from the group consisting of acute granulocytic leukemia, acute lymphocytic leukemia, acute myeloid leukemia, adrenal cortex cancer, bladder cancer, brain cancer, breast cancer, cervical cancer, cervical hyperplasia, cervical cancer, chorio cancer, chronic granulocytic leukemia, chronic lymphocytic leukemia, chronic myeloid leukemia, colon cancer, endometrial cancer, esophageal cancer, essential thrombocytosis, genitourinary carcinoma, glioma, glioblastoma, hairy cell leukemia, head and neck carcinoma, Hodgkin's disease, Kaposi's sarcoma, lung carcinoma, lymphoma, malignant carcinoid carcinoma, malignant hypercalcemia, malignant melanoma, malignant pancreatic insulinoma, medullary thyroid carcinoma, melanoma, multiple myeloma, mycosis fungoides, neuroblastoma, non-Hodgkin's lymphoma, non-small cell lung cancer, osteogenic sarcoma, ovarian carcinoma, pancreatic carcinoma, polycythemia vera, primary brain carcinoma, primary macroglobulinemia, prostatic cancer, renal cell cancer, rhabdomyosarcoma, skin cancer, small-cell lung cancer, soft-tissue sarcoma, squamous cell cancer, stomach cancer, testicular cancer, thyroid cancer and Wilms' tumor.

18. The compound for use according to claim 14 or the pharmaceutical composition for use according to claim 14 or the use of claim 15 wherein the disease or disorder to be treated or prevented is a hyperproliferative disease or disorder, and wherein said hyperproliferative disease or disorder is selected from the group consisting of age-related macular degeneration, Crohn's disease, cirrhosis, a chronic inflammatory-related disorder, proliferative diabetic retinopathy, proliferative vitreoretinopathy, retinopathy of prematurity, granulomatosis, immune hyperproliferation associated with organ or tissue transplantation, and an immunoproliferative disease/disorder.

19. The compound for use according to claim 18 or the pharmaceutical composition for use according to claim 18 or the use of claim 18 wherein the disease or disorder to be treated or prevented is an immunoproliferative disease or disorder, and wherein the immunoproliferative disease or disorder is selected from the group consisting of inflammatory bowel disease, psoriasis, rheumatoid arthritis, systemic lupus erythematosus (SLE), vascular hyperproliferation secondary to retinal hypoxia, and vasculitis.

20. The compound for use according to claim 14 or the pharmaceutical composition for use according to claim 14 or the use of claim 15 wherein the disease or disorder to be treated or prevented is an infectious disease or disorder, and wherein said infectious disease or disorder is selected from the group consisting of:
(a) virally induced infectious diseases which are caused by retroviruses, hepadnaviruses, herpesviruses, flaviviridae and/or adenoviruses, wherein the retroviruses are selected from lentiviruses or oncoretroviruses, wherein the lentivirus is selected from the group consisting of HIV-1, HIV-2, FIV, BIV, SIVs, SHIV, CAEV, VMV and EIAV, wherein the oncoretrovirus is selected from the group consisting of HTLV-I, HTLV-II and BLV, wherein the hepadnavirus is selected from the group consisting of HBV, GSHV and WHV, wherein the herpesvirus is selected from the group consisting of HSV I, HSV II, EBV, VZV, HCMV and HHV 8, and wherein the flaviviridae are selected from the group consisting of HCV, West nile and Yellow Fever;
(b) bacterial infectious diseases which are caused by Gram-positive bacteria, wherein the Gram-positive bacteria are selected from the group consisting of methicillin-susceptible and methicillin-resistant staphylococci (including Staphylococcus aureus, Staphylococcus epidermidis, Staphylococcus haemolyticus, Staphylococcus hominis, Staphylococcus saprophyticus, and coagulase-negative staphylococci), glycopeptides-intermediate susceptible Staphylococcus aureus (GISA), penicillin-susceptible and penicillin-resistant streptococci (including Streptococcus pneumoniae, Streptococcus pyogenes, Streptococcus agalactiae, Streptococcus avium, Streptococcus bovis, Streptococcus lactis, Streptococcus sanguis and Streptococci Group C (GCS), Streptococci Group G (GGS) and viridans streptococci), enterococci (including vancomycinsusceptible and vancomycin-resistant strains such as Enterococcus faecalis and Enterococcus faecium), Clostridium difficile, Listeria monocytogenes, Corynebacterium jeikeium, Chlamydia spp (including C. pneumoniae) and Mycobacterium tuberculosis;
(c) bacterial infectious diseases which are caused by Gram-negative bacteria, wherein the Gram-negative bacteria are selected from the group consisting of the genus Enterobacteriaceae, including Escherichia spp. (including Escherichia coli), Klebsiella spp., Enterobacter spp., Citrobacter spp., Serratia spp., Proteus spp., Providencia spp., Salmonella spp., Shigella spp., the genus Pseudomonas (including P. aeruginosa), Moraxella spp. (including M. catarrhalis), Haemophilus spp. and Neisseria spp.; and
(d) infectious diseases induced by intracellular active parasites selected from the group consisting of the phylum Apicomplexa, Sarcomastigophora (including Trypanosoma, Plasmodia, Leishmania, Babesia or Theileria), Cryptosporidia, Sacrocystida, Amoebia, Coccidia and Trichomonadia.

21. The compound for use according to any one of claims 14 or 16 to 20 or the pharmaceutical composition for use according to any one of claims 14 or 16 to 20 or the use of any one of claims 15 or 16 to 20, wherein said compound, said pharmaceutical composition or said medicament is to be administered in combination with one or more further therapeutic agents.

22. The compound for use according to any one of claims 14 or 16 to 21 or the pharmaceutical composition for use according to any one of claims 14 or 16 to 21 or the use of any one of claims 15 or 16 to 21 wherein the subject to be treated is a human.

23. A set or kit comprising separate packs of:
(a)a compound as defined in any one of claims 1 to 11; and
(b) a further therapeutic agent.

24. *In vitro* use of a compound as defined in any one of claims 1 to 11 as a dual adenosine A_{2A} and A_{2B} receptor antagonist.

## Patentansprüche

1. Verbindung der Formel (I) 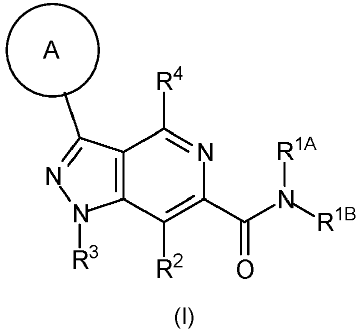 wobei:
R^{1A} und R^{1B} miteinander verknüpft sind, so dass sie, zusammen mit dem Stickstoffatom, an das sie gebunden sind, ein Heterocycloalkyl bilden, das gegebenenfalls mit einer oder mehreren Gruppen R¹¹ substituiert ist;
oder R^{1A} und R^{1B} alternativ jeweils unabhängig ausgewählt sind aus Wasserstoff, C₁₋₅-Alkyl, C₂₋₅-Alkenyl, C₂₋₅-Alkinyl, -CO(C₁₋₅-Alkyl), Carbocyclyl und Heterocyclyl, wobei das Alkyl, das Alkenyl, das Alkinyl und der Alkylteil des -CO(C₁₋₅-Alkyls) jeweils gegebenenfalls mit einer oder mehreren Gruppen R¹² substituiert sind und wobei ferner das Carbocyclyl und das Heterocyclyl jeweils gegebenenfalls mit einer oder mehreren Gruppen R¹³ substituiert sind;
R¹¹ jeweils unabhängig ausgewählt ist aus C₁₋₅-Alkyl, C₂₋₅-Alkenyl, C₂₋₅-Alkinyl, -(C₀₋₃-Alkylen)-OH, -(C₀₋₃-Alkylen)-O(C₁₋₅-alkyl), -(C₀₋₃-Alkylen)-O(C₁₋₅-alkylen)-OH, -(C₀₋₃-Alkylen)-O(C₁₋₅-alkylen)-O(C₁₋₅-alkyl), -(C₀₋₃-Alkylen)-SH, -(C₀₋₃-Alkylen)-S(C₁₋₅-alkyl), -(C₀₋₃-Alkylen)-NH₂, -(C₀₋₃-Alkylen)-NH(C₁₋₅-alkyl), -(C₀₋₃-Alkylen)-N(C₁₋₅-alkyl)(C₁₋₅-alkyl), -(C₀₋₃-Alkylen)-halogen, -(C₀₋₃-Alkylen)-(C₁₋₅-halogenalkyl), -(C₀₋₃-Alkylen)-O-(C₁₋₅-halogenalkyl), -(C₀₋₃-Alkylen)-CF₃, -(C₀₋₃-Alkylen)-CN, -(C₀₋₃-Alkylen)-NO₂, -(C₀₋₃-Alkylen)-CHO, -(C₀₋₃-Alkylen)-CO-(C₁₋₅-alkyl), -(C₀₋₃-Alkylen)-COOH, -(C₀₋₃-Alkylen)-CO-O-(C₁₋₅-alkyl), -(C₀₋₃-Alkylen)-O-CO-(C₁₋₅-alkyl), -(C₀₋₃-Alkylen)-CO-NH₂, -(C₀₋₃-Alkylen)-CO-NH(C₁₋₅-alkyl), -(C₀₋₃-Alkylen)-CO-N(C₁₋₅-alkyl)(C₁₋₅-alkyl), -(C₀₋₃-Alkylen)-NH-CO-(C₁₋₅-alkyl), -(C₀₋₃-Alkylen)-N(C₁₋₅-alkyl)-CO-(C₁₋₅-alkyl), -(C₀₋₃-Alkylen)-NH-CO-O-(C₁₋₅-alkyl), -(C₀₋₃-Alkylen)-N(C₁₋₅-alkyl)-CO-O-(C₁₋₅-alkyl), -(C₀₋₃-Alkylen)-O-CO-NH-(C₁₋₅-alkyl), -(C₀₋₃-Alkylen)-O-CO-N(C₁₋₅-alkyl)-(C₁₋₅-alkyl), -(C₀₋₃-Alkylen)-SO₂-NH₂, -(C₀₋₃-Alkylen)-SO₂-NH(C₁₋₅-alkyl), -(C₀₋₃-Alkylen)-SO₂-N(C₁₋₅-alkyl)(C₁₋₅-alkyl), -(C₀₋₃-Alkylen)-NH-SO₂-(C₁₋₅-alkyl), -(C₀₋₃-Alkylen)-N(C₁₋₅-alkyl)-SO₂-(C₁₋₅-alkyl), -(C₀₋₃-Alkylen)-SO-(C₁₋₅-alkyl), -(C₀₋₃-Alkylen)-SO₂-(C₁₋₅-alkyl), -(C₀₋₃-Alkylen)-carbocyclyl und -(C₀₋₃-Alkylen)-heterocyclyl, wobei der Carbocyclylteil des -(C₀₋₃-Alkylen)-carbocyclyls und der Heterocyclylteil des -(C₀₋₃-Alkylen)-heterocyclyls jeweils gegebenenfalls mit einer oder mehreren Gruppen substituiert sind, die unabhängig ausgewählt sind aus C₁₋₅-Alkyl, C₂₋₅-Alkenyl, C₂₋₅-Alkinyl, -OH, -O(C₁₋₅-Alkyl), -O(C₁₋₅-Alkylen)-OH, -O(C₁₋₅-Alkylen)-O(C₁₋₅-alkyl), -SH, -S(C₁₋₅-Alkyl), -NH₂, -NH(C₁₋₅-Alkyl), -N(C₁₋₅-Alkyl)(C₁₋₅-alkyl), Halogen, C₁₋₅-Halogenalkyl, -O-(C₁₋₅-Halogenalkyl), -CF₃, -CN, -NO₂, -CHO, -CO-(C₁₋₅-Alkyl), -COOH, -CO-O-(C₁₋₅-Alkyl), -O-CO-(C₁₋₅-Alkyl), -CO-NH₂, -CO-NH(C₁₋₅-Alkyl), -CO-N(C₁₋₅-Alkyl)(C₁₋₅-Alkyl), -NH-CO-(C₁₋₅-Alkyl), -N(C₁₋₅-Alkyl)-CO-(C₁₋₅-alkyl), -NH-CO-O-(C₁₋₅-Alkyl), -N(C₁₋₅-Alkyl)-CO-O-(C₁₋₅-alkyl), -O-CO-NH-(C₁₋₅-Alkyl), -O-CO-N(C₁₋₅-Alkyl)-(C₁₋₅-alkyl), -SO₂-NH₂, -SO₂-NH(C₁₋₅-Alkyl), -SO₂-N(C₁₋₅-Alkyl)(C₁₋₅-alkyl), -NH-SO₂-(C₁₋₅-Alkyl), -N(C₁₋₅-Alkyl)-SO₂-(C₁₋₅-alkyl), -SO-(C₁₋₅-Alkyl), -SO₂-(C₁₋₅-Alkyl), Cycloalkyl und Heterocycloalkyl;
R¹² jeweils unabhängig ausgewählt ist aus -OH, -O(C₁₋₅-Alkyl), -O(C₁₋₅-Alkylen)-OH, -O(C₁₋₅-Alkylen)-O(C₁₋₅-alkyl), -SH, -S(C₁₋₅-Alkyl), -NH₂, -NH(C₁₋₅-Alkyl), -N(C₁₋₅-Alkyl)(C₁₋₅-alkyl), Halogen, C₁₋₅-Halogenalkyl, -O-(C₁₋₅-Halogenalkyl), -CF₃, -CN, -NO₂, -CHO, -CO-(C₁₋₅-Alkyl), -COOH, -CO-O-(C₁₋₅-Alkyl), -O-CO-(C₁₋₅-Alkyl), -CO-NH₂, -CO-NH(C₁₋₅-Alkyl), -CO-N(C₁₋₅-Alkyl)(C₁₋₅-alkyl), -NH-CO-(C₁₋₅-Alkyl), -N(C₁₋₅-Alkyl)-CO-(C₁₋₅-alkyl), -NH-CO-O-(C₁₋₅-Alkyl), -N(C₁₋₅-Alkyl)-CO-O-(C₁₋₅-alkyl), -O-CO-NH-(C₁₋₅-Alkyl), -O-CO-N(C₁₋₅-Alkyl)-(C₁₋₅-alkyl), -SO₂-NH₂, -SO₂-NH(C₁₋₅-Alkyl), -SO₂-N(C₁₋₅-Alkyl)(C₁₋₅-alkyl), -NH-SO₂-(C₁₋₅-Alkyl), -N(C₁₋₅-Alkyl)-SO₂-(C₁₋₅-alkyl), -SO-(C₁₋₅-Alkyl), -SO₂-(C₁₋₅-Alkyl), Cycloalkyl und Heterocycloalkyl;
R¹³ jeweils unabhängig ausgewählt ist aus C₁₋₅-Alkyl, C₂₋₅-Alkenyl, C₂₋₅-Alkinyl, -(C₀₋₃-Alkylen)-OH, -(C₀₋₃-Alkylen)-O(C₁₋₅-alkyl), -(C₀₋₃-Alkylen)-O(C₁₋₅-alkylen)-OH, -(C₀₋₃-Alkylen)-O(C₁₋₅-alkylen)-O(C₁₋₅-alkyl), -(C₀₋₃-Alkylen)-SH, -(C₀₋₃-Alkylen)-S(C₁₋₅-alkyl), -(C₀₋₃-Alkylen)-NH₂, -(C₀₋₃-Alkylen)-NH(C₁₋₅-alkyl), -(C₀₋₃-Alkylen)-N(C₁₋₅-alkyl)(C₁₋₅-alkyl), -(C₀₋₃-Alkylen)-halogen, -(C₀₋₃-Alkylen)-(C₁₋₅-halogenalkyl), -(C₀₋₃-Alkylen)-O-(C₁₋₅-halogenalkyl), -(C₀₋₃-Alkylen)-CF₃, -(C₀₋₃-Alkylen)-CN, -(C₀₋₃-Alkylen)-NO₂, -(C₀₋₃-Alkylen)-CHO, -(C₀₋₃-Alkylen)-CO-(C₁₋₅-alkyl), -(C₀₋₃-Alkylen)-COOH, -(C₀₋₃-Alkylen)-CO-O-(C₁₋₅-alkyl), -(C₀₋₃-Alkylen)-O-CO-(C₁₋₅-alkyl), -(C₀₋₃-Alkylen)-CO-NH₂, -(C₀₋₃-Alkylen)-CO-NH(C₁₋₅-alkyl), -(C₀₋₃-Alkylen)-CO-N(C₁₋₅-alkyl)(C₁₋₅-alkyl), -(C₀₋₃-Alkylen)-NH-CO-(C₁₋₅-alkyl), -(C₀₋₃-Alkylen)-N(C₁₋₅-alkyl)-CO-(C₁₋₅-alkyl), -(C₀₋₃-Alkylen)-NH-CO-O-(C₁₋₅-alkyl), -(C₀₋₃-Alkylen)-N(C₁₋₅-alkyl)-CO-O-(C₁₋₅-alkyl), -(C₀₋₃-Alkylen)-O-CO-NH-(C₁₋₅-alkyl), -(C₀₋₃-Alkylen)-O-CO-N(C₁₋₅-alkyl)-(C₁₋₅-alkyl), -(C₀₋₃-Alkylen)-SO₂-NH₂, -(C₀₋₃-Alkylen)-SO₂-NH(C₁₋₅-alkyl), -(C₀₋₃-Alkylen)-SO₂-N(C₁₋₅-alkyl)(C₁₋₅-alkyl), -(C₀₋₃-Alkylen)-NH-SO₂-(C₁₋₅-alkyl), -(C₀₋₃-Alkylen)-N(C₁₋₅-alkyl)-SO₂-(C₁₋₅-alkyl), -(C₀₋₃-Alkylen)-SO-(C₁₋₅-alkyl), -(C₀₋₃-Alkylen)-SO₂-(C₁₋₅-alkyl), -(C₀₋₃-Alkylen)-cycloalkyl und -(C₀₋₃-Alkylen)-heterocycloalkyl;
R² und R⁴ jeweils unabhängig ausgewählt sind aus Wasserstoff, C₁₋₅-Alkyl, C₂₋₅-Alkenyl, C₂₋₅-Alkinyl, -(C₀₋₃-Alkylen)-OH, -(C₀₋₃-Alkylen)-O(C₁₋₅-alkyl), -(C₀₋₃-Alkylen)-O(C₁₋₅-alkylen)-OH, -(C₀₋₃-Alkylen)-O(C₁₋₅-alkylen)-O(C₁₋₅-alkyl), -(C₀₋₃-Alkylen)-SH, -(C₀₋₃-Alkylen)-S(C₁₋₅-alkyl), -(C₀₋₃-Alkylen)-NH₂, -(C₀₋₃-Alkylen)-NH(C₁₋₅-alkyl), -(C₀₋₃-Alkylen)-N(C₁₋₅-alkyl)(C₁₋₅-alkyl), -(C₀₋₃-Alkylen)-halogen, -(C₀₋₃-Alkylen)-(C₁₋₅-halogenalkyl), -(C₀₋₃-Alkylen)-O-(C₁₋₅-halogenalkyl), -(C₀₋₃-Alkylen)-CF₃, -(C₀₋₃-Alkylen)-CN, -(C₀₋₃-Alkylen)-NO₂, -(C₀₋₃-Alkylen)-CHO, -(C₀₋₃-Alkylen)-CO-(C₁₋₅-alkyl), -(C₀₋₃-Alkylen)-COOH, -(C₀₋₃-Alkylen)-CO-O-(C₁₋₅-alkyl), -(C₀₋₃-Alkylen)-O-CO-(C₁₋₅-alkyl), -(C₀₋₃-Alkylen)-CO-NH₂, -(C₀₋₃-Alkylen)-CO-NH(C₁₋₅-alkyl), -(C₀₋₃-Alkylen)-CO-N(C₁₋₅-alkyl)(C₁₋₅-alkyl), -(C₀₋₃-Alkylen)-NH-CO-(C₁₋₅-alkyl), -(C₀₋₃-Alkylen)-N(C₁₋₅-alkyl)-CO-(C₁₋₅-alkyl), -(C₀₋₃-Alkylen)-NH-CO-O-(C₁₋₅-alkyl), -(C₀₋₃-Alkylen)-N(C₁₋₅-alkyl)-CO-O-(C₁₋₅-alkyl), -(C₀₋₃-Alkylen)-O-CO-NH-(C₁₋₅-alkyl), -(C₀₋₃-Alkylen)-O-CO-N(C₁₋₅-alkyl)-(C₁₋₅-alkyl), -(C₀₋₃-Alkylen)-SO₂-NH₂, -(C₀₋₃-Alkylen)-SO₂-NH(C₁₋₅-alkyl), -(C₀₋₃-Alkylen)-SO₂-N(C₁₋₅-alkyl)(C₁₋₅-alkyl), -(C₀₋₃-Alkylen)-NH-SO₂-(C₁₋₅-alkyl), -(C₀₋₃-Alkylen)-N(C₁₋₅-alkyl)-SO₂-(C₁₋₅-alkyl), -(C₀₋₃-Alkylen)-SO-(C₁₋₅-alkyl), -(C₀₋₃-Alkylen)-SO₂-(C₁₋₅-alkyl), -(C₀₋₃-Alkylen)-carbocyclyl und -(C₀₋₃-Alkylen)-heterocyclyl, wobei der Carbocyclylteil des -(C₀₋₃-Alkylen)-carbocyclyls und der Heterocyclylteil des -(C₀₋₃-Alkylen)-heterocyclyls jeweils gegebenenfalls mit einer oder mehreren Gruppen substituiert sind, die unabhängig ausgewählt sind aus C₁₋₅-Alkyl, C₂₋₅-Alkenyl, C₂₋₅-Alkinyl, -OH, -O(C₁₋₅-Alkyl), -O(C₁₋₅-Alkylen)-OH, -O(C₁₋₅-Alkylen)-O(C₁₋₅-alkyl), -SH, -S(C₁₋₅-Alkyl), -NH₂, -NH(C₁₋₅-Alkyl), -N(C₁₋₅-Alkyl)(C₁₋₅-alkyl), Halogen, C₁₋₅-Halogenalkyl, -O-(C₁₋₅-Halogenalkyl), -CF₃, -CN, -NO₂, -CHO, -CO-(C₁₋₅-Alkyl), -COOH, -CO-O-(C₁₋₅-Alkyl), -O-CO-(C₁₋₅-Alkyl), -CO-NH₂, -CO-NH(C₁₋₅-Alkyl), -CO-N(C₁₋₅-Alkyl)(C₁₋₅-alkyl), -NH-CO-(C₁₋₅-Alkyl), -N(C₁₋₅-Alkyl)-CO-(C₁₋₅-alkyl), -NH-CO-O-(C₁₋₅-Alkyl), -N(C₁₋₅-Alkyl)-CO-O-(C₁₋₅-alkyl), -O-CO-NH-(C₁₋₅-Alkyl), -O-CO-N(C₁₋₅-Alkyl)-(C₁₋₅-alkyl), -SO₂-NH₂, -SO₂-NH(C₁₋₅-Alkyl), -SO₂-N(C₁₋₅-Alkyl)(C₁₋₅-alkyl), -NH-SO₂-(C₁₋₅-Alkyl), -N(C₁₋₅-Alkyl)-SO₂-(C₁₋₅-alkyl), -SO-(C₁₋₅-Alkyl), -SO₂-(C₁₋₅-Alkyl), Cycloalkyl und Heterocycloalkyl;
R³ ausgewählt ist aus Wasserstoff, C₁₋₅-Alkyl, C₂₋₅-Alkenyl, C₂₋₅-Alkinyl, -(C₁₋₅-Alkylen)-OH, -(C₁₋₅-Alkylen)-O(C₁₋₅-alkyl), -(C₁₋₅-Alkylen)-O(C₁₋₅-alkylen)-OH, -(C₁₋₅-Alkylen)-O(C₁₋₅-alkylen)-O(C₁₋₅-alkyl), -(C₁₋₅-Alkylen)-SH, -(C₁₋₅-alkylen)-S(C₁₋₅-alkyl), -(C₁₋₅-Alkylen)-NH₂, -(C₁₋₅-Alkylen)-NH(C₁₋₅-alkyl), -(C₁₋₅-Alkylen)-N(C₁₋₅-alkyl)(C₁₋₅-alkyl), -(C₁₋₅-Alkylen)-halogen, C₁₋₅-Halogenalkyl, -(C₁₋₅-Alkylen)-O-(C₁₋₅-halogenalkyl), -(C₁₋₅-Alkylen)-CF₃, -(C₁₋₅-Alkylen)-CN, -(C₁₋₅-Alkylen)-NO₂, -(C₁₋₅-Alkylen)-Si(C₁₋₅-alkyl)₃, -(C₁₋₅-Alkylen)-O(C₁₋₅-alkylen)-Si(C₁₋₅-alkyl)₃, -(C₁₋₅-Alkylen)-CHO, -(C₁₋₅-alkylen)-CO-(C₁₋₅-alkyl), -(C₁₋₅-Alkylen)-COOH, -(C₁₋₅-Alkylen)-CO-O-(C₁₋₅-alkyl), -(C₁₋₅-Alkylen)-O-CO-(C₁₋₅-alkyl), -(C₁₋₅-Alkylen)-CO-NH₂, -(C₁₋₅-Alkylen)-CO-NH(C₁₋₅-alkyl), -(C₁₋₅-Alkylen)-CO-N(C₁₋₅-alkyl)(C₁₋₅-alkyl), -(C₁₋₅-Alkylen)-NH-CO-(C₁₋₅-alkyl), -(C₁₋₅-Alkylen)-N(C₁₋₅-alkyl)-CO-(C₁₋₅-alkyl), -(C₁₋₅-Alkylen)-NH-CO-O-(C₁₋₅-alkyl), -(C₁₋₅-Alkylen)-N(C₁₋₅-alkyl)-CO-O-(C₁₋₅-alkyl), -(C₁₋₅-Alkylen)-O-CO-NH-(C₁₋₅-alkyl), -(C₁₋₅-Alkylen)-O-CO-N(C₁₋₅-alkyl)-(C₁₋₅-alkyl), -(C₁₋₅-Alkylen)-SO₂-NH₂, -(C₁₋₅-Alkylen)-SO₂-NH(C₁₋₅-alkyl), -(C₁₋₅-Alkylen)-SO₂-N(C₁₋₅-alkyl)(C₁₋₅-alkyl), -(C₁₋₅-Alkylen)-NH-SO₂-(C₁₋₅-alkyl), -(C₁₋₅-Alkylen)-N(C₁₋₅-alkyl)-SO₂-(C₁₋₅-alkyl), -(C₁₋₅-Alkylen)-SO-(C₁₋₅-alkyl), -(C₁₋₅-Alkylen)-SO₂-(C₁₋₅-alkyl), -(C₀₋₅-Alkylen)-carbocyclyl und -(C₀₋₅-Alkylen)-heterocyclyl, wobei der Carbocyclylteil des -(C₀₋₅-Alkylen)-carbocyclyls und der Heterocyclylteil des -(C₀₋₅-Alkylen)-heterocyclyls jeweils gegebenenfalls mit einer oder mehreren Gruppen substituiert sind, die unabhängig ausgewählt sind aus C₁₋₅-Alkyl, C₂₋₅-Alkenyl, C₂₋₅-Alkinyl, -OH, -O(C₁₋₅-Alkyl), -O(C₁₋₅-Alkylen)-OH, -O(C₁₋₅-Alkylen)-O(C₁₋₅-alkyl), -SH, -S(C₁₋₅-Alkyl), -NH₂, -NH(C₁₋₅-Alkyl), -N(C₁₋₅-Alkyl)(C₁₋₅-alkyl), Halogen, C₁₋₅-Halogenalkyl, -O-(C₁₋₅-Halogenalkyl), -CF₃, -CN, -NO₂, -CHO, -CO-(C₁₋₅-Alkyl), -COOH, -CO-O-(C₁₋₅-Alkyl), -O-CO-(C₁₋₅-Alkyl), -CO-NH₂, -CO-NH(C₁₋₅-Alkyl), -CO-N(C₁₋₅-Alkyl)(C₁₋₅-alkyl), -NH-CO-(C₁₋₅-Alkyl), -N(C₁₋₅-Alkyl)-CO-(C₁₋₅-alkyl), -NH-CO-O-(C₁₋₅-Alkyl), -N(C₁₋₅-Alkyl)-CO-O-(C₁₋₅-alkyl), -O-CO-NH-(C₁₋₅-Alkyl), -O-CO-N(C₁₋₅-Alkyl)-(C₁₋₅-alkyl), -SO₂-NH₂, -SO₂-NH(C₁₋₅-Alkyl), -SO₂-N(C₁₋₅-Alkyl)(C₁₋₅-alkyl), -NH-SO₂-(C₁₋₅-Alkyl), -N(C₁₋₅-Alkyl)-SO₂-(C₁₋₅-alkyl), -SO-(C₁₋₅-Alkyl), -SO₂-(C₁₋₅-Alkyl), Cycloalkyl und Heterocycloalkyl;
der Ring A ein bicyclisches Heteroaryl ist, wobei das bicyclische Heteroaryl gegebenenfalls mit einer oder mehreren Gruppen R^{A} substituiert ist; und
R^{A} jeweils unabhängig ausgewählt ist aus C₁₋₅-Alkyl, C₂₋₅-Alkenyl, C₂₋₅-Alkinyl, -(C₀₋₃-Alkylen)-OH, -(C₀₋₃-Alkylen)-O(C₁₋₅-alkyl), -(C₀₋₃-Alkylen)-O(C₁₋₅-alkylen)-OH, -(C₀₋₃-Alkylen)-O(C₁₋₅-alkylen)-O(C₁₋₅-alkyl), -(C₀₋₃-Alkylen)-SH, -(C₀₋₃-Alkylen)-S(C₁₋₅-alkyl), -(C₀₋₃-Alkylen)-NH₂, -(C₀₋₃-Alkylen)-NH(C₁₋₅-alkyl), -(C₀₋₃-Alkylen)-N(C₁₋₅-alkyl)(C₁₋₅-alkyl), -(C₀₋₃-Alkylen)-halogen, -(C₀₋₃-Alkylen)-(C₁₋₅-halogenalkyl), -(C₀₋₃-Alkylen)-O-(C₁₋₅-halogenalkyl), -(C₀₋₃-Alkylen)-CF₃, -(C₀₋₃-Alkylen)-CN, -(C₀₋₃-Alkylen)-NO₂, -(C₀₋₃-Alkylen)-CHO, -(C₀₋₃-Alkylen)-CO-(C₁₋₅-alkyl), -(C₀₋₃-Alkylen)-COOH, -(C₀₋₃-Alkylen)-CO-O-(C₁₋₅-alkyl), -(C₀₋₃-Alkylen)-O-CO-(C₁₋₅-alkyl), -(C₀₋₃-Alkylen)-CO-NH₂, -(C₀₋₃-Alkylen)-CO-NH(C₁₋₅-alkyl), -(C₀₋₃-Alkylen)-CO-N(C₁₋₅-alkyl)(C₁₋₅-alkyl), -(C₀₋₃-Alkylen)-NH-CO-(C₁₋₅-alkyl), -(C₀₋₃-Alkylen)-N(C₁₋₅-alkyl)-CO-(C₁₋₅-alkyl), -(C₀₋₃-Alkylen)-NH-CO-O-(C₁₋₅-alkyl), -(C₀₋₃-Alkylen)-N(C₁₋₅-alkyl)-CO-O-(C₁₋₅-alkyl), -(C₀₋₃-Alkylen)-O-CO-NH-(C₁₋₅-alkyl), -(C₀₋₃-Alkylen)-O-CO-N(C₁₋₅-alkyl)-(C₁₋₅-alkyl), -(C₀₋₃-Alkylen)-SO₂-NH₂, -(C₀₋₃-Alkylen)-SO₂-NH(C₁₋₅-alkyl), -(C₀₋₃-Alkylen)-SO₂-N(C₁₋₅-alkyl)(C₁₋₅-alkyl), -(C₀₋₃-Alkylen)-NH-SO₂-(C₁₋₅-alkyl), -(C₀₋₃-Alkylen)-N(C₁₋₅-alkyl)-SO₂-(C₁₋₅-alkyl), -(C₀₋₃-Alkylen)-SO-(C₁₋₅-alkyl), -(C₀₋₃-Alkylen)-SO₂-(C₁₋₅-alkyl), -(C₀₋₃-Alkylen)-carbocyclyl und -(C₀₋₃-Alkylen)-heterocyclyl, wobei der Carbocyclylteil des -(C₀₋₃-Alkylen)-carbocyclyls und der Heterocyclylteil des -(C₀₋₃-Alkylen)-heterocyclyls jeweils gegebenenfalls mit einer oder mehreren Gruppen substituiert sind, die unabhängig ausgewählt sind aus C₁₋₅-Alkyl, C₂₋₅-Alkenyl, C₂₋₅-Alkinyl, -OH, -O(C₁₋₅-Alkyl), -O(C₁₋₅-Alkylen)-OH, -O(C₁₋₅-Alkylen)-O(C₁₋₅-alkyl), -SH, -S(C₁₋₅-Alkyl), -NH₂, -NH(C₁₋₅-Alkyl), -N(C₁₋₅-Alkyl)(C₁₋₅-alkyl), Halogen, C₁₋₅-Halogenalkyl, -O-(C₁₋₅-Halogenalkyl), -CF₃, -CN, -NO₂, -CHO, -CO-(C₁₋₅-Alkyl), -COOH, -CO-O-(C₁₋₅-Alkyl), -O-CO-(C₁₋₅-Alkyl), -CO-NH₂, -CO-NH(C₁₋₅-Alkyl), -CO-N(C₁₋₅-Alkyl)(C₁₋₅-alkyl), -NH-CO-(C₁₋₅-Alkyl), -N(C₁₋₅-Alkyl)-CO-(C₁₋₅-alkyl), -NH-CO-O-(C₁₋₅-Alkyl), -N(C₁₋₅-Alkyl)-CO-O-(C₁₋₅-alkyl), -O-CO-NH-(C₁₋₅-Alkyl), -O-CO-N(C₁₋₅-Alkyl)-(C₁₋₅-alkyl), -SO₂-NH₂, -SO₂-NH(C₁₋₅-Alkyl), -SO₂-N(C₁₋₅-Alkyl)(C₁₋₅-alkyl), -NH-SO₂-(C₁₋₅-Alkyl), -N(C₁₋₅-Alkyl)-SO₂-(C₁₋₅-alkyl), -SO-(C₁₋₅-Alkyl), -SO₂-(C₁₋₅-Alkyl), Cycloalkyl und Heterocycloalkyl;
oder ein pharmazeutisch unbedenkliches Salz oder Solvat davon.

2. Verbindung des Anspruchs 1, wobei R^{1A} und R^{1B} miteinander verknüpft sind, so dass sie, zusammen mit dem Stickstoffatom, an das sie gebunden sind, ein Heterocycloalkyl bilden, das gegebenenfalls mit einer oder mehreren Gruppen R¹¹ substituiert ist.

3. Verbindung des Anspruchs 1 oder 2, wobei R^{1A} und R^{1B} miteinander verknüpft sind, so dass sie, zusammen mit dem Stickstoffatom, an das sie gebunden sind, eine Gruppe bilden, die ausgewählt ist aus 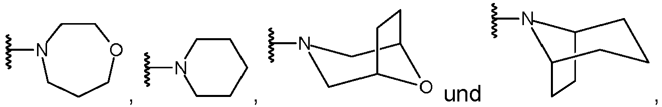 wobei jede einzelne der vorstehend dargestellten Gruppen gegebenenfalls mit einem oder mehreren R¹¹ substituiert ist.

4. Verbindung irgendeines der Ansprüche 1 bis 3, wobei R² und R⁴ jeweils unabhängig ausgewählt sind aus Wasserstoff, C₁₋₅-Alkyl, -OH, -O(C₁₋₅-Alkyl), -O(C₁₋₅-Alkylen)-OH, -O(C₁₋₅-Alkylen)-O(C₁₋₅-alkyl), -SH, -S(C₁₋₅-Alkyl), -NH₂, -NH(C₁₋₅-Alkyl), -N(C₁₋₅-Alkyl)(C₁₋₅-alkyl), Halogen, C₁₋₅-Halogenalkyl, -CF₃ und -CN.

5. Verbindung irgendeines der Ansprüche 1 bis 4, wobei R³ ausgewählt ist aus C₁₋₅-Halogenalkyl, -(C₁₋₅-Alkylen)-CN, -(C₁₋₅-Alkylen)-SO-(C₁₋₅-alkyl) und -(C₁₋₅-Alkylen)-SO₂-(C₁₋₅-alkyl).

6. Verbindung irgendeines der Ansprüche 1 bis 5, wobei R³ -CH₂-CF₃ oder -CH₂-SO₂-CH₃ ist.

7. Verbindung irgendeines der Ansprüche 1 bis 6, wobei der Ring A ein kondensiertes bicyclisches Heteroaryl ist, wobei einer der beiden kondensierten Ringe des kondensierten bicyclischen Heteroaryls 5 Ringatome aufweist und der andere kondensierte Ring 6 Ringatome aufweist und wobei ferner das bicyclische Heteroaryl gegebenenfalls mit einer oder mehreren Gruppen R^{A} substituiert ist.

8. Verbindung irgendeines der Ansprüche 1 bis 7, wobei der Ring A ein bicyclisches Heteroaryl mit der folgenden Formel (II) ist: 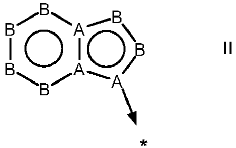 wobei jeder der beiden kondensierten Ringe des bicyclischen Heteroaryls aromatisch ist, wobei das Ringatom A des bicyclischen Heteroaryls jeweils unabhängig ein Kohlenstoff- oder Stickstoffatom ist, wobei das Ringatom B jeweils unabhängig ein Kohlenstoff-, Stickstoff-, Sauerstoff- oder Schwefelatom ist, wobei mindestens eines der Ringatome A und/oder B von Kohlenstoff verschieden ist und wobei das bicyclische Heteroaryl gegebenenfalls mit einer oder mehreren Gruppen R^{A} substituiert ist.

9. Verbindung irgendeines der Ansprüche 1 bis 8, wobei der Ring A aus irgendeiner der folgenden Gruppen ausgewählt ist: 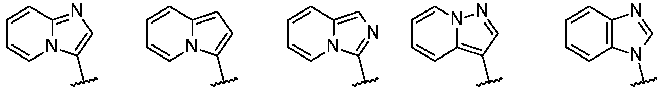 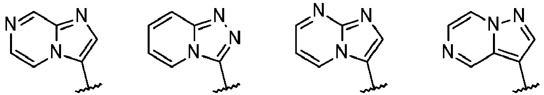 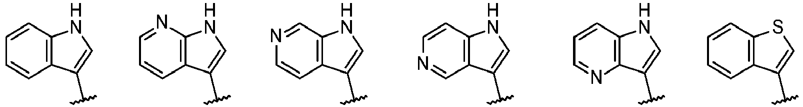 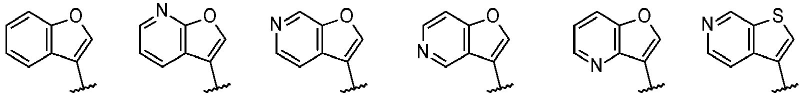
wobei jede der vorstehend dargestellten Gruppen gegebenenfalls mit einer oder mehreren Gruppen R^{A} substituiert ist;
und wobei der Ring A vorzugsweise aus irgendeiner der folgenden Gruppen ausgewählt ist: 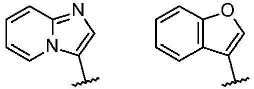 wobei jede der vorstehend dargestellten Gruppen gegebenenfalls mit einer oder mehreren Gruppen R^{A} substituiert ist.

10. Verbindung irgendeines der Ansprüche 1 bis 9, wobei R^{A} jeweils unabhängig ausgewählt ist aus C₁₋₅-Alkyl, -OH, -O(C₁₋₅-Alkyl), -O(C₁₋₅-Alkylen)-OH, -O(C₁₋₅-Alkylen)-O(C₁₋₅-alkyl), -SH, -S(C₁₋₅-Alkyl), -NH₂, -NH(C₁₋₅-Alkyl), -N(C₁₋₅-Alkyl)(C₁₋₅-alkyl), Halogen, C₁₋₅-Halogenalkyl, -CF₃ und -CN, wobei vorzugsweise R^{A} jeweils unabhängig Halogen ist; und wobei stärker bevorzugt R^{A} jeweils -F ist.

11. Verbindung des Anspruchs 1, wobei die Verbindung ausgewählt ist aus:
| | |
|---|---|
| 1 | [3-(1-Methyl-1H-indol-3-yl)-1-(2,2,2-trifluor-ethyl)-1H-pyrazolo[4,3-c]pyridin-6-yl]-1,4-oxazepan-4-yl-methanon |
| 2 | Azepan-1-yl-[3-(6-fluor-imidazo[1,2-a]pyridin-3-yl)-1-(2-methansulfonyl-ethyl)-1H-pyrazolo[4,3-c]pyridin-6-yl]-methanon |
| 3 | Azepan-1-yl-[1-(2,2-difluor-ethyl)-3-(6-fluor-imidazo[1,2-a]pyridin-3-yl)-1H-pyrazolo[4,3-c]pyridin-6-yl]-methanon |
| 4 | Azepan-1-yl-[1-(2-fluor-ethyl)-3-(6-fluor-imidazo[1,2-a]pyridin-3-yl)-1H-pyrazolo[4,3-c]pyridin-6-yl]-methanon |
| 5 | Azepan-1-yl-[3-(6-fluor-imidazo[1,2-a]pyridin-3-yl)-1-propyl-1H-pyrazolo[4,3-c]pyridin-6-yl]-methanon |
| 6 | Azepan-1-yl-[3-(6-fluor-imidazo[1,2-a]pyridin-3-yl)-1-methoxymethyl-1H-pyrazolo[4,3-c]pyridin-6-yl]-methanon |
| 7 | [6-(Azepan-1-carbonyl)-3-(6-fluor-imidazo[1,2-a]pyridin-3-yl)-pyrazolo[4,3-c]pyridin-1-yl]-acetonitril |
| 8 | Azepan-1-yl-[3-(6,8-difluor-imidazo[1,2-a]pyridin-3-yl)-1-(2-methoxy-ethyl)-1H-pyrazolo[4,3-c]pyridin-6-yl]-methanon |
| 9 | [1-Cyclopropylmethyl-3-(6,8-difluor-imidazo[1,2-a]pyridin-3-yl)-1H-pyrazolo[4,3-c]pyridin-6-yl]-1,4-oxazepan-4-yl-methanon |
| 10 | [3-(6,8-Difluor-imidazo[1,2-a]pyridin-3-yl)-1-methansulfonyl-1H-pyrazolo[4,3-c]pyridin-6-yl]-1,4-oxazepan-4-yl-methanon |
| 11 | 3-[3-(6,8-Difluor-imidazo[1,2-a]pyridin-3-yl)-6-(1,4-oxazepan-4-carbonyl)-pyrazolo[4,3-c]pyridin-1-ylmethyl]-azetidin-1-carbonsäure-tert-butylester |
| 12 | [3-(6,8-Difluor-imidazo[1,2-a]pyridin-3-yl)-1-hydroxymethyl-1H-pyrazolo[4,3-c]pyridin-6-yl]-1,4-oxazepan-4-yl-methanon |
| 13 | [3-Benzofuran-3-yl-6-(1,4-oxazepan-4-carbonyl)-pyrazolo[4,3-c]pyridin-1-yl]-acetonitril |
| 14 | (3-Aza-bicyclo[3.2.1]oct-3-yl)-[3-(6,8-difluor-imidazo[1,2-a]pyridin-3-yl)-1-methylsulfanylmethyl-1H-pyrazolo[4,3-c]pyridin-6-yl]-methanon |
| 15 | [3-(6,8-Difluor-imidazo[1,2-a]pyridin-3-yl)-1-methansulfonylmethyl-1H-pyrazolo[4,3-c]pyridin-6-yl]-1,4-oxazepan-4-yl-methanon |
| 16 | (3-Benzofuran-3-yl-1-methansulfonylmethyl-1H-pyrazolo[4,3-c]pyridin-6-yl)-1,4-oxazepan-4-yl-methanon |
| 17 | (1-Methansulfonylmethyl-3-pyrazolo[1,5-a]pyridin-3-yl-1H-pyrazolo[4,3-c]pyridin-6-yl)-1,4-oxazepan-4-yl-methanon |
| 18 | (3-Aza-bicyclo[3.2.1]oct-3-yl)-(1-methansulfonylmethyl-3-pyrazolo[1,5-a]pyridin-3-yl-1H-pyrazolo[4,3-c]pyridin-6-yl)-methanon |
| 19 | (3-Aza-bicyclo[3.2.1]oct-3-yl)-(3-benzofuran-3-yl-1-methansulfonylmethyl-1H-pyrazolo[4,3-c]pyridin-6-yl)-methanon |
| 20 | Azepan-1-yl-[3-(6-fluor-imidazo[1,2-a]pyridin-3-yl)-1-methansulfonylmethyl-1H-pyrazolo[4,3-c]pyridin-6-yl]-methanon |
| 21 | Azepan-1-yl-[3-(6-fluor-imidazo[1,2-a]pyridin-3-yl)-1-methansulfinylmethyl-1H-pyrazolo[4,3-c]pyridin-6-yl]-methanon |
| 22 | [3-(6,8-Difluor-imidazo[1,2-a]pyridin-3-yl)-1-ethansulfonylmethyl-1H-pyrazolo[4,3-c]pyridin-6-yl]-1,4-oxazepan-4-yl-methanon |
| 23 | (3-Aza-bicyclo[3.2.1]oct-3-yl)-[3-(6,8-difluor-imidazo[1,2-a]pyridin-3-yl)-1-methansulfonylmethyl-1H-pyrazolo[4,3-c]pyridin-6-yl]-methanon |
| 24 | [3-(6,8-Difluor-imidazo[1,2-a]pyridin-3-yl)-1-methansulfonylmethyl-1H-pyrazolo[4,3-c]pyridin-6-yl]-(8-endo-hydroxy-8-exo-methyl-3-aza-bicyclo[3.2.1]oct-3-yl)-methanon |
| 25 | [3-(6,8-Difluor-imidazo[1,2-a]pyridin-3-yl)-1-methansulfonylmethyl-1H-pyrazolo[4,3-c]pyridin-6-yl]-(3-endo-hydroxy-8-azabicyclo[3.2.1]oct-8-yl)-methanon |
| 26 | [3-(6,8-Difluor-imidazo[1,2-a]pyridin-3-yl)-1-methansulfonylmethyl-1H-pyrazolo[4,3-c]pyridin-6-yl]-(2-oxa-5-aza-bicyclo[2.2.1]hept-5-yl)-methanon |
| 27 | [3-(6,8-Difluor-imidazo[1,2-a]pyridin-3-yl)-1-methansulfonylmethyl-1H-pyrazolo[4,3-c]pyridin-6-yl]-(8-oxa-3-aza-bicyclo[3.2.1]oct-3-yl)-methanon |
| 28 | (3-Benzofuran-3-yl-1-methansulfonylmethyl-1H-pyrazolo[4,3-c]pyridin-6-yl)-(8-endo-hydroxy-8-exo-methyl-d3-3-azabicyclo[3.2.1]oct-3-yl)-methanon |
| 29 | (3-Benzofuran-3-yl-1-methansulfonylmethyl-1H-pyrazolo[4,3-c]pyridin-6-yl)-(6,6-difluor-1,4-oxazepan-4-yl)-methanon |
| 30 | (3-Benzofuran-3-yl-1-methansulfonylmethyl-1H-pyrazolo[4,3-c]pyridin-6-yl)-(8-endo-hydroxy-3-aza-bicyclo[3.2.1]oct-3-yl)-methanon |
| 31 | (3-Benzofuran-3-yl-1-methansulfonylmethyl-1H-pyrazolo[4,3-c]pyridin-6-yl)-(5-endo-hydroxy-5-exo-methyl-2-azabicyclo[2.2.1]hept-2-yl)-methanon |
| 32 | (3-Benzofuran-3-yl-1-methansulfonylmethyl-1H-pyrazolo[4,3-c]pyridin-6-yl)-(4-hydroxy-4-methyl-piperidin-1-yl)-methanon |
| 33 | (3-Benzofuran-3-yl-1-methansulfonylmethyl-1H-pyrazolo[4,3-c]pyridin-6-yl)-(4,4-difluor-piperidin-1-yl)-methanon |
| 34 | (3-Benzofuran-3-yl-1-methansulfonylmethyl-1H-pyrazolo[4,3-c]pyridin-6-yl)-(2-methoxymethyl-pyrrolidin-1-yl)-methanon |
| 35 | (3-Benzofuran-3-yl-1-methansulfonylmethyl-1H-pyrazolo[4,3-c]pyridin-6-yl)-(4-hydroxy-2,2 dimethyl-piperidin-1-yl)-methanon |
| 36 | (3-Benzofuran-3-yl-1-methansulfonylmethyl-1H-pyrazolo[4,3-c]pyridin-6-yl)-(4-hydroxy-piperidin-1-yl)-methanon |
| 37 | [3-(Benzofuran-3-yl)-1-(methylsulfonylmethyl)pyrazolo[4,3-c]pyridin-6-yl]-(3-endo-hydroxy-8-azabicyclo[3.2.1]octan-8-yl)methanon |
| 38 | (3-Benzofuran-3-yl-1-methansulfonylmethyl-1H-pyrazolo[4,3-c]pyridin-6-yl)-(3-hydroxy-piperidin-1-yl)-methanon |
| 39 | (3-Benzofuran-3-yl-1-methansulfonylmethyl-1H-pyrazolo[4,3-c]pyridin-6-yl)-(3,3-difluor-piperidin-1-yl)-methanon |
| 40 | (3-Benzofuran-3-yl-1-methansulfonylmethyl-1H-pyrazolo[4,3-c]pyridin-6-yl)-(4-hydroxyazepan-1-yl)-methanon |
| 41 | Azepan-1-yl-[3-(6-fluor-imidazo[1,2-a]pyridin-3-yl)-1-methyl-1H-pyrazolo[4,3-c]pyridin-6-yl]-methanon |
| 42 | 3-[6-(Azepan-1-carbonyl)-1-methyl-1H-pyrazolo[4,3-c]pyridin-3-yl]-imidazo[1,2-a]pyridin-6-carbonitril |
| 43 | 3-[6-(Azepan-1-carbonyl)-1-propyl-1H-pyrazolo[4,3-c]pyridin-3-yl]-imidazo[1,2-a]pyridin-6-carbonitril |
| 44 | Azepan-1-yl-[3-(6-fluor-imidazo[1,2-a]pyridin-3-yl)-1-(2,2,2-trifluorethyl)-1H-pyrazolo[4,3-c]pyridin-6-yl]-methanon |
| 45 | 3-[6-(Azepan-1-carbonyl)-1-(2,2,2-trifluor-ethyl)-1H-pyrazolo[4,3-c]pyridin-3-yl]-imidazo[1,2-a]pyridin-6-carbonitril |
| 46 | 3-[6-(Azepan-1-carbonyl)-1-(2,2,2-trifluor-ethyl)-1H-pyrazolo[4,3-c]-pyridin-3-yl]-imidazo[1,2-a]pyridin-6-carbonsäure-amid |
| 47 | 3-[6-(Azepan-1-carbonyl)-1-(2,2,2-trifluor-ethyl)-1H-pyrazolo[4,3-c]pyridin-3-yl]-8-methyl-imidazo[1,2-a]pyridin-6-carbonitril |
| 48 | Azepan-1-yl-[3-(6-chlor-imidazo[1,2-a]pyridin-3-yl)-1-(2,2,2-trifluor-ethyl)-1H-pyrazolo[4,3-c]pyridin-6-yl]-methanon |
| 49 | 3-[6-(Azepan-1-carbonyl)-1-(2,2,2-trifluor-ethyl)-1H-pyrazolo[4,3-c]pyridin-3-yl]-imidazo[1,2-a]pyridin-6-carbonsäure-methylamid |
| 50 | Azepan-1-yl-[3-(6-fluor-imidazo[1,2-a]pyrimidin-3-yl)-1-(2,2,2-trifluor-ethyl)-1H-pyrazolo[4,3-c]pyridin-6-yl]-methanon |
| 51 | Azepan-1-yl-[3-(6-methoxymethyl-imidazo[1,2-a]pyridin-3-yl)-1-(2,2,2-trifluor-ethyl)-1H-pyrazolo[4,3-c]pyridin-6-yl]-methanon |
| 52 | Azepan-1-yl-[3-indolizin-3-yl-1-(2,2,2-trifluor-ethyl)-1H-pyrazolo[4,3-c]pyridin-6-yl]-methanon |
| 53 | 3-[6-(Azepan-1-carbonyl)-1-(2,2,2-trifluor-ethyl)-1H-pyrazolo[4,3-c]pyridin-3-yl]-imidazo[1,2-a]pyridin-6-carbonsäure-dimethylamid |
| 54 | Azepan-1-yl-[3-imidazo[1,5-a]pyridin-3-yl-1-(2,2,2-trifluor-ethyl)-1H-pyrazolo[4,3-c]pyridin-6-yl]-methanon |
| 55 | Azepan-1-yl-[3-imidazo[1,2-a]pyridin-3-yl-1-(2,2,2-trifluor-ethyl)-1H-pyrazolo[4,3-c]pyridin-6-yl]-methanon |
| 56 | Azepan-1-yl-[3-imidazo[1,2-a]pyrazin-3-yl-1-(2,2,2-trifluor-ethyl)-1H-pyrazolo[4,3-c]pyridin-6-yl]-methanon |
| 57 | 3-[6-(Azepan-1-carbonyl)-1-(2,2,2-trifluor-ethyl)-1H-pyrazolo[4,3-c]pyridin-3-yl]-7-methyl-imidazo[1,2-a]pyridin-6-carbonsäure-amid |
| 58 | Azepan-1-yl-[1-(2,2,2-trifluor-ethyl)-3-(6-trifluormethyl-imidazo[1,2-a]pyridin-3-yl)-1H-pyrazolo[4,3-c]pyridin-6-yl]-methanon |
| 59 | Azepan-1-yl-[3-(6-methoxy-imidazo[1,2-a]pyridin-3-yl)-1-(2,2,2-trifluor-ethyl)-1H-pyrazolo[4,3-c]pyridin-6-yl]-methanon |
| 60 | Azepan-1-yl-[3-(6,8-difluor-imidazo[1,2-a]pyridin-3-yl)-1-(2,2,2-trifluor-ethyl)-1H-pyrazolo[4,3-c]pyridin-6-yl]-methanon |
| 61 | Azepan-1-yl-[3-(6-methyl-imidazo[1,2-a]pyridin-3-yl)-1-(2,2,2-trifluor-ethyl)-1H-pyrazolo[4,3-c]pyridin-6-yl]-methanon |
| 62 | Azepan-1-yl-[3-(8-fluor-imidazo[1,2-a]pyridin-3-yl)-1-(2,2,2-trifluorethyl)-1H-pyrazolo[4,3-c]pyridin-6-yl]-methanon |
| 63 | 3-[6-(Azepan-1-carbonyl)-1-(2,2,2-trifluor-ethyl)-1H-pyrazolo[4,3-c]pyridin-3-yl]-7-hydroxy-imidazo[1,2-a]pyridin-6-carbonitril |
| 64 | Azepan-1-yl-[3-[6-(1-hydroxy-ethyl)-imidazo[1,2-a]pyridin-3-yl]-1-(2,2,2-trifluor-ethyl)-1H-pyrazolo[4,3-c]pyridin-6-yl]-methanon |
| 65 | Azepan-1-yl-[3-(6-cyclopropyl-imidazo[1,2-a]pyridin-3-yl)-1-(2,2,2-trifluor-ethyl)-1H-pyrazolo[4,3-c]pyridin-6-yl]-methanon |
| 66 | Azepan-1-yl-[1-(2,2,2-trifluor-ethyl)-3-(6-trifluormethoxy-imidazo[1,2-a]pyridin-3-yl)-1H-pyrazolo[4,3-c]pyridin-6-yl]-methanon |
| 67 | Azepan-1-yl-[3-(7-fluor-imidazo[1,2-a]pyridin-3-yl)-1-(2,2,2-trifluorethyl)-1H-pyrazolo[4,3-c]pyridin-6-yl]-methanon |
| 68 | Azepan-1-yl-[3-(6-hydroxy-imidazo[1,2-a]pyridin-3-yl)-1-(2,2,2-trifluor-ethyl)-1H-pyrazolo[4,3-c]pyridin-6-yl]-methanon |
| 69 | Azepan-1-yl-[3-(6-difluormethyl-imidazo[1,2-a]pyridin-3-yl)-1-(2,2,2-trifluor-ethyl)-1H-pyrazolo[4,3-c]pyridin-6-yl]-methanon |
| 70 | Azepan-1-yl-[3-[6-(2,2,2-trifluor-ethoxy)-imidazo[1,2-a]pyridin-3-yl]-1-(2,2,2-trifluor-ethyl)-1H-pyrazolo[4,3-c]pyridin-6-yl]-methanon |
| 71 | Azepan-1-yl-[3-[1,2,4]triazolo[4,3-a]pyridin-3-yl-1-(2,2,2-trifluorethyl)-1H-pyrazolo[4,3-c]pyridin-6-yl]-methanon |
| 72 | Azepan-1-yl-[3-[6-(1-hydroxy-1-methyl-ethyl)-imidazo[1,2-a]pyridin-3-yl]-1-(2,2,2-trifluor-ethyl)-1H-pyrazolo[4,3-c]pyridin-6-yl]-methanon |
| 73 | Azepan-1-yl-[3-(7-chlor-6-fluor-imidazo[1,2-a]pyridin-3-yl)-1-(2,2,2-trifluor-ethyl)-1H-pyrazolo[4,3-c]pyridin-6-yl]-methanon |
| 74 | Azepan-1-yl-[3-(6-chlor-7-fluor-imidazo[1,2-a]pyridin-3-yl)-1-(2,2,2-trifluor-ethyl)-1H-pyrazolo[4,3-c]pyridin-6-yl]-methanon |
| 75 | Azepan-1-yl-[3-(6-chlor-7-hydroxy-imidazo[1,2-a]pyridin-3-yl)-1-(2,2,2-trifluor-ethyl)-1H-pyrazolo[4,3-c]pyridin-6-yl]-methanon |
| 76 | Azepan-1-yl-[3-(6,8-difluor-imidazo[1,2-a]pyridin-3-yl)-1-(2-trimethylsilanyl-ethoxymethyl)-1H-pyrazolo[4,3-c]pyridin-6-yl]-methanon |
| 77 | Azepan-1-yl-[3-(6-fluor-imidazo[1,2-a]pyridin-3-yl)-1-(2-trimethylsilanyl-ethoxymethyl)-1H-pyrazolo[4,3-c]pyridin-6-yl]-methanon |
| 78 | Azepan-1-yl-[3-(6,8-difluor-imidazo[1,2-a]pyridin-3-yl)-1-(4-methoxy-benzyl)-1H-pyrazolo[4,3-c]pyridin-6-yl]-methanon |
| 79 | Azepan-1-yl-[3-(6,8-difluor-imidazo[1,2-a]pyridin-3-yl)-1-(tetrahydro-pyran-2-yl)-1H-pyrazolo[4,3-c]pyridin-6-yl]-methanon |
| 80 | [3-(6-Fluor-imidazo[1,2-a]pyridin-3-yl)-1-(2,2,2-trifluor-ethyl)-1H-pyrazolo[4,3-c]pyridin-6-yl]-1,4-oxazepan-4-yl-methanon |
| 81 | 3-[6-(1,4-Oxazepan-4-carbonyl)-1-(2,2,2-trifluor-ethyl)-1H-pyrazolo[4,3-c]pyridin-3-yl]-imidazo[1,2-a]pyridin-6-carbonitril |
| 82 | [3-(6-Fluor-imidazo[1,2-a]pyridin-3-yl)-1-(2,2,2-trifluor-ethyl)-1H-pyrazolo[4,3-c]pyridin-6-yl]-(8-oxa-3-aza-bicyclo[3.2.1]oct-3-yl)-methanon |
| 83 | [3-(6,8-Difluor-imidazo[1,2-a]pyridin-3-yl)-1-(2,2,2-trifluor-ethyl)-1H-pyrazolo[4,3c]pyridin-6-yl]-(8-oxa-3-aza-bicyclo[3.2.1]oct-3-yl)-methanon |
| 84 | 3-[6-(8-Oxa-3-aza-bicyclo[3.2.1]octan-3-carbonyl)-1-(2,2,2-trifluorethyl)-1H-pyrazolo[4,3-c]pyridin-3-yl]-imidazo[1,2-a]pyridin-6-carbonitril |
| 85 | [3-(8-Fluorimidazo[1 ,2-a]pyridin-3-yl)-1-(2,2,2-trifluorethyl)pyrazolo[4,3-c]pyridin-6-yl]-(3-endo-hydroxy-8-azabicyclo[3.2.1]octan-8-yl) methanon |
| 86 | [3-(6-Fluorimidazo[1 ,2-a]pyridin-3-yl)-1-(2,2,2-trifluorethyl)pyrazolo[4,3-c]pyridin-6-yl]-(3-endo-hydroxy-8-azabicyclo[3.2.1]octan-8-yl) methanon |
| 87 | [3-(Imidazo[1,2-a]pyridin-3-yl)-1-(2,2,2-trifluorethyl)pyrazolo[4,3-c]pyridin-6-yl]-(3-endo-hydroxy-8-azabicyclo[3.2.1]octan-8-yl)methanon |
| 88 | (3-endo-Hydroxy-8-azabicyclo[3.2.1]octan-8-yl)-[3-(6-hydroxyimidazo[1,2-a]pyridin-3-yl)-1-(2,2,2-trifluorethyl)pyrazolo[4,3-c]pyridin-6-yl]methanon |
| 89 | Azepan-1-yl-[3-pyrazolo[1,5-a]pyridin-3-yl-1-(2,2,2-trifluor-ethyl)-1H-pyrazolo[4,3-c]pyridin-6-yl]-methanon-hydrochlorid |
| 90 | Azepan-1-yl-[3-benzofuran-3-yl-1-(2,2,2-trifluor-ethyl)-1H-pyrazolo[4,3-c]pyridin-6-yl]-methanon |
| 91 | Azepan-1-yl-[3-(1H-indol-3-yl)-1-(2,2,2-trifluor-ethyl)-1H-pyrazolo[4,3-c]pyridin-6-yl]-methanon |
| 92 | Azepan-1-yl-[3-(1H-pyrrolo[2,3-b]pyridin-3-yl)-1-(2,2,2-trifluorethyl)-1H-pyrazolo[4,3-c]pyridin-6-yl]-methanon |
| 93 | 1,4-Oxazepan-4-yl-[3-thieno[2,3-c]pyridin-3-yl-1-(2,2,2-trifluorethyl)-1H-pyrazolo[4,3-c]pyridin-6-yl]-methanon |
| 94 | [3-Benzofuran-3-yl-1-(2,2,2-trifluor-ethyl)-1H-pyrazolo[4,3-c]pyridin-6-yl]-1,4-oxazepan-4-yl-methanon |
| 95 | 1,4-Oxazepan-4-yl-[3-pyrazolo[1,5-a]pyridin-3-yl-1-(2,2,2-trifluorethyl)-1H-pyrazolo[4,3-c]pyridin-6-yl]-methanon |
| 96 | [3-(1H-Indol-3-yl)-1-(2,2,2-trifluor-ethyl)-1H-pyrazolo[4,3-c]pyridin-6-yl]-1,4-oxazepan-4-yl-methanon |
| 97 | 1,4-Oxazepan-4-yl-[3-(1H-pyrrolo[2,3-b]pyridin-3-yl)-1-(2,2,2-trifluor-ethyl)-1H-pyrazolo[4,3-c]pyridin-6-yl]-methanon |
| 98 | [3-Benzo[b]thiophen-3-yl-1-(2,2,2-trifluor-ethyl)-1H-pyrazolo[4,3-c]pyridin-6-yl]-1,4-oxazepan-4-yl-methanon |
| 99 | (8-Oxa-3-aza-bicyclo[3.2.1]oct-3-yl)-[3-pyrazolo[1 ,5-a]pyridin-3-yl-1-(2,2,2-trifluor-ethyl)-1H-pyrazolo[4,3-c]pyridin-6-yl]-methanon |
| 100 | [3-Benzofuran-3-yl-1-(2,2,2-trifluor-ethyl)-1H-pyrazolo[4,3-c]pyridin-6-yl]-(8-oxa-3-aza-bicyclo[3.2.1]oct-3-yl)-methanon |
| 101 | (3-Aza-bicyclo[3.2.1]oct-3-yl)-[3-benzofuran-3-yl-1-(2,2,2-trifluorethyl)-1H-pyrazolo[4,3-c]pyridin-6-yl]-methanon |
| 102 | (3-Aza-bicyclo[3.2.1]oct-3-yl)-[3-pyrazolo[1,5-a]pyridin-3-yl-1-(2,2,2-trifluor-ethyl)-1H-pyrazolo[4,3-c]pyridin-6-yl]-methanon |
| 103 | [3-Benzofuran-3-yl-1-(2,2-difluor-ethyl)-1H-pyrazolo[4,3-c]pyridin-6-yl]-1,4-oxazepan-4-yl-methanon |
| 104 | [3-Benzofuran-3-yl-1-(2-fluor-ethyl)-1H-pyrazolo[4,3-c]pyridin-6-yl]-1,4-oxazepan-4-yl-methanon |
| 105 | 2-[3-Benzofuran-3-yl-6-(1,4-oxazepan-4-carbonyl)-pyrazolo[4,3-c]pyridin-1-yl]-acetamid |
| 106 | (3-Benzofuran-3-yl-1-propyl-1H-pyrazolo[4,3-c]pyridin-6-yl)-1,4-oxazepan-4-yl-methanon |
| 107 | (3-Benzofuran-3-yl-1-methansulfonylmethyl-1H-pyrazolo[4,3-c]pyridin-6-yl)-(8-oxa-3-aza-bicyclo[3.2.1]oct-3-yl)-methanon |
| 108 | (1-Methansulfonylmethyl-3-pyrazolo[1,5-a]pyridin-3-yl-1H-pyrazolo[4,3-c]pyridin-6-yl)-(8-oxa-3-aza-bicyclo[3.2.1]oct-3-yl)-methanon |
| 109 | [3-(5-Fluorbenzofuran-3-yl)-1-(2,2,2-trifluorethyl)pyrazolo[4,3-c]pyridin-6-yl]-(1,4-oxazepan-4-yl)methanon |
| 110 | [3-(5-Fluorbenzofuran-3-yl)-1-(methylsulfonylmethyl)pyrazolo[4,3-c]pyridin-6-yl]-(1,4-oxazepan-4-yl)methanon |
| 111 | [3-(5-Fluorbenzofuran-3-yl)-1-(methylsulfonylmethyl)pyrazolo[4,3-c]pyridin-6-yl]-(8-oxa-3-azabicyclo[3.2.1]octan-3-yl)methanon |
| 112 | (3-endo-Hydroxy-8-azabicyclo[3.2.1]octan-8-yl)-[3-(1H-indol-3-yl)-1-(2,2,2-trifluorethyl)pyrazolo[4,3-c]pyridin-6-yl]methanon |
| 113 | (3-endo-Hydroxy-8-azabicyclo[3.2.1]octan-8-yl)-[3-pyrazolo[1,5-a]pyridin-3-yl-1-(2,2,2-trifluorethyl)pyrazolo[4,3-c]pyridin-6-yl]methanon |
| 114 | (4-Hydroxy-1-piperidyl)-[3-pyrazolo[1,5-a]pyridin-3-yl-1-(2,2,2-trifluorethyl)pyrazolo[4,3-c]pyridin-6-yl]methanon |
| 115 | [3-(5-Fluorbenzofuran-3-yl)-1-(2,2,2-trifluorethyl)pyrazolo[4,3-c]pyridin-6-yl]-(4-hydroxy-1-piperidyl)methanon |
| 116 | [3-(5-Fluorbenzofuran-3-yl)-1-(2,2,2-trifluorethyl)pyrazolo[4,3-c]pyridin-6-yl]-(4-hydroxy-4-methyl-1-piperidyl)methanon |
| 117 | (4-Hydroxy-4-methyl-1-piperidyl)-[3-pyrazolo[1,5-a]pyridin-3-yl-1-(2,2,2-trifluorethyl)pyrazolo[4,3-c]pyridin-6-yl]methanon |
| 118 | [3-(5-Fluorbenzofuran-3-yl)-1-(2,2,2-trifluorethyl)pyrazolo[4,3-c]pyridin-6-yl]-(6-hydroxy-1,4-oxazepan-4-yl)methanon |
| 119 | (6-Hydroxy-1,4-oxazepan-4-yl)-[3-pyrazolo[1,5-a]pyridin-3-yl-1-(2,2,2-trifluorethyl)pyrazolo[4,3-c]pyridin-6-yl]methanon |
| 120 | [3-(5-Fluorbenzofuran-3-yl)-1-(2,2,2-trifluorethyl)pyrazolo[4,3-c]pyridin-6-yl]-(6,6-dideuterio-1,4-oxazepan-4-yl)methanon |
| 121 | (6,6-Dideuterio-1,4-oxazepan-4-yl)-[3-pyrazolo[1,5-a]pyridin-3-yl-1-(2,2,2-trifluorethyl)pyrazolo[4,3-c]pyridin-6-yl]methanon |
| 122 | [3-(5-Fluorbenzofuran-3-yl)-1-(2,2,2-trifluorethyl)pyrazolo[4,3-c]pyridin-6-yl]-(4-hydroxyazepan-1-yl)methanon |
| 123 | [3-(5-Fluor-1H-indol-3-yl)-1-(2,2,2-trifluorethyl)pyrazolo[4,3-c]pyridin-6-yl]-(1,4-oxazepan-4-yl)methanon |
| 124 | Methyl-3-[6-(1,4-oxazepan-4-carbonyl)-1-(2,2,2-trifluorethyl)pyrazolo[4,3-c]pyridin-3-yl]benzofuran-5-carboxylat |
| 125 | [3-(5-Methylbenzofuran-3-yl)-1-(2,2,2-trifluorethyl)pyrazolo[4,3-c]pyridin-6-yl]-(1,4-oxazepan-4-yl)methanon |
| 126 | [3-(2-Methylbenzofuran-3-yl)-1-(2,2,2-trifluorethyl)pyrazolo[4,3-c]pyridin-6-yl]-(1,4-oxazepan-4-yl)methanon |
| 127 | [3-(5-Fluorbenzofuran-3-yl)-1-(2,2,2-trifluorethyl)pyrazolo[4,3-c]pyridin-6-yl]-(8-oxa-3-azabicyclo[3.2.1]octan-3-yl)methanon |
| 128 | 3-(6,8-Difluor-imidazo[1,2-a]pyridin-3-yl)-1-(2,2,2-trifluor-ethyl)-1H-pyrazolo[4,3-c]pyridin-6-carbonsäure-amid |
| 129 | 3-[6-(1,4-Oxazepan-4-carbonyl)-1-(2,2,2-trifluorethyl)pyrazolo[4,3-c]pyridin-3-yl]benzofuran-5-carbonsäure |
| 130 | 1-[3-(Benzofuran-3-yl)-1-(2,2,2-trifluorethyl)pyrazolo[4,3-c]pyridin-6-carbonyl]piperidin-4-carbonsäure |
| 131 | [3-(6-Fluor-imidazo[1,2-a]pyridin-3-yl)-1-(2,2,2-trifluor-ethyl)-1H-pyrazolo[4,3-c]pyridin-6-yl]-(4-hydroxy-piperidin-1-yl)-methanon |
| 132 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-piperidin-1-yl)-methanon |
| 133 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-4-methyl-piperidin-1-yl)-methanon |
| 134 | [3-(6,8-Difluor-imidazo[1,2-a]pyridin-3-yl)-1-(2,2,2-trifluor-ethyl)-1H-pyrazolo[4,3-c]pyridin-6-yl]-(3-hydroxy-azepan-1-yl)-methanon |
| 135 | [3-(6,8-Difluor-imidazo[1,2-a]pyridin-3-yl)-1-(2,2,2-trifluor-ethyl)-1H-pyrazolo[4,3-c]pyridin-6-yl]-(2-oxa-5-aza-bicyclo[2.2.1]hept-5-yl)-methanon |
| 136 | [3-(6,8-Difluor-[1,2,4]triazolo[4,3-a]pyridin-3-yl)-1-(2,2,2-trifluorethyl)-1H-pyrazolo[4,3-c]pyridin-6-yl]-(3-hydroxy-8-azabicyclo[3.2.1]oct-8-yl)-methanon |
| 137 | [3-(6,8-Difluor-[1,2,4]triazolo[4,3-a]pyridin-3-yl)-1-(2,2,2-trifluorethyl)-1H-pyrazolo[4,3-c]pyridin-6-yl]-1,4-oxazepan-4-yl-methanon |
| 138 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-2-methyl-piperidin-1-yl)-methanon |
| 139 | (3-Aza-bicyclo[3.2.1]oct-3-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 140 | [3-(6,8-Difluor-imidazo[1,2-a]pyridin-3-yl)-1-(2,2,2-trifluor-ethyl)-1H-pyrazolo[4,3-c]pyridin-6-yl]-(9-hydroxy-3-azabicyclo[3.3.1]non-3-yl)-methanon |
| 141 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-2-isopropyl-piperidin-1-yl)-methanon |
| 142 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-2,2-dimethyl-piperidin-1-yl)-methanon |
| 143 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-3-isobutyl-piperidin-1-yl)-methanon |
| 144 | [3-(6,8-Difluor-imidazo[1,2-a]pyridin-3-yl)-1-(2,2,2-trifluor-ethyl)-1H-pyrazolo[4,3-c]pyridin-6-yl]-(4-fluor-piperidin-1-yl)-methanon |
| 145 | [3-(6,8-Difluor-imidazo[1,2-a]pyridin-3-yl)-1-(2,2,2-trifluor-ethyl)-1H-pyrazolo[4,3-c]pyridin-6-yl]-(8-oxa-2-aza-spiro[4.5]dec-2-yl)-methanon |
| 146 | [3-(6,8-Difluor-imidazo[1,2-a]pyridin-3-yl)-1-(2,2,2-trifluor-ethyl)-1H-pyrazolo[4,3-c]pyridin-6-yl]-(4,4-difluor-piperidin-1-yl)-methanon |
| 147 | [3-(6,8-Difluor-imidazo[1,2-a]pyridin-3-yl)-1-(2,2,2-trifluor-ethyl)-1H-pyrazolo[4,3-c]pyridin-6-yl]-(4'-hydroxy-3',4',5',6'-tetrahydro-2'H-[3,4']bipyridinyl-1'-yl)-methanon |
| 148 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-4-trifluormethyl-piperidin-1-yl)-methanon |
| 149 | [3-(6,8-Difluor-imidazo[1,2-a]pyridin-3-yl)-1-(2,2,2-trifluor-ethyl)-1H-pyrazolo[4,3-c]pyridin-6-yl]-(2-methoxymethyl-pyrrolidin-1-yl)-methanon |
| 150 | [3-(6,8-Difluor-imidazo[1,2-a]pyridin-3-yl)-1-(2,2,2-trifluor-ethyl)-1H-pyrazolo[4,3-c]pyridin-6-yl]-(4-methyl-1,4-diazepan-1-yl)-methanon |
| 151 | [3-(6,8-Difluor-imidazo[1,2-a]pyridin-3-yl)-1-(2,2,2-trifluor-ethyl)-1H-pyrazolo[4,3-c]pyridin-6-yl]-(3-hydroxy-piperidin-1-yl)-methanon |
| 152 | [3-(6,8-Difluor-imidazo[1,2-a]pyridin-3-yl)-1-(2,2,2-trifluor-ethyl)-1H-pyrazolo[4,3-c]pyridin-6-yl]-(7-oxa-2-aza-spiro[4.5]dec-2-yl)-methanon |
| 153 | [3-(6,8-Difluor-imidazo[1,2-a]pyridin-3-yl)-1-(2,2,2-trifluor-ethyl)-1H-pyrazolo[4,3-c]pyridin-6-yl]-(3-oxa-9-aza-spiro[5.5]undec-9-yl)-methanon |
| 154 | [3-(6,8-Difluor-imidazo[1,2-a]pyridin-3-yl)-1-(2,2,2-trifluor-ethyl)-1H-pyrazolo[4,3-c]pyridin-6-yl]-(tetrahydro-furo[3,4-c]pyrrol-5-yl)-methanon |
| 155 | 8-[3-(6,8-Difluor-imidazo[1,2-a]pyridin-3-yl)-1-(2,2,2-trifluor-ethyl)-1H-pyrazolo[4,3-c]pyridin-6-carbonyl]-2,8-diaza-spiro[4.5]decan-3-on |
| 156 | 8-[3-(6,8-Difluor-imidazo[1,2-a]pyridin-3-yl)-1-(2,2,2-trifluor-ethyl)-1H-pyrazolo[4,3-c]pyridin-6-carbonyl]-2,8-diaza-spiro[4.5]decan-1-on |
| 157 | [3-(6,8-Difluor-imidazo[1,2-a]pyridin-3-yl)-1-(2,2,2-trifluor-ethyl)-1H-pyrazolo[4,3-c]pyridin-6-yl]-[3-(2-methoxy-ethyl)-pyrrolidin-1-yl]-methanon |
| 158 | [3-(6,8-Difluor-imidazo[1,2-a]pyridin-3-yl)-1-(2,2,2-trifluor-ethyl)-1H-pyrazolo[4,3-c]pyridin-6-yl]-morpholin-4-yl-methanon |
| 159 | [3-(6,8-Difluor-imidazo[1,2-a]pyridin-3-yl)-1-(2,2,2-trifluor-ethyl)-1H-pyrazolo[4,3-c]pyridin-6-yl]-(3,3-difluor-piperidin-1-yl)-methanon |
| 160 | [3-(6,8-Difluor-imidazo[1,2-a]pyridin-3-yl)-1-(2,2,2-trifluor-ethyl)-1H-pyrazolo[4,3-c]pyridin-6-yl]-(3,3-difluor-piperidin-1-yl)-methanon |
| 161 | [3-(6,8-Difluor-imidazo[1,2-a]pyridin-3-yl)-1-(2,2,2-trifluor-ethyl)-1H-pyrazolo[4,3-c]pyridin-6-yl]-(3-hydroxymethyl-piperidin-1-yl)-methanon |
| 162 | [3-(6,8-Difluor-imidazo[1,2-a]pyridin-3-yl)-1-(2,2,2-trifluor-ethyl)-1H-pyrazolo[4,3-c]pyridin-6-yl]-(8-endo-hydroxy-8-exo-methyl-3-aza-bicyclo[3.2.1]oct-3-yl)-methanon |
| 163 | [3-(6,8-Difluor-imidazo[1,2-a]pyridin-3-yl)-1-(2,2,2-trifluor-ethyl)-1H-pyrazolo[4,3-c]pyridin-6-yl]-(2-trifluormethyl-morpholin-4-yl)-methanon |
| 164 | [3-(6,8-Difluor-imidazo[1,2-a]pyridin-3-yl)-1-(2,2,2-trifluor-ethyl)-1H-pyrazolo[4,3-c]pyridin-6-yl]-(4-trifluormethyl-piperidin-1-yl)-methanon |
| 165 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxymethyl-piperidin-1-yl)-methanon |
| 166 | [3-(6,8-Difluor-imidazo[1,2-a]pyridin-3-yl)-1-(2,2,2-trifluor-ethyl)-1H-pyrazolo[4,3-c]pyridin-6-yl]-(2-oxa-7-aza-spiro[3.5]non-7-yl)-methanon |
| 167 | [3-(6,8-Difluor-imidazo[1,2-a]pyridin-3-yl)-1-(2,2,2-trifluor-ethyl)-1H-pyrazolo[4,3-c]pyridin-6-yl]-(3-methoxymethyl-morpholin-4-yl)-methanon |
| 168 | [3-(6,8-Difluor-imidazo[1,2-a]pyridin-3-yl)-1-(2,2,2-trifluor-ethyl)-1H-pyrazolo[4,3-c]pyridin-6-yl]-(3-endo-hydroxy-8-azabicyclo[3.2.1]oct-8-yl)-methanon |
| 169 | (4-Aza-tricyclo[4.3.1.1*3,8*]undec-4-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 170 | [3-(6,8-Difluor-imidazo[1,2-a]pyridin-3-yl)-1-(2,2,2-trifluor-ethyl)-1H-pyrazolo[4,3-c]pyridin-6-yl]-(8-exo-hydroxy-3-azabicyclo[3.2.1]oct-3-yl)-methanon |
| 171 | [3-(6,8-Difluor-imidazo[1,2-a]pyridin-3-yl)-1-(2,2,2-trifluor-ethyl)-1H-pyrazolo[4,3-c]pyridin-6-yl]-(3-exo-hydroxy-8-azabicyclo[3.2.1]oct-8-yl)-methanon |
| 172 | (8-Aza-bicyclo[3.2.1]oct-8-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 173 | [3-(6,8-Difluor-imidazo[1,2-a]pyridin-3-yl)-1-(2,2,2-trifluor-ethyl)-1H-pyrazolo[4,3-c]pyridin-6-yl]-(4-difluormethyl-piperidin-1-yl)-methanon |
| 174 | 3-(6,8-Difluor-imidazo[1,2-a]pyridin-3-yl)-1-(2,2,2-trifluor-ethyl)-1H-pyrazolo[4,3-c]pyridin-6-carbonsäure-(2-hydroxy-2-methylpropyl)-amid |
| 175 | [3-(6,8-Difluor-imidazo[1,2-a]pyridin-3-yl)-1-(2,2,2-trifluor-ethyl)-1H-pyrazolo[4,3-c]pyridin-6-yl]-(2-oxa-6-azatricyclo[3.3.1.1*3,7*]dec-6-yl)-methanon |
| 176 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxymethyl-4-methylpiperidin-1-yl)-methanon |
| 177 | [3-(6,8-Difluor-imidazo[1,2-a]pyridin-3-yl)-1-(2,2,2-trifluor-ethyl)-1H-pyrazolo[4,3-c]pyridin-6-yl]-(4-hydroxy-azepan-1-yl)-methanon |
| 178 | (2-Aza-tricyclo[3.3.1.1*3,7*]dec-2-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 179 | [3-(6,8-Difluor-imidazo[1,2-a]pyridin-3-yl)-1-(2,2,2-trifluor-ethyl)-1H-pyrazolo[4,3-c]pyridin-6-yl]-(5-oxa-8-aza-spiro[2.6]non-8-yl)-methanon |
| 180 | [3-(6,8-Difluor-imidazo[1,2-a]pyridin-3-yl)-1-(2,2,2-trifluor-ethyl)-1H-pyrazolo[4,3-c]pyridin-6-yl]-1,4-thiazepan-1,1-dioxid-4-yl-methanon |
| 181 | cis-[3-(6,8-Difluor-imidazo[1,2-a]pyridin-3-yl)-1-(2,2,2-trifluorethyl)-1H-pyrazolo[4,3-c]pyridin-6-yl]-(2,6-dimethyl-morpholin-4-yl)-methanon |
| 182 | [3-(6,8-Difluor-imidazo[1,2-a]pyridin-3-yl)-1-(2,2,2-trifluor-ethyl)-1H-pyrazolo[4,3-c]pyridin-6-yl]-(2,2-dimethyl-1,4-oxazepan-4-yl)-methanon |
| 183 | cis-[3-(6,8-Difluor-imidazo[1,2-a]pyridin-3-yl)-1-(2,2,2-trifluorethyl)-1H-pyrazolo[4,3-c]pyridin-6-yl]-(3,5-dimethyl-morpholin-4-yl)-methanon |
| 184 | [3-(6,8-Difluor-imidazo[1,2-a]pyridin-3-yl)-1-(2,2,2-trifluor-ethyl)-1H-pyrazolo[4,3-c]pyridin-6-yl]-(3-oxa-8-aza-bicyclo[3.2.1]oct-8-yl)-methanon |
| 185 | [3-(6,8-Difluor-imidazo[1,2-a]pyridin-3-yl)-1-(2,2,2-trifluor-ethyl)-1H-pyrazolo[4,3-c]pyridin-6-yl]-(6,6-difluor-1,4-oxazepan-4-yl)-methanon |
| 186 | [3-(6,8-Difluor-imidazo[1,2-a]pyridin-3-yl)-1-(2,2,2-trifluor-ethyl)-1H-pyrazolo[4,3-c]pyridin-6-yl]-(8-oxa-4-aza-spiro[2.6]non-4-yl)-methanon |
| 187 | [3-(6,8-Difluor-imidazo[1,2-a]pyridin-3-yl)-1-(2,2,2-trifluor-ethyl)-1H-pyrazolo[4,3-c]pyridin-6-yl]-(8-endo-hydroxy-3-azabicyclo[3.2.1]oct-3-yl)-methanon |
| 188 | [3-(6,8-Difluor-imidazo[1,2-a]pyridin-3-yl)-1-(2,2,2-trifluor-ethyl)-1H-pyrazolo[4,3-c]pyridin-6-yl]-(7-endo-hydroxy-3-oxa-9-azabicyclo[3.3.1]non-9-yl)-methanon |
| 189 | 1-[3-(6,8-Difluor-imidazo[1,2-a]pyridin-3-yl)-1-(2,2,2-trifluor-ethyl)-1H-pyrazolo[4,3-c]pyridin-6-carbonyl]-1,4-diazepan-5-on |
| 190 | [3-(6,8-Difluor-imidazo[1,2-a]pyridin-3-yl)-1-(2,2,2-trifluor-ethyl)-1H-pyrazolo[4,3-c]pyridin-6-yl]-1,4-oxazepan-deuterium-4-yl-methanon |
| 191 | [3-(6,8-Difluor-imidazo[1,2-a]pyridin-3-yl)-1-(2,2,2-trifluor-ethyl)-1H-pyrazolo[4,3-c]pyridin-6-yl]-[1,2]oxazepan-2-yl-methanon |
| 192 | [3-(6,8-Difluor-imidazo[1,2-a]pyridin-3-yl)-1-(2,2,2-trifluor-ethyl)-1H-pyrazolo[4,3-c]pyridin-6-yl]-(5-oxa-8-aza-spiro[3.5]non-8-yl)-methanon |
| 193 | [3-(6,8-Difluor-imidazo[1,2-a]pyridin-3-yl)-1-(2,2,2-trifluor-ethyl)-1H-pyrazolo[4,3-c]pyridin-6-yl]-(6-hydroxy-1,4-oxazepan-4-yl)-methanon |
| 194 | 5-[3-(6,8-Difluor-imidazo[1,2-a]pyridin-3-yl)-1-(2,2,2-trifluor-ethyl)-1H-pyrazolo[4,3-c]pyridin-6-carbonyl]-2,5-diaza-bicyclo[2.2.1]heptan-2-carbonsäure-tert-butylester |
| 195 | 1,4-Diazepan-1-yl-[3-(6,8-difluor-imidazo[1,2-a]pyridin-3-yl)-1-(2,2,2-trifluor-ethyl)-1H-pyrazolo[4,3-c]pyridin-6-yl]-methanon |
| 196 | [3-(6,8-Difluor-imidazo[1,2-a]pyridin-3-yl)-1-(2,2,2-trifluor-ethyl)-1H-pyrazolo[4,3-c]pyridin-6-yl]-(8-oxa-5-aza-spiro[3.5]non-5-yl)-methanon |
| 197 | (2-{[3-(6,8-Difluor-imidazo[1,2-a]pyridin-3-yl)-1-(2,2,2-trifluorethyl)-1H-pyrazolo[4,3-c]pyridin-6-carbonyl]-methyl-amino}-ethyl)-carbamidsäure-tert-butylester |
| 198 | 3-(6,8-Difluor-imidazo[1,2-a]pyridin-3-yl)-1-(2,2,2-trifluor-ethyl)-1H-pyrazolo[4,3-c]pyridin-6-carbonsäure-(4-hydroxy-tetrahydro-pyran-3-yl)-amid |
| 199 | [3-(6,8-Difluor-imidazo[1,2-a]pyridin-3-yl)-1-(2,2,2-trifluor-ethyl)-1H-pyrazolo[4,3-c]pyridin-6-yl]-(6-oxa-3-aza-bicyclo[3.2.1]oct-3-yl)-methanon |
| 200 | 3-[3-(6,8-Difluor-imidazo[1,2-a]pyridin-3-yl)-1-(2,2,2-trifluor-ethyl)-1H-pyrazolo[4,3-c]pyridin-6-carbonyl]-3,8-diaza-bicyclo[3.2.1]octan-8-carbonsäure-tert-butylester |
| 201 | 4-[3-(6,8-Difluor-imidazo[1,2-a]pyridin-3-yl)-1-(2,2,2-trifluor-ethyl)-1H-pyrazolo[4,3-c]pyridin-6-carbonyl]-1,4-diazepan-2-on |
| 202 | {1-[3-(6,8-Difluor-imidazo[1,2-a]pyridin-3-yl)-1-(2,2,2-trifluor-ethyl)-1H-pyrazolo[4,3-c]pyridin-6-carbonyl]-azepan-3-yl}-carbamidsäure-tert-butylester |
| 203 | (3,3-Difluor-4-hydroxy-piperidin-1-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 204 | {4-[3-(6,8-Difluor-imidazo[1,2-a]pyridin-3-yl)-1-(2,2,2-trifluor-ethyl)-1H-pyrazolo[4,3-c]pyridin-6-carbonyl]-1,4-oxazepan-6-yl}-carbamidsäure-tert-butylester |
| 205 | [3-(6,8-Difluor-imidazo[1,2-a]pyridin-3-yl)-1-(2,2,2-trifluor-ethyl)-1H-pyrazolo[4,3-c]pyridin-6-yl]-(2-oxa-5-aza-bicyclo[4.1.0]hept-5-yl)-methanon |
| 206 | [3-(6,8-Difluor-imidazo[1,2-a]pyridin-3-yl)-1-(2,2,2-trifluor-ethyl)-1H-pyrazolo[4,3-c]pyridin-6-yl]-(7-oxa-4-aza-spiro[2.6]non-4-yl)-methanon |
| 207 | [3-Benzofuran-3-yl-1-(2,2,2-trifluor-ethyl)-1H-pyrazolo[4,3-c]pyridin-6-yl]-(4-hydroxy-piperidin-1-yl)-methanon |
| 208 | [3-Benzofuran-3-yl-1-(2,2,2-trifluor-ethyl)-1H-pyrazolo[4,3-c]pyridin-6-yl]-(4-hydroxy-4-methyl-piperidin-1-yl)-methanon |
| 209 | [3-Benzofuran-3-yl-1-(2,2,2-trifluor-ethyl)-1H-pyrazolo[4,3-c]pyridin-6-yl]-(3-endo-hydroxy-8-aza-bicyclo[3.2.1]oct-8-yl)-methanon |
| 210 | [3-Benzofuran-3-yl-1-(2,2,2-trifluor-ethyl)-1H-pyrazolo[4,3-c]pyridin-6-yl]-(8-exo-ethyl-8-endo-hydroxy-3-azabicyclo[3.2.1]oct-3-yl)-methanon |
| 211 | [3-Benzofuran-3-yl-1-(2,2,2-trifluor-ethyl)-1H-pyrazolo[4,3-c]pyridin-6-yl]-(5-endo-hydroxy-5-exo-methyl-2-azabicyclo[2.2.1]hept-2-yl)-methanon |
| 212 | [3-Benzofuran-3-yl-1-(2,2,2-trifluor-ethyl)-1H-pyrazolo[4,3-c]pyridin-6-yl]-(8-endo-hydroxy-8-exo-methyl-3-azabicyclo[3.2.1]oct-3-yl)-methanon |
| 213 | [3-Benzofuran-3-yl-1-(2,2,2-trifluor-ethyl)-1H-pyrazolo[4,3-c]pyridin-6-yl]-(8-endo-hydroxy-8-exo-methyl-d3-3-azabicyclo[3.2.1]oct-3-yl)-methanon |
| 214 | [3-Benzofuran-3-yl-1-(2,2,2-trifluor-ethyl)-1H-pyrazolo[4,3-c]pyridin-6-yl]-(8-exo-ethinyl-8-endo-hydroxy-3-azabicyclo[3.2.1]oct-3-yl)-methanon |
| 215 | [3-Benzofuran-3-yl-1-(2,2,2-trifluor-ethyl)-1H-pyrazolo[4,3-c]pyridin-6-yl]-(2-methoxymethyl-pyrrolidin-1-yl)-methanon |
| 216 | [3-Benzofuran-3-yl-1-(2,2,2-trifluor-ethyl)-1H-pyrazolo[4,3-c]pyridin-6-yl]-(4-fluor-piperidin-1-yl)-methanon |
| 217 | 3-Benzofuran-3-yl-1-(2,2,2-trifluor-ethyl)-1H-pyrazolo[4,3-c]pyridin-6-yl]-(6,6-difluor-1,4-oxazepan-4-yl)-methanon |
| 218 | [3-Benzofuran-3-yl-1-(2,2,2-trifluor-ethyl)-1H-pyrazolo[4,3-c]pyridin-6-yl]-(8-endo-hydroxy-3-aza-bicyclo[3.2.1]oct-3-yl)-methanon |
| 219 | [3-Benzofuran-3-yl-1-(2,2,2-trifluor-ethyl)-1H-pyrazolo[4,3-c]pyridin-6-yl]-(4-hydroxy-2,2-dimethyl-piperidin-1-yl)-methanon |
| 220 | [3-Benzofuran-3-yl-1-(2,2-difluor-ethyl)-1H-pyrazolo[4,3-c]pyridin-6-yl]-(3-endo-hydroxy-8-aza-bicyclo[3.2.1]oct-8-yl)-methanon |
| 221 | [3-Benzofuran-3-yl-1-(2-fluor-ethyl)-1H-pyrazolo[4,3-c]pyridin-6-yl]-(3-endo-hydroxy-8-aza-bicyclo[3.2.1]oct-8-yl)-methanon |
| 222 | (3-Benzofuran-3-yl-1-propyl-1H-pyrazolo[4,3-c]pyridin-6-yl)-(3-endo-hydroxy-8-aza-bicyclo[3.2.1]oct-8-yl)-methanon |
| 223 | tert-Butyl-N-[4-[3-(6,8-difluorimidazo[1,2-a]pyridin-3-yl)-1-(2,2,2-trifluorethyl)pyrazolo[4,3-c]pyridin-6-carbonyl]-1,4-oxazepan-6-yl]carbamat |
| 224 | [3-(Benzofuran-3-yl)-1-(2,2,2-trifluorethyl)pyrazolo[4,3-c]pyridin-6-yl]-(6-hydroxy-1,4-oxazepan-4-yl)methanon |
| 225 | [3-(Benzofuran-3-yl)-1-(2,2,2-trifluorethyl)pyrazolo[4,3-c]pyridin-6-yl]-(4,4-difluor-1-piperidyl)methanon |
| 226 | [3-(Benzofuran-3-yl)-1-(2,2,2-trifluorethyl)pyrazolo[4,3-c]pyridin-6-yl]-(3,3-difluor-1-piperidyl)methanon |
| 227 | [3-Benzofuran-3-yl-1-(2,2,2-trifluor-ethyl)-1H-pyrazolo[4,3-c]pyridin-6-yl]-(3-hydroxy-piperidin-1-yl)-methanon |
| 228 | [3-(Benzofuran-3-yl)-1-(2,2,2-trifluorethyl)pyrazolo[4,3-c]pyridin-6-yl]-(4-hydroxyazepan-1-yl)methanon |
| 229 | [3-(Benzofuran-3-yl)-1-(2,2,2-trifluorethyl)pyrazolo[4,3-c]pyridin-6-yl]-(6,6-dideuterio-1,4-oxazepan-4-yl)methanon |
| 230 | [3-(Benzofuran-3-yl)-1-(2,2,2-trifluorethyl)pyrazolo[4,3-c]pyridin-6-yl]-[6-fluor-1,4-oxazepan-4-yl]methanon |
| 231 | 3-(Benzofuran-3-yl)-N, N-di(cyclobutyl)-1-(2,2,2-trifluorethyl)pyrazolo[4,3-c]pyridin-6-carboxamid |
| 232 | 3-(Benzofuran-3-yl)-N, N-di(cyclopropyl)-1-(2,2,2-trifluorethyl)pyrazolo[4,3-c]pyridin-6-carboxamid |
| 233 | 3-(Benzofuran-3-yl)-N, N-diisopropyl-1-(2,2,2-trifluorethyl)pyrazolo[4,3-c]pyridin-6-carboxamid |
| 234 | 3-(Benzofuran-3-yl)-N-ethyl-N-isopropyl-1-(2,2,2-trifluorethyl)pyrazolo[4,3-c]pyridin-6-carboxamid |
| 235 | 3-(Benzofuran-3-yl)-N-cyclohexyl-N-ethyl-1-(2,2,2-trifluorethyl)pyrazolo[4,3-c]pyridin-6-carboxamid |
| 236 | [3-(Benzofuran-3-yl)-1-(2,2,2-trifluorethyl)pyrazolo[4,3-c]pyridin-6-yl]-(8-hydroxy-8-isopropyl-3-azabicyclo[3.2.1]octan-3-yl)methanon |
| 237 | 3-[6-(1,4-Oxazepan-4-carbonyl)-1-(2,2,2-trifluorethyl)pyrazolo[4,3-c]pyridin-3-yl]benzofuran-5-carboxamid |
| 238 | (3,8-Diaza-bicyclo[3.2.1]oct-3-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 239 | (2,5-Diaza-bicyclo[2.2.1]hept-2-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 240 | (3-Amino-azepan-1-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 241 | (6-Amino-1,4-oxazepan-4-yl)-[3-(6,8-difluor-imidazo[1,2-a]pyridin-3-yl)-1-(2,2,2-trifluor-ethyl)-1H-pyrazolo[4,3-c]pyridin-6-yl]-methanon |
| 242 | (6,6-Dideuterio-1,4-oxazepan-4-yl)-[3-(1H-indol-3-yl)-1-(2,2,2-trifluorethyl)pyrazolo[4,3-c]pyridin-6-yl]methanon |
| 243 | [3-(1H-Indol-3-yl)-1-(2,2,2-trifluorethyl)pyrazolo[4,3-c]pyridin-6-yl]-(8-oxa-3-azabicyclo[3.2.1]octan-3-yl) methanon |
| 244 | 3-[6-(Azepan-1-carbonyl)-1-(2,2,2-trifluor-ethyl)-1H-pyrazolo[4,3-c]pyridin-3-yl]-8-methyl-imidazo[1,2-a]pyridin-6-carbonsäure-amid |
| 245 | 3-[6-(Azepan-1-carbonyl)-1-propyl-1H-pyrazolo[4,3-c]pyridin-3-yl]-imidazo[1,2-a]pyridin-6-carbonsäure-amid |
| 246 | 3-[6-(1,4-Oxazepan-4-carbonyl)-1-(2,2,2-trifluor-ethyl)-1H-pyrazolo[4,3-c]pyridin-3-yl]-imidazo[1,2-a]pyridin-6-carbonsäure-amid |
| 247 | 3-[6-(8-Oxa-3-aza-bicyclo[3.2.1]octan-3-carbonyl)-1-(2,2,2-trifluorethyl)-1H-pyrazolo[4,3-c]pyridin-3-yl]-imidazo[1,2-a]pyridin-6-carbonsäure-amid |
| 248 | Azepan-1-yl-[3-pyrazolo[1,5-a]pyrazin-3-yl-1-(2,2,2-trifluor-ethyl)-1H-pyrazolo[4,3-c]pyridin-6-yl]-methanon |
| 249 | 1,4-Oxazepan-4-yl-[3-pyrazolo[1,5-a]pyrazin-3-yl-1-(2,2,2-trifluorethyl)-1H-pyrazolo[4,3-c]pyridin-6-yl]-methanon |
| 250 | (1-Methansulfonylmethyl-3-pyrazolo[1,5-a]pyrazin-3-yl-1H-pyrazolo[4,3-c]pyridin-6-yl)-(1,4-oxazepan-4-yl)-methanon |
| 251 | [3-(Benzimidazol-1-yl)-1-(2,2,2-trifluorethyl)pyrazolo[4,3-c]pyridin-6-yl]-(1,4-oxazepan-4-yl)methanon |
| 252 | [3-(6-Fluorbenzimidazol-1-yl)-1-(2,2,2-trifluorethyl)pyrazolo[4,3-c]pyridin-6-yl]-(1,4-oxazepan-4-yl)methanon |
| 253 | [3-Furo[3,2-b]pyridin-3-yl-1-(2,2,2-trifluorethyl)pyrazolo[4,3-c]pyridin-6-yl]-(1,4-oxazepan-4-yl)methanon |
| 254 | [1,4]Oxazepan-4-yl-[3-(1H-pyrrolo[3,2-b]pyridin-3-yl)-1-(2,2,2-trifluor-ethyl)-1H-pyrazolo[4,3-c]pyridin-6-yl]-methanon |
| 255 | [1,4]Oxazepan-4-yl-[3-(1H-pyrrolo[2,3-c]pyridin-3-yl)-1-(2,2,2-trifluor-ethyl)-1H-pyrazolo[4,3-c]pyridin-6-yl]-methanon |
| 256 | (8-Oxa-3-aza-bicyclo[3.2.1]oct-3-yl)-[3-(1H-pyrrolo[3,2-c]pyridin-3-yl)-1-(2,2,2-trifluor-ethyl)-1H-pyrazolo[4,3-c]pyridin-6-yl]-methanon |
und pharmazeutisch unbedenkliche Salze oder Solvate von irgendeiner der vorstehend genannten Verbindungen.

12. Verbindung irgendeines der Ansprüche 1 bis 11 zur Verwendung als Arzneimittel.

13. Pharmazeutische Zusammensetzung enthaltend die Verbindung irgendeines der Ansprüche 1 bis 11 und einen pharmazeutisch unbedenklichen Hilfsstoff.

14. Verbindung irgendeines der Ansprüche 1 bis 11 oder pharmazeutische Zusammensetzung des Anspruchs 13 zur Verwendung bei der Behandlung oder Prävention einer hyperproliferativen Krankheit oder Störung oder einer Infektionskrankheit oder -störung.

15. Verwendung der Verbindung irgendeines der Ansprüche 1 bis 11 bei der Herstellung eines Arzneimittels für die Behandlung oder Prävention einer hyperproliferativen Krankheit oder Störung oder einer Infektionskrankheit oder -störung.

16. Verbindung zur Verwendung nach Anspruch 14 oder pharmazeutische Zusammensetzung zur Verwendung nach Anspruch 14 oder Verwendung des Anspruchs 15, wobei die zu behandelnde oder verhindernde Krankheit oder Störung eine hyperproliferative Krankheit oder Störung ist und wobei es sich bei der hyperproliferativen Krankheit oder Störung um Krebs handelt.

17. Verbindung zur Verwendung nach Anspruch 16 oder pharmazeutische Zusammensetzung zur Verwendung nach Anspruch 16 oder Verwendung des Anspruchs 16, wobei der Krebs ausgewählt ist aus der Gruppe bestehend aus akuter Granulozytenleukämie, akuter lymphatischer Leukämie, akuter myeloischer Leukämie, Nierenrindenkrebs, Blasenkrebs, Hirnkrebs, Brustkrebs, Gebärmutterhalskrebs, Gebärmutterhalshyperplasie, Aderhautkrebs, chronischer Granulozytenleukämie, chronischer lymphatischer Leukämie, chronischer myeloischer Leukämie, Kolonkrebs, Endometriumkrebs, Speiseröhrenkrebs, essentieller Thrombozythämie, Urogenitalkarzinom, Gliom, Glioblastom, Haarzellenleukämie, Kopf- und Halskarzinom, Morbus Hodgkin, Kaposi-Sarkom, Lungenkarzinom, Lymphom, bösartigem Karzinoid-Karzinom, bösartiger Hyperkalzämie, bösartigem Melanom, bösartigem Insulinom der Bauchspeicheldrüse, medullärem Schilddrüsenkarzinom, Melanom, multiplem Myelom, Mycosis Fungoides, Neuroblastom, Non-Hodgkin-Lymphom, nicht-kleinzelligem Lungenkrebs, osteogenem Sarkom, Eierstockkarzinom, Bauchspeicheldrüsenkarzinom, Polycythemia vera, primärem Hirnkarzinom, primärer Makroglobulinämie, Prostatakrebs, Nierenzellkrebs, Rhabdomyosarkom, Hautkrebs, kleinzelligem Lungenkrebs, Weichteilsarkom, Plattenepithelkrebs, Magenkrebs, Hodenkrebs, Schilddrüsenkrebs und Wilms-Tumor.

18. Verbindung zur Verwendung nach Anspruch 14 oder pharmazeutische Zusammensetzung zur Verwendung nach Anspruch 14 oder Verwendung des Anspruchs 15, wobei die zu behandelnde oder verhindernde Krankheit oder Störung eine hyperproliferative Krankheit oder Störung ist und wobei die hyperproliferative Krankheit oder Störung ausgewählt ist aus der Gruppe bestehend aus altersbedingter Makuladegeneration, Morbus Crohn, Zirrhose, einer mit chronischer Entzündung zusammenhängenden Störung, proliferativer diabetischer Retinopathie, proliferativer Vitreoretinopathie, Frühgeborenenretinopathie, Granulomatose, Immunhyperproliferation im Zusammenhang mit Organ- oder Gewebetransplantation und einer immunproliferativen Krankheit/Störung.

19. Verbindung zur Verwendung nach Anspruch 18 oder pharmazeutische Zusammensetzung zur Verwendung nach Anspruch 18 oder Verwendung des Anspruchs 18, wobei die zu behandelnde oder verhindernde Krankheit oder Störung eine immunproliferative Krankheit oder Störung ist und wobei die immunproliferative Krankheit oder Störung ausgewählt ist aus der Gruppe bestehend aus chronisch-entzündlicher Darmerkrankung, Psoriasis, rheumatoider Arthritis, systemischem Lupus erythematodes (SLE), Gefäßhyperproliferation als Begleiterscheinung von Netzhauthypoxie und Vaskulitis.

20. Verbindung zur Verwendung nach Anspruch 14 oder pharmazeutische Zusammensetzung zur Verwendung nach Anspruch 14 oder Verwendung des Anspruchs 15, wobei die zu behandelnde oder verhindernde Krankheit oder Störung eine Infektionskrankheit oder -störung ist und wobei die Infektionskrankheit oder -störung ausgewählt ist aus der Gruppe bestehend aus:
(a) durch Viren ausgelösten Infektionskrankheiten, die verursacht werden durch Retroviren, Hepadnaviren, Herpesviren, Flaviviridae und/oder Adenoviren, wobei die Retroviren ausgewählt sind aus Lentiviren oder Onkoretroviren, wobei das Lentivirus ausgewählt ist aus der Gruppe bestehend aus HIV-1, HIV-2, FIV, BIV, SIVs, SHIV, CAEV, VMV und EIAV, wobei das Onkoretrovirus ausgewählt ist aus der Gruppe bestehend aus HTLV-I, HTLV-II und BLV, wobei das Hepadnavirus ausgewählt ist aus der Gruppe bestehend aus HBV, GSHV und WHV, wobei das Herpesvirus ausgewählt ist aus der Gruppe bestehend aus HSV I, HSV II, EBV, VZV, HCMV und HHV 8 und wobei die Flaviviridae ausgewählt sind aus der Gruppe bestehend aus HCV, West-Nil- und Gelbfieber;
(b) bakteriellen Infektionskrankheiten, die verursacht werden durch Grampositive Bakterien, wobei die Gram-positiven Bakterien ausgewählt sind aus der Gruppe bestehend aus Methicillin-empfindlichen und Methicillinresistenten Staphylokokken (darunter Staphylococcus aureus, Staphylococcus epidermidis, Staphylococcus haemolyticus, Staphylococcus hominis, Staphylococcus saprophyticus und Coagulase-negative Staphylokokken), für Glykopeptid-Zwischenprodukte empfindlichem Staphylococcus aureus (GISA), Penicillin-empfindlichen und Penicillin-resistenten Streptokokken (darunter Streptococcus pneumoniae, Streptococcus pyogenes, Streptococcus agalactiae, Streptococcus avium, Streptococcus bovis, Streptococcus lactis, Streptococcus sanguis und Gruppe-C-Streptokokken (GCS), Gruppe-G-Streptokokken (GGS) und Viridans-Streptokokken), Enterokokken (darunter Vancomycin-empfindliche und Vancomycin-resistente Stämme wie Enterococcus faecalis und Enterococcus faecium), Clostridium difficile, Listeria monocytogenes, Corynebacterium jeikeium, Chlamydia spp. (darunter C. pneumoniae) und Mycobacterium tuberculosis;
(c) bakteriellen Infektionskrankheiten, die verursacht werden durch Gramnegative Bakterien, wobei die Gram-negativen Bakterien ausgewählt sind aus der Gruppe bestehend aus der Gattung Enterobacteriaceae, darunter Escherichia spp. (darunter Escherichia coli), Klebsiella spp., Enterobacter spp., Citrobacter spp., Serratia spp., Proteus spp., Providencia spp., Salmonella spp., Shigella spp., der Gattung Pseudomonas (darunter P. aeruginosa), Moraxella spp. (darunter M. catarrhalis), Haemophilus spp. und Neisseria spp.; und
(d) Infektionskrankheiten, die ausgelöst werden durch intrazellulär aktive Parasiten, die ausgewählt sind aus der Gruppe bestehend aus dem Stamm Apicomplexa, Sarcomastigophora (darunter Trypanosoma, Plasmodia, Leishmania, Babesia oder Theileria), Cryptosporidia, Sacrocystida, Amoebia, Coccidia und Trichomonadia.

21. Verbindung zur Verwendung nach irgendeinem der Ansprüche 14 oder 16 bis 20 oder pharmazeutische Zusammensetzung zur Verwendung nach irgendeinem der Ansprüche 14 oder 16 bis 20 oder Verwendung irgendeines der Ansprüche 15 oder 16 bis 20, wobei die Verbindung, die pharmazeutische Zusammensetzung oder das Arzneimittel in Kombination mit einem oder mehreren weiteren Therapeutika zu verabreichen ist.

22. Verbindung zur Verwendung nach irgendeinem der Ansprüche 14 oder 16 bis 21 oder pharmazeutische Zusammensetzung zur Verwendung nach irgendeinem der Ansprüche 14 oder 16 bis 21 oder Verwendung irgendeines der Ansprüche 15 oder 16 bis 21, wobei das zu behandelnde Individuum ein Mensch ist.

23. Set oder Kit umfassend getrennte Packungen von:
(a) einer Verbindung wie in irgendeinem der Ansprüche 1 bis 11 definiert; und
(b) einem weiteren Therapeutikum.

24. *In-vitro*-Verwendung einer Verbindung wie in irgendeinem der Ansprüche 1 bis 11 definiert als dualer A_{2A}- und A_{2B}-Rezeptor-Antagonist.

## Revendications

1. Composé de formule (I) 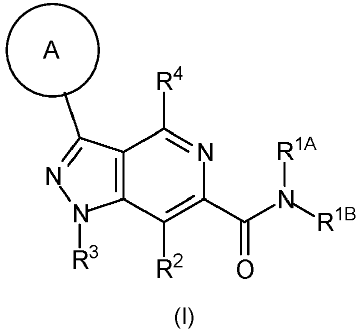 dans lequel :
R^{1A} et R^{1B} sont liés mutuellement pour former, conjointement avec l'atome d'azote auquel ils sont liés, un hétérocycloalkyle qui est éventuellement substitué par un ou plusieurs groupements R¹¹ ;
ou, de manière alternative, R^{1A} et R^{1B} sont chacun choisis indépendamment parmi hydrogène, C₁₋₅ alkyle, C₂₋₅ alcényle, C₂₋₅ alcynyle, -CO(C₁₋₅ alkyl), carbocyclyle, et hétérocyclyle, où ledit alkyle, ledit alcényle, ledit alcynyle, et le motif alkyle dudit -CO(C₁₋₅ alkyl) sont chacun éventuellement substitués par un ou plusieurs groupements R¹², et en outre où ledit carbocyclyle et ledit hétérocyclyle sont chacun éventuellement substitués par un ou plusieurs groupements R¹³;
chaque R¹¹ est choisi indépendamment parmi C₁₋₅ alkyle, C₂₋₅ alcényle, C₂₋₅ alcynyle, -(C₀₋₃ alkylène)-OH, -(C₀₋₃ alkylène)-O(C₁₋₅ alkyl), -(C₀₋₃ alkylène)-O(C₁₋₅ alkylène)-OH, -(C₀₋₃ alkylène)-O(C₁₋₅ alkylène)-O(C₁₋₅ alkyl), -(C₀₋₃ alkylène)-SH, -(C₀₋₃ alkylène)-S(C₁₋₅ alkyl), -(C₀₋₃ alkylène)-NH₂, -(C₀₋₃ alkylène)-NH(C₁₋₅ alkyl), -(C₀₋₃ alkylène)-N(C₁₋₅ alkyl)(C₁₋₅ alkyl), -(C₀₋₃ alkylène)-halogène, -(C₀₋₃ alkylène)-(C₁₋₅ halogénoalkyl), -(C₀₋₃ alkylène)-O-(C₁₋₅ halogénoalkyl), -(C₀₋₃ alkylène)-CF₃, -(C₀₋₃ alkylène)-CN, -(C₀₋₃ alkylène)-NO₂, -(C₀₋₃ alkylène)-CHO, -(C₀₋₃ alkylène)-CO-(C₁₋₅ alkyl), -(C₀₋₃ alkylène)-COOH, -(C₀₋₃ alkylène)-CO-O-(C₁₋₅ alkyl), -(C₀₋₃ alkylène)-O-CO-(C₁₋₅ alkyl), -(C₀₋₃ alkylène)-CO-NH₂, -(C₀₋₃ alkylène)-CO-NH(C₁₋₅ alkyl), -(C₀₋₃ alkylène)-CO-N(C₁₋₅ alkyl)(C₁₋₅ alkyl), -(C₀₋₃ alkylène)-NH-CO-(C₁₋₅ alkyl), -(C₀₋₃ alkylène)-N(C₁₋₅ alkyl)-CO-(C₁₋₅ alkyl), -(C₀₋₃ alkylène)-NH-CO-O-(C₁₋₅ alkyl), -(C₀₋₃ alkylène)-N(C₁₋₅ alkyl)-CO-O-(C₁₋₅ alkyl), -(C₀₋₃ alkylène)-O-CO-NH-(C₁₋₅ alkyl), -(C₀₋₃ alkylène)-O-CO-N(C₁₋₅ alkyl)-(C₁₋₅ alkyl), -(C₀₋₃ alkylène)-SO₂-NH₂, -(C₀₋₃ alkylène)-SO₂-NH(C₁₋₅ alkyl), -(C₀₋₃ alkylène)-SO₂-N(C₁₋₅ alkyl)(C₁₋₅ alkyl), -(C₀₋₃ alkylène)-NH-SO₂-(C₁₋₅ alkyl), -(C₀₋₃ alkylène)-N(C₁₋₅ alkyl)-SO₂-(C₁₋₅ alkyl), -(C₀₋₃ alkylène)-SO-(C₁₋₅ alkyl), -(C₀₋₃ alkylène)-SO₂-(C₁₋₅ alkyl), -(C₀₋₃ alkylène)-carbocyclyle, et -(C₀₋₃ alkylène)-hétérocyclyle, où le motif carbocyclyle dudit -(C₀₋₃ alkylène)-carbocyclyle et le motif hétérocyclyle dudit -(C₀₋₃ alkylène)-hétérocyclyle sont chacun éventuellement substitués par un ou plusieurs groupements choisis indépendamment parmi C₁₋₅ alkyle, C₂₋₅ alcényle, C₂₋₅ alcynyle, -OH, -O(C₁₋₅ alkyl), -O(C₁₋₅ alkylène)-OH, -O(C₁₋₅ alkylène)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogène, C₁₋₅ halogénoalkyle, -O-(C₁₋₅ halogénoalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyle, et hétérocycloalkyle ;
chaque R¹² est choisi indépendamment parmi -OH, -O(C₁₋₅ alkyl), -O(C₁₋₅ alkylène)-OH, -O(C₁₋₅ alkylène)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogène, C₁₋₅ halogénoalkyle, -O-(C₁₋₅ halogénoalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyle, et hétérocycloalkyle ;
chaque R¹³ est choisi indépendamment parmi C₁₋₅ alkyle, C₂₋₅ alcényle, C₂₋₅ alcynyle, -(C₀₋₃ alkylène)-OH, -(C₀₋₃ alkylène)-O(C₁₋₅ alkyl), -(C₀₋₃ alkylène)-O(C₁₋₅ alkylène)-OH, -(C₀₋₃ alkylène)-O(C₁₋₅ alkylène)-O(C₁₋₅ alkyl), -(C₀₋₃ alkylène)-SH, -(C₀₋₃ alkylène)-S(C₁₋₅ alkyl), -(C₀₋₃ alkylène)-NH₂, -(C₀₋₃ alkylène)-NH(C₁₋₅ alkyl), -(C₀₋₃ alkylène)-N(C₁₋₅ alkyl)(C₁₋₅ alkyl), -(C₀₋₃ alkylène)-halogène, -(C₀₋₃ alkylène)-(C₁₋₅ halogénoalkyl), -(C₀₋₃ alkylène)-O-(C₁₋₅ halogénoalkyl), -(C₀₋₃ alkylène)-CF₃, -(C₀₋₃ alkylène)-CN, -(C₀₋₃ alkylène)-NO₂, -(C₀₋₃ alkylène)-CHO, -(C₀₋₃ alkylène)-CO-(C₁₋₅ alkyl), -(C₀₋₃ alkylène)-COOH, -(C₀₋₃ alkylène)-CO-O-(C₁₋₅ alkyl), -(C₀₋₃ alkylène)-O-CO-(C₁₋₅ alkyl), -(C₀₋₃ alkylène)-CO-NH₂, -(C₀₋₃ alkylène)-CO-NH(C₁₋₅ alkyl), -(C₀₋₃ alkylène)-CO-N(C₁₋₅ alkyl)(C₁₋₅ alkyl), -(C₀₋₃ alkylène)-NH-CO-(C₁₋₅ alkyl), -(C₀₋₃ alkylène)-N(C₁₋₅ alkyl)-CO-(C₁₋₅ alkyl), -(C₀₋₃ alkylène)-NH-CO-O-(C₁₋₅ alkyl), -(C₀₋₃ alkylène)-N(C₁₋₅ alkyl)-CO-O-(C₁₋₅ alkyl), -(C₀₋₃ alkylène)-O-CO-NH-(C₁₋₅ alkyl), -(C₀₋₃ alkylène)-O-CO-N(C₁₋₅ alkyl)-(C₁₋₅ alkyl), -(C₀₋₃ alkylène)-SO₂-NH₂, -(C₀₋₃ alkylène)-SO₂-NH(C₁₋₅ alkyl), -(C₀₋₃ alkylène)-SO₂-N(C₁₋₅ alkyl)(C₁₋₅ alkyl), -(C₀₋₃ alkylène)-NH-SO₂-(C₁₋₅ alkyl), -(C₀₋₃ alkylène)-N(C₁₋₅ alkyl)-SO₂-(C₁₋₅ alkyl), -(C₀₋₃ alkylène)-SO-(C₁₋₅ alkyl), -(C₀₋₃ alkylène)-SO₂-(C₁₋₅ alkyl), -(C₀₋₃ alkylène)-cycloalkyle, et -(C₀₋₃ alkylène)-hétérocycloalkyle ;
R² et R⁴ sont chacun choisis indépendamment parmi hydrogène, C₁₋₅ alkyle, C₂₋₅ alcényle, C₂₋₅ alcynyle, -(C₀₋₃ alkylène)-OH, -(C₀₋₃ alkylène)-O(C₁₋₅ alkyl), -(C₀₋₃ alkylène)-O(C₁₋₅ alkylène)-OH, -(C₀₋₃ alkylène)-O(C₁₋₅ alkylène)-O(C₁₋₅ alkyl), -(C₀₋₃ alkylène)-SH, -(C₀₋₃ alkylène)-S(C₁₋₅ alkyl), -(C₀₋₃ alkylène)-NH₂, -(C₀₋₃ alkylène)-NH(C₁₋₅ alkyl), -(C₀₋₃ alkylène)-N(C₁₋₅ alkyl)(C₁₋₅ alkyl), -(C₀₋₃ alkylène)-halogène, -(C₀₋₃ alkylène)-(C₁₋₅ halogénoalkyl), -(C₀₋₃ alkylène)-O-(C₁₋₅ halogénoalkyl), -(C₀₋₃ alkylène)-CF₃, -(C₀₋₃ alkylène)-CN, -(C₀₋₃ alkylène)-NO₂, -(C₀₋₃ alkylène)-CHO, -(C₀₋₃ alkylène)-CO-(C₁₋₅ alkyl), -(C₀₋₃ alkylène)-COOH, -(C₀₋₃ alkylène)-CO-O-(C₁₋₅ alkyl), -(C₀₋₃ alkylène)-O-CO-(C₁₋₅ alkyl), -(C₀₋₃ alkylène)-CO-NH₂, -(C₀₋₃ alkylène)-CO-NH(C₁₋₅ alkyl), -(C₀₋₃ alkylène)-CO-N(C₁₋₅ alkyl)(C₁₋₅ alkyl), -(C₀₋₃ alkylène)-NH-CO-(C₁₋₅ alkyl), -(C₀₋₃ alkylène)-N(C₁₋₅ alkyl)-CO-(C₁₋₅ alkyl), -(C₀₋₃ alkylène)-NH-CO-O-(C₁₋₅ alkyl), -(C₀₋₃ alkylène)-N(C₁₋₅ alkyl)-CO-O-(C₁₋₅ alkyl), -(C₀₋₃ alkylène)-O-CO-NH-(C₁₋₅ alkyl), -(C₀₋₃ alkylène)-O-CO-N(C₁₋₅ alkyl)-(C₁₋₅ alkyl), -(C₀₋₃ alkylène)-SO₂-NH₂, -(C₀₋₃ alkylène)-SO₂-NH(C₁₋₅ alkyl), -(C₀₋₃ alkylène)-SO₂-N(C₁₋₅ alkyl)(C₁₋₅ alkyl), -(C₀₋₃ alkylène)-NH-SO₂-(C₁₋₅ alkyl), -(C₀₋₃ alkylène)-N(C₁₋₅ alkyl)-SO₂-(C₁₋₅ alkyl), -(C₀₋₃ alkylène)-SO-(C₁₋₅ alkyl), -(C₀₋₃ alkylène)-SO₂-(C₁₋₅ alkyl), -(C₀₋₃ alkylène)-carbocyclyle, et -(C₀₋₃ alkylène)-hétérocyclyle, où le motif carbocyclyle dudit -(C₀₋₃ alkylène)-carbocyclyle et le motif hétérocyclyle dudit -(C₀₋₃ alkylène)-hétérocyclyle sont chacun éventuellement substitués par un ou plusieurs groupements choisis indépendamment parmi C₁₋₅ alkyle, C₂₋₅ alcényle, C₂₋₅ alcynyle, -OH, -O(C₁₋₅ alkyl), -O(C₁₋₅ alkylène)-OH, -O(C₁₋₅ alkylène)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogène, C₁₋₅ halogénoalkyle, -O-(C₁₋₅ halogénoalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyle, et hétérocycloalkyle ;
R³ est choisi parmi hydrogène, C₁₋₅ alkyle, C₂₋₅ alcényle, C₂₋₅ alcynyle, -(C₁₋₅ alkylène)-OH, -(C₁₋₅ alkylène)-O(C₁₋₅ alkyl), -(C₁₋₅ alkylène)-O(C₁₋₅ alkylène)-OH, -(C₁₋₅ alkylène)-O(C₁₋₅ alkylène)-O(C₁₋₅ alkyl), -(C₁₋₅ alkylène)-SH, -(C₁₋₅ alkylène)-S(C₁₋₅ alkyl), -(C₁₋₅ alkylène)-NH₂, -(C₁₋₅ alkylène)-NH(C₁₋₅ alkyl), -(C₁₋₅ alkylène)-N(C₁₋₅ alkyl)(C₁₋₅ alkyl), -(C₁₋₅ alkylène)-halogène, C₁₋₅ halogénoalkyle, -(C₁₋₅ alkylène)-O-(C₁₋₅ halogénoalkyl), -(C₁₋₅ alkylène)-CF₃, -(C₁₋₅ alkylène)-CN, -(C₁₋₅ alkylène)-NO₂, -(C₁₋₅ alkylène)-Si(C₁₋₅ alkyl)₃, -(C₁₋₅ alkylène)-O(C₁₋₅ alkylène)-Si(C₁₋₅ alkyl)₃, -(C₁₋₅ alkylène)-CHO, -(C₁₋₅ alkylène)-CO-(C₁₋₅ alkyl), -(C₁₋₅ alkylène)-COOH, -(C₁₋₅ alkylène)-CO-O-(C₁₋₅ alkyl), -(C₁₋₅ alkylène)-O-CO-(C₁₋₅ alkyl), -(C₁₋₅ alkylène)-CO-NH₂, -(C₁₋₅ alkylène)-CO-NH(C₁₋₅ alkyl), -(C₁₋₅ alkylène)-CO-N(C₁₋₅ alkyl)(C₁₋₅ alkyl), -(C₁₋₅ alkylène)-NH-CO-(C₁₋₅ alkyl), -(C₁₋₅ alkylène)-N(C₁₋₅ alkyl)-CO-(C₁₋₅ alkyl), -(C₁₋₅ alkylène)-NH-CO-O-(C₁₋₅ alkyl), -(C₁₋₅ alkylène)-N(C₁₋₅ alkyl)-CO-O-(C₁₋₅ alkyl), -(C₁₋₅ alkylène)-O-CO-NH-(C₁₋₅ alkyl), -(C₁₋₅ alkylène)-O-CO-N(C₁₋₅ alkyl)-(C₁₋₅ alkyl), -(C₁₋₅ alkylène)-SO₂-NH₂, -(C₁₋₅ alkylène)-SO₂-NH(C₁₋₅ alkyl), -(C₁₋₅ alkylène)-SO₂-N(C₁₋₅ alkyl)(C₁₋₅ alkyl), -(C₁₋₅ alkylène)-NH-SO₂-(C₁₋₅ alkyl), -(C₁₋₅ alkylène)-N(C₁₋₅ alkyl)-SO₂-(C₁₋₅ alkyl), -(C₁₋₅ alkylène)-SO-(C₁₋₅ alkyl), -(C₁₋₅ alkylène)-SO₂-(C₁₋₅ alkyl), -(C₀₋₅ alkylène)-carbocyclyle, et -(C₀₋₅ alkylène)-hétérocyclyle, où le motif carbocyclyle dudit -(C₀₋₅ alkylène)-carbocyclyle et le motif hétérocyclyle dudit -(C₀₋₅ alkylène)-hétérocyclyle sont chacun éventuellement substitués par un ou plusieurs groupements choisis indépendamment parmi C₁₋₅ alkyle, C₂₋₅ alcényle, C₂₋₅ alcynyle, -OH, -O(C₁₋₅ alkyl), -O(C₁₋₅ alkylène)-OH, -O(C₁₋₅ alkylène)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogène, C₁₋₅ halogénoalkyle, -O-(C₁₋₅ halogénoalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyle, et hétérocycloalkyle ;
le cycle A est un hétéroaryle bicyclique, où ledit hétéroaryle bicyclique est éventuellement substitué par un ou plusieurs groupements R^{A} ; et
chaque R^{A} est choisi indépendamment parmi C₁₋₅ alkyle, C₂₋₅ alcényle, C₂₋₅ alcynyle, -(C₀₋₃ alkylène)-OH, -(C₀₋₃ alkylène)-O(C₁₋₅ alkyl), -(C₀₋₃ alkylène)-O(C₁₋₅ alkylène)-OH, -(C₀₋₃ alkylène)-O(C₁₋₅ alkylène)-O(C₁₋₅ alkyl), -(C₀₋₃ alkylène)-SH, -(C₀₋₃ alkylène)-S(C₁₋₅ alkyl), -(C₀₋₃ alkylène)-NH₂, -(C₀₋₃ alkylène)-NH(C₁₋₅ alkyl), -(C₀₋₃ alkylène)-N(C₁₋₅ alkyl)(C₁₋₅ alkyl), -(C₀₋₃ alkylène)-halogène, -(C₀₋₃ alkylène)-(C₁₋₅ halogénoalkyl), -(C₀₋₃ alkylène)-O-(C₁₋₅ halogénoalkyl), -(C₀₋₃ alkylène)-CF₃, -(C₀₋₃ alkylène)-CN, -(C₀₋₃ alkylène)-NO₂, -(C₀₋₃ alkylène)-CHO, -(C₀₋₃ alkylène)-CO-(C₁₋₅ alkyl), -(C₀₋₃ alkylène)-COOH, -(C₀₋₃ alkylène)-CO-O-(C₁₋₅ alkyl), -(C₀₋₃ alkylène)-O-CO-(C₁₋₅ alkyl), -(C₀₋₃ alkylène)-CO-NH₂, -(C₀₋₃ alkylène)-CO-NH(C₁₋₅ alkyl), -(C₀₋₃ alkylène)-CO-N(C₁₋₅ alkyl)(C₁₋₅ alkyl), -(C₀₋₃ alkylène)-NH-CO-(C₁₋₅ alkyl), -(C₀₋₃ alkylène)-N(C₁₋₅ alkyl)-CO-(C₁₋₅ alkyl), -(C₀₋₃ alkylène)-NH-CO-O-(C₁₋₅ alkyl), -(C₀₋₃ alkylène)-N(C₁₋₅ alkyl)-CO-O-(C₁₋₅ alkyl), -(C₀₋₃ alkylène)-O-CO-NH-(C₁₋₅ alkyl), -(C₀₋₃ alkylène)-O-CO-N(C₁₋₅ alkyl)-(C₁₋₅ alkyl), -(C₀₋₃ alkylène)-SOz-NH₂, -(C₀₋₃ alkylène)-SO₂-NH(C₁₋₅ alkyl), -(C₀₋₃ alkylène)-SO₂-N(C₁₋₅ alkyl)(C₁₋₅ alkyl), -(C₀₋₃ alkylène)-NH-SO₂-(C₁₋₅ alkyl), -(C₀₋₃ alkylène)-N(C₁₋₅ alkyl)-SO₂-(C₁₋₅ alkyl), -(C₀₋₃ alkylène)-SO-(C₁₋₅ alkyl), -(C₀₋₃ alkylène)-SO₂-(C₁₋₅ alkyl), -(C₀₋₃ alkylène)-carbocyclyle, et -(C₀₋₃ alkylène)-hétérocyclyle, où le motif carbocyclyle dudit -(C₀₋₃ alkylène)-carbocyclyle et le motif hétérocyclyle dudit -(C₀₋₃ alkylène)-hétérocyclyle sont chacun éventuellement substitués par un ou plusieurs groupements choisis indépendamment parmi C₁₋₅ alkyle, C₂₋₅ alcényle, C₂₋₅ alcynyle, -OH, -O(C₁₋₅ alkyl), -O(C₁₋₅ alkylène)-OH, -O(C₁₋₅ alkylène)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogène, C₁₋₅ halogénoalkyle, -O-(C₁₋₅ halogénoalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -NH-CO-O-(C₁₋₅ alkyl), -N(C₁₋₅ alkyl)-CO-O-(C₁₋₅ alkyl), -O-CO-NH-(C₁₋₅ alkyl), -O-CO-N(C₁₋₅ alkyl)-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), -SO₂-(C₁₋₅ alkyl), cycloalkyle, et hétérocycloalkyle ;
ou un sel ou solvate pharmaceutiquement acceptable de celui-ci.

2. Composé selon la revendication 1, dans lequel R^{1A} et R^{1B} sont liés mutuellement pour former, conjointement avec l'atome d'azote auquel ils sont liés, un hétérocycloalkyle qui est éventuellement substitué par un ou plusieurs groupements R¹¹.

3. Composé selon la revendication 1 ou 2, dans lequel R^{1A} et R^{1B} sont liés mutuellement pour former, conjointement avec l'atome d'azote auquel ils sont liés, un groupement qui est choisi parmi 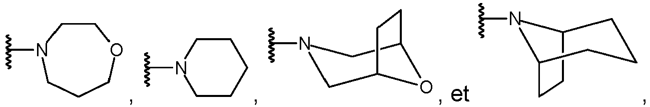 où chacun parmi les groupements illustrés ci-dessus éventuellement substitué par un ou plusieurs R¹¹.

4. Composé selon l'une quelconque des revendications 1 à 3, dans lequel R² et R⁴ sont chacun choisis indépendamment parmi hydrogène, C₁₋₅ alkyle, -OH, -O(C₁₋₅ alkyl), -O(C₁₋₅ alkylène)-OH, -O(C₁₋₅ alkylène)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogène, C₁₋₅ halogénoalkyle, -CF₃, et -CN.

5. Composé selon l'une quelconque des revendications 1 à 4, dans lequel R³ est choisi parmi C₁₋₅ halogénoalkyle, -(C₁₋₅ alkylène)-CN, -(C₁₋₅ alkylène)-SO-(C₁₋₅ alkyl), et -(C₁₋₅ alkylène)-SO₂-(C₁₋₅ alkyl).

6. Composé selon l'une quelconque des revendications 1 à 5, dans lequel R³ est -CH₂-CF₃ ou -CH₂-SO₂-CH₃.

7. Composé selon l'une quelconque des revendications 1 à 6, dans lequel le cycle A est un hétéroaryle bicyclique condensé, où l'un parmi les deux cycles condensé dudit hétéroaryle bicyclique condensé possède 5 atomes de cycle et l'autre cycle condensé possède 6 atomes de cycle, et en outre où ledit hétéroaryle bicyclique est éventuellement substitué par un ou plusieurs groupements R^{A}.

8. Composé selon l'une quelconque des revendications 1 à 7, dans lequel le cycle A est un hétéroaryle bicyclique répondant à la formule (II) suivante : 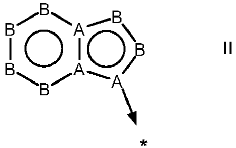 dans laquelle chacun parmi les deux cycles condensés dudit hétéroaryle bicyclique est aromatique, où chaque atome A de cycle de l'hétéroaryle bicyclique est indépendamment un atome de carbone ou d'azote, où chaque atome B de cycle est indépendamment un atome de carbone, d'azote, d'oxygène ou de soufre, où au moins l'un parmi les atomes A et/ou B de cycle est autre que le carbone, et où ledit hétéroaryle bicyclique est éventuellement substitué par un ou plusieurs groupements R^{A}.

9. Composé selon l'une quelconque des revendications 1 à 8, dans lequel le cycle A est choisi parmi l'un quelconque parmi les groupements suivants : 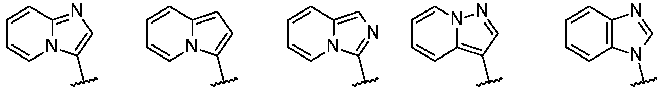 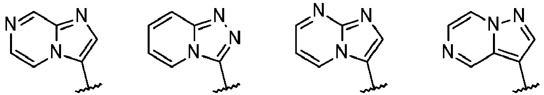 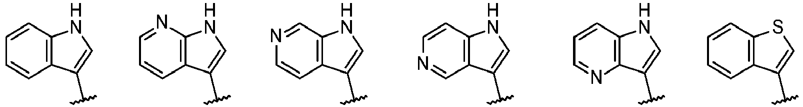 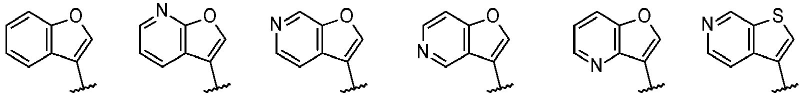
où chacun parmi les groupements illustrés ci-dessus est éventuellement substitué par un ou plusieurs groupements R^{A} ;
et préférablement où le cycle A est choisi parmi l'un quelconque parmi les groupements suivants : 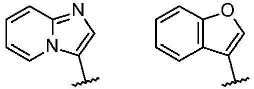 où chacun parmi les groupements illustrés ci-dessus est éventuellement substitué par un ou plusieurs groupements R^{A}.

10. Composé selon l'une quelconque des revendications 1 à 9, dans lequel chaque R^{A} est choisi indépendamment parmi C₁₋₅ alkyle, -OH, -O(C₁₋₅ alkyl), -O(C₁₋₅ alkylène)-OH, -O(C₁₋₅ alkylène)-O(C₁₋₅ alkyl), -SH, -S(C₁₋₅ alkyl), -NH₂, -NH(C₁₋₅ alkyl), -N(C₁₋₅ alkyl)(C₁₋₅ alkyl), halogène, C₁₋₅ halogénoalkyle, -CF₃, et -CN ; préférablement où chaque R^{A} est indépendamment halogène ; et plus préférablement où chaque R^{A} est -F.

11. Composé selon la revendication 1, ledit composé étant choisi parmi :
| | |
|---|---|
| 1 | [3-(1-Méthyl-1H-indol-3-yl)-1-(2,2,2-trifluoro-éthyl)-1H-pyrazolo-[4,3-c]pyridine-6-yl]-1,4-oxazépan-4-yl-méthanone |
| 2 | Azépan-1-yl-[3-(6-fluoro-imidazo[1,2-a]pyridin-3-yl)-1-(2-méthane-sulfonyl-éthyl)-1H-pyrazolo[4,3-c]pyridin-6-yl]-méthanone |
| 3 | Azépan-1-yl-[1-(2,2-difluoro-éthyl)-3-(6-fluoro-imidazo[1,2-a]-pyridin-3-yl)-1H-pyrazolo[4,3-c]pyridin-6-yl]-méthanone |
| 4 | Azépan-1-yl-[1-(2-fluoro-éthyl)-3-(6-fluoro-imidazo[1,2-a]pyridin-3-yl)-1H-pyrazolo[4,3-c]pyridin-6-yl]-méthanone |
| 5 | Azépan-1-yl-[3-(6-fluoro-imidazo[1,2-a]pyridin-3-yl)-1-propyl-1H-pyrazolo[4,3-c]pyridin-6-yl]-méthanone |
| 6 | Azépan-1-yl-[3-(6-fluoro-imidazo[1,2-a]pyridin-3-yl)-1-méthoxy-méthyl-1H-pyrazolo[4,3-c]pyridin-6-yl]-méthanone |
| 7 | [6-(Azépane-1-carbonyl)-3-(6-fluoro-imidazo[1,2-a]pyridin-3-yl)-pyrazolo[4,3-c]pyridin-1-yl]-acétonitrile |
| 8 | Azépan-1-yl-[3-(6,8-difluoro-imidazo[1,2-a]pyridin-3-yl)-1-(2-méthoxy-éthyl)-1H-pyrazolo[4,3-c]pyridin-6-yl]-méthanone |
| 9 | [1-Cyclopropylméthyl-3-(6,8-difluoro-imidazo[1,2-a]pyridin-3-yl)-1H-pyrazolo[4,3-c]pyridin-6-yl]-1,4-oxazépan-4-yl-méthanone |
| 10 | [3-(6,8-Difluoro-imidazo[1,2-a]pyridin-3-yl)-1-méthanesulfonyl-1H-pyrazolo[4,3-c]pyridin-6-yl]-1,4-oxazépan-4-yl-méthanone |
| 11 | Acide 3-[3-(6,8-difluoro-imidazo[1,2-a]pyridin-3-yl)-6-(1,4-oxazépane-4-carbonyl)-pyrazolo[4,3-c]pyridin-1-ylméthyl]-azétidine-1-carboxylique tertio-butyl ester |
| 12 | [3-(6,8-Difluoro-imidazo[1,2-a]pyridin-3-yl)-1-hydroxyméthyl-1H-pyrazolo[4,3-c]pyridin-6-yl]-1,4-oxazépan-4-yl-méthanone |
| 13 | [3-Benzofuran-3-yl-6-(1,4-oxazépane-4-carbonyl)-pyrazolo[4,3-c]-pyridin-1-yl]-acétonitrile |
| 14 | (3-Azabicyclo[3.2.1]oct-3-yl)-[3-(6,8-difluoro-imidazo[1,2-a]pyridin-3-yl)-1-méthylsulfanylméthyl-1H-pyrazolo[4,3-c]pyridin-6-yl]-méthanone |
| 15 | [3-(6,8-Difluoro-imidazo[1,2-a]pyridin-3-yl)-1-méthanesulfonyl-méthyl-1H-pyrazolo[4,3-c]pyridin-6-yl]-1,4-oxazépan-4-yl-méthanone |
| 16 | (3-Benzofuran-3-yl-1-méthanesulfonylméthyl-1H-pyrazolo[4,3-c]-pyridine-6-yl)-1,4-oxazépan-4-yl-méthanone |
| 17 | (1-Méthanesulfonylméthyl-3-pyrazolo[1,5-a]pyridin-3-yl-1H-pyrazolo[4,3-c]pyridin-6-yl)-1,4-oxazépan-4-yl-méthanone |
| 18 | (3-Azabicyclo[3.2.1]oct-3-yl)-(1-méthanesulfonylméthyl-3-pyrazolo[1,5-a]pyridin-3-yl-1H-pyrazolo[4,3-c]pyridin-6-yl)-méthanone |
| 19 | (3-Azabicyclo[3.2.1]oct-3-yl)-(3-benzofuran-3-yl-1-méthane-sulfonylméthyl-1 H-pyrazolo[4,3-c]pyridin-6-yl)-méthanone |
| 20 | Azépan-1-yl-[3-(6-fluoro-imidazo[1,2-a]pyridin-3-yl)-1-méthane-sulfonylméthyl-1H-pyrazolo[4,3-c]pyridin-6-yl]-méthanone |
| 21 | Azépan-1-yl-[3-(6-fluoro-imidazo[1,2-a]pyridin-3-yl)-1-méthane-sulfinylméthyl-1H-pyrazolo[4,3-c]pyridin-6-yl]-méthanone |
| 22 | [3-(6,8-Difluoro-imidazo[1,2-a]pyridin-3-yl)-1-éthanesulfonyl-méthyl-1 H-pyrazolo[4,3-c]pyridin-6-yl]-1,4-oxazépan-4-yl-méthanone |
| 23 | (3-Azabicyclo[3.2.1]oct-3-yl)-[3-(6,8-difluoro-imidazo[1,2-a]pyridin-3-yl)-1-méthanesulfonylméthyl-1H-pyrazolo[4,3-c]pyridin-6-yl]-méthanone |
| 24 | [3-(6,8-Difluoro-imidazo[1 ,2-a]pyridin-3-yl)-1-méthanesulfonyl-méthyl-1 H-pyrazolo[4,3-c]pyridin-6-yl]-(8-endo-hydroxy-8-exo-méthyl-3-azabicyclo[3.2.1]oct-3-yl)-méthanone |
| 25 | [3-(6,8-Difluoro-imidazo[1,2-a]pyridin-3-yl)-1-méthanesulfonyl-méthyl-1H-pyrazolo[4,3-c]pyridin-6-yl]-(3-endo-hydroxy-8-azabicyclo[3.2.1]oct-8-yl)-méthanone |
| 26 | [3-(6,8-Difluoro-imidazo[1,2-a]pyridin-3-yl)-1-méthanesulfonyl-méthyl-1H-pyrazolo[4,3-c]pyridin-6-yl]-(2-oxa-5-azabicyclo[2.2.1]-hept-5-yl)-méthanone |
| 27 | [3-(6,8-Difluoro-imidazo[1,2-a]pyridin-3-yl)-1-méthanesulfonyl-méthyl-1 H-pyrazolo[4,3-c]pyridin-6-yl]-(8-oxa-3-azabicyclo[3.2.1]-oct-3-yl)-méthanone |
| 28 | (3-Benzofuran-3-yl-1-méthanesulfonylméthyl-1H-pyrazolo[4,3-c]-pyridin-6-yl)-(8-endo-hydroxy-8-exo-méthyl-d3-3-azabicyclo-[3.2.1]oct-3-yl)-méthanone |
| 29 | (3-Benzofuran-3-yl-1-méthanesulfonylméthyl-1H-pyrazolo[4,3-c]-pyridin-6-yl)-(6,6-difluoro-1,4-oxazépan-4-yl)-méthanone |
| 30 | (3-Benzofuran-3-yl-1-méthanesulfonylméthyl-1H-pyrazolo[4,3-c]-pyridin-6-yl)-(8-endo-hydroxy-3-azabicyclo[3.2.1]oct-3-yl)-méthanone |
| 31 | (3-Benzofuran-3-yl-1-méthanesulfonylméthyl-1H-pyrazolo[4,3-c]-pyridin-6-yl)-(5-endo-hydroxy-5-exo-méthyl-2-azabicyclo[2.2.1]-hept-2-yl)-méthanone |
| 32 | (3-Benzofuran-3-yl-1-méthanesulfonylméthyl-1H-pyrazolo[4,3-c]-pyridin-6-yl)-(4-hydroxy-4-méthyl-pipéridin-1-yl)-méthanone |
| 33 | (3-Benzofuran-3-yl-1-méthanesulfonylméthyl-1H-pyrazolo[4,3-c]-pyridin-6-yl)-(4,4-difluoro-pipéridin-1-yl)-méthanone |
| 34 | (3-Benzofuran-3-yl-1-méthanesulfonylméthyl-1H-pyrazolo[4,3-c]-pyridin-6-yl)-(2-méthoxyméthyl-pyrrolidin-1-yl)-méthanone |
| 35 | (3-Benzofuran-3-yl-1-méthanesulfonylméthyl-1H-pyrazolo[4,3-c]-pyridin-6-yl)-(4-hydroxy-2,2 diméthyl-pipéridin-1-yl)-méthanone |
| 36 | (3-Benzofuran-3-yl-1-méthanesulfonylméthyl-1H-pyrazolo[4,3-c]-pyridin-6-yl)-(4-hydroxy-pipéridin-1-yl)-méthanone |
| 37 | [3-(Benzofuran-3-yl)-1-(méthylsulfonylméthyl)pyrazolo[4,3-c]-pyridin-6-yl]-(3-endo-hydroxy-8-azabicyclo[3.2.1]octan-8-yl)méthanone |
| 38 | (3-Benzofuran-3-yl-1-méthanesulfonylméthyl-1H-pyrazolo[4,3-c]-pyridin-6-yl)-(3-hydroxy-pipéridin-1-yl)-méthanone |
| 39 | (3-Benzofuran-3-yl-1-méthanesulfonylméthyl-1H-pyrazolo[4,3-c]-pyridin-6-yl)-(3,3-difluoro-pipéridin-1-yl)-méthanone |
| 40 | (3-Benzofuran-3-yl-1-méthanesulfonylméthyl-1H-pyrazolo[4,3-c]-pyridin-6-yl)-(4-hydroxyazépan-1-yl)-méthanone |
| 41 | Azépan-1-yl-[3-(6-fluoro-imidazo[1,2-a]pyridin-3-yl)-1-méthyl-1H-pyrazolo[4,3-c]pyridin-6-yl]-méthanone |
| 42 | 3-[6-(Azépane-1-carbonyl)-1-méthyl-1H-pyrazolo[4,3-c]pyridin-3-yl]-imidazo[1,2-a]pyridine-6-carbonitrile |
| 43 | 3-[6-(Azépane-1-carbonyl)-1-propyl-1H-pyrazolo[4,3-c]pyridin-3-yl]-imidazo[1,2-a]pyridine-6-carbonitrile |
| 44 | Azépan-1-yl-[3-(6-fluoro-imidazo[1,2-a]pyridin-3-yl)-1-(2,2,2-trifluoro-éthyl)-1H-pyrazolo[4,3-c]pyridin-6-yl]-méthanone |
| 45 | 3-[6-(Azépane-1-carbonyl)-1-(2,2,2-trifluoro-éthyl)-1H-pyrazolo-[4,3-c]pyridin-3-yl]-imidazo[1,2-a]pyridine-6-carbonitrile |
| 46 | Acide 3-[6-(azépane-1-carbonyl)-1-(2,2,2-trifluoro-éthyl)-1H-pyrazolo[4,3-c]-pyridin-3-yl]-imidazo[1,2-a]pyridine-6-carboxylique amide |
| 47 | 3-[6-(Azépane-1-carbonyl)-1-(2,2,2-trifluoro-éthyl)-1H-pyrazolo-[4,3-c]pyridin-3-yl]-8-méthyl-imidazo[1,2-a]pyridine-6-carbonitrile |
| 48 | Azépan-1-yl-[3-(6-chloro-imidazo[1,2-a]pyridin-3-yl)-1-(2,2,2-trifluoro-éthyl)-1H-pyrazolo[4,3-c]pyridin-6-yl]-méthanone |
| 49 | Acide 3-[6-(azépane-1-carbonyl)-1-(2,2,2-trifluoro-éthyl)-1H-pyrazolo[4,3-c]pyridin-3-yl]-imidazo[1,2-a]pyridine-6-carboxylique méthylamide |
| 50 | Azépan-1-yl-[3-(6-fluoro-imidazo[1,2-a]pyrimidin-3-yl)-1-(2,2,2-trifluoro-éthyl)-1H-pyrazolo[4,3-c]pyridin-6-yl]-méthanone |
| 51 | Azépan-1-yl-[3-(6-méthoxyméthyl-imidazo[1,2-a]pyridin-3-yl)-1-(2,2,2-trifluoro-éthyl)-1H-pyrazolo[4,3-c]pyridin-6-yl]-méthanone |
| 52 | Azépan-1-yl-[3-indolizin-3-yl-1-(2,2,2-trifluoro-éthyl)-1H-pyrazolo-[4,3-c]pyridin-6-yl]-méthanone |
| 53 | Acide 3-[6-(azépane-1-carbonyl)-1-(2,2,2-trifluoro-éthyl)-1H-pyrazolo[4,3-c]pyridin-3-yl]-imidazo[1,2-a]pyridine-6-carboxylique diméthylamide |
| 54 | Azépan-1-yl-[3-imidazo[1,5-a]pyridin-3-yl-1-(2,2,2-trifluoro-éthyl)-1H-pyrazolo[4,3-c]pyridin-6-yl]-méthanone |
| 55 | Azépan-1-yl-[3-imidazo[1,2-a]pyridin-3-yl-1-(2,2,2-trifluoro-éthyl)-1H-pyrazolo[4,3-c]pyridin-6-yl]-méthanone |
| 56 | Azépan-1-yl-[3-imidazo[1,2-a]pyrazin-3-yl-1-(2,2,2-trifluoro-éthyl)-1H-pyrazolo[4,3-c]pyridin-6-yl]-méthanone |
| 57 | Acide 3-[6-(azépane-1-carbonyl)-1-(2,2,2-trifluoro-éthyl)-1H-pyrazolo[4,3-c]pyridin-3-yl]-7-méthyl-imidazo[1,2-a]pyridine-6-carboxylique amide |
| 58 | Azépan-1-yl-[1-(2,2,2-trifluoro-éthyl)-3-(6-trifluorométhyl-imidazo-[1,2-a]pyridin-3-yl)-1H-pyrazolo[4,3-c]pyridin-6-yl]-méthanone |
| 59 | Azépan-1-yl-[3-(6-méthoxy-imidazo[1,2-a]pyridin-3-yl)-1-(2,2,2-trifluoro-éthyl)-1H-pyrazolo[4,3-c]pyridin-6-yl]-méthanone |
| 60 | Azépan-1-yl-[3-(6,8-difluoro-imidazo[1,2-a]pyridin-3-yl)-1-(2,2,2-trifluoro-éthyl)-1H-pyrazolo[4,3-c]pyridin-6-yl]-méthanone |
| 61 | Azépan-1-yl-[3-(6-méthyl-imidazo[1,2-a]pyridin-3-yl)-1-(2,2,2-trifluoro-éthyl)-1H-pyrazolo[4,3-c]pyridin-6-yl]-méthanone |
| 62 | Azépan-1-yl-[3-(8-fluoro-imidazo[1,2-a]pyridin-3-yl)-1-(2,2,2-trifluoro-éthyl)-1H-pyrazolo[4,3-c]pyridin-6-yl]-méthanone |
| 63 | 3-[6-(Azépane-1-carbonyl)-1-(2,2,2-trifluoro-éthyl)-1H-pyrazolo-[4,3-c]pyridin-3-yl]-7-hydroxy-imidazo[1,2-a]pyridine-6-carbonitrile |
| 64 | Azépan-1-yl-[3-[6-(1-hydroxy-éthyl)-imidazo[1,2-a]pyridin-3-yl]-1-(2,2,2-trifluoro-éthyl)-1H-pyrazolo[4,3-c]pyridin-6-yl]-méthanone |
| 65 | Azépan-1-yl-[3-(6-cyclopropyl-imidazo[1,2-a]pyridin-3-yl)-1-(2,2,2-trifluoro-éthyl)-1H-pyrazolo[4,3-c]pyridin-6-yl]-méthanone |
| 66 | Azépan-1-yl-[1-(2,2,2-trifluoro-éthyl)-3-(6-trifluorométhoxy-imidazo[1,2-a]pyridin-3-yl)-1H-pyrazolo[4,3-c]pyridin-6-yl]-méthanone |
| 67 | Azépan-1-yl-[3-(7-fluoro-imidazo[1,2-a]pyridin-3-yl)-1-(2,2,2-trifluoro-éthyl)-1H-pyrazolo[4,3-c]pyridin-6-yl]-méthanone |
| 68 | Azépan-1-yl-[3-(6-hydroxy-imidazo[1,2-a]pyridin-3-yl)-1-(2,2,2-trifluoro-éthyl)-1H-pyrazolo[4,3-c]pyridin-6-yl]-méthanone |
| 69 | Azépan-1-yl-[3-(6-difluorométhyl-imidazo[1,2-a]pyridin-3-yl)-1-(2,2,2-trifluoro-éthyl)-1H-pyrazolo[4,3-c]pyridin-6-yl]-méthanone |
| 70 | Azépan-1-yl-[3-[6-(2,2,2-trifluoro-éthoxy)-imidazo[1,2-a]pyridin-3-yl]-1-(2,2,2-trifluoro-éthyl)-1H-pyrazolo[4,3-c]pyridin-6-yl]-méthanone |
| 71 | Azépan-1-yl-[3-[1,2,4]triazolo[4,3-a]pyridin-3-yl-1-(2,2,2-trifluoro-éthyl)-1H-pyrazolo[4,3-c]pyridin-6-yl]-méthanone |
| 72 | Azépan-1-yl-[3-[6-(1-hydroxy-1-méthyl-éthyl)-imidazo[1,2-a]-pyridin-3-yl]-1-(2,2,2-trifluoro-éthyl)-1H-pyrazolo[4,3-c]pyridin-6-yl]-méthanone |
| 73 | Azépan-1-yl-[3-(7-chloro-6-fluoro-imidazo[1,2-a]pyridin-3-yl)-1-(2,2,2-trifluoro-éthyl)-1H-pyrazolo[4,3-c]pyridin-6-yl]-méthanone |
| 74 | Azépan-1-yl-[3-(6-chloro-7-fluoro-imidazo[1,2-a]pyridin-3-yl)-1-(2,2,2-trifluoro-éthyl)-1H-pyrazolo[4,3-c]pyridin-6-yl]-méthanone |
| 75 | Azépan-1-yl-[3-(6-chloro-7-hydroxy-imidazo[1,2-a]pyridin-3-yl)-1-(2,2,2-trifluoro-éthyl)-1H-pyrazolo[4,3-c]pyridin-6-yl]-méthanone |
| 76 | Azépan-1-yl-[3-(6,8-difluoro-imidazo[1,2-a]pyridin-3-yl)-1-(2-triméthylsilanyl-éthoxyméthyl)-1H-pyrazolo[4,3-c]pyridin-6-yl]-méthanone |
| 77 | Azépan-1-yl-[3-(6-fluoro-imidazo[1,2-a]pyridin-3-yl)-1-(2-triméthylsilanyl-éthoxyméthyl)-1H-pyrazolo[4,3-c]pyridin-6-yl]-méthanone |
| 78 | Azépan-1-yl-[3-(6,8-difluoro-imidazo[1,2-a]pyridin-3-yl)-1-(4-méthoxy-benzyl)-1H-pyrazolo[4,3-c]pyridin-6-yl]-méthanone |
| 79 | Azépan-1-yl-[3-(6,8-difluoro-imidazo[1,2-a]pyridin-3-yl)-1-(tétrahydro-pyran-2-yl)-1H-pyrazolo[4,3-c]pyridin-6-yl]-méthanone |
| 80 | [3-(6-Fluoro-imidazo[1,2-a]pyridin-3-yl)-1-(2,2,2-trifluoro-éthyl)-1H-pyrazolo[4,3-c]pyridin-6-yl]-1,4-oxazépan-4-yl-méthanone |
| 81 | 3-[6-(1,4-Oxazépane-4-carbonyl)-1-(2,2,2-trifluoro-éthyl)-1H-pyrazolo[4,3-c]pyridin-3-yl]-imidazo[1,2-a]pyridine-6-carbonitrile |
| 82 | [3-(6-Fluoro-imidazo[1,2-a]pyridin-3-yl)-1-(2,2,2-trifluoro-éthyl)-1H-pyrazolo[4,3-c]pyridin-6-yl]-(8-oxa-3-azabicyclo[3.2.1]oct-3-yl)-méthanone |
| 83 | [3-(6,8-Difluoro-imidazo[1,2-a]pyridin-3-yl)-1-(2,2,2-trifluoro-éthyl)-1H-pyrazolo[4,3c]pyridin-6-yl]-(8-oxa-3-azabicyclo[3.2.1]oct-3-yl)-méthanone |
| 84 | 3-[6-(8-Oxa-3-azabicyclo[3.2.1]octane-3-carbonyl)-1-(2,2,2-trifluoro-éthyl)-1H-pyrazolo[4,3-c]pyridin-3-yl]-imidazo[1,2-a]-pyridine-6-carbonitrile |
| 85 | [3-(8-fluoroimidazo[1,2-a]pyridin-3-yl)-1-(2,2,2-trifluoroéthyl)-pyrazolo[4,3-c]pyridin-6-yl]-(3-endo-hydroxy-8-azabicyclo[3.2.1]-octan-8-yl)méthanone |
| 86 | [3-(6-Fluoroimidazo[1,2-a]pyridin-3-yl)-1-(2,2,2-trifluoroéthyl)-pyrazolo[4,3-c]pyridin-6-yl]-(3-endo-hydroxy-8-azabicyclo[3.2.1]-octan-8-yl)méthanone |
| 87 | [3-(Imidazo[1,2-a]pyridin-3-yl)-1-(2,2,2-trifluoroéthyl)pyrazolo[4,3-c]pyridin-6-yl]-(3-endo-hydroxy-8-azabicyclo[3.2.1]octan-8-yl)-méthanone |
| 88 | (3-Endo-hydroxy-8-azabicyclo[3.2.1]octan-8-yl)-[3-(6-hydroxy-imidazo[1,2-a]pyridin-3-yl)-1-(2,2,2-trifluoroéthyl)pyrazolo[4,3-c]pyridin-6-yl]méthanone |
| 89 | Chlorhydrate d'azépan-1-yl-[3-pyrazolo[1,5-a]pyridin-3-yl-1-(2,2,2-trifluoro-éthyl)-1H-pyrazolo[4,3-c]pyridin-6-yl]-méthanone |
| 90 | Azépan-1-yl-[3-benzofuran-3-yl-1-(2,2,2-trifluoro-éthyl)-1H-pyrazolo[4,3-c]pyridin-6-yl]-méthanone |
| 91 | Azépan-1-yl-[3-(1H-indol-3-yl)-1-(2,2,2-trifluoro-éthyl)-1H-pyrazolo[4,3-c]pyridin-6-yl]-méthanone |
| 92 | Azépan-1-yl-[3-(1H-pyrrolo[2,3-b]pyridin-3-yl)-1-(2,2,2-trifluoro-éthyl)-1H-pyrazolo[4,3-c]pyridin-6-yl]-méthanone |
| 93 | 1,4-Oxazépan-4-yl-[3-thiéno[2,3-c]pyridin-3-yl-1-(2,2,2-trifluoro-éthyl)-1H-pyrazolo[4,3-c]pyridin-6-yl]-méthanone |
| 94 | [3-Benzofuran-3-yl-1-(2,2,2-trifluoro-éthyl)-1 H-pyrazolo[4,3-c]-pyridin-6-yl]-1,4-oxazépan-4-yl-méthanone |
| 95 | 1,4-Oxazépan-4-yl-[3-pyrazolo[1,5-a]pyridine-3-yl-1-(2,2,2-trifluoro-éthyl)-1H-pyrazolo[4,3-c]pyridin-6-yl]-méthanone |
| 96 | [3-(1H-Indol-3-yl)-1-(2,2,2-trifluoro-éthyl)-1H-pyrazolo[4,3-c]-pyridin-6-yl]-1,4-oxazépan-4-yl-méthanone |
| 97 | 1,4-Oxazépan-4-yl-[3-(1H-pyrrolo[2,3-b]pyridin-3-yl)-1-(2,2,2-trifluoro-éthyl)-1H-pyrazolo[4,3-c]pyridin-6-yl]-méthanone |
| 98 | [3-Benzo[b]thiophén-3-yl-1-(2,2,2-trifluoro-éthyl)-1H-pyrazolo[4,3-c]pyridin-6-yl]-1,4-oxazépan-4-yl-méthanone |
| 99 | (8-Oxa-3-azabicyclo[3.2.1]oct-3-yl)-[3-pyrazolo[1 ,5-a]pyridin-3-yl-1-(2,2,2-trifluoro-éthyl)-1H-pyrazolo[4,3-c]pyridin-6-yl]-méthanone |
| 100 | [3-Benzofuran-3-yl-1-(2,2,2-trifluoro-éthyl)-1H-pyrazolo[4,3-c]-pyridin-6-yl]-(8-oxa-3-azabicyclo[3.2.1]oct-3-yl)-méthanone |
| 101 | (3-Azabicyclo[3.2.1]oct-3-yl)-[3-benzofuran-3-yl-1-(2,2,2-trifluoro-éthyl)-1H-pyrazolo[4,3-c]pyridin-6-yl]-méthanone |
| 102 | (3-Azabicyclo[3.2.1]oct-3-yl)-[3-pyrazolo[1,5-a]pyridin-3-yl-1-(2,2,2-trifluoro-éthyl)-1H-pyrazolo[4,3-c]pyridin-6-yl]-méthanone |
| 103 | [3-Benzofuran-3-yl-1-(2,2-difluoro-éthyl)-1H-pyrazolo[4,3-c]-pyridin-6-yl]-1,4-oxazépan-4-yl-méthanone |
| 104 | [3-Benzofuran-3-yl-1-(2-fluoro-éthyl)-1H-pyrazolo[4,3-c]pyridin-6-yl]-1,4-oxazépan-4-yl-méthanone |
| 105 | 2-[3-Benzofuran-3-yl-6-(1,4-oxazépane-4-carbonyl)-pyrazolo[4,3-c]pyridin-1-yl]-acétamide |
| 106 | (3-Benzofuran-3-yl-1-propyl-1H-pyrazolo[4,3-c]pyridin-6-yl)-1,4-oxazépan-4-yl-méthanone |
| 107 | (3-Benzofuran-3-yl-1-méthanesulfonylméthyl-1H-pyrazolo[4,3-c]-pyridin-6-yl)-(8-oxa-3-azabicyclo[3.2.1]oct-3-yl)-méthanone |
| 108 | (1-Méthanesulfonylméthyl-3-pyrazolo[1,5-a]pyridin-3-yl-1H-pyrazolo[4,3-c]pyridin-6-yl)-(8-oxa-3-azabicyclo[3.2.1]oct-3-yl)-méthanone |
| 109 | [3-(5-Fluorobenzofuran-3-yl)-1-(2,2,2-trifluoroéthyl)pyrazolo[4,3-c]pyridin-6-yl]-(1,4-oxazépan-4-yl)méthanone |
| 110 | [3-(5-Fluorobenzofuran-3-yl)-1-(méthylsulfonylméthyl)pyrazolo-[4,3-c]pyridin-6-yl]-(1,4-oxazépan-4-yl)méthanone |
| 111 | [3-(5-Fluorobenzofuran-3-yl)-1-(méthylsulfonylméthyl)pyrazolo-[4,3-c]pyridin-6-yl]-(8-oxa-3-azabicyclo[3.2.1]octan-3-yl)-méthanone |
| 112 | (3-Endo-hydroxy-8-azabicyclo[3.2.1]octan-8-yl)-[3-(1H-indol-3-yl)-1-(2,2,2-trifluoroéthyl)pyrazolo[4,3-c]pyridin-6-yl]méthanone |
| 113 | (3-Endo-hydroxy-8-azabicyclo[3.2.1]octan-8-yl)-[3-pyrazolo[1,5-a]pyridin-3-yl-1-(2,2,2-trifluoroéthyl)pyrazolo[4,3-c]pyridin-6-yl]-méthanone |
| 114 | (4-hydroxy-1-pipéridyl)-[3-pyrazolo[1,5-a]pyridin-3-yl-1-(2,2,2-trifluoroéthyl)pyrazolo[4,3-c]pyridin-6-yl]méthanone |
| 115 | [3-(5-Fluorobenzofuran-3-yl)-1-(2,2,2-trifluoroéthyl)pyrazolo[4,3-c]pyridin-6-yl]-(4-hydroxy-1-pipéridyl)méthanone |
| 116 | [3-(5-Fluorobenzofuran-3-yl)-1-(2,2,2-trifluoroéthyl)pyrazolo[4,3-c]pyridin-6-yl]-(4-hydroxy-4-méthyl-1-pipéridyl)méthanone |
| 117 | (4-hydroxy-4-méthyl-1-pipéridyl)-[3-pyrazolo[1,5-a]pyridin-3-yl-1-(2,2,2-trifluoroéthyl)pyrazolo[4,3-c]pyridin-6-yl]méthanone |
| 118 | [3-(5-Fluorobenzofuran-3-yl)-1-(2,2,2-trifluoroéthyl)pyrazolo[4,3-c]pyridin-6-yl]-(6-hydroxy-1,4-oxazépan-4-yl)méthanone |
| 119 | (6-hydroxy-1,4-oxazépan-4-yl)-[3-pyrazolo[1,5-a]pyridin-3-yl-1-(2,2,2-trifluoroéthyl)pyrazolo[4,3-c]pyridin-6-yl]méthanone |
| 120 | [3-(5-Fluorobenzofuran-3-yl)-1-(2,2,2-trifluoroéthyl)pyrazolo[4,3-c]pyridin-6-yl]-(6,6-dideutério-1,4-oxazépan-4-yl)méthanone |
| 121 | (6,6-dideutério-1,4-oxazépan-4-yl)-[3-pyrazolo[1,5-a]pyridin-3-yl-1-(2,2,2-trifluoroéthyl)pyrazolo[4,3-c]pyridin-6-yl]méthanone |
| 122 | [3-(5-Fluorobenzofuran-3-yl)-1-(2,2,2-trifluoroéthyl)pyrazolo[4,3-c]pyridin-6-yl]-(4-hydroxyazépan-1-yl)méthanone |
| 123 | [3-(5-Fluoro-1H-indol-3-yl)-1-(2,2,2-trifluoroéthyl)pyrazolo[4,3-c]-pyridin-6-yl]-(1,4-oxazépan-4-yl)méthanone |
| 124 | 3-[6-(1,4-Oxazépane-4-carbonyl)-1-(2,2,2-trifluoroéthyl)pyrazolo-[4,3-c]pyridin-3-yl]benzofuran-5-carboxylate de méthyle |
| 125 | [3-(5-Méthylbenzofuran-3-yl)-1-(2,2,2-trifluoroéthyl)pyrazolo[4,3-c]pyridin-6-yl]-(1,4-oxazépan-4-yl)méthanone |
| 126 | [3-(2-Méthylbenzofuran-3-yl)-1-(2,2,2-trifluoroéthyl)pyrazolo[4,3-c]pyridin-6-yl]-(1,4-oxazépan-4-yl)méthanone |
| 127 | [3-(5-Fluorobenzofuran-3-yl)-1-(2,2,2-trifluoroéthyl)pyrazolo[4,3-c]pyridin-6-yl]-(8-oxa-3-azabicyclo[3.2.1]octan-3-yl)méthanone |
| 128 | Acide 3-(6,8-difluoro-imidazo[1,2-a]pyridin-3-yl)-1-(2,2,2-trifluoro-éthyl)-1H-pyrazolo[4,3-c]pyridine-6-carboxylique amide |
| 129 | Acide 3-[6-(1,4-oxazépane-4-carbonyl)-1-(2,2,2-trifluoroéthyl)-pyrazolo[4,3-c]pyridin-3-yl]benzofuran-5-carboxylique |
| 130 | Acide 1-[3-(benzofuran-3-yl)-1-(2,2,2-trifluoroéthyl)pyrazolo[4,3-c]pyridine-6-carbonyl]pipéridine-4-carboxylique |
| 131 | [3-(6-Fluoro-imidazo[1,2-a]pyridin-3-yl)-1-(2,2,2-trifluoro-éthyl)-1H-pyrazolo[4,3-c]pyridin-6-yl]-(4-hydroxy-pipéridin-1-yl)-méthanone |
| 132 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-pipéridin-1-yl)-méthanone |
| 133 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-4-méthyl-pipéridin-1-yl)-méthanone |
| 134 | [3-(6,8-Difluoro-imidazo[1,2-a]pyridin-3-yl)-1-(2,2,2-trifluoro-éthyl)-1H-pyrazolo[4,3-c]pyridin-6-yl]-(3-hydroxy-azépan-1-yl)-méthanone |
| 135 | [3-(6,8-Difluoro-imidazo[1,2-a]pyridin-3-yl)-1-(2,2,2-trifluoro-éthyl)-1H-pyrazolo[4,3-c]pyridin-6-yl]-(2-oxa-5-azabicyclo[2.2.1]hept-5-yl)-méthanone |
| 136 | [3-(6,8-Difluoro-[1,2,4]triazolo[4,3-a]pyridin-3-yl)-1-(2,2,2-trifluoro-éthyl)-1H-pyrazolo[4,3-c]pyridin-6-yl]-(3-hydroxy-8-azabicyclo-[3.2.1]oct-8-yl)-méthanone |
| 137 | [3-(6,8-Difluoro-[1,2,4]triazolo[4,3-a]pyridin-3-yl)-1-(2,2,2-trifluoro-éthyl)-1H-pyrazolo[4,3-c]pyridin-6-yl]-1,4-oxazépan-4-yl-méthanone |
| 138 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-2-méthyl-pipéridin-1-yl)-méthanone |
| 139 | (3-Azabicyclo[3.2.1]oct-3-yl)-[3-(6,8-difluoro-imidazo[1,2-a]pyridin-3-yl)-1-(2,2,2-trifluoro-éthyl)-1H-pyrazolo[4,3-c]pyridin-6-yl]-méthanone |
| 140 | [3-(6,8-Difluoro-imidazo[1,2-a]pyridin-3-yl)-1-(2,2,2-trifluoro-éthyl)-1H-pyrazolo[4,3-c]pyridin-6-yl]-(9-hydroxy-3-azabicyclo[3.3.1]non-3-yl)-méthanone |
| 141 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-2-isopropyl-pipéridin-1-yl)-méthanone |
| 142 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-2,2-diméthyl-pipéridin-1-yl)-méthanone |
| 143 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-3-isobutyl-pipéridin-1-yl)-méthanone |
| 144 | [3-(6,8-Difluoro-imidazo[1,2-a]pyridin-3-yl)-1-(2,2,2-trifluoro-éthyl)-1H-pyrazolo[4,3-c]pyridin-6-yl]-(4-fluoro-pipéridin-1-yl)-méthanone |
| 145 | [3-(6,8-Difluoro-imidazo[1,2-a]pyridin-3-yl)-1-(2,2,2-trifluoro-éthyl)-1H-pyrazolo[4,3-c]pyridin-6-yl]-(8-oxa-2-aza-spiro[4.5]déc-2-yl)-méthanone |
| 146 | [3-(6,8-Difluoro-imidazo[1,2-a]pyridin-3-yl)-1-(2,2,2-trifluoro-éthyl)-1H-pyrazolo[4,3-c]pyridin-6-yl]-(4,4-difluoro-pipéridin-1-yl)-méthanone |
| 147 | [3-(6,8-Difluoro-imidazo[1,2-a]pyridin-3-yl)-1-(2,2,2-trifluoro-éthyl)-1H-pyrazolo[4,3-c]pyridin-6-yl]-(4'-hydroxy-3',4',5',6'-tétrahydro-2'H-[3,4']bipyridinyl-1'-yl)-méthanone |
| 148 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-4-trifluorométhyl-pipéridin-1-yl)-méthanone |
| 149 | [3-(6,8-Difluoro-imidazo[1,2-a]pyridin-3-yl)-1-(2,2,2-trifluoro-éthyl)-1H-pyrazolo[4,3-c]pyridin-6-yl]-(2-méthoxyméthyl-pyrrolidin-1-yl)-méthanone |
| 150 | [3-(6,8-Difluoro-imidazo[1,2-a]pyridin-3-yl)-1-(2,2,2-trifluoro-éthyl)-1H-pyrazolo[4,3-c]pyridin-6-yl]-(4-méthyl-1 ,4-diazépan-1-yl)-méthanone |
| 151 | [3-(6,8-Difluoro-imidazo[1,2-a]pyridin-3-yl)-1-(2,2,2-trifluoro-éthyl)-1H-pyrazolo[4,3-c]pyridin-6-yl]-(3-hydroxy-pipéridin-1-yl)-méthanone |
| 152 | [3-(6,8-Difluoro-imidazo[1,2-a]pyridin-3-yl)-1-(2,2,2-trifluoro-éthyl)-1H-pyrazolo[4,3-c]pyridin-6-yl]-(7-oxa-2-aza-spiro[4.5]déc-2-yl)-méthanone |
| 153 | [3-(6,8-Difluoro-imidazo[1,2-a]pyridin-3-yl)-1-(2,2,2-trifluoro-éthyl)-1H-pyrazolo[4,3-c]pyridin-6-yl]-(3-oxa-9-aza-spiro[5.5]undéc-9-yl)-méthanone |
| 154 | [3-(6,8-Difluoro-imidazo[1,2-a]pyridin-3-yl)-1-(2,2,2-trifluoro-éthyl)-1H-pyrazolo[4,3-c]pyridin-6-yl]-(tétrahydro-furo[3,4-c]pyrrol-5-yl)-méthanone |
| 155 | 8-[3-(6,8-Difluoro-imidazo[1,2-a]pyridin-3-yl)-1-(2,2,2-trifluoro-éthyl)-1H-pyrazolo[4,3-c]pyridine-6-carbonyl]-2,8-diazaspiro[4.5]-décan-3-one |
| 156 | 8-[3-(6,8-Difluoro-imidazo[1,2-a]pyridin-3-yl)-1-(2,2,2-trifluoro-éthyl)-1H-pyrazolo[4,3-c]pyridine-6-carbonyl]-2,8-diazaspiro[4.5]-décan-1-one |
| 157 | [3-(6,8-Difluoro-imidazo[1,2-a]pyridin-3-yl)-1-(2,2,2-trifluoro-éthyl)-1H-pyrazolo[4,3-c]pyridin-6-yl]-[3-(2-méthoxy-éthyl)-pyrrolidin-1-yl]-méthanone |
| 158 | [3-(6,8-Difluoro-imidazo[1,2-a]pyridin-3-yl)-1-(2,2,2-trifluoro-éthyl)-1H-pyrazolo[4,3-c]pyridin-6-yl]-morpholin-4-yl-méthanone |
| 159 | [3-(6,8-Difluoro-imidazo[1,2-a]pyridin-3-yl)-1-(2,2,2-trifluoro-éthyl)-1H-pyrazolo[4,3-c]pyridin-6-yl]-(3,3-difluoro-pipéridin-1-yl)-méthanone |
| 160 | [3-(6,8-Difluoro-imidazo[1,2-a]pyridin-3-yl)-1-(2,2,2-trifluoro-éthyl)-1H-pyrazolo[4,3-c]pyridin-6-yl]-(3,3-difluoro-pipéridin-1-yl)-méthanone |
| 161 | [3-(6,8-Difluoro-imidazo[1,2-a]pyridin-3-yl)-1-(2,2,2-trifluoro-éthyl)-1H-pyrazolo[4,3-c]pyridin-6-yl]-(3-hydroxyméthyl-pipéridin-1-yl)-méthanone |
| 162 | [3-(6,8-Difluoro-imidazo[1,2-a]pyridin-3-yl)-1-(2,2,2-trifluoro-éthyl)-1H-pyrazolo[4,3-c]pyridin-6-yl]-(8-endo-hydroxy-8-exo-méthyl-3-azabicyclo[3.2.1]oct-3-yl)-méthanone |
| 163 | [3-(6,8-Difluoro-imidazo[1,2-a]pyridin-3-yl)-1-(2,2,2-trifluoro-éthyl)-1H-pyrazolo[4,3-c]pyridin-6-yl]-(2-trifluorométhyl-morpholin-4-yl)-méthanone |
| 164 | [3-(6,8-Difluoro-imidazo[1,2-a]pyridin-3-yl)-1-(2,2,2-trifluoro-éthyl)-1H-pyrazolo[4,3-c]pyridin-6-yl]-(4-trifluorométhyl-pipéridin-1-yl)-méthanone |
| 165 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxyméthyl-pipéridin-1-yl)-méthanone |
| 166 | [3-(6,8-Difluoro-imidazo[1,2-a]pyridin-3-yl)-1-(2,2,2-trifluoro-éthyl)-1H-pyrazolo[4,3-c]pyridin-6-yl]-(2-oxa-7-aza-spiro[3.5]non-7-yl)-méthanone |
| 167 | [3-(6,8-Difluoro-imidazo[1,2-a]pyridin-3-yl)-1-(2,2,2-trifluoro-éthyl)-1H-pyrazolo[4,3-c]pyridin-6-yl]-(3-méthoxyméthyl-morpholin-4-yl)-méthanone |
| 168 | [3-(6,8-Difluoro-imidazo[1,2-a]pyridin-3-yl)-1-(2,2,2-trifluoro-éthyl)-1H-pyrazolo[4,3-c]pyridin-6-yl]-(3-endo-hydroxy-8-azabicyclo-[3.2.1]oct-8-yl)-méthanone |
| 169 | (4-Aza-tricyclo[4.3.1.1*3,8*]undéc-4-yl)-[3-(6,8-difluoroimidazo-[1,2-a]pyridin-3-yl)-1-(2,2,2-trifluoro-éthyl)-1H-pyrazolo[4,3-c]-pyridin-6-yl]-méthanone |
| 170 | [3-(6,8-Difluoro-imidazo[1,2-a]pyridin-3-yl)-1-(2,2,2-trifluoro-éthyl)-1H-pyrazolo[4,3-c]pyridin-6-yl]-(8-exo-hydroxy-3-azabicyclo-[3.2.1]oct-3-yl)-méthanone |
| 171 | [3-(6,8-Difluoro-imidazo[1,2-a]pyridin-3-yl)-1-(2,2,2-trifluoro-éthyl)-1H-pyrazolo[4,3-c]pyridin-6-yl]-(3-exo-hydroxy-8-azabicyclo-[3.2.1]oct-8-yl)-méthanone |
| 172 | (8-Azabicyclo[3.2.1]oct-8-yl)-[3-(6,8-difluoro-imidazo[1,2-a]pyridin-3-yl)-1-(2,2,2-trifluoro-éthyl)-1H-pyrazolo[4,3-c]pyridin-6-yl]-méthanone |
| 173 | [3-(6,8-Difluoro-imidazo[1,2-a]pyridin-3-yl)-1-(2,2,2-trifluoro-éthyl)-1H-pyrazolo[4,3-c]pyridin-6-yl]-(4-difluorométhyl-pipéridin-1-yl)-méthanone |
| 174 | Acide 3-(6,8-difluoro-imidazo[1,2-a]pyridin-3-yl)-1-(2,2,2-trifluoro-éthyl)-1H-pyrazolo[4,3-c]pyridine-6-carboxylique (2-hydroxy-2-méthyl-propyl)-amide |
| 175 | [3-(6,8-Difluoro-imidazo[1,2-a]pyridin-3-yl)-1-(2,2,2-trifluoro-éthyl)-1H-pyrazolo[4,3-c]pyridin-6-yl]-(2-oxa-6-azatricyclo[3.3.1.1*3,7*]-déc-6-yl)-méthanone |
| 176 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxyméthyl-4-méthyl-pipéridin-1-yl)-méthanone |
| 177 | [3-(6,8-Difluoro-imidazo[1,2-a]pyridin-3-yl)-1-(2,2,2-trifluoro-éthyl)-1H-pyrazolo[4,3-c]pyridin-6-yl]-(4-hydroxy-azépan-1-yl)-méthanone |
| 178 | (2-Aza-tricyclo[3.3.1.1*3,7*]déc-2-yl)-[3-(6,8-difluoro-imidazo[1,2-a]pyridin-3-yl)-1-(2,2,2-trifluoro-éthyl)-1H-pyrazolo[4,3-c]pyridin-6-yl]-méthanone |
| 179 | [3-(6,8-Difluoro-imidazo[1,2-a]pyridin-3-yl)-1-(2,2,2-trifluoro-éthyl)-1H-pyrazolo[4,3-c]pyridin-6-yl]-(5-oxa-8-aza-spiro[2.6]non-8-yl)-méthanone |
| 180 | [3-(6,8-Difluoro-imidazo[1,2-a]pyridin-3-yl)-1-(2,2,2-trifluoro-éthyl)-1H-pyrazolo[4, 3-c] pyrid i n-6-yl]-1, 4-thiazépan-1,1-dioxyde-4-yl-méthanone |
| 181 | Cis-[3-(6,8-Difluoro-imidazo[1,2-a]pyridin-3-yl)-1-(2,2,2-trifluoro-éthyl)-1H-pyrazolo[4,3-c]pyridin-6-yl]-(2,6-diméthyl-morpholin-4-yl)-méthanone |
| 182 | [3-(6,8-Difluoro-imidazo[1,2-a]pyridin-3-yl)-1-(2,2,2-trifluoro-éthyl)-1H-pyrazolo[4,3-c]pyridin-6-yl]-(2,2-diméthyl-1,4-oxazépan-4-yl)-méthanone |
| 183 | Cis-[3-(6,8-Difluoro-imidazo[1,2-a]pyridin-3-yl)-1-(2,2,2-trifluoro-éthyl)-1H-pyrazolo[4,3-c]pyridin-6-yl]-(3,5-diméthyl-morpholin-4-yl)-méthanone |
| 184 | [3-(6,8-Difluoro-imidazo[1,2-a]pyridin-3-yl)-1-(2,2,2-trifluoro-éthyl)-1H-pyrazolo[4,3-c]pyridin-6-yl]-(3-oxa-8-azabicyclo[3.2.1]oct-8-yl)-méthanone |
| 185 | [3-(6,8-Difluoro-imidazo[1,2-a]pyridin-3-yl)-1-(2,2,2-trifluoro-éthyl)-1H-pyrazolo[4,3-c]pyridin-6-yl]-(6,6-difluoro-1,4-oxazépan-4-yl)-méthanone |
| 186 | [3-(6,8-Difluoro-imidazo[1,2-a]pyridin-3-yl)-1-(2,2,2-trifluoro-éthyl)-1H-pyrazolo[4,3-c]pyridin-6-yl]-(8-oxa-4-aza-spiro[2.6]non-4-yl)-méthanone |
| 187 | [3-(6,8-Difluoro-imidazo[1,2-a]pyridin-3-yl)-1-(2,2,2-trifluoro-éthyl)-1H-pyrazolo[4,3-c]pyridin-6-yl]-(8-endo-hydroxy-3-azabicyclo-[3.2.1]oct-3-yl)-méthanone |
| 188 | [3-(6,8-Difluoro-imidazo[1,2-a]pyridin-3-yl)-1-(2,2,2-trifluoro-éthyl)-1H-pyrazolo[4,3-c]pyridin-6-yl]-(7-endo-hydroxy-3-oxa-9-azabicyclo[3.3.1]non-9-yl)-méthanone |
| 189 | 1-[3-(6,8-Difluoro-imidazo[1,2-a]pyridin-3-yl)-1-(2,2,2-trifluoro-éthyl)-1H-pyrazolo[4,3-c]pyridine-6-carbonyl]-1,4-diazépan-5-one |
| 190 | [3-(6,8-Difluoro-imidazo[1,2-a]pyridin-3-yl)-1-(2,2,2-trifluoro-éthyl)-1H-pyrazolo[4,3-c]pyridin-6-yl]-1,4-oxazépan-deutérium-4-yl-méthanone |
| 191 | [3-(6,8-Difluoro-imidazo[1,2-a]pyridin-3-yl)-1-(2,2,2-trifluoro-éthyl)-1H-pyrazolo[4,3-c]pyridin-6-yl]-[1,2]oxazépan-2-yl-méthanone |
| 192 | [3-(6,8-Difluoro-imidazo[1,2-a]pyridin-3-yl)-1-(2,2,2-trifluoro-éthyl)-1H-pyrazolo[4,3-c]pyridin-6-yl]-(5-oxa-8-aza-spiro[3.5]non-8-yl)-méthanone |
| 193 | [3-(6,8-Difluoro-imidazo[1,2-a]pyridin-3-yl)-1-(2,2,2-trifluoro-éthyl)-1H-pyrazolo[4, 3-c] pyrid i n-6-yl]-(6-hyd roxy-1, 4-oxazépan-4-yl)-méthanone |
| 194 | Acide 5-[3-(6,8-difluoro-imidazo[1,2-a]pyridin-3-yl)-1-(2,2,2-trifluoro-éthyl)-1H-pyrazolo[4,3-c]pyridine-6-carbonyl]-2,5-diazabicyclo[2.2.1]heptane-2-carboxylique tertio-butyl ester |
| 195 | 1,4-Diazépan-1-yl-[3-(6,8-difluoro-imidazo[1,2-a]pyridin-3-yl)-1-(2,2,2-trifluoro-éthyl)-1H-pyrazolo[4,3-c]pyridin-6-yl]-méthanone |
| 196 | [3-(6,8-Difluoro-imidazo[1,2-a]pyridin-3-yl)-1-(2,2,2-trifluoro-éthyl)-1H-pyrazolo[4,3-c]pyridin-6-yl]-(8-oxa-5-aza-spiro[3.5]non-5-yl)-méthanone |
| 197 | Acide (2-{[3-(6,8-Difluoro-imidazo[1,2-a]pyridin-3-yl)-1-(2,2,2-trifluoro-éthyl)-1H-pyrazolo[4,3-c]pyridine-6-carbonyl]-méthyl-amino}-éthyl)-carbamique tertio-butyl ester |
| 198 | Acide 3-(6,8-difluoro-imidazo[1,2-a]pyridin-3-yl)-1-(2,2,2-trifluoro-éthyl)-1H-pyrazolo[4,3-c]pyridine-6-carboxylique (4-hydroxy-tétrahydro-pyran-3-yl)-amide |
| 199 | [3-(6,8-Difluoro-imidazo[1,2-a]pyridin-3-yl)-1-(2,2,2-trifluoro-éthyl)-1H-pyrazolo[4,3-c]pyridin-6-yl]-(6-oxa-3-azabicyclo[3.2.1]oct-3-yl)-méthanone |
| 200 | Acide 3-[3-(6,8-difluoro-imidazo[1,2-a]pyridin-3-yl)-1-(2,2,2-trifluoro-éthyl)-1H-pyrazolo[4,3-c]pyridine-6-carbonyl]-3,8-diazabicyclo[3.2.1]octane-8-carboxylique tertio-butyl ester |
| 201 | 4-[3-(6,8-Difluoro-imidazo[1,2-a]pyridin-3-yl)-1-(2,2,2-trifluoro-éthyl)-1H-pyrazolo[4,3-c]pyridine-6-carbonyl]-1,4-diazépan-2-one |
| 202 | Acide {1-[3-(6,8-difluoro-imidazo[1,2-a]pyridin-3-yl)-1-(2,2,2-trifluoro-éthyl)-1H-pyrazolo[4,3-c]pyridine-6-carbonyl]-azépan-3-yl}-carbamique tertio-butyl ester |
| 203 | (3,3-Difluoro-4-hydroxy-pipéridin-1-yl)-[3-(6,8-difluoro-imidazo[1,2-a]pyridin-3-yl)-1-(2,2,2-trifluoro-éthyl)-1H-pyrazolo[4,3-c]pyridin-6-yl]-méthanone |
| 204 | Acide {4-[3-(6,8-Difluoro-imidazo[1,2-a]pyridin-3-yl)-1-(2,2,2-trifluoro-éthyl)-1 H-pyrazolo[4,3-c]pyridine-6-carbonyl]-1,4-oxazépan-6-yl}-carbamique tertio-butyl ester |
| 205 | [3-(6,8-Difluoro-imidazo[1,2-a]pyridin-3-yl)-1-(2,2,2-trifluoro-éthyl)-1H-pyrazolo[4,3-c]pyridin-6-yl]-(2-oxa-5-azabicyclo[4.1.0]hept-5-yl)-méthanone |
| 206 | [3-(6,8-Difluoro-imidazo[1,2-a]pyridin-3-yl)-1-(2,2,2-trifluoro-éthyl)-1H-pyrazolo[4,3-c]pyridin-6-yl]-(7-oxa-4-aza-spiro[2.6]non-4-yl)-méthanone |
| 207 | [3-Benzofuran-3-yl-1-(2,2,2-trifluoro-éthyl)-1H-pyrazolo[4,3-c]-pyridin-6-yl]-(4-hydroxy-pipéridin-1-yl)-méthanone |
| 208 | [3-Benzofuran-3-yl-1-(2,2,2-trifluoro-éthyl)-1H-pyrazolo[4,3-c]-pyridin-6-yl]-(4-hydroxy-4-méthyl-pipéridin-1-yl)-méthanone |
| 209 | [3-Benzofuran-3-yl-1-(2,2,2-trifluoro-éthyl)-1H-pyrazolo[4,3-c]-pyridin-6-yl]-(3-endo-hydroxy-8-azabicyclo[3.2.1]oct-8-yl)-méthanone |
| 210 | [3-Benzofuran-3-yl-1-(2,2,2-trifluoro-éthyl)-1H-pyrazolo[4,3-c]-pyridin-6-yl]-(8-exo-éthyl-8-endo-hydroxy-3-azabicyclo[3.2.1]oct-3-yl)-méthanone |
| 211 | [3-Benzofuran-3-yl-1-(2,2,2-trifluoro-éthyl)-1H-pyrazolo[4,3-c]-pyridin-6-yl]-(5-endo-hydroxy-5-exo-méthyl-2-azabicyclo[2.2.1]-hept-2-yl)-méthanone |
| 212 | [3-Benzofuran-3-yl-1-(2,2,2-trifluoro-éthyl)-1H-pyrazolo[4,3-c]-pyridin-6-yl]-(8-endo-hydroxy-8-exo-méthyl-3-azabicyclo[3.2.1]oct-3-yl)-méthanone |
| 213 | [3-Benzofuran-3-yl-1-(2,2,2-trifluoro-éthyl)-1H-pyrazolo[4,3-c]-pyridin-6-yl]-(8-endo-hydroxy-8-exo-méthyl-d3-3-azabicyclo-[3.2.1]oct-3-yl)-méthanone |
| 214 | [3-Benzofuran-3-yl-1-(2,2,2-trifluoro-éthyl)-1H-pyrazolo[4,3-c]-pyridin-6-yl]-(8-exo-éthynyl-8-endo-hydroxy-3-azabicyclo-[3.2.1]oct-3-yl)-méthanone |
| 215 | [3-Benzofuran-3-yl-1-(2,2,2-trifluoro-éthyl)-1H-pyrazolo[4,3-c]-pyridin-6-yl]-(2-méthoxyméthyl-pyrrolidin-1-yl)-méthanone |
| 216 | [3-Benzofuran-3-yl-1-(2,2,2-trifluoro-éthyl)-1H-pyrazolo[4,3-c]-pyridin-6-yl]-(4-fluoro-pipéridin-1-yl)-méthanone |
| 217 | 3-Benzofuran-3-yl-1-(2,2,2-trifluoro-éthyl)-1H-pyrazolo[4,3-c]-pyridin-6-yl]-(6,6-difluoro-1,4-oxazépan-4-yl)-méthanone |
| 218 | [3-Benzofuran-3-yl-1-(2,2,2-trifluoro-éthyl)-1H-pyrazolo[4,3-c]-pyridin-6-yl]-(8-endo-hydroxy-3-azabicyclo[3.2.1]oct-3-yl)-méthanone |
| 219 | [3-Benzofuran-3-yl-1-(2,2,2-trifluoro-éthyl)-1H-pyrazolo[4,3-c]-pyridin-6-yl]-(4-hydroxy-2,2-diméthyl-pipéridin-1-yl)-méthanone |
| 220 | [3-Benzofuran-3-yl-1-(2,2-difluoro-éthyl)-1H-pyrazolo[4,3-c]-pyridin-6-yl]-(3-endo-hydroxy-8-azabicyclo[3.2.1]oct-8-yl)-méthanone |
| 221 | [3-Benzofuran-3-yl-1-(2-fluoro-éthyl)-1H-pyrazolo[4,3-c]pyridin-6-yl]-(3-endo-hydroxy-8-azabicyclo[3.2.1]oct-8-yl)-méthanone |
| 222 | (3-Benzofuran-3-yl-1-propyl-1H-pyrazolo[4,3-c]pyridin-6-yl)-(3-endo-hydroxy-8-azabicyclo[3.2.1]oct-8-yl)-méthanone |
| 223 | N-[4-[3-(6,8-Difluoroimidazo[1,2-a]pyridin-3-yl)-1-(2,2,2-trifluoroéthyl)pyrazolo[4,3-c]pyridine-6-carbonyl]-1,4-oxazépan-6-yl]carbamate de tertio-butyle |
| 224 | [3-(Benzofuran-3-yl)-1-(2,2,2-trifluoroéthyl)pyrazolo[4,3-c]pyridin-6-yl]-(6-hydroxy-1,4-oxazépan-4-yl)méthanone |
| 225 | [3-(Benzofuran-3-yl)-1-(2,2,2-trifluoroéthyl)pyrazolo[4,3-c]pyridin-6-yl]-(4,4-difluoro-1-pipéridyl)méthanone |
| 226 | [3-(Benzofuran-3-yl)-1-(2,2,2-trifluoroéthyl)pyrazolo[4,3-c]pyridin-6-yl]-(3,3-difluoro-1-pipéridyl)méthanone |
| 227 | [3-Benzofuran-3-yl-1-(2,2,2-trifluoro-éthyl)-1H-pyrazolo[4,3-c]-pyridin-6-yl]-(3-hydroxy-pipéridin-1-yl)-méthanone |
| 228 | [3-(Benzofuran-3-yl)-1-(2,2,2-trifluoroéthyl)pyrazolo[4,3-c]pyridin-6-yl]-(4-hydroxyazépan-1-yl)méthanone |
| 229 | [3-(Benzofuran-3-yl)-1-(2,2,2-trifluoroéthyl)pyrazolo[4,3-c]pyridin-6-yl]-(6,6-dideutério-1,4-oxazépan-4-yl)méthanone |
| 230 | [3-(Benzofuran-3-yl)-1-(2,2,2-trifluoroéthyl)pyrazolo[4,3-c]pyridin-6-yl]-[6-fluoro-1,4-oxazépan-4-yl]méthanone |
| 231 | 3-(Benzofuran-3-yl)-N, N-di(cyclobutyl)-1-(2,2,2-trifluoroéthyl)-pyrazolo[4,3-c]pyridine-6-carboxamide |
| 232 | 3-(Benzofuran-3-yl)-N,N-di(cyclopropyl)-1-(2,2,2-trifluoroéthyl)-pyrazolo[4,3-c]pyridine-6-carboxamide |
| 233 | 3-(Benzofuran-3-yl)-N,N-diisopropyl-1-(2,2,2-trifluoroéthyl)-pyrazolo[4,3-c]pyridine-6-carboxamide |
| 234 | 3-(Benzofuran-3-yl)-N-éthyl-N-isopropyl-1-(2,2,2-trifluoroéthyl)-pyrazolo[4,3-c]pyridine-6-carboxamide |
| 235 | 3-(Benzofuran-3-yl)-N-cyclohexyl-N-éthyl-1-(2,2,2-trifluoroéthyl)-pyrazolo[4,3-c]pyridine-6-carboxamide |
| 236 | [3-(Benzofuran-3-yl)-1-(2,2,2-trifluoroéthyl)pyrazolo[4,3-c]pyridin-6-yl]-(8-hydroxy-8-isopropyl-3-azabicyclo[3.2.1]octan-3-yl)-méthanone |
| 237 | 3-[6-(1,4-oxazépane-4-carbonyl)-1-(2,2,2-trifluoroéthyl)pyrazolo-[4,3-c]pyridin-3-yl]benzofuran-5-carboxamide |
| 238 | (3,8-Diazabicyclo[3.2.1]oct-3-yl)-[3-(6,8-difluoro-imidazo[1,2-a]-pyridin-3-yl)-1-(2,2,2-trifluoro-éthyl)-1H-pyrazolo[4,3-c]pyridin-6-yl]-méthanone |
| 239 | (2,5-Diazabicyclo[2.2.1]hept-2-yl)-[3-(6,8-difluoro-imidazo[1,2-a]-pyridin-3-yl)-1-(2,2,2-trifluoro-éthyl)-1H-pyrazolo[4,3-c]pyridin-6-yl]-méthanone |
| 240 | (3-Amino-azépan-1-yl)-[3-(6,8-difluoro-imidazo[1,2-a]pyridin-3-yl)-1-(2,2,2-trifluoro-éthyl)-1H-pyrazolo[4,3-c]pyridin-6-yl]-méthanone |
| 241 | (6-Amino-1,4-oxazépan-4-yl)-[3-(6,8-difluoro-imidazo[1,2-a]-pyridin-3-yl)-1-(2,2,2-trifluoro-éthyl)-1H-pyrazolo[4,3-c]pyridin-6-yl]-méthanone |
| 242 | (6,6-Dideutério-1,4-oxazépan-4-yl)-[3-(1H-indol-3-yl)-1-(2,2,2-trifluoroéthyl)pyrazolo[4,3-c]pyridin-6-yl]méthanone |
| 243 | [3-(1H-indol-3-yl)-1-(2,2,2-trifluoroéthyl)pyrazolo[4,3-c]pyridin-6-yl]-(8-oxa-3-azabicyclo[3.2. 1]octan-3-yl)méthanone |
| 244 | Acide 3-[6-(azépane-1-carbonyl)-1-(2,2,2-trifluoro-éthyl)-1H-pyrazolo[4,3-c]pyridin-3-yl]-8-méthyl-imidazo[1,2-a]pyridine-6-carboxylique amide |
| 245 | Acide 3-[6-(azépane-1-carbonyl)-1-propyl-1H-pyrazolo[4,3-c]-pyridin-3-yl]-imidazo[1,2-a]pyridine-6-carboxylique amide |
| 246 | Acide 3-[6-(1,4-oxazépane-4-carbonyl)-1-(2,2,2-trifluoro-éthyl)-1H-pyrazolo[4,3-c]pyridin-3-yl]-imidazo[1,2-a]pyridine-6-carboxylique amide |
| 247 | Acide 3-[6-(8-oxa-3-azabicyclo[3.2.1]octane-3-carbonyl)-1-(2,2,2-trifluoro-éthyl)-1H-pyrazolo[4,3-c]pyridin-3-yl]-imidazo[1,2-a]-pyridine-6-carboxylique amide |
| 248 | Azépan-1-yl-[3-pyrazolo[1,5-a]pyrazin-3-yl-1-(2,2,2-trifluoro-éthyl)-1H-pyrazolo[4,3-c]pyridin-6-yl]-méthanone |
| 249 | 1,4-Oxazépan-4-yl-[3-pyrazolo[1,5-a]pyrazin-3-yl-1-(2,2,2-trifluoro-éthyl)-1H-pyrazolo[4,3-c]pyridin-6-yl]-méthanone |
| 250 | (1-Méthanesulfonylméthyl-3-pyrazolo[1,5-a]pyrazin-3-yl-1H-pyrazolo[4,3-c]pyridin-6-yl)-(1,4-oxazépan-4-yl)-méthanone |
| 251 | [3-(Benzimidazol-1-yl)-1-(2,2,2-trifluoroéthyl)pyrazolo[4,3-c]-pyridin-6-yl]-(1,4-oxazépan-4-yl)méthanone |
| 252 | [3-(6-Fluorobenzimidazol-1-yl)-1-(2,2,2-trifluoroéthyl)pyrazolo[4,3-c]pyridin-6-yl]-(1,4-oxazépan-4-yl)méthanone |
| 253 | [3-Furo[3,2-b]pyridin-3-yl-1-(2,2,2-trifluoroéthyl)pyrazolo[4,3-c]-pyridin-6-yl]-(1,4-oxazépan-4-yl)méthanone |
| 254 | [1,4]-Oxazépan-4-yl-[3-(1H-pyrrolo[3,2-b]pyridin-3-yl)-1-(2,2,2-trifluoro-éthyl)-1H-pyrazolo[4,3-c]pyridin-6-yl]-méthanone |
| 255 | [1,4]-Oxazépan-4-yl-[3-(1H-pyrrolo[2,3-c]pyridin-3-yl)-1-(2,2,2-trifluoro-éthyl)-1H-pyrazolo[4,3-c]pyridin-6-yl]-méthanone |
| 256 | (8-Oxa-3-azabicyclo[3.2.1]oct-3-yl)-[3-(1H-pyrrolo[3,2-c]pyridin-3-yl)-1-(2,2,2-trifluoro-éthyl)-1H-pyrazolo[4,3-c]pyridin-6-yl]-méthanone |
ainsi que les sels ou solvates pharmaceutiquement acceptables de l'un quelconque parmi les composés susmentionnés.

12. Composé selon l'une quelconque des revendications 1 à 11, pour une utilisation comme médicament.

13. Composition pharmaceutique comprenant le composé selon l'une quelconque des revendications 1 à 11 et un excipient pharmaceutiquement acceptable.

14. Composé selon l'une quelconque des revendications 1 à 11, ou composition pharmaceutique selon la revendication 13, pour une utilisation dans le traitement ou la prévention d'une maladie ou d'un trouble hyper-prolifératif ou d'une maladie ou d'un trouble infectieux.

15. Utilisation du composé selon l'une quelconque des revendications 1 à 11, dans la préparation d'un médicament destiné au traitement ou à la prévention d'une maladie ou d'un trouble hyper-prolifératif ou d'une maladie ou d'un trouble infectieux.

16. Composé pour une utilisation selon la revendication 14, ou composition pharmaceutique pour une utilisation selon la revendication 14, ou utilisation selon la revendication 15, la maladie ou le trouble devant être traité ou empêché étant une maladie ou un trouble hyper-prolifératif, et ladite maladie ou ledit trouble hyper-prolifératif étant un cancer.

17. Composé pour une utilisation selon la revendication 16, ou composition pharmaceutique pour une utilisation selon la revendication 16, ou utilisation selon la revendication 16, le cancer étant choisi dans le groupe constitué par la leucémie granuleuse aiguë, la leucémie lymphoïde aiguë, la leucémie myéloïde aiguë, le cancer du cortex surrénal, le cancer de la vessie, le cancer du cerveau, le cancer du sein, le cancer du col de l'utérus, l'hyperplasie du col de l'utérus, le cancer chorionique, la leucémie granuleuse chronique, la leucémie lymphoïde chronique, la leucémie myéloïde chronique, le cancer du côlon, le cancer de l'endomètre, le cancer de l'oesophage, la thrombocytose essentielle, un carcinome génito-urinaire, un gliome, un glioblastome, la leucémie à cellules velues, un carcinome de la tête et du cou, la maladie de Hodgkin, le sarcome de Kaposi, un carcinome pulmonaire, un lymphome, un carcinome carcinoïde malin, l'hypercal-cémie maligne, un mélanome malin, un insulinome pancréatique malin, un carcinome médullaire de la thyroïde, un mélanome, un myélome multiple, le mycosis fongoïde, un neuroblastome, un lymphome non hodgkinien, le cancer du poumon non à petites cellules, un sarcome ostéogénique, un carcinome ovarien, un carcinome pancréatique, la maladie de Vasquez, un carcinome cérébral primitif, la macroglobulinémie primitive, le cancer de la prostate, le cancer des cellules rénales, un rhabdomyosarcome, le cancer de la peau, le cancer du poumon à petites cellules, un sarcome des tissus mous, le cancer épidermoïde, le cancer de l'estomac, le cancer des testicules, le cancer de la thyroïde et la tumeur de Wilms.

18. Composé pour une utilisation selon la revendication 14, ou composition pharmaceutique pour une utilisation selon la revendication 14, ou utilisation selon la revendication 15, la maladie ou le trouble devant être traité ou empêché étant une maladie ou un trouble hyper-prolifératif, et ladite maladie ou ledit trouble hyper-prolifératif étant choisi dans le groupe constitué par la dégénérescence maculaire liée à l'âge, la maladie de Crohn, la cirrhose, un trouble chronique lié aux inflammations, la rétinopathie diabétique proliférante, la vitréorétinopathie proliférante, la rétinopathie du prématuré, la granulomatose, l'hyperprolifération immunitaire associée à la greffe d'organes ou de tissus, et un(e) maladie/trouble immuno-prolifératif.

19. Composé pour une utilisation selon la revendication 18, ou composition pharmaceutique pour une utilisation selon la revendication 18, ou utilisation selon la revendication 18, la maladie ou le trouble devant être traité ou empêché étant une maladie ou un trouble immuno-prolifératif, et la maladie ou le trouble immuno-prolifératif étant choisi dans le groupe constitué par l'affection abdominale inflammatoire, le psoriasis, la polyarthrite rhumatoïde, le lupus érythémateux disséminé (LED), l'hyperprolifération vasculaire secondaire à l'hypoxie rétinienne et la vascularite.

20. Composé pour une utilisation selon la revendication 14, ou composition pharmaceutique pour une utilisation selon la revendication 14, ou utilisation selon la revendication 15, la maladie ou le trouble devant être traité ou empêché étant une maladie ou un trouble infectieux, et ladite maladie ou ledit trouble infectieux étant choisi dans le groupe constitué par :
(a) les maladies infectieuses induites par voie virale, qui sont provoquées par les rétrovirus, les hépadnavirus, les herpèsvirus, les Flaviviridae et/ou les adénovirus, où les rétrovirus sont choisis parmi les lentivirus ou les oncorétrovirus, où le lentivirus est choisi dans le groupe constitué par le VIH-1, le VIH-2, le VIF, le BIV, les SIV, le SHIV, le CAEV, le VMV et l'EIAV, où l'oncorétrovirus est choisi dans le groupe constitué par le HTLV-I, le HTLV-II et le BLV, où l'hépadnavirus est choisi dans le groupe constitué par le HBV, le GSHV et le WHV, où l'herpèsvirus est choisi dans le groupe constitué par le HSV I, le HSV II, l'EBV, le VZV, le HCMV ou le HHV 8 et où les Flaviviridae sont choisis dans le groupe constitué par le HCV, le virus du Nil occidental et le virus de la fièvre jaune ;
(b) les maladies infectieuses bactériennes qui sont provoquées par les bactéries à Gram positif, où les bactéries à Gram positif sont choisies dans le groupe constitué par les staphylocoques sensibles à la méthicilline et résistants à la méthicilline (y compris *Staphylococcus aureus, Staphylococcus epidermidis, Staphylococcus haemolyticus, Staphylococcus hominis, Staphylococcus saprophyticus,* et les staphylocoques négatifs à la coagulase), *Staphylococcus aureus* de sensibilité intermédiaire aux glycopeptides (GISA), les streptocoques sensibles à la pénicilline et résistants à la pénicilline (y compris *Streptococcus pneumoniae, Streptococcus pyogenes, Streptococcus agalactiae, Streptococcus avium, Streptococcus bovis, Streptococcus lactis, Streptococcus sanguis* et les Streptocoques du Groupe C (GCS), les Streptocoques du Groupe G (GGS) et les streptocoques viridans), les entérocoques (y compris les souches sensibles à la vancomycine et résistantes à la vancomycine telles *qu'Enterococcus faecalis* et *Enterococcus faecium), Clostridium difficile, Listeria monocytogenes, Corynebacterium jeikeium, Chlamydia* spp. (y compris *C*. *pneumoniae*) et *Mycobacterium tuberculosis* ;
(c) les maladies infectieuses bactériennes qui sont provoquées par les bactéries à Gram négatif, où les bactéries à Gram négatif sont choisies dans le groupe constitué par le genre *Enterobacteriaceae,* y compris *Escherichia* spp. (y compris *Escherichia coli*)*, Klebsiella* spp., *Enterobacterspp., Citrobacter* spp., *Serratia* spp., *Proteus* spp., *Providencia* spp., *Salmonella* spp., *Shigella* spp., le genre *Pseudomonas* (y compris *P. aeruginosa), Moraxella spp. (y* compris *M. catarrhalis*), *Haemophilus* spp. et *Neisseria* spp. ; et
(d) les maladies infectieuses induites par les parasites actifs par voie intracellulaire choisis dans le groupe constitué par le phylum *Apicomplexa, Sarcomastigophora* (y compris *Trypanosoma, Plasmodia, Leishmania, Babesia* ou *Theileria), Cryptosporidiidae, Sacrocystidae, Amobidae, Coccidia* et *Trichomonadida.*

21. Composé pour une utilisation selon l'une quelconque des revendications 14 ou 16 à 20, ou composition pharmaceutique pour une utilisation selon l'une quelconque des revendications 14 ou 16 à 20, ou utilisation selon l'une quelconque des revendications 15 ou 16 à 20, ledit composé, ladite composition pharmaceutique ou ledit médicament devant être administré(e) en combinaison avec un ou plusieurs autres agents thérapeutiques.

22. Composé pour une utilisation selon l'une quelconque des revendications 14 ou 16 à 21, ou composition pharmaceutique pour une utilisation selon l'une quelconque des revendications 14 ou 16 à 21, ou utilisation selon l'une quelconque des revendications 15 ou 16 à 21, le sujet devant être traité étant un être humain.

23. Ensemble ou kit comprenant des conditionnements séparés :
(a) d'un composé tel que défini selon l'une quelconque des revendications 1 à 11 ; et
(b) d'un autre agent thérapeutique.

24. Utilisation in *vitro* d'un composé tel que défini selon l'une quelconque des revendications 1 à 11, comme double antagoniste des récepteurs A_{2A} et A_{2B} de l'adénosine.
